# EUROPÄISCHE PATENTSCHRIFT

(11) **EP 1 613 746 B1**
(45) Veröffentlichungstag und Bekanntmachung des Hinweises auf die Patenterteilung: **06.03.2013**
(21) Anmeldenummer: 04723591.6
(22) Anmeldetag: 26.03.2004
(51) Int. Cl.: C12N 9/10, C12N 15/82, C12P 7/64, A01H 5/00

(54) **NEUE PFLANZLICHE ACYLTRANSFERASEN SPEZIFISCH FÜR LANGKETTIGE, MEHRFACH UNGESÄTTIGTE FETTSÄUREN**
NOVEL PLANT ACYLTRANSFERASES SPECIFIC FOR LONG-CHAINED, MULTIPLY UNSATURATED FATTY ACIDS
NOUVELLES ACYLTRANSFERASES VEGETALES SPECIFIQUES POUR ACIDES GRAS A LONGUE CHAINE POLYINSATURES

(30) Priorität: 31.03.2003 DE 10314759; 17.10.2003 DE 10348996
(43) Veröffentlichungstag der Anmeldung: 11.01.2006
(60) Teilanmeldung: 11156830.9
(73) Patentinhaber: UNIVERSITY OF BRISTOL, Bristol BS8 1TH (GB)
(72) Erfinder: RENZ, Andreas, 67117 Limburgerhof (DE); BAUER, Jörg, 67063 Ludwigshafen (DE); FRENTZEN, Margit, 52072 Aachen (DE); SÖZER, Nursen, 52531 Übach-Palenberg (DE); KEITH, Stobart, Bristol BS7 8EN (GB); FRASER, Thomas, Bristol BS9 4NL (GB); LAZARUS, Colin, M., Bristol BS6 5QG (GB); QI, Baoxiu, Bath BAI 2BG (GB); ABBADI, Amine, 22549 Hamburg (DE); HEINZ, Ernst, 22609 Hamburg (DE)
(74) Vertreter: Pressler, Uwe
(86) Internationale Anmeldenummer: PCT/EP2004/003224
(87) Internationale Veröffentlichungsnummer: WO 2004/087902

(56) Entgegenhaltungen:
- WO-A-00/18889
- WO-A-01/59128
- WO-A-02/072742
- LOPEZ ALONSO D ET AL: "Plants as 'chemical factories' for the production of polyunsaturated fatty acids" BIOTECHNOLOGY ADVANCES, ELSEVIER PUBLISHING, BARKING, GB, Bd. 18, Nr. 6, Oktober 2000 (2000-10), Seiten 481-497, XP002208312 ISSN: 0734-9750
- KNUTZON D S ET AL: "CLONING OF A COCONUT ENDOSPERM CDNA ENCODING A 1-ACYL-SN-GLYCEROL-3-PHOSPHATE ACYLTRANSFERASE THAT ACCEPTS MEDIUM-CHAIN-LENGHT SUBSTRATES" PLANT PHYSIOLOGY, AMERICAN SOCIETY OF PLANT PHYSIOLOGISTS, ROCKVILLE, MD, US, Bd. 109, Nr. 3, 1995, Seiten 999-1006, XP000907268 ISSN: 0032-0889 in der Anmeldung erwähnt
- LASSNER M W ET AL: "LYSOPHOSPHATIDIC ACID ACYLTRANSFERASE FROM MEADOWFOAM MEDIATES INSERTION OF ERUCIC ACID AT THE SN-2 POSITION OF TRIACYLGLYCEROL IN TRANSGENIC RAPESEED OIL" PLANT PHYSIOLOGY, AMERICAN SOCIETY OF PLANT PHYSIOLOGISTS, ROCKVILLE, MD, US, Bd. 109, 1995, Seiten 1389-1394, XP002003604 ISSN: 0032-0889
- HOBBS D H ET AL: "Cloning of a cDNA encoding diacylglycerol acyltransferase from Arabidopsis thaliana and its functional expression" FEBS LETTERS, ELSEVIER SCIENCE PUBLISHERS, AMSTERDAM, NL, Bd. 452, Nr. 3, 11. Juni 1999 (1999-06-11), Seiten 145-149, XP004259739 ISSN: 0014-5793
- DATABASE EMBL [Online] Vicia faba putative GPAT mRNA 23. September 1998 (1998-09-23), LIU ET AL.: "Cloning and Sequencing of the cDNA (Accession No. AF090734) Coding for Glycerol-3-Phosphate Acyltransferase from Vicia faba. (PGR99-094)" XP002295954 gefunden im EBI Database accession no. AF090734
- JAKO C ET AL: "Seed-specific over-expression of an Arabidopsis cDNA encoding a diacylglycerol acyltransferase enhances seed oil content and seed weight" PLANT PHYSIOLOGY, AMERICAN SOCIETY OF PLANT PHYSIOLOGISTS, ROCKVILLE, MD, US, Bd. 126, Nr. 2, Juni 2001 (2001-06), Seiten 861-874, XP002242963 ISSN: 0032-0889
- ZOU ET AL: "The Arabidopsis thaliana TAG1 mutant has a mutation in a diacylglycerol acyltransferase gene" PLANT JOURNAL, BLACKWELL SCIENTIFIC PUBLICATIONS, OXFORD, GB, Bd. 19, Nr. 6, 1999, Seiten 645-653, XP002133607 ISSN: 0960-7412
- MCLEAN J ET AL: "CLONING AND EXPRESSION OF HUMAN LECITHIN-CHOLESTEROL ACYLTRANSFERASE CDNA" PROCEEDINGS OF THE NATIONAL ACADEMY OF SCIENCES OF USA, NATIONAL ACADEMY OF SCIENCE. WASHINGTON, US, Bd. 83, 1. April 1986 (1986-04-01), Seiten 2335-2339, XP002008904 ISSN: 0027-8424
- OELKERS PETER ET AL: "A lecithin cholesterol acyltransferase-like gene mediates diacylglycerol esterification in yeast" JOURNAL OF BIOLOGICAL CHEMISTRY, Bd. 275, Nr. 21, 26. Mai 2000 (2000-05-26), Seiten 15609-15612, XP002295953 ISSN: 0021-9258

### SEQUENCE LISTING

<110> BASF Plant Science GmbH
<120> Neue pflanzliche Acyltransferasen spezifisch für langkettige mehrfach ungesättigte Fettsäuren
<130> AE20011000
<140> AE20011000
   <141> 2003-03-31
<160> 74
<170> PatentIn version 3.1
<210> 1
   <211> 1047
   <212> DNA
   <213> Thraustochytrium
<220>
   <221> CDS
   <222> (38)..(952)
   <223> LPAAT
<400> 1
   gggcggtgtc cggccgttcg agcgcgtgga cgccaac atg agc gcg tgg acg agg       55
                                           Met Ser Ala Trp Thr Arg
                                           1               5
   gcc aag acc gcc gtg ggc ctc ctg acg ctg gcg cct gcg cgg ata gtg       103
   Ala Lys Thr Ala Val Gly Leu Leu Thr Leu Ala Pro Ala Arg Ile Val
               10                  15                  20
   ttc ctc gtg act gtc ctg ggc acg tac ggg ctc acg gtc gcg gcc tgc       151
   Phe Leu Val Thr Val Leu Gly Thr Tyr Gly Leu Thr Val Ala Ala Cys
           25                  30                  35
   acg cga ctt ggc gtc ccg aaa agc ttc gtg ctg ggc ctg acg cgg tgc       199
   Thr Arg Leu Gly Val Pro Lys Ser Phe Val Leu Gly Leu Thr Arg Cys
       40                  45                  50
   gtc gcg cga ctc acg ctc tgg ggg ctt ggg ttc tac cac att gag gtc       247
   Val Ala Arg Leu Thr Leu Trp Gly Leu Gly Phe Tyr His Ile Glu Val
   55                  60                  65                  70
   tct tgc gac gcc caa ggc ctt cgg gag tgg ccg cgc gtg att gtc gcg       295
   Ser Cys Asp Ala Gln Gly Leu Arg Glu Trp Pro Arg Val Ile Val Ala
                   75                  80                  85
   aac cac gtc tcg tac ctg gag atc ttg tac ttc atg tcg acc gtg cac       343
   Asn His Val Ser Tyr Leu Glu Ile Leu Tyr Phe Met Ser Thr Val His
               90                  95                  100
   tgc ccg tct ttc gtc atg aag aag acc tgc ctc cga gtc ccg ctt gtc       391
   Cys Pro Ser Phe Val Met Lys Lys Thr Cys Leu Arg Val Pro Leu Val
           105                 110                 115
   ggc tac att gcc atg gag ctg ggc ggt gtg att gtg gac cgc gag ggc      439
   Gly Tyr Ile Ala Met Glu Leu Gly Gly Val Ile Val Asp Arg Glu Gly
       120                 125                 130
   ggc ggt caa agc gca tcg gcg atc att cgc gac cgc gtg cag gag cct      487
   Gly Gly Gln Ser Ala Ser Ala Ile Ile Arg Asp Arg Val Gln Glu Pro
   135                 140                 145                 150
   cct cga gat tcg tcg agc gag aag cac cac gcg cag ccg ctt ctt gtg      535
   Pro Arg Asp Ser Ser Ser Glu Lys His His Ala Gln Pro Leu Leu Val
                   155                 160                 165
   ttc ccc gag ggg acc acc acc aat gga agc tgc ctg ctc caa ttc aag      583
   Phe Pro Glu Gly Thr Thr Thr Asn Gly Ser Cys Leu Leu Gln Phe Lys
               170                 175                 180
   acg gga gcc ttt cgt cct ggg gct ccg gtg ctt ccg gtc gtg ctt gag      631
   Thr Gly Ala Phe Arg Pro Gly Ala Pro Val Leu Pro Val Val Leu Glu
           185                 190                 195
   ttt ccg att gac aaa gcg cgt ggt gac ttt tcc ccg gcg tac gaa tcg      679
   Phe Pro Ile Asp Lys Ala Arg Gly Asp Phe Ser Pro Ala Tyr Glu Ser
       200                 205                 210
   gtc cac acg cca gct cac ctc ctt cgc atg ctc gca caa tgg agg cac      727
   Val His Thr Pro Ala His Leu Leu Arg Met Leu Ala Gln Trp Arg His
   215                 220                 225                 230
   cgg ctt cgg gtg cgc tat ctt cct ctg tat gag ccc tct gcg gct gag      775
   Arg Leu Arg Val Arg Tyr Leu Pro Leu Tyr Glu Pro Ser Ala Ala Glu
                   235                 240                 245
   aag gtt gat gca gac ctt tat gcg cgg aac gtg cgc gac gaa atg gcg      823
   Lys Val Asp Ala Asp Leu Tyr Ala Arg Asn Val Arg Asp Glu Met Ala
               250                 255                 260
   cgc gcg ctc aag gta ccc act gtg gag cag tct tac cgc gac aag ctc      871
   Arg Ala Leu Lys Val Pro Thr Val Glu Gln Ser Tyr Arg Asp Lys Leu
           265                 270                 275
   gtc tac cac gcg gat ctc atg ccg cac tac cag aag gcc ggc ccc gga      919
   Val Tyr His Ala Asp Leu Met Pro His Tyr Gln Lys Ala Gly Pro Gly
       280                 285                 290
   gcg ctc tat ctg tac gtc cga cct gac ctc ttg tagcactcat gcgcgtccca    972
   Ala Leu Tyr Leu Tyr Val Arg Pro Asp Leu Leu
   295                 300                 305
   agcggtccag caacgggaga ttaaaacacg atttcttagc ctacaaaaaa aaaaaaaaaa   1032
   aaaaaaaaaa aaaaa                                                    1047
<210> 2
   <211> 305
   <212> PRT
   <213> Thraustochytrium
<400> 2
   Met Ser Ala Trp Thr Arg Ala Lys Thr Ala Val Gly Leu Leu Thr Leu
   1               5                   10                  15
   Ala Pro Ala Arg Ile Val Phe Leu Val Thr Val Leu Gly Thr Tyr Gly
               20                  25                  30
   Leu Thr Val Ala Ala Cys Thr Arg Leu Gly Val Pro Lys Ser Phe Val
           35                  40                  45
   Leu Gly Leu Thr Arg Cys Val Ala Arg Leu Thr Leu Trp Gly Leu Gly
       50                  55                  60
   Phe Tyr His Ile Glu Val Ser Cys Asp Ala Gln Gly Leu Arg Glu Trp
   65                  70                  75                  80
   Pro Arg Val Ile Val Ala Asn His Val Ser Tyr Leu Glu Ile Leu Tyr
                   85                  90                  95
   Phe Met Ser Thr Val His Cys Pro Ser Phe Val Met Lys Lys Thr Cys
               100                 105                 110
   Leu Arg Val Pro Leu Val Gly Tyr Ile Ala Met Glu Leu Gly Gly Val
           115                 120                 125
   Ile Val Asp Arg Glu Gly Gly Gly Gln Ser Ala Ser Ala Ile Ile Arg
       130                 135                 140
   Asp Arg Val Gln Glu Pro Pro Arg Asp Ser Ser Ser Glu Lys His His
   145                 150                 155                 160
   Ala Gln Pro Leu Leu Val Phe Pro Glu Gly Thr Thr Thr Asn Gly Ser
                   165                 170                 175
   Cys Leu Leu Gln Phe Lys Thr Gly Ala Phe Arg Pro Gly Ala Pro Val
               180                 185                 190
   Leu Pro Val Val Leu Glu Phe Pro Ile Asp Lys Ala Arg Gly Asp Phe
           195                 200                 205
   Ser Pro Ala Tyr Glu Ser Val His Thr Pro Ala His Leu Leu Arg Met
       210                 215                 220
   Leu Ala Gln Trp Arg His Arg Leu Arg Val Arg Tyr Leu Pro Leu Tyr
   225                 230                 235                 240
   Glu Pro Ser Ala Ala Glu Lys Val Asp Ala Asp Leu Tyr Ala Arg Asn
                   245                 250                 255
   Val Arg Asp Glu Met Ala Arg Ala Leu Lys Val Pro Thr Val Glu Gln
               260                 265                 270
   Ser Tyr Arg Asp Lys Leu Val Tyr His Ala Asp Leu Met Pro His Tyr
           275                 280                 285
   Gln Lys Ala Gly Pro Gly Ala Leu Tyr Leu Tyr Val Arg Pro Asp Leu
       290                 295                 300
   Leu
   305
<210> 3
   <211> 1701
   <212> DNA
   <213> Physcomitrella patens
<220>
   <221> misc_feature
   <223> LPAAT
<400> 3
   ggcacgaggg aaattggctt tctatgtggc cgtacttatt cgaggaggtc aacgaaacaa      60
   aggtatgtct tattaatgaa aatgtctcca cacatgtatg ttgtttaggt atattctgtc     120
   aactgaaaac ttgttttaat tttttcttaa attgaaattc tgtgcctgaa agccaactct     180
   aggtccatca taatgtagca atatgatcag aagcgctcaa atgtgtcgtg aaagtttgct     240
   tttgcaattt tcttttgctg ttaacctatt gattatgttg gaaccacaat acagacgctg     300
   cttcacttca ttcttatggc aatgaatgtc gtgatgattc cggttaattt catcctacag     360
   ggatatggat gttgtaaagg tgatttttgc aggtgataaa gtacctaagg agaaccgtgt     420
   gatggtcatg tgcaaccatc gtaccgaagt ggactggatg tacatttgga acttagcaat     480
   tcggaaaggc aagattgggt actgcaagta tgcggtgaag aactcagtga aaaacttacc     540
   cttgtttggt tgggcatttt acgtttttga gtttctgatg ctgcatagaa agtgggaagt     600
   ggatgctccc gtcatcaaga catacattga cagttttcaa gataaaagag atcctctctg     660
   gctagtcgtg tttcctgaag gcacagattt ttcgtaaggc tgaagtaccc atccatggct     720
   ttgatgtata tctgcaatct tctctataat ctgcatttat tctctgttgt ttctctagca     780
   agtaaatcat acttgcttaa tgtacttagc aatttgtcat ttttgactta ttgtgatgta     840
   aatgtgattg actactatga cagtgaagcg aaacgggaca cgggcaatgc aattggaaga     900
   gagaaaggct atccggagct tgtcaatgtg cttcaacctc gcactcgtgg ctttgtgact     960
   tgcctttctc aatcgcgctg ctctttggat gcagtttatg acctcactat agggtacaag    1020
   aagcggtgtc ccttgttcat caacaatgta ttcggaaccg atccatcgga agtgcacatt    1080
   cacattcgcc gaataccaat ttctgagatt cctcaatcag aagacggtat gacgcagtgg    1140
   ctgtatgatc tattttatca aaaggaccag atgttggcca gttttagtaa gacaggctct    1200
   ttccctgaca gtggaattga agagagccct ttgaacatag tggaaggtgt ttgcaatgtt    1260
   gctctacacg tagtccttag cggttgggta ttctggtgct tgtttcattc ggtttggttg    1320
   aagctttatg tggctttcgc tagtttgctg ctcgcgttta gtacctattt tgattggaga    1380
   cctaaaccgg tttactctag tctacgtact aaaagaaaaa tcgtgtaaaa taaattcgtt    1440
   agttgtaatt ggtttgttta ttccgattcc aaagctgagt ttaagggtga ggctcctctt    1500
   taagctgatt tttgctatta attggctgct cccttgtttg tctgccgtaa attggcttta    1560
   atacggttgt cttctgctga tgaacctcag tgcttcaaga cgatgtggcc ttttagcctt    1620
   ctcctttacc catcttgacc agatgccaaa ctcgcaataa agcagatcaa taggtcgtgc    1680
   cccaaaaaaa aaaaaaaaaa a                                               1701
<210> 4
   <211> 714
   <212> DNA
   <213> Physcomitrella patens
<220>
   <221> CDS
   <222> (1)..(714)
   <223> LPAAT
<400> 4
   atg gct ttg atg tat atc tgc aat ctt ctc tat aat ctg cat tta ttc      48
   Met Ala Leu Met Tyr Ile Cys Asn Leu Leu Tyr Asn Leu His Leu Phe
   1               5                   10                  15
   tct gtt gtt tct cta gca agt aaa tca tac ttg ctt aat gta ctt agc      96
   Ser Val Val Ser Leu Ala Ser Lys Ser Tyr Leu Leu Asn Val Leu Ser
               20                  25                  30
   aat ttg tca ttt ttg act tat tgt gat gta aat gtg att gac tac tat     144
   Asn Leu Ser Phe Leu Thr Tyr Cys Asp Val Asn Val Ile Asp Tyr Tyr
           35                  40                  45
   gac agt gaa gcg aaa cgg gac acg ggc aat gca att gga aga gag aaa     192
   Asp Ser Glu Ala Lys Arg Asp Thr Gly Asn Ala Ile Gly Arg Glu Lys
       50                  55                  60
   ggc tat ccg gag ctt gtc aat gtg ctt caa cct cgc act cgt ggc ttt     240
   Gly Tyr Pro Glu Leu Val Asn Val Leu Gln Pro Arg Thr Arg Gly Phe
   65                  70                  75                  80
   gtg act tgc ctt tct caa tcg cgc tgc tct ttg gat gca gtt tat gac      288
   Val Thr Cys Leu Ser Gln Ser Arg Cys Ser Leu Asp Ala Val Tyr Asp
                   85                  90                  95
   ctc act ata ggg tac aag aag cgg tgt ccc ttg ttc atc aac aat gta      336
   Leu Thr Ile Gly Tyr Lys Lys Arg Cys Pro Leu Phe Ile Asn Asn Val
               100                 105                 110
   ttc gga acc gat cca tcg gaa gtg cac att cac att cgc cga ata cca      384
   Phe Gly Thr Asp Pro Ser Glu Val His Ile His Ile Arg Arg Ile Pro
           115                 120                 125
   att tct gag att cct caa tca gaa gac ggt atg acg cag tgg ctg tat      432
   Ile Ser Glu Ile Pro Gln Ser Glu Asp Gly Met Thr Gln Trp Leu Tyr
       130                 135                 140
   gat cta ttt tat caa aag gac cag atg ttg gcc agt ttt agt aag aca      480
   Asp Leu Phe Tyr Gln Lys Asp Gln Met Leu Ala Ser Phe Ser Lys Thr
   145                 150                 155                 160
   ggc tct ttc cct gac agt gga att gaa gag agc cct ttg aac ata gtg      528
   Gly Ser Phe Pro Asp Ser Gly Ile Glu Glu Ser Pro Leu Asn Ile Val
                   165                 170                 175
   gaa ggt gtt tgc aat gtt gct cta cac gta gtc ctt agc ggt tgg gta      576
   Glu Gly Val Cys Asn Val Ala Leu His Val Val Leu Ser Gly Trp Val
               180                 185                 190
   ttc tgg tgc ttg ttt cat tcg gtt tgg ttg aag ctt tat gtg gct ttc      624
   Phe Trp Cys Leu Phe His Ser Val Trp Leu Lys Leu Tyr Val Ala Phe
           195                 200                 205
   gct agt ttg ctg ctc gcg ttt agt acc tat ttt gat tgg aga cct aaa      672
   Ala Ser Leu Leu Leu Ala Phe Ser Thr Tyr Phe Asp Trp Arg Pro Lys
       210                 215                 220
   ccg gtt tac tct agt cta cgt act aaa aga aaa atc gtg taa              714
   Pro Val Tyr Ser Ser Leu Arg Thr Lys Arg Lys Ile Val
   225                 230                 235
<210> 5
   <211> 237
   <212> PRT
   <213> Physcomitrella patens
<400> 5
   Met Ala Leu Met Tyr Ile Cys Asn Leu Leu Tyr Asn Leu His Leu Phe
   1               5                   10                  15
   Ser Val Val Ser Leu Ala Ser Lys Ser Tyr Leu Leu Asn Val Leu Ser
               20                  25                  30
   Asn Leu Ser Phe Leu Thr Tyr Cys Asp Val Asn Val Ile Asp Tyr Tyr
           35                  40                  45
   Asp Ser Glu Ala Lys Arg Asp Thr Gly Asn Ala Ile Gly Arg Glu Lys
       50                  55                  60
   Gly Tyr Pro Glu Leu Val Asn Val Leu Gln Pro Arg Thr Arg Gly Phe
   65                  70                  75                  80
   Val Thr Cys Leu Ser Gln Ser Arg Cys Ser Leu Asp Ala Val Tyr Asp
                   85                  90                  95
   Leu Thr Ile Gly Tyr Lys Lys Arg Cys Pro Leu Phe Ile Asn Asn Val
               100                 105                 110
   Phe Gly Thr Asp Pro Ser Glu Val His Ile His Ile Arg Arg Ile Pro
           115                 120                 125
   Ile Ser Glu Ile Pro Gln Ser Glu Asp Gly Met Thr Gln Trp Leu Tyr
       130                 135                 140
   Asp Leu Phe Tyr Gln Lys Asp Gln Met Leu Ala Ser Phe Ser Lys Thr
   145                 150                 155                 160
   Gly Ser Phe Pro Asp Ser Gly Ile Glu Glu Ser Pro Leu Asn Ile Val
                   165                 170                 175
   Glu Gly Val Cys Asn Val Ala Leu His Val Val Leu Ser Gly Trp Val
               180                 185                 190
   Phe Trp Cys Leu Phe His Ser Val Trp Leu Lys Leu Tyr Val Ala Phe
           195                 200                 205
   Ala Ser Leu Leu Leu Ala Phe Ser Thr Tyr Phe Asp Trp Arg Pro Lys
       210                 215                 220
   Pro Val Tyr Ser Ser Leu Arg Thr Lys Arg Lys Ile Val
   225                 230                 235
<210> 6
   <211> 507
   <212> DNA
   <213> Physcomitrella patens
<220>
   <221> misc_feature
   <223> LPAAT
<400> 6
   accaggtcga gatgcccatt attggactgt ttttgcaagc tttgcaaata atacccgtgg     60
   accggactga tgctcagtct aggcaccatg cggctggcaa cgttcggcga agggctgtgg    120
   acaatatgtg gtcccacgtc atgttgttcc cggagggcac taccaccaat ggcagagcaa    180
   taatcgcctt caaaacagga gcattttcgc ctggtctccc tgtgcagcca atggttatta    240
   gataccctca caagtatgtc aacccctctt ggtgtgacca aggaggtccg ttggtcgttg    300
   tgttgcagct gatgactcag ttcatcaacc acatggaggt tgaatatttg ccggtcatga    360
   agccaactgt gagagagatg aaataccctc atgaattcgc aagtagagtt cgcagcgaga    420
   tggctaaagc gttaggcatc gtgtgcacag aacacagctt tctggatatt aagctagcgc    480
   tggctgcaga aaagctcaaa cagcctt                                         507
<210> 7
   <211> 1566
   <212> DNA
   <213> Physcomitrella patens
<220>
   <221> CDS
   <222> (1)..(1566)
   <223> LPAAT
<400> 7
   atg gag agc aca gca gat gtc gga atg tcc gac gac gat cct atc ctt      48
   Met Glu Ser Thr Ala Asp Val Gly Met Ser Asp Asp Asp Pro Ile Leu
   1               5                   10                  15
   ctc aac ggg ctc gaa acg cca cta ctg gct gaa ttt cct ctt ggc gaa      96
   Leu Asn Gly Leu Glu Thr Pro Leu Leu Ala Glu Phe Pro Leu Gly Glu
               20                  25                  30
   cgg cct aca ata ggg ccg gag gca cca gta aat ccc ttc cat gaa ccc     144
   Arg Pro Thr Ile Gly Pro Glu Ala Pro Val Asn Pro Phe His Glu Pro
           35                  40                  45
   gat ggt ggt tgg aag acc aac aac gag tgg aat tac ttt caa atg atg     192
   Asp Gly Gly Trp Lys Thr Asn Asn Glu Trp Asn Tyr Phe Gln Met Met
       50                  55                  60
   aaa tcc att ttg ctg att cca ctt ctt ctc gtt cgt cta gtg agc atg     240
   Lys Ser Ile Leu Leu Ile Pro Leu Leu Leu Val Arg Leu Val Ser Met
   65                  70                  75                  80
   ata aca atc gta gca ttt gga tat gtg tgg atc agg att tgt ctg atc     288
   Ile Thr Ile Val Ala Phe Gly Tyr Val Trp Ile Arg Ile Cys Leu Ile
                   85                  90                  95
   ggc gtc aca gat ccc ttg ttt aag cct ttc aat ccg tgt cga cgg ttc     336
   Gly Val Thr Asp Pro Leu Phe Lys Pro Phe Asn Pro Cys Arg Arg Phe
               100                 105                 110
   atg ctg tgg ggc ata cgg tta gta gca aga gca gtg atg ttt acc atg     384
   Met Leu Trp Gly Ile Arg Leu Val Ala Arg Ala Val Met Phe Thr Met
           115                 120                 125
   ggt tat tac tac att ccc atc aag gga aaa ccg gct cac cga tca gag     432
   Gly Tyr Tyr Tyr Ile Pro Ile Lys Gly Lys Pro Ala His Arg Ser Glu
       130                 135                 140
   gcg ccc att att gtg tcc aat cac att gga ttt ctg gat ccc atc ttt     480
   Ala Pro Ile Ile Val Ser Asn His Ile Gly Phe Leu Asp Pro Ile Phe
   145                 150                 155                 160
   gtg ttc tat cgg cac ttg ccg gcc atc gtc tca gcc aag gag aac gtc     528
   Val Phe Tyr Arg His Leu Pro Ala Ile Val Ser Ala Lys Glu Asn Val
                   165                 170                 175
   gag atg ccc att att gga ctg ttt ttg caa gct ttg caa ata ata ccc     576
   Glu Met Pro Ile Ile Gly Leu Phe Leu Gln Ala Leu Gln Ile Ile Pro
               180                 185                 190
   gtg gac cgg act gat gct cag tct agg cac cac gcg gct ggc aac gtt     624
   Val Asp Arg Thr Asp Ala Gln Ser Arg His His Ala Ala Gly Asn Val
           195                 200                 205
   cgg cga agg gct gtg gac aat atg tgg tcc cac gtc atg ttg ttc ccg     672
   Arg Arg Arg Ala Val Asp Asn Met Trp Ser His Val Met Leu Phe Pro
       210                 215                 220
   cag ggc act acc acc aat ggc aga gca ata atc gcc ttc aaa aca gga     720
   Gln Gly Thr Thr Thr Asn Gly Arg Ala Ile Ile Ala Phe Lys Thr Gly
   225                 230                 235                 240
   gca ttt tcg cct ggt ctc cct gtg cag cca atg gtt att aga tac cct     768
   Ala Phe Ser Pro Gly Leu Pro Val Gln Pro Met Val Ile Arg Tyr Pro
                   245                 250                 255
   cac aag tat gtc aac ccc tct tgg tgt gac caa gga ggt ccg ttg gtc     816
   His Lys Tyr Val Asn Pro Ser Trp Cys Asp Gln Gly Gly Pro Leu Val
               260                 265                 270
   gtt gtg ttg cag ctg atg act cag ttc atc aac cac atg gag gtt gaa      864
   Val Val Leu Gln Leu Met Thr Gln Phe Ile Asn His Met Glu Val Glu
           275                 280                 285
   tat ttg ccg gtc atg aag cca act gtg aga gag atg aaa tac cct cat      912
   Tyr Leu Pro Val Met Lys Pro Thr Val Arg Glu Met Lys Tyr Pro His
       290                 295                 300
   gaa ttc gca agt aga gtt cgc agc gag atg gct aaa gcg tta ggc atc      960
   Glu Phe Ala Ser Arg Val Arg Ser Glu Met Ala Lys Ala Leu Gly Ile
   305                 310                 315                 320
   gtg tgc aca gaa cac agc ttt ctg gat att aag cta gcg ctg gct gca     1008
   Val Cys Thr Glu His Ser Phe Leu Asp Ile Lys Leu Ala Leu Ala Ala
                   325                 330                 335
   gaa aag ctc aaa cag cct tca ggt cgg tcg ttg gtt gag ttt gct cgc     1056
   Glu Lys Leu Lys Gln Pro Ser Gly Arg Ser Leu Val Glu Phe Ala Arg
               340                 345                 350
   atg gag aag tta ttt cgg ctg gat ttt cct acg gcg aag gaa tac ttg     1104
   Met Glu Lys Leu Phe Arg Leu Asp Phe Pro Thr Ala Lys Glu Tyr Leu
           355                 360                 365
   gaa aag ttc agc gcc atg gac cgc aca cac agt ggc ttt gtt aca ttt     1152
   Glu Lys Phe Ser Ala Met Asp Arg Thr His Ser Gly Phe Val Thr Phe
       370                 375                 380
   gag gag tta tgt acg gca ctg gat ctt cca cgc tca cca att act aag     1200
   Glu Glu Leu Cys Thr Ala Leu Asp Leu Pro Arg Ser Pro Ile Thr Lys
   385                 390                 395                 400
   cag gtg ttc aac ctt ttc gat aag gat ggg cat gga agc ata aac ttt     1248
   Gln Val Phe Asn Leu Phe Asp Lys Asp Gly His Gly Ser Ile Asn Phe
                   405                 410                 415
   cga gag ttt ttg gca ggg ctc gcc ttt gtg tcc agc cac aca tca ttt     1296
   Arg Glu Phe Leu Ala Gly Leu Ala Phe Val Ser Ser His Thr Ser Phe
               420                 425                 430
   tca agt aca atg gag gct gca ttt aaa gca tgt gat gtg aat ggc gat     1344
   Ser Ser Thr Met Glu Ala Ala Phe Lys Ala Cys Asp Val Asn Gly Asp
           435                 440                 445
   ggc act ctt tct cgt gat gaa gtg gag agg agt ttg ctt gat atc ttt     1392
   Gly Thr Leu Ser Arg Asp Glu Val Glu Arg Ser Leu Leu Asp Ile Phe
       450                 455                 460
   cca gag ctc cct cca ata acg gtg ttc aag ctt ttt gac acg tta gat     1440
   Pro Glu Leu Pro Pro Ile Thr Val Phe Lys Leu Phe Asp Thr Leu Asp
   465                 470                 475                 480
   ata aat cat gat gag aaa atc agc tgg gag gag ttc agt agc ttt ctg     1488
   Ile Asn His Asp Glu Lys Ile Ser Trp Glu Glu Phe Ser Ser Phe Leu
                   485                 490                 495
   cag cga aac cca gag tat ctg gcc atc att ata tat gcg cac cct act     1536
   Gln Arg Asn Pro Glu Tyr Leu Ala Ile Ile Ile Tyr Ala His Pro Thr
               500                 505                 510
   ctg ctg aag cca ccc aca tcg act agc tga                             1566
   Leu Leu Lys Pro Pro Thr Ser Thr Ser
           515                 520
<210> 8
   <211> 521
   <212> PRT
   <213> Physcomitrella patens
<400> 8
   Met Glu Ser Thr Ala Asp Val Gly Met Ser Asp Asp Asp Pro Ile Leu
   1               5                   10                  15
   Leu Asn Gly Leu Glu Thr Pro Leu Leu Ala Glu Phe Pro Leu Gly Glu
               20                  25                  30
   Arg Pro Thr Ile Gly Pro Glu Ala Pro Val Asn Pro Phe His Glu Pro
           35                  40                  45
   Asp Gly Gly Trp Lys Thr Asn Asn Glu Trp Asn Tyr Phe Gln Met Met
       50                  55                  60
   Lys Ser Ile Leu Leu Ile Pro Leu Leu Leu Val Arg Leu Val Ser Met
   65                  70                  75                  80
   Ile Thr Ile Val Ala Phe Gly Tyr Val Trp Ile Arg Ile Cys Leu Ile
                   85                  90                  95
   Gly Val Thr Asp Pro Leu Phe Lys Pro Phe Asn Pro Cys Arg Arg Phe
               100                 105                 110
   Met Leu Trp Gly Ile Arg Leu Val Ala Arg Ala Val Met Phe Thr Met
           115                 120                 125
   Gly Tyr Tyr Tyr Ile Pro Ile Lys Gly Lys Pro Ala His Arg Ser Glu
       130                 135                 140
   Ala Pro Ile Ile Val Ser Asn His Ile Gly Phe Leu Asp Pro Ile Phe
   145                 150                 155                 160
   Val Phe Tyr Arg His Leu Pro Ala Ile Val Ser Ala Lys Glu Asn Val
                   165                 170                 175
   Glu Met Pro Ile Ile Gly Leu Phe Leu Gln Ala Leu Gln Ile Ile Pro
               180                 185                 190
   Val Asp Arg Thr Asp Ala Gln Ser Arg His His Ala Ala Gly Asn Val
           195                 200                 205
   Arg Arg Arg Ala Val Asp Asn Met Trp Ser His Val Met Leu Phe Pro
       210                 215                 220
   Gln Gly Thr Thr Thr Asn Gly Arg Ala Ile Ile Ala Phe Lys Thr Gly
   225                 230                 235                 240
   Ala Phe Ser Pro Gly Leu Pro Val Gln Pro Met Val Ile Arg Tyr Pro
                   245                 250                 255
   His Lys Tyr Val Asn Pro Ser Trp Cys Asp Gln Gly Gly Pro Leu Val
               260                 265                 270
   Val Val Leu Gln Leu Met Thr Gln Phe Ile Asn His Met Glu Val Glu
           275                 280                 285
   Tyr Leu Pro Val Met Lys Pro Thr Val Arg Glu Met Lys Tyr Pro His
       290                 295                 300
   Glu Phe Ala Ser Arg Val Arg Ser Glu Met Ala Lys Ala Leu Gly Ile
   305                 310                 315                 320
   Val Cys Thr Glu His Ser Phe Leu Asp Ile Lys Leu Ala Leu Ala Ala
                   325                 330                 335
   Glu Lys Leu Lys Gln Pro Ser Gly Arg Ser Leu Val Glu Phe Ala Arg
               340                 345                 350
   Met Glu Lys Leu Phe Arg Leu Asp Phe Pro Thr Ala Lys Glu Tyr Leu
           355                 360                 365
   Glu Lys Phe Ser Ala Met Asp Arg Thr His Ser Gly Phe Val Thr Phe
       370                 375                 380
   Glu Glu Leu Cys Thr Ala Leu Asp Leu Pro Arg Ser Pro Ile Thr Lys
   385                 390                 395                 400
   Gln Val Phe Asn Leu Phe Asp Lys Asp Gly His Gly Ser Ile Asn Phe
                   405                 410                 415
   Arg Glu Phe Leu Ala Gly Leu Ala Phe Val Ser Ser His Thr Ser Phe
               420                 425                 430
   Ser Ser Thr Met Glu Ala Ala Phe Lys Ala Cys Asp Val Asn Gly Asp
           435                 440                 445
   Gly Thr Leu Ser Arg Asp Glu Val Glu Arg Ser Leu Leu Asp Ile Phe
       450                 455                 460
   Pro Glu Leu Pro Pro Ile Thr Val Phe Lys Leu Phe Asp Thr Leu Asp
   465                 470                 475                 480
   Ile Asn His Asp Glu Lys Ile Ser Trp Glu Glu Phe Ser Ser Phe Leu
                   485                 490                 495
   Gln Arg Asn Pro Glu Tyr Leu Ala Ile Ile Ile Tyr Ala His Pro Thr
               500                 505                 510
   Leu Leu Lys Pro Pro Thr Ser Thr Ser
           515                 520
<210> 9
   <211> 2217
   <212> DNA
   <213> Physcomitrella patens
<220>
   <221> CDS
   <222> (281)..(1837)
   <223> LPAAT2
<400> 9
   ggcgcgccag aggacgagac aaggggggcg ctgtggactt ggtacaactc caaatgtggc      60
   tctgaatcat caactaaggg tatggttata caaagtgcgt gccgccgaag agacagacct    120
   tcttggttac ccaagactga atgaagatgg gaagtggaac gatagtatga tggctcagag    180
   acgagtggct ccgagttttt tggtactcag taggaagttg caagtggggt ttgcatgctg    240
   aagaatcgac actgcacagg cctcaccatc gacggatagc atg acc agc acg gaa      295
                                               Met Thr Ser Thr Glu
                                               1               5
   aat act gcg atg ttc aca gaa gac act agc act cta aac ggc tcc aca      343
   Asn Thr Ala Met Phe Thr Glu Asp Thr Ser Thr Leu Asn Gly Ser Thr
                   10                  15                  20
   gag gca aat cat gct gag ttt cct ctt gga gag cgg ccg acg ata ggg      391
   Glu Ala Asn His Ala Glu Phe Pro Leu Gly Glu Arg Pro Thr Ile Gly
               25                  30                  35
   ccg gag cca cca gtg aac ccc ttc cac gag tcc agc acg tgg agc atc      439
   Pro Glu Pro Pro Val Asn Pro Phe His Glu Ser Ser Thr Trp Ser Ile
           40                  45                  50
   ccc caa gtg atc aag acc att ctg cta gtc ccc ttg ctc gtc ata cgc      487
   Pro Gln Val Ile Lys Thr Ile Leu Leu Val Pro Leu Leu Val Ile Arg
       55                  60                  65
   ttg ctc agc atg ttc gct ctc atg atg ttg ggc tac ata tgc gtc aag      535
   Leu Leu Ser Met Phe Ala Leu Met Met Leu Gly Tyr Ile Cys Val Lys
   70                  75                  80                  85
   gtc gct atg atc gga tgc aaa gac ccg ttg ttc aag cct ttc aat cct      583
   Val Ala Met Ile Gly Cys Lys Asp Pro Leu Phe Lys Pro Phe Asn Pro
                   90                  95                  100
   ttg cgg cga ctc ttg ttg gta agt gtg agg tta ata gca aga ggg gtg      631
   Leu Arg Arg Leu Leu Leu Val Ser Val Arg Leu Ile Ala Arg Gly Val
               105                 110                 115
   atg gtg gcc atg ggg tat tac tat atc ctc gtc aag gga aaa cca gcc      679
   Met Val Ala Met Gly Tyr Tyr Tyr Ile Leu Val Lys Gly Lys Pro Ala
           120                 125                 130
   cac cgg tct gtg gcg ccc att atc gta tcc aac cac atc ggc ttt gtg      727
   His Arg Ser Val Ala Pro Ile Ile Val Ser Asn His Ile Gly Phe Val
       135                 140                 145
   gat ccc att ttt gtg ttc tat agg cac ttg ccg gtc atc gtc tca gcc      775
   Asp Pro Ile Phe Val Phe Tyr Arg His Leu Pro Val Ile Val Ser Ala
   150                 155                 160                 165
   aag gaa att gtg gag atg ccc ata atc gga atg ttc tta caa gct ctg      823
   Lys Glu Ile Val Glu Met Pro Ile Ile Gly Met Phe Leu Gln Ala Leu
                   170                 175                 180
   cag atc ata cct gtg gac cga ata aac ccc gcg tcc agg cac cat gcg      871
   Gln Ile Ile Pro Val Asp Arg Ile Asn Pro Ala Ser Arg His His Ala
               185                 190                 195
   gct gga aat atc cga cga aga gct atg gac aac gag tgg ccg cat gtc      919
   Ala Gly Asn Ile Arg Arg Arg Ala Met Asp Asn Glu Trp Pro His Val
           200                 205                 210
   atg ctg ttt cca gag ggg act acc aca aat ggc aaa gcg ttg atc tcc      967
   Met Leu Phe Pro Glu Gly Thr Thr Thr Asn Gly Lys Ala Leu Ile Ser
       215                 220                 225
   ttc aaa aca gga gca ttt tcg cct ggt cta cct gtg caa ccc atg gtc     1015
   Phe Lys Thr Gly Ala Phe Ser Pro Gly Leu Pro Val Gln Pro Met Val
   230                 235                 240                 245
   att aaa tac ccc cac aag tat gtg aat ccg tgt tgg tgt aac caa ggg     1063
   Ile Lys Tyr Pro His Lys Tyr Val Asn Pro Cys Trp Cys Asn Gln Gly
                   250                 255                 260
   ggg cca ttg gtc att ctc ttt cag ctg atg act cag ttt gta aat tac     1111
   Gly Pro Leu Val Ile Leu Phe Gln Leu Met Thr Gln Phe Val Asn Tyr
               265                 270                 275
   atg gag gtg gag tat ttg cct gtg atg acg cca aat gtg cat gag att     1159
   Met Glu Val Glu Tyr Leu Pro Val Met Thr Pro Asn Val His Glu Ile
           280                 285                 290
   aaa aat ccc cat gaa ttt gct aat aga gta cgg act gag atg gcc aaa     1207
   Lys Asn Pro His Glu Phe Ala Asn Arg Val Arg Thr Glu Met Ala Lys
       295                 300                 305
   gcg ctg ggc gtt gtg tgc acg gaa cat aac ttt cta gat atc aaa cta     1255
   Ala Leu Gly Val Val Cys Thr Glu His Asn Phe Leu Asp Ile Lys Leu
   310                 315                 320                 325
   aaa atg gct gca gag aag ctc aag cag cct tca gga cgc tca ttg gtt     1303
   Lys Met Ala Ala Glu Lys Leu Lys Gln Pro Ser Gly Arg Ser Leu Val
                   330                 335                 340
   gaa ttc gca cgc atg gag aag ctt ttt cga ctg gac tat tcc aag gcc     1351
   Glu Phe Ala Arg Met Glu Lys Leu Phe Arg Leu Asp Tyr Ser Lys Ala
               345                 350                 355
   cag gaa tac ttg gaa aaa ttc agt gct atg gat cct tca cac agt ggt     1399
   Gln Glu Tyr Leu Glu Lys Phe Ser Ala Met Asp Pro Ser His Ser Gly
               360                 365                 370
   tat gtc aca tac gat gag ttc ctt aaa gca ctc cat ctt ccg ccc acc     1447
   Tyr Val Thr Tyr Asp Glu Phe Leu Lys Ala Leu His Leu Pro Pro Thr
           375                 380                 385
   cag atc act gag cag gtg ttc aac ctt ttc gac aag aac gga cac ggt     1495
   Gln Ile Thr Glu Gln Val Phe Asn Leu Phe Asp Lys Asn Gly His Gly
   390                 395                 400                 405
   tct ata aac ttt cga gag ttt gtg gca ggg ctt gct ttc ctg tct acc     1543
   Ser Ile Asn Phe Arg Glu Phe Val Ala Gly Leu Ala Phe Leu Ser Thr
                   410                 415                 420
   cac act tca ttc cag act aca atg aag gct gca ttc aaa gct tgt gat     1591
   His Thr Ser Phe Gln Thr Thr Met Lys Ala Ala Phe Lys Ala Cys Asp
               425                 430                 435
   gtg gat ggc gat ggc acc ctc act cgt aat gag gtg gaa agc agc ttg     1639
   Val Asp Gly Asp Gly Thr Leu Thr Arg Asn Glu Val Glu Ser Ser Leu
           440                 445                 450
   atg gcc gta ttc ccg gag ctc ccc cca gca acg gtg tta aaa ctt ttc     1687
   Met Ala Val Phe Pro Glu Leu Pro Pro Ala Thr Val Leu Lys Leu Phe
       455                 460                 465
   gac acg ctg gat tta aat cgt gac ggg agc att aac tgg gag gag ttc     1735
   Asp Thr Leu Asp Leu Asn Arg Asp Gly Ser Ile Asn Trp Glu Glu Phe
   470                 475                 480                 485
   agc agc ttt ctg caa cga aat cct gag tat ttg gcc atc ata ttg gct     1783
   Ser Ser Phe Leu Gln Arg Asn Pro Glu Tyr Leu Ala Ile Ile Leu Ala
                   490                 495                 500
   gca cac cct act ctg ttg cag gca cca aag tcg gaa gag agt gaa act     1831
   Ala His Pro Thr Leu Leu Gln Ala Pro Lys Ser Glu Glu Ser Glu Thr
               505                 510                 515
   aac atc tagagttctg tcaatcgata tctattagat catctctttc acatgctgtg      1887
   Asn Ile
   ggaccttttg gagctgcaat tcctcgagca tgatataacc actctattac agttgcgctt     1947
   agtgggtgca tcttctggat ttgaatcgac tcggggacat aaaagcagca gtggtttgct    2007
   gtcaccgttg acatggttta ggaacttagc atcgagatag atccttactt gagatcattt    2067
   tgtatttcca cagactattg ctgttaccag tagctctgct agagctagaa tttctatgat    2127
   gtggacgaaa gtcaacttat tcttaagaat caaaagttaa gctccggtct ttgtaacgtt    2187
   tttactgcaa aaaaaaaaaa aaaaaaaaaa                                     2217
<210> 10
   <211> 519
   <212> PRT
   <213> Physcomitrella patens
<400> 10
   Met Thr Ser Thr Glu Asn Thr Ala Met Phe Thr Glu Asp Thr Ser Thr
   1               5                   10                  15
   Leu Asn Gly Ser Thr Glu Ala Asn His Ala Glu Phe Pro Leu Gly Glu
               20                  25                  30
   Arg Pro Thr Ile Gly Pro Glu Pro Pro Val Asn Pro Phe His Glu Ser
           35                  40                  45
   Ser Thr Trp Ser Ile Pro Gln Val Ile Lys Thr Ile Leu Leu Val Pro
       50                  55                  60
   Leu Leu Val Ile Arg Leu Leu Ser Met Phe Ala Leu Met Met Leu Gly
   65                  70                  75                  80
   Tyr Ile Cys Val Lys Val Ala Met Ile Gly Cys Lys Asp Pro Leu Phe
                   85                  90                  95
   Lys Pro Phe Asn Pro Leu Arg Arg Leu Leu Leu Val Ser Val Arg Leu
               100                 105                 110
   Ile Ala Arg Gly Val Met Val Ala Met Gly Tyr Tyr Tyr Ile Leu Val
           115                 120                 125
   Lys Gly Lys Pro Ala His Arg Ser Val Ala Pro Ile Ile Val Ser Asn
       130                 135                 140
   His Ile Gly Phe Val Asp Pro Ile Phe Val Phe Tyr Arg His Leu Pro
   145                 150                 155                 160
   Val Ile Val Ser Ala Lys Glu Ile Val Glu Met Pro Ile Ile Gly Met
                   165                 170                 175
   Phe Leu Gln Ala Leu Gln Ile Ile Pro Val Asp Arg Ile Asn Pro Ala
               180                 185                 190
   Ser Arg His His Ala Ala Gly Asn Ile Arg Arg Arg Ala Met Asp Asn
           195                 200                 205
   Glu Trp Pro His Val Met Leu Phe Pro Glu Gly Thr Thr Thr Asn Gly
       210                 215                 220
   Lys Ala Leu Ile Ser Phe Lys Thr Gly Ala Phe Ser Pro Gly Leu Pro
   225                 230                 235                 240
   Val Gln Pro Met Val Ile Lys Tyr Pro His Lys Tyr Val Asn Pro Cys
                   245                 250                 255
   Trp Cys Asn Gln Gly Gly Pro Leu Val Ile Leu Phe Gln Leu Met Thr
               260                 265                 270
   Gln Phe Val Asn Tyr Met Glu Val Glu Tyr Leu Pro Val Met Thr Pro
           275                 280                 285
   Asn Val His Glu Ile Lys Asn Pro His Glu Phe Ala Asn Arg Val Arg
       290                 295                 300
   Thr Glu Met Ala Lys Ala Leu Gly Val Val Cys Thr Glu His Asn Phe
   305                 310                 315                 320
   Leu Asp Ile Lys Leu Lys Met Ala Ala Glu Lys Leu Lys Gln Pro Ser
                   325                 330                 335
   Gly Arg Ser Leu Val Glu Phe Ala Arg Met Glu Lys Leu Phe Arg Leu
               340                 345                 350
   Asp Tyr Ser Lys Ala Gln Glu Tyr Leu Glu Lys Phe Ser Ala Met Asp
           355                 360                 365
   Pro Ser His Ser Gly Tyr Val Thr Tyr Asp Glu Phe Leu Lys Ala Leu
       370                 375                 380
   His Leu Pro Pro Thr Gln Ile Thr Glu Gln Val Phe Asn Leu Phe Asp
   385                 390                 395                 400
   Lys Asn Gly His Gly Ser Ile Asn Phe Arg Glu Phe Val Ala Gly Leu
                   405                 410                 415
   Ala Phe Leu Ser Thr His Thr Ser Phe Gln Thr Thr Met Lys Ala Ala
               420                 425                 430
   Phe Lys Ala Cys Asp Val Asp Gly Asp Gly Thr Leu Thr Arg Asn Glu
           435                 440                 445
   Val Glu Ser Ser Leu Met Ala Val Phe Pro Glu Leu Pro Pro Ala Thr
       450                 455                 460
   Val Leu Lys Leu Phe Asp Thr Leu Asp Leu Asn Arg Asp Gly Ser Ile
   465                 470                 475                 480
   Asn Trp Glu Glu Phe Ser Ser Phe Leu Gln Arg Asn Pro Glu Tyr Leu
                       485                 490                 495
   Ala Ile Ile Leu Ala Ala His Pro Thr Leu Leu Gln Ala Pro Lys Ser
               500                 505                 510
   Glu Glu Ser Glu Thr Asn Ile
           515
<210> 11
   <211> 1014
   <212> DNA
   <213> Physcomitrella patens
<220>
   <221> CDS
   <222> (1)..(1014)
   <223> LPAAT
<400> 11
   atg att atg atg gag gtg ctg tgg tcg gag ctt ata tgg ctg ctg gat       48
   Met Ile Met Met Glu Val Leu Trp Ser Glu Leu Ile Trp Leu Leu Asp
   1               5                   10                  15
   tgg tgg gca aat gtg aag gtg aag gtt tac acg cca aag gag tcg tgg       96
   Trp Trp Ala Asn Val Lys Val Lys Val Tyr Thr Pro Lys Glu Ser Trp
               20                  25                  30
   gag cac tta gga aag gag cac gca tta ctc att tgt aat cac cgc agt      144
   Glu His Leu Gly Lys Glu His Ala Leu Leu Ile Cys Asn His Arg Ser
           35                  40                  45
   gac att gat tgg ctc gta gga tgg att att gcc cag aga ttg ggg tgt      192
   Asp Ile Asp Trp Leu Val Gly Trp Ile Ile Ala Gln Arg Leu Gly Cys
       50                  55                  60
   cta ggt ggg act cga gct gtt atg aag aag tcc acc aaa ttt ctt ccg      240
   Leu Gly Gly Thr Arg Ala Val Met Lys Lys Ser Thr Lys Phe Leu Pro
   65                  70                  75                  80
   gtc att ggc tgg tct atg tgg ttt tca gag tat gtg ttt tta tca aga      288
   Val Ile Gly Trp Ser Met Trp Phe Ser Glu Tyr Val Phe Leu Ser Arg
                   85                  90                  95
   gat tgg gcc aaa gat gag aag gtc ttg aag aat ggt tat tca agt ctt      336
   Asp Trp Ala Lys Asp Glu Lys Val Leu Lys Asn Gly Tyr Ser Ser Leu
               100                 105                 110
   aag ggc ttc ccc agg acc ttg tgg gtg gct ctt ttt gtg gaa ggc act      384
   Lys Gly Phe Pro Arg Thr Leu Trp Val Ala Leu Phe Val Glu Gly Thr
           115                 120                 125
   cga ttt acg aag gct aaa ctt gag gtt gcc caa aaa ttt gcg gcg gat      432
   Arg Phe Thr Lys Ala Lys Leu Glu Val Ala Gln Lys Phe Ala Ala Asp
       130                 135                 140
   aca ggg cta cgt gtt cca agg tat gtg ctt gtt cct cgc aca aaa ggg      480
   Thr Gly Leu Arg Val Pro Arg Tyr Val Leu Val Pro Arg Thr Lys Gly
   145                 150                 155                 160
   ttc gtt tcg gct gtg gag aac ttg cgt gaa ttt gtt ccg gta gtt tat      528
   Phe Val Ser Ala Val Glu Asn Leu Arg Glu Phe Val Pro Val Val Tyr
                   165                 170                 175
   gac atg acc gtt gct ata tct aaa gag ctg ccc aat cct aca atg atc      576
   Asp Met Thr Val Ala Ile Ser Lys Glu Leu Pro Asn Pro Thr Met Ile
               180                 185                 190
   cgg att ttc aga ggg caa cca tct gtg gtt cat gtg tac gtg agg cgg      624
   Arg Ile Phe Arg Gly Gln Pro Ser Val Val His Val Tyr Val Arg Arg
           195                 200                 205
   gtc cct atg tct gat ctg cct gag gga gcc aac gcg att tct aaa tgg      672
   Val Pro Met Ser Asp Leu Pro Glu Gly Ala Asn Ala Ile Ser Lys Trp
       210                 215                 220
   tgt cac gat gcc ttt cac atc aag gac gat cgg ctg gag cag cac gaa      720
   Cys His Asp Ala Phe His Ile Lys Asp Asp Arg Leu Glu Gln His Glu
   225                 230                 235                 240
   aaa gag aat acg ttt ggg gag gac ttg tat att cct att gaa cgg cca      768
   Lys Glu Asn Thr Phe Gly Glu Asp Leu Tyr Ile Pro Ile Glu Arg Pro
                   245                 250                 255
   ctt aaa cct ctt att att gtg atc tcc tgg gcc atc act ttg ctg gct      816
   Leu Lys Pro Leu Ile Ile Val Ile Ser Trp Ala Ile Thr Leu Leu Ala
               260                 265                 270
   gca gca tgg tgg ttt cta aga cga gtt tta tcc act tgg aaa gga atc      864
   Ala Ala Trp Trp Phe Leu Arg Arg Val Leu Ser Thr Trp Lys Gly Ile
           275                 280                 285
   gcc tgg gtg gca gga gta ctc gtg gtc gtc atg ctg tgt gtc cag att      912
   Ala Trp Val Ala Gly Val Leu Val Val Val Met Leu Cys Val Gln Ile
       290                 295                 300
   tta gtg atg tcg tca caa tcg gaa aga agt tca gat cct gca gct aag      960
   Leu Val Met Ser Ser Gln Ser Glu Arg Ser Ser Asp Pro Ala Ala Lys
   305                 310                 315                 320
   aag gcc aat caa aaa cag gcg gct tct gtt gct cac ctc ggc aaa acg     1008
   Lys Ala Asn Gln Lys Gln Ala Ala Ser Val Ala His Leu Gly Lys Thr
                   325                 330                 335
   gac tga                                                             1014
   Asp
<210> 12
   <211> 337
   <212> PRT
   <213> Physcomitrella patens
<400> 12
   Met Ile Met Met Glu Val Leu Trp Ser Glu Leu Ile Trp Leu Leu Asp
   1               5                   10                  15
   Trp Trp Ala Asn Val Lys Val Lys Val Tyr Thr Pro Lys Glu Ser Trp
               20                  25                  30
   Glu His Leu Gly Lys Glu His Ala Leu Leu Ile Cys Asn His Arg Ser
           35                  40                  45
   Asp Ile Asp Trp Leu Val Gly Trp Ile Ile Ala Gln Arg Leu Gly Cys
       50                  55                  60
   Leu Gly Gly Thr Arg Ala Val Met Lys Lys Ser Thr Lys Phe Leu Pro
   65                  70                  75                  80
   Val Ile Gly Trp Ser Met Trp Phe Ser Glu Tyr Val Phe Leu Ser Arg
                   85                  90                  95
   Asp Trp Ala Lys Asp Glu Lys Val Leu Lys Asn Gly Tyr Ser Ser Leu
                       100                 105                 110
   Lys Gly Phe Pro Arg Thr Leu Trp Val Ala Leu Phe Val Glu Gly Thr
                   115                 120                 125
   Arg Phe Thr Lys Ala Lys Leu Glu Val Ala Gln Lys Phe Ala Ala Asp
       130                 135                 140
   Thr Gly Leu Arg Val Pro Arg Tyr Val Leu Val Pro Arg Thr Lys Gly
   145                 150                 155                 160
   Phe Val Ser Ala Val Glu Asn Leu Arg Glu Phe Val Pro Val Val Tyr
                   165                 170                 175
   Asp Met Thr Val Ala Ile Ser Lys Glu Leu Pro Asn Pro Thr Met Ile
               180                 185                 190
   Arg Ile Phe Arg Gly Gln Pro Ser Val Val His Val Tyr Val Arg Arg
           195                 200                 205
   Val Pro Met Ser Asp Leu Pro Glu Gly Ala Asn Ala Ile Ser Lys Trp
       210                 215                 220
   Cys His Asp Ala Phe His Ile Lys Asp Asp Arg Leu Glu Gln His Glu
   225                 230                 235                 240
   Lys Glu Asn Thr Phe Gly Glu Asp Leu Tyr Ile Pro Ile Glu Arg Pro
                   245                 250                 255
   Leu Lys Pro Leu Ile Ile Val Ile Ser Trp Ala Ile Thr Leu Leu Ala
               260                 265                 270
   Ala Ala Trp Trp Phe Leu Arg Arg Val Leu Ser Thr Trp Lys Gly Ile
           275                 280                 285
   Ala Trp Val Ala Gly Val Leu Val Val Val Met Leu Cys Val Gln Ile
       290                 295                 300
   Leu Val Met Ser Ser Gln Ser Glu Arg Ser Ser Asp Pro Ala Ala Lys
   305                 310                 315                 320
   Lys Ala Asn Gln Lys Gln Ala Ala Ser Val Ala His Leu Gly Lys Thr
                   325                 330                 335
   Asp
<210> 13
   <211> 643
   <212> DNA
   <213> Physcomitrella patens
<220>
   <221> misc_feature
   <223> LPAAT2
<400> 13
   ggcgcgccag aggacgagac aaggggagtc aattggaatg cctgaagacc tgcatgaaac      60
   tggttaaaga aggtgtgtct gctctgtttt tccctgaggg cacaaggaca acggatggag     120
   caatggctgc cttcaagaaa ggagctttct ctgtggcggc caagggaggt gtgtcagttg     180
   tacctataac gttaattggc tcaggcaagt tgatgccaaa tggtttagaa tatacattac     240
   ggcctggcgt tgtgaaaatg attgtccacc cagctatccg cagtaaaaat gccgatgagc     300
   tttgtgatca gtctaggaag gttattgcag agaccttgat caaacacggt cttcctgttc     360
   attagttgct gtgattgatg atcgcctatc aggatgatgc gatcaagtga tcaagccctg     420
   tttgtcgttc ttagtgatta aggagtcatt tctgtccatc gtttatgccc cgcaagagat     480
   ttaaggagat cacaaagtcg gttgtagcaa gagagttgga cactgtgata agcccaatta     540
   acttatgttg aagtgtcatt tattctttga aaaaaaaaaa aataaaaaaa aaaaaaaaaa     600
   aaaaaaaaaa aaaaaaaaaa aaaaaaaaaa aaaaagcggc cgc                       643
<210> 14
   <211> 657
   <212> DNA
   <213> Physcomitrella patens
<220>
   <221> CDS
   <222> (1)..(657)
   <223> LPAAT
<400> 14
   atg ctg ata tta cag ccc ttc gta ctc tta ctc gac aag caa cgt aga       48
   Met Leu Ile Leu Gln Pro Phe Val Leu Leu Leu Asp Lys Gln Arg Arg
   1               5                   10                  15
   aga gct cag cac ctt gtg aac aag gtg tgg gca att ttg aca acg tct       96
   Arg Ala Gln His Leu Val Asn Lys Val Trp Ala Ile Leu Thr Thr Ser
               20                  25                  30
   ttg ttt tat aaa act gag att gaa ggt tgg gaa aat ctt cca gca tct      144
   Leu Phe Tyr Lys Thr Glu Ile Glu Gly Trp Glu Asn Leu Pro Ala Ser
           35                  40                  45
   gat gag ggt gca gtg tat gtt gcc aat cat caa agc ttt ttg gac atc      192
   Asp Glu Gly Ala Val Tyr Val Ala Asn His Gln Ser Phe Leu Asp Ile
       50                  55                  60
   tat aca ctc ttt caa tta gga cga cca ttt aag ttt att agc aag acc      240
   Tyr Thr Leu Phe Gln Leu Gly Arg Pro Phe Lys Phe Ile Ser Lys Thr
   65                  70                  75                  80
   agc aat ttt ctc att ccg att att ggt tgg tcc atg tac atg acg ggc      288
   Ser Asn Phe Leu Ile Pro Ile Ile Gly Trp Ser Met Tyr Met Thr Gly
                   85                  90                  95
   cac att ccc cta aag cgt atg gac aag agg agt caa ttg gaa tgc ctg      336
   His Ile Pro Leu Lys Arg Met Asp Lys Arg Ser Gln Leu Glu Cys Leu
               100                 105                 110
   aag acc tgc atg aag ctg gtt aaa gaa ggt gtg tct gtt ctg ttt ttc      384
   Lys Thr Cys Met Lys Leu Val Lys Glu Gly Val Ser Val Leu Phe Phe
           115                 120                 125
   cct gag ggc aca agg aca acg gat gga gca atg gct gcc ttc aag aaa      432
   Pro Glu Gly Thr Arg Thr Thr Asp Gly Ala Met Ala Ala Phe Lys Lys
       130                 135                 140
   gga gct ttc tct gtg gcg gcc aag gga ggt gtg cca gtt gta cct ata      480
   Gly Ala Phe Ser Val Ala Ala Lys Gly Gly Val Pro Val Val Pro Ile
   145                 150                 155                 160
   acg tta att ggc tca ggc aag ttg atg cca aat ggt tta gaa tat aca      528
   Thr Leu Ile Gly Ser Gly Lys Leu Met Pro Asn Gly Leu Glu Tyr Thr
                   165                 170                 175
   tta cgg cct ggc gtt gtg aaa atg att gtc cac cca gct atc cgc agt      576
   Leu Arg Pro Gly Val Val Lys Met Ile Val His Pro Ala Ile Arg Ser
               180                 185                 190
   aaa aat gcc gat gag ctt tgt gat cag tct agg aag gtt att gca gag      624
   Lys Asn Ala Asp Glu Leu Cys Asp Gln Ser Arg Lys Val Ile Ala Glu
           195                 200                 205
   acc ttg atc caa cac ggt ctt cct gtt cat tag                          657
   Thr Leu Ile Gln His Gly Leu Pro Val His
       210                 215
<210> 15
   <211> 218
   <212> PRT
   <213> Physcomitrella patens
<400> 15
   Met Leu Ile Leu Gln Pro Phe Val Leu Leu Leu Asp Lys Gln Arg Arg
   1               5                   10                  15
   Arg Ala Gln His Leu Val Asn Lys Val Trp Ala Ile Leu Thr Thr Ser
               20                  25                  30
   Leu Phe Tyr Lys Thr Glu Ile Glu Gly Trp Glu Asn Leu Pro Ala Ser
           35                  40                  45
   Asp Glu Gly Ala Val Tyr Val Ala Asn His Gln Ser Phe Leu Asp Ile
       50                  55                  60
   Tyr Thr Leu Phe Gln Leu Gly Arg Pro Phe Lys Phe Ile Ser Lys Thr
   65                  70                  75                  80
   Ser Asn Phe Leu Ile Pro Ile Ile Gly Trp Ser Met Tyr Met Thr Gly
                   85                  90                  95
   His Ile Pro Leu Lys Arg Met Asp Lys Arg Ser Gln Leu Glu Cys Leu
               100                 105                 110
   Lys Thr Cys Met Lys Leu Val Lys Glu Gly Val Ser Val Leu Phe Phe
           115                 120                 125
   Pro Glu Gly Thr Arg Thr Thr Asp Gly Ala Met Ala Ala Phe Lys Lys
       130                 135                 140
   Gly Ala Phe Ser Val Ala Ala Lys Gly Gly Val Pro Val Val Pro Ile
   145                 150                 155                 160
   Thr Leu Ile Gly Ser Gly Lys Leu Met Pro Asn Gly Leu Glu Tyr Thr
                   165                 170                 175
   Leu Arg Pro Gly Val Val Lys Met Ile Val His Pro Ala Ile Arg Ser
               180                 185                 190
   Lys Asn Ala Asp Glu Leu Cys Asp Gln Ser Arg Lys Val Ile Ala Glu
           195                 200                 205
   Thr Leu Ile Gln His Gly Leu Pro Val His
       210                 215
<210> 16
   <211> 1254
   <212> DNA
   <213> Mortierella alpina
<220>
   <221> CDS
   <222> (1)..(1251)
   <223> LPAAT
<400> 16
   atg gat gaa tcc acc acg acc acc acg cac cac tca gag acc agc agc      48
   Met Asp Glu Ser Thr Thr Thr Thr Thr His His Ser Glu Thr Ser Ser
   1               5                   10                  15
   aag acg tcc tcg cac ccc cgc cgg ctc ggt ccc gag atg aac cct atc      96
   Lys Thr Ser Ser His Pro Arg Arg Leu Gly Pro Glu Met Asn Pro Ile
               20                  25                  30
   tac aag ggt ctg cga gcc att gtc tgg gcc ttt tac ttc aac ctg gga     144
   Tyr Lys Gly Leu Arg Ala Ile Val Trp Ala Phe Tyr Phe Asn Leu Gly
           35                  40                  45
   gcg tcg ctt ata tcg atc acg cag gtg ctg tcg ctg cct ctg gcg ttg     192
   Ala Ser Leu Ile Ser Ile Thr Gln Val Leu Ser Leu Pro Leu Ala Leu
       50                  55                  60
   att gct cca ggg gtc tac cag tgg cac atc agc aaa aca cag ggt cac     240
   Ile Ala Pro Gly Val Tyr Gln Trp His Ile Ser Lys Thr Gln Gly His
   65                  70                  75                  80
   ttt gga gct ttc ctg ctc cgg atg aac cag ctc ttt gcg ccg tca gat     288
   Phe Gly Ala Phe Leu Leu Arg Met Asn Gln Leu Phe Ala Pro Ser Asp
                   85                  90                  95
   att gtc ttg aca ggg gac gag agt gtc agg gga atc gtc aag gtc tac     336
   Ile Val Leu Thr Gly Asp Glu Ser Val Arg Gly Ile Val Lys Val Tyr
               100                 105                 110
   aaa gga cgg aac ctg aag gag gcc ggt gag cca ggc agc ggt cag gga     384
   Lys Gly Arg Asn Leu Lys Glu Ala Gly Glu Pro Gly Ser Gly Gln Gly
           115                 120                 125
   gag gac att ctt ctg gat atg ccc gag agg atg gtt ttc att gcg aac     432
   Glu Asp Ile Leu Leu Asp Met Pro Glu Arg Met Val Phe Ile Ala Asn
       130                 135                 140
   cac cag atc tac tct gac tgg atg tac ctc tgg tgc ttc tcc tat ttt     480
   His Gln Ile Tyr Ser Asp Trp Met Tyr Leu Trp Cys Phe Ser Tyr Phe
   145                 150                 155                 160
   gca gag agg cac agg gca ctg aag att att ctt cgg ggc gac ctg acc     528
   Ala Glu Arg His Arg Ala Leu Lys Ile Ile Leu Arg Gly Asp Leu Thr
                   165                 170                 175
   tgg atc cct gtc ttt ggc tgg ggt atg cgg ttc ttt gac ttt atc ttt     576
   Trp Ile Pro Val Phe Gly Trp Gly Met Arg Phe Phe Asp Phe Ile Phe
               180                 185                 190
   ttg aaa cgt aat gac tgg gca cac gat cgc cgt gcc att gag gaa aac     624
   Leu Lys Arg Asn Asp Trp Ala His Asp Arg Arg Ala Ile Glu Glu Asn
           195                 200                 205
   ttg gga cgt gtc aag gaa aag gat ccc ctc tgg ctc gtg gtc ttc ccc     672
   Leu Gly Arg Val Lys Glu Lys Asp Pro Leu Trp Leu Val Val Phe Pro
       210                 215                 220
   gag gga aca gtc gtc tcc aag gaa acg cgt ctc cga tcc gtt gcc ttt     720
   Glu Gly Thr Val Val Ser Lys Glu Thr Arg Leu Arg Ser Val Ala Phe
   225                 230                 235                 240
   tca aag aag gct agt ctg tcg gat cac cgc cat gtg ctg ctt cca agg     768
   Ser Lys Lys Ala Ser Leu Ser Asp His Arg His Val Leu Leu Pro Arg
                   245                 250                 255
   acc agc ggt ctg ttt gtg tgc atc aac aag ttg cgt gga tct gtc gac     816
   Thr Ser Gly Leu Phe Val Cys Ile Asn Lys Leu Arg Gly Ser Val Asp
               260                 265                 270
   tac ttg tac gat gca acc gtt ggc tac tcg aat gtc gag tat ggc gag     864
   Tyr Leu Tyr Asp Ala Thr Val Gly Tyr Ser Asn Val Glu Tyr Gly Glu
           275                 280                 285
   att ccg cag gag ctt tac ccg tta cca gga ctg tat atc aac aaa gca      912
   Ile Pro Gln Glu Leu Tyr Pro Leu Pro Gly Leu Tyr Ile Asn Lys Ala
       290                 295                 300
   cag ccc aag gag atc aac atg cac ctg cgt cga ttt gcg atc aag gat      960
   Gln Pro Lys Glu Ile Asn Met His Leu Arg Arg Phe Ala Ile Lys Asp
   305                 310                 315                 320
   atc ccc acg tca gaa ccc gaa ttt gtg gaa tgg gtc cga gct cgg tgg     1008
   Ile Pro Thr Ser Glu Pro Glu Phe Val Glu Trp Val Arg Ala Arg Trp
                   325                 330                 335
   gtg gag aag gat gag ttg atg gaa gag ttt tat acc aag ggc cga ttt     1056
   Val Glu Lys Asp Glu Leu Met Glu Glu Phe Tyr Thr Lys Gly Arg Phe
               340                 345                 350
   cca tca caa ctg acg gcc gcc gac att ggt gag aag gag gtc aag acg     1104
   Pro Ser Gln Leu Thr Ala Ala Asp Ile Gly Glu Lys Glu Val Lys Thr
           355                 360                 365
   gca gga ggt cca acg gag gga cag agt gtc agg atc ccg ctc aag gcg     1152
   Ala Gly Gly Pro Thr Glu Gly Gln Ser Val Arg Ile Pro Leu Lys Ala
       370                 375                 380
   cga ggc atg atg gac tac ctc atg ccc tcg gtc atg aat ctg atc gcc     1200
   Arg Gly Met Met Asp Tyr Leu Met Pro Ser Val Met Asn Leu Ile Ala
   385                 390                 395                 400
   ctt cct gtg ctg gcg ttt gcg atg aga tat gca gtg cag caa gca tcg     1248
   Leu Pro Val Leu Ala Phe Ala Met Arg Tyr Ala Val Gln Gln Ala Ser
                   405                 410                 415
   ggc tga                                                             1254
   Gly
<210> 17
   <211> 417
   <212> PRT
   <213> Mortierella alpina
<400> 17
   Met Asp Glu Ser Thr Thr Thr Thr Thr His His Ser Glu Thr Ser Ser
   1               5                   10                  15
   Lys Thr Ser Ser His Pro Arg Arg Leu Gly Pro Glu Met Asn Pro Ile
               20                  25                  30
   Tyr Lys Gly Leu Arg Ala Ile Val Trp Ala Phe Tyr Phe Asn Leu Gly
           35                  40                  45
   Ala Ser Leu Ile Ser Ile Thr Gln Val Leu Ser Leu Pro Leu Ala Leu
       50                  55                  60
   Ile Ala Pro Gly Val Tyr Gln Trp His Ile Ser Lys Thr Gln Gly His
   65                  70                  75                  80
   Phe Gly Ala Phe Leu Leu Arg Met Asn Gln Leu Phe Ala Pro Ser Asp
                   85                  90                  95
   Ile Val Leu Thr Gly Asp Glu Ser Val Arg Gly Ile Val Lys Val Tyr
               100                 105                 110
   Lys Gly Arg Asn Leu Lys Glu Ala Gly Glu Pro Gly Ser Gly Gln Gly
           115                 120                 125
   Glu Asp Ile Leu Leu Asp Met Pro Glu Arg Met Val Phe Ile Ala Asn
       130                 135                  140
   His Gln Ile Tyr Ser Asp Trp Met Tyr Leu Trp Cys Phe Ser Tyr Phe
   145                 150                 155                 160
   Ala Glu Arg His Arg Ala Leu Lys Ile Ile Leu Arg Gly Asp Leu Thr
                   165                 170                 175
   Trp Ile Pro Val Phe Gly Trp Gly Met Arg Phe Phe Asp Phe Ile Phe
               180                 185                 190
   Leu Lys Arg Asn Asp Trp Ala His Asp Arg Arg Ala Ile Glu Glu Asn
           195                 200                 205
   Leu Gly Arg Val Lys Glu Lys Asp Pro Leu Trp Leu Val Val Phe Pro
       210                 215                 220
   Glu Gly Thr Val Val Ser Lys Glu Thr Arg Leu Arg Ser Val Ala Phe
   225                 230                 235                 240
   Ser Lys Lys Ala Ser Leu Ser Asp His Arg His Val Leu Leu Pro Arg
                   245                 250                 255
   Thr Ser Gly Leu Phe Val Cys Ile Asn Lys Leu Arg Gly Ser Val Asp
               260                 265                 270
   Tyr Leu Tyr Asp Ala Thr Val Gly Tyr Ser Asn Val Glu Tyr Gly Glu
           275                 280                 285
   Ile Pro Gln Glu Leu Tyr Pro Leu Pro Gly Leu Tyr Ile Asn Lys Ala
       290                 295                 300
   Gln Pro Lys Glu Ile Asn Met His Leu Arg Arg Phe Ala Ile Lys Asp
   305                 310                 315                 320
   Ile Pro Thr Ser Glu Pro Glu Phe Val Glu Trp Val Arg Ala Arg Trp
                   325                 330                 335
   Val Glu Lys Asp Glu Leu Met Glu Glu Phe Tyr Thr Lys Gly Arg Phe
               340                 345                 350
   Pro Ser Gln Leu Thr Ala Ala Asp Ile Gly Glu Lys Glu Val Lys Thr
           355                 360                 365
   Ala Gly Gly Pro Thr Glu Gly Gln Ser Val Arg Ile Pro Leu Lys Ala
       370                 375                 380
   Arg Gly Met Met Asp Tyr Leu Met Pro Ser Val Met Asn Leu Ile Ala
   385                 390                 395                 400
   Leu Pro Val Leu Ala Phe Ala Met Arg Tyr Ala Val Gln Gln Ala Ser
                   405                 410                 415
   Gly
<210> 18
   <211> 1170
   <212> DNA
   <213> Mortierella alpina
<220>
   <221> CDS
   <222> (1)..(1167)
   <223> LPAAT
<400> 18
   atg aac cct atc tac aag ggt ctg cga gcc att gtc tgg gcc ttt tac      48
   Met Asn Pro Ile Tyr Lys Gly Leu Arg Ala Ile Val Trp Ala Phe Tyr
   1               5                   10                  15
   ttc aac ctg gga gcg tcg ctt ata tcg atc acg cag gtg ctg tcg ctg      96
   Phe Asn Leu Gly Ala Ser Leu Ile Ser Ile Thr Gln Val Leu Ser Leu
               20                  25                  30
   cct ctg gcg ttg att gct cca ggg gtc tac cag tgg cac atc agc aaa      144
   Pro Leu Ala Leu Ile Ala Pro Gly Val Tyr Gln Trp His Ile Ser Lys
           35                  40                  45
   aca cag ggt cac ttt gga gct ttc ctg ctc cgg atg aac cag ctc ttt      192
   Thr Gln Gly His Phe Gly Ala Phe Leu Leu Arg Met Asn Gln Leu Phe
       50                  55                  60
   gcg ccg tca gat att gtc ttg aca ggg gac gag agt gtc agg gga atc      240
   Ala Pro Ser Asp Ile Val Leu Thr Gly Asp Glu Ser Val Arg Gly Ile
   65                  70                  75                  80
   gtc aag gtc tac aaa gga cgg aac ctg aag gag gcc ggt gag cca ggc      288
   Val Lys Val Tyr Lys Gly Arg Asn Leu Lys Glu Ala Gly Glu Pro Gly
                   85                  90                  95
   agc ggt cag gga gag gac att ctt ctg gat atg ccc gag agg atg gtt      336
   Ser Gly Gln Gly Glu Asp Ile Leu Leu Asp Met Pro Glu Arg Met Val
               100                 105                 110
   ttc att gcg aac cac cag atc tac tct gac tgg atg tac ctc tgg tgc      384
   Phe Ile Ala Asn His Gln Ile Tyr Ser Asp Trp Met Tyr Leu Trp Cys
           115                 120                 125
   ttc tcc tat ttt gca gag agg cac agg gca ctg aag att att ctt cgg      432
   Phe Ser Tyr Phe Ala Glu Arg His Arg Ala Leu Lys Ile Ile Leu Arg
       130                 135                 140
   ggc gac ctg acc tgg atc cct gtc ttt ggc tgg ggt atg cgg ttc ttt      480
   Gly Asp Leu Thr Trp Ile Pro Val Phe Gly Trp Gly Met Arg Phe Phe
   145                 150                 155                 160
   gac ttt atc ttt ttg aaa cgt aat gac tgg gca cac gat cgc cgt gcc      528
   Asp Phe Ile Phe Leu Lys Arg Asn Asp Trp Ala His Asp Arg Arg Ala
                   165                 170                 175
   att gag gaa aac ttg gga cgt gtc aag gaa aag gat ccc ctc tgg ctc      576
   Ile Glu Glu Asn Leu Gly Arg Val Lys Glu Lys Asp Pro Leu Trp Leu
               180                 185                 190
   gtg gtc ttc ccc gag gga aca gtc gtc tcc aag gaa acg cgt ctc cga      624
   Val Val Phe Pro Glu Gly Thr Val Val Ser Lys Glu Thr Arg Leu Arg
           195                 200                 205
   tcc gtt gcc ttt tca aag aag gct agt ctg tcg gat cac cgc cat gtg      672
   Ser Val Ala Phe Ser Lys Lys Ala Ser Leu Ser Asp His Arg His Val
       210                 215                 220
   ctg ctt cca agg acc agc ggt ctg ttt gtg tgc atc aac aag ttg cgt      720
   Leu Leu Pro Arg Thr Ser Gly Leu Phe Val Cys Ile Asn Lys Leu Arg
   225                 230                 235                 240
   gga tct gtc gac tac ttg tac gat gca acc gtt ggc tac tcg aat gtc      768
   Gly Ser Val Asp Tyr Leu Tyr Asp Ala Thr Val Gly Tyr Ser Asn Val
                   245                 250                 255
   gag tat ggc gag att ccg cag gag ctt tac ccg tta cca gga ctg tat      816
   Glu Tyr Gly Glu Ile Pro Gln Glu Leu Tyr Pro Leu Pro Gly Leu Tyr
               260                 265                 270
   atc aac aaa gca cag ccc aag gag atc aac atg cac ctg cgt cga ttt      864
   Ile Asn Lys Ala Gln Pro Lys Glu Ile Asn Met His Leu Arg Arg Phe
           275                 280                 285
   gcg atc aag gat atc ccc acg tca gaa ccc gaa ttt gtg gaa tgg gtc      912
   Ala Ile Lys Asp Ile Pro Thr Ser Glu Pro Glu Phe Val Glu Trp Val
       290                 295                 300
   cga gct cgg tgg gtg gag aag gat gag ttg atg gaa gag ttt tat acc      960
   Arg Ala Arg Trp Val Glu Lys Asp Glu Leu Met Glu Glu Phe Tyr Thr
   305                 310                 315                 320
   aag ggc cga ttt cca tca caa ctg acg gcc gcc gac att ggt gag aag      1008
   Lys Gly Arg Phe Pro Ser Gln Leu Thr Ala Ala Asp Ile Gly Glu Lys
                   325                 330                 335
   gag gtc aag acg gca gga ggt cca acg gag gga cag agt gtc agg atc      1056
   Glu Val Lys Thr Ala Gly Gly Pro Thr Glu Gly Gln Ser Val Arg Ile
               340                 345                 350
   ccg ctc aag gcg cga ggc atg atg gac tac ctc atg ccc tcg gtc atg      1104
   Pro Leu Lys Ala Arg Gly Met Met Asp Tyr Leu Met Pro Ser Val Met
           355                 360                 365
   aat ctg atc gcc ctt cct gtg ctg gcg ttt gcg atg aga tat gca gtg      1152
   Asn Leu Ile Ala Leu Pro Val Leu Ala Phe Ala Met Arg Tyr Ala Val
       370                 375                 380
   cag caa gca tcg ggc tga                                              1170
   Gln Gln Ala Ser Gly
   385
<210> 19
   <211> 389
   <212> PRT
   <213> Mortierella alpina
<400> 19
   Met Asn Pro Ile Tyr Lys Gly Leu Arg Ala Ile Val Trp Ala Phe Tyr
   1               5                   10                  15
   Phe Asn Leu Gly Ala Ser Leu Ile Ser Ile Thr Gln Val Leu Ser Leu
               20                  25                  30
   Pro Leu Ala Leu Ile Ala Pro Gly Val Tyr Gln Trp His Ile Ser Lys
           35                  40                  45
   Thr Gln Gly His Phe Gly Ala Phe Leu Leu Arg Met Asn Gln Leu Phe
       50                  55                  60
   Ala Pro Ser Asp Ile Val Leu Thr Gly Asp Glu Ser Val Arg Gly Ile
   65                  70                  75                  80
   Val Lys Val Tyr Lys Gly Arg Asn Leu Lys Glu Ala Gly Glu Pro Gly
                   85                  90                  95
   Ser Gly Gln Gly Glu Asp Ile Leu Leu Asp Met Pro Glu Arg Met Val
               100                 105                 110
   Phe Ile Ala Asn His Gln Ile Tyr Ser Asp Trp Met Tyr Leu Trp Cys
           115                 120                 125
   Phe Ser Tyr Phe Ala Glu Arg His Arg Ala Leu Lys Ile Ile Leu Arg
       130                 135                 140
   Gly Asp Leu Thr Trp Ile Pro Val Phe Gly Trp Gly Met Arg Phe Phe
   145                 150                 155                 160
   Asp Phe Ile Phe Leu Lys Arg Asn Asp Trp Ala His Asp Arg Arg Ala
                   165                 170                 175
   Ile Glu Glu Asn Leu Gly Arg Val Lys Glu Lys Asp Pro Leu Trp Leu
               180                 185                 190
   Val Val Phe Pro Glu Gly Thr Val Val Ser Lys Glu Thr Arg Leu Arg
           195                 200                 205
   Ser Val Ala Phe Ser Lys Lys Ala Ser Leu Ser Asp His Arg His Val
       210                 215                 220
   Leu Leu Pro Arg Thr Ser Gly Leu Phe Val Cys Ile Asn Lys Leu Arg
   225 230 235 240
   Gly Ser Val Asp Tyr Leu Tyr Asp Ala Thr Val Gly Tyr Ser Asn Val
   245 250 255
   Glu Tyr Gly Glu Ile Pro Gln Glu Leu Tyr Pro Leu Pro Gly Leu Tyr
   260 265 270
   Ile Asn Lys Ala Gln Pro Lys Glu Ile Asn Met His Leu Arg Arg Phe
   275 280 285
   Ala Ile Lys Asp Ile Pro Thr Ser Glu Pro Glu Phe Val Glu Trp Val
   290 295 300
   Arg Ala Arg Trp Val Glu Lys Asp Glu Leu Met Glu Glu Phe Tyr Thr
   305 310 315 320
   Lys Gly Arg Phe Pro Ser Gln Leu Thr Ala Ala Asp Ile Gly Glu Lys
   325 330 335
   Glu Val Lys Thr Ala Gly Gly Pro Thr Glu Gly Gln Ser Val Arg Ile
   340 345 350
   Pro Leu Lys Ala Arg Gly Met Met Asp Tyr Leu Met Pro Ser Val Met
   355 360 365
   Asn Leu Ile Ala Leu Pro Val Leu Ala Phe Ala Met Arg Tyr Ala Val
   370 375 380
   Gln Gln Ala Ser Gly
   385
<210> 20
   <211> 687
   <212> DNA
   <213> Shewanella hanedai
<220>
   <221> CDS
   <222> (1)..(684)
   <223> LPAAT
<400> 20
   atg tta ctg cta gca ttt gtt ttt ggt ggt ctt gtt tgt tta tta aga 48
   Met Leu Leu Leu Ala Phe Val Phe Gly Gly Leu Val Cys Leu Leu Arg
   1 5 10 15
   ccg aga cat cgt gac aat gta cac atg ttc gct aaa att ttc tcc tat 96
   Pro Arg His Arg Asp Asn Val His Met Phe Ala Lys Ile Phe Ser Tyr
   20 25 30
   gct gcg cca gta tta ggt atc aag gtc ata gta cgt aaa cct agc gta 144
   Ala Ala Pro Val Leu Gly Ile Lys Val Ile Val Arg Lys Pro Ser Val
   35 40 45
   gcg acg act gag cct tgt gtc ttt ttg gca aat cat cag aat aat ttc 192
   Ala Thr Thr Glu Pro Cys Val Phe Leu Ala Asn His Gln Asn Asn Phe
   50 55 60
   gat atg ttt acc cat act gcg gca gta ccg aaa ggg acg gtc agt ctt 240
   Asp Met Phe Thr His Thr Ala Ala Val Pro Lys Gly Thr Val Ser Leu
   65 70 75 80
   gga aag aag agt tta gct tgg gtg cct ttt ttt ggt cag att tac tgg 288
   Gly Lys Lys Ser Leu Ala Trp Val Pro Phe Phe Gly Gln Ile Tyr Trp
   85 90 95
   ttg tcc ggt aat att cta att gac aga aaa aac cgc aat aga gcg ttt      336
   Leu Ser Gly Asn Ile Leu Ile Asp Arg Lys Asn Arg Asn Arg Ala Phe
               100                 105                 110
   gaa acc atg gcg caa acc gcc aaa aag att aaa gat aag tgc tta tct      384
   Glu Thr Met Ala Gln Thr Ala Lys Lys Ile Lys Asp Lys Cys Leu Ser
           115                 120                 125
   atc tgg ata ttt ccg gaa ggt acg cgc tct cgt ggc aag ggc tta ttg      432
   Ile Trp Ile Phe Pro Glu Gly Thr Arg Ser Arg Gly Lys Gly Leu Leu
       130                 135                 140
   cct ttt aaa tct ggt gca ttt cat act gca ata gat gcg gga gtg gct      480
   Pro Phe Lys Ser Gly Ala Phe His Thr Ala Ile Asp Ala Gly Val Ala
   145                 150                 155                 160
   atg gta cct gtg ttg gca tca aat caa agc cat ata aaa ctt aat cgt      528
   Met Val Pro Val Leu Ala Ser Asn Gln Ser His Ile Lys Leu Asn Arg
                   165                 170                 175
   tgg aat aat ggt gtg gtt att atc gag atg atg gat cca atc gaa act      576
   Trp Asn Asn Gly Val Val Ile Ile Glu Met Met Asp Pro Ile Glu Thr
               180                 185                 190
   aaa ggt ttg gct aag tct cag gta aag gag ttg tct aaa cgt atc cac      624
   Lys Gly Leu Ala Lys Ser Gln Val Lys Glu Leu Ser Lys Arg Ile His
           195                 200                 205
   gct atg atg tcg aat cgt tta act cag ttg gat caa gaa gct tca gcc      672
   Ala Met Met Ser Asn Arg Leu Thr Gln Leu Asp Gln Glu Ala Ser Ala
       210                 215                 220
   tta atg gca aag taa                                                  687
   Leu Met Ala Lys
   225
<210> 21
   <211> 228
   <212> PRT
   <213> Shewanella hanedai
<400> 21
   Met Leu Leu Leu Ala Phe Val Phe Gly Gly Leu Val Cys Leu Leu Arg
   1               5                   10                  15
   Pro Arg His Arg Asp Asn Val His Met Phe Ala Lys Ile Phe Ser Tyr
               20                  25                  30
   Ala Ala Pro Val Leu Gly Ile Lys Val Ile Val Arg Lys Pro Ser Val
           35                  40                  45
   Ala Thr Thr Glu Pro Cys Val Phe Leu Ala Asn His Gln Asn Asn Phe
       50                  55                  60
   Asp Met Phe Thr His Thr Ala Ala Val Pro Lys Gly Thr Val Ser Leu
   65                  70                  75                  80
   Gly Lys Lys Ser Leu Ala Trp Val Pro Phe Phe Gly Gln Ile Tyr Trp
                   85                  90                  95
   Leu Ser Gly Asn Ile Leu Ile Asp Arg Lys Asn Arg Asn Arg Ala Phe
               100                 105                 110
   Glu Thr Met Ala Gln Thr Ala Lys Lys Ile Lys Asp Lys Cys Leu Ser
           115                 120                 125
   Ile Trp Ile Phe Pro Glu Gly Thr Arg Ser Arg Gly Lys Gly Leu Leu
       130                 135                 140
   Pro Phe Lys Ser Gly Ala Phe His Thr Ala Ile Asp Ala Gly Val Ala
   145                 150                 155                 160
   Met Val Pro Val Leu Ala Ser Asn Gln Ser His Ile Lys Leu Asn Arg
                   165                 170                 175
   Trp Asn Asn Gly Val Val Ile Ile Glu Met Met Asp Pro Ile Glu Thr
               180                 185                 190
   Lys Gly Leu Ala Lys Ser Gln Val Lys Glu Leu Ser Lys Arg Ile His
           195                 200                 205
   Ala Met Met Ser Asn Arg Leu Thr Gln Leu Asp Gln Glu Ala Ser Ala
       210                 215                 220
   Leu Met Ala Lys
   225
<210> 22
   <211> 1352
   <212> DNA
   <213> Physcomitrella patens
<220>
   <221> CDS
   <222> (39)..(1340)
   <223> GPAT
<400> 22
   ggccgcaagg taaccgcctt ctgccgcaag ccttgact atg ccg tcg ctg ttt cgg      56
                                             Met Pro Ser Leu Phe Arg
                                             1               5
   gcg aaa cgc aat ggc aga agg acg ccg ggg aat gcc gtg acc aat ttc      104
   Ala Lys Arg Asn Gly Arg Arg Thr Pro Gly Asn Ala Val Thr Asn Phe
               10                  15                  20
   ggg aaa tct gaa ttc cat cgt gaa att agt ggg agt acg cgg gcg acc      152
   Gly Lys Ser Glu Phe His Arg Glu Ile Ser Gly Ser Thr Arg Ala Thr
           25                  30                  35
   acg cag gtg gct gaa gcc acc aca gct ggt ctt agg gag acc att gag      200
   Thr Gln Val Ala Glu Ala Thr Thr Ala Gly Leu Arg Glu Thr Ile Glu
       40                  45                  50
   gac cgc gct att atc gac ggt cat tct cac agt ttt gaa gga att caa      248
   Asp Arg Ala Ile Ile Asp Gly His Ser His Ser Phe Glu Gly Ile Gln
   55                  60                  65                  70
   tcg gaa gaa gag ttg atg cag gta att gaa aag gag gtg gaa tcc ggt      296
   Ser Glu Glu Glu Leu Met Gln Val Ile Glu Lys Glu Val Glu Ser Gly
                   75                  80                  85
   cgg ctg ccg aag cgt gct ggc gcg gga atg gta gag ttg tat cgc aat      344
   Arg Leu Pro Lys Arg Ala Gly Ala Gly Met Val Glu Leu Tyr Arg Asn
               90                  95                  100
   tat cga gat gct gta gtg agc agt ggc gta gaa aat gcg atg gat att      392
   Tyr Arg Asp Ala Val Val Ser Ser Gly Val Glu Asn Ala Met Asp Ile
           105                 110                 115
   gtt gtg aaa gtc atg tca act gtg ttg gac cgg att ctt ctg cag ttc      440
   Val Val Lys Val Met Ser Thr Val Leu Asp Arg Ile Leu Leu Gln Phe
       120                 125                 130
   gag gag cca ttc aca ttt gga tcg cac cac aag aga atg gtg gag ccg      488
   Glu Glu Pro Phe Thr Phe Gly Ser His His Lys Arg Met Val Glu Pro
   135                 140                 145                 150
   tat gat tac tac aca ttt ggt cag aac tat gtg cgt cct ctc cta gat      536
   Tyr Asp Tyr Tyr Thr Phe Gly Gln Asn Tyr Val Arg Pro Leu Leu Asp
                   155                 160                 165
   ttc agg aac tct tac ctt ggg aac tta aag atc ttt gac cag ata gag      584
   Phe Arg Asn Ser Tyr Leu Gly Asn Leu Lys Ile Phe Asp Gln Ile Glu
               170                 175                 180
   aag aac ctg aaa gag ggg cac aac gtc att ttt cta tcc aat cac cag      632
   Lys Asn Leu Lys Glu Gly His Asn Val Ile Phe Leu Ser Asn His Gln
           185                 190                 195
   act gag gca gat cct gct gtt atg gcg ctg ttg ctt gag cac tct cac      680
   Thr Glu Ala Asp Pro Ala Val Met Ala Leu Leu Leu Glu His Ser His
       200                 205                 210
   ccc tat ttg gca gag aac ttg acc tat gtg gct gga gac agg gtt gtg      728
   Pro Tyr Leu Ala Glu Asn Leu Thr Tyr Val Ala Gly Asp Arg Val Val
   215                 220                 225                 230
   ctg gat cca ttc tgc aaa cct ttt agt atg ggc agg aat ctc ttg tgc      776
   Leu Asp Pro Phe Cys Lys Pro Phe Ser Met Gly Arg Asn Leu Leu Cys
                   235                 240                 245
   gtg tat tca aaa aag cac att cac gat gta ccg gac ctt gct gaa atg      824
   Val Tyr Ser Lys Lys His Ile His Asp Val Pro Asp Leu Ala Glu Met
               250                 255                 260
   aaa atc aaa gct aat gcg aag act ttg aga cag atg acg atc ctg ctg      872
   Lys Ile Lys Ala Asn Ala Lys Thr Leu Arg Gln Met Thr Ile Leu Leu
           265                 270                 275
   agg cag gga ggt caa tta tta tgg gta gca ccc agt ggt gga cgc gat      920
   Arg Gln Gly Gly Gln Leu Leu Trp Val Ala Pro Ser Gly Gly Arg Asp
       280                 285                 290
   cgc cct gat cct gag acc aac gaa tgg gtt cct gca cat ttt gac tcg      968
   Arg Pro Asp Pro Glu Thr Asn Glu Trp Val Pro Ala His Phe Asp Ser
   295                 300                 305                 310
   tct gct gtg gag aat atg aag cga cta tct gac att gtc cga gta cct     1016
   Ser Ala Val Glu Asn Met Lys Arg Leu Ser Asp Ile Val Arg Val Pro
                   315                 320                 325
   gct cat tta cat gcc cta tca tta cta tgt ttt gag att atg cca cct     1064
   Ala His Leu His Ala Leu Ser Leu Leu Cys Phe Glu Ile Met Pro Pro
               330                 335                 340
   cct gtc cag gta caa aag gag cta gga gag cga aga gca gta gga ttt     1112
   Pro Val Gln Val Gln Lys Glu Leu Gly Glu Arg Arg Ala Val Gly Phe
           345                 350                 355
   agc gga gtt ggt cta gcc gtt tcc gag caa cta gat tat gat tcc att     1160
   Ser Gly Val Gly Leu Ala Val Ser Glu Gln Leu Asp Tyr Asp Ser Ile
       360                 365                 370
   gcg aag tta gtc gac gat tcc aaa aat gcg aag gat gcc ttt tcg gat     1208
   Ala Lys Leu Val Asp Asp Ser Lys Asn Ala Lys Asp Ala Phe Ser Asp
   375                 380                 385                 390
   gcg gca tgg agc gaa gtc aat gat atg tat aac gtg tta aaa gaa gca     1256
   Ala Ala Trp Ser Glu Val Asn Asp Met Tyr Asn Val Leu Lys Glu Ala
                   395                 400                 405
   att tat ggt gac caa ggt tgt gct gtt agc aca gat tcc ttg aga ctg     1304
   Ile Tyr Gly Asp Gln Gly Cys Ala Val Ser Thr Asp Ser Leu Arg Leu
               410                 415                 420
   gaa cag ccc tgg ttt gat gga agc agg cga act gat tgaaaatagg gc       1352
   Glu Gln Pro Trp Phe Asp Gly Ser Arg Arg Thr Asp
   425 430
<210> 23
   <211> 434
   <212> PRT
   <213> Physcomitrella patens
<400> 23
   Met Pro Ser Leu Phe Arg Ala Lys Arg Asn Gly Arg Arg Thr Pro Gly
   1 5 10 15
   Asn Ala Val Thr Asn Phe Gly Lys Ser Glu Phe His Arg Glu Ile Ser
   20 25 30
   Gly Ser Thr Arg Ala Thr Thr Gln Val Ala Glu Ala Thr Thr Ala Gly
   35 40 45
   Leu Arg Glu Thr Ile Glu Asp Arg Ala Ile Ile Asp Gly His Ser His
   50 55 60
   Ser Phe Glu Gly Ile Gln Ser Glu Glu Glu Leu Met Gln Val Ile Glu
   65 70 75 80
   Lys Glu Val Glu Ser Gly Arg Leu Pro Lys Arg Ala Gly Ala Gly Met
   85 90 95
   Val Glu Leu Tyr Arg Asn Tyr Arg Asp Ala Val Val Ser Ser Gly Val
   100 105 110
   Glu Asn Ala Met Asp Ile Val Val Lys Val Met Ser Thr Val Leu Asp
   115 120 125
   Arg Ile Leu Leu Gln Phe Glu Glu Pro Phe Thr Phe Gly Ser His His
   130 135 140
   Lys Arg Met Val Glu Pro Tyr Asp Tyr Tyr Thr Phe Gly Gln Asn Tyr
   145 150 155 160
   Val Arg Pro Leu Leu Asp Phe Arg Asn Ser Tyr Leu Gly Asn Leu Lys
   165 170 175
   Ile Phe Asp Gln Ile Glu Lys Asn Leu Lys Glu Gly His Asn Val Ile
   180 185 190
   Phe Leu Ser Asn His Gln Thr Glu Ala Asp Pro Ala Val Met Ala Leu
   195 200 205
   Leu Leu Glu His Ser His Pro Tyr Leu Ala Glu Asn Leu Thr Tyr Val
   210 215 220
   Ala Gly Asp Arg Val Val Leu Asp Pro Phe Cys Lys Pro Phe Ser Met
   225 230 235 240
   Gly Arg Asn Leu Leu Cys Val Tyr Ser Lys Lys His Ile His Asp Val
   245 250 255
   Pro Asp Leu Ala Glu Met Lys Ile Lys Ala Asn Ala Lys Thr Leu Arg
   260 265 270
   Gln Met Thr Ile Leu Leu Arg Gln Gly Gly Gln Leu Leu Trp Val Ala
   275 280 285
   Pro Ser Gly Gly Arg Asp Arg Pro Asp Pro Glu Thr Asn Glu Trp Val
   290 295 300
   Pro Ala His Phe Asp Ser Ser Ala Val Glu Asn Met Lys Arg Leu Ser
   305 310 315 320
   Asp Ile Val Arg Val Pro Ala His Leu His Ala Leu Ser Leu Leu Cys
   325 330 335
   Phe Glu Ile Met Pro Pro Pro Val Gln Val Gln Lys Glu Leu Gly Glu
                       340                 345                 350
   Arg Arg Ala Val Gly Phe Ser Gly Val Gly Leu Ala Val Ser Glu Gln
                   355                 360                 365
   Leu Asp Tyr Asp Ser Ile Ala Lys Leu Val Asp Asp Ser Lys Asn Ala
       370                 375                 380
   Lys Asp Ala Phe Ser Asp Ala Ala Trp Ser Glu Val Asn Asp Met Tyr
   385                 390                 395                 400
   Asn Val Leu Lys Glu Ala Ile Tyr Gly Asp Gln Gly Cys Ala Val Ser
                   405                 410                 415
   Thr Asp Ser Leu Arg Leu Glu Gln Pro Trp Phe Asp Gly Ser Arg Arg
               420                 425                 430
   Thr Asp
<210> 24
   <211> 444
   <212> DNA
   <213> Physcomitrella patens
<220>
   <221> CDS
   <222> (1)..(444)
   <223> GPAT/LPAAT
<400> 24
   atg atc cgg att ttc aga ggg caa cca tct gtg gtt cat gtg cac gtg      48
   Met Ile Arg Ile Phe Arg Gly Gln Pro Ser Val Val His Val His Val
   1               5                   10                  15
   agg cgg gtc cct atg tct gat ctg cct gag gga gcc aac gcg att tct      96
   Arg Arg Val Pro Met Ser Asp Leu Pro Glu Gly Ala Asn Ala Ile Ser
               20                  25                  30
   aaa tgg tgt cac gat gcc ttt cac atc aag gac gat cgg ctg gag cag     144
   Lys Trp Cys His Asp Ala Phe His Ile Lys Asp Asp Arg Leu Glu Gln
           35                  40                  45
   cac gaa aaa gag aat acg ttt ggg gag gac ttg tat att cct att gaa     192
   His Glu Lys Glu Asn Thr Phe Gly Glu Asp Leu Tyr Ile Pro Ile Glu
       50                  55                  60
   cgg cca ctt aaa cct ctt att att gtg atc tcc tgg gcc atc act ttg     240
   Arg Pro Leu Lys Pro Leu Ile Ile Val Ile Ser Trp Ala Ile Thr Leu
   65                  70                  75                  80
   ctg gct gca gca tgg tgg ttt cta aga cga gtt tta tcc act tgg aaa     288
   Leu Ala Ala Ala Trp Trp Phe Leu Arg Arg Val Leu Ser Thr Trp Lys
                   85                  90                  95
   gga atc gcc tgg gtg gca gga gta ctc gtg gtc gtc atg ctg tgt gtc     336
   Gly Ile Ala Trp Val Ala Gly Val Leu Val Val Val Met Leu Cys Val
               100                 105                 110
   cag att tta gtg atg tcg tca caa tcg gaa aga agt tca gat cct gca     384
   Gln Ile Leu Val Met Ser Ser Gln Ser Glu Arg Ser Ser Asp Pro Ala
           115                 120                 125
   gct aag aag gcc aat caa aaa cag gcg gct tct gtt gct cac ctc ggc     432
   Ala Lys Lys Ala Asn Gln Lys Gln Ala Ala Ser Val Ala His Leu Gly
       130                 135                 140
   aaa acg gac tga                                                    444
   Lys Thr Asp
   145
<210> 25
   <211> 147
   <212> PRT
   <213> Physcomitrella patens
<400> 25
   Met Ile Arg Ile Phe Arg Gly Gln Pro Ser Val Val His Val His Val
   1               5                   10                  15
   Arg Arg Val Pro Met Ser Asp Leu Pro Glu Gly Ala Asn Ala Ile Ser
               20                  25                  30
   Lys Trp Cys His Asp Ala Phe His Ile Lys Asp Asp Arg Leu Glu Gln
           35                  40                  45
   His Glu Lys Glu Asn Thr Phe Gly Glu Asp Leu Tyr Ile Pro Ile Glu
       50                  55                  60
   Arg Pro Leu Lys Pro Leu Ile Ile Val Ile Ser Trp Ala Ile Thr Leu
   65                  70                  75                  80
   Leu Ala Ala Ala Trp Trp Phe Leu Arg Arg Val Leu Ser Thr Trp Lys
                   85                  90                  95
   Gly Ile Ala Trp Val Ala Gly Val Leu Val Val Val Met Leu Cys Val
               100                 105                 110
   Gln Ile Leu Val Met Ser Ser Gln Ser Glu Arg Ser Ser Asp Pro Ala
           115                 120                 125
   Ala Lys Lys Ala Asn Gln Lys Gln Ala Ala Ser Val Ala His Leu Gly
       130                 135                 140
   Lys Thr Asp
   145
<210> 26
   <211> 1710
   <212> DNA
   <213> Physcomitrella patens
<220>
   <221> CDS
   <222> (246)..(1394)
   <223> GPAT/LPAAT
<400> 26
   gaattcgccc tttctctttt tcgtgctgct ccagccgata ttcatgacct gcccgggcag      60
   gtcacattgc gtgttggcca tgtcctggtt gcagctctcg tgaccctcac gctcgcgagc     120
   ggcaccgctc gtcttctgcc tcttgcttgc tcttgcttgc tttctgagga acagccccag     180
   ctccggcacc agcataaggt cgtgtaggga gagagagaga gggggagaga agtaagcttg     240
   gagtc atg gag ggc ggg ggc tcc ata atc gct ctt cct ctg ggg ctt atg     290
         Met Glu Gly Gly Gly Ser Ile Ile Ala Leu Pro Leu Gly Leu Met
         1               5                   10                  15
   ttc ctc ttc tcc ggg ttc ttt atc aat atc ctg cag ctg ctg tcg gtg       338
   Phe Leu Phe Ser Gly Phe Phe Ile Asn Ile Leu Gln Leu Leu Ser Val
                   20                  25                  30
   tta ttc att ttg ccg ttt tcg agg agg gcg tac cga gta gtg aat atg      386
   Leu Phe Ile Leu Pro Phe Ser Arg Arg Ala Tyr Arg Val Val Asn Met
               35                  40                  45
   att atg atg gag gtg ctg tgg tcg gag ctt ata tgg ctg ctg gat tgg      434
   Ile Met Met Glu Val Leu Trp Ser Glu Leu Ile Trp Leu Leu Asp Trp
           50                  55                  60
   tgg gcg aat gtg aag gtg aag gtt tac acg cca aag gag tcg tgg gag      482
   Trp Ala Asn Val Lys Val Lys Val Tyr Thr Pro Lys Glu Ser Trp Glu
       65                  70                  75
   cac tta gga aag gag cac gca tta ctc att tgt aat cac cgc agt gac      530
   His Leu Gly Lys Glu His Ala Leu Leu Ile Cys Asn His Arg Ser Asp
   80                  85                  90                  95
   ata gat tgg ctc gta gga tgg att att gcc cag aga ttg ggg tgt cta      578
   Ile Asp Trp Leu Val Gly Trp Ile Ile Ala Gln Arg Leu Gly Cys Leu
                   100                 105                 110
   ggt ggg act cga gct gtt atg aag aag tcc acc aaa ttt ctt ccg gtc      626
   Gly Gly Thr Arg Ala Val Met Lys Lys Ser Thr Lys Phe Leu Pro Val
               115                 120                 125
   att ggc tgg tct atg tgg ttt tca gag tat gtg ttt tta tca aga gat      674
   Ile Gly Trp Ser Met Trp Phe Ser Glu Tyr Val Phe Leu Ser Arg Asp
           130                 135                 140
   tgg gcc aaa gat gag aag gtc ttg aag aat ggt tat tca agt ctt aag      722
   Trp Ala Lys Asp Glu Lys Val Leu Lys Asn Gly Tyr Ser Ser Leu Lys
       145                 150                 155
   ggc ttc ccc agg acc ttg tgg gtg gct ctt ttt gtg gaa ggc act cga      770
   Gly Phe Pro Arg Thr Leu Trp Val Ala Leu Phe Val Glu Gly Thr Arg
   160                 165                 170                 175
   ttt acg aag gcc aaa ctt gag gct gcc caa aaa ttt gca gcg gat aca      818
   Phe Thr Lys Ala Lys Leu Glu Ala Ala Gln Lys Phe Ala Ala Asp Thr
                   180                 185                 190
   ggg cta cgt gtt cca agg cat gtg ctt gtt cct cgc aca aaa ggg ttc      866
   Gly Leu Arg Val Pro Arg His Val Leu Val Pro Arg Thr Lys Gly Phe
               195                 200                 205
   gtt tcg gct gtg gag aac ttg cgt gaa ttt gtt ccg gta gtt tat gac      914
   Val Ser Ala Val Glu Asn Leu Arg Glu Phe Val Pro Val Val Tyr Asp
           210                 215                 220
   atg acc gtt gct ata tct aaa gag ctg ccc aat cct aca atg atc cgg      962
   Met Thr Val Ala Ile Ser Lys Glu Leu Pro Asn Pro Thr Met Ile Arg
       225                 230                 235
   att ttc aga ggg caa cca tct gtg gtt cat gtg cac gtg aga cgg gtc     1010
   Ile Phe Arg Gly Gln Pro Ser Val Val His Val His Val Arg Arg Val
   240                 245                 250                 255
   cct atg tct gat ctg cct gag gga gcc aac gcg att tct aaa tgg tgt     1058
   Pro Met Ser Asp Leu Pro Glu Gly Ala Asn Ala Ile Ser Lys Trp Cys
                   260                 265                 270
   cac gat gcc ttt cac atc aag gac gat cgg ctg gag cag cac gaa aaa     1106
   His Asp Ala Phe His Ile Lys Asp Asp Arg Leu Glu Gln His Glu Lys
               275                 280                 285
   gag aat acg ttt ggg gag gac ttg tat att cct att gaa cgg cca ctt     1154
   Glu Asn Thr Phe Gly Glu Asp Leu Tyr Ile Pro Ile Glu Arg Pro Leu
           290                 295                 300
   aaa cct ctt att att gtg atc tcc tgg gcc atc act ttg ctg gct gca     1202
   Lys Pro Leu Ile Ile Val Ile Ser Trp Ala Ile Thr Leu Leu Ala Ala
       305                 310                 315
   gca tgg tgg ttt cta aga cga gtt tta tcc act tgg aaa gga atc gcc      1250
   Ala Trp Trp Phe Leu Arg Arg Val Leu Ser Thr Trp Lys Gly Ile Ala
   320                 325                 330                 335
   tgg gtg gca gga gta ctc gtg gtc gtc atg ctg tgt gtc cag att tta      1298
   Trp Val Ala Gly Val Leu Val Val Val Met Leu Cys Val Gln Ile Leu
                   340                 345                 350
   gtg atg tcg tca caa tcg gaa aga agt tca gat cct gca gct aag aag      1346
   Val Met Ser Ser Gln Ser Glu Arg Ser Ser Asp Pro Ala Ala Lys Lys
               355                 360                 365
   gcc aat caa aaa cag gcg gct tct gtt gct cac ctc ggc aaa acg gac      1394
   Ala Asn Gln Lys Gln Ala Ala Ser Val Ala His Leu Gly Lys Thr Asp
           370                 375                 380
   tgagaacttt tgctttaacg caatccaaga cttaggcgtg ctagtctcag ttacaattag    1454
   cattcaggca ctccagatgt gtcaagaaat tttagttact ctagccaaga attgtttgac    1514
   accttgtagt ccacctaatt tccttgaacg attaagagca gcggccatta gatgattcga    1574
   tttggtttct tgatagtatc tggtaccttc ttcttcaagc attgtgtatt ccgcttcagc    1634
   cattcctttt tttaagatgt attgcttctc gttcgagggt aggtcatttc tgatctaatt    1694
   ttgaaagcac taattc                                                    1710
<210> 27
   <211> 383
   <212> PRT
   <213> Physcomitrella patens
<400> 27
   Met Glu Gly Gly Gly Ser Ile Ile Ala Leu Pro Leu Gly Leu Met Phe
   1               5                   10                  15
   Leu Phe Ser Gly Phe Phe Ile Asn Ile Leu Gln Leu Leu Ser Val Leu
               20                  25                  30
   Phe Ile Leu Pro Phe Ser Arg Arg Ala Tyr Arg Val Val Asn Met Ile
           35                  40                  45
   Met Met Glu Val Leu Trp Ser Glu Leu Ile Trp Leu Leu Asp Trp Trp
       50                  55                  60
   Ala Asn Val Lys Val Lys Val Tyr Thr Pro Lys Glu Ser Trp Glu His
   65                  70                  75                  80
   Leu Gly Lys Glu His Ala Leu Leu Ile Cys Asn His Arg Ser Asp Ile
                   85                  90                  95
   Asp Trp Leu Val Gly Trp Ile Ile Ala Gln Arg Leu Gly Cys Leu Gly
               100                 105                 110
   Gly Thr Arg Ala Val Met Lys Lys Ser Thr Lys Phe Leu Pro Val Ile
           115                 120                 125
   Gly Trp Ser Met Trp Phe Ser Glu Tyr Val Phe Leu Ser Arg Asp Trp
       130                 135                 140
   Ala Lys Asp Glu Lys Val Leu Lys Asn Gly Tyr Ser Ser Leu Lys Gly
   145                 150                 155                 160
   Phe Pro Arg Thr Leu Trp Val Ala Leu Phe Val Glu Gly Thr Arg Phe
                   165                 170                 175
   Thr Lys Ala Lys Leu Glu Ala Ala Gln Lys Phe Ala Ala Asp Thr Gly
               180                 185                 190
   Leu Arg Val Pro Arg His Val Leu Val Pro Arg Thr Lys Gly Phe Val
           195                 200                 205
   Ser Ala Val Glu Asn Leu Arg Glu Phe Val Pro Val Val Tyr Asp Met
       210                 215                 220
   Thr Val Ala Ile Ser Lys Glu Leu Pro Asn Pro Thr Met Ile Arg Ile
   225                 230                 235                 240
   Phe Arg Gly Gln Pro Ser Val Val His Val His Val Arg Arg Val Pro
                   245                 250                 255
   Met Ser Asp Leu Pro Glu Gly Ala Asn Ala Ile Ser Lys Trp Cys His
               260                 265                 270
   Asp Ala Phe His Ile Lys Asp Asp Arg Leu Glu Gln His Glu Lys Glu
           275                 280                 285
   Asn Thr Phe Gly Glu Asp Leu Tyr Ile Pro Ile Glu Arg Pro Leu Lys
       290                 295                 300
   Pro Leu Ile Ile Val Ile Ser Trp Ala Ile Thr Leu Leu Ala Ala Ala
   305                 310                 315                 320
   Trp Trp Phe Leu Arg Arg Val Leu Ser Thr Trp Lys Gly Ile Ala Trp
                   325                 330                 335
   Val Ala Gly Val Leu Val Val Val Met Leu Cys Val Gln Ile Leu Val
               340                 345                 350
   Met Ser Ser Gln Ser Glu Arg Ser Ser Asp Pro Ala Ala Lys Lys Ala
           355                 360                 365
   Asn Gln Lys Gln Ala Ala Ser Val Ala His Leu Gly Lys Thr Asp
       370                 375                 380
<210> 28
   <211> 628
   <212> DNA
   <213> Cryptocodinium cohnii
<220>
   <221> CDS
   <222> (3)..(578)
   <223> DAGAT
<400> 28
   tt gat gat tgg atc gcc gcg ttg gcg act gct tgt gca agc acg gat        47
      Asp Asp Trp Ile Ala Ala Leu Ala Thr Ala Cys Ala Ser Thr Asp
      1               5                   10                  15
   ggg gtt acg gac gtc gac agc ctg aag ccc tca gca agt gca gtt ccc       95
   Gly Val Thr Asp Val Asp Ser Leu Lys Pro Ser Ala Ser Ala Val Pro
                   20                  25                  30
   cat gga ccc ccc aag gcg aag gtc agt gag cta tcg gcc ctg cgc aag      143
   His Gly Pro Pro Lys Ala Lys Val Ser Glu Leu Ser Ala Leu Arg Lys
               35                  40                  45
   gtg cac aat cga aac cgg acc agc gtt ttg acc aac gag gac gga ggc      191
   Val His Asn Arg Asn Arg Thr Ser Val Leu Thr Asn Glu Asp Gly Gly
           50                  55                  60
   att cct gag tgc aac gtt gtg ggg atc gtg aac ctc tgt gtt act gtg      239
   Ile Pro Glu Cys Asn Val Val Gly Ile Val Asn Leu Cys Val Thr Val
       65                  70                  75
   atg gtc ttg atc cac ctg cgc ctc att tat gag agc atc cgg aag cac      287
   Met Val Leu Ile His Leu Arg Leu Ile Tyr Glu Ser Ile Arg Lys His
   80                   85                   90                   95
   ggt gtt ttg ttg gac acc ttc cgg gtg gcg gcc cac acc gca ctc aag       335
   Gly Val Leu Leu Asp Thr Phe Arg Val Ala Ala His Thr Ala Leu Lys
                   100                  105                  110
   cca ggt aac ttc cag tgt acg ctt tgt ttc ttc gct ttg ccg gtc ctg       383
   Pro Gly Asn Phe Gln Cys Thr Leu Cys Phe Phe Ala Leu Pro Val Leu
               115                  120                  125
   gcc atc ttg gcg acc ttc att gag gtc ttg gcg agc aag gga cag ttg       431
   Ala Ile Leu Ala Thr Phe Ile Glu Val Leu Ala Ser Lys Gly Gln Leu
           130                  135                  140
   ggg atc tcg ctt cgc gag cac cct gca tgc cgg gct ttg tac aat ctg       479
   Gly Ile Ser Leu Arg Glu His Pro Ala Cys Arg Ala Leu Tyr Asn Leu
       145                  150                  155
   cct tac cat ccc tgt cct ggt cat cca cca ctt tca ggc aac tcc tct       527
   Pro Tyr His Pro Cys Pro Gly His Pro Pro Leu Ser Gly Asn Ser Ser
   160                  165                  170                  175
   cgt ggg agc ctc gtt gct gat tgc tgc gac cac tct ctt ctt gaa agt       575
   Arg Gly Ser Leu Val Ala Asp Cys Cys Asp His Ser Leu Leu Glu Ser
                   180                  185                  190
   tgg tgagcttcgc ccacgtgaat tggctctcgg cgacagtgga aggcgatgga            628
   Trp
<210> 29
   <211> 192
   <212> PRT
   <213> Cryptocodinium cohnii
<400> 29
   Asp Asp Trp Ile Ala Ala Leu Ala Thr Ala Cys Ala Ser Thr Asp Gly
   1               5                   10                  15
   Val Thr Asp Val Asp Ser Leu Lys Pro Ser Ala Ser Ala Val Pro His
               20                  25                  30
   Gly Pro Pro Lys Ala Lys Val Ser Glu Leu Ser Ala Leu Arg Lys Val
           35                  40                  45
   His Asn Arg Asn Arg Thr Ser Val Leu Thr Asn Glu Asp Gly Gly Ile
       50                  55                  60
   Pro Glu Cys Asn Val Val Gly Ile Val Asn Leu Cys Val Thr Val Met
   65                  70                  75                  80
   Val Leu Ile His Leu Arg Leu Ile Tyr Glu Ser Ile Arg Lys His Gly
                   85                  90                  95
   Val Leu Leu Asp Thr Phe Arg Val Ala Ala His Thr Ala Leu Lys Pro
               100                 105                 110
   Gly Asn Phe Gln Cys Thr Leu Cys Phe Phe Ala Leu Pro Val Leu Ala
           115                 120                 125
   Ile Leu Ala Thr Phe Ile Glu Val Leu Ala Ser Lys Gly Gln Leu Gly
       130                 135                 140
   Ile Ser Leu Arg Glu His Pro Ala Cys Arg Ala Leu Tyr Asn Leu Pro
   145                 150                 155                 160
   Tyr His Pro Cys Pro Gly His Pro Pro Leu Ser Gly Asn Ser Ser Arg
                   165                 170                 175
   Gly Ser Leu Val Ala Asp Cys Cys Asp His Ser Leu Leu Glu Ser Trp
                   180                 185                 190
<210> 30
   <211> 1272
   <212> DNA
   <213> Cryrptocodinium cohnii
<220>
   <221> CDS
   <222> (164)..(1120)
   <223> DAGAT
<400> 30
   ggacactgac atggactgaa ggagtagaaa gccgtagcca ttttggctca agctccagtg      60
   aacagtcgcg ccctgactgc agaggggtgc ggcacaaacc ctcagataca cacacatccc     120
   gtgagtttat agattcttgt ctcgcgctct tcttgtgcaa gcg atg gct gga aag       175
                                                   Met Ala Gly Lys
                                                   1
   tgg atg ctg ctc agt ggt ggt gca gca gct gca gcg ttg gcg ctt ctg       223
   Trp Met Leu Leu Ser Gly Gly Ala Ala Ala Ala Ala Leu Ala Leu Leu
   5                   10                  15                  20
   gag ggc acc cag ctt cga gcg tcg aca tcg gca cgc gcc cgg ata ttg       271
   Glu Gly Thr Gln Leu Arg Ala Ser Thr Ser Ala Arg Ala Arg Ile Leu
                   25                  30                  35
   ctg gtt tcg ttg gca gca tat ctc cca acg tac ctc gat gga agc gag       319
   Leu Val Ser Leu Ala Ala Tyr Leu Pro Thr Tyr Leu Asp Gly Ser Glu
               40                  45                  50
   tac cgg gct gcc cct cga cga agc gag cga gcc tca cgg gtc ctg cgg       367
   Tyr Arg Ala Ala Pro Arg Arg Ser Glu Arg Ala Ser Arg Val Leu Arg
           55                  60                  65
   cag ttg tac aaa gtc atg gta aat tgg ttc ttc aca atc aaa cgg cca       415
   Gln Leu Tyr Lys Val Met Val Asn Trp Phe Phe Thr Ile Lys Arg Pro
       70                  75                  80
   gta atc gag gct tcc gaa gag ctg aca gct tgt gac cag tgc atc ttg       463
   Val Ile Glu Ala Ser Glu Glu Leu Thr Ala Cys Asp Gln Cys Ile Leu
   85                  90                  95                  100
   gcg gtc cat ccc cat gga gta cct tct ctc gac cat ttg ctg acg gtc       511
   Ala Val His Pro His Gly Val Pro Ser Leu Asp His Leu Leu Thr Val
                   105                 110                 115
   atc gcc tat gat cct gac ttg gaa cgg gtg ttg ccc cag ttg cgg aga       559
   Ile Ala Tyr Asp Pro Asp Leu Glu Arg Val Leu Pro Gln Leu Arg Arg
               120                 125                 130
   agt gcc ttg agt gca ggt gtc ctg ttc aag att ccc att ctg cgc gag       607
   Ser Ala Leu Ser Ala Gly Val Leu Phe Lys Ile Pro Ile Leu Arg Glu
           135                 140                 145
   gtc ctt ctg tgg act ggc tgt gtc gac gct ggc ggg aag acc gtg gac       655
   Val Leu Leu Trp Thr Gly Cys Val Asp Ala Gly Gly Lys Thr Val Asp
       150                 155                 160
   tct tgc ttg aag gct ggt ctc agc ctt tct gtt gtg ccc ggc ggc gaa       703
   Ser Cys Leu Lys Ala Gly Leu Ser Leu Ser Val Val Pro Gly Gly Glu
   165                 170                 175                 180
   cgc gag caa ctt ctc gca cag cga ggg aac aag gaa atc ctc gtg ctg      751
   Arg Glu Gln Leu Leu Ala Gln Arg Gly Asn Lys Glu Ile Leu Val Leu
                   185                 190                 195
   aaa cac agg aag ggc ttt gtc aag tac gcc ttg agg cat ggc att ccg      799
   Lys His Arg Lys Gly Phe Val Lys Tyr Ala Leu Arg His Gly Ile Pro
               200                 205                 210
   ttg gta cct gtg tat tgc ttc ggc gag aac caa ctt ttt tgg cag tcc      847
   Leu Val Pro Val Tyr Cys Phe Gly Glu Asn Gln Leu Phe Trp Gln Ser
           215                 220                 225
   tcc ttc ctc ttc aag gtt cgc agt tgg ctg cgg cgc act ctg gga gtg      895
   Ser Phe Leu Phe Lys Val Arg Ser Trp Leu Arg Arg Thr Leu Gly Val
       230                 235                 240
   gcg ctc gtg ttg ccc tac gga ggc tgc tgc aat ctg cct ggt gtg ccc      943
   Ala Leu Val Leu Pro Tyr Gly Gly Cys Cys Asn Leu Pro Gly Val Pro
   245                 250                 255                 260
   ttc tcg gag ccg gtg cag ctc gtc gtc gga gct ccc ttg aag ctt ccg      991
   Phe Ser Glu Pro Val Gln Leu Val Val Gly Ala Pro Leu Lys Leu Pro
                   265                 270                 275
   aag atc gaa gag ccg agc gga gtg gaa ata gcc aag tgg cac gct cgg     1039
   Lys Ile Glu Glu Pro Ser Gly Val Glu Ile Ala Lys Trp His Ala Arg
               280                 285                 290
   tac atg gag tgt ttg gaa gcc ttg ttc aag cgg cac cga gtt gaa gct     1087
   Tyr Met Glu Cys Leu Glu Ala Leu Phe Lys Arg His Arg Val Glu Ala
           295                 300                 305
   gga tat cct gaa ttg gaa ctc gag ttc atc tga aggtttcaag tttacatgtg   1140
   Gly Tyr Pro Glu Leu Glu Leu Glu Phe Ile
       310                 315
   tctcacagtc ctccgctctg agccccactc attgtagtta ctcttctatg tgtgcaacgt   1200
   cgaccacagg agttaccgtc aaagacggtt gctccttgct gcttcgagag aaaaaaaaaa   1260
   aaaaaaaaaa aa                                                       1272
<210> 31
   <211> 318
   <212> PRT
   <213> Cryptocodinium cohnii
<400> 31
   Met Ala Gly Lys Trp Met Leu Leu Ser Gly Gly Ala Ala Ala Ala Ala
   1               5                   10                  15
   Leu Ala Leu Leu Glu Gly Thr Gln Leu Arg Ala Ser Thr Ser Ala Arg
               20                  25                  30
   Ala Arg Ile Leu Leu Val Ser Leu Ala Ala Tyr Leu Pro Thr Tyr Leu
           35                  40                  45
   Asp Gly Ser Glu Tyr Arg Ala Ala Pro Arg Arg Ser Glu Arg Ala Ser
       50                  55                  60
   Arg Val Leu Arg Gln Leu Tyr Lys Val Met Val Asn Trp Phe Phe Thr
   65                  70                  75                  80
   Ile Lys Arg Pro Val Ile Glu Ala Ser Glu Glu Leu Thr Ala Cys Asp
                   85                  90                  95
   Gln Cys Ile Leu Ala Val His Pro His Gly Val Pro Ser Leu Asp His
               100                 105                 110
   Leu Leu Thr Val Ile Ala Tyr Asp Pro Asp Leu Glu Arg Val Leu Pro
           115                 120                 125
   Gln Leu Arg Arg Ser Ala Leu Ser Ala Gly Val Leu Phe Lys Ile Pro
       130                 135                 140
   Ile Leu Arg Glu Val Leu Leu Trp Thr Gly Cys Val Asp Ala Gly Gly
   145                 150                 155                 160
   Lys Thr Val Asp Ser Cys Leu Lys Ala Gly Leu Ser Leu Ser Val Val
                   165                 170                 175
   Pro Gly Gly Glu Arg Glu Gln Leu Leu Ala Gln Arg Gly Asn Lys Glu
               180                 185                 190
   Ile Leu Val Leu Lys His Arg Lys Gly Phe Val Lys Tyr Ala Leu Arg
           195                 200                 205
   His Gly Ile Pro Leu Val Pro Val Tyr Cys Phe Gly Glu Asn Gln Leu
       210                 215                 220
   Phe Trp Gln Ser Ser Phe Leu Phe Lys Val Arg Ser Trp Leu Arg Arg
   225                 230                 235                 240
   Thr Leu Gly Val Ala Leu Val Leu Pro Tyr Gly Gly Cys Cys Asn Leu
                   245                 250                 255
   Pro Gly Val Pro Phe Ser Glu Pro Val Gln Leu Val Val Gly Ala Pro
               260                 265                 270
   Leu Lys Leu Pro Lys Ile Glu Glu Pro Ser Gly Val Glu Ile Ala Lys
           275                 280                 285
   Trp His Ala Arg Tyr Met Glu Cys Leu Glu Ala Leu Phe Lys Arg His
       290                 295                 300
   Arg Val Glu Ala Gly Tyr Pro Glu Leu Glu Leu Glu Phe Ile
   305                 310                 315
<210> 32
   <211> 448
   <212> DNA
   <213> Cryptocodinium cohnii
<220>
   <221> CDS
   <222> (1)..(426)
   <223> DAGAT
<400> 32
   atc aag atg gtg ccg ttt ttg aag aac gtg ctg ggg ctc ttt ggg ctg      48
   Ile Lys Met Val Pro Phe Leu Lys Asn Val Leu Gly Leu Phe Gly Leu
   1               5                   10                  15
   atc gac gcg agc aag cag gtg ttg gtc aag cga ttg aag cgc cca ggt      96
   Ile Asp Ala Ser Lys Gln Val Leu Val Lys Arg Leu Lys Arg Pro Gly
               20                  25                  30
   ggt tcc ctg gtg att tac atc gga ggg atg gtg gag ctc ttc atg tcc     144
   Gly Ser Leu Val Ile Tyr Ile Gly Gly Met Val Glu Leu Phe Met Ser
           35                  40                  45
   agc ccc aag cag gaa gtc gtc ttc ttg aag aag agg aag ggt ttt atc     192
   Ser Pro Lys Gln Glu Val Val Phe Leu Lys Lys Arg Lys Gly Phe Ile
       50                  55                  60
   cga ctc gct ctg agc aca ggt gcc gat gtc gtg ccg atc tac ttg ttc     240
   Arg Leu Ala Leu Ser Thr Gly Ala Asp Val Val Pro Ile Tyr Leu Phe
   65                  70                  75                  80
   ggc aac acc acc gtg ctc tca gtg ctg acc gct ggc cct ctg gcc tct     288
   Gly Asn Thr Thr Val Leu Ser Val Leu Thr Ala Gly Pro Leu Ala Ser
                   85                  90                  95
   ctg agc cgt gcc gcc ggg gtg tca gtg acc att ttt tgg gga cgc ttc      336
   Leu Ser Arg Ala Ala Gly Val Ser Val Thr Ile Phe Trp Gly Arg Phe
               100                 105                 110
   ggc ttg ccg atg ccc tac ccc gtc aag ctc acc tat gcc cgt ggc cgt      384
   Gly Leu Pro Met Pro Tyr Pro Val Lys Leu Thr Tyr Ala Arg Gly Arg
           115                 120                 125
   ccc atc ggt ctc cct cat atc gaa atc cta cag atg aga cat              426
   Pro Ile Gly Leu Pro His Ile Glu Ile Leu Gln Met Arg His
       130                 135                 140
   tgaccgttgg catgacgtgt ac                                             448
<210> 33
   <211> 142
   <212> PRT
   <213> Cryptocodinium cohnii
<400> 33
   Ile Lys Met Val Pro Phe Leu Lys Asn Val Leu Gly Leu Phe Gly Leu
   1               5                   10                  15
   Ile Asp Ala Ser Lys Gln Val Leu Val Lys Arg Leu Lys Arg Pro Gly
               20                  25                  30
   Gly Ser Leu Val Ile Tyr Ile Gly Gly Met Val Glu Leu Phe Met Ser
           35                  40                  45
   Ser Pro Lys Gln Glu Val Val Phe Leu Lys Lys Arg Lys Gly Phe Ile
       50                  55                  60
   Arg Leu Ala Leu Ser Thr Gly Ala Asp Val Val Pro Ile Tyr Leu Phe
   65                  70                  75                  80
   Gly Asn Thr Thr Val Leu Ser Val Leu Thr Ala Gly Pro Leu Ala Ser
                   85                  90                  95
   Leu Ser Arg Ala Ala Gly Val Ser Val Thr Ile Phe Trp Gly Arg Phe
               100                 105                 110
   Gly Leu Pro Met Pro Tyr Pro Val Lys Leu Thr Tyr Ala Arg Gly Arg
           115                 120                 125
   Pro Ile Gly Leu Pro His Ile Glu Ile Leu Gln Met Arg His
       130                 135                 140
<210> 34
   <211> 1757
   <212> DNA
   <213> Physcomitrella patens
<220>
   <221> CDS
   <222> (76)..(1578)
   <223> LCAT
<400> 34
   ggcgcgccag aggacgagac aagggggact tgtgagaatc ttcgagcttc aacctgtcaa      60
   gcttcggtct ccacc atg tgt tca att tct tgt gga tcc act ccg cag caa     111
                    Met Cys Ser Ile Ser Cys Gly Ser Thr Pro Gln Gln
                    1               5                   10
   ctc tgt cat tac agg aag agc ggg gag ctg att aca aga aag agt cgc      159
   Leu Cys His Tyr Arg Lys Ser Gly Glu Leu Ile Thr Arg Lys Ser Arg
           15                  20                  25
   gca gct att cgg tgg tgg agg tat ggc caa caa tgc aag gtg ctg ttg      207
   Ala Ala Ile Arg Trp Trp Arg Tyr Gly Gln Gln Cys Lys Val Leu Leu
       30                  35                  40
   ccg ttg gat ttg att cga tca tcg tct caa ttc ttc atc gta gtt ctc      255
   Pro Leu Asp Leu Ile Arg Ser Ser Ser Gln Phe Phe Ile Val Val Leu
   45                  50                  55                  60
   act ctg acg ctc ttc ctg ttc acc acg tgt gga gct gtg cat act gcg      303
   Thr Leu Thr Leu Phe Leu Phe Thr Thr Cys Gly Ala Val His Thr Ala
                   65                  70                  75
   gca caa gac aga tca ttc gca aca ttg agc caa aga tca aga gcg tct      351
   Ala Gln Asp Arg Ser Phe Ala Thr Leu Ser Gln Arg Ser Arg Ala Ser
               80                  85                  90
   ctc ttc agt gtg gga cgg gca caa gca agg aac aaa cac cat ttg gcg      399
   Leu Phe Ser Val Gly Arg Ala Gln Ala Arg Asn Lys His His Leu Ala
           95                  100                 105
   ccg gtg gtc ata gtt cca ggc acc ggc ggg aat caa cta gag gcc agg      447
   Pro Val Val Ile Val Pro Gly Thr Gly Gly Asn Gln Leu Glu Ala Arg
       110                 115                 120
   ttg aca gct gat tac gag gct aac aag cca tgg tgc tac agc ttc aga      495
   Leu Thr Ala Asp Tyr Glu Ala Asn Lys Pro Trp Cys Tyr Ser Phe Arg
   125                 130                 135                 140
   aaa gat tac ttc agg ttg tgg ctg gat gtg aaa aca ctg ttt cca cct      543
   Lys Asp Tyr Phe Arg Leu Trp Leu Asp Val Lys Thr Leu Phe Pro Pro
                   145                 150                 155
   ttc acg acg tgt ttc gcc gac cgc ctg agc ttg gac tac aac ccg cag      591
   Phe Thr Thr Cys Phe Ala Asp Arg Leu Ser Leu Asp Tyr Asn Pro Gln
               160                 165                 170
   tcc gat gcc tat agc aac atc aag ggc gtg aag acg cgg gta ccg ttt      639
   Ser Asp Ala Tyr Ser Asn Ile Lys Gly Val Lys Thr Arg Val Pro Phe
           175                 180                 185
   ttt ggt act acc gaa gga atg gag tac ctg gat ccc tca ctc aaa ttc      687
   Phe Gly Thr Thr Glu Gly Met Glu Tyr Leu Asp Pro Ser Leu Lys Phe
       190                 195                 200
   ttg aca ggc tac atg ata cac ttg gtg aac gca tta aaa gct cat ggt      735
   Leu Thr Gly Tyr Met Ile His Leu Val Asn Ala Leu Lys Ala His Gly
   205                 210                 215                 220
   tac gag aac gga aag tca tta tac gga gct cca tac gac ttt cgg ttc      783
   Tyr Glu Asn Gly Lys Ser Leu Tyr Gly Ala Pro Tyr Asp Phe Arg Phe
                   225                 230                 235
   gca ccg ggg cca cat gca tcc aac gta gct cta gag tac ctg aaa gac      831
   Ala Pro Gly Pro His Ala Ser Asn Val Ala Leu Glu Tyr Leu Lys Asp
               240                 245                 250
   ctg aaa gat ctc ata gaa acc gcg tac tca gta aat gcc aac gag ccg      879
   Leu Lys Asp Leu Ile Glu Thr Ala Tyr Ser Val Asn Ala Asn Glu Pro
           255                 260                 265
   gtg gtc atc ctc gct cac agc atg ggc ggg ttg tgg act ctc ttc ttc      927
   Val Val Ile Leu Ala His Ser Met Gly Gly Leu Trp Thr Leu Phe Phe
       270                 275                 280
   ctg aac cag caa tcc atg gag tgg agg aac aaa tac gtt tcc cgc ttt      975
   Leu Asn Gln Gln Ser Met Glu Trp Arg Asn Lys Tyr Val Ser Arg Phe
   285                 290                 295                 300
   gtg tct gta gct acc ccg tgg gga ggg gcg gtc gaa cag atg atg acc     1023
   Val Ser Val Ala Thr Pro Trp Gly Gly Ala Val Glu Gln Met Met Thr
                   305                 310                 315
   ttc gca tcc ggc aat ccg gag gga gtt ccc ttt gtg aac tcc ctg gtc     1071
   Phe Ala Ser Gly Asn Pro Glu Gly Val Pro Phe Val Asn Ser Leu Val
               320                 325                 330
   gtg cgc gaa gag cag cgg cgc tca gag tct aac ttg tgg ctg ctg cca     1119
   Val Arg Glu Glu Gln Arg Arg Ser Glu Ser Asn Leu Trp Leu Leu Pro
           335                 340                 345
   gtg cgg cgc tgc ttc aga gac cga cca ttg gta att acc tcg tcg cgc     1167
   Val Arg Arg Cys Phe Arg Asp Arg Pro Leu Val Ile Thr Ser Ser Arg
       350                 355                 360
   aac tac aca gct ggg gac atg gaa cag ttt ctg tgc gac atc ggt ttc     1215
   Asn Tyr Thr Ala Gly Asp Met Glu Gln Phe Leu Cys Asp Ile Gly Phe
   365                 370                 375                 380
   cct gaa ggg gtc gcg cca tac aaa tcc cgg ata ccg cac cta acg gac     1263
   Pro Glu Gly Val Ala Pro Tyr Lys Ser Arg Ile Pro His Leu Thr Asp
                   385                 390                 395
   att cta caa cct cct caa gtc ccc gtc acc cta att cac ggc tat ggc     1311
   Ile Leu Gln Pro Pro Gln Val Pro Val Thr Leu Ile His Gly Tyr Gly
               400                 405                 410
   gtg ccg acg gcg gag aca cta agc tac gag aag aag gga ttc gac aac     1359
   Val Pro Thr Ala Glu Thr Leu Ser Tyr Glu Lys Lys Gly Phe Asp Asn
           415                 420                 425
   cat ccc gaa atc aca gaa ggt gat ggc gac ggg acg gtg aat gtg tgc     1407
   His Pro Glu Ile Thr Glu Gly Asp Gly Asp Gly Thr Val Asn Val Cys
       430                 435                 440
   agc ttg acc gcg gtg gtt gag gaa tgg gag cga gtc gca ggt cag gag     1455
   Ser Leu Thr Ala Val Val Glu Glu Trp Glu Arg Val Ala Gly Gln Glu
   445                 450                 455                 460
   ttg gaa atg att gcg ctg cat ggc aaa caa cat atg caa atc ttg cac     1503
   Leu Glu Met Ile Ala Leu His Gly Lys Gln His Met Gln Ile Leu His
                   465                 470                 475
   gac gac cat tct gtg caa gtg atc gtg gac gcc att ctc aat gtt acc     1551
   Asp Asp His Ser Val Gln Val Ile Val Asp Ala Ile Leu Asn Val Thr
               480                 485                 490
   cca cag gaa cag ctt atg ttc cac taa gccctaatcg taaccctaaa           1598
   Pro Gln Glu Gln Leu Met Phe His
           495                 500
   cctagctcca atcctcacag gatcaggcca cattctcctt gaaaaacagc ataaggtcga   1658
   ttctccgcag cctctcttcc attccacctc cccctttgta tctctctcca ttcaattgta   1718
   caattgtttt tttattcaaa aaaaaaaaaa aaaaaaaaa                          1757
<210> 35
   <211> 500
   <212> PRT
   <213> Physcomitrella patens
<400> 35
   Met Cys Ser Ile Ser Cys Gly Ser Thr Pro Gln Gln Leu Cys His Tyr
   1               5                   10                  15
   Arg Lys Ser Gly Glu Leu Ile Thr Arg Lys Ser Arg Ala Ala Ile Arg
               20                  25                  30
   Trp Trp Arg Tyr Gly Gln Gln Cys Lys Val Leu Leu Pro Leu Asp Leu
           35                  40                  45
   Ile Arg Ser Ser Ser Gln Phe Phe Ile Val Val Leu Thr Leu Thr Leu
       50                  55                  60
   Phe Leu Phe Thr Thr Cys Gly Ala Val His Thr Ala Ala Gln Asp Arg
   65                  70                  75                  80
   Ser Phe Ala Thr Leu Ser Gln Arg Ser Arg Ala Ser Leu Phe Ser Val
                   85                  90                  95
   Gly Arg Ala Gln Ala Arg Asn Lys His His Leu Ala Pro Val Val Ile
               100                 105                 110
   Val Pro Gly Thr Gly Gly Asn Gln Leu Glu Ala Arg Leu Thr Ala Asp
           115                 120                 125
   Tyr Glu Ala Asn Lys Pro Trp Cys Tyr Ser Phe Arg Lys Asp Tyr Phe
       130                 135                 140
   Arg Leu Trp Leu Asp Val Lys Thr Leu Phe Pro Pro Phe Thr Thr Cys
   145                 150                 155                 160
   Phe Ala Asp Arg Leu Ser Leu Asp Tyr Asn Pro Gln Ser Asp Ala Tyr
                   165                 170                 175
   Ser Asn Ile Lys Gly Val Lys Thr Arg Val Pro Phe Phe Gly Thr Thr
               180                 185                 190
   Glu Gly Met Glu Tyr Leu Asp Pro Ser Leu Lys Phe Leu Thr Gly Tyr
           195                 200                 205
   Met Ile His Leu Val Asn Ala Leu Lys Ala His Gly Tyr Glu Asn Gly
       210                 215                 220
   Lys Ser Leu Tyr Gly Ala Pro Tyr Asp Phe Arg Phe Ala Pro Gly Pro
   225                 230                 235                 240
   His Ala Ser Asn Val Ala Leu Glu Tyr Leu Lys Asp Leu Lys Asp Leu
                   245                 250                 255
   Ile Glu Thr Ala Tyr Ser Val Asn Ala Asn Glu Pro Val Val Ile Leu
               260                 265                 270
   Ala His Ser Met Gly Gly Leu Trp Thr Leu Phe Phe Leu Asn Gln Gln
           275                 280                 285
   Ser Met Glu Trp Arg Asn Lys Tyr Val Ser Arg Phe Val Ser Val Ala
       290                 295                 300
   Thr Pro Trp Gly Gly Ala Val Glu Gln Met Met Thr Phe Ala Ser Gly
   305                 310                 315                 320
   Asn Pro Glu Gly Val Pro Phe Val Asn Ser Leu Val Val Arg Glu Glu
                   325                 330                 335
   Gln Arg Arg Ser Glu Ser Asn Leu Trp Leu Leu Pro Val Arg Arg Cys
               340                 345                 350
   Phe Arg Asp Arg Pro Leu Val Ile Thr Ser Ser Arg Asn Tyr Thr Ala
           355                 360                 365
   Gly Asp Met Glu Gln Phe Leu Cys Asp Ile Gly Phe Pro Glu Gly Val
       370                 375                 380
   Ala Pro Tyr Lys Ser Arg Ile Pro His Leu Thr Asp Ile Leu Gln Pro
   385                 390                 395                 400
   Pro Gln Val Pro Val Thr Leu Ile His Gly Tyr Gly Val Pro Thr Ala
                   405                 410                 415
   Glu Thr Leu Ser Tyr Glu Lys Lys Gly Phe Asp Asn His Pro Glu Ile
               420                 425                 430
   Thr Glu Gly Asp Gly Asp Gly Thr Val Asn Val Cys Ser Leu Thr Ala
   435 440 445
   Val Val Glu Glu Trp Glu Arg Val Ala Gly Gln Glu Leu Glu Met Ile
   450 455 460
   Ala Leu His Gly Lys Gln His Met Gln Ile Leu His Asp Asp His Ser
   465 470 475 480
   Val Gln Val Ile Val Asp Ala Ile Leu Asn Val Thr Pro Gln Glu Gln
   485 490 495
   Leu Met Phe His
   500
<210> 36
   <211> 1893
   <212> DNA
   <213> Fusarium graminaeum
<220>
   <221> CDS
   <222> (1)..(1893)
   <223> LCAT
<400> 36
   atg gga aag tcc act tta cga cgc cgg aat ggc caa gat gcg aca aat 48
   Met Gly Lys Ser Thr Leu Arg Arg Arg Asn Gly Gln Asp Ala Thr Asn
   1 5 10 15
   aac gat agc gcc gac gct gac gac act ccg aga gaa gaa agc cca acg 96
   Asn Asp Ser Ala Asp Ala Asp Asp Thr Pro Arg Glu Glu Ser Pro Thr
   20 25 30
   gct gag ccg acc aca cac gtt cga gtt gtt caa cac gcc gtg ccc aga 144
   Ala Glu Pro Thr Thr His Val Arg Val Val Gln His Ala Val Pro Arg
   35 40 45
   acc cga aaa cgc cgc aac acc ttc gtc ttc ttc ctt ggt agt ttg ttt 192
   Thr Arg Lys Arg Arg Asn Thr Phe Val Phe Phe Leu Gly Ser Leu Phe
   50 55 60
   gga att ata gcc gcc gga ttt ttc gct tcc agc aat gat ctt att gac 240
   Gly Ile Ile Ala Ala Gly Phe Phe Ala Ser Ser Asn Asp Leu Ile Asp
   65 70 75 80
   ctc ccc gag ttt acc gac ttg tcg atg gat aac ttg atg gat gtt ctg 288
   Leu Pro Glu Phe Thr Asp Leu Ser Met Asp Asn Leu Met Asp Val Leu
   85 90 95
   cct gcc ggc ttg ata aag gac atg cgc gac ctt gtt cag ggc gag cgg 336
   Pro Ala Gly Leu Ile Lys Asp Met Arg Asp Leu Val Gln Gly Glu Arg
   100 105 110
   gac att gcc gaa tcg tac gag cca ttc tct gtt ggc gaa aag gct cga 384
   Asp Ile Ala Glu Ser Tyr Glu Pro Phe Ser Val Gly Glu Lys Ala Arg
   115 120 125
   tcc gag ggt cta gga gtt cac cat cct atg atc atg ata cct ggt gtt 432
   Ser Glu Gly Leu Gly Val His His Pro Met Ile Met Ile Pro Gly Val
   130 135 140
   atc tca act gga ctc gaa tcg tgg ggt acg gct aat atc tcg aaa ccc 480
   Ile Ser Thr Gly Leu Glu Ser Trp Gly Thr Ala Asn Ile Ser Lys Pro
   145 150 155 160
   tac ttt aga aaa cga ctt tgg ggt agt tgg aca atg atg aga gct ctg      528
   Tyr Phe Arg Lys Arg Leu Trp Gly Ser Trp Thr Met Met Arg Ala Leu
                   165                 170                 175
   gtt atg gac aag gag gtt tgg aag aag cac gtc atg ctc gac aag agg      576
   Val Met Asp Lys Glu Val Trp Lys Lys His Val Met Leu Asp Lys Arg
               180                 185                 190
   acg ggc ctt gac ccg cct gac gta aag ttg agg gct gcc caa ggg ttc      624
   Thr Gly Leu Asp Pro Pro Asp Val Lys Leu Arg Ala Ala Gln Gly Phe
           195                 200                 205
   gat gcg acc gat ttc ttc atc acg gga tat tgg atc tgg agc aaa atc      672
   Asp Ala Thr Asp Phe Phe Ile Thr Gly Tyr Trp Ile Trp Ser Lys Ile
       210                 215                 220
   ttt gag aat ctc gca tcc atc ggc tac gac cca acg aac tcg ttc acg      720
   Phe Glu Asn Leu Ala Ser Ile Gly Tyr Asp Pro Thr Asn Ser Phe Thr
   225                 230                 235                 240
   gct gct tac gat tgg cgc ttg tcg tat ccc aac ctt gag gta cgg gac      768
   Ala Ala Tyr Asp Trp Arg Leu Ser Tyr Pro Asn Leu Glu Val Arg Asp
                   245                 250                 255
   cgc tac ttc act cgg cta aag tcg cat atc gaa atc gcg gtg gcc act      816
   Arg Tyr Phe Thr Arg Leu Lys Ser His Ile Glu Ile Ala Val Ala Thr
               260                 265                 270
   gag gac aaa aaa gtc gtc ctc gca tca cac agt atg ggg agc caa gtc      864
   Glu Asp Lys Lys Val Val Leu Ala Ser His Ser Met Gly Ser Gln Val
           275                 280                 285
   ctt tac tat ttt ctc cac tgg gtg cag tca gaa aga ggc gga cgc ggt      912
   Leu Tyr Tyr Phe Leu His Trp Val Gln Ser Glu Arg Gly Gly Arg Gly
       290                 295                 300
   ggg ccg gat tgg gtt gag cgt cac att gac gcc tgg atc aac atc agc      960
   Gly Pro Asp Trp Val Glu Arg His Ile Asp Ala Trp Ile Asn Ile Ser
   305                 310                 315                 320
   gga tgc atg ctt gga gca gtc aag gat ttg acc gct gtg ctc tcc ggc     1008
   Gly Cys Met Leu Gly Ala Val Lys Asp Leu Thr Ala Val Leu Ser Gly
                   325                 330                 335
   gag atg cgc gac aca gct caa ctg aac ccg ttc gct att tac ggc ctg     1056
   Glu Met Arg Asp Thr Ala Gln Leu Asn Pro Phe Ala Ile Tyr Gly Leu
               340                 345                 350
   gaa aag ttc ttg agt aaa gag gag aga gcc gag atc ttt cgc ggc atg     1104
   Glu Lys Phe Leu Ser Lys Glu Glu Arg Ala Glu Ile Phe Arg Gly Met
           355                 360                 365
   ccc ggg ata tcc tcc atg ttg ccc atc ggc ggc aac tct gta tgg ggt     1152
   Pro Gly Ile Ser Ser Met Leu Pro Ile Gly Gly Asn Ser Val Trp Gly
       370                 375                 380
   aac ttg acc tgg gct cca gac gac ttg cca ggc cag aac cgt tca tat     1200
   Asn Leu Thr Trp Ala Pro Asp Asp Leu Pro Gly Gln Asn Arg Ser Tyr
   385                 390                 395                 400
   gga tct ctc ttg aac ttt agg gtc ggt tcg aac tgg aca act cct gat     1248
   Gly Ser Leu Leu Asn Phe Arg Val Gly Ser Asn Trp Thr Thr Pro Asp
                   405                 410                 415
   cgt aac ttt acc gtc gag gaa ggt gtg tcc tat ttg ctt aac aca acg     1296
   Arg Asn Phe Thr Val Glu Glu Gly Val Ser Tyr Leu Leu Asn Thr Thr
               420                 425                 430
   gag gac tgg tat caa gac cag atc aag ggc agt tat tct cgg ggc att     1344
   Glu Asp Trp Tyr Gln Asp Gln Ile Lys Gly Ser Tyr Ser Arg Gly Ile
           435                 440                 445
   gct cat tcc ata gat gag gtc gaa gcc aat gag aat gac ccc aag aag      1392
   Ala His Ser Ile Asp Glu Val Glu Ala Asn Glu Asn Asp Pro Lys Lys
       450                 455                 460
   tgg atc aat cct ctc gag acg cga ttg cca ctt gct cct agc ctc aag      1440
   Trp Ile Asn Pro Leu Glu Thr Arg Leu Pro Leu Ala Pro Ser Leu Lys
   465                 470                 475                 480
   atc tac tgc ttt tat ggt gtt gga aaa ccg acc gag cga ggg tac ttc      1488
   Ile Tyr Cys Phe Tyr Gly Val Gly Lys Pro Thr Glu Arg Gly Tyr Phe
                   485                 490                 495
   tat aag cca ccg gat cag cca tca ttg acc aac ctc aac atc aca ata      1536
   Tyr Lys Pro Pro Asp Gln Pro Ser Leu Thr Asn Leu Asn Ile Thr Ile
               500                 505                 510
   gat acg ggc tat acc gaa gga gac gtg gat cat ggc gtt gtc atg ggc      1584
   Asp Thr Gly Tyr Thr Glu Gly Asp Val Asp His Gly Val Val Met Gly
           515                 520                 525
   gag gga gat ggt acc gtg aac ctc ctc agt aca ggc tac atg tgt aat      1632
   Glu Gly Asp Gly Thr Val Asn Leu Leu Ser Thr Gly Tyr Met Cys Asn
       530                 535                 540
   cat ggc tgg aat atg aaa cgc tac aac cca gca ggc gtc aag gtt aca      1680
   His Gly Trp Asn Met Lys Arg Tyr Asn Pro Ala Gly Val Lys Val Thr
   545                 550                 555                 560
   gtt gtc gag atg cct cac gag ccg gac cgc ttc aat cct cga gga ggg      1728
   Val Val Glu Met Pro His Glu Pro Asp Arg Phe Asn Pro Arg Gly Gly
                   565                 570                 575
   cct cgc acg gcc gac cac gtt gac atc ttg ggg cga tac aac ctg aac      1776
   Pro Arg Thr Ala Asp His Val Asp Ile Leu Gly Arg Tyr Asn Leu Asn
               580                 585                 590
   gag ttg ctg tta cga gta gcg agc ggc aaa ggt gac acg att acg aac      1824
   Glu Leu Leu Leu Arg Val Ala Ser Gly Lys Gly Asp Thr Ile Thr Asn
           595                 600                 605
   tat gtt gtg agc aac atc aaa gaa tat gca tcc agg gtt aag att tac      1872
   Tyr Val Val Ser Asn Ile Lys Glu Tyr Ala Ser Arg Val Lys Ile Tyr
       610                 615                 620
   gat gat gag gag act tca tag                                          1893
   Asp Asp Glu Glu Thr Ser
   625                 630
<210> 37
   <211> 630
   <212> PRT
   <213> Fusarium graminaeum
<400> 37
   Met Gly Lys Ser Thr Leu Arg Arg Arg Asn Gly Gln Asp Ala Thr Asn
   1               5                   10                  15
   Asn Asp Ser Ala Asp Ala Asp Asp Thr Pro Arg Glu Glu Ser Pro Thr
               20                  25                  30
   Ala Glu Pro Thr Thr His Val Arg Val Val Gln His Ala Val Pro Arg
           35                  40                  45
   Thr Arg Lys Arg Arg Asn Thr Phe Val Phe Phe Leu Gly Ser Leu Phe
       50                  55                  60
   Gly Ile Ile Ala Ala Gly Phe Phe Ala Ser Ser Asn Asp Leu Ile Asp
   65                  70                  75                  80
   Leu Pro Glu Phe Thr Asp Leu Ser Met Asp Asn Leu Met Asp Val Leu
                   85                  90                  95
   Pro Ala Gly Leu Ile Lys Asp Met Arg Asp Leu Val Gln Gly Glu Arg
               100                 105                 110
   Asp Ile Ala Glu Ser Tyr Glu Pro Phe Ser Val Gly Glu Lys Ala Arg
           115                 120                 125
   Ser Glu Gly Leu Gly Val His His Pro Met Ile Met Ile Pro Gly Val
       130                 135                 140
   Ile Ser Thr Gly Leu Glu Ser Trp Gly Thr Ala Asn Ile Ser Lys Pro
   145                 150                 155                 160
   Tyr Phe Arg Lys Arg Leu Trp Gly Ser Trp Thr Met Met Arg Ala Leu
                   165                 170                 175
   Val Met Asp Lys Glu Val Trp Lys Lys His Val Met Leu Asp Lys Arg
               180                 185                 190
   Thr Gly Leu Asp Pro Pro Asp Val Lys Leu Arg Ala Ala Gln Gly Phe
           195                 200                 205
   Asp Ala Thr Asp Phe Phe Ile Thr Gly Tyr Trp Ile Trp Ser Lys Ile
       210                 215                 220
   Phe Glu Asn Leu Ala Ser Ile Gly Tyr Asp Pro Thr Asn Ser Phe Thr
   225                 230                 235                 240
   Ala Ala Tyr Asp Trp Arg Leu Ser Tyr Pro Asn Leu Glu Val Arg Asp
                   245                 250                 255
   Arg Tyr Phe Thr Arg Leu Lys Ser His Ile Glu Ile Ala Val Ala Thr
               260                 265                 270
   Glu Asp Lys Lys Val Val Leu Ala Ser His Ser Met Gly Ser Gln Val
           275                 280                 285
   Leu Tyr Tyr Phe Leu His Trp Val Gln Ser Glu Arg Gly Gly Arg Gly
       290                 295                 300
   Gly Pro Asp Trp Val Glu Arg His Ile Asp Ala Trp Ile Asn Ile Ser
   305                 310                 315                 320
   Gly Cys Met Leu Gly Ala Val Lys Asp Leu Thr Ala Val Leu Ser Gly
                   325                 330                 335
   Glu Met Arg Asp Thr Ala Gln Leu Asn Pro Phe Ala Ile Tyr Gly Leu
               340                 345                 350
   Glu Lys Phe Leu Ser Lys Glu Glu Arg Ala Glu Ile Phe Arg Gly Met
           355                 360                 365
   Pro Gly Ile Ser Ser Met Leu Pro Ile Gly Gly Asn Ser Val Trp Gly
       370                 375                 380
   Asn Leu Thr Trp Ala Pro Asp Asp Leu Pro Gly Gln Asn Arg Ser Tyr
   385                 390                 395                 400
   Gly Ser Leu Leu Asn Phe Arg Val Gly Ser Asn Trp Thr Thr Pro Asp
                   405                 410                 415
   Arg Asn Phe Thr Val Glu Glu Gly Val Ser Tyr Leu Leu Asn Thr Thr
               420                 425                 430
   Glu Asp Trp Tyr Gln Asp Gln Ile Lys Gly Ser Tyr Ser Arg Gly Ile
           435                 440                 445
   Ala His Ser Ile Asp Glu Val Glu Ala Asn Glu Asn Asp Pro Lys Lys
       450                 455                 460
   Trp Ile Asn Pro Leu Glu Thr Arg Leu Pro Leu Ala Pro Ser Leu Lys
   465                 470                 475                 480
   Ile Tyr Cys Phe Tyr Gly Val Gly Lys Pro Thr Glu Arg Gly Tyr Phe
                   485                 490                 495
   Tyr Lys Pro Pro Asp Gln Pro Ser Leu Thr Asn Leu Asn Ile Thr Ile
                       500                     505                     510
   Asp Thr Gly Tyr Thr Glu Gly Asp Val Asp His Gly Val Val Met Gly
               515                     520                     525
   Glu Gly Asp Gly Thr Val Asn Leu Leu Ser Thr Gly Tyr Met Cys Asn
       530                     535                     540
   His Gly Trp Asn Met Lys Arg Tyr Asn Pro Ala Gly Val Lys Val Thr
   545                     550                     555                     560
   Val Val Glu Met Pro His Glu Pro Asp Arg Phe Asn Pro Arg Gly Gly
                   565                     570                     575
   Pro Arg Thr Ala Asp His Val Asp Ile Leu Gly Arg Tyr Asn Leu Asn
               580                     585                     590
   Glu Leu Leu Leu Arg Val Ala Ser Gly Lys Gly Asp Thr Ile Thr Asn
           595                     600                     605
   Tyr Val Val Ser Asn Ile Lys Glu Tyr Ala Ser Arg Val Lys Ile Tyr
       610                     615                     620
   Asp Asp Glu Glu Thr Ser
   625                 630
<210> 38
   <211> 849
   <212> DNA
   <213> Caenorhabditis elegans
<220>
   <221> CDS
   <222> (1)..(849)
   <223> Acyl-CoA:Lysophospholipid-Acyltransferase
<400> 38
   atg gag aac ttc tgg tcg atc gtc gtg ttt ttt cta ctc tca att ctc      48
   Met Glu Asn Phe Trp Ser Ile Val Val Phe Phe Leu Leu Ser Ile Leu
   1               5                   10                  15
   ttc att tta tat aac ata tcg aca gta tgc cac tac tat atg cgg att      96
   Phe Ile Leu Tyr Asn Ile Ser Thr Val Cys His Tyr Tyr Met Arg Ile
               20                  25                  30
   tcg ttt tat tac ttc aca att tta ttg cat gga atg gaa gtt tgt gtt     144
   Ser Phe Tyr Tyr Phe Thr Ile Leu Leu His Gly Met Glu Val Cys Val
           35                  40                  45
   aca atg atc cct tct tgg cta aat ggg aag ggt gct gat tac gtg ttt     192
   Thr Met Ile Pro Ser Trp Leu Asn Gly Lys Gly Ala Asp Tyr Val Phe
       50                  55                  60
   cac tcg ttt ttc tat tgg tgt aaa tgg act ggt gtt cat aca aca gtc     240
   His Ser Phe Phe Tyr Trp Cys Lys Trp Thr Gly Val His Thr Thr Val
   65                  70                  75                  80
   tat gga tat gaa aaa aca caa gtt gaa ggt ccg gct gta gtt att tgt     288
   Tyr Gly Tyr Glu Lys Thr Gln Val Glu Gly Pro Ala Val Val Ile Cys
                   85                  90                  95
   aat cat cag agt tct ctc gac att cta tcg atg gca tca atc tgg ccg     336
   Asn His Gln Ser Ser Leu Asp Ile Leu Ser Met Ala Ser Ile Trp Pro
               100                 105                 110
   aag aat tgt gtt gta atg atg aaa cga att ctt gcc tat gtt cca ttc     384
   Lys Asn Cys Val Val Met Met Lys Arg Ile Leu Ala Tyr Val Pro Phe
           115                 120                 125
   ttc aat ctc gga gcc tac ttt tcc aac aca atc ttc atc gat cga tat      432
   Phe Asn Leu Gly Ala Tyr Phe Ser Asn Thr Ile Phe Ile Asp Arg Tyr
       130                 135                 140
   aac cgt gaa cgt gcg atg gct tca gtt gat tat tgt gca tct gaa atg      480
   Asn Arg Glu Arg Ala Met Ala Ser Val Asp Tyr Cys Ala Ser Glu Met
   145                 150                 155                 160
   aag aac aga aat ctt aaa ctt tgg gta ttt ccg gaa gga aca aga aat      528
   Lys Asn Arg Asn Leu Lys Leu Trp Val Phe Pro Glu Gly Thr Arg Asn
                   165                 170                 175
   cgt gaa gga ggg ttc att cca ttc aag aaa gga gca ttc aat att gca      576
   Arg Glu Gly Gly Phe Ile Pro Phe Lys Lys Gly Ala Phe Asn Ile Ala
               180                 185                 190
   gtt cgt gcg cag att ccc att att cca gtt gta ttc tca gac tat cgg      624
   Val Arg Ala Gln Ile Pro Ile Ile Pro Val Val Phe Ser Asp Tyr Arg
           195                 200                 205
   gat ttc tac tca aag cca ggc cga tat ttc aag aat gat gga gaa gtt      672
   Asp Phe Tyr Ser Lys Pro Gly Arg Tyr Phe Lys Asn Asp Gly Glu Val
       210                 215                 220
   gtt att cga gtt ctg gat gcg att cca aca aaa ggg ctc act ctt gat      720
   Val Ile Arg Val Leu Asp Ala Ile Pro Thr Lys Gly Leu Thr Leu Asp
   225                 230                 235                 240
   gac gtc agc gag ttg tct gat atg tgt cgg gac gtt atg ttg gca gcc      768
   Asp Val Ser Glu Leu Ser Asp Met Cys Arg Asp Val Met Leu Ala Ala
                   245                 250                 255
   tat aag gaa gtt act cta gaa gct cag caa cga aat gcg aca cgg cgt      816
   Tyr Lys Glu Val Thr Leu Glu Ala Gln Gln Arg Asn Ala Thr Arg Arg
               260                 265                 270
   gga gaa aca aaa gac ggg aag aaa tct gag taa                          849
   Gly Glu Thr Lys Asp Gly Lys Lys Ser Glu
           275                 280
<210> 39
   <211> 282
   <212> PRT
   <213> Caenorhabditis elegans
<400> 39
   Met Glu Asn Phe Trp Ser Ile Val Val Phe Phe Leu Leu Ser Ile Leu
   1               5                   10                  15
   Phe Ile Leu Tyr Asn Ile Ser Thr Val Cys His Tyr Tyr Met Arg Ile
               20                  25                  30
   Ser Phe Tyr Tyr Phe Thr Ile Leu Leu His Gly Met Glu Val Cys Val
           35                  40                  45
   Thr Met Ile Pro Ser Trp Leu Asn Gly Lys Gly Ala Asp Tyr Val Phe
       50                  55                  60
   His Ser Phe Phe Tyr Trp Cys Lys Trp Thr Gly Val His Thr Thr Val
   65                  70                  75                  80
   Tyr Gly Tyr Glu Lys Thr Gln Val Glu Gly Pro Ala Val Val Ile Cys
                   85                  90                  95
   Asn His Gln Ser Ser Leu Asp Ile Leu Ser Met Ala Ser Ile Trp Pro
               100                 105                 110
   Lys Asn Cys Val Val Met Met Lys Arg Ile Leu Ala Tyr Val Pro Phe
           115                 120                 125
   Phe Asn Leu Gly Ala Tyr Phe Ser Asn Thr Ile Phe Ile Asp Arg Tyr
       130                 135                 140
   Asn Arg Glu Arg Ala Met Ala Ser Val Asp Tyr Cys Ala Ser Glu Met
   145                 150                 155                 160
   Lys Asn Arg Asn Leu Lys Leu Trp Val Phe Pro Glu Gly Thr Arg Asn
                   165                 170                 175
   Arg Glu Gly Gly Phe Ile Pro Phe Lys Lys Gly Ala Phe Asn Ile Ala
               180                 185                 190
   Val Arg Ala Gln Ile Pro Ile Ile Pro Val Val Phe Ser Asp Tyr Arg
           195                 200                 205
   Asp Phe Tyr Ser Lys Pro Gly Arg Tyr Phe Lys Asn Asp Gly Glu Val
       210                 215                 220
   Val Ile Arg Val Leu Asp Ala Ile Pro Thr Lys Gly Leu Thr Leu Asp
   225                 230                 235                 240
   Asp Val Ser Glu Leu Ser Asp Met Cys Arg Asp Val Met Leu Ala Ala
                   245                 250                 255
   Tyr Lys Glu Val Thr Leu Glu Ala Gln Gln Arg Asn Ala Thr Arg Arg
               260                 265                 270
   Gly Glu Thr Lys Asp Gly Lys Lys Ser Glu
           275                 280
<210> 40
   <211> 849
   <212> DNA
   <213> Caenorhabditis elegans
<220>
   <221> CDS
   <222> (1)..(849)
   <223> Acyl-CoA:Lysophospholipid-Acyltransferase
<400> 40
   atg gag aac ttc tgg tcg atc gtc gtg ttt ttt cta ctc tca att ctc       48
   Met Glu Asn Phe Trp Ser Ile Val Val Phe Phe Leu Leu Ser Ile Leu
   1               5                   10                  15
   ttc att tta tat aac ata tcg aca gta tgc cac tac tat atg cgg att       96
   Phe Ile Leu Tyr Asn Ile Ser Thr Val Cys His Tyr Tyr Met Arg Ile
               20                  25                  30
   tcg ttt tat tac ttc aca att tta ttg cat gga atg gaa gtt tgt gtt      144
   Ser Phe Tyr Tyr Phe Thr Ile Leu Leu His Gly Met Glu Val Cys Val
           35                  40                  45
   aca atg atc cct tct tgg cta aat ggg aag ggt gct gat tac gtg ttt      192
   Thr Met Ile Pro Ser Trp Leu Asn Gly Lys Gly Ala Asp Tyr Val Phe
       50                  55                  60
   cac tcg ttt ttc tat tgg tgt aaa tgg act ggt gtt cat aca aca gtc      240
   His Ser Phe Phe Tyr Trp Cys Lys Trp Thr Gly Val His Thr Thr Val
   65                  70                  75                  80
   tat gga tat gaa aaa aca caa gtt gaa ggt ccg gct gta gtt att tgt      288
   Tyr Gly Tyr Glu Lys Thr Gln Val Glu Gly Pro Ala Val Val Ile Cys
                   85                  90                  95
   aat cat cag agt tct ctc gac att cta tcg atg gca tca atc tgg ccg      336
   Asn His Gln Ser Ser Leu Asp Ile Leu Ser Met Ala Ser Ile Trp Pro
               100                 105                 110
   aag aat tgt gtt gta atg atg aaa cga att ctt gcc tat gtt cca ttc      384
   Lys Asn Cys Val Val Met Met Lys Arg Ile Leu Ala Tyr Val Pro Phe
           115                 120                 125
   ttc aat ctc gga gcc tac ttt tcc aac aca atc ttc atc gat cga tat      432
   Phe Asn Leu Gly Ala Tyr Phe Ser Asn Thr Ile Phe Ile Asp Arg Tyr
       130                 135                 140
   aac cgt gaa cgt gcg atg gct tca gtt gat tat tgt gca tct gaa atg      480
   Asn Arg Glu Arg Ala Met Ala Ser Val Asp Tyr Cys Ala Ser Glu Met
   145                 150                 155                 160
   aag aac aga aat ctt aaa ctt tgg gta tct ccg gaa gga aca aga aat      528
   Lys Asn Arg Asn Leu Lys Leu Trp Val Ser Pro Glu Gly Thr Arg Asn
                   165                 170                 175
   cgt gaa gga ggg ttc att cca ttc aag aaa gga gca ttc aat att gca      576
   Arg Glu Gly Gly Phe Ile Pro Phe Lys Lys Gly Ala Phe Asn Ile Ala
               180                 185                 190
   gtt cgt gcg cag att ccc att att cca gtt gta ttc tca gac tat cgg      624
   Val Arg Ala Gln Ile Pro Ile Ile Pro Val Val Phe Ser Asp Tyr Arg
           195                 200                 205
   gat ttc tac tca aag cca ggc cga tat ttc aag aat gat gga gaa gtt      672
   Asp Phe Tyr Ser Lys Pro Gly Arg Tyr Phe Lys Asn Asp Gly Glu Val
       210                 215                 220
   gtt att cga gtt ctg gat gcg att cca aca aaa ggg ctc act ctt gat      720
   Val Ile Arg Val Leu Asp Ala Ile Pro Thr Lys Gly Leu Thr Leu Asp
   225                 230                 235                 240
   gac gtc agc gag ttg tct gat atg tgt cgg gac gtt atg ttg gca gcc      768
   Asp Val Ser Glu Leu Ser Asp Met Cys Arg Asp Val Met Leu Ala Ala
                   245                 250                 255
   tat aag gaa gtt act cta gaa gct cag caa cga aat gcg aca cgg cgt      816
   Tyr Lys Glu Val Thr Leu Glu Ala Gln Gln Arg Asn Ala Thr Arg Arg
               260                 265                 270
   gga gaa aca aaa gac ggg aag aaa tct gag taa                          849
   Gly Glu Thr Lys Asp Gly Lys Lys Ser Glu
           275                 280
<210> 41
   <211> 282
   <212> PRT
   <213> Caenorhabditis elegans
<400> 41
   Met Glu Asn Phe Trp Ser Ile Val Val Phe Phe Leu Leu Ser Ile Leu
   1               5                   10                  15
   Phe Ile Leu Tyr Asn Ile Ser Thr Val Cys His Tyr Tyr Met Arg Ile
               20                  25                  30
   Ser Phe Tyr Tyr Phe Thr Ile Leu Leu His Gly Met Glu Val Cys Val
           35                  40                  45
   Thr Met Ile Pro Ser Trp Leu Asn Gly Lys Gly Ala Asp Tyr Val Phe
       50                  55                  60
   His Ser Phe Phe Tyr Trp Cys Lys Trp Thr Gly Val His Thr Thr Val
   65                  70                  75                  80
   Tyr Gly Tyr Glu Lys Thr Gln Val Glu Gly Pro Ala Val Val Ile Cys
                   85                  90                  95
   Asn His Gln Ser Ser Leu Asp Ile Leu Ser Met Ala Ser Ile Trp Pro
               100                 105                 110
   Lys Asn Cys Val Val Met Met Lys Arg Ile Leu Ala Tyr Val Pro Phe
           115                 120                 125
   Phe Asn Leu Gly Ala Tyr Phe Ser Asn Thr Ile Phe Ile Asp Arg Tyr
       130                 135                 140
   Asn Arg Glu Arg Ala Met Ala Ser Val Asp Tyr Cys Ala Ser Glu Met
   145                 150                 155                 160
   Lys Asn Arg Asn Leu Lys Leu Trp Val Ser Pro Glu Gly Thr Arg Asn
                   165                 170                 175
   Arg Glu Gly Gly Phe Ile Pro Phe Lys Lys Gly Ala Phe Asn Ile Ala
               180                 185                 190
   Val Arg Ala Gln Ile Pro Ile Ile Pro Val Val Phe Ser Asp Tyr Arg
           195                 200                 205
   Asp Phe Tyr Ser Lys Pro Gly Arg Tyr Phe Lys Asn Asp Gly Glu Val
       210                 215                 220
   Val Ile Arg Val Leu Asp Ala Ile Pro Thr Lys Gly Leu Thr Leu Asp
   225                 230                 235                 240
   Asp Val Ser Glu Leu Ser Asp Met Cys Arg Asp Val Met Leu Ala Ala
                   245                 250                 255
   Tyr Lys Glu Val Thr Leu Glu Ala Gln Gln Arg Asn Ala Thr Arg Arg
               260                 265                 270
   Gly Glu Thr Lys Asp Gly Lys Lys Ser Glu
           275                 280
<210> 42
   <211> 849
   <212> DNA
   <213> Caenorhabditis elegans
<220>
   <221> CDS
   <222> (1)..(849)
   <223> Acyl-CoA:Lysophospholipid-Acyltransferase
<400> 42
   atg gag aac ttc tgg tcg atc gtc gtg ttt ttt cta ctc tca att ctc      48
   Met Glu Asn Phe Trp Ser Ile Val Val Phe Phe Leu Leu Ser Ile Leu
   1               5                   10                  15
   ttc att tta tat aac ata tcg aca gta tgc cac tac tat gtg cgg att      96
   Phe Ile Leu Tyr Asn Ile Ser Thr Val Cys His Tyr Tyr Val Arg Ile
               20                  25                  30
   tcg ttt tat tac ttc aca att tta ttg cat gga atg gaa gtt tgt gtt     144
   Ser Phe Tyr Tyr Phe Thr Ile Leu Leu His Gly Met Glu Val Cys Val
           35                  40                  45
   aca atg atc cct tct tgg cta aat ggg aag ggt gct gat tac gtg ttt     192
   Thr Met Ile Pro Ser Trp Leu Asn Gly Lys Gly Ala Asp Tyr Val Phe
       50                  55                  60
   cac tcg ttt ttc tat tgg tgt aaa tgg act ggt gtt cat aca aca gtc     240
   His Ser Phe Phe Tyr Trp Cys Lys Trp Thr Gly Val His Thr Thr Val
   65                  70                  75                  80
   tat gga tat gaa aaa aca caa gtt gaa ggt ccg gct gta gtt att tgt      288
   Tyr Gly Tyr Glu Lys Thr Gln Val Glu Gly Pro Ala Val Val Ile Cys
                   85                  90                  95
   aat cat cag agt tct ctc gac att cta tcg atg gca tca atc tgg ccg      336
   Asn His Gln Ser Ser Leu Asp Ile Leu Ser Met Ala Ser Ile Trp Pro
               100                 105                 110
   aag aat tgt gtt gta atg atg aaa cga att ctt gcc tat gtt cca ttc      384
   Lys Asn Cys Val Val Met Met Lys Arg Ile Leu Ala Tyr Val Pro Phe
           115                 120                 125
   ttc aat ctc gga gcc tac ttt tcc aac aca atc ttc atc gat cga tat      432
   Phe Asn Leu Gly Ala Tyr Phe Ser Asn Thr Ile Phe Ile Asp Arg Tyr
       130                 135                 140
   aac cgt gaa cgt gcg atg gct tca gtt gat tat tgt gca tct gaa atg      480
   Asn Arg Glu Arg Ala Met Ala Ser Val Asp Tyr Cys Ala Ser Glu Met
   145                 150                 155                 160
   aag aac aga aat ctt aaa ctt tgg gta ttt ccg gaa gga aca aga aat      528
   Lys Asn Arg Asn Leu Lys Leu Trp Val Phe Pro Glu Gly Thr Arg Asn
                   165                 170                 175
   cgt gaa gga ggg ttc att cca ttc aag aaa gga gca ttc aat att gca      576
   Arg Glu Gly Gly Phe Ile Pro Phe Lys Lys Gly Ala Phe Asn Ile Ala
               180                 185                 190
   gtt cgt gcg cag att ccc att att cca gtt gta ttc tca gac tat cgg      624
   Val Arg Ala Gln Ile Pro Ile Ile Pro Val Val Phe Ser Asp Tyr Arg
           195                 200                 205
   gat ttc tac tca aag cca ggc cga tat ttc aag aat gat gga gaa gtt      672
   Asp Phe Tyr Ser Lys Pro Gly Arg Tyr Phe Lys Asn Asp Gly Glu Val
       210                 215                 220
   gtt att cga gtt ctg gat gcg att cca aca aaa ggg ctc act ctt gat      720
   Val Ile Arg Val Leu Asp Ala Ile Pro Thr Lys Gly Leu Thr Leu Asp
   225                 230                 235                 240
   gac gtc agc gag ttg tct gat atg tgt cgg gac gtt atg ttg gca gcc      768
   Asp Val Ser Glu Leu Ser Asp Met Cys Arg Asp Val Met Leu Ala Ala
                   245                 250                 255
   tat aag gaa gtt act cta gaa gct cag caa cga aat gcg aca cgg cgt      816
   Tyr Lys Glu Val Thr Leu Glu Ala Gln Gln Arg Asn Ala Thr Arg Arg
               260                 265                 270
   gga gaa aca aaa gac ggg aag aaa tct gag taa                          849
   Gly Glu Thr Lys Asp Gly Lys Lys Ser Glu
           275                 280
<210> 43
   <211> 282
   <212> PRT
   <213> Caenorhabditis elegans
<400> 43
   Met Glu Asn Phe Trp Ser Ile Val Val Phe Phe Leu Leu Ser Ile Leu
   1               5                   10                  15
   Phe Ile Leu Tyr Asn Ile Ser Thr Val Cys His Tyr Tyr Val Arg Ile
               20                  25                  30
   Ser Phe Tyr Tyr Phe Thr Ile Leu Leu His Gly Met Glu Val Cys Val
                   35                  40                  45
   Thr Met Ile Pro Ser Trp Leu Asn Gly Lys Gly Ala Asp Tyr Val Phe
       50                  55                  60
   His Ser Phe Phe Tyr Trp Cys Lys Trp Thr Gly Val His Thr Thr Val
   65                  70                  75                  80
   Tyr Gly Tyr Glu Lys Thr Gln Val Glu Gly Pro Ala Val Val Ile Cys
                   85                  90                  95
   Asn His Gln Ser Ser Leu Asp Ile Leu Ser Met Ala Ser Ile Trp Pro
               100                 105                 110
   Lys Asn Cys Val Val Met Met Lys Arg Ile Leu Ala Tyr Val Pro Phe
           115                 120                 125
   Phe Asn Leu Gly Ala Tyr Phe Ser Asn Thr Ile Phe Ile Asp Arg Tyr
       130                 135                 140
   Asn Arg Glu Arg Ala Met Ala Ser Val Asp Tyr Cys Ala Ser Glu Met
   145                 150                 155                 160
   Lys Asn Arg Asn Leu Lys Leu Trp Val Phe Pro Glu Gly Thr Arg Asn
                   165                 170                 175
   Arg Glu Gly Gly Phe Ile Pro Phe Lys Lys Gly Ala Phe Asn Ile Ala
               180                 185                 190
   Val Arg Ala Gln Ile Pro Ile Ile Pro Val Val Phe Ser Asp Tyr Arg
           195                 200                 205
   Asp Phe Tyr Ser Lys Pro Gly Arg Tyr Phe Lys Asn Asp Gly Glu Val
       210                 215                 220
   Val Ile Arg Val Leu Asp Ala Ile Pro Thr Lys Gly Leu Thr Leu Asp
   225                 230                 235                 240
   Asp Val Ser Glu Leu Ser Asp Met Cys Arg Asp Val Met Leu Ala Ala
                   245                 250                 255
   Tyr Lys Glu Val Thr Leu Glu Ala Gln Gln Arg Asn Ala Thr Arg Arg
               260                 265                 270
   Gly Glu Thr Lys Asp Gly Lys Lys Ser Glu
           275                 280
<210> 44
   <211> 849
   <212> DNA
   <213> Caenorhabditis elegans
<220>
   <221> CDS
   <222> (1)..(849)
   <223> Acyl-CoA:Lysophospholipid-Acyltransferase
<400> 44
   atg gag aac ttc tgg tcg atc gtc gtg ttt ttt cta ctc tca att ctc      48
   Met Glu Asn Phe Trp Ser Ile Val Val Phe Phe Leu Leu Ser Ile Leu
   1               5                   10                  15
   ttc att tta tat aac ata tcg aca gta tgc cac tac tat atg cgg att      96
   Phe Ile Leu Tyr Asn Ile Ser Thr Val Cys His Tyr Tyr Met Arg Ile
               20                  25                  30
   tcg ttt tat tac ttc aca att tta ttg cat gga atg gaa gtt tgt gtt     144
   Ser Phe Tyr Tyr Phe Thr Ile Leu Leu His Gly Met Glu Val Cys Val
               35                  40                  45
   aca atg atc cct tct tgg cta aat ggg aag ggt gct gat tac gtg ttt      192
   Thr Met Ile Pro Ser Trp Leu Asn Gly Lys Gly Ala Asp Tyr Val Phe
       50                  55                  60
   cac tcg ttt ttc tat tgg tgt aaa tgg act ggt gtt cat aca aca gtc      240
   His Ser Phe Phe Tyr Trp Cys Lys Trp Thr Gly Val His Thr Thr Val
   65                  70                  75                  80
   tat gga tat gaa aaa aca caa gtt gaa ggt ccg gcc gta gtt att tgt      288
   Tyr Gly Tyr Glu Lys Thr Gln Val Glu Gly Pro Ala Val Val Ile Cys
                   85                  90                  95
   aat cat cag ggt tct ctc gac att cta tcg atg gca tca atc tgg ccg      336
   Asn His Gln Gly Ser Leu Asp Ile Leu Ser Met Ala Ser Ile Trp Pro
               100                 105                 110
   aag aat tgt gtt gta atg atg aaa cga att ctt gcc tat gtt cca ttc      384
   Lys Asn Cys Val Val Met Met Lys Arg Ile Leu Ala Tyr Val Pro Phe
           115                 120                 125
   ttc aat ctc gga gcc tac ttt tcc aac aca atc ttc atc gat cga tat      432
   Phe Asn Leu Gly Ala Tyr Phe Ser Asn Thr Ile Phe Ile Asp Arg Tyr
       130                 135                 140
   aac cgt gaa cgt gcg atg gct tca gtt gat tat tgt gca tct gaa atg      480
   Asn Arg Glu Arg Ala Met Ala Ser Val Asp Tyr Cys Ala Ser Glu Met
   145                 150                 155                 160
   aag aac aga aat ctt aaa ctt tgg gta ttt ccg gaa gga aca aga aat      528
   Lys Asn Arg Asn Leu Lys Leu Trp Val Phe Pro Glu Gly Thr Arg Asn
                   165                 170                 175
   cgt gaa gga ggg ttc att cca ttc aag aaa gga gca ttc aat att gca      576
   Arg Glu Gly Gly Phe Ile Pro Phe Lys Lys Gly Ala Phe Asn Ile Ala
               180                 185                 190
   gtt cgt gcg cag att ccc att att cca gtt gta ttc tca gac tat cgg      624
   Val Arg Ala Gln Ile Pro Ile Ile Pro Val Val Phe Ser Asp Tyr Arg
           195                 200                 205
   gat ttc tac tca aag cca ggc cga tat ttc aag aat gat gga gaa gtt      672
   Asp Phe Tyr Ser Lys Pro Gly Arg Tyr Phe Lys Asn Asp Gly Glu Val
       210                 215                 220
   gtt att cga gtt ctg gat gcg att cca aca aaa ggg ctc act ctt gat      720
   Val Ile Arg Val Leu Asp Ala Ile Pro Thr Lys Gly Leu Thr Leu Asp
   225                 230                 235                 240
   gac gtc agc gag ttg tct gat atg tgt cgg gac gtt atg ttg gca gcc      768
   Asp Val Ser Glu Leu Ser Asp Met Cys Arg Asp Val Met Leu Ala Ala
                   245                 250                 255
   tat aag gaa gtt act cta gaa gct cag caa cga aat gcg aca cgg cgt      816
   Tyr Lys Glu Val Thr Leu Glu Ala Gln Gln Arg Asn Ala Thr Arg Arg
               260                 265                 270
   gga gaa aca aaa gac ggg aag aaa tct gag taa                          849
   Gly Glu Thr Lys Asp Gly Lys Lys Ser Glu
           275                 280
<210> 45
   <211> 282
   <212> PRT
   <213> Caenorhabditis elegans
<400> 45
   Met Glu Asn Phe Trp Ser Ile Val Val Phe Phe Leu Leu Ser Ile Leu
   1               5                   10                  15
   Phe Ile Leu Tyr Asn Ile Ser Thr Val Cys His Tyr Tyr Met Arg Ile
               20                  25                  30
   Ser Phe Tyr Tyr Phe Thr Ile Leu Leu His Gly Met Glu Val Cys Val
           35                  40                  45
   Thr Met Ile Pro Ser Trp Leu Asn Gly Lys Gly Ala Asp Tyr Val Phe
       50                  55                  60
   His Ser Phe Phe Tyr Trp Cys Lys Trp Thr Gly Val His Thr Thr Val
   65                  70                  75                  80
   Tyr Gly Tyr Glu Lys Thr Gln Val Glu Gly Pro Ala Val Val Ile Cys
                   85                  90                  95
   Asn His Gln Gly Ser Leu Asp Ile Leu Ser Met Ala Ser Ile Trp Pro
               100                 105                 110
   Lys Asn Cys Val Val Met Met Lys Arg Ile Leu Ala Tyr Val Pro Phe
           115                 120                 125
   Phe Asn Leu Gly Ala Tyr Phe Ser Asn Thr Ile Phe Ile Asp Arg Tyr
       130                 135                 140
   Asn Arg Glu Arg Ala Met Ala Ser Val Asp Tyr Cys Ala Ser Glu Met
   145                 150                 155                 160
   Lys Asn Arg Asn Leu Lys Leu Trp Val Phe Pro Glu Gly Thr Arg Asn
                   165                 170                 175
   Arg Glu Gly Gly Phe Ile Pro Phe Lys Lys Gly Ala Phe Asn Ile Ala
               180                 185                 190
   Val Arg Ala Gln Ile Pro Ile Ile Pro Val Val Phe Ser Asp Tyr Arg
           195                 200                 205
   Asp Phe Tyr Ser Lys Pro Gly Arg Tyr Phe Lys Asn Asp Gly Glu Val
       210                 215                 220
   Val Ile Arg Val Leu Asp Ala Ile Pro Thr Lys Gly Leu Thr Leu Asp
   225                 230                 235                 240
   Asp Val Ser Glu Leu Ser Asp Met Cys Arg Asp Val Met Leu Ala Ala
                   245                 250                 255
   Tyr Lys Glu Val Thr Leu Glu Ala Gln Gln Arg Asn Ala Thr Arg Arg
               260                 265                 270
   Gly Glu Thr Lys Asp Gly Lys Lys Ser Glu
           275                 280
<210> 46
   <211> 1578
   <212> DNA
   <213> Physcomitrella patens
<220>
   <221> CDS
   <222> (1)..(1578)
   <223> Delta-6-Desaturase
<400> 46
   atg gta ttc gcg ggc ggt gga ctt cag cag ggc tct ctc gaa gaa aac      48
   Met Val Phe Ala Gly Gly Gly Leu Gln Gln Gly Ser Leu Glu Glu Asn
   1               5                   10                  15
   atc gac gtc gag cac att gcc agt atg tct ctc ttc agc gac ttc ttc        96
   Ile Asp Val Glu His Ile Ala Ser Met Ser Leu Phe Ser Asp Phe Phe
               20                  25                  30
   agt tat gtg tct tca act gtt ggt tcg tgg agc gta cac agt ata caa       144
   Ser Tyr Val Ser Ser Thr Val Gly Ser Trp Ser Val His Ser Ile Gln
           35                  40                  45
   cct ttg aag cgc ctg acg agt aag aag cgt gtt tcg gaa agc gct gcc       192
   Pro Leu Lys Arg Leu Thr Ser Lys Lys Arg Val Ser Glu Ser Ala Ala
       50                  55                  60
   gtg caa tgt ata tca gct gaa gtt cag aga aat tcg agt acc cag gga       240
   Val Gln Cys Ile Ser Ala Glu Val Gln Arg Asn Ser Ser Thr Gln Gly
   65                  70                  75                  80
   act gcg gag gca ctc gca gaa tca gtc gtg aag ccc acg aga cga agg       288
   Thr Ala Glu Ala Leu Ala Glu Ser Val Val Lys Pro Thr Arg Arg Arg
                   85                  90                  95
   tca tct cag tgg aag aag tcg aca cac ccc cta tca gaa gta gca gta       336
   Ser Ser Gln Trp Lys Lys Ser Thr His Pro Leu Ser Glu Val Ala Val
               100                 105                 110
   cac aac aag cca agc gat tgc tgg att gtt gta aaa aac aag gtg tat       384
   His Asn Lys Pro Ser Asp Cys Trp Ile Val Val Lys Asn Lys Val Tyr
           115                 120                 125
   gat gtt tcc aat ttt gcg gac gag cat ccc gga gga tca gtt att agt       432
   Asp Val Ser Asn Phe Ala Asp Glu His Pro Gly Gly Ser Val Ile Ser
       130                 135                 140
   act tat ttt gga cga gac ggc aca gat gtt ttc tct agt ttt cat gca       480
   Thr Tyr Phe Gly Arg Asp Gly Thr Asp Val Phe Ser Ser Phe His Ala
   145                 150                 155                 160
   gct tct aca tgg aaa att ctt caa gac ttt tac att ggt gac gtg gag       528
   Ala Ser Thr Trp Lys Ile Leu Gln Asp Phe Tyr Ile Gly Asp Val Glu
                   165                 170                 175
   agg gtg gag ccg act cca gag ctg ctg aaa gat ttc cga gaa atg aga       576
   Arg Val Glu Pro Thr Pro Glu Leu Leu Lys Asp Phe Arg Glu Met Arg
               180                 185                 190
   gct ctt ttc ctg agg gag caa ctt ttc aaa agt tcg aaa ttg tac tat       624
   Ala Leu Phe Leu Arg Glu Gln Leu Phe Lys Ser Ser Lys Leu Tyr Tyr
           195                 200                 205
   gtt atg aag ctg ctc acg aat gtt gct att ttt gct gcg agc att gca       672
   Val Met Lys Leu Leu Thr Asn Val Ala Ile Phe Ala Ala Ser Ile Ala
       210                 215                 220
   ata ata tgt tgg agc aag act att tca gcg gtt ttg gct tca gct tgt       720
   Ile Ile Cys Trp Ser Lys Thr Ile Ser Ala Val Leu Ala Ser Ala Cys
   225                 230                 235                 240
   atg atg gct ctg tgt ttc caa cag tgc gga tgg cta tcc cat gat ttt       768
   Met Met Ala Leu Cys Phe Gln Gln Cys Gly Trp Leu Ser His Asp Phe
                   245                 250                 255
   ctc cac aat cag gtg ttt gag aca cgc tgg ctt aat gaa gtt gtc ggg       816
   Leu His Asn Gln Val Phe Glu Thr Arg Trp Leu Asn Glu Val Val Gly
               260                 265                 270
   tat gtg atc ggc aac gcc gtt ctg ggg ttt agt aca ggg tgg tgg aag       864
   Tyr Val Ile Gly Asn Ala Val Leu Gly Phe Ser Thr Gly Trp Trp Lys
           275                 280                 285
   gag aag cat aac ctt cat cat gct gct cca aat gaa tgc gat cag act       912
   Glu Lys His Asn Leu His His Ala Ala Pro Asn Glu Cys Asp Gln Thr
       290                 295                 300
   tac caa cca att gat gaa gat att gat act ctc ccc ctc att gcc tgg      960
   Tyr Gln Pro Ile Asp Glu Asp Ile Asp Thr Leu Pro Leu Ile Ala Trp
   305                 310                 315                 320
   agc aag gac ata ctg gcc aca gtt gag aat aag aca ttc ttg cga atc     1008
   Ser Lys Asp Ile Leu Ala Thr Val Glu Asn Lys Thr Phe Leu Arg Ile
                   325                 330                 335
   ctc caa tac cag cat ctg ttc ttc atg ggt ctg tta ttt ttc gcc cgt     1056
   Leu Gln Tyr Gln His Leu Phe Phe Met Gly Leu Leu Phe Phe Ala Arg
               340                 345                 350
   ggt agt tgg ctc ttt tgg agc tgg aga tat acc tct aca gca gtg ctc     1104
   Gly Ser Trp Leu Phe Trp Ser Trp Arg Tyr Thr Ser Thr Ala Val Leu
           355                 360                 365
   tca cct gtc gac agg ttg ttg gag aag gga act gtt ctg ttt cac tac     1152
   Ser Pro Val Asp Arg Leu Leu Glu Lys Gly Thr Val Leu Phe His Tyr
       370                 375                 380
   ttt tgg ttc gtc ggg aca gcg tgc tat ctt ctc cct ggt tgg aag cca     1200
   Phe Trp Phe Val Gly Thr Ala Cys Tyr Leu Leu Pro Gly Trp Lys Pro
   385                 390                 395                 400
   tta gta tgg atg gcg gtg act gag ctc atg tcc ggc atg ctg ctg ggc     1248
   Leu Val Trp Met Ala Val Thr Glu Leu Met Ser Gly Met Leu Leu Gly
                   405                 410                 415
   ttt gta ttt gta ctt agc cac aat ggg atg gag gtt tat aat tcg tct     1296
   Phe Val Phe Val Leu Ser His Asn Gly Met Glu Val Tyr Asn Ser Ser
               420                 425                 430
   aaa gaa ttc gtg agt gca cag atc gta tcc aca cgg gat atc aaa gga     1344
   Lys Glu Phe Val Ser Ala Gln Ile Val Ser Thr Arg Asp Ile Lys Gly
           435                 440                 445
   aac ata ttc aac gac tgg ttc act ggt ggc ctt aac agg caa ata gag     1392
   Asn Ile Phe Asn Asp Trp Phe Thr Gly Gly Leu Asn Arg Gln Ile Glu
       450                 455                 460
   cat cat ctt ttc cca aca atg ccc agg cat aat tta aac aaa ata gca     1440
   His His Leu Phe Pro Thr Met Pro Arg His Asn Leu Asn Lys Ile Ala
   465                 470                 475                 480
   cct aga gtg gag gtg ttc tgt aag aaa cac ggt ctg gtg tac gaa gac     1488
   Pro Arg Val Glu Val Phe Cys Lys Lys His Gly Leu Val Tyr Glu Asp
                   485                 490                 495
   gta tct att gct acc ggc act tgc aag gtt ttg aaa gca ttg aag gaa     1536
   Val Ser Ile Ala Thr Gly Thr Cys Lys Val Leu Lys Ala Leu Lys Glu
               500                 505                 510
   gtc gcg gag gct gcg gca gag cag cat gct acc acc agt taa             1578
   Val Ala Glu Ala Ala Ala Glu Gln His Ala Thr Thr Ser
           515                 520                 525
<210> 47
   <211> 525
   <212> PRT
   <213> Physcomitrella patens
<400> 47
   Met Val Phe Ala Gly Gly Gly Leu Gln Gln Gly Ser Leu Glu Glu Asn
   1               5                   10                  15
   Ile Asp Val Glu His Ile Ala Ser Met Ser Leu Phe Ser Asp Phe Phe
   20 25 30
   Ser Tyr Val Ser Ser Thr Val Gly Ser Trp Ser Val His Ser Ile Gln
   35 40 45
   Pro Leu Lys Arg Leu Thr Ser Lys Lys Arg Val Ser Glu Ser Ala Ala
   50 55 60
   Val Gln Cys Ile Ser Ala Glu Val Gln Arg Asn Ser Ser Thr Gln Gly
   65 70 75 80
   Thr Ala Glu Ala Leu Ala Glu Ser Val Val Lys Pro Thr Arg Arg Arg
   85 90 95
   Ser Ser Gln Trp Lys Lys Ser Thr His Pro Leu Ser Glu Val Ala Val
   100 105 110
   His Asn Lys Pro Ser Asp Cys Trp Ile Val Val Lys Asn Lys Val Tyr
   115 120 125
   Asp Val Ser Asn Phe Ala Asp Glu His Pro Gly Gly Ser Val Ile Ser
   130 135 140
   Thr Tyr Phe Gly Arg Asp Gly Thr Asp Val Phe Ser Ser Phe His Ala
   145 150 155 160
   Ala Ser Thr Trp Lys Ile Leu Gln Asp Phe Tyr Ile Gly Asp Val Glu
   165 170 175
   Arg Val Glu Pro Thr Pro Glu Leu Leu Lys Asp Phe Arg Glu Met Arg
   180 185 190
   Ala Leu Phe Leu Arg Glu Gln Leu Phe Lys Ser Ser Lys Leu Tyr Tyr
   195 200 205
   Val Met Lys Leu Leu Thr Asn Val Ala Ile Phe Ala Ala Ser Ile Ala
   210 215 220
   Ile Ile Cys Trp Ser Lys Thr Ile Ser Ala Val Leu Ala Ser Ala Cys
   225 230 235 240
   Met Met Ala Leu Cys Phe Gln Gln Cys Gly Trp Leu Ser His Asp Phe
   245 250 255
   Leu His Asn Gln Val Phe Glu Thr Arg Trp Leu Asn Glu Val Val Gly
   260 265 270
   Tyr Val Ile Gly Asn Ala Val Leu Gly Phe Ser Thr Gly Trp Trp Lys
   275 280 285
   Glu Lys His Asn Leu His His Ala Ala Pro Asn Glu Cys Asp Gln Thr
   290 295 300
   Tyr Gln Pro Ile Asp Glu Asp Ile Asp Thr Leu Pro Leu Ile Ala Trp
   305 310 315 320
   Ser Lys Asp Ile Leu Ala Thr Val Glu Asn Lys Thr Phe Leu Arg Ile
   325 330 335
   Leu Gln Tyr Gln His Leu Phe Phe Met Gly Leu Leu Phe Phe Ala Arg
   340 345 350
   Gly Ser Trp Leu Phe Trp Ser Trp Arg Tyr Thr Ser Thr Ala Val Leu
   355 360 365
   Ser Pro Val Asp Arg Leu Leu Glu Lys Gly Thr Val Leu Phe His Tyr
   370 375 380
   Phe Trp Phe Val Gly Thr Ala Cys Tyr Leu Leu Pro Gly Trp Lys Pro
   385 390 395 400
   Leu Val Trp Met Ala Val Thr Glu Leu Met Ser Gly Met Leu Leu Gly
   405 410 415
   Phe Val Phe Val Leu Ser His Asn Gly Met Glu Val Tyr Asn Ser Ser
   420 425 430
   Lys Glu Phe Val Ser Ala Gln Ile Val Ser Thr Arg Asp Ile Lys Gly
   435 440 445
   Asn Ile Phe Asn Asp Trp Phe Thr Gly Gly Leu Asn Arg Gln Ile Glu
       450                 455                 460
   His His Leu Phe Pro Thr Met Pro Arg His Asn Leu Asn Lys Ile Ala
   465                 470                 475                 480
   Pro Arg Val Glu Val Phe Cys Lys Lys His Gly Leu Val Tyr Glu Asp
                   485                 490                 495
   Val Ser Ile Ala Thr Gly Thr Cys Lys Val Leu Lys Ala Leu Lys Glu
               500                 505                 510
   Val Ala Glu Ala Ala Ala Glu Gln His Ala Thr Thr Ser
           515                 520                 525
<210> 48
   <211> 1192
   <212> DNA
   <213> Physcomitrella patens
<220>
   <221> CDS
   <222> (58)..(930)
   <223> Delta-6-Elongase
<400> 48
   ctgcttcgtc tcatcttggg ggtgtgattc gggagtgggt tgagttggtg gagcgca        57
   atg gag gtc gtg gag aga ttc tac ggt gag ttg gat ggg aag gtc tcg      105
   Met Glu Val Val Glu Arg Phe Tyr Gly Glu Leu Asp Gly Lys Val Ser
   1               5                   10                  15
   cag ggc gtg aat gca ttg ctg ggt agt ttt ggg gtg gag ttg acg gat      153
   Gln Gly Val Asn Ala Leu Leu Gly Ser Phe Gly Val Glu Leu Thr Asp
               20                  25                  30
   acg ccc act acc aaa ggc ttg ccc ctc gtt gac agt ccc aca ccc atc      201
   Thr Pro Thr Thr Lys Gly Leu Pro Leu Val Asp Ser Pro Thr Pro Ile
           35                  40                  45
   gtc ctc ggt gtt tct gta tac ttg act att gtc att gga ggg ctt ttg      249
   Val Leu Gly Val Ser Val Tyr Leu Thr Ile Val Ile Gly Gly Leu Leu
       50                  55                  60
   tgg ata aag gcc agg gat ctg aaa ccg cgc gcc tcg gag cca ttt ttg      297
   Trp Ile Lys Ala Arg Asp Leu Lys Pro Arg Ala Ser Glu Pro Phe Leu
   65                  70                  75                  80
   ctc caa gct ttg gtg ctt gtg cac aac ctg ttc tgt ttt gcg ctc agt      345
   Leu Gln Ala Leu Val Leu Val His Asn Leu Phe Cys Phe Ala Leu Ser
                   85                  90                  95
   ctg tat atg tgc gtg ggc atc gct tat cag gct att acc tgg cgg tac      393
   Leu Tyr Met Cys Val Gly Ile Ala Tyr Gln Ala Ile Thr Trp Arg Tyr
               100                 105                 110
   tct ctc tgg ggc aat gca tac aat cct aaa cat aaa gag atg gcg att      441
   Ser Leu Trp Gly Asn Ala Tyr Asn Pro Lys His Lys Glu Met Ala Ile
           115                 120                 125
   ctg gta tac ttg ttc tac atg tct aag tac gtg gaa ttc atg gat acc      489
   Leu Val Tyr Leu Phe Tyr Met Ser Lys Tyr Val Glu Phe Met Asp Thr
       130                 135                 140
   gtt atc atg ata ctg aag cgc agc acc agg caa ata agc ttc ctc cac      537
   Val Ile Met Ile Leu Lys Arg Ser Thr Arg Gln Ile Ser Phe Leu His
   145                 150                 155                 160
   gtt tat cat cat tct tca att tcc ctc att tgg tgg gct att gct cat       585
   Val Tyr His His Ser Ser Ile Ser Leu Ile Trp Trp Ala Ile Ala His
                   165                 170                 175
   cac gct cct ggc ggt gaa gca tat tgg tct gcg gct ctg aac tca gga       633
   His Ala Pro Gly Gly Glu Ala Tyr Trp Ser Ala Ala Leu Asn Ser Gly
               180                 185                 190
   gtg cat gtt ctc atg tat gcg tat tac ttc ttg gct gcc tgc ctt cga       681
   Val His Val Leu Met Tyr Ala Tyr Tyr Phe Leu Ala Ala Cys Leu Arg
           195                 200                 205
   agt agc cca aag tta aaa aat aag tac ctt ttt tgg ggc agg tac ttg       729
   Ser Ser Pro Lys Leu Lys Asn Lys Tyr Leu Phe Trp Gly Arg Tyr Leu
       210                 215                 220
   aca caa ttc caa atg ttc cag ttt atg ctg aac tta gtg cag gct tac       777
   Thr Gln Phe Gln Met Phe Gln Phe Met Leu Asn Leu Val Gln Ala Tyr
   225                 230                 235                 240
   tac gac atg aaa acg aat gcg cca tat cca caa tgg ctg atc aag att       825
   Tyr Asp Met Lys Thr Asn Ala Pro Tyr Pro Gln Trp Leu Ile Lys Ile
                   245                 250                 255
   ttg ttc tac tac atg atc tcg ttg ctg ttt ctt ttc ggc aat ttt tac       873
   Leu Phe Tyr Tyr Met Ile Ser Leu Leu Phe Leu Phe Gly Asn Phe Tyr
               260                 265                 270
   gta caa aaa tac atc aaa ccc tct gac gga aag caa aag gga gct aaa       921
   Val Gln Lys Tyr Ile Lys Pro Ser Asp Gly Lys Gln Lys Gly Ala Lys
           275                 280                 285
   act gag tga gctgtatcaa gccatagaaa ctctattatg ttagaacctg               970
   Thr Glu
       290
   aagttggtgc tttcttatct ccacttatct tttaagcagc atcagttttg aaatgatgtg   1030
   tgggcgtggt ctgcaagtag tcatcaatat aatcggcctg agcacttcag atggattgtt   1090
   agaacatgag taaaagcggt tattacggtg tttattttgt accaaatcac cgcacgggtg   1150
   aattgaaata tttcagattt gatcaatttc atctgaaaaa aa                      1192
<210> 49
   <211> 290
   <212> PRT
   <213> Physcomitrella patens
<400> 49
   Met Glu Val Val Glu Arg Phe Tyr Gly Glu Leu Asp Gly Lys Val Ser
   1               5                   10                  15
   Gln Gly Val Asn Ala Leu Leu Gly Ser Phe Gly Val Glu Leu Thr Asp
               20                  25                  30
   Thr Pro Thr Thr Lys Gly Leu Pro Leu Val Asp Ser Pro Thr Pro Ile
           35                  40                  45
   Val Leu Gly Val Ser Val Tyr Leu Thr Ile Val Ile Gly Gly Leu Leu
       50                  55                  60
   Trp Ile Lys Ala Arg Asp Leu Lys Pro Arg Ala Ser Glu Pro Phe Leu
   65                  70                  75                  80
   Leu Gln Ala Leu Val Leu Val His Asn Leu Phe Cys Phe Ala Leu Ser
                   85                  90                  95
   Leu Tyr Met Cys Val Gly Ile Ala Tyr Gln Ala Ile Thr Trp Arg Tyr
               100                 105                 110
   Ser Leu Trp Gly Asn Ala Tyr Asn Pro Lys His Lys Glu Met Ala Ile
           115                 120                 125
   Leu Val Tyr Leu Phe Tyr Met Ser Lys Tyr Val Glu Phe Met Asp Thr
       130                 135                 140
   Val Ile Met Ile Leu Lys Arg Ser Thr Arg Gln Ile Ser Phe Leu His
   145                 150                 155                 160
   Val Tyr His His Ser Ser Ile Ser Leu Ile Trp Trp Ala Ile Ala His
                   165                 170                 175
   His Ala Pro Gly Gly Glu Ala Tyr Trp Ser Ala Ala Leu Asn Ser Gly
               180                 185                 190
   Val His Val Leu Met Tyr Ala Tyr Tyr Phe Leu Ala Ala Cys Leu Arg
           195                 200                 205
   Ser Ser Pro Lys Leu Lys Asn Lys Tyr Leu Phe Trp Gly Arg Tyr Leu
       210                 215                 220
   Thr Gln Phe Gln Met Phe Gln Phe Met Leu Asn Leu Val Gln Ala Tyr
   225                 230                 235                 240
   Tyr Asp Met Lys Thr Asn Ala Pro Tyr Pro Gln Trp Leu Ile Lys Ile
                   245                 250                 255
   Leu Phe Tyr Tyr Met Ile Ser Leu Leu Phe Leu Phe Gly Asn Phe Tyr
               260                 265                 270
   Val Gln Lys Tyr Ile Lys Pro Ser Asp Gly Lys Gln Lys Gly Ala Lys
           275                 280                 285
   Thr Glu
       290
<210> 50
   <211> 1410
   <212> DNA
   <213> Phaeodactylum tricornutum
<220>
   <221> CDS
   <222> (1)..(1410)
   <223> Delta-5-Desaturase
<400> 50
   atg gct ccg gat gcg gat aag ctt cga caa cgc cag acg act gcg gta      48
   Met Ala Pro Asp Ala Asp Lys Leu Arg Gln Arg Gln Thr Thr Ala Val
   1               5                   10                  15
   gcg aag cac aat gct gct acc ata tcg acg cag gaa cgc ctt tgc agt      96
   Ala Lys His Asn Ala Ala Thr Ile Ser Thr Gln Glu Arg Leu Cys Ser
               20                  25                  30
   ctg tct tcg ctc aaa ggc gaa gaa gtc tgc atc gac gga atc atc tat     144
   Leu Ser Ser Leu Lys Gly Glu Glu Val Cys Ile Asp Gly Ile Ile Tyr
           35                  40                  45
   gac ctc caa tca ttc gat cat ccc ggg ggt gaa acg atc aaa atg ttt     192
   Asp Leu Gln Ser Phe Asp His Pro Gly Gly Glu Thr Ile Lys Met Phe
       50                  55                  60
   ggt ggc aac gat gtc act gta cag tac aag atg att cac ccg tac cat     240
   Gly Gly Asn Asp Val Thr Val Gln Tyr Lys Met Ile His Pro Tyr His
   65                  70                  75                  80
   acc gag aag cat ttg gaa aag atg aag cgt gtc ggc aag gtg acg gat     288
   Thr Glu Lys His Leu Glu Lys Met Lys Arg Val Gly Lys Val Thr Asp
                   85                  90                  95
   ttc gtc tgc gag tac aag ttc gat acc gaa ttt gaa cgc gaa atc aaa      336
   Phe Val Cys Glu Tyr Lys Phe Asp Thr Glu Phe Glu Arg Glu Ile Lys
               100                 105                 110
   cga gaa gtc ttc aag att gtg cga cga ggc aag gat ttc ggt act ttg      384
   Arg Glu Val Phe Lys Ile Val Arg Arg Gly Lys Asp Phe Gly Thr Leu
           115                 120                 125
   gga tgg ttc ttc cgt gcg ttt tgc tac att gcc att ttc ttc tac ctg      432
   Gly Trp Phe Phe Arg Ala Phe Cys Tyr Ile Ala Ile Phe Phe Tyr Leu
       130                 135                 140
   cag tac cat tgg gtc acc acg gga acc tct tgg ctg ctg gcc gtg gcc      480
   Gln Tyr His Trp Val Thr Thr Gly Thr Ser Trp Leu Leu Ala Val Ala
   145                 150                 155                 160
   tac gga atc tcc caa gcg atg att ggc atg aat gtc cag cac gat gcc      528
   Tyr Gly Ile Ser Gln Ala Met Ile Gly Met Asn Val Gln His Asp Ala
                   165                 170                 175
   aac cac ggg gcc acc tcc aag cgt ccc tgg gtc aac gac atg cta ggc      576
   Asn His Gly Ala Thr Ser Lys Arg Pro Trp Val Asn Asp Met Leu Gly
               180                 185                 190
   ctc ggt gcg gat ttt att ggt ggt tcc aag tgg ctc tgg cag gaa caa      624
   Leu Gly Ala Asp Phe Ile Gly Gly Ser Lys Trp Leu Trp Gln Glu Gln
           195                 200                 205
   cac tgg acc cac cac gct tac acc aat cac gcc gag atg gat ccc gat      672
   His Trp Thr His His Ala Tyr Thr Asn His Ala Glu Met Asp Pro Asp
       210                 215                 220
   agc ttt ggt gcc gaa cca atg ctc cta ttc aac gac tat ccc ttg gat      720
   Ser Phe Gly Ala Glu Pro Met Leu Leu Phe Asn Asp Tyr Pro Leu Asp
   225                 230                 235                 240
   cat ccc gct cgt acc tgg cta cat cgc ttt caa gca ttc ttt tac atg      768
   His Pro Ala Arg Thr Trp Leu His Arg Phe Gln Ala Phe Phe Tyr Met
                   245                 250                 255
   ccc gtc ttg gct gga tac tgg ttg tcc gct gtc ttc aat cca caa att      816
   Pro Val Leu Ala Gly Tyr Trp Leu Ser Ala Val Phe Asn Pro Gln Ile
               260                 265                 270
   ctt gac ctc cag caa cgc ggc gca ctt tcc gtc ggt atc cgt ctc gac      864
   Leu Asp Leu Gln Gln Arg Gly Ala Leu Ser Val Gly Ile Arg Leu Asp
           275                 280                 285
   aac gct ttc att cac tcg cga cgc aag tat gcg gtt ttc tgg cgg gct      912
   Asn Ala Phe Ile His Ser Arg Arg Lys Tyr Ala Val Phe Trp Arg Ala
       290                 295                 300
   gtg tac att gcg gtg aac gtg att gct ccg ttt tac aca aac tcc ggc      960
   Val Tyr Ile Ala Val Asn Val Ile Ala Pro Phe Tyr Thr Asn Ser Gly
   305                 310                 315                 320
   ctc gaa tgg tcc tgg cgt gtc ttt gga aac atc atg ctc atg ggt gtg     1008
   Leu Glu Trp Ser Trp Arg Val Phe Gly Asn Ile Met Leu Met Gly Val
                   325                 330                 335
   gcg gaa tcg ctc gcg ctg gcg gtc ctg ttt tcg ttg tcg cac aat ttc     1056
   Ala Glu Ser Leu Ala Leu Ala Val Leu Phe Ser Leu Ser His Asn Phe
               340                 345                 350
   gaa tcc gcg gat cgc gat ccg acc gcc cca ctg aaa aag acg gga gaa     1104
   Glu Ser Ala Asp Arg Asp Pro Thr Ala Pro Leu Lys Lys Thr Gly Glu
           355                 360                 365
   cca gtc gac tgg ttc aag aca cag gtc gaa act tcc tgc act tac ggt     1152
   Pro Val Asp Trp Phe Lys Thr Gln Val Glu Thr Ser Cys Thr Tyr Gly
   370 375 380
   gga ttc ctt tcc ggt tgc ttc acg gga ggt ctc aac ttt cag gtt gaa 1200
   Gly Phe Leu Ser Gly Cys Phe Thr Gly Gly Leu Asn Phe Gln Val Glu
   385 390 395 400
   cac cac ttg ttc cca cgc atg agc agc gct tgg tat ccc tac att gcc 1248
   His His Leu Phe Pro Arg Met Ser Ser Ala Trp Tyr Pro Tyr Ile Ala
   405 410 415
   ccc aag gtc cgc gaa att tgc gcc aaa cac ggc gtc cac tac gcc tac 1296
   Pro Lys Val Arg Glu Ile Cys Ala Lys His Gly Val His Tyr Ala Tyr
   420 425 430
   tac ccg tgg atc cac caa aac ttt ctc tcc acc gtc cgc tac atg cac 1344
   Tyr Pro Trp Ile His Gln Asn Phe Leu Ser Thr Val Arg Tyr Met His
   435 440 445
   gcg gcc ggg acc ggt gcc aac tgg cgc cag atg gcc aga gaa aat ccc 1392
   Ala Ala Gly Thr Gly Ala Asn Trp Arg Gln Met Ala Arg Glu Asn Pro
   450 455 460
   ttg acc gga cgg gcg taa 1410
   Leu Thr Gly Arg Ala
   465
<210> 51
   <211> 469
   <212> PRT
   <213> Phaeodactylum tricornutum
<400> 51
   Met Ala Pro Asp Ala Asp Lys Leu Arg Gln Arg Gln Thr Thr Ala Val
   1 5 10 15
   Ala Lys His Asn Ala Ala Thr Ile Ser Thr Gln Glu Arg Leu Cys Ser
   20 25 30
   Leu Ser Ser Leu Lys Gly Glu Glu Val Cys Ile Asp Gly Ile Ile Tyr
   35 40 45
   Asp Leu Gln Ser Phe Asp His Pro Gly Gly Glu Thr Ile Lys Met Phe
   50 55 60
   Gly Gly Asn Asp Val Thr Val Gln Tyr Lys Met Ile His Pro Tyr His
   65 70 75 80
   Thr Glu Lys His Leu Glu Lys Met Lys Arg Val Gly Lys Val Thr Asp
   85 90 95
   Phe Val Cys Glu Tyr Lys Phe Asp Thr Glu Phe Glu Arg Glu Ile Lys
   100 105 110
   Arg Glu Val Phe Lys Ile Val Arg Arg Gly Lys Asp Phe Gly Thr Leu
   115 120 125
   Gly Trp Phe Phe Arg Ala Phe Cys Tyr Ile Ala Ile Phe Phe Tyr Leu
   130 135 140
   Gln Tyr His Trp Val Thr Thr Gly Thr Ser Trp Leu Leu Ala Val Ala
   145 150 155 160
   Tyr Gly Ile Ser Gln Ala Met Ile Gly Met Asn Val Gln His Asp Ala
   165 170 175
   Asn His Gly Ala Thr Ser Lys Arg Pro Trp Val Asn Asp Met Leu Gly
   180 185 190
   Leu Gly Ala Asp Phe Ile Gly Gly Ser Lys Trp Leu Trp Gln Glu Gln
                   195                     200                     205
   His Trp Thr His His Ala Tyr Thr Asn His Ala Glu Met Asp Pro Asp
       210                     215                     220
   Ser Phe Gly Ala Glu Pro Met Leu Leu Phe Asn Asp Tyr Pro Leu Asp
   225                     230                     235                     240
   His Pro Ala Arg Thr Trp Leu His Arg Phe Gln Ala Phe Phe Tyr Met
                   245                     250                     255
   Pro Val Leu Ala Gly Tyr Trp Leu Ser Ala Val Phe Asn Pro Gln Ile
               260                     265                     270
   Leu Asp Leu Gln Gln Arg Gly Ala Leu Ser Val Gly Ile Arg Leu Asp
           275                     280                     285
   Asn Ala Phe Ile His Ser Arg Arg Lys Tyr Ala Val Phe Trp Arg Ala
       290                     295                     300
   Val Tyr Ile Ala Val Asn Val Ile Ala Pro Phe Tyr Thr Asn Ser Gly
   305                     310                     315                     320
   Leu Glu Trp Ser Trp Arg Val Phe Gly Asn Ile Met Leu Met Gly Val
                   325                     330                     335
   Ala Glu Ser Leu Ala Leu Ala Val Leu Phe Ser Leu Ser His Asn Phe
               340                     345                     350
   Glu Ser Ala Asp Arg Asp Pro Thr Ala Pro Leu Lys Lys Thr Gly Glu
           355                     360                     365
   Pro Val Asp Trp Phe Lys Thr Gln Val Glu Thr Ser Cys Thr Tyr Gly
       370                     375                     380
   Gly Phe Leu Ser Gly Cys Phe Thr Gly Gly Leu Asn Phe Gln Val Glu
   385                     390                     395                     400
   His His Leu Phe Pro Arg Met Ser Ser Ala Trp Tyr Pro Tyr Ile Ala
                   405                     410                     415
   Pro Lys Val Arg Glu Ile Cys Ala Lys His Gly Val His Tyr Ala Tyr
               420                     425                     430
   Tyr Pro Trp Ile His Gln Asn Phe Leu Ser Thr Val Arg Tyr Met His
           435                     440                     445
   Ala Ala Gly Thr Gly Ala Asn Trp Arg Gln Met Ala Arg Glu Asn Pro
       450                     455                     460
   Leu Thr Gly Arg Ala
   465
<210> 52
   <211> 3598
   <212> DNA
   <213> artificial sequence
<220>
   <221> misc_feature
   <223> Sequenz stellt eine pflanzliche Promotor-Terminator-Expressionska ssette in Vektor pUC19 dar
<400> 52
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatgcg gtgtgaaata ccgcacagat gcgtaaggag aaaataccgc atcaggcgcc     240
   attcgccatt caggctgcgc aactgttggg aagggcgatc ggtgcgggcc tcttcgctat     300
   tacgccagct ggcgaaaggg ggatgtgctg caaggcgatt aagttgggta acgccagggt 360
   tttcccagtc acgacgttgt aaaacgacgg ccagtgaatt cggcgcgccg agctcctcga 420
   gcaaatttac acattgccac taaacgtcta aacccttgta atttgttttt gttttactat 480
   gtgtgttatg tatttgattt gcgataaatt tttatatttg gtactaaatt tataacacct 540
   tttatgctaa cgtttgccaa cacttagcaa tttgcaagtt gattaattga ttctaaatta 600
   tttttgtctt ctaaatacat atactaatca actggaaatg taaatatttg ctaatatttc 660
   tactatagga gaattaaagt gagtgaatat ggtaccacaa ggtttggaga tttaattgtt 720
   gcaatgctgc atggatggca tatacaccaa acattcaata attcttgagg ataataatgg 780
   taccacacaa gatttgaggt gcatgaacgt cacgtggaca aaaggtttag taatttttca 840
   agacaacaat gttaccacac acaagttttg aggtgcatgc atggatgccc tgtggaaagt 900
   ttaaaaatat tttggaaatg atttgcatgg aagccatgtg taaaaccatg acatccactt 960
   ggaggatgca ataatgaaga aaactacaaa tttacatgca actagttatg catgtagtct 1020
   atataatgag gattttgcaa tactttcatt catacacact cactaagttt tacacgatta 1080
   taatttcttc atagccagcc caccgcggtg ggcggccgcc tgcagtctag aaggcctcct 1140
   gctttaatga gatatgcgag acgcctatga tcgcatgata tttgctttca attctgttgt 1200
   gcacgttgta aaaaacctga gcatgtgtag ctcagatcct taccgccggt ttcggttcat 1260
   tctaatgaat atatcacccg ttactatcgt atttttatga ataatattct ccgttcaatt 1320
   tactgattgt ccgtcgacga attcgagctc ggcgcgccaa gcttggcgta atcatggtca 1380
   tagctgtttc ctgtgtgaaa ttgttatccg ctcacaattc cacacaacat acgagccgga 1440
   agcataaagt gtaaagcctg gggtgcctaa tgagtgagct aactcacatt aattgcgttg 1500
   cgctcactgc ccgctttcca gtcgggaaac ctgtcgtgcc agctgcatta atgaatcggc 1560
   caacgcgcgg ggagaggcgg tttgcgtatt gggcgctctt ccgcttcctc gctcactgac 1620
   tcgctgcgct cggtcgttcg gctgcggcga gcggtatcag ctcactcaaa ggcggtaata 1680
   cggttatcca cagaatcagg ggataacgca ggaaagaaca tgtgagcaaa aggccagcaa 1740
   aaggccagga accgtaaaaa ggccgcgttg ctggcgtttt tccataggct ccgcccccct 1800
   gacgagcatc acaaaaatcg acgctcaagt cagaggtggc gaaacccgac aggactataa 1860
   agataccagg cgtttccccc tggaagctcc ctcgtgcgct ctcctgttcc gaccctgccg 1920
   cttaccggat acctgtccgc ctttctccct tcgggaagcg tggcgctttc tcatagctca 1980
   cgctgtaggt atctcagttc ggtgtaggtc gttcgctcca agctgggctg tgtgcacgaa 2040
   ccccccgttc agcccgaccg ctgcgcctta tccggtaact atcgtcttga gtccaacccg 2100
   gtaagacacg acttatcgcc actggcagca gccactggta acaggattag cagagcgagg 2160
   tatgtaggcg gtgctacaga gttcttgaag tggtggccta actacggcta cactagaagg 2220
   acagtatttg gtatctgcgc tctgctgaag ccagttacct tcggaaaaag agttggtagc 2280
   tcttgatccg gcaaacaaac caccgctggt agcggtggtt tttttgtttg caagcagcag 2340
   attacgcgca gaaaaaaagg atctcaagaa gatcctttga tcttttctac ggggtctgac 2400
   gctcagtgga acgaaaactc acgttaaggg attttggtca tgagattatc aaaaaggatc 2460
   ttcacctaga tccttttaaa ttaaaaatga agttttaaat caatctaaag tatatatgag 2520
   taaacttggt ctgacagtta ccaatgctta atcagtgagg cacctatctc agcgatctgt 2580
   ctatttcgtt catccatagt tgcctgactc cccgtcgtgt agataactac gatacgggag 2640
   ggcttaccat ctggccccag tgctgcaatg ataccgcgag acccacgctc accggctcca 2700
   gatttatcag caataaacca gccagccgga agggccgagc gcagaagtgg tcctgcaact 2760
   ttatccgcct ccatccagtc tattaattgt tgccgggaag ctagagtaag tagttcgcca 2820
   gttaatagtt tgcgcaacgt tgttgccatt gctacaggca tcgtggtgtc acgctcgtcg 2880
   tttggtatgg cttcattcag ctccggttcc caacgatcaa ggcgagttac atgatccccc 2940
   atgttgtgca aaaaagcggt tagctccttc ggtcctccga tcgttgtcag aagtaagttg 3000
   gccgcagtgt tatcactcat ggttatggca gcactgcata attctcttac tgtcatgcca 3060
   tccgtaagat gcttttctgt gactggtgag tactcaacca agtcattctg agaatagtgt 3120
   atgcggcgac cgagttgctc ttgcccggcg tcaatacggg ataataccgc gccacatagc 3180
   agaactttaa aagtgctcat cattggaaaa cgttcttcgg ggcgaaaact ctcaaggatc 3240
   ttaccgctgt tgagatccag ttcgatgtaa cccactcgtg cacccaactg atcttcagca 3300
   tcttttactt tcaccagcgt ttctgggtga gcaaaaacag gaaggcaaaa tgccgcaaaa 3360
   aagggaataa gggcgacacg gaaatgttga atactcatac tcttcctttt tcaatattat 3420
   tgaagcattt atcagggtta ttgtctcatg agcggataca tatttgaatg tatttagaaa 3480
   aataaacaaa taggggttcc gcgcacattt ccccgaaaag tgccacctga cgtctaagaa 3540
   accattatta tcatgacatt aacctataaa aataggcgta tcacgaggcc ctttcgtc 3598
<210> 53
   <211> 3590
   <212> DNA
   <213> artificial sequence
<220>
   <221> misc_feature
   <223> Sequenz stellt eine pflanzliche Promotor-Terminator-Expressionska ssette in Vektor pUC19 dar
<400> 53
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt cggcgcgccg agctcctcga 420
   gcaaatttac acattgccac taaacgtcta aacccttgta atttgttttt gttttactat 480
   gtgtgttatg tatttgattt gcgataaatt tttatatttg gtactaaatt tataacacct 540
   tttatgctaa cgtttgccaa cacttagcaa tttgcaagtt gattaattga ttctaaatta 600
   tttttgtctt ctaaatacat atactaatca actggaaatg taaatatttg ctaatatttc 660
   tactatagga gaattaaagt gagtgaatat ggtaccacaa ggtttggaga tttaattgtt 720
   gcaatgctgc atggatggca tatacaccaa acattcaata attcttgagg ataataatgg 780
   taccacacaa gatttgaggt gcatgaacgt cacgtggaca aaaggtttag taatttttca 840
   agacaacaat gttaccacac acaagttttg aggtgcatgc atggatgccc tgtggaaagt 900
   ttaaaaatat tttggaaatg atttgcatgg aagccatgtg taaaaccatg acatccactt 960
   ggaggatgca ataatgaaga aaactacaaa tttacatgca actagttatg catgtagtct 1020
   atataatgag gattttgcaa tactttcatt catacacact cactaagttt tacacgatta 1080
   taatttcttc atagccagcg gatccgatat cgggcccgct agcgttaacc ctgctttaat 1140
   gagatatgcg agacgcctat gatcgcatga tatttgcttt caattctgtt gtgcacgttg 1200
   taaaaaacct gagcatgtgt agctcagatc cttaccgccg gtttcggttc attctaatga 1260
   atatatcacc cgttactatc gtatttttat gaataatatt ctccgttcaa tttactgatt 1320
   gtccgtcgac gaattcgagc tcggcgcgcc aagcttggcg taatcatggt catagctgtt 1380
   tcctgtgtga aattgttatc cgctcacaat tccacacaac atacgagccg gaagcataaa 1440
   gtgtaaagcc tggggtgcct aatgagtgag ctaactcaca ttaattgcgt tgcgctcact 1500
   gcccgctttc cagtcgggaa acctgtcgtg ccagctgcat taatgaatcg gccaacgcgc 1560
   ggggagaggc ggtttgcgta ttgggcgctc ttccgcttcc tcgctcactg actcgctgcg 1620
   ctcggtcgtt cggctgcggc gagcggtatc agctcactca aaggcggtaa tacggttatc 1680
   cacagaatca ggggataacg caggaaagaa catgtgagca aaaggccagc aaaaggccag 1740
   gaaccgtaaa aaggccgcgt tgctggcgtt tttccatagg ctccgccccc ctgacgagca 1800
   tcacaaaaat cgacgctcaa gtcagaggtg gcgaaacccg acaggactat aaagatacca 1860
   ggcgtttccc cctggaagct ccctcgtgcg ctctcctgtt ccgaccctgc cgcttaccgg 1920
   atacctgtcc gcctttctcc cttcgggaag cgtggcgctt tctcatagct cacgctgtag 1980
   gtatctcagt tcggtgtagg tcgttcgctc caagctgggc tgtgtgcacg aaccccccgt 2040
   tcagcccgac cgctgcgcct tatccggtaa ctatcgtctt gagtccaacc cggtaagaca 2100
   cgacttatcg ccactggcag cagccactgg taacaggatt agcagagcga ggtatgtagg 2160
   cggtgctaca gagttcttga agtggtggcc taactacggc tacactagaa ggacagtatt 2220
   tggtatctgc gctctgctga agccagttac cttcggaaaa agagttggta gctcttgatc 2280
   cggcaaacaa accaccgctg gtagcggtgg tttttttgtt tgcaagcagc agattacgcg 2340
   cagaaaaaaa ggatctcaag aagatccttt gatcttttct acggggtctg acgctcagtg   2400
   gaacgaaaac tcacgttaag ggattttggt catgagatta tcaaaaagga tcttcaccta   2460
   gatcctttta aattaaaaat gaagttttaa atcaatctaa agtatatatg agtaaacttg   2520
   gtctgacagt taccaatgct taatcagtga ggcacctatc tcagcgatct gtctatttcg   2580
   ttcatccata gttgcctgac tccccgtcgt gtagataact acgatacggg agggcttacc   2640
   atctggcccc agtgctgcaa tgataccgcg agacccacgc tcaccggctc cagatttatc   2700
   agcaataaac cagccagccg gaagggccga gcgcagaagt ggtcctgcaa ctttatccgc   2760
   ctccatccag tctattaatt gttgccggga agctagagta agtagttcgc cagttaatag   2820
   tttgcgcaac gttgttgcca ttgctacagg catcgtggtg tcacgctcgt cgtttggtat   2880
   ggcttcattc agctccggtt cccaacgatc aaggcgagtt acatgatccc ccatgttgtg   2940
   caaaaaagcg gttagctcct tcggtcctcc gatcgttgtc agaagtaagt tggccgcagt   3000
   gttatcactc atggttatgg cagcactgca taattctctt actgtcatgc catccgtaag   3060
   atgcttttct gtgactggtg agtactcaac caagtcattc tgagaatagt gtatgcggcg   3120
   accgagttgc tcttgcccgg cgtcaatacg ggataatacc gcgccacata gcagaacttt   3180
   aaaagtgctc atcattggaa aacgttcttc ggggcgaaaa ctctcaagga tcttaccgct   3240
   gttgagatcc agttcgatgt aacccactcg tgcacccaac tgatcttcag catcttttac   3300
   tttcaccagc gtttctgggt gagcaaaaac aggaaggcaa aatgccgcaa aaaagggaat   3360
   aagggcgaca cggaaatgtt gaatactcat actcttcctt tttcaatatt attgaagcat   3420
   ttatcagggt tattgtctca tgagcggata catatttgaa tgtatttaga aaaataaaca   3480
   aataggggtt ccgcgcacat ttccccgaaa agtgccacct gacgtctaag aaaccattat   3540
   tatcatgaca ttaacctata aaaataggcg tatcacgagg ccctttcgtc              3590
<210> 54
   <211> 3584
   <212> DNA
   <213> artificial sequence
<220>
   <221> misc_feature
   <223> Sequenz stellt eine pflanzliche Promotor-Terminator-Expressionska ssette in Vektor pUC19 dar
<400> 54
   tcgcgcgttt cggtgatgac ggtgaaaacc tctgacacat gcagctcccg gagacggtca     60
   cagcttgtct gtaagcggat gccgggagca gacaagcccg tcagggcgcg tcagcgggtg    120
   ttggcgggtg tcggggctgg cttaactatg cggcatcaga gcagattgta ctgagagtgc    180
   accatatgcg gtgtgaaata ccgcacagat gcgtaaggag aaaataccgc atcaggcgcc    240
   attcgccatt caggctgcgc aactgttggg aagggcgatc ggtgcgggcc tcttcgctat    300
   tacgccagct ggcgaaaggg ggatgtgctg caaggcgatt aagttgggta acgccagggt    360
   tttcccagtc acgacgttgt aaaacgacgg ccagtgaatt cggcgcgccg agctcctcga    420
   gcaaatttac acattgccac taaacgtcta aacccttgta atttgttttt gttttactat    480
   gtgtgttatg tatttgattt gcgataaatt tttatatttg gtactaaatt tataacacct    540
   tttatgctaa cgtttgccaa cacttagcaa tttgcaagtt gattaattga ttctaaatta    600
   tttttgtctt ctaaatacat atactaatca actggaaatg taaatatttg ctaatatttc    660
   tactatagga gaattaaagt gagtgaatat ggtaccacaa ggtttggaga tttaattgtt    720
   gcaatgctgc atggatggca tatacaccaa acattcaata attcttgagg ataataatgg    780
   taccacacaa gatttgaggt gcatgaacgt cacgtggaca aaaggtttag taatttttca    840
   agacaacaat gttaccacac acaagttttg aggtgcatgc atggatgccc tgtggaaagt    900
   ttaaaaatat tttggaaatg atttgcatgg aagccatgtg taaaaccatg acatccactt    960
   ggaggatgca ataatgaaga aaactacaaa tttacatgca actagttatg catgtagtct   1020
   atataatgag gattttgcaa tactttcatt catacacact cactaagttt tacacgatta   1080
   taatttcttc atagccagca gatctgccgg catcgatccc gggccatggc ctgctttaat   1140
   gagatatgcg agacgcctat gatcgcatga tatttgcttt caattctgtt gtgcacgttg   1200
   taaaaaacct gagcatgtgt agctcagatc cttaccgccg gtttcggttc attctaatga   1260
   atatatcacc cgttactatc gtatttttat gaataatatt ctccgttcaa tttactgatt   1320
   gtccgtcgac gagctcggcg cgccaagctt ggcgtaatca tggtcatagc tgtttcctgt   1380
   gtgaaattgt tatccgctca caattccaca caacatacga gccggaagca taaagtgtaa   1440
   agcctggggt gcctaatgag tgagctaact cacattaatt gcgttgcgct cactgcccgc   1500
   tttccagtcg ggaaacctgt cgtgccagct gcattaatga atcggccaac gcgcggggag   1560
   aggcggtttg cgtattgggc gctcttccgc ttcctcgctc actgactcgc tgcgctcggt   1620
   cgttcggctg cggcgagcgg tatcagctca ctcaaaggcg gtaatacggt tatccacaga   1680
   atcaggggat aacgcaggaa agaacatgtg agcaaaaggc cagcaaaagg ccaggaaccg   1740
   taaaaaggcc gcgttgctgg cgtttttcca taggctccgc ccccctgacg agcatcacaa   1800
   aaatcgacgc tcaagtcaga ggtggcgaaa cccgacagga ctataaagat accaggcgtt   1860
   tccccctgga agctccctcg tgcgctctcc tgttccgacc ctgccgctta ccggatacct   1920
   gtccgccttt ctcccttcgg gaagcgtggc gctttctcat agctcacgct gtaggtatct   1980
   cagttcggtg taggtcgttc gctccaagct gggctgtgtg cacgaacccc ccgttcagcc   2040
   cgaccgctgc gccttatccg gtaactatcg tcttgagtcc aacccggtaa gacacgactt   2100
   atcgccactg gcagcagcca ctggtaacag gattagcaga gcgaggtatg taggcggtgc   2160
   tacagagttc ttgaagtggt ggcctaacta cggctacact agaaggacag tatttggtat   2220
   ctgcgctctg ctgaagccag ttaccttcgg aaaaagagtt ggtagctctt gatccggcaa   2280
   acaaaccacc gctggtagcg gtggtttttt tgtttgcaag cagcagatta cgcgcagaaa   2340
   aaaaggatct caagaagatc ctttgatctt ttctacgggg tctgacgctc agtggaacga   2400
   aaactcacgt taagggattt tggtcatgag attatcaaaa aggatcttca cctagatcct   2460
   tttaaattaa aaatgaagtt ttaaatcaat ctaaagtata tatgagtaaa cttggtctga   2520
   cagttaccaa tgcttaatca gtgaggcacc tatctcagcg atctgtctat ttcgttcatc   2580
   catagttgcc tgactccccg tcgtgtagat aactacgata cgggagggct taccatctgg   2640
   ccccagtgct gcaatgatac cgcgagaccc acgctcaccg gctccagatt tatcagcaat   2700
   aaaccagcca gccggaaggg ccgagcgcag aagtggtcct gcaactttat ccgcctccat   2760
   ccagtctatt aattgttgcc gggaagctag agtaagtagt tcgccagtta atagtttgcg   2820
   caacgttgtt gccattgcta caggcatcgt ggtgtcacgc tcgtcgtttg gtatggcttc   2880
   attcagctcc ggttcccaac gatcaaggcg agttacatga tcccccatgt tgtgcaaaaa   2940
   agcggttagc tccttcggtc ctccgatcgt tgtcagaagt aagttggccg cagtgttatc   3000
   actcatggtt atggcagcac tgcataattc tcttactgtc atgccatccg taagatgctt   3060
   ttctgtgact ggtgagtact caaccaagtc attctgagaa tagtgtatgc ggcgaccgag   3120
   ttgctcttgc ccggcgtcaa tacgggataa taccgcgcca catagcagaa ctttaaaagt   3180
   gctcatcatt ggaaaacgtt cttcggggcg aaaactctca aggatcttac cgctgttgag   3240
   atccagttcg atgtaaccca ctcgtgcacc caactgatct tcagcatctt ttactttcac   3300
   cagcgtttct gggtgagcaa aaacaggaag gcaaaatgcc gcaaaaaagg gaataagggc   3360
   gacacggaaa tgttgaatac tcatactctt cctttttcaa tattattgaa gcatttatca   3420
   gggttattgt ctcatgagcg gatacatatt tgaatgtatt tagaaaaata aacaaatagg   3480
   ggttccgcgc acatttcccc gaaaagtgcc acctgacgtc taagaaacca ttattatcat   3540
   gacattaacc tataaaaata ggcgtatcac gaggcccttt cgtc                    3584
<210> 55
   <211> 4507
   <212> DNA
   <213> artificial sequence
<220>
   <221> misc_feature
   <223> Sequenz stellt eine pflanzliche Promotor-Terminator-Expressionska ssette in Vektor pUC19 dar
<400> 55
   tcgcgcgttt cggtgatgac ggtgaaaacc tctgacacat gcagctcccg gagacggtca     60
   cagcttgtct gtaagcggat gccgggagca gacaagcccg tcagggcgcg tcagcgggtg    120
   ttggcgggtg tcggggctgg cttaactatg cggcatcaga gcagattgta ctgagagtgc    180
   accatatgcg gtgtgaaata ccgcacagat gcgtaaggag aaaataccgc atcaggcgcc    240
   attcgccatt caggctgcgc aactgttggg aagggcgatc ggtgcgggcc tcttcgctat    300
   tacgccagct ggcgaaaggg ggatgtgctg caaggcgatt aagttgggta acgccagggt    360
   tttcccagtc acgacgttgt aaaacgacgg ccagtgaatt cggcgcgccg agctcctcga    420
   gcaaatttac acattgccac taaacgtcta aacccttgta atttgttttt gttttactat    480
   gtgtgttatg tatttgattt gcgataaatt tttatatttg gtactaaatt tataacacct    540
   tttatgctaa cgtttgccaa cacttagcaa tttgcaagtt gattaattga ttctaaatta    600
   tttttgtctt ctaaatacat atactaatca actggaaatg taaatatttg ctaatatttc    660
   tactatagga gaattaaagt gagtgaatat ggtaccacaa ggtttggaga tttaattgtt    720
   gcaatgctgc atggatggca tatacaccaa acattcaata attcttgagg ataataatgg    780
   taccacacaa gatttgaggt gcatgaacgt cacgtggaca aaaggtttag taatttttca    840
   agacaacaat gttaccacac acaagttttg aggtgcatgc atggatgccc tgtggaaagt    900
   ttaaaaatat tttggaaatg atttgcatgg aagccatgtg taaaaccatg acatccactt    960
   ggaggatgca ataatgaaga aaactacaaa tttacatgca actagttatg catgtagtct   1020
   atataatgag gattttgcaa tactttcatt catacacact cactaagttt tacacgatta   1080
   taatttcttc atagccagcc caccgcggtg ggcggccgcc tgcagtctag aaggcctcct   1140
   gctttaatga gatatgcgag acgcctatga tcgcatgata tttgctttca attctgttgt   1200
   gcacgttgta aaaaacctga gcatgtgtag ctcagatcct taccgccggt ttcggttcat   1260
   tctaatgaat atatcacccg ttactatcgt atttttatga ataatattct ccgttcaatt   1320
   tactgattgt ccgtcgagca aatttacaca ttgccactaa acgtctaaac ccttgtaatt   1380
   tgtttttgtt ttactatgtg tgttatgtat ttgatttgcg ataaattttt atatttggta   1440
   ctaaatttat aacacctttt atgctaacgt ttgccaacac ttagcaattt gcaagttgat   1500
   taattgattc taaattattt ttgtcttcta aatacatata ctaatcaact ggaaatgtaa   1560
   atatttgcta atatttctac tataggagaa ttaaagtgag tgaatatggt accacaaggt   1620
   ttggagattt aattgttgca atgctgcatg gatggcatat acaccaaaca ttcaataatt   1680
   cttgaggata ataatggtac cacacaagat ttgaggtgca tgaacgtcac gtggacaaaa   1740
   ggtttagtaa tttttcaaga caacaatgtt accacacaca agttttgagg tgcatgcatg   1800
   gatgccctgt ggaaagttta aaaatatttt ggaaatgatt tgcatggaag ccatgtgtaa   1860
   aaccatgaca tccacttgga ggatgcaata atgaagaaaa ctacaaattt acatgcaact   1920
   agttatgcat gtagtctata taatgaggat tttgcaatac tttcattcat acacactcac   1980
   taagttttac acgattataa tttcttcata gccagcggat ccgatatcgg gcccgctagc   2040
   gttaaccctg ctttaatgag atatgcgaga cgcctatgat cgcatgatat ttgctttcaa   2100
   ttctgttgtg cacgttgtaa aaaacctgag catgtgtagc tcagatcctt accgccggtt   2160
   tcggttcatt ctaatgaata tatcacccgt tactatcgta tttttatgaa taatattctc   2220
   cgttcaattt actgattgtc cgtcgacgaa ttcgagctcg gcgcgccaag cttggcgtaa   2280
   tcatggtcat agctgtttcc tgtgtgaaat tgttatccgc tcacaattcc acacaacata   2340
   cgagccggaa gcataaagtg taaagcctgg ggtgcctaat gagtgagcta actcacatta   2400
   attgcgttgc gctcactgcc cgctttccag tcgggaaacc tgtcgtgcca gctgcattaa   2460
   tgaatcggcc aacgcgcggg gagaggcggt ttgcgtattg ggcgctcttc cgcttcctcg   2520
   ctcactgact cgctgcgctc ggtcgttcgg ctgcggcgag cggtatcagc tcactcaaag   2580
   gcggtaatac ggttatccac agaatcaggg gataacgcag gaaagaacat gtgagcaaaa   2640
   ggccagcaaa aggccaggaa ccgtaaaaag gccgcgttgc tggcgttttt ccataggctc   2700
   cgcccccctg acgagcatca caaaaatcga cgctcaagtc agaggtggcg aaacccgaca   2760
   ggactataaa gataccaggc gtttccccct ggaagctccc tcgtgcgctc tcctgttccg   2820
   accctgccgc ttaccggata cctgtccgcc tttctccctt cgggaagcgt ggcgctttct   2880
   catagctcac gctgtaggta tctcagttcg gtgtaggtcg ttcgctccaa gctgggctgt   2940
   gtgcacgaac cccccgttca gcccgaccgc tgcgccttat ccggtaacta tcgtcttgag   3000
   tccaacccgg taagacacga cttatcgcca ctggcagcag ccactggtaa caggattagc   3060
   agagcgaggt atgtaggcgg tgctacagag ttcttgaagt ggtggcctaa ctacggctac   3120
   actagaagga cagtatttgg tatctgcgct ctgctgaagc cagttacctt cggaaaaaga   3180
   gttggtagct cttgatccgg caaacaaacc accgctggta gcggtggttt ttttgtttgc   3240
   aagcagcaga ttacgcgcag aaaaaaagga tctcaagaag atcctttgat cttttctacg   3300
   gggtctgacg ctcagtggaa cgaaaactca cgttaaggga ttttggtcat gagattatca   3360
   aaaaggatct tcacctagat ccttttaaat taaaaatgaa gttttaaatc aatctaaagt   3420
   atatatgagt aaacttggtc tgacagttac caatgcttaa tcagtgaggc acctatctca   3480
   gcgatctgtc tatttcgttc atccatagtt gcctgactcc ccgtcgtgta gataactacg   3540
   atacgggagg gcttaccatc tggccccagt gctgcaatga taccgcgaga cccacgctca   3600
   ccggctccag atttatcagc aataaaccag ccagccggaa gggccgagcg cagaagtggt   3660
   cctgcaactt tatccgcctc catccagtct attaattgtt gccgggaagc tagagtaagt   3720
   agttcgccag ttaatagttt gcgcaacgtt gttgccattg ctacaggcat cgtggtgtca   3780
   cgctcgtcgt ttggtatggc ttcattcagc tccggttccc aacgatcaag gcgagttaca   3840
   tgatccccca tgttgtgcaa aaaagcggtt agctccttcg gtcctccgat cgttgtcaga   3900
   agtaagttgg ccgcagtgtt atcactcatg gttatggcag cactgcataa ttctcttact   3960
   gtcatgccat ccgtaagatg cttttctgtg actggtgagt actcaaccaa gtcattctga   4020
   gaatagtgta tgcggcgacc gagttgctct tgcccggcgt caatacggga taataccgcg   4080
   ccacatagca gaactttaaa agtgctcatc attggaaaac gttcttcggg gcgaaaactc   4140
   tcaaggatct taccgctgtt gagatccagt tcgatgtaac ccactcgtgc acccaactga   4200
   tcttcagcat cttttacttt caccagcgtt tctgggtgag caaaaacagg aaggcaaaat   4260
   gccgcaaaaa agggaataag ggcgacacgg aaatgttgaa tactcatact cttccttttt   4320
   caatattatt gaagcattta tcagggttat tgtctcatga gcggatacat atttgaatgt   4380
   atttagaaaa ataaacaaat aggggttccg cgcacatttc cccgaaaagt gccacctgac   4440
   gtctaagaaa ccattattat catgacatta acctataaaa ataggcgtat cacgaggccc   4500
   tttcgtc                                                              4507
<210> 56
   <211> 17752
   <212> DNA
   <213> Phaeodactylum tricornutum, Physcomitrella patens
<220>
   <221> CDS
   <222> (11543)..(12415)
   <223> Delta-6-Elongase
<220>
   <221> CDS
   <222> (13313)..(14890)
   <223> Delta-6-Desaturase
<220>
   <221> CDS
   <222> (15791)..(17200)
   <223> Delta-5-Desaturase
<400> 56
   gatctggcgc cggccagcga gacgagcaag attggccgcc gcccgaaacg atccgacagc     60
   gcgcccagca caggtgcgca ggcaaattgc accaacgcat acagcgccag cagaatgcca    120
   tagtgggcgg tgacgtcgtt cgagtgaacc agatcgcgca ggaggcccgg cagcaccggc    180
   ataatcaggc cgatgccgac agcgtcgagc gcgacagtgc tcagaattac gatcaggggt    240
   atgttgggtt tcacgtctgg cctccggacc agcctccgct ggtccgattg aacgcgcgga    300
   ttctttatca ctgataagtt ggtggacata ttatgtttat cagtgataaa gtgtcaagca    360
   tgacaaagtt gcagccgaat acagtgatcc gtgccgccct ggacctgttg aacgaggtcg    420
   gcgtagacgg tctgacgaca cgcaaactgg cggaacggtt gggggttcag cagccggcgc 480
   tttactggca cttcaggaac aagcgggcgc tgctcgacgc actggccgaa gccatgctgg 540
   cggagaatca tacgcattcg gtgccgagag ccgacgacga ctggcgctca tttctgatcg 600
   ggaatgcccg cagcttcagg caggcgctgc tcgcctaccg cgatggcgcg cgcatccatg 660
   ccggcacgcg accgggcgca ccgcagatgg aaacggccga cgcgcagctt cgcttcctct 720
   gcgaggcggg tttttcggcc ggggacgccg tcaatgcgct gatgacaatc agctacttca 780
   ctgttggggc cgtgcttgag gagcaggccg gcgacagcga tgccggcgag cgcggcggca 840
   ccgttgaaca ggctccgctc tcgccgctgt tgcgggccgc gatagacgcc ttcgacgaag 900
   ccggtccgga cgcagcgttc gagcagggac tcgcggtgat tgtcgatgga ttggcgaaaa 960
   ggaggctcgt tgtcaggaac gttgaaggac cgagaaaggg tgacgattga tcaggaccgc 1020
   tgccggagcg caacccactc actacagcag agccatgtag acaacatccc ctcccccttt 1080
   ccaccgcgtc agacgcccgt agcagcccgc tacgggcttt ttcatgccct gccctagcgt 1140
   ccaagcctca cggccgcgct cggcctctct ggcggccttc tggcgctctt ccgcttcctc 1200
   gctcactgac tcgctgcgct cggtcgttcg gctgcggcga gcggtatcag ctcactcaaa 1260
   ggcggtaata cggttatcca cagaatcagg ggataacgca ggaaagaaca tgtgagcaaa 1320
   aggccagcaa aaggccagga accgtaaaaa ggccgcgttg ctggcgtttt tccataggct 1380
   ccgcccccct gacgagcatc acaaaaatcg acgctcaagt cagaggtggc gaaacccgac 1440
   aggactataa agataccagg cgtttccccc tggaagctcc ctcgtgcgct ctcctgttcc 1500
   gaccctgccg cttaccggat acctgtccgc ctttctccct tcgggaagcg tggcgctttt 1560
   ccgctgcata accctgcttc ggggtcatta tagcgatttt ttcggtatat ccatcctttt 1620
   tcgcacgata tacaggattt tgccaaaggg ttcgtgtaga ctttccttgg tgtatccaac 1680
   ggcgtcagcc gggcaggata ggtgaagtag gcccacccgc gagcgggtgt tccttcttca 1740
   ctgtccctta ttcgcacctg gcggtgctca acgggaatcc tgctctgcga ggctggccgg 1800
   ctaccgccgg cgtaacagat gagggcaagc ggatggctga tgaaaccaag ccaaccagga 1860
   agggcagccc acctatcaag gtgtactgcc ttccagacga acgaagagcg attgaggaaa 1920
   aggcggcggc ggccggcatg agcctgtcgg cctacctgct ggccgtcggc cagggctaca 1980
   aaatcacggg cgtcgtggac tatgagcacg tccgcgagct ggcccgcatc aatggcgacc 2040
   tgggccgcct gggcggcctg ctgaaactct ggctcaccga cgacccgcgc acggcgcggt 2100
   tcggtgatgc cacgatcctc gccctgctgg cgaagatcga agagaagcag gacgagcttg 2160
   gcaaggtcat gatgggcgtg gtccgcccga gggcagagcc atgacttttt tagccgctaa 2220
   aacggccggg gggtgcgcgt gattgccaag cacgtcccca tgcgctccat caagaagagc 2280
   gacttcgcgg agctggtgaa gtacatcacc gacgagcaag gcaagaccga gcgcctttgc 2340
   gacgctcacc gggctggttg ccctcgccgc tgggctggcg gccgtctatg gccctgcaaa 2400
   cgcgccagaa acgccgtcga agccgtgtgc gagacaccgc ggccgccggc gttgtggata 2460
   cctcgcggaa aacttggccc tcactgacag atgaggggcg gacgttgaca cttgaggggc 2520
   cgactcaccc ggcgcggcgt tgacagatga ggggcaggct cgatttcggc cggcgacgtg 2580
   gagctggcca gcctcgcaaa tcggcgaaaa cgcctgattt tacgcgagtt tcccacagat 2640
   gatgtggaca agcctgggga taagtgccct gcggtattga cacttgaggg gcgcgactac 2700
   tgacagatga ggggcgcgat ccttgacact tgaggggcag agtgctgaca gatgaggggc 2760
   gcacctattg acatttgagg ggctgtccac aggcagaaaa tccagcattt gcaagggttt 2820
   ccgcccgttt ttcggccacc gctaacctgt cttttaacct gcttttaaac caatatttat 2880
   aaaccttgtt tttaaccagg gctgcgccct gtgcgcgtga ccgcgcacgc cgaagggggg 2940
   tgccccccct tctcgaaccc tcccggcccg ctaacgcggg cctcccatcc ccccaggggc 3000
   tgcgcccctc ggccgcgaac ggcctcaccc caaaaatggc agcgctggca gtccttgcca 3060
   ttgccgggat cggggcagta acgggatggg cgatcagccc gagcgcgacg cccggaagca 3120
   ttgacgtgcc gcaggtgctg gcatcgacat tcagcgacca ggtgccgggc agtgagggcg 3180
   gcggcctggg tggcggcctg cccttcactt cggccgtcgg ggcattcacg gacttcatgg 3240
   cggggccggc aatttttacc ttgggcattc ttggcatagt ggtcgcgggt gccgtgctcg 3300
   tgttcggggg tgcgataaac ccagcgaacc atttgaggtg ataggtaaga ttataccgag 3360
   gtatgaaaac gagaattgga cctttacaga attactctat gaagcgccat atttaaaaag 3420
   ctaccaagac gaagaggatg aagaggatga ggaggcagat tgccttgaat atattgacaa 3480
   tactgataag ataatatatc ttttatatag aagatatcgc cgtatgtaag gatttcaggg 3540
   ggcaaggcat aggcagcgcg cttatcaata tatctataga atgggcaaag cataaaaact 3600
   tgcatggact aatgcttgaa acccaggaca ataaccttat agcttgtaaa ttctatcata 3660
   attgggtaat gactccaact tattgatagt gttttatgtt cagataatgc ccgatgactt 3720
   tgtcatgcag ctccaccgat tttgagaacg acagcgactt ccgtcccagc cgtgccaggt 3780
   gctgcctcag attcaggtta tgccgctcaa ttcgctgcgt atatcgcttg ctgattacgt 3840
   gcagctttcc cttcaggcgg gattcataca gcggccagcc atccgtcatc catatcacca 3900
   cgtcaaaggg tgacagcagg ctcataagac gccccagcgt cgccatagtg cgttcaccga 3960
   atacgtgcgc aacaaccgtc ttccggagac tgtcatacgc gtaaaacagc cagcgctggc 4020
   gcgatttagc cccgacatag ccccactgtt cgtccatttc cgcgcagacg atgacgtcac 4080
   tgcccggctg tatgcgcgag gttaccgact gcggcctgag ttttttaagt gacgtaaaat 4140
   cgtgttgagg ccaacgccca taatgcgggc tgttgcccgg catccaacgc cattcatggc 4200
   catatcaatg attttctggt gcgtaccggg ttgagaagcg gtgtaagtga actgcagttg 4260
   ccatgtttta cggcagtgag agcagagata gcgctgatgt ccggcggtgc ttttgccgtt 4320
   acgcaccacc ccgtcagtag ctgaacagga gggacagctg atagacacag aagccactgg 4380
   agcacctcaa aaacaccatc atacactaaa tcagtaagtt ggcagcatca cccataattg 4440
   tggtttcaaa atcggctccg tcgatactat gttatacgcc aactttgaaa acaactttga 4500
   aaaagctgtt ttctggtatt taaggtttta gaatgcaagg aacagtgaat tggagttcgt 4560
   cttgttataa ttagcttctt ggggtatctt taaatactgt agaaaagagg aaggaaataa 4620
   taaatggcta aaatgagaat atcaccggaa ttgaaaaaac tgatcgaaaa ataccgctgc 4680
   gtaaaagata cggaaggaat gtctcctgct aaggtatata agctggtggg agaaaatgaa 4740
   aacctatatt taaaaatgac ggacagccgg tataaaggga ccacctatga tgtggaacgg 4800
   gaaaaggaca tgatgctatg gctggaagga aagctgcctg ttccaaaggt cctgcacttt 4860
   gaacggcatg atggctggag caatctgctc atgagtgagg ccgatggcgt cctttgctcg 4920
   gaagagtatg aagatgaaca aagccctgaa aagattatcg agctgtatgc ggagtgcatc 4980
   aggctctttc actccatcga catatcggat tgtccctata cgaatagctt agacagccgc 5040
   ttagccgaat tggattactt actgaataac gatctggccg atgtggattg cgaaaactgg 5100
   gaagaagaca ctccatttaa agatccgcgc gagctgtatg attttttaaa gacggaaaag 5160
   cccgaagagg aacttgtctt ttcccacggc gacctgggag acagcaacat ctttgtgaaa 5220
   gatggcaaag taagtggctt tattgatctt gggagaagcg gcagggcgga caagtggtat 5280
   gacattgcct tctgcgtccg gtcgatcagg gaggatatcg gggaagaaca gtatgtcgag 5340
   ctattttttg acttactggg gatcaagcct gattgggaga aaataaaata ttatatttta 5400
   ctggatgaat tgttttagta cctagatgtg gcgcaacgat gccggcgaca agcaggagcg 5460
   caccgacttc ttccgcatca agtgttttgg ctctcaggcc gaggcccacg gcaagtattt 5520
   gggcaagggg tcgctggtat tcgtgcaggg caagattcgg aataccaagt acgagaagga 5580
   cggccagacg gtctacggga ccgacttcat tgccgataag gtggattatc tggacaccaa 5640
   ggcaccaggc gggtcaaatc aggaataagg gcacattgcc ccggcgtgag tcggggcaat 5700
   cccgcaagga gggtgaatga atcggacgtt tgaccggaag gcatacaggc aagaactgat 5760
   cgacgcgggg ttttccgccg aggatgccga aaccatcgca agccgcaccg tcatgcgtgc 5820
   gccccgcgaa accttccagt ccgtcggctc gatggtccag caagctacgg ccaagatcga 5880
   gcgcgacagc gtgcaactgg ctccccctgc cctgcccgcg ccatcggccg ccgtggagcg 5940
   ttcgcgtcgt ctcgaacagg aggcggcagg tttggcgaag tcgatgacca tcgacacgcg 6000
   aggaactatg acgaccaaga agcgaaaaac cgccggcgag gacctggcaa aacaggtcag 6060
   cgaggccaag caggccgcgt tgctgaaaca cacgaagcag cagatcaagg aaatgcagct 6120
   ttccttgttc gatattgcgc cgtggccgga cacgatgcga gcgatgccaa acgacacggc 6180
   ccgctctgcc ctgttcacca cgcgcaacaa gaaaatcccg cgcgaggcgc tgcaaaacaa 6240
   ggtcattttc cacgtcaaca aggacgtgaa gatcacctac accggcgtcg agctgcgggc 6300
   cgacgatgac gaactggtgt ggcagcaggt gttggagtac gcgaagcgca cccctatcgg 6360
   cgagccgatc accttcacgt tctacgagct ttgccaggac ctgggctggt cgatcaatgg 6420
   ccggtattac acgaaggccg aggaatgcct gtcgcgccta caggcgacgg cgatgggctt 6480
   cacgtccgac cgcgttgggc acctggaatc ggtgtcgctg ctgcaccgct tccgcgtcct 6540
   ggaccgtggc aagaaaacgt cccgttgcca ggtcctgatc gacgaggaaa tcgtcgtgct 6600
   gtttgctggc gaccactaca cgaaattcat atgggagaag taccgcaagc tgtcgccgac 6660
   ggcccgacgg atgttcgact atttcagctc gcaccgggag ccgtacccgc tcaagctgga 6720
   aaccttccgc ctcatgtgcg gatcggattc cacccgcgtg aagaagtggc gcgagcaggt 6780
   cggcgaagcc tgcgaagagt tgcgaggcag cggcctggtg gaacacgcct gggtcaatga 6840
   tgacctggtg cattgcaaac gctagggcct tgtggggtca gttccggctg ggggttcagc 6900
   agccagcgct ttactggcat ttcaggaaca agcgggcact gctcgacgca cttgcttcgc   6960
   tcagtatcgc tcgggacgca cggcgcgctc tacgaactgc cgataaacag aggattaaaa   7020
   ttgacaattg tgattaaggc tcagattcga cggcttggag cggccgacgt gcaggatttc   7080
   cgcgagatcc gattgtcggc cctgaagaaa gctccagaga tgttcgggtc cgtttacgag   7140
   cacgaggaga aaaagcccat ggaggcgttc gctgaacggt tgcgagatgc cgtggcattc   7200
   ggcgcctaca tcgacggcga gatcattggg ctgtcggtct tcaaacagga ggacggcccc   7260
   aaggacgctc acaaggcgca tctgtccggc gttttcgtgg agcccgaaca gcgaggccga   7320
   ggggtcgccg gtatgctgct gcgggcgttg ccggcgggtt tattgctcgt gatgatcgtc   7380
   cgacagattc caacgggaat ctggtggatg cgcatcttca tcctcggcgc acttaatatt   7440
   tcgctattct ggagcttgtt gtttatttcg gtctaccgcc tgccgggcgg ggtcgcggcg   7500
   acggtaggcg ctgtgcagcc gctgatggtc gtgttcatct ctgccgctct gctaggtagc   7560
   ccgatacgat tgatggcggt cctgggggct atttgcggaa ctgcgggcgt ggcgctgttg   7620
   gtgttgacac caaacgcagc gctagatcct gtcggcgtcg cagcgggcct ggcgggggcg   7680
   gtttccatgg cgttcggaac cgtgctgacc cgcaagtggc aacctcccgt gcctctgctc   7740
   acctttaccg cctggcaact ggcggccgga ggacttctgc tcgttccagt agctttagtg   7800
   tttgatccgc caatcccgat gcctacagga accaatgttc tcggcctggc gtggctcggc   7860
   ctgatcggag cgggtttaac ctacttcctt tggttccggg ggatctcgcg actcgaacct   7920
   acagttgttt ccttactggg ctttctcagc cccagatctg gggtcgatca gccggggatg   7980
   catcaggccg acagtcggaa cttcgggtcc ccgacctgta ccattcggtg agcaatggat   8040
   aggggagttg atatcgtcaa cgttcacttc taaagaaata gcgccactca gcttcctcag   8100
   cggctttatc cagcgatttc ctattatgtc ggcatagttc tcaagatcga cagcctgtca   8160
   cggttaagcg agaaatgaat aagaaggctg ataattcgga tctctgcgag ggagatgata   8220
   tttgatcaca ggcagcaacg ctctgtcatc gttacaatca acatgctacc ctccgcgaga   8280
   tcatccgtgt ttcaaacccg gcagcttagt tgccgttctt ccgaatagca tcggtaacat   8340
   gagcaaagtc tgccgcctta caacggctct cccgctgacg ccgtcccgga ctgatgggct   8400
   gcctgtatcg agtggtgatt ttgtgccgag ctgccggtcg gggagctgtt ggctggctgg   8460
   tggcaggata tattgtggtg taaacaaatt gacgcttaga caacttaata acacattgcg   8520
   gacgttttta atgtactggg gtggtttttc ttttcaccag tgagacgggc aacagctgat   8580
   tgcccttcac cgcctggccc tgagagagtt gcagcaagcg gtccacgctg gtttgcccca   8640
   gcaggcgaaa atcctgtttg atggtggttc cgaaatcggc aaaatccctt ataaatcaaa   8700
   agaatagccc gagatagggt tgagtgttgt tccagtttgg aacaagagtc cactattaaa   8760
   gaacgtggac tccaacgtca aagggcgaaa aaccgtctat cagggcgatg gcccactacg   8820
   tgaaccatca cccaaatcaa gttttttggg gtcgaggtgc cgtaaagcac taaatcggaa   8880
   ccctaaaggg agcccccgat ttagagcttg acggggaaag ccggcgaacg tggcgagaaa   8940
   ggaagggaag aaagcgaaag gagcgggcgc cattcaggct gcgcaactgt tgggaagggc   9000
   gatcggtgcg ggcctcttcg ctattacgcc agctggcgaa agggggatgt gctgcaaggc   9060
   gattaagttg ggtaacgcca gggttttccc agtcacgacg ttgtaaaacg acggccagtg   9120
   aattaattcc catcttgaaa gaaatatagt ttaaatattt attgataaaa taacaagtca   9180
   ggtattatag tccaagcaaa aacataaatt tattgatgca agtttaaatt cagaaatatt   9240
   tcaataactg attatatcag ctggtacatt gccgtagatg aaagactgag tgcgatatta   9300
   tgtgtaatac ataaattgat gatatagcta gcttagctca tcgggggatc cgtcgaagct   9360
   agcttgggtc ccgctcagaa gaactcgtca agaaggcgat agaaggcgat gcgctgcgaa   9420
   tcgggagcgg cgataccgta aagcacgagg aagcggtcag cccattcgcc gccaagctct   9480
   tcagcaatat cacgggtagc caacgctatg tcctgatagc ggtccgccac acccagccgg   9540
   ccacagtcga tgaatccaga aaagcggcca ttttccacca tgatattcgg caagcaggca   9600
   tcgccatggg tcacgacgag atcctcgccg tcgggcatgc gcgccttgag cctggcgaac   9660
   agttcggctg gcgcgagccc ctgatgctct tcgtccagat catcctgatc gacaagaccg   9720
   gcttccatcc gagtacgtgc tcgctcgatg cgatgtttcg cttggtggtc gaatgggcag   9780
   gtagccggat caagcgtatg cagccgccgc attgcatcag ccatgatgga tactttctcg   9840
   gcaggagcaa ggtgagatga caggagatcc tgccccggca cttcgcccaa tagcagccag   9900
   tcccttcccg cttcagtgac aacgtcgagc acagctgcgc aaggaacgcc cgtcgtggcc   9960
   agccacgata gccgcgctgc ctcgtcctgc agttcattca gggcaccgga caggtcggtc  10020
   ttgacaaaaa gaaccgggcg cccctgcgct gacagccgga acacggcggc atcagagcag  10080
   ccgattgtct gttgtgccca gtcatagccg aatagcctct ccacccaagc ggccggagaa  10140
   cctgcgtgca atccatcttg ttcaatccaa gctcccatgg gccctcgact agagtcgaga  10200
   tctggattga gagtgaatat gagactctaa ttggataccg aggggaattt atggaacgtc  10260
   agtggagcat ttttgacaag aaatatttgc tagctgatag tgaccttagg cgacttttga  10320
   acgcgcaata atggtttctg acgtatgtgc ttagctcatt aaactccaga aacccgcggc  10380
   tgagtggctc cttcaacgtt gcggttctgt cagttccaaa cgtaaaacgg cttgtcccgc  10440
   gtcatcggcg ggggtcataa cgtgactccc ttaattctcc gctcatgatc ttgatcccct  10500
   gcgccatcag atccttggcg gcaagaaagc catccagttt actttgcagg gcttcccaac  10560
   cttaccagag ggcgccccag ctggcaattc cggttcgctt gctgtccata aaaccgccca  10620
   gtctagctat cgccatgtaa gcccactgca agctacctgc tttctctttg cgcttgcgtt  10680
   ttcccttgtc cagatagccc agtagctgac attcatccgg ggtcagcacc gtttctgcgg  10740
   actggctttc tacgtgttcc gcttccttta gcagcccttg cgccctgagt gcttgcggca  10800
   gcgtgaagct tgcatgcctg caggtcgacg gcgcgccgag ctcctcgagc aaatttacac  10860
   attgccacta aacgtctaaa cccttgtaat ttgtttttgt tttactatgt gtgttatgta  10920
   tttgatttgc gataaatttt tatatttggt actaaattta taacaccttt tatgctaacg  10980
   tttgccaaca cttagcaatt tgcaagttga ttaattgatt ctaaattatt tttgtcttct  11040
   aaatacatat actaatcaac tggaaatgta aatatttgct aatatttcta ctataggaga  11100
   attaaagtga gtgaatatgg taccacaagg tttggagatt taattgttgc aatgctgcat  11160
   ggatggcata tacaccaaac attcaataat tcttgaggat aataatggta ccacacaaga  11220
   tttgaggtgc atgaacgtca cgtggacaaa aggtttagta atttttcaag acaacaatgt  11280
   taccacacac aagttttgag gtgcatgcat ggatgccctg tggaaagttt aaaaatattt  11340
   tggaaatgat ttgcatggaa gccatgtgta aaaccatgac atccacttgg aggatgcaat  11400
   aatgaagaaa actacaaatt tacatgcaac tagttatgca tgtagtctat ataatgagga  11460
   ttttgcaata ctttcattca tacacactca ctaagtttta cacgattata atttcttcat  11520
   agccagccca ccgcggtgga aa atg gag gtc gtg gag aga ttc tac ggt gag  11572
                            Met Glu Val Val Glu Arg Phe Tyr Gly Glu
                            1               5                   10
   ttg gat ggg aag gtc tcg cag ggc gtg aat gca ttg ctg ggt agt ttt  11620
   Leu Asp Gly Lys Val Ser Gln Gly Val Asn Ala Leu Leu Gly Ser Phe
                   15                  20                  25
   ggg gtg gag ttg acg gat acg ccc act acc aaa ggc ttg ccc ctc gtt  11668
   Gly Val Glu Leu Thr Asp Thr Pro Thr Thr Lys Gly Leu Pro Leu Val
               30                  35                  40
   gac agt ccc aca ccc atc gtc ctc ggt gtt tct gta tac ttg act att  11716
   Asp Ser Pro Thr Pro Ile Val Leu Gly Val Ser Val Tyr Leu Thr Ile
           45                  50                  55
   gtc att gga ggg ctt ttg tgg ata aag gcc agg gat ctg aaa ccg cgc  11764
   Val Ile Gly Gly Leu Leu Trp Ile Lys Ala Arg Asp Leu Lys Pro Arg
       60                  65                  70
   gcc tcg gag cca ttt ttg ctc caa gct ttg gtg ctt gtg cac aac ctg  11812
   Ala Ser Glu Pro Phe Leu Leu Gln Ala Leu Val Leu Val His Asn Leu
   75                  80                  85                  90
   ttc tgt ttt gcg ctc agt ctg tat atg tgc gtg ggc atc gct tat cag  11860
   Phe Cys Phe Ala Leu Ser Leu Tyr Met Cys Val Gly Ile Ala Tyr Gln
                   95                  100                 105
   gct att acc tgg cgg tac tct ctc tgg ggc aat gca tac aat cct aaa  11908
   Ala Ile Thr Trp Arg Tyr Ser Leu Trp Gly Asn Ala Tyr Asn Pro Lys
               110                 115                 120
   cat aaa gag atg gcg att ctg gta tac ttg ttc tac atg tct aag tac  11956
   His Lys Glu Met Ala Ile Leu Val Tyr Leu Phe Tyr Met Ser Lys Tyr
           125                 130                 135
   gtg gaa ttc atg gat acc gtt atc atg ata ctg aag cgc agc acc agg  12004
   Val Glu Phe Met Asp Thr Val Ile Met Ile Leu Lys Arg Ser Thr Arg
       140                 145                 150
   caa ata agc ttc ctc cac gtt tat cat cat tct tca att tcc ctc att  12052
   Gln Ile Ser Phe Leu His Val Tyr His His Ser Ser Ile Ser Leu Ile
   155                 160                 165                 170
   tgg tgg gct att gct cat cac gct cct ggc ggt gaa gca tat tgg tct     12100
   Trp Trp Ala Ile Ala His His Ala Pro Gly Gly Glu Ala Tyr Trp Ser
                   175                 180                 185
   gcg gct ctg aac tca gga gtg cat gtt ctc atg tat gcg tat tac ttc     12148
   Ala Ala Leu Asn Ser Gly Val His Val Leu Met Tyr Ala Tyr Tyr Phe
               190                 195                 200
   ttg gct gcc tgc ctt cga agt agc cca aag tta aaa aat aag tac ctt     12196
   Leu Ala Ala Cys Leu Arg Ser Ser Pro Lys Leu Lys Asn Lys Tyr Leu
           205                 210                 215
   ttt tgg ggc agg tac ttg aca caa ttc caa atg ttc cag ttt atg ctg     12244
   Phe Trp Gly Arg Tyr Leu Thr Gln Phe Gln Met Phe Gln Phe Met Leu
       220                 225                 230
   aac tta gtg cag gct tac tac gac atg aaa acg aat gcg cca tat cca     12292
   Asn Leu Val Gln Ala Tyr Tyr Asp Met Lys Thr Asn Ala Pro Tyr Pro
   235                 240                 245                 250
   caa tgg ctg atc aag att ttg ttc tac tac atg atc tcg ttg ctg ttt     12340
   Gln Trp Leu Ile Lys Ile Leu Phe Tyr Tyr Met Ile Ser Leu Leu Phe
                   255                 260                 265
   ctt ttc ggc aat ttt tac gta caa aaa tac atc aaa ccc tct gac gga     12388
   Leu Phe Gly Asn Phe Tyr Val Gln Lys Tyr Ile Lys Pro Ser Asp Gly
               270                 275                 280
   aag caa aag gga gct aaa act gag tga tctagaaggc ctcctgcttt           12435
   Lys Gln Lys Gly Ala Lys Thr Glu
           285                 290
   aatgagatat gcgagacgcc tatgatcgca tgatatttgc tttcaattct gttgtgcacg  12495
   ttgtaaaaaa cctgagcatg tgtagctcag atccttaccg ccggtttcgg ttcattctaa  12555
   tgaatatatc acccgttact atcgtatttt tatgaataat attctccgtt caatttactg  12615
   attgtccgtc gagcaaattt acacattgcc actaaacgtc taaacccttg taatttgttt  12675
   ttgttttact atgtgtgtta tgtatttgat ttgcgataaa tttttatatt tggtactaaa  12735
   tttataacac cttttatgct aacgtttgcc aacacttagc aatttgcaag ttgattaatt  12795
   gattctaaat tatttttgtc ttctaaatac atatactaat caactggaaa tgtaaatatt  12855
   tgctaatatt tctactatag gagaattaaa gtgagtgaat atggtaccac aaggtttgga  12915
   gatttaattg ttgcaatgct gcatggatgg catatacacc aaacattcaa taattcttga  12975
   ggataataat ggtaccacac aagatttgag gtgcatgaac gtcacgtgga caaaaggttt  13035
   agtaattttt caagacaaca atgttaccac acacaagttt tgaggtgcat gcatggatgc  13095
   cctgtggaaa gtttaaaaat attttggaaa tgatttgcat ggaagccatg tgtaaaacca  13155
   tgacatccac ttggaggatg caataatgaa gaaaactaca aatttacatg caactagtta  13215
   tgcatgtagt ctatataatg aggattttgc aatactttca ttcatacaca ctcactaagt  13275
   tttacacgat tataatttct tcatagccag cggatcc atg gta ttc gcg ggc ggt   13330
                                            Met Val Phe Ala Gly Gly
                                                            295
   gga ctt cag cag ggc tct ctc gaa gaa aac atc gac gtc gag cac att     13378
   Gly Leu Gln Gln Gly Ser Leu Glu Glu Asn Ile Asp Val Glu His Ile
               300                 305                 310
   gcc agt atg tct ctc ttc agc gac ttc ttc agt tat gtg tct tca act     13426
   Ala Ser Met Ser Leu Phe Ser Asp Phe Phe Ser Tyr Val Ser Ser Thr
           315                 320                 325
   gtt ggt tcg tgg agc gta cac agt ata caa cct ttg aag cgc ctg acg     13474
   Val Gly Ser Trp Ser Val His Ser Ile Gln Pro Leu Lys Arg Leu Thr
       330                 335                 340
   agt aag aag cgt gtt tcg gaa agc gct gcc gtg caa tgt ata tca gct     13522
   Ser Lys Lys Arg Val Ser Glu Ser Ala Ala Val Gln Cys Ile Ser Ala
   345                 350                 355                 360
   gaa gtt cag aga aat tcg agt acc cag gga act gcg gag gca ctc gca     13570
   Glu Val Gln Arg Asn Ser Ser Thr Gln Gly Thr Ala Glu Ala Leu Ala
                   365                 370                 375
   gaa tca gtc gtg aag ccc acg aga cga agg tca tct cag tgg aag aag     13618
   Glu Ser Val Val Lys Pro Thr Arg Arg Arg Ser Ser Gln Trp Lys Lys
               380                 385                 390
   tcg aca cac ccc cta tca gaa gta gca gta cac aac aag cca agc gat     13666
   Ser Thr His Pro Leu Ser Glu Val Ala Val His Asn Lys Pro Ser Asp
           395                 400                 405
   tgc tgg att gtt gta aaa aac aag gtg tat gat gtt tcc aat ttt gcg     13714
   Cys Trp Ile Val Val Lys Asn Lys Val Tyr Asp Val Ser Asn Phe Ala
       410                 415                 420
   gac gag cat ccc gga gga tca gtt att agt act tat ttt gga cga gac     13762
   Asp Glu His Pro Gly Gly Ser Val Ile Ser Thr Tyr Phe Gly Arg Asp
   425                 430                 435                 440
   ggc aca gat gtt ttc tct agt ttt cat gca gct tct aca tgg aaa att     13810
   Gly Thr Asp Val Phe Ser Ser Phe His Ala Ala Ser Thr Trp Lys Ile
                   445                 450                 455
   ctt caa gac ttt tac att ggt gac gtg gag agg gtg gag ccg act cca     13858
   Leu Gln Asp Phe Tyr Ile Gly Asp Val Glu Arg Val Glu Pro Thr Pro
               460                 465                 470
   gag ctg ctg aaa gat ttc cga gaa atg aga gct ctt ttc ctg agg gag     13906
   Glu Leu Leu Lys Asp Phe Arg Glu Met Arg Ala Leu Phe Leu Arg Glu
           475                 480                 485
   caa ctt ttc aaa agt tcg aaa ttg tac tat gtt atg aag ctg ctc acg     13954
   Gln Leu Phe Lys Ser Ser Lys Leu Tyr Tyr Val Met Lys Leu Leu Thr
       490                 495                 500
   aat gtt gct att ttt gct gcg agc att gca ata ata tgt tgg agc aag     14002
   Asn Val Ala Ile Phe Ala Ala Ser Ile Ala Ile Ile Cys Trp Ser Lys
   505                 510                 515                 520
   act att tca gcg gtt ttg gct tca gct tgt atg atg gct ctg tgt ttc     14050
   Thr Ile Ser Ala Val Leu Ala Ser Ala Cys Met Met Ala Leu Cys Phe
                   525                 530                 535
   caa cag tgc gga tgg cta tcc cat gat ttt ctc cac aat cag gtg ttt     14098
   Gln Gln Cys Gly Trp Leu Ser His Asp Phe Leu His Asn Gln Val Phe
               540                 545                 550
   gag aca cgc tgg ctt aat gaa gtt gtc ggg tat gtg atc ggc aac gcc     14146
   Glu Thr Arg Trp Leu Asn Glu Val Val Gly Tyr Val Ile Gly Asn Ala
           555                 560                 565
   gtt ctg ggg ttt agt aca ggg tgg tgg aag gag aag cat aac ctt cat     14194
   Val Leu Gly Phe Ser Thr Gly Trp Trp Lys Glu Lys His Asn Leu His
       570                 575                 580
   cat gct gct cca aat gaa tgc gat cag act tac caa cca att gat gaa     14242
   His Ala Ala Pro Asn Glu Cys Asp Gln Thr Tyr Gln Pro Ile Asp Glu
   585                 590                 595                 600
   gat att gat act ctc ccc ctc att gcc tgg agc aag gac ata ctg gcc     14290
   Asp Ile Asp Thr Leu Pro Leu Ile Ala Trp Ser Lys Asp Ile Leu Ala
                   605                 610                 615
   aca gtt gag aat aag aca ttc ttg cga atc ctc caa tac cag cat ctg     14338
   Thr Val Glu Asn Lys Thr Phe Leu Arg Ile Leu Gln Tyr Gln His Leu
               620                 625                 630
   ttc ttc atg ggt ctg tta ttt ttc gcc cgt ggt agt tgg ctc ttt tgg     14386
   Phe Phe Met Gly Leu Leu Phe Phe Ala Arg Gly Ser Trp Leu Phe Trp
       635                 640                 645
   agc tgg aga tat acc tct aca gca gtg ctc tca cct gtc gac agg ttg    14434
   Ser Trp Arg Tyr Thr Ser Thr Ala Val Leu Ser Pro Val Asp Arg Leu
       650                 655                 660
   ttg gag aag gga act gtt ctg ttt cac tac ttt tgg ttc gtc ggg aca    14482
   Leu Glu Lys Gly Thr Val Leu Phe His Tyr Phe Trp Phe Val Gly Thr
   665                 670                 675                 680
   gcg tgc tat ctt ctc cct ggt tgg aag cca tta gta tgg atg gcg gtg    14530
   Ala Cys Tyr Leu Leu Pro Gly Trp Lys Pro Leu Val Trp Met Ala Val
                   685                 690                 695
   act gag ctc atg tcc ggc atg ctg ctg ggc ttt gta ttt gta ctt agc    14578
   Thr Glu Leu Met Ser Gly Met Leu Leu Gly Phe Val Phe Val Leu Ser
               700                 705                 710
   cac aat ggg atg gag gtt tat aat tcg tct aaa gaa ttc gtg agt gca    14626
   His Asn Gly Met Glu Val Tyr Asn Ser Ser Lys Glu Phe Val Ser Ala
           715                 720                 725
   cag atc gta tcc aca cgg gat atc aaa gga aac ata ttc aac gac tgg    14674
   Gln Ile Val Ser Thr Arg Asp Ile Lys Gly Asn Ile Phe Asn Asp Trp
       730                 735                 740
   ttc act ggt ggc ctt aac agg caa ata gag cat cat ctt ttc cca aca    14722
   Phe Thr Gly Gly Leu Asn Arg Gln Ile Glu His His Leu Phe Pro Thr
   745                 750                 755                 760
   atg ccc agg cat aat tta aac aaa ata gca cct aga gtg gag gtg ttc    14770
   Met Pro Arg His Asn Leu Asn Lys Ile Ala Pro Arg Val Glu Val Phe
                   765                 770                 775
   tgt aag aaa cac ggt ctg gtg tac gaa gac gta tct att gct acc ggc    14818
   Cys Lys Lys His Gly Leu Val Tyr Glu Asp Val Ser Ile Ala Thr Gly
               780                 785                 790
   act tgc aag gtt ttg aaa gca ttg aag gaa gtc gcg gag gct gcg gca    14866
   Thr Cys Lys Val Leu Lys Ala Leu Lys Glu Val Ala Glu Ala Ala Ala
           795                 800                 805
   gag cag cat gct acc acc agt taa gctagcgtta accctgcttt aatgagatat    14920
   Glu Gln His Ala Thr Thr Ser
       810                 815
   gcgagacgcc tatgatcgca tgatatttgc tttcaattct gttgtgcacg ttgtaaaaaa  14980
   cctgagcatg tgtagctcag atccttaccg ccggtttcgg ttcattctaa tgaatatatc  15040
   acccgttact atcgtatttt tatgaataat attctccgtt caatttactg attgtccgtc  15100
   gagcaaattt acacattgcc actaaacgtc taaacccttg taatttgttt ttgttttact  15160
   atgtgtgtta tgtatttgat ttgcgataaa tttttatatt tggtactaaa tttataacac  15220
   cttttatgct aacgtttgcc aacacttagc aatttgcaag ttgattaatt gattctaaat  15280
   tatttttgtc ttctaaatac atatactaat caactggaaa tgtaaatatt tgctaatatt  15340
   tctactatag gagaattaaa gtgagtgaat atggtaccac aaggtttgga gatttaattg  15400
   ttgcaatgct gcatggatgg catatacacc aaacattcaa taattcttga ggataataat  15460
   ggtaccacac aagatttgag gtgcatgaac gtcacgtgga caaaaggttt agtaattttt  15520
   caagacaaca atgttaccac acacaagttt tgaggtgcat gcatggatgc cctgtggaaa  15580
   gtttaaaaat attttggaaa tgatttgcat ggaagccatg tgtaaaacca tgacatccac  15640
   ttggaggatg caataatgaa gaaaactaca aatttacatg caactagtta tgcatgtagt  15700
   ctatataatg aggattttgc aatactttca ttcatacaca ctcactaagt tttacacgat  15760
   tataatttct tcatagccag cagatctaaa atg gct ccg gat gcg gat aag ctt  15814
                                    Met Ala Pro Asp Ala Asp Lys Leu
                                                        820
   cga caa cgc cag acg act gcg gta gcg aag cac aat gct gct acc ata    15862
   Arg Gln Arg Gln Thr Thr Ala Val Ala Lys His Asn Ala Ala Thr Ile
       825                 830                 835
   tcg acg cag gaa cgc ctt tgc agt ctg tct tcg ctc aaa ggc gaa gaa      15910
   Ser Thr Gln Glu Arg Leu Cys Ser Leu Ser Ser Leu Lys Gly Glu Glu
   840                 845                 850                 855
   gtc tgc atc gac gga atc atc tat gac ctc caa tca ttc gat cat ccc      15958
   Val Cys Ile Asp Gly Ile Ile Tyr Asp Leu Gln Ser Phe Asp His Pro
                   860                 865                 870
   ggg ggt gaa acg atc aaa atg ttt ggt ggc aac gat gtc act gta cag      16006
   Gly Gly Glu Thr Ile Lys Met Phe Gly Gly Asn Asp Val Thr Val Gln
               875                 880                 885
   tac aag atg att cac ccg tac cat acc gag aag cat ttg gaa aag atg      16054
   Tyr Lys Met Ile His Pro Tyr His Thr Glu Lys His Leu Glu Lys Met
           890                 895                 900
   aag cgt gtc ggc aag gtg acg gat ttc gtc tgc gag tac aag ttc gat      16102
   Lys Arg Val Gly Lys Val Thr Asp Phe Val Cys Glu Tyr Lys Phe Asp
       905                 910                 915
   acc gaa ttt gaa cgc gaa atc aaa cga gaa gtc ttc aag att gtg cga      16150
   Thr Glu Phe Glu Arg Glu Ile Lys Arg Glu Val Phe Lys Ile Val Arg
   920                 925                 930                 935
   cga ggc aag gat ttc ggt act ttg gga tgg ttc ttc cgt gcg ttt tgc      16198
   Arg Gly Lys Asp Phe Gly Thr Leu Gly Trp Phe Phe Arg Ala Phe Cys
                   940                 945                 950
   tac att gcc att ttc ttc tac ctg cag tac cat tgg gtc acc acg gga      16246
   Tyr Ile Ala Ile Phe Phe Tyr Leu Gln Tyr His Trp Val Thr Thr Gly
               955                 960                 965
   acc tct tgg ctg ctg gcc gtg gcc tac gga atc tcc caa gcg atg att      16294
   Thr Ser Trp Leu Leu Ala Val Ala Tyr Gly Ile Ser Gln Ala Met Ile
           970                 975                 980
   ggc atg aat gtc cag cac gat gcc aac cac ggg gcc acc tcc aag cgt      16342
   Gly Met Asn Val Gln His Asp Ala Asn His Gly Ala Thr Ser Lys Arg
       985                 990                 995
   ccc  tgg gtc aac gac atg  cta ggc ctc ggt gcg  gat ttt att ggt      16387
   Pro  Trp Val Asn Asp Met  Leu Gly Leu Gly Ala  Asp Phe Ile Gly
   1000                 1005                 1010
   ggt  tcc aag tgg ctc tgg  cag gaa caa cac tgg  acc cac cac gct      16432
   Gly  Ser Lys Trp Leu Trp  Gln Glu Gln His Trp  Thr His His Ala
   1015                 1020                 1025
   tac  acc aat cac gcc gag  atg gat ccc gat agc  ttt ggt gcc gaa      16477
   Tyr  Thr Asn His Ala Glu  Met Asp Pro Asp Ser  Phe Gly Ala Glu
   1030                 1035                 1040
   cca  atg ctc cta ttc aac  gac tat ccc ttg gat  cat ccc gct cgt      16522
   Pro  Met Leu Leu Phe Asn  Asp Tyr Pro Leu Asp  His Pro Ala Arg
   1045                 1050                 1055
   acc  tgg cta cat cgc ttt  caa gca ttc ttt tac  atg ccc gtc ttg      16567
   Thr  Trp Leu His Arg Phe  Gln Ala Phe Phe Tyr  Met Pro Val Leu
   1060                 1065                 1070
   gct  gga tac tgg ttg tcc  gct gtc ttc aat cca  caa att ctt gac      16612
   Ala  Gly Tyr Trp Leu Ser  Ala Val Phe Asn Pro  Gln Ile Leu Asp
   1075                 1080                 1085
   ctc  cag caa cgc ggc gca  ctt tcc gtc ggt atc  cgt ctc gac aac      16657
   Leu  Gln Gln Arg Gly Ala  Leu Ser Val Gly Ile  Arg Leu Asp Asn
   1090                 1095                 1100
   gct  ttc att cac tcg cga  cgc aag tat gcg gtt  ttc tgg cgg gct      16702
   Ala  Phe Ile His Ser Arg  Arg Lys Tyr Ala Val  Phe Trp Arg Ala
   1105                 1110                 1115
   gtg  tac att gcg gtg aac  gtg att gct ccg ttt  tac aca aac tcc      16747
   Val  Tyr Ile Ala Val Asn  Val Ile Ala Pro Phe  Tyr Thr Asn Ser
   1120                 1125                 1130
   ggc  ctc gaa tgg tcc tgg  cgt gtc ttt gga aac  atc atg ctc atg      16792
   Gly  Leu Glu Trp Ser Trp  Arg Val Phe Gly Asn  Ile Met Leu Met
   1135                 1140                 1145
   ggt  gtg gcg gaa tcg ctc  gcg ctg gcg gtc ctg  ttt tcg ttg tcg      16837
   Gly  Val Ala Glu Ser Leu  Ala Leu Ala Val Leu  Phe Ser Leu Ser
   1150                 1155                 1160
   cac  aat ttc gaa tcc gcg  gat cgc gat ccg acc  gcc cca ctg aaa      16882
   His  Asn Phe Glu Ser Ala  Asp Arg Asp Pro Thr  Ala Pro Leu Lys
   1165                 1170                 1175
   aag  acg gga gaa cca gtc  gac tgg ttc aag aca  cag gtc gaa act      16927
   Lys  Thr Gly Glu Pro Val  Asp Trp Phe Lys Thr  Gln Val Glu Thr
   1180                 1185                 1190
   tcc  tgc act tac ggt gga  ttc ctt tcc ggt tgc  ttc acg gga ggt      16972
   Ser  Cys Thr Tyr Gly Gly  Phe Leu Ser Gly Cys  Phe Thr Gly Gly
   1195                 1200                 1205
   ctc  aac ttt cag gtt gaa  cac cac ttg ttc cca  cgc atg agc agc      17017
   Leu  Asn Phe Gln Val Glu  His His Leu Phe Pro  Arg Met Ser Ser
   1210                 1215                 1220
   gct  tgg tat ccc tac att  gcc ccc aag gtc cgc  gaa att tgc gcc      17062
   Ala  Trp Tyr Pro Tyr Ile  Ala Pro Lys Val Arg  Glu Ile Cys Ala
   1225                 1230                 1235
   aaa  cac ggc gtc cac tac  gcc tac tac ccg tgg  atc cac caa aac      17107
   Lys  His Gly Val His Tyr  Ala Tyr Tyr Pro Trp  Ile His Gln Asn
   1240                 1245                 1250
   ttt  ctc tcc acc gtc cgc  tac atg cac gcg gcc  ggg acc ggt gcc      17152
   Phe  Leu Ser Thr Val Arg  Tyr Met His Ala Ala  Gly Thr Gly Ala
   1255                 1260                 1265
   aac  tgg cgc cag atg gcc  aga gaa aat ccc ttg  acc gga cgg gcg      17197
   Asn  Trp Arg Gln Met Ala  Arg Glu Asn Pro Leu  Thr Gly Arg Ala
   1270                 1275                 1280
   taa agatctgccg gcatcgatcc cgggccatgg cctgctttaa tgagatatgc          17250
   gagacgccta tgatcgcatg atatttgctt tcaattctgt tgtgcacgtt gtaaaaaacc  17310
   tgagcatgtg tagctcagat ccttaccgcc ggtttcggtt cattctaatg aatatatcac  17370
   ccgttactat cgtattttta tgaataatat tctccgttca atttactgat tgtccgtcga  17430
   cgagctcggc gcgcctctag aggatcgatg aattcagatc ggctgagtgg ctccttcaac  17490
   gttgcggttc tgtcagttcc aaacgtaaaa cggcttgtcc cgcgtcatcg gcgggggtca  17550
   taacgtgact cccttaattc tccgctcatg atcagattgt cgtttcccgc cttcagttta  17610
   aactatcagt gtttgacagg atatattggc gggtaaacct aagagaaaag agcgtttatt  17670
   agaataatcg gatatttaaa agggcgtgaa aaggtttatc cttcgtccat ttgtatgtgc  17730
   atgccaacca cagggttccc ca                                            17752
<210> 57
   <211> 290
   <212> PRT
   <213> Phaeodactylum tricornutum, Physcomitrella patens
<400> 57
   Met Glu Val Val Glu Arg Phe Tyr Gly Glu Leu Asp Gly Lys Val Ser
   1               5                   10                  15
   Gln Gly Val Asn Ala Leu Leu Gly Ser Phe Gly Val Glu Leu Thr Asp
               20                  25                  30
   Thr Pro Thr Thr Lys Gly Leu Pro Leu Val Asp Ser Pro Thr Pro Ile
           35                  40                  45
   Val Leu Gly Val Ser Val Tyr Leu Thr Ile Val Ile Gly Gly Leu Leu
       50                  55                  60
   Trp Ile Lys Ala Arg Asp Leu Lys Pro Arg Ala Ser Glu Pro Phe Leu
   65                  70                  75                  80
   Leu Gln Ala Leu Val Leu Val His Asn Leu Phe Cys Phe Ala Leu Ser
                   85                  90                  95
   Leu Tyr Met Cys Val Gly Ile Ala Tyr Gln Ala Ile Thr Trp Arg Tyr
               100                 105                 110
   Ser Leu Trp Gly Asn Ala Tyr Asn Pro Lys His Lys Glu Met Ala Ile
           115                 120                 125
   Leu Val Tyr Leu Phe Tyr Met Ser Lys Tyr Val Glu Phe Met Asp Thr
       130                 135                 140
   Val Ile Met Ile Leu Lys Arg Ser Thr Arg Gln Ile Ser Phe Leu His
   145                 150                 155                 160
   Val Tyr His His Ser Ser Ile Ser Leu Ile Trp Trp Ala Ile Ala His
                   165                 170                 175
   His Ala Pro Gly Gly Glu Ala Tyr Trp Ser Ala Ala Leu Asn Ser Gly
               180                 185                 190
   Val His Val Leu Met Tyr Ala Tyr Tyr Phe Leu Ala Ala Cys Leu Arg
           195                 200                 205
   Ser Ser Pro Lys Leu Lys Asn Lys Tyr Leu Phe Trp Gly Arg Tyr Leu
       210                 215                 220
   Thr Gln Phe Gln Met Phe Gln Phe Met Leu Asn Leu Val Gln Ala Tyr
   225                 230                 235                 240
   Tyr Asp Met Lys Thr Asn Ala Pro Tyr Pro Gln Trp Leu Ile Lys Ile
                   245                 250                 255
   Leu Phe Tyr Tyr Met Ile Ser Leu Leu Phe Leu Phe Gly Asn Phe Tyr
               260                 265                 270
   Val Gln Lys Tyr Ile Lys Pro Ser Asp Gly Lys Gln Lys Gly Ala Lys
           275                 280                 285
   Thr Glu
       290
<210> 58
   <211> 525
   <212> PRT
   <213> Phaeodactylum tricornutum, Physcomitrella patens
<400> 58
   Met Val Phe Ala Gly Gly Gly Leu Gln Gln Gly Ser Leu Glu Glu Asn
   1               5                   10                  15
   Ile Asp Val Glu His Ile Ala Ser Met Ser Leu Phe Ser Asp Phe Phe
               20                  25                  30
   Ser Tyr Val Ser Ser Thr Val Gly Ser Trp Ser Val His Ser Ile Gln
           35                  40                  45
   Pro Leu Lys Arg Leu Thr Ser Lys Lys Arg Val Ser Glu Ser Ala Ala
       50                  55                  60
   Val Gln Cys Ile Ser Ala Glu Val Gln Arg Asn Ser Ser Thr Gln Gly
   65                  70                  75                  80
   Thr Ala Glu Ala Leu Ala Glu Ser Val Val Lys Pro Thr Arg Arg Arg
                   85                  90                  95
   Ser Ser Gln Trp Lys Lys Ser Thr His Pro Leu Ser Glu Val Ala Val
               100                 105                 110
   His Asn Lys Pro Ser Asp Cys Trp Ile Val Val Lys Asn Lys Val Tyr
           115                 120                 125
   Asp Val Ser Asn Phe Ala Asp Glu His Pro Gly Gly Ser Val Ile Ser
       130                 135                 140
   Thr Tyr Phe Gly Arg Asp Gly Thr Asp Val Phe Ser Ser Phe His Ala
   145                 150                 155                 160
   Ala Ser Thr Trp Lys Ile Leu Gln Asp Phe Tyr Ile Gly Asp Val Glu
                   165                 170                 175
   Arg Val Glu Pro Thr Pro Glu Leu Leu Lys Asp Phe Arg Glu Met Arg
               180                 185                 190
   Ala Leu Phe Leu Arg Glu Gln Leu Phe Lys Ser Ser Lys Leu Tyr Tyr
           195                 200                 205
   Val Met Lys Leu Leu Thr Asn Val Ala Ile Phe Ala Ala Ser Ile Ala
       210                 215                 220
   Ile Ile Cys Trp Ser Lys Thr Ile Ser Ala Val Leu Ala Ser Ala Cys
   225                 230                 235                 240
   Met Met Ala Leu Cys Phe Gln Gln Cys Gly Trp Leu Ser His Asp Phe
                   245                 250                 255
   Leu His Asn Gln Val Phe Glu Thr Arg Trp Leu Asn Glu Val Val Gly
               260                 265                 270
   Tyr Val Ile Gly Asn Ala Val Leu Gly Phe Ser Thr Gly Trp Trp Lys
           275                 280                 285
   Glu Lys His Asn Leu His His Ala Ala Pro Asn Glu Cys Asp Gln Thr
       290                 295                 300
   Tyr Gln Pro Ile Asp Glu Asp Ile Asp Thr Leu Pro Leu Ile Ala Trp
   305                 310                 315                 320
   Ser Lys Asp Ile Leu Ala Thr Val Glu Asn Lys Thr Phe Leu Arg Ile
                   325                 330                 335
   Leu Gln Tyr Gln His Leu Phe Phe Met Gly Leu Leu Phe Phe Ala Arg
               340                 345                 350
   Gly Ser Trp Leu Phe Trp Ser Trp Arg Tyr Thr Ser Thr Ala Val Leu
           355                 360                 365
   Ser Pro Val Asp Arg Leu Leu Glu Lys Gly Thr Val Leu Phe His Tyr
       370                 375                 380
   Phe Trp Phe Val Gly Thr Ala Cys Tyr Leu Leu Pro Gly Trp Lys Pro
   385                 390                 395                 400
   Leu Val Trp Met Ala Val Thr Glu Leu Met Ser Gly Met Leu Leu Gly
                   405                 410                 415
   Phe Val Phe Val Leu Ser His Asn Gly Met Glu Val Tyr Asn Ser Ser
               420                 425                 430
   Lys Glu Phe Val Ser Ala Gln Ile Val Ser Thr Arg Asp Ile Lys Gly
           435                 440                 445
   Asn Ile Phe Asn Asp Trp Phe Thr Gly Gly Leu Asn Arg Gln Ile Glu
       450                 455                 460
   His His Leu Phe Pro Thr Met Pro Arg His Asn Leu Asn Lys Ile Ala
   465                 470                 475                 480
   Pro Arg Val Glu Val Phe Cys Lys Lys His Gly Leu Val Tyr Glu Asp
                   485                 490                 495
   Val Ser Ile Ala Thr Gly Thr Cys Lys Val Leu Lys Ala Leu Lys Glu
               500                 505                 510
   Val Ala Glu Ala Ala Ala Glu Gln His Ala Thr Thr Ser
           515                 520                 525
<210> 59
   <211> 469
   <212> PRT
   <213> Phaeodactylum, tricornutum, Physcomitrella patens
<400> 59
   Met Ala Pro Asp Ala Asp Lys Leu Arg Gln Arg Gln Thr Thr Ala Val
   1               5                   10                  15
   Ala Lys His Asn Ala Ala Thr Ile Ser Thr Gln Glu Arg Leu Cys Ser
               20                  25                  30
   Leu Ser Ser Leu Lys Gly Glu Glu Val Cys Ile Asp Gly Ile Ile Tyr
           35                  40                  45
   Asp Leu Gln Ser Phe Asp His Pro Gly Gly Glu Thr Ile Lys Met Phe
       50                  55                  60
   Gly Gly Asn Asp Val Thr Val Gln Tyr Lys Met Ile His Pro Tyr His
   65                  70                  75                  80
   Thr Glu Lys His Leu Glu Lys Met Lys Arg Val Gly Lys Val Thr Asp
                   85                  90                  95
   Phe Val Cys Glu Tyr Lys Phe Asp Thr Glu Phe Glu Arg Glu Ile Lys
               100                 105                 110
   Arg Glu Val Phe Lys Ile Val Arg Arg Gly Lys Asp Phe Gly Thr Leu
           115                 120                 125
   Gly Trp Phe Phe Arg Ala Phe Cys Tyr Ile Ala Ile Phe Phe Tyr Leu
       130                 135                 140
   Gln Tyr His Trp Val Thr Thr Gly Thr Ser Trp Leu Leu Ala Val Ala
   145                 150                 155                 160
   Tyr Gly Ile Ser Gln Ala Met Ile Gly Met Asn Val Gln His Asp Ala
                   165                 170                 175
   Asn His Gly Ala Thr Ser Lys Arg Pro Trp Val Asn Asp Met Leu Gly
               180                 185                 190
   Leu Gly Ala Asp Phe Ile Gly Gly Ser Lys Trp Leu Trp Gln Glu Gln
           195                 200                 205
   His Trp Thr His His Ala Tyr Thr Asn His Ala Glu Met Asp Pro Asp
       210                 215                 220
   Ser Phe Gly Ala Glu Pro Met Leu Leu Phe Asn Asp Tyr Pro Leu Asp
   225                 230                 235                 240
   His Pro Ala Arg Thr Trp Leu His Arg Phe Gln Ala Phe Phe Tyr Met
                   245                 250                 255
   Pro Val Leu Ala Gly Tyr Trp Leu Ser Ala Val Phe Asn Pro Gln Ile
               260                 265                 270
   Leu Asp Leu Gln Gln Arg Gly Ala Leu Ser Val Gly Ile Arg Leu Asp
           275                 280                 285
   Asn Ala Phe Ile His Ser Arg Arg Lys Tyr Ala Val Phe Trp Arg Ala
       290                 295                 300
   Val Tyr Ile Ala Val Asn Val Ile Ala Pro Phe Tyr Thr Asn Ser Gly
   305                 310                 315                 320
   Leu Glu Trp Ser Trp Arg Val Phe Gly Asn Ile Met Leu Met Gly Val
                   325                 330                 335
   Ala Glu Ser Leu Ala Leu Ala Val Leu Phe Ser Leu Ser His Asn Phe
               340                 345                 350
   Glu Ser Ala Asp Arg Asp Pro Thr Ala Pro Leu Lys Lys Thr Gly Glu
           355                 360                 365
   Pro Val Asp Trp Phe Lys Thr Gln Val Glu Thr Ser Cys Thr Tyr Gly
       370                 375                 380
   Gly Phe Leu Ser Gly Cys Phe Thr Gly Gly Leu Asn Phe Gln Val Glu
   385                 390                 395                 400
   His His Leu Phe Pro Arg Met Ser Ser Ala Trp Tyr Pro Tyr Ile Ala
                   405                 410                 415
   Pro Lys Val Arg Glu Ile Cys Ala Lys His Gly Val His Tyr Ala Tyr
               420                 425                 430
   Tyr Pro Trp Ile His Gln Asn Phe Leu Ser Thr Val Arg Tyr Met His
           435                 440                 445
   Ala Ala Gly Thr Gly Ala Asn Trp Arg Gln Met Ala Arg Glu Asn Pro
       450                 455                 460
   Leu Thr Gly Arg Ala
   465
<210> 60
   <211> 26
   <212> DNA
   <213> artificial sequence
<400> 60
   gaattcggcg cgccgagctc ctcgag 26
<210> 61
   <211> 265
   <212> DNA
   <213> artificial sequence
<400> 61
   ccaccgcggt gggcggccgc ctgcagtcta gaaggcctcc tgctttaatg agatatgcga    60
   gacgcctatg atcgcatgat atttgctttc aattctgttg tgcacgttgt aaaaaacctg   120
   agcatgtgta gctcagatcc ttaccgccgg tttcggttca ttctaatgaa tatatcaccc   180
   gttactatcg tatttttatg aataatattc tccgttcaat ttactgattg tccgtcgacg   240
   aattcgagct cggcgcgcca agctt                                         265
<210> 62
   <211> 257
   <212> DNA
   <213> artificial sequence
<400> 62
   ggatccgata tcgggcccgc tagcgttaac cctgctttaa tgagatatgc gagacgccta    60
   tgatcgcatg atatttgctt tcaattctgt tgtgcacgtt gtaaaaaacc tgagcatgtg   120
   tagctcagat ccttaccgcc ggtttcggtt cattctaatg aatatatcac ccgttactat   180
   cgtattttta tgaataatat tctccgttca atttactgat tgtccgtcga cgaattcgag   240
   ctcggcgcgc caagctt                                                  257
<210> 63
   <211> 5410
   <212> DNA
   <213> artificial sequence
<400> 63
   ttttggaaat gatttgcatg gaagccatgt gtaaaaccat gacatccact tggaggatgc      60
   aataatgaag aaaactacaa atttacatgc aactagttat gcatgtagtc tatataatga     120
   ggattttgca atactttcat tcatacacac tcactaagtt ttacacgatt ataatttctt     180
   catagccagc ggatccgata tcgggcccgc tagcgttaac cctgctttaa tgagatatgc     240
   gagacgccta tgatcgcatg atatttgctt tcaattctgt tgtgcacgtt gtaaaaaacc     300
   tgagcatgtg tagctcagat ccttaccgcc ggtttcggtt cattctaatg aatatatcac     360
   ccgttactat cgtattttta tgaataatat tctccgttca atttactgat tgtccgtcga     420
   gcaaatttac acattgccac taaacgtcta aacccttgta atttgttttt gttttactat     480
   gtgtgttatg tatttgattt gcgataaatt tttatatttg gtactaaatt tataacacct     540
   tttatgctaa cgtttgccaa cacttagcaa tttgcaagtt gattaattga ttctaaatta     600
   tttttgtctt ctaaatacat atactaatca actggaaatg taaatatttg ctaatatttc     660
   tactatagga gaattaaagt gagtgaatat ggtaccacaa ggtttggaga tttaattgtt     720
   gcaatgctgc atggatggca tatacaccaa acattcaata attcttgagg ataataatgg     780
   taccacacaa gatttgaggt gcatgaacgt cacgtggaca aaaggtttag taatttttca     840
   agacaacaat gttaccacac acaagttttg aggtgcatgc atggatgccc tgtggaaagt     900
   ttaaaaatat tttggaaatg atttgcatgg aagccatgtg taaaaccatg acatccactt     960
   ggaggatgca ataatgaaga aaactacaaa tttacatgca actagttatg catgtagtct    1020
   atataatgag gattttgcaa tactttcatt catacacact cactaagttt tacacgatta    1080
   taatttcttc atagccagca gatctgccgg catcgatccc gggccatggc ctgctttaat    1140
   gagatatgcg agacgcctat gatcgcatga tatttgcttt caattctgtt gtgcacgttg    1200
   taaaaaacct gagcatgtgt agctcagatc cttaccgccg gtttcggttc attctaatga    1260
   atatatcacc cgttactatc gtatttttat gaataatatt ctccgttcaa tttactgatt    1320
   gtccgtcgac gagctcggcg cgccaagctt ggcgtaatca tggtcatagc tgtttcctgt    1380
   gtgaaattgt tatccgctca caattccaca caacatacga gccggaagca taaagtgtaa    1440
   agcctggggt gcctaatgag tgagctaact cacattaatt gcgttgcgct cactgcccgc    1500
   tttccagtcg ggaaacctgt cgtgccagct gcattaatga atcggccaac gcgcggggag    1560
   aggcggtttg cgtattgggc gctcttccgc ttcctcgctc actgactcgc tgcgctcggt    1620
   cgttcggctg cggcgagcgg tatcagctca ctcaaaggcg gtaatacggt tatccacaga    1680
   atcaggggat aacgcaggaa agaacatgtg agcaaaaggc cagcaaaagg ccaggaaccg    1740
   taaaaaggcc gcgttgctgg cgtttttcca taggctccgc ccccctgacg agcatcacaa    1800
   aaatcgacgc tcaagtcaga ggtggcgaaa cccgacagga ctataaagat accaggcgtt    1860
   tccccctgga agctccctcg tgcgctctcc tgttccgacc ctgccgctta ccggatacct    1920
   gtccgccttt ctcccttcgg gaagcgtggc gctttctcat agctcacgct gtaggtatct    1980
   cagttcggtg taggtcgttc gctccaagct gggctgtgtg cacgaacccc ccgttcagcc    2040
   cgaccgctgc gccttatccg gtaactatcg tcttgagtcc aacccggtaa gacacgactt    2100
   atcgccactg gcagcagcca ctggtaacag gattagcaga gcgaggtatg taggcggtgc    2160
   tacagagttc ttgaagtggt ggcctaacta cggctacact agaaggacag tatttggtat    2220
   ctgcgctctg ctgaagccag ttaccttcgg aaaaagagtt ggtagctctt gatccggcaa    2280
   acaaaccacc gctggtagcg gtggtttttt tgtttgcaag cagcagatta cgcgcagaaa    2340
   aaaaggatct caagaagatc ctttgatctt ttctacgggg tctgacgctc agtggaacga    2400
   aaactcacgt taagggattt tggtcatgag attatcaaaa aggatcttca cctagatcct    2460
   tttaaattaa aaatgaagtt ttaaatcaat ctaaagtata tatgagtaaa cttggtctga    2520
   cagttaccaa tgcttaatca gtgaggcacc tatctcagcg atctgtctat ttcgttcatc    2580
   catagttgcc tgactccccg tcgtgtagat aactacgata cgggagggct taccatctgg    2640
   ccccagtgct gcaatgatac cgcgagaccc acgctcaccg gctccagatt tatcagcaat    2700
   aaaccagcca gccggaaggg ccgagcgcag aagtggtcct gcaactttat ccgcctccat    2760
   ccagtctatt aattgttgcc gggaagctag agtaagtagt tcgccagtta atagtttgcg    2820
   caacgttgtt gccattgcta caggcatcgt ggtgtcacgc tcgtcgtttg gtatggcttc    2880
   attcagctcc ggttcccaac gatcaaggcg agttacatga tcccccatgt tgtgcaaaaa    2940
   agcggttagc tccttcggtc ctccgatcgt tgtcagaagt aagttggccg cagtgttatc    3000
   actcatggtt atggcagcac tgcataattc tcttactgtc atgccatccg taagatgctt    3060
   ttctgtgact ggtgagtact caaccaagtc attctgagaa tagtgtatgc ggcgaccgag    3120
   ttgctcttgc ccggcgtcaa tacgggataa taccgcgcca catagcagaa ctttaaaagt    3180
   gctcatcatt ggaaaacgtt cttcggggcg aaaactctca aggatcttac cgctgttgag    3240
   atccagttcg atgtaaccca ctcgtgcacc caactgatct tcagcatctt ttactttcac    3300
   cagcgtttct gggtgagcaa aaacaggaag gcaaaatgcc gcaaaaaagg gaataagggc    3360
   gacacggaaa tgttgaatac tcatactctt cctttttcaa tattattgaa gcatttatca    3420
   gggttattgt ctcatgagcg gatacatatt tgaatgtatt tagaaaaata aacaaatagg    3480
   ggttccgcgc acatttcccc gaaaagtgcc acctgacgtc taagaaacca ttattatcat    3540
   gacattaacc tataaaaata ggcgtatcac gaggcccttt cgtctcgcgc gtttcggtga    3600
   tgacggtgaa aacctctgac acatgcagct cccggagacg gtcacagctt gtctgtaagc    3660
   ggatgccggg agcagacaag cccgtcaggg cgcgtcagcg ggtgttggcg ggtgtcgggg    3720
   ctggcttaac tatgcggcat cagagcagat tgtactgaga gtgcaccata tgcggtgtga    3780
   aataccgcac agatgcgtaa ggagaaaata ccgcatcagg cgccattcgc cattcaggct    3840
   gcgcaactgt tgggaagggc gatcggtgcg ggcctcttcg ctattacgcc agctggcgaa    3900
   agggggatgt gctgcaaggc gattaagttg ggtaacgcca gggttttccc agtcacgacg    3960
   ttgtaaaacg acggccagtg aattcggcgc gccgagctcc tcgagcaaat ttacacattg    4020
   ccactaaacg tctaaaccct tgtaatttgt ttttgtttta ctatgtgtgt tatgtatttg    4080
   atttgcgata aatttttata tttggtacta aatttataac accttttatg ctaacgtttg    4140
   ccaacactta gcaatttgca agttgattaa ttgattctaa attatttttg tcttctaaat    4200
   acatatacta atcaactgga aatgtaaata tttgctaata tttctactat aggagaatta    4260
   aagtgagtga atatggtacc acaaggtttg gagatttaat tgttgcaatg ctgcatggat    4320
   ggcatataca ccaaacattc aataattctt gaggataata atggtaccac acaagatttg    4380
   aggtgcatga acgtcacgtg gacaaaaggt ttagtaattt ttcaagacaa caatgttacc    4440
   acacacaagt tttgaggtgc atgcatggat gccctgtgga aagtttaaaa atattttgga    4500
   aatgatttgc atggaagcca tgtgtaaaac catgacatcc acttggagga tgcaataatg    4560
   aagaaaacta caaatttaca tgcaactagt tatgcatgta gtctatataa tgaggatttt    4620
   gcaatacttt cattcataca cactcactaa gttttacacg attataattt cttcatagcc    4680
   agcccaccgc ggtgggcggc cgcctgcagt ctagaaggcc tcctgcttta atgagatatg    4740
   cgagacgcct atgatcgcat gatatttgct ttcaattctg ttgtgcacgt tgtaaaaaac    4800
   ctgagcatgt gtagctcaga tccttaccgc cggtttcggt tcattctaat gaatatatca    4860
   cccgttacta tcgtattttt atgaataata ttctccgttc aatttactga ttgtccgtcg    4920
   agcaaattta cacattgcca ctaaacgtct aaacccttgt aatttgtttt tgttttacta    4980
   tgtgtgttat gtatttgatt tgcgataaat ttttatattt ggtactaaat ttataacacc    5040
   ttttatgcta acgtttgcca acacttagca atttgcaagt tgattaattg attctaaatt    5100
   atttttgtct tctaaataca tatactaatc aactggaaat gtaaatattt gctaatattt    5160
   ctactatagg agaattaaag tgagtgaata tggtaccaca aggtttggag atttaattgt    5220
   tgcaatgctg catggatggc atatacacca aacattcaat aattcttgag gataataatg    5280
   gtaccacaca agatttgagg tgcatgaacg tcacgtggac aaaaggttta gtaatttttc    5340
   aagacaacaa tgttaccaca cacaagtttt gaggtgcatg catggatgcc ctgtggaaag    5400
   tttaaaaata                                                           5410
<210> 64
   <211> 12093
   <212> DNA
   <213> artificial sequence
<400> 64
   gatctggcgc cggccagcga gacgagcaag attggccgcc gcccgaaacg atccgacagc 60
   gcgcccagca caggtgcgca ggcaaattgc accaacgcat acagcgccag cagaatgcca 120
   tagtgggcgg tgacgtcgtt cgagtgaacc agatcgcgca ggaggcccgg cagcaccggc 180
   ataatcaggc cgatgccgac agcgtcgagc gcgacagtgc tcagaattac gatcaggggt 240
   atgttgggtt tcacgtctgg cctccggacc agcctccgct ggtccgattg aacgcgcgga 300
   ttctttatca ctgataagtt ggtggacata ttatgtttat cagtgataaa gtgtcaagca 360
   tgacaaagtt gcagccgaat acagtgatcc gtgccgccct ggacctgttg aacgaggtcg 420
   gcgtagacgg tctgacgaca cgcaaactgg cggaacggtt gggggttcag cagccggcgc 480
   tttactggca cttcaggaac aagcgggcgc tgctcgacgc actggccgaa gccatgctgg 540
   cggagaatca tacgcattcg gtgccgagag ccgacgacga ctggcgctca tttctgatcg 600
   ggaatgcccg cagcttcagg caggcgctgc tcgcctaccg cgatggcgcg cgcatccatg 660
   ccggcacgcg accgggcgca ccgcagatgg aaacggccga cgcgcagctt cgcttcctct 720
   gcgaggcggg tttttcggcc ggggacgccg tcaatgcgct gatgacaatc agctacttca 780
   ctgttggggc cgtgcttgag gagcaggccg gcgacagcga tgccggcgag cgcggcggca 840
   ccgttgaaca ggctccgctc tcgccgctgt tgcgggccgc gatagacgcc ttcgacgaag 900
   ccggtccgga cgcagcgttc gagcagggac tcgcggtgat tgtcgatgga ttggcgaaaa 960
   ggaggctcgt tgtcaggaac gttgaaggac cgagaaaggg tgacgattga tcaggaccgc 1020
   tgccggagcg caacccactc actacagcag agccatgtag acaacatccc ctcccccttt 1080
   ccaccgcgtc agacgcccgt agcagcccgc tacgggcttt ttcatgccct gccctagcgt 1140
   ccaagcctca cggccgcgct cggcctctct ggcggccttc tggcgctctt ccgcttcctc 1200
   gctcactgac tcgctgcgct cggtcgttcg gctgcggcga gcggtatcag ctcactcaaa 1260
   ggcggtaata cggttatcca cagaatcagg ggataacgca ggaaagaaca tgtgagcaaa 1320
   aggccagcaa aaggccagga accgtaaaaa ggccgcgttg ctggcgtttt tccataggct 1380
   ccgcccccct gacgagcatc acaaaaatcg acgctcaagt cagaggtggc gaaacccgac 1440
   aggactataa agataccagg cgtttccccc tggaagctcc ctcgtgcgct ctcctgttcc 1500
   gaccctgccg cttaccggat acctgtccgc ctttctccct tcgggaagcg tggcgctttt 1560
   ccgctgcata accctgcttc ggggtcatta tagcgatttt ttcggtatat ccatcctttt 1620
   tcgcacgata tacaggattt tgccaaaggg ttcgtgtaga ctttccttgg tgtatccaac 1680
   ggcgtcagcc gggcaggata ggtgaagtag gcccacccgc gagcgggtgt tccttcttca 1740
   ctgtccctta ttcgcacctg gcggtgctca acgggaatcc tgctctgcga ggctggccgg 1800
   ctaccgccgg cgtaacagat gagggcaagc ggatggctga tgaaaccaag ccaaccagga 1860
   agggcagccc acctatcaag gtgtactgcc ttccagacga acgaagagcg attgaggaaa 1920
   aggcggcggc ggccggcatg agcctgtcgg cctacctgct ggccgtcggc cagggctaca 1980
   aaatcacggg cgtcgtggac tatgagcacg tccgcgagct ggcccgcatc aatggcgacc 2040
   tgggccgcct gggcggcctg ctgaaactct ggctcaccga cgacccgcgc acggcgcggt 2100
   tcggtgatgc cacgatcctc gccctgctgg cgaagatcga agagaagcag gacgagcttg 2160
   gcaaggtcat gatgggcgtg gtccgcccga gggcagagcc atgacttttt tagccgctaa 2220
   aacggccggg gggtgcgcgt gattgccaag cacgtcccca tgcgctccat caagaagagc 2280
   gacttcgcgg agctggtgaa gtacatcacc gacgagcaag gcaagaccga gcgcctttgc 2340
   gacgctcacc gggctggttg ccctcgccgc tgggctggcg gccgtctatg gccctgcaaa 2400
   cgcgccagaa acgccgtcga agccgtgtgc gagacaccgc ggccgccggc gttgtggata 2460
   cctcgcggaa aacttggccc tcactgacag atgaggggcg gacgttgaca cttgaggggc 2520
   cgactcaccc ggcgcggcgt tgacagatga ggggcaggct cgatttcggc cggcgacgtg 2580
   gagctggcca gcctcgcaaa tcggcgaaaa cgcctgattt tacgcgagtt tcccacagat 2640
   gatgtggaca agcctgggga taagtgccct gcggtattga cacttgaggg gcgcgactac 2700
   tgacagatga ggggcgcgat ccttgacact tgaggggcag agtgctgaca gatgaggggc 2760
   gcacctattg acatttgagg ggctgtccac aggcagaaaa tccagcattt gcaagggttt 2820
   ccgcccgttt ttcggccacc gctaacctgt cttttaacct gcttttaaac caatatttat 2880
   aaaccttgtt tttaaccagg gctgcgccct gtgcgcgtga ccgcgcacgc cgaagggggg 2940
   tgccccccct tctcgaaccc tcccggcccg ctaacgcggg cctcccatcc ccccaggggc 3000
   tgcgcccctc ggccgcgaac ggcctcaccc caaaaatggc agcgctggca gtccttgcca 3060
   ttgccgggat cggggcagta acgggatggg cgatcagccc gagcgcgacg cccggaagca 3120
   ttgacgtgcc gcaggtgctg gcatcgacat tcagcgacca ggtgccgggc agtgagggcg 3180
   gcggcctggg tggcggcctg cccttcactt cggccgtcgg ggcattcacg gacttcatgg 3240
   cggggccggc aatttttacc ttgggcattc ttggcatagt ggtcgcgggt gccgtgctcg 3300
   tgttcggggg tgcgataaac ccagcgaacc atttgaggtg ataggtaaga ttataccgag 3360
   gtatgaaaac gagaattgga cctttacaga attactctat gaagcgccat atttaaaaag 3420
   ctaccaagac gaagaggatg aagaggatga ggaggcagat tgccttgaat atattgacaa 3480
   tactgataag ataatatatc ttttatatag aagatatcgc cgtatgtaag gatttcaggg 3540
   ggcaaggcat aggcagcgcg cttatcaata tatctataga atgggcaaag cataaaaact 3600
   tgcatggact aatgcttgaa acccaggaca ataaccttat agcttgtaaa ttctatcata 3660
   attgggtaat gactccaact tattgatagt gttttatgtt cagataatgc ccgatgactt 3720
   tgtcatgcag ctccaccgat tttgagaacg acagcgactt ccgtcccagc cgtgccaggt 3780
   gctgcctcag attcaggtta tgccgctcaa ttcgctgcgt atatcgcttg ctgattacgt 3840
   gcagctttcc cttcaggcgg gattcataca gcggccagcc atccgtcatc catatcacca 3900
   cgtcaaaggg tgacagcagg ctcataagac gccccagcgt cgccatagtg cgttcaccga 3960
   atacgtgcgc aacaaccgtc ttccggagac tgtcatacgc gtaaaacagc cagcgctggc 4020
   gcgatttagc cccgacatag ccccactgtt cgtccatttc cgcgcagacg atgacgtcac 4080
   tgcccggctg tatgcgcgag gttaccgact gcggcctgag ttttttaagt gacgtaaaat 4140
   cgtgttgagg ccaacgccca taatgcgggc tgttgcccgg catccaacgc cattcatggc 4200
   catatcaatg attttctggt gcgtaccggg ttgagaagcg gtgtaagtga actgcagttg 4260
   ccatgtttta cggcagtgag agcagagata gcgctgatgt ccggcggtgc ttttgccgtt 4320
   acgcaccacc ccgtcagtag ctgaacagga gggacagctg atagacacag aagccactgg 4380
   agcacctcaa aaacaccatc atacactaaa tcagtaagtt ggcagcatca cccataattg 4440
   tggtttcaaa atcggctccg tcgatactat gttatacgcc aactttgaaa acaactttga 4500
   aaaagctgtt ttctggtatt taaggtttta gaatgcaagg aacagtgaat tggagttcgt 4560
   cttgttataa ttagcttctt ggggtatctt taaatactgt agaaaagagg aaggaaataa 4620
   taaatggcta aaatgagaat atcaccggaa ttgaaaaaac tgatcgaaaa ataccgctgc 4680
   gtaaaagata cggaaggaat gtctcctgct aaggtatata agctggtggg agaaaatgaa 4740
   aacctatatt taaaaatgac ggacagccgg tataaaggga ccacctatga tgtggaacgg 4800
   gaaaaggaca tgatgctatg gctggaagga aagctgcctg ttccaaaggt cctgcacttt 4860
   gaacggcatg atggctggag caatctgctc atgagtgagg ccgatggcgt cctttgctcg 4920
   gaagagtatg aagatgaaca aagccctgaa aagattatcg agctgtatgc ggagtgcatc 4980
   aggctctttc actccatcga catatcggat tgtccctata cgaatagctt agacagccgc 5040
   ttagccgaat tggattactt actgaataac gatctggccg atgtggattg cgaaaactgg 5100
   gaagaagaca ctccatttaa agatccgcgc gagctgtatg attttttaaa gacggaaaag 5160
   cccgaagagg aacttgtctt ttcccacggc gacctgggag acagcaacat ctttgtgaaa 5220
   gatggcaaag taagtggctt tattgatctt gggagaagcg gcagggcgga caagtggtat 5280
   gacattgcct tctgcgtccg gtcgatcagg gaggatatcg gggaagaaca gtatgtcgag 5340
   ctattttttg acttactggg gatcaagcct gattgggaga aaataaaata ttatatttta 5400
   ctggatgaat tgttttagta cctagatgtg gcgcaacgat gccggcgaca agcaggagcg 5460
   caccgacttc ttccgcatca agtgttttgg ctctcaggcc gaggcccacg gcaagtattt 5520
   gggcaagggg tcgctggtat tcgtgcaggg caagattcgg aataccaagt acgagaagga 5580
   cggccagacg gtctacggga ccgacttcat tgccgataag gtggattatc tggacaccaa 5640
   ggcaccaggc gggtcaaatc aggaataagg gcacattgcc ccggcgtgag tcggggcaat 5700
   cccgcaagga gggtgaatga atcggacgtt tgaccggaag gcatacaggc aagaactgat 5760
   cgacgcgggg ttttccgccg aggatgccga aaccatcgca agccgcaccg tcatgcgtgc 5820
   gccccgcgaa accttccagt ccgtcggctc gatggtccag caagctacgg ccaagatcga 5880
   gcgcgacagc gtgcaactgg ctccccctgc cctgcccgcg ccatcggccg ccgtggagcg 5940
   ttcgcgtcgt ctcgaacagg aggcggcagg tttggcgaag tcgatgacca tcgacacgcg 6000
   aggaactatg acgaccaaga agcgaaaaac cgccggcgag gacctggcaa aacaggtcag 6060
   cgaggccaag caggccgcgt tgctgaaaca cacgaagcag cagatcaagg aaatgcagct 6120
   ttccttgttc gatattgcgc cgtggccgga cacgatgcga gcgatgccaa acgacacggc 6180
   ccgctctgcc ctgttcacca cgcgcaacaa gaaaatcccg cgcgaggcgc tgcaaaacaa 6240
   ggtcattttc cacgtcaaca aggacgtgaa gatcacctac accggcgtcg agctgcgggc 6300
   cgacgatgac gaactggtgt ggcagcaggt gttggagtac gcgaagcgca cccctatcgg 6360
   cgagccgatc accttcacgt tctacgagct ttgccaggac ctgggctggt cgatcaatgg 6420
   ccggtattac acgaaggccg aggaatgcct gtcgcgccta caggcgacgg cgatgggctt 6480
   cacgtccgac cgcgttgggc acctggaatc ggtgtcgctg ctgcaccgct tccgcgtcct 6540
   ggaccgtggc aagaaaacgt cccgttgcca ggtcctgatc gacgaggaaa tcgtcgtgct 6600
   gtttgctggc gaccactaca cgaaattcat atgggagaag taccgcaagc tgtcgccgac 6660
   ggcccgacgg atgttcgact atttcagctc gcaccgggag ccgtacccgc tcaagctgga 6720
   aaccttccgc ctcatgtgcg gatcggattc cacccgcgtg aagaagtggc gcgagcaggt 6780
   cggcgaagcc tgcgaagagt tgcgaggcag cggcctggtg gaacacgcct gggtcaatga 6840
   tgacctggtg cattgcaaac gctagggcct tgtggggtca gttccggctg ggggttcagc 6900
   agccagcgct ttactggcat ttcaggaaca agcgggcact gctcgacgca cttgcttcgc 6960
   tcagtatcgc tcgggacgca cggcgcgctc tacgaactgc cgataaacag aggattaaaa 7020
   ttgacaattg tgattaaggc tcagattcga cggcttggag cggccgacgt gcaggatttc 7080
   cgcgagatcc gattgtcggc cctgaagaaa gctccagaga tgttcgggtc cgtttacgag 7140
   cacgaggaga aaaagcccat ggaggcgttc gctgaacggt tgcgagatgc cgtggcattc 7200
   ggcgcctaca tcgacggcga gatcattggg ctgtcggtct tcaaacagga ggacggcccc 7260
   aaggacgctc acaaggcgca tctgtccggc gttttcgtgg agcccgaaca gcgaggccga 7320
   ggggtcgccg gtatgctgct gcgggcgttg ccggcgggtt tattgctcgt gatgatcgtc 7380
   cgacagattc caacgggaat ctggtggatg cgcatcttca tcctcggcgc acttaatatt 7440
   tcgctattct ggagcttgtt gtttatttcg gtctaccgcc tgccgggcgg ggtcgcggcg 7500
   acggtaggcg ctgtgcagcc gctgatggtc gtgttcatct ctgccgctct gctaggtagc 7560
   ccgatacgat tgatggcggt cctgggggct atttgcggaa ctgcgggcgt ggcgctgttg 7620
   gtgttgacac caaacgcagc gctagatcct gtcggcgtcg cagcgggcct ggcgggggcg 7680
   gtttccatgg cgttcggaac cgtgctgacc cgcaagtggc aacctcccgt gcctctgctc 7740
   acctttaccg cctggcaact ggcggccgga ggacttctgc tcgttccagt agctttagtg 7800
   tttgatccgc caatcccgat gcctacagga accaatgttc tcggcctggc gtggctcggc 7860
   ctgatcggag cgggtttaac ctacttcctt tggttccggg ggatctcgcg actcgaacct 7920
   acagttgttt ccttactggg ctttctcagc cccagatctg gggtcgatca gccggggatg 7980
   catcaggccg acagtcggaa cttcgggtcc ccgacctgta ccattcggtg agcaatggat 8040
   aggggagttg atatcgtcaa cgttcacttc taaagaaata gcgccactca gcttcctcag 8100
   cggctttatc cagcgatttc ctattatgtc ggcatagttc tcaagatcga cagcctgtca 8160
   cggttaagcg agaaatgaat aagaaggctg ataattcgga tctctgcgag ggagatgata 8220
   tttgatcaca ggcagcaacg ctctgtcatc gttacaatca acatgctacc ctccgcgaga 8280
   tcatccgtgt ttcaaacccg gcagcttagt tgccgttctt ccgaatagca tcggtaacat 8340
   gagcaaagtc tgccgcctta caacggctct cccgctgacg ccgtcccgga ctgatgggct 8400
   gcctgtatcg agtggtgatt ttgtgccgag ctgccggtcg gggagctgtt ggctggctgg 8460
   tggcaggata tattgtggtg taaacaaatt gacgcttaga caacttaata acacattgcg 8520
   gacgttttta atgtactggg gtggtttttc ttttcaccag tgagacgggc aacagctgat 8580
   tgcccttcac cgcctggccc tgagagagtt gcagcaagcg gtccacgctg gtttgcccca 8640
   gcaggcgaaa atcctgtttg atggtggttc cgaaatcggc aaaatccctt ataaatcaaa 8700
   agaatagccc gagatagggt tgagtgttgt tccagtttgg aacaagagtc cactattaaa 8760
   gaacgtggac tccaacgtca aagggcgaaa aaccgtctat cagggcgatg gcccactacg 8820
   tgaaccatca cccaaatcaa gttttttggg gtcgaggtgc cgtaaagcac taaatcggaa 8880
   ccctaaaggg agcccccgat ttagagcttg acggggaaag ccggcgaacg tggcgagaaa 8940
   ggaagggaag aaagcgaaag gagcgggcgc cattcaggct gcgcaactgt tgggaagggc 9000
   gatcggtgcg ggcctcttcg ctattacgcc agctggcgaa agggggatgt gctgcaaggc 9060
   gattaagttg ggtaacgcca gggttttccc agtcacgacg ttgtaaaacg acggccagtg 9120
   aattaattcc catcttgaaa gaaatatagt ttaaatattt attgataaaa taacaagtca 9180
   ggtattatag tccaagcaaa aacataaatt tattgatgca agtttaaatt cagaaatatt 9240
   tcaataactg attatatcag ctggtacatt gccgtagatg aaagactgag tgcgatatta 9300
   tgtgtaatac ataaattgat gatatagcta gcttagctca tcgggggatc cgtcgaagct 9360
   agcttgggtc ccgctcagaa gaactcgtca agaaggcgat agaaggcgat gcgctgcgaa 9420
   tcgggagcgg cgataccgta aagcacgagg aagcggtcag cccattcgcc gccaagctct 9480
   tcagcaatat cacgggtagc caacgctatg tcctgatagc ggtccgccac acccagccgg   9540
   ccacagtcga tgaatccaga aaagcggcca ttttccacca tgatattcgg caagcaggca   9600
   tcgccatggg tcacgacgag atcctcgccg tcgggcatgc gcgccttgag cctggcgaac   9660
   agttcggctg gcgcgagccc ctgatgctct tcgtccagat catcctgatc gacaagaccg   9720
   gcttccatcc gagtacgtgc tcgctcgatg cgatgtttcg cttggtggtc gaatgggcag   9780
   gtagccggat caagcgtatg cagccgccgc attgcatcag ccatgatgga tactttctcg   9840
   gcaggagcaa ggtgagatga caggagatcc tgccccggca cttcgcccaa tagcagccag   9900
   tcccttcccg cttcagtgac aacgtcgagc acagctgcgc aaggaacgcc cgtcgtggcc   9960
   agccacgata gccgcgctgc ctcgtcctgc agttcattca gggcaccgga caggtcggtc   10020
   ttgacaaaaa gaaccgggcg cccctgcgct gacagccgga acacggcggc atcagagcag   10080
   ccgattgtct gttgtgccca gtcatagccg aatagcctct ccacccaagc ggccggagaa   10140
   cctgcgtgca atccatcttg ttcaatccaa gctcccatgg gccctcgact agagtcgaga   10200
   tctggattga gagtgaatat gagactctaa ttggataccg aggggaattt atggaacgtc   10260
   agtggagcat ttttgacaag aaatatttgc tagctgatag tgaccttagg cgacttttga   10320
   acgcgcaata atggtttctg acgtatgtgc ttagctcatt aaactccaga aacccgcggc   10380
   tgagtggctc cttcaacgtt gcggttctgt cagttccaaa cgtaaaacgg cttgtcccgc   10440
   gtcatcggcg ggggtcataa cgtgactccc ttaattctcc gctcatgatc ttgatcccct   10500
   gcgccatcag atccttggcg gcaagaaagc catccagttt actttgcagg gcttcccaac   10560
   cttaccagag ggcgccccag ctggcaattc cggttcgctt gctgtccata aaaccgccca   10620
   gtctagctat cgccatgtaa gcccactgca agctacctgc tttctctttg cgcttgcgtt   10680
   ttcccttgtc cagatagccc agtagctgac attcatccgg ggtcagcacc gtttctgcgg   10740
   actggctttc tacgtgttcc gcttccttta gcagcccttg cgccctgagt gcttgcggca   10800
   gcgtgaagct tgcatgcctg caggtcgacg gcgcgccgag ctcctcgagc aaatttacac   10860
   attgccacta aacgtctaaa cccttgtaat ttgtttttgt tttactatgt gtgttatgta   10920
   tttgatttgc gataaatttt tatatttggt actaaattta taacaccttt tatgctaacg   10980
   tttgccaaca cttagcaatt tgcaagttga ttaattgatt ctaaattatt tttgtcttct   11040
   aaatacatat actaatcaac tggaaatgta aatatttgct aatatttcta ctataggaga   11100
   attaaagtga gtgaatatgg taccacaagg tttggagatt taattgttgc aatgctgcat   11160
   ggatggcata tacaccaaac attcaataat tcttgaggat aataatggta ccacacaaga   11220
   tttgaggtgc atgaacgtca cgtggacaaa aggtttagta atttttcaag acaacaatgt   11280
   taccacacac aagttttgag gtgcatgcat ggatgccctg tggaaagttt aaaaatattt   11340
   tggaaatgat ttgcatggaa gccatgtgta aaaccatgac atccacttgg aggatgcaat   11400
   aatgaagaaa actacaaatt tacatgcaac tagttatgca tgtagtctat ataatgagga   11460
   ttttgcaata ctttcattca tacacactca ctaagtttta cacgattata atttcttcat   11520
   agccagccca ccgcggtggg cggccgcctg cagtctagaa ggcctcctgc tttaatgaga   11580
   tatgcgagac gcctatgatc gcatgatatt tgctttcaat tctgttgtgc acgttgtaaa   11640
   aaacctgagc atgtgtagct cagatcctta ccgccggttt cggttcattc taatgaatat   11700
   atcacccgtt actatcgtat ttttatgaat aatattctcc gttcaattta ctgattgtcc   11760
   gtcgacgaat tcgagctcgg cgcgcctcta gaggatcgat gaattcagat cggctgagtg   11820
   gctccttcaa cgttgcggtt ctgtcagttc caaacgtaaa acggcttgtc ccgcgtcatc   11880
   ggcgggggtc ataacgtgac tcccttaatt ctccgctcat gatcagattg tcgtttcccg   11940
   ccttcagttt aaactatcag tgtttgacag gatatattgg cgggtaaacc taagagaaaa   12000
   gagcgtttat tagaataatc ggatatttaa aagggcgtga aaaggtttat ccttcgtcca   12060
   tttgtatgtg catgccaacc acagggttcc cca                                12093
<210> 65
   <211> 12085
   <212> DNA
   <213> artificial sequence
<220>
   <221> misc_feature
   <223> pflanzlicher Expressionsvektor mit einer Promotor-Terminator-Expr essionskassette
<400> 65
   gatctggcgc cggccagcga gacgagcaag attggccgcc gcccgaaacg atccgacagc     60
   gcgcccagca caggtgcgca ggcaaattgc accaacgcat acagcgccag cagaatgcca    120
   tagtgggcgg tgacgtcgtt cgagtgaacc agatcgcgca ggaggcccgg cagcaccggc    180
   ataatcaggc cgatgccgac agcgtcgagc gcgacagtgc tcagaattac gatcaggggt    240
   atgttgggtt tcacgtctgg cctccggacc agcctccgct ggtccgattg aacgcgcgga    300
   ttctttatca ctgataagtt ggtggacata ttatgtttat cagtgataaa gtgtcaagca    360
   tgacaaagtt gcagccgaat acagtgatcc gtgccgccct ggacctgttg aacgaggtcg    420
   gcgtagacgg tctgacgaca cgcaaactgg cggaacggtt gggggttcag cagccggcgc     480
   tttactggca cttcaggaac aagcgggcgc tgctcgacgc actggccgaa gccatgctgg    540
   cggagaatca tacgcattcg gtgccgagag ccgacgacga ctggcgctca tttctgatcg    600
   ggaatgcccg cagcttcagg caggcgctgc tcgcctaccg cgatggcgcg cgcatccatg    660
   ccggcacgcg accgggcgca ccgcagatgg aaacggccga cgcgcagctt cgcttcctct    720
   gcgaggcggg tttttcggcc ggggacgccg tcaatgcgct gatgacaatc agctacttca    780
   ctgttggggc cgtgcttgag gagcaggccg gcgacagcga tgccggcgag cgcggcggca    840
   ccgttgaaca ggctccgctc tcgccgctgt tgcgggccgc gatagacgcc ttcgacgaag    900
   ccggtccgga cgcagcgttc gagcagggac tcgcggtgat tgtcgatgga ttggcgaaaa    960
   ggaggctcgt tgtcaggaac gttgaaggac cgagaaaggg tgacgattga tcaggaccgc   1020
   tgccggagcg caacccactc actacagcag agccatgtag acaacatccc ctcccccttt   1080
   ccaccgcgtc agacgcccgt agcagcccgc tacgggcttt ttcatgccct gccctagcgt   1140
   ccaagcctca cggccgcgct cggcctctct ggcggccttc tggcgctctt ccgcttcctc   1200
   gctcactgac tcgctgcgct cggtcgttcg gctgcggcga gcggtatcag ctcactcaaa   1260
   ggcggtaata cggttatcca cagaatcagg ggataacgca ggaaagaaca tgtgagcaaa   1320
   aggccagcaa aaggccagga accgtaaaaa ggccgcgttg ctggcgtttt tccataggct   1380
   ccgcccccct gacgagcatc acaaaaatcg acgctcaagt cagaggtggc gaaacccgac    1440
   aggactataa agataccagg cgtttccccc tggaagctcc ctcgtgcgct ctcctgttcc   1500
   gaccctgccg cttaccggat acctgtccgc ctttctccct tcgggaagcg tggcgctttt   1560
   ccgctgcata accctgcttc ggggtcatta tagcgatttt ttcggtatat ccatcctttt   1620
   tcgcacgata tacaggattt tgccaaaggg ttcgtgtaga ctttccttgg tgtatccaac   1680
   ggcgtcagcc gggcaggata ggtgaagtag gcccacccgc gagcgggtgt tccttcttca   1740
   ctgtccctta ttcgcacctg gcggtgctca acgggaatcc tgctctgcga ggctggccgg   1800
   ctaccgccgg cgtaacagat gagggcaagc ggatggctga tgaaaccaag ccaaccagga   1860
   agggcagccc acctatcaag gtgtactgcc ttccagacga acgaagagcg attgaggaaa   1920
   aggcggcggc ggccggcatg agcctgtcgg cctacctgct ggccgtcggc cagggctaca   1980
   aaatcacggg cgtcgtggac tatgagcacg tccgcgagct ggcccgcatc aatggcgacc   2040
   tgggccgcct gggcggcctg ctgaaactct ggctcaccga cgacccgcgc acggcgcggt   2100
   tcggtgatgc cacgatcctc gccctgctgg cgaagatcga agagaagcag gacgagcttg   2160
   gcaaggtcat gatgggcgtg gtccgcccga gggcagagcc atgacttttt tagccgctaa   2220
   aacggccggg gggtgcgcgt gattgccaag cacgtcccca tgcgctccat caagaagagc   2280
   gacttcgcgg agctggtgaa gtacatcacc gacgagcaag gcaagaccga gcgcctttgc   2340
   gacgctcacc gggctggttg ccctcgccgc tgggctggcg gccgtctatg gccctgcaaa   2400
   cgcgccagaa acgccgtcga agccgtgtgc gagacaccgc ggccgccggc gttgtggata   2460
   cctcgcggaa aacttggccc tcactgacag atgaggggcg gacgttgaca cttgaggggc   2520
   cgactcaccc ggcgcggcgt tgacagatga ggggcaggct cgatttcggc cggcgacgtg   2580
   gagctggcca gcctcgcaaa tcggcgaaaa cgcctgattt tacgcgagtt tcccacagat   2640
   gatgtggaca agcctgggga taagtgccct gcggtattga cacttgaggg gcgcgactac   2700
   tgacagatga ggggcgcgat ccttgacact tgaggggcag agtgctgaca gatgaggggc   2760
   gcacctattg acatttgagg ggctgtccac aggcagaaaa tccagcattt gcaaggtttt   2820
   ccgcccgttt ttcggccacc gctaacctgt cttttaacct gcttttaaac caatatttat   2880
   aaaccttgtt tttaaccagg gctgcgccct gtgcgcgtga ccgcgcacgc cgaagggggg   2940
   tgccccccct tctcgaaccc tcccggcccg ctaacgcggg cctcccatcc ccccaggggc    3000
   tgcgcccctc ggccgcgaac ggcctcaccc caaaaatggc agcgctggca gtccttgcca 3060
   ttgccgggat cggggcagta acgggatggg cgatcagccc gagcgcgacg cccggaagca 3120
   ttgacgtgcc gcaggtgctg gcatcgacat tcagcgacca ggtgccgggc agtgagggcg 3180
   gcggcctggg tggcggcctg cccttcactt cggccgtcgg ggcattcacg gacttcatgg 3240
   cggggccggc aatttttacc ttgggcattc ttggcatagt ggtcgcgggt gccgtgctcg 3300
   tgttcggggg tgcgataaac ccagcgaacc atttgaggtg ataggtaaga ttataccgag 3360
   gtatgaaaac gagaattgga cctttacaga attactctat gaagcgccat atttaaaaag 3420
   ctaccaagac gaagaggatg aagaggatga ggaggcagat tgccttgaat atattgacaa 3480
   tactgataag ataatatatc ttttatatag aagatatcgc cgtatgtaag gatttcaggg 3540
   ggcaaggcat aggcagcgcg cttatcaata tatctataga atgggcaaag cataaaaact 3600
   tgcatggact aatgcttgaa acccaggaca ataaccttat agcttgtaaa ttctatcata 3660
   attgggtaat gactccaact tattgatagt gttttatgtt cagataatgc ccgatgactt 3720
   tgtcatgcag ctccaccgat tttgagaacg acagcgactt ccgtcccagc cgtgccaggt 3780
   gctgcctcag attcaggtta tgccgctcaa ttcgctgcgt atatcgcttg ctgattacgt 3840
   gcagctttcc cttcaggcgg gattcataca gcggccagcc atccgtcatc catatcacca 3900
   cgtcaaaggg tgacagcagg ctcataagac gccccagcgt cgccatagtg cgttcaccga 3960
   atacgtgcgc aacaaccgtc ttccggagac tgtcatacgc gtaaaacagc cagcgctggc 4020
   gcgatttagc cccgacatag ccccactgtt cgtccatttc cgcgcagacg atgacgtcac 4080
   tgcccggctg tatgcgcgag gttaccgact gcggcctgag ttttttaagt gacgtaaaat 4140
   cgtgttgagg ccaacgccca taatgcgggc tgttgcccgg catccaacgc cattcatggc 4200
   catatcaatg attttctggt gcgtaccggg ttgagaagcg gtgtaagtga actgcagttg 4260
   ccatgtttta cggcagtgag agcagagata gcgctgatgt ccggcggtgc ttttgccgtt 4320
   acgcaccacc ccgtcagtag ctgaacagga gggacagctg atagacacag aagccactgg 4380
   agcacctcaa aaacaccatc atacactaaa tcagtaagtt ggcagcatca cccataattg 4440
   tggtttcaaa atcggctccg tcgatactat gttatacgcc aactttgaaa acaactttga 4500
   aaaagctgtt ttctggtatt taaggtttta gaatgcaagg aacagtgaat tggagttcgt 4560
   cttgttataa ttagcttctt ggggtatctt taaatactgt agaaaagagg aaggaaataa 4620
   taaatggcta aaatgagaat atcaccggaa ttgaaaaaac tgatcgaaaa ataccgctgc 4680
   gtaaaagata cggaaggaat gtctcctgct aaggtatata agctggtggg agaaaatgaa 4740
   aacctatatt taaaaatgac ggacagccgg tataaaggga ccacctatga tgtggaacgg 4800
   gaaaaggaca tgatgctatg gctggaagga aagctgcctg ttccaaaggt cctgcacttt 4860
   gaacggcatg atggctggag caatctgctc atgagtgagg ccgatggcgt cctttgctcg 4920
   gaagagtatg aagatgaaca aagccctgaa aagattatcg agctgtatgc ggagtgcatc 4980
   aggctctttc actccatcga catatcggat tgtccctata cgaatagctt agacagccgc 5040
   ttagccgaat tggattactt actgaataac gatctggccg atgtggattg cgaaaactgg 5100
   gaagaagaca ctccatttaa agatccgcgc gagctgtatg attttttaaa gacggaaaag 5160
   cccgaagagg aacttgtctt ttcccacggc gacctgggag acagcaacat ctttgtgaaa 5220
   gatggcaaag taagtggctt tattgatctt gggagaagcg gcagggcgga caagtggtat 5280
   gacattgcct tctgcgtccg gtcgatcagg gaggatatcg gggaagaaca gtatgtcgag 5340
   ctattttttg acttactggg gatcaagcct gattgggaga aaataaaata ttatatttta 5400
   ctggatgaat tgttttagta cctagatgtg gcgcaacgat gccggcgaca agcaggagcg 5460
   caccgacttc ttccgcatca agtgttttgg ctctcaggcc gaggcccacg gcaagtattt 5520
   gggcaagggg tcgctggtat tcgtgcaggg caagattcgg aataccaagt acgagaagga 5580
   cggccagacg gtctacggga ccgacttcat tgccgataag gtggattatc tggacaccaa 5640
   ggcaccaggc gggtcaaatc aggaataagg gcacattgcc ccggcgtgag tcggggcaat 5700
   cccgcaagga gggtgaatga atcggacgtt tgaccggaag gcatacaggc aagaactgat 5760
   cgacgcgggg ttttccgccg aggatgccga aaccatcgca agccgcaccg tcatgcgtgc 5820
   gccccgcgaa accttccagt ccgtcggctc gatggtccag caagctacgg ccaagatcga 5880
   gcgcgacagc gtgcaactgg ctccccctgc cctgcccgcg ccatcggccg ccgtggagcg 5940
   ttcgcgtcgt ctcgaacagg aggcggcagg tttggcgaag tcgatgacca tcgacacgcg 6000
   aggaactatg acgaccaaga agcgaaaaac cgccggcgag gacctggcaa aacaggtcag 6060
   cgaggccaag caggccgcgt tgctgaaaca cacgaagcag cagatcaagg aaatgcagct 6120
   ttccttgttc gatattgcgc cgtggccgga cacgatgcga gcgatgccaa acgacacggc 6180
   ccgctctgcc ctgttcacca cgcgcaacaa gaaaatcccg cgcgaggcgc tgcaaaacaa 6240
   ggtcattttc cacgtcaaca aggacgtgaa gatcacctac accggcgtcg agctgcgggc   6300
   cgacgatgac gaactggtgt ggcagcaggt gttggagtac gcgaagcgca cccctatcgg   6360
   cgagccgatc accttcacgt tctacgagct ttgccaggac ctgggctggt cgatcaatgg   6420
   ccggtattac acgaaggccg aggaatgcct gtcgcgccta caggcgacgg cgatgggctt   6480
   cacgtccgac cgcgttgggc acctggaatc ggtgtcgctg ctgcaccgct tccgcgtcct   6540
   ggaccgtggc aagaaaacgt cccgttgcca ggtcctgatc gacgaggaaa tcgtcgtgct   6600
   gtttgctggc gaccactaca cgaaattcat atgggagaag taccgcaagc tgtcgccgac   6660
   ggcccgacgg atgttcgact atttcagctc gcaccgggag ccgtacccgc tcaagctgga   6720
   aaccttccgc ctcatgtgcg gatcggattc cacccgcgtg aagaagtggc gcgagcaggt   6780
   cggcgaagcc tgcgaagagt tgcgaggcag cggcctggtg gaacacgcct gggtcaatga   6840
   tgacctggtg cattgcaaac gctagggcct tgtggggtca gttccggctg ggggttcagc   6900
   agccagcgct ttactggcat ttcaggaaca agcgggcact gctcgacgca cttgcttcgc   6960
   tcagtatcgc tcgggacgca cggcgcgctc tacgaactgc cgataaacag aggattaaaa   7020
   ttgacaattg tgattaaggc tcagattcga cggcttggag cggccgacgt gcaggatttc   7080
   cgcgagatcc gattgtcggc cctgaagaaa gctccagaga tgttcgggtc cgtttacgag   7140
   cacgaggaga aaaagcccat ggaggcgttc gctgaacggt tgcgagatgc cgtggcattc   7200
   ggcgcctaca tcgacggcga gatcattggg ctgtcggtct tcaaacagga ggacggcccc   7260
   aaggacgctc acaaggcgca tctgtccggc gttttcgtgg agcccgaaca gcgaggccga   7320
   ggggtcgccg gtatgctgct gcgggcgttg ccggcgggtt tattgctcgt gatgatcgtc   7380
   cgacagattc caacgggaat ctggtggatg cgcatcttca tcctcggcgc acttaatatt   7440
   tcgctattct ggagcttgtt gtttatttcg gtctaccgcc tgccgggcgg ggtcgcggcg   7500
   acggtaggcg ctgtgcagcc gctgatggtc gtgttcatct ctgccgctct gctaggtagc   7560
   ccgatacgat tgatggcggt cctgggggct atttgcggaa ctgcgggcgt ggcgctgttg   7620
   gtgttgacac caaacgcagc gctagatcct gtcggcgtcg cagcgggcct ggcgggggcg   7680
   gtttccatgg cgttcggaac cgtgctgacc cgcaagtggc aacctcccgt gcctctgctc   7740
   acctttaccg cctggcaact ggcggccgga ggacttctgc tcgttccagt agctttagtg   7800
   tttgatccgc caatcccgat gcctacagga accaatgttc tcggcctggc gtggctcggc   7860
   ctgatcggag cgggtttaac ctacttcctt tggttccggg ggatctcgcg actcgaacct   7920
   acagttgttt ccttactggg ctttctcagc cccagatctg gggtcgatca gccggggatg   7980
   catcaggccg acagtcggaa cttcgggtcc ccgacctgta ccattcggtg agcaatggat   8040
   aggggagttg atatcgtcaa cgttcacttc taaagaaata gcgccactca gcttcctcag   8100
   cggctttatc cagcgatttc ctattatgtc ggcatagttc tcaagatcga cagcctgtca   8160
   cggttaagcg agaaatgaat aagaaggctg ataattcgga tctctgcgag ggagatgata   8220
   tttgatcaca ggcagcaacg ctctgtcatc gttacaatca acatgctacc ctccgcgaga   8280
   tcatccgtgt ttcaaacccg gcagcttagt tgccgttctt ccgaatagca tcggtaacat   8340
   gagcaaagtc tgccgcctta caacggctct cccgctgacg ccgtcccgga ctgatgggct   8400
   gcctgtatcg agtggtgatt ttgtgccgag ctgccggtcg gggagctgtt ggctggctgg   8460
   tggcaggata tattgtggtg taaacaaatt gacgcttaga caacttaata acacattgcg   8520
   gacgttttta atgtactggg gtggtttttc ttttcaccag tgagacgggc aacagctgat   8580
   tgcccttcac cgcctggccc tgagagagtt gcagcaagcg gtccacgctg gtttgcccca   8640
   gcaggcgaaa atcctgtttg atggtggttc cgaaatcggc aaaatccctt ataaatcaaa   8700
   agaatagccc gagatagggt tgagtgttgt tccagtttgg aacaagagtc cactattaaa   8760
   gaacgtggac tccaacgtca aagggcgaaa aaccgtctat cagggcgatg gcccactacg   8820
   tgaaccatca cccaaatcaa gttttttggg gtcgaggtgc cgtaaagcac taaatcggaa   8880
   ccctaaaggg agcccccgat ttagagcttg acggggaaag ccggcgaacg tggcgagaaa   8940
   ggaagggaag aaagcgaaag gagcgggcgc cattcaggct gcgcaactgt tgggaagggc   9000
   gatcggtgcg ggcctcttcg ctattacgcc agctggcgaa agggggatgt gctgcaaggc   9060
   gattaagttg ggtaacgcca gggttttccc agtcacgacg ttgtaaaacg acggccagtg   9120
   aattaattcc catcttgaaa gaaatatagt ttaaatattt attgataaaa taacaagtca   9180
   ggtattatag tccaagcaaa aacataaatt tattgatgca agtttaaatt cagaaatatt   9240
   tcaataactg attatatcag ctggtacatt gccgtagatg aaagactgag tgcgatatta   9300
   tgtgtaatac ataaattgat gatatagcta gcttagctca tcgggggatc cgtcgaagct   9360
   agcttgggtc ccgctcagaa gaactcgtca agaaggcgat agaaggcgat gcgctgcgaa   9420
   tcgggagcgg cgataccgta aagcacgagg aagcggtcag cccattcgcc gccaagctct   9480
   tcagcaatat cacgggtagc caacgctatg tcctgatagc ggtccgccac acccagccgg    9540
   ccacagtcga tgaatccaga aaagcggcca ttttccacca tgatattcgg caagcaggca    9600
   tcgccatggg tcacgacgag atcctcgccg tcgggcatgc gcgccttgag cctggcgaac    9660
   agttcggctg gcgcgagccc ctgatgctct tcgtccagat catcctgatc gacaagaccg    9720
   gcttccatcc gagtacgtgc tcgctcgatg cgatgtttcg cttggtggtc gaatgggcag    9780
   gtagccggat caagcgtatg cagccgccgc attgcatcag ccatgatgga tactttctcg    9840
   gcaggagcaa ggtgagatga caggagatcc tgccccggca cttcgcccaa tagcagccag    9900
   tcccttcccg cttcagtgac aacgtcgagc acagctgcgc aaggaacgcc cgtcgtggcc    9960
   agccacgata gccgcgctgc ctcgtcctgc agttcattca gggcaccgga caggtcggtc   10020
   ttgacaaaaa gaaccgggcg cccctgcgct gacagccgga acacggcggc atcagagcag   10080
   ccgattgtct gttgtgccca gtcatagccg aatagcctct ccacccaagc ggccggagaa   10140
   cctgcgtgca atccatcttg ttcaatccaa gctcccatgg gccctcgact agagtcgaga   10200
   tctggattga gagtgaatat gagactctaa ttggataccg aggggaattt atggaacgtc   10260
   agtggagcat ttttgacaag aaatatttgc tagctgatag tgaccttagg cgacttttga   10320
   acgcgcaata atggtttctg acgtatgtgc ttagctcatt aaactccaga aacccgcggc   10380
   tgagtggctc cttcaacgtt gcggttctgt cagttccaaa cgtaaaacgg cttgtcccgc   10440
   gtcatcggcg ggggtcataa cgtgactccc ttaattctcc gctcatgatc ttgatcccct   10500
   gcgccatcag atccttggcg gcaagaaagc catccagttt actttgcagg gcttcccaac   10560
   cttaccagag ggcgccccag ctggcaattc cggttcgctt gctgtccata aaaccgccca   10620
   gtctagctat cgccatgtaa gcccactgca agctacctgc tttctctttg cgcttgcgtt   10680
   ttcccttgtc cagatagccc agtagctgac attcatccgg ggtcagcacc gtttctgcgg   10740
   actggctttc tacgtgttcc gcttccttta gcagcccttg cgccctgagt gcttgcggca   10800
   gcgtgaagct tgcatgcctg caggtcgacg gcgcgccgag ctcctcgagc aaatttacac   10860
   attgccacta aacgtctaaa cccttgtaat ttgtttttgt tttactatgt gtgttatgta   10920
   tttgatttgc gataaatttt tatatttggt actaaattta taacaccttt tatgctaacg   10980
   tttgccaaca cttagcaatt tgcaagttga ttaattgatt ctaaattatt tttgtcttct   11040
   aaatacatat actaatcaac tggaaatgta aatatttgct aatatttcta ctataggaga   11100
   attaaagtga gtgaatatgg taccacaagg tttggagatt taattgttgc aatgctgcat   11160
   ggatggcata tacaccaaac attcaataat tcttgaggat aataatggta ccacacaaga   11220
   tttgaggtgc atgaacgtca cgtggacaaa aggtttagta atttttcaag acaacaatgt   11280
   taccacacac aagttttgag gtgcatgcat ggatgccctg tggaaagttt aaaaatattt   11340
   tggaaatgat ttgcatggaa gccatgtgta aaaccatgac atccacttgg aggatgcaat   11400
   aatgaagaaa actacaaatt tacatgcaac tagttatgca tgtagtctat ataatgagga   11460
   ttttgcaata ctttcattca tacacactca ctaagtttta cacgattata atttcttcat   11520
   agccagcgga tccgatatcg ggcccgctag cgttaaccct gctttaatga gatatgcgag   11580
   acgcctatga tcgcatgata tttgctttca attctgttgt gcacgttgta aaaaacctga   11640
   gcatgtgtag ctcagatcct taccgccggt ttcggttcat tctaatgaat atatcacccg   11700
   ttactatcgt atttttatga ataatattct ccgttcaatt tactgattgt ccgtcgacga   11760
   attcgagctc ggcgcgcctc tagaggatcg atgaattcag atcggctgag tggctccttc   11820
   aacgttgcgg ttctgtcagt tccaaacgta aaacggcttg tcccgcgtca tcggcggggg   11880
   tcataacgtg actcccttaa ttctccgctc atgatcagat tgtcgtttcc cgccttcagt   11940
   ttaaactatc agtgtttgac aggatatatt ggcgggtaaa cctaagagaa aagagcgttt   12000
   attagaataa tcggatattt aaaagggcgt gaaaaggttt atccttcgtc catttgtatg   12060
   tgcatgccaa ccacagggtt cccca                                         12085
<210> 66
   <211> 12079
   <212> DNA
   <213> artificial sequence
<400> 66
   gatctggcgc cggccagcga gacgagcaag attggccgcc gcccgaaacg atccgacagc      60
   gcgcccagca caggtgcgca ggcaaattgc accaacgcat acagcgccag cagaatgcca 120
   tagtgggcgg tgacgtcgtt cgagtgaacc agatcgcgca ggaggcccgg cagcaccggc 180
   ataatcaggc cgatgccgac agcgtcgagc gcgacagtgc tcagaattac gatcaggggt 240
   atgttgggtt tcacgtctgg cctccggacc agcctccgct ggtccgattg aacgcgcgga 300
   ttctttatca ctgataagtt ggtggacata ttatgtttat cagtgataaa gtgtcaagca 360
   tgacaaagtt gcagccgaat acagtgatcc gtgccgccct ggacctgttg aacgaggtcg 420
   gcgtagacgg tctgacgaca cgcaaactgg cggaacggtt gggggttcag cagccggcgc 480
   tttactggca cttcaggaac aagcgggcgc tgctcgacgc actggccgaa gccatgctgg 540
   cggagaatca tacgcattcg gtgccgagag ccgacgacga ctggcgctca tttctgatcg 600
   ggaatgcccg cagcttcagg caggcgctgc tcgcctaccg cgatggcgcg cgcatccatg 660
   ccggcacgcg accgggcgca ccgcagatgg aaacggccga cgcgcagctt cgcttcctct 720
   gcgaggcggg tttttcggcc ggggacgccg tcaatgcgct gatgacaatc agctacttca 780
   ctgttggggc cgtgcttgag gagcaggccg gcgacagcga tgccggcgag cgcggcggca 840
   ccgttgaaca ggctccgctc tcgccgctgt tgcgggccgc gatagacgcc ttcgacgaag 900
   ccggtccgga cgcagcgttc gagcagggac tcgcggtgat tgtcgatgga ttggcgaaaa 960
   ggaggctcgt tgtcaggaac gttgaaggac cgagaaaggg tgacgattga tcaggaccgc 1020
   tgccggagcg caacccactc actacagcag agccatgtag acaacatccc ctcccccttt 1080
   ccaccgcgtc agacgcccgt agcagcccgc tacgggcttt ttcatgccct gccctagcgt 1140
   ccaagcctca cggccgcgct cggcctctct ggcggccttc tggcgctctt ccgcttcctc 1200
   gctcactgac tcgctgcgct cggtcgttcg gctgcggcga gcggtatcag ctcactcaaa 1260
   ggcggtaata cggttatcca cagaatcagg ggataacgca ggaaagaaca tgtgagcaaa 1320
   aggccagcaa aaggccagga accgtaaaaa ggccgcgttg ctggcgtttt tccataggct 1380
   ccgcccccct gacgagcatc acaaaaatcg acgctcaagt cagaggtggc gaaacccgac 1440
   aggactataa agataccagg cgtttccccc tggaagctcc ctcgtgcgct ctcctgttcc 1500
   gaccctgccg cttaccggat acctgtccgc ctttctccct tcgggaagcg tggcgctttt 1560
   ccgctgcata accctgcttc ggggtcatta tagcgatttt ttcggtatat ccatcctttt 1620
   tcgcacgata tacaggattt tgccaaaggg ttcgtgtaga ctttccttgg tgtatccaac 1680
   ggcgtcagcc gggcaggata ggtgaagtag gcccacccgc gagcgggtgt tccttcttca 1740
   ctgtccctta ttcgcacctg gcggtgctca acgggaatcc tgctctgcga ggctggccgg 1800
   ctaccgccgg cgtaacagat gagggcaagc ggatggctga tgaaaccaag ccaaccagga 1860
   agggcagccc acctatcaag gtgtactgcc ttccagacga acgaagagcg attgaggaaa 1920
   aggcggcggc ggccggcatg agcctgtcgg cctacctgct ggccgtcggc cagggctaca 1980
   aaatcacggg cgtcgtggac tatgagcacg tccgcgagct ggcccgcatc aatggcgacc 2040
   tgggccgcct gggcggcctg ctgaaactct ggctcaccga cgacccgcgc acggcgcggt 2100
   tcggtgatgc cacgatcctc gccctgctgg cgaagatcga agagaagcag gacgagcttg 2160
   gcaaggtcat gatgggcgtg gtccgcccga gggcagagcc atgacttttt tagccgctaa 2220
   aacggccggg gggtgcgcgt gattgccaag cacgtcccca tgcgctccat caagaagagc 2280
   gacttcgcgg agctggtgaa gtacatcacc gacgagcaag gcaagaccga gcgcctttgc 2340
   gacgctcacc gggctggttg ccctcgccgc tgggctggcg gccgtctatg gccctgcaaa 2400
   cgcgccagaa acgccgtcga agccgtgtgc gagacaccgc ggccgccggc gttgtggata 2460
   cctcgcggaa aacttggccc tcactgacag atgaggggcg gacgttgaca cttgaggggc 2520
   cgactcaccc ggcgcggcgt tgacagatga ggggcaggct cgatttcggc cggcgacgtg 2580
   gagctggcca gcctcgcaaa tcggcgaaaa cgcctgattt tacgcgagtt tcccacagat 2640
   gatgtggaca agcctgggga taagtgccct gcggtattga cacttgaggg gcgcgactac 2700
   tgacagatga ggggcgcgat ccttgacact tgaggggcag agtgctgaca gatgaggggc 2760
   gcacctattg acatttgagg ggctgtccac aggcagaaaa tccagcattt gcaagggttt 2820
   ccgcccgttt ttcggccacc gctaacctgt cttttaacct gcttttaaac caatatttat 2880
   aaaccttgtt tttaaccagg gctgcgccct gtgcgcgtga ccgcgcacgc cgaagggggg 2940
   tgccccccct tctcgaaccc tcccggcccg ctaacgcggg cctcccatcc ccccaggggc 3000
   tgcgcccctc ggccgcgaac ggcctcaccc caaaaatggc agcgctggca gtccttgcca 3060
   ttgccgggat cggggcagta acgggatggg cgatcagccc gagcgcgacg cccggaagca 3120
   ttgacgtgcc gcaggtgctg gcatcgacat tcagcgacca ggtgccgggc agtgagggcg 3180
   gcggcctggg tggcggcctg cccttcactt cggccgtcgg ggcattcacg gacttcatgg 3240
   cggggccggc aatttttacc ttgggcattc ttggcatagt ggtcgcgggt gccgtgctcg 3300
   tgttcggggg tgcgataaac ccagcgaacc atttgaggtg ataggtaaga ttataccgag 3360
   gtatgaaaac gagaattgga cctttacaga attactctat gaagcgccat atttaaaaag 3420
   ctaccaagac gaagaggatg aagaggatga ggaggcagat tgccttgaat atattgacaa 3480
   tactgataag ataatatatc ttttatatag aagatatcgc cgtatgtaag gatttcaggg 3540
   ggcaaggcat aggcagcgcg cttatcaata tatctataga atgggcaaag cataaaaact 3600
   tgcatggact aatgcttgaa acccaggaca ataaccttat agcttgtaaa ttctatcata 3660
   attgggtaat gactccaact tattgatagt gttttatgtt cagataatgc ccgatgactt 3720
   tgtcatgcag ctccaccgat tttgagaacg acagcgactt ccgtcccagc cgtgccaggt 3780
   gctgcctcag attcaggtta tgccgctcaa ttcgctgcgt atatcgcttg ctgattacgt 3840
   gcagctttcc cttcaggcgg gattcataca gcggccagcc atccgtcatc catatcacca 3900
   cgtcaaaggg tgacagcagg ctcataagac gccccagcgt cgccatagtg cgttcaccga 3960
   atacgtgcgc aacaaccgtc ttccggagac tgtcatacgc gtaaaacagc cagcgctggc 4020
   gcgatttagc cccgacatag ccccactgtt cgtccatttc cgcgcagacg atgacgtcac 4080
   tgcccggctg tatgcgcgag gttaccgact gcggcctgag ttttttaagt gacgtaaaat 4140
   cgtgttgagg ccaacgccca taatgcgggc tgttgcccgg catccaacgc cattcatggc 4200
   catatcaatg attttctggt gcgtaccggg ttgagaagcg gtgtaagtga actgcagttg 4260
   ccatgtttta cggcagtgag agcagagata gcgctgatgt ccggcggtgc ttttgccgtt 4320
   acgcaccacc ccgtcagtag ctgaacagga gggacagctg atagacacag aagccactgg 4380
   agcacctcaa aaacaccatc atacactaaa tcagtaagtt ggcagcatca cccataattg 4440
   tggtttcaaa atcggctccg tcgatactat gttatacgcc aactttgaaa acaactttga 4500
   aaaagctgtt ttctggtatt taaggtttta gaatgcaagg aacagtgaat tggagttcgt 4560
   cttgttataa ttagcttctt ggggtatctt taaatactgt agaaaagagg aaggaaataa 4620
   taaatggcta aaatgagaat atcaccggaa ttgaaaaaac tgatcgaaaa ataccgctgc 4680
   gtaaaagata cggaaggaat gtctcctgct aaggtatata agctggtggg agaaaatgaa 4740
   aacctatatt taaaaatgac ggacagccgg tataaaggga ccacctatga tgtggaacgg 4800
   gaaaaggaca tgatgctatg gctggaagga aagctgcctg ttccaaaggt cctgcacttt 4860
   gaacggcatg atggctggag caatctgctc atgagtgagg ccgatggcgt cctttgctcg 4920
   gaagagtatg aagatgaaca aagccctgaa aagattatcg agctgtatgc ggagtgcatc 4980
   aggctctttc actccatcga catatcggat tgtccctata cgaatagctt agacagccgc 5040
   ttagccgaat tggattactt actgaataac gatctggccg atgtggattg cgaaaactgg 5100
   gaagaagaca ctccatttaa agatccgcgc gagctgtatg attttttaaa gacggaaaag 5160
   cccgaagagg aacttgtctt ttcccacggc gacctgggag acagcaacat ctttgtgaaa 5220
   gatggcaaag taagtggctt tattgatctt gggagaagcg gcagggcgga caagtggtat 5280
   gacattgcct tctgcgtccg gtcgatcagg gaggatatcg gggaagaaca gtatgtcgag 5340
   ctattttttg acttactggg gatcaagcct gattgggaga aaataaaata ttatatttta 5400
   ctggatgaat tgttttagta cctagatgtg gcgcaacgat gccggcgaca agcaggagcg 5460
   caccgacttc ttccgcatca agtgttttgg ctctcaggcc gaggcccacg gcaagtattt 5520
   gggcaagggg tcgctggtat tcgtgcaggg caagattcgg aataccaagt acgagaagga 5580
   cggccagacg gtctacggga ccgacttcat tgccgataag gtggattatc tggacaccaa 5640
   ggcaccaggc gggtcaaatc aggaataagg gcacattgcc ccggcgtgag tcggggcaat 5700
   cccgcaagga gggtgaatga atcggacgtt tgaccggaag gcatacaggc aagaactgat 5760
   cgacgcgggg ttttccgccg aggatgccga aaccatcgca agccgcaccg tcatgcgtgc 5820
   gccccgcgaa accttccagt ccgtcggctc gatggtccag caagctacgg ccaagatcga 5880
   gcgcgacagc gtgcaactgg ctccccctgc cctgcccgcg ccatcggccg ccgtggagcg 5940
   ttcgcgtcgt ctcgaacagg aggcggcagg tttggcgaag tcgatgacca tcgacacgcg 6000
   aggaactatg acgaccaaga agcgaaaaac cgccggcgag gacctggcaa aacaggtcag 6060
   cgaggccaag caggccgcgt tgctgaaaca cacgaagcag cagatcaagg aaatgcagct 6120
   ttccttgttc gatattgcgc cgtggccgga cacgatgcga gcgatgccaa acgacacggc 6180
   ccgctctgcc ctgttcacca cgcgcaacaa gaaaatcccg cgcgaggcgc tgcaaaacaa 6240
   ggtcattttc cacgtcaaca aggacgtgaa gatcacctac accggcgtcg agctgcgggc 6300
   cgacgatgac gaactggtgt ggcagcaggt gttggagtac gcgaagcgca cccctatcgg 6360
   cgagccgatc accttcacgt tctacgagct ttgccaggac ctgggctggt cgatcaatgg 6420
   ccggtattac acgaaggccg aggaatgcct gtcgcgccta caggcgacgg cgatgggctt 6480
   cacgtccgac cgcgttgggc acctggaatc ggtgtcgctg ctgcaccgct tccgcgtcct 6540
   ggaccgtggc aagaaaacgt cccgttgcca ggtcctgatc gacgaggaaa tcgtcgtgct    6600
   gtttgctggc gaccactaca cgaaattcat atgggagaag taccgcaagc tgtcgccgac    6660
   ggcccgacgg atgttcgact atttcagctc gcaccgggag ccgtacccgc tcaagctgga    6720
   aaccttccgc ctcatgtgcg gatcggattc cacccgcgtg aagaagtggc gcgagcaggt    6780
   cggcgaagcc tgcgaagagt tgcgaggcag cggcctggtg gaacacgcct gggtcaatga    6840
   tgacctggtg cattgcaaac gctagggcct tgtggggtca gttccggctg ggggttcagc     6900
   agccagcgct ttactggcat ttcaggaaca agcgggcact gctcgacgca cttgcttcgc    6960
   tcagtatcgc tcgggacgca cggcgcgctc tacgaactgc cgataaacag aggattaaaa    7020
   ttgacaattg tgattaaggc tcagattcga cggcttggag cggccgacgt gcaggatttc    7080
   cgcgagatcc gattgtcggc cctgaagaaa gctccagaga tgttcgggtc cgtttacgag    7140
   cacgaggaga aaaagcccat ggaggcgttc gctgaacggt tgcgagatgc cgtggcattc    7200
   ggcgcctaca tcgacggcga gatcattggg ctgtcggtct tcaaacagga ggacggcccc    7260
   aaggacgctc acaaggcgca tctgtccggc gttttcgtgg agcccgaaca gcgaggccga    7320
   ggggtcgccg gtatgctgct gcgggcgttg ccggcgggtt tattgctcgt gatgatcgtc    7380
   cgacagattc caacgggaat ctggtggatg cgcatcttca tcctcggcgc acttaatatt    7440
   tcgctattct ggagcttgtt gtttatttcg gtctaccgcc tgccgggcgg ggtcgcggcg    7500
   acggtaggcg ctgtgcagcc gctgatggtc gtgttcatct ctgccgctct gctaggtagc    7560
   ccgatacgat tgatggcggt cctgggggct atttgcggaa ctgcgggcgt ggcgctgttg    7620
   gtgttgacac caaacgcagc gctagatcct gtcggcgtcg cagcgggcct ggcgggggcg    7680
   gtttccatgg cgttcggaac cgtgctgacc cgcaagtggc aacctcccgt gcctctgctc    7740
   acctttaccg cctggcaact ggcggccgga ggacttctgc tcgttccagt agctttagtg    7800
   tttgatccgc caatcccgat gcctacagga accaatgttc tcggcctggc gtggctcggc    7860
   ctgatcggag cgggtttaac ctacttcctt tggttccggg ggatctcgcg actcgaacct    7920
   acagttgttt ccttactggg ctttctcagc cccagatctg gggtcgatca gccggggatg     7980
   catcaggccg acagtcggaa cttcgggtcc ccgacctgta ccattcggtg agcaatggat    8040
   aggggagttg atatcgtcaa cgttcacttc taaagaaata gcgccactca gcttcctcag    8100
   cggctttatc cagcgatttc ctattatgtc ggcatagttc tcaagatcga cagcctgtca    8160
   cggttaagcg agaaatgaat aagaaggctg ataattcgga tctctgcgag ggagatgata    8220
   tttgatcaca ggcagcaacg ctctgtcatc gttacaatca acatgctacc ctccgcgaga    8280
   tcatccgtgt ttcaaacccg gcagcttagt tgccgttctt ccgaatagca tcggtaacat    8340
   gagcaaagtc tgccgcctta caacggctct cccgctgacg ccgtcccgga ctgatgggct    8400
   gcctgtatcg agtggtgatt ttgtgccgag ctgccggtcg gggagctgtt ggctggctgg    8460
   tggcaggata tattgtggtg taaacaaatt gacgcttaga caacttaata acacattgcg    8520
   gacgttttta atgtactggg gtggtttttc ttttcaccag tgagacgggc aacagctgat    8580
   tgcccttcac cgcctggccc tgagagagtt gcagcaagcg gtccacgctg gtttgcccca    8640
   gcaggcgaaa atcctgtttg atggtggttc cgaaatcggc aaaatccctt ataaatcaaa    8700
   agaatagccc gagatagggt tgagtgttgt tccagtttgg aacaagagtc cactattaaa    8760
   gaacgtggac tccaacgtca aagggcgaaa aaccgtctat cagggcgatg gcccactacg    8820
   tgaaccatca cccaaatcaa gttttttggg gtcgaggtgc cgtaaagcac taaatcggaa    8880
   ccctaaaggg agcccccgat ttagagcttg acggggaaag ccggcgaacg tggcgagaaa    8940
   ggaagggaag aaagcgaaag gagcgggcgc cattcaggct gcgcaactgt tgggaagggc    9000
   gatcggtgcg ggcctcttcg ctattacgcc agctggcgaa agggggatgt gctgcaaggc   9060
   gattaagttg ggtaacgcca gggttttccc agtcacgacg ttgtaaaacg acggccagtg    9120
   aattaattcc catcttgaaa gaaatatagt ttaaatattt attgataaaa taacaagtca    9180
   ggtattatag tccaagcaaa aacataaatt tattgatgca agtttaaatt cagaaatatt    9240
   tcaataactg attatatcag ctggtacatt gccgtagatg aaagactgag tgcgatatta    9300
   tgtgtaatac ataaattgat gatatagcta gcttagctca tcgggggatc cgtcgaagct    9360
   agcttgggtc ccgctcagaa gaactcgtca agaaggcgat agaaggcgat gcgctgcgaa    9420
   tcgggagcgg cgataccgta aagcacgagg aagcggtcag cccattcgcc gccaagctct    9480
   tcagcaatat cacgggtagc caacgctatg tcctgatagc ggtccgccac acccagccgg    9540
   ccacagtcga tgaatccaga aaagcggcca ttttccacca tgatattcgg caagcaggca    9600
   tcgccatggg tcacgacgag atcctcgccg tcgggcatgc gcgccttgag cctggcgaac    9660
   agttcggctg gcgcgagccc ctgatgctct tcgtccagat catcctgatc gacaagaccg    9720
   gcttccatcc gagtacgtgc tcgctcgatg cgatgtttcg cttggtggtc gaatgggcag    9780
   gtagccggat caagcgtatg cagccgccgc attgcatcag ccatgatgga tactttctcg   9840
   gcaggagcaa ggtgagatga caggagatcc tgccccggca cttcgcccaa tagcagccag   9900
   tcccttcccg cttcagtgac aacgtcgagc acagctgcgc aaggaacgcc cgtcgtggcc   9960
   agccacgata gccgcgctgc ctcgtcctgc agttcattca gggcaccgga caggtcggtc  10020
   ttgacaaaaa gaaccgggcg cccctgcgct gacagccgga acacggcggc atcagagcag  10080
   ccgattgtct gttgtgccca gtcatagccg aatagcctct ccacccaagc ggccggagaa  10140
   cctgcgtgca atccatcttg ttcaatccaa gctcccatgg gccctcgact agagtcgaga  10200
   tctggattga gagtgaatat gagactctaa ttggataccg aggggaattt atggaacgtc  10260
   agtggagcat ttttgacaag aaatatttgc tagctgatag tgaccttagg cgacttttga  10320
   acgcgcaata atggtttctg acgtatgtgc ttagctcatt aaactccaga aacccgcggc  10380
   tgagtggctc cttcaacgtt gcggttctgt cagttccaaa cgtaaaacgg cttgtcccgc  10440
   gtcatcggcg ggggtcataa cgtgactccc ttaattctcc gctcatgatc ttgatcccct  10500
   gcgccatcag atccttggcg gcaagaaagc catccagttt actttgcagg gcttcccaac  10560
   cttaccagag ggcgccccag ctggcaattc cggttcgctt gctgtccata aaaccgccca  10620
   gtctagctat cgccatgtaa gcccactgca agctacctgc tttctctttg cgcttgcgtt  10680
   ttcccttgtc cagatagccc agtagctgac attcatccgg ggtcagcacc gtttctgcgg  10740
   actggctttc tacgtgttcc gcttccttta gcagcccttg cgccctgagt gcttgcggca  10800
   gcgtgaagct tgcatgcctg caggtcgacg gcgcgccgag ctcctcgagc aaatttacac  10860
   attgccacta aacgtctaaa cccttgtaat ttgtttttgt tttactatgt gtgttatgta  10920
   tttgatttgc gataaatttt tatatttggt actaaattta taacaccttt tatgctaacg  10980
   tttgccaaca cttagcaatt tgcaagttga ttaattgatt ctaaattatt tttgtcttct  11040
   aaatacatat actaatcaac tggaaatgta aatatttgct aatatttcta ctataggaga  11100
   attaaagtga gtgaatatgg taccacaagg tttggagatt taattgttgc aatgctgcat  11160
   ggatggcata tacaccaaac attcaataat tcttgaggat aataatggta ccacacaaga  11220
   tttgaggtgc atgaacgtca cgtggacaaa aggtttagta atttttcaag acaacaatgt  11280
   taccacacac aagttttgag gtgcatgcat ggatgccctg tggaaagttt aaaaatattt  11340
   tggaaatgat ttgcatggaa gccatgtgta aaaccatgac atccacttgg aggatgcaat  11400
   aatgaagaaa actacaaatt tacatgcaac tagttatgca tgtagtctat ataatgagga  11460
   ttttgcaata ctttcattca tacacactca ctaagtttta cacgattata atttcttcat  11520
   agccagcaga tctgccggca tcgatcccgg gccatggcct gctttaatga gatatgcgag  11580
   acgcctatga tcgcatgata tttgctttca attctgttgt gcacgttgta aaaaacctga  11640
   gcatgtgtag ctcagatcct taccgccggt ttcggttcat tctaatgaat atatcacccg  11700
   ttactatcgt atttttatga ataatattct ccgttcaatt tactgattgt ccgtcgacga  11760
   gctcggcgcg cctctagagg atcgatgaat tcagatcggc tgagtggctc cttcaacgtt  11820
   gcggttctgt cagttccaaa cgtaaaacgg cttgtcccgc gtcatcggcg ggggtcataa  11880
   cgtgactccc ttaattctcc gctcatgatc agattgtcgt ttcccgcctt cagtttaaac  11940
   tatcagtgtt tgacaggata tattggcggg taaacctaag agaaaagagc gtttattaga  12000
   ataatcggat atttaaaagg gcgtgaaaag gtttatcctt cgtccatttg tatgtgcatg  12060
   ccaaccacag ggttcccca                                                12079
<210> 67
   <211> 13002
   <212> DNA
   <213> artificial sequence
<220>
   <223> pflanzlicher Expressionsvektor mit zwei Promotor-Terminator-Expre ssionskassetten
<400> 67
   gatctggcgc cggccagcga gacgagcaag attggccgcc gcccgaaacg atccgacagc     60
   gcgcccagca caggtgcgca ggcaaattgc accaacgcat acagcgccag cagaatgcca    120
   tagtgggcgg tgacgtcgtt cgagtgaacc agatcgcgca ggaggcccgg cagcaccggc    180
   ataatcaggc cgatgccgac agcgtcgagc gcgacagtgc tcagaattac gatcaggggt 240
   atgttgggtt tcacgtctgg cctccggacc agcctccgct ggtccgattg aacgcgcgga 300
   ttctttatca ctgataagtt ggtggacata ttatgtttat cagtgataaa gtgtcaagca 360
   tgacaaagtt gcagccgaat acagtgatcc gtgccgccct ggacctgttg aacgaggtcg 420
   gcgtagacgg tctgacgaca cgcaaactgg cggaacggtt gggggttcag cagccggcgc 480
   tttactggca cttcaggaac aagcgggcgc tgctcgacgc actggccgaa gccatgctgg 540
   cggagaatca tacgcattcg gtgccgagag ccgacgacga ctggcgctca tttctgatcg 600
   ggaatgcccg cagcttcagg caggcgctgc tcgcctaccg cgatggcgcg cgcatccatg 660
   ccggcacgcg accgggcgca ccgcagatgg aaacggccga cgcgcagctt cgcttcctct 720
   gcgaggcggg tttttcggcc ggggacgccg tcaatgcgct gatgacaatc agctacttca 780
   ctgttggggc cgtgcttgag gagcaggccg gcgacagcga tgccggcgag cgcggcggca 840
   ccgttgaaca ggctccgctc tcgccgctgt tgcgggccgc gatagacgcc ttcgacgaag 900
   ccggtccgga cgcagcgttc gagcagggac tcgcggtgat tgtcgatgga ttggcgaaaa 960
   ggaggctcgt tgtcaggaac gttgaaggac cgagaaaggg tgacgattga tcaggaccgc 1020
   tgccggagcg caacccactc actacagcag agccatgtag acaacatccc ctcccccttt 1080
   ccaccgcgtc agacgcccgt agcagcccgc tacgggcttt ttcatgccct gccctagcgt 1140
   ccaagcctca cggccgcgct cggcctctct ggcggccttc tggcgctctt ccgcttcctc 1200
   gctcactgac tcgctgcgct cggtcgttcg gctgcggcga gcggtatcag ctcactcaaa 1260
   ggcggtaata cggttatcca cagaatcagg ggataacgca ggaaagaaca tgtgagcaaa 1320
   aggccagcaa aaggccagga accgtaaaaa ggccgcgttg ctggcgtttt tccataggct 1380
   ccgcccccct gacgagcatc acaaaaatcg acgctcaagt cagaggtggc gaaacccgac 1440
   aggactataa agataccagg cgtttccccc tggaagctcc ctcgtgcgct ctcctgttcc 1500
   gaccctgccg cttaccggat acctgtccgc ctttctccct tcgggaagcg tggcgctttt 1560
   ccgctgcata accctgcttc ggggtcatta tagcgatttt ttcggtatat ccatcctttt 1620
   tcgcacgata tacaggattt tgccaaaggg ttcgtgtaga ctttccttgg tgtatccaac 1680
   ggcgtcagcc gggcaggata ggtgaagtag gcccacccgc gagcgggtgt tccttcttca 1740
   ctgtccctta ttcgcacctg gcggtgctca acgggaatcc tgctctgcga ggctggccgg 1800
   ctaccgccgg cgtaacagat gagggcaagc ggatggctga tgaaaccaag ccaaccagga 1860
   agggcagccc acctatcaag gtgtactgcc ttccagacga acgaagagcg attgaggaaa 1920
   aggcggcggc ggccggcatg agcctgtcgg cctacctgct ggccgtcggc cagggctaca 1980
   aaatcacggg cgtcgtggac tatgagcacg tccgcgagct ggcccgcatc aatggcgacc 2040
   tgggccgcct gggcggcctg ctgaaactct ggctcaccga cgacccgcgc acggcgcggt 2100
   tcggtgatgc cacgatcctc gccctgctgg cgaagatcga agagaagcag gacgagcttg 2160
   gcaaggtcat gatgggcgtg gtccgcccga gggcagagcc atgacttttt tagccgctaa 2220
   aacggccggg gggtgcgcgt gattgccaag cacgtcccca tgcgctccat caagaagagc 2280
   gacttcgcgg agctggtgaa gtacatcacc gacgagcaag gcaagaccga gcgcctttgc 2340
   gacgctcacc gggctggttg ccctcgccgc tgggctggcg gccgtctatg gccctgcaaa 2400
   cgcgccagaa acgccgtcga agccgtgtgc gagacaccgc ggccgccggc gttgtggata 2460
   cctcgcggaa aacttggccc tcactgacag atgaggggcg gacgttgaca cttgaggggc 2520
   cgactcaccc ggcgcggcgt tgacagatga ggggcaggct cgatttcggc cggcgacgtg 2580
   gagctggcca gcctcgcaaa tcggcgaaaa cgcctgattt tacgcgagtt tcccacagat 2640
   gatgtggaca agcctgggga taagtgccct gcggtattga cacttgaggg gcgcgactac 2700
   tgacagatga ggggcgcgat ccttgacact tgaggggcag agtgctgaca gatgaggggc 2760
   gcacctattg acatttgagg ggctgtccac aggcagaaaa tccagcattt gcaagggttt 2820
   ccgcccgttt ttcggccacc gctaacctgt cttttaacct gcttttaaac caatatttat 2880
   aaaccttgtt tttaaccagg gctgcgccct gtgcgcgtga ccgcgcacgc cgaagggggg 2940
   tgccccccct tctcgaaccc tcccggcccg ctaacgcggg cctcccatcc ccccaggggc 3000
   tgcgcccctc ggccgcgaac ggcctcaccc caaaaatggc agcgctggca gtccttgcca 3060
   ttgccgggat cggggcagta acgggatggg cgatcagccc gagcgcgacg cccggaagca 3120
   ttgacgtgcc gcaggtgctg gcatcgacat tcagcgacca ggtgccgggc agtgagggcg 3180
   gcggcctggg tggcggcctg cccttcactt cggccgtcgg ggcattcacg gacttcatgg 3240
   cggggccggc aatttttacc ttgggcattc ttggcatagt ggtcgcgggt gccgtgctcg 3300
   tgttcggggg tgcgataaac ccagcgaacc atttgaggtg ataggtaaga ttataccgag 3360
   gtatgaaaac gagaattgga cctttacaga attactctat gaagcgccat atttaaaaag 3420
   ctaccaagac gaagaggatg aagaggatga ggaggcagat tgccttgaat atattgacaa   3480
   tactgataag ataatatatc ttttatatag aagatatcgc cgtatgtaag gatttcaggg   3540
   ggcaaggcat aggcagcgcg cttatcaata tatctataga atgggcaaag cataaaaact   3600
   tgcatggact aatgcttgaa acccaggaca ataaccttat agcttgtaaa ttctatcata   3660
   attgggtaat gactccaact tattgatagt gttttatgtt cagataatgc ccgatgactt   3720
   tgtcatgcag ctccaccgat tttgagaacg acagcgactt ccgtcccagc cgtgccaggt   3780
   gctgcctcag attcaggtta tgccgctcaa ttcgctgcgt atatcgcttg ctgattacgt   3840
   gcagctttcc cttcaggcgg gattcataca gcggccagcc atccgtcatc catatcacca   3900
   cgtcaaaggg tgacagcagg ctcataagac gccccagcgt cgccatagtg cgttcaccga   3960
   atacgtgcgc aacaaccgtc ttccggagac tgtcatacgc gtaaaacagc cagcgctggc   4020
   gcgatttagc cccgacatag ccccactgtt cgtccatttc cgcgcagacg atgacgtcac   4080
   tgcccggctg tatgcgcgag gttaccgact gcggcctgag ttttttaagt gacgtaaaat   4140
   cgtgttgagg ccaacgccca taatgcgggc tgttgcccgg catccaacgc cattcatggc   4200
   catatcaatg attttctggt gcgtaccggg ttgagaagcg gtgtaagtga actgcagttg   4260
   ccatgtttta cggcagtgag agcagagata gcgctgatgt ccggcggtgc ttttgccgtt   4320
   acgcaccacc ccgtcagtag ctgaacagga gggacagctg atagacacag aagccactgg   4380
   agcacctcaa aaacaccatc atacactaaa tcagtaagtt ggcagcatca cccataattg   4440
   tggtttcaaa atcggctccg tcgatactat gttatacgcc aactttgaaa acaactttga   4500
   aaaagctgtt ttctggtatt taaggtttta gaatgcaagg aacagtgaat tggagttcgt   4560
   cttgttataa ttagcttctt ggggtatctt taaatactgt agaaaagagg aaggaaataa   4620
   taaatggcta aaatgagaat atcaccggaa ttgaaaaaac tgatcgaaaa ataccgctgc   4680
   gtaaaagata cggaaggaat gtctcctgct aaggtatata agctggtggg agaaaatgaa   4740
   aacctatatt taaaaatgac ggacagccgg tataaaggga ccacctatga tgtggaacgg   4800
   gaaaaggaca tgatgctatg gctggaagga aagctgcctg ttccaaaggt cctgcacttt   4860
   gaacggcatg atggctggag caatctgctc atgagtgagg ccgatggcgt cctttgctcg   4920
   gaagagtatg aagatgaaca aagccctgaa aagattatcg agctgtatgc ggagtgcatc   4980
   aggctctttc actccatcga catatcggat tgtccctata cgaatagctt agacagccgc   5040
   ttagccgaat tggattactt actgaataac gatctggccg atgtggattg cgaaaactgg   5100
   gaagaagaca ctccatttaa agatccgcgc gagctgtatg attttttaaa gacggaaaag   5160
   cccgaagagg aacttgtctt ttcccacggc gacctgggag acagcaacat ctttgtgaaa   5220
   gatggcaaag taagtggctt tattgatctt gggagaagcg gcagggcgga caagtggtat   5280
   gacattgcct tctgcgtccg gtcgatcagg gaggatatcg gggaagaaca gtatgtcgag   5340
   ctattttttg acttactggg gatcaagcct gattgggaga aaataaaata ttatatttta   5400
   ctggatgaat tgttttagta cctagatgtg gcgcaacgat gccggcgaca agcaggagcg   5460
   caccgacttc ttccgcatca agtgttttgg ctctcaggcc gaggcccacg gcaagtattt   5520
   gggcaagggg tcgctggtat tcgtgcaggg caagattcgg aataccaagt acgagaagga   5580
   cggccagacg gtctacggga ccgacttcat tgccgataag gtggattatc tggacaccaa   5640
   ggcaccaggc gggtcaaatc aggaataagg gcacattgcc ccggcgtgag tcggggcaat   5700
   cccgcaagga gggtgaatga atcggacgtt tgaccggaag gcatacaggc aagaactgat   5760
   cgacgcgggg ttttccgccg aggatgccga aaccatcgca agccgcaccg tcatgcgtgc   5820
   gccccgcgaa accttccagt ccgtcggctc gatggtccag caagctacgg ccaagatcga   5880
   gcgcgacagc gtgcaactgg ctccccctgc cctgcccgcg ccatcggccg ccgtggagcg   5940
   ttcgcgtcgt ctcgaacagg aggcggcagg tttggcgaag tcgatgacca tcgacacgcg   6000
   aggaactatg acgaccaaga agcgaaaaac cgccggcgag gacctggcaa aacaggtcag   6060
   cgaggccaag caggccgcgt tgctgaaaca cacgaagcag cagatcaagg aaatgcagct   6120
   ttccttgttc gatattgcgc cgtggccgga cacgatgcga gcgatgccaa acgacacggc   6180
   ccgctctgcc ctgttcacca cgcgcaacaa gaaaatcccg cgcgaggcgc tgcaaaacaa   6240
   ggtcattttc cacgtcaaca aggacgtgaa gatcacctac accggcgtcg agctgcgggc   6300
   cgacgatgac gaactggtgt ggcagcaggt gttggagtac gcgaagcgca cccctatcgg   6360
   cgagccgatc accttcacgt tctacgagct ttgccaggac ctgggctggt cgatcaatgg   6420
   ccggtattac acgaaggccg aggaatgcct gtcgcgccta caggcgacgg cgatgggctt   6480
   cacgtccgac cgcgttgggc acctggaatc ggtgtcgctg ctgcaccgct tccgcgtcct   6540
   ggaccgtggc aagaaaacgt cccgttgcca ggtcctgatc gacgaggaaa tcgtcgtgct   6600
   gtttgctggc gaccactaca cgaaattcat atgggagaag taccgcaagc tgtcgccgac   6660
   ggcccgacgg atgttcgact atttcagctc gcaccgggag ccgtacccgc tcaagctgga   6720
   aaccttccgc ctcatgtgcg gatcggattc cacccgcgtg aagaagtggc gcgagcaggt   6780
   cggcgaagcc tgcgaagagt tgcgaggcag cggcctggtg gaacacgcct gggtcaatga   6840
   tgacctggtg cattgcaaac gctagggcct tgtggggtca gttccggctg ggggttcagc   6900
   agccagcgct ttactggcat ttcaggaaca agcgggcact gctcgacgca cttgcttcgc   6960
   tcagtatcgc tcgggacgca cggcgcgctc tacgaactgc cgataaacag aggattaaaa   7020
   ttgacaattg tgattaaggc tcagattcga cggcttggag cggccgacgt gcaggatttc   7080
   cgcgagatcc gattgtcggc cctgaagaaa gctccagaga tgttcgggtc cgtttacgag   7140
   cacgaggaga aaaagcccat ggaggcgttc gctgaacggt tgcgagatgc cgtggcattc   7200
   ggcgcctaca tcgacggcga gatcattggg ctgtcggtct tcaaacagga ggacggcccc   7260
   aaggacgctc acaaggcgca tctgtccggc gttttcgtgg agcccgaaca gcgaggccga   7320
   ggggtcgccg gtatgctgct gcgggcgttg ccggcgggtt tattgctcgt gatgatcgtc   7380
   cgacagattc caacgggaat ctggtggatg cgcatcttca tcctcggcgc acttaatatt   7440
   tcgctattct ggagcttgtt gtttatttcg gtctaccgcc tgccgggcgg ggtcgcggcg   7500
   acggtaggcg ctgtgcagcc gctgatggtc gtgttcatct ctgccgctct gctaggtagc   7560
   ccgatacgat tgatggcggt cctgggggct atttgcggaa ctgcgggcgt ggcgctgttg   7620
   gtgttgacac caaacgcagc gctagatcct gtcggcgtcg cagcgggcct ggcgggggcg   7680
   gtttccatgg cgttcggaac cgtgctgacc cgcaagtggc aacctcccgt gcctctgctc   7740
   acctttaccg cctggcaact ggcggccgga ggacttctgc tcgttccagt agctttagtg   7800
   tttgatccgc caatcccgat gcctacagga accaatgttc tcggcctggc gtggctcggc   7860
   ctgatcggag cgggtttaac ctacttcctt tggttccggg ggatctcgcg actcgaacct   7920
   acagttgttt ccttactggg ctttctcagc cccagatctg gggtcgatca gccggggatg   7980
   catcaggccg acagtcggaa cttcgggtcc ccgacctgta ccattcggtg agcaatggat   8040
   aggggagttg atatcgtcaa cgttcacttc taaagaaata gcgccactca gcttcctcag   8100
   cggctttatc cagcgatttc ctattatgtc ggcatagttc tcaagatcga cagcctgtca   8160
   cggttaagcg agaaatgaat aagaaggctg ataattcgga tctctgcgag ggagatgata   8220
   tttgatcaca ggcagcaacg ctctgtcatc gttacaatca acatgctacc ctccgcgaga   8280
   tcatccgtgt ttcaaacccg gcagcttagt tgccgttctt ccgaatagca tcggtaacat   8340
   gagcaaagtc tgccgcctta caacggctct cccgctgacg ccgtcccgga ctgatgggct   8400
   gcctgtatcg agtggtgatt ttgtgccgag ctgccggtcg gggagctgtt ggctggctgg   8460
   tggcaggata tattgtggtg taaacaaatt gacgcttaga caacttaata acacattgcg   8520
   gacgttttta atgtactggg gtggtttttc ttttcaccag tgagacgggc aacagctgat   8580
   tgcccttcac cgcctggccc tgagagagtt gcagcaagcg gtccacgctg gtttgcccca   8640
   gcaggcgaaa atcctgtttg atggtggttc cgaaatcggc aaaatccctt ataaatcaaa   8700
   agaatagccc gagatagggt tgagtgttgt tccagtttgg aacaagagtc cactattaaa   8760
   gaacgtggac tccaacgtca aagggcgaaa aaccgtctat cagggcgatg gcccactacg   8820
   tgaaccatca cccaaatcaa gttttttggg gtcgaggtgc cgtaaagcac taaatcggaa   8880
   ccctaaaggg agcccccgat ttagagcttg acggggaaag ccggcgaacg tggcgagaaa   8940
   ggaagggaag aaagcgaaag gagcgggcgc cattcaggct gcgcaactgt tgggaagggc   9000
   gatcggtgcg ggcctcttcg ctattacgcc agctggcgaa agggggatgt gctgcaaggc   9060
   gattaagttg ggtaacgcca gggttttccc agtcacgacg ttgtaaaacg acggccagtg   9120
   aattaattcc catcttgaaa gaaatatagt ttaaatattt attgataaaa taacaagtca   9180
   ggtattatag tccaagcaaa aacataaatt tattgatgca agtttaaatt cagaaatatt   9240
   tcaataactg attatatcag ctggtacatt gccgtagatg aaagactgag tgcgatatta   9300
   tgtgtaatac ataaattgat gatatagcta gcttagctca tcgggggatc cgtcgaagct   9360
   agcttgggtc ccgctcagaa gaactcgtca agaaggcgat agaaggcgat gcgctgcgaa   9420
   tcgggagcgg cgataccgta aagcacgagg aagcggtcag cccattcgcc gccaagctct   9480
   tcagcaatat cacgggtagc caacgctatg tcctgatagc ggtccgccac acccagccgg   9540
   ccacagtcga tgaatccaga aaagcggcca ttttccacca tgatattcgg caagcaggca   9600
   tcgccatggg tcacgacgag atcctcgccg tcgggcatgc gcgccttgag cctggcgaac   9660
   agttcggctg gcgcgagccc ctgatgctct tcgtccagat catcctgatc gacaagaccg   9720
   gcttccatcc gagtacgtgc tcgctcgatg cgatgtttcg cttggtggtc gaatgggcag   9780
   gtagccggat caagcgtatg cagccgccgc attgcatcag ccatgatgga tactttctcg   9840
   gcaggagcaa ggtgagatga caggagatcc tgccccggca cttcgcccaa tagcagccag   9900
   tcccttcccg cttcagtgac aacgtcgagc acagctgcgc aaggaacgcc cgtcgtggcc     9960
   agccacgata gccgcgctgc ctcgtcctgc agttcattca gggcaccgga caggtcggtc    10020
   ttgacaaaaa gaaccgggcg cccctgcgct gacagccgga acacggcggc atcagagcag    10080
   ccgattgtct gttgtgccca gtcatagccg aatagcctct ccacccaagc ggccggagaa    10140
   cctgcgtgca atccatcttg ttcaatccaa gctcccatgg gccctcgact agagtcgaga    10200
   tctggattga gagtgaatat gagactctaa ttggataccg aggggaattt atggaacgtc    10260
   agtggagcat ttttgacaag aaatatttgc tagctgatag tgaccttagg cgacttttga    10320
   acgcgcaata atggtttctg acgtatgtgc ttagctcatt aaactccaga aacccgcggc    10380
   tgagtggctc cttcaacgtt gcggttctgt cagttccaaa cgtaaaacgg cttgtcccgc    10440
   gtcatcggcg ggggtcataa cgtgactccc ttaattctcc gctcatgatc ttgatcccct    10500
   gcgccatcag atccttggcg gcaagaaagc catccagttt actttgcagg gcttcccaac    10560
   cttaccagag ggcgccccag ctggcaattc cggttcgctt gctgtccata aaaccgccca    10620
   gtctagctat cgccatgtaa gcccactgca agctacctgc tttctctttg cgcttgcgtt    10680
   ttcccttgtc cagatagccc agtagctgac attcatccgg ggtcagcacc gtttctgcgg    10740
   actggctttc tacgtgttcc gcttccttta gcagcccttg cgccctgagt gcttgcggca    10800
   gcgtgaagct tgcatgcctg caggtcgacg gcgcgccgag ctcctcgagc aaatttacac    10860
   attgccacta aacgtctaaa cccttgtaat ttgtttttgt tttactatgt gtgttatgta    10920
   tttgatttgc gataaatttt tatatttggt actaaattta taacaccttt tatgctaacg    10980
   tttgccaaca cttagcaatt tgcaagttga ttaattgatt ctaaattatt tttgtcttct    11040
   aaatacatat actaatcaac tggaaatgta aatatttgct aatatttcta ctataggaga    11100
   attaaagtga gtgaatatgg taccacaagg tttggagatt taattgttgc aatgctgcat    11160
   ggatggcata tacaccaaac attcaataat tcttgaggat aataatggta ccacacaaga    11220
   tttgaggtgc atgaacgtca cgtggacaaa aggtttagta atttttcaag acaacaatgt    11280
   taccacacac aagttttgag gtgcatgcat ggatgccctg tggaaagttt aaaaatattt    11340
   tggaaatgat ttgcatggaa gccatgtgta aaaccatgac atccacttgg aggatgcaat    11400
   aatgaagaaa actacaaatt tacatgcaac tagttatgca tgtagtctat ataatgagga    11460
   ttttgcaata ctttcattca tacacactca ctaagtttta cacgattata atttcttcat    11520
   agccagccca ccgcggtggg cggccgcctg cagtctagaa ggcctcctgc tttaatgaga    11580
   tatgcgagac gcctatgatc gcatgatatt tgctttcaat tctgttgtgc acgttgtaaa    11640
   aaacctgagc atgtgtagct cagatcctta ccgccggttt cggttcattc taatgaatat    11700
   atcacccgtt actatcgtat ttttatgaat aatattctcc gttcaattta ctgattgtcc    11760
   gtcgagcaaa tttacacatt gccactaaac gtctaaaccc ttgtaatttg tttttgtttt    11820
   actatgtgtg ttatgtattt gatttgcgat aaatttttat atttggtact aaatttataa    11880
   caccttttat gctaacgttt gccaacactt agcaatttgc aagttgatta attgattcta     11940
   aattattttt gtcttctaaa tacatatact aatcaactgg aaatgtaaat atttgctaat    12000
   atttctacta taggagaatt aaagtgagtg aatatggtac cacaaggttt ggagatttaa    12060
   ttgttgcaat gctgcatgga tggcatatac accaaacatt caataattct tgaggataat    12120
   aatggtacca cacaagattt gaggtgcatg aacgtcacgt ggacaaaagg tttagtaatt    12180
   tttcaagaca acaatgttac cacacacaag ttttgaggtg catgcatgga tgccctgtgg    12240
   aaagtttaaa aatattttgg aaatgatttg catggaagcc atgtgtaaaa ccatgacatc     12300
   cacttggagg atgcaataat gaagaaaact acaaatttac atgcaactag ttatgcatgt    12360
   agtctatata atgaggattt tgcaatactt tcattcatac acactcacta agttttacac    12420
   gattataatt tcttcatagc cagcggatcc gatatcgggc ccgctagcgt taaccctgct    12480
   ttaatgagat atgcgagacg cctatgatcg catgatattt gctttcaatt ctgttgtgca    12540
   cgttgtaaaa aacctgagca tgtgtagctc agatccttac cgccggtttc ggttcattct    12600
   aatgaatata tcacccgtta ctatcgtatt tttatgaata atattctccg ttcaatttac    12660
   tgattgtccg tcgacgaatt cgagctcggc gcgcctctag aggatcgatg aattcagatc    12720
   ggctgagtgg ctccttcaac gttgcggttc tgtcagttcc aaacgtaaaa cggcttgtcc    12780
   cgcgtcatcg gcgggggtca taacgtgact cccttaattc tccgctcatg atcagattgt    12840
   cgtttcccgc cttcagttta aactatcagt gtttgacagg atatattggc gggtaaacct    12900
   aagagaaaag agcgtttatt agaataatcg gatatttaaa agggcgtgaa aaggtttatc    12960
   cttcgtccat ttgtatgtgc atgccaacca cagggttccc ca                        13002
<210> 68
   <211> 13905
   <212> DNA
   <213> artificial sequence
<220>
   <223> pflanzlicher Expressionsvektor mit drei Promotor-Terminator-Expre ssionskassetten
<400> 68
   gatctggcgc cggccagcga gacgagcaag attggccgcc gcccgaaacg atccgacagc      60
   gcgcccagca caggtgcgca ggcaaattgc accaacgcat acagcgccag cagaatgcca     120
   tagtgggcgg tgacgtcgtt cgagtgaacc agatcgcgca ggaggcccgg cagcaccggc     180
   ataatcaggc cgatgccgac agcgtcgagc gcgacagtgc tcagaattac gatcaggggt     240
   atgttgggtt tcacgtctgg cctccggacc agcctccgct ggtccgattg aacgcgcgga     300
   ttctttatca ctgataagtt ggtggacata ttatgtttat cagtgataaa gtgtcaagca     360
   tgacaaagtt gcagccgaat acagtgatcc gtgccgccct ggacctgttg aacgaggtcg     420
   gcgtagacgg tctgacgaca cgcaaactgg cggaacggtt gggggttcag cagccggcgc     480
   tttactggca cttcaggaac aagcgggcgc tgctcgacgc actggccgaa gccatgctgg     540
   cggagaatca tacgcattcg gtgccgagag ccgacgacga ctggcgctca tttctgatcg     600
   ggaatgcccg cagcttcagg caggcgctgc tcgcctaccg cgatggcgcg cgcatccatg     660
   ccggcacgcg accgggcgca ccgcagatgg aaacggccga cgcgcagctt cgcttcctct     720
   gcgaggcggg tttttcggcc ggggacgccg tcaatgcgct gatgacaatc agctacttca     780
   ctgttggggc cgtgcttgag gagcaggccg gcgacagcga tgccggcgag cgcggcggca     840
   ccgttgaaca ggctccgctc tcgccgctgt tgcgggccgc gatagacgcc ttcgacgaag     900
   ccggtccgga cgcagcgttc gagcagggac tcgcggtgat tgtcgatgga ttggcgaaaa     960
   ggaggctcgt tgtcaggaac gttgaaggac cgagaaaggg tgacgattga tcaggaccgc    1020
   tgccggagcg caacccactc actacagcag agccatgtag acaacatccc ctcccccttt    1080
   ccaccgcgtc agacgcccgt agcagcccgc tacgggcttt ttcatgccct gccctagcgt    1140
   ccaagcctca cggccgcgct cggcctctct ggcggccttc tggcgctctt ccgcttcctc    1200
   gctcactgac tcgctgcgct cggtcgttcg gctgcggcga gcggtatcag ctcactcaaa    1260
   ggcggtaata cggttatcca cagaatcagg ggataacgca ggaaagaaca tgtgagcaaa    1320
   aggccagcaa aaggccagga accgtaaaaa ggccgcgttg ctggcgtttt tccataggct    1380
   ccgcccccct gacgagcatc acaaaaatcg acgctcaagt cagaggtggc gaaacccgac    1440
   aggactataa agataccagg cgtttccccc tggaagctcc ctcgtgcgct ctcctgttcc    1500
   gaccctgccg cttaccggat acctgtccgc ctttctccct tcgggaagcg tggcgctttt    1560
   ccgctgcata accctgcttc ggggtcatta tagcgatttt ttcggtatat ccatcctttt    1620
   tcgcacgata tacaggattt tgccaaaggg ttcgtgtaga ctttccttgg tgtatccaac    1680
   ggcgtcagcc gggcaggata ggtgaagtag gcccacccgc gagcgggtgt tccttcttca    1740
   ctgtccctta ttcgcacctg gcggtgctca acgggaatcc tgctctgcga ggctggccgg    1800
   ctaccgccgg cgtaacagat gagggcaagc ggatggctga tgaaaccaag ccaaccagga    1860
   agggcagccc acctatcaag gtgtactgcc ttccagacga acgaagagcg attgaggaaa    1920
   aggcggcggc ggccggcatg agcctgtcgg cctacctgct ggccgtcggc cagggctaca    1980
   aaatcacggg cgtcgtggac tatgagcacg tccgcgagct ggcccgcatc aatggcgacc    2040
   tgggccgcct gggcggcctg ctgaaactct ggctcaccga cgacccgcgc acggcgcggt    2100
   tcggtgatgc cacgatcctc gccctgctgg cgaagatcga agagaagcag gacgagcttg    2160
   gcaaggtcat gatgggcgtg gtccgcccga gggcagagcc atgacttttt tagccgctaa    2220
   aacggccggg gggtgcgcgt gattgccaag cacgtcccca tgcgctccat caagaagagc    2280
   gacttcgcgg agctggtgaa gtacatcacc gacgagcaag gcaagaccga gcgcctttgc    2340
   gacgctcacc gggctggttg ccctcgccgc tgggctggcg gccgtctatg gccctgcaaa    2400
   cgcgccagaa acgccgtcga agccgtgtgc gagacaccgc ggccgccggc gttgtggata    2460
   cctcgcggaa aacttggccc tcactgacag atgaggggcg gacgttgaca cttgaggggc    2520
   cgactcaccc ggcgcggcgt tgacagatga ggggcaggct cgatttcggc cggcgacgtg 2580
   gagctggcca gcctcgcaaa tcggcgaaaa cgcctgattt tacgcgagtt tcccacagat 2640
   gatgtggaca agcctgggga taagtgccct gcggtattga cacttgaggg gcgcgactac 2700
   tgacagatga ggggcgcgat ccttgacact tgaggggcag agtgctgaca gatgaggggc 2760
   gcacctattg acatttgagg ggctgtccac aggcagaaaa tccagcattt gcaagggttt 2820
   ccgcccgttt ttcggccacc gctaacctgt cttttaacct gcttttaaac caatatttat 2880
   aaaccttgtt tttaaccagg gctgcgccct gtgcgcgtga ccgcgcacgc cgaagggggg 2940
   tgccccccct tctcgaaccc tcccggcccg ctaacgcggg cctcccatcc ccccaggggc 3000
   tgcgcccctc ggccgcgaac ggcctcaccc caaaaatggc agcgctggca gtccttgcca 3060
   ttgccgggat cggggcagta acgggatggg cgatcagccc gagcgcgacg cccggaagca 3120
   ttgacgtgcc gcaggtgctg gcatcgacat tcagcgacca ggtgccgggc agtgagggcg 3180
   gcggcctggg tggcggcctg cccttcactt cggccgtcgg ggcattcacg gacttcatgg 3240
   cggggccggc aatttttacc ttgggcattc ttggcatagt ggtcgcgggt gccgtgctcg 3300
   tgttcggggg tgcgataaac ccagcgaacc atttgaggtg ataggtaaga ttataccgag 3360
   gtatgaaaac gagaattgga cctttacaga attactctat gaagcgccat atttaaaaag 3420
   ctaccaagac gaagaggatg aagaggatga ggaggcagat tgccttgaat atattgacaa 3480
   tactgataag ataatatatc ttttatatag aagatatcgc cgtatgtaag gatttcaggg 3540
   ggcaaggcat aggcagcgcg cttatcaata tatctataga atgggcaaag cataaaaact 3600
   tgcatggact aatgcttgaa acccaggaca ataaccttat agcttgtaaa ttctatcata 3660
   attgggtaat gactccaact tattgatagt gttttatgtt cagataatgc ccgatgactt 3720
   tgtcatgcag ctccaccgat tttgagaacg acagcgactt ccgtcccagc cgtgccaggt 3780
   gctgcctcag attcaggtta tgccgctcaa ttcgctgcgt atatcgcttg ctgattacgt 3840
   gcagctttcc cttcaggcgg gattcataca gcggccagcc atccgtcatc catatcacca 3900
   cgtcaaaggg tgacagcagg ctcataagac gccccagcgt cgccatagtg cgttcaccga 3960
   atacgtgcgc aacaaccgtc ttccggagac tgtcatacgc gtaaaacagc cagcgctggc 4020
   gcgatttagc cccgacatag ccccactgtt cgtccatttc cgcgcagacg atgacgtcac 4080
   tgcccggctg tatgcgcgag gttaccgact gcggcctgag ttttttaagt gacgtaaaat 4140
   cgtgttgagg ccaacgccca taatgcgggc tgttgcccgg catccaacgc cattcatggc 4200
   catatcaatg attttctggt gcgtaccggg ttgagaagcg gtgtaagtga actgcagttg 4260
   ccatgtttta cggcagtgag agcagagata gcgctgatgt ccggcggtgc ttttgccgtt 4320
   acgcaccacc ccgtcagtag ctgaacagga gggacagctg atagacacag aagccactgg 4380
   agcacctcaa aaacaccatc atacactaaa tcagtaagtt ggcagcatca cccataattg 4440
   tggtttcaaa atcggctccg tcgatactat gttatacgcc aactttgaaa acaactttga 4500
   aaaagctgtt ttctggtatt taaggtttta gaatgcaagg aacagtgaat tggagttcgt 4560
   cttgttataa ttagcttctt ggggtatctt taaatactgt agaaaagagg aaggaaataa 4620
   taaatggcta aaatgagaat atcaccggaa ttgaaaaaac tgatcgaaaa ataccgctgc 4680
   gtaaaagata cggaaggaat gtctcctgct aaggtatata agctggtggg agaaaatgaa 4740
   aacctatatt taaaaatgac ggacagccgg tataaaggga ccacctatga tgtggaacgg 4800
   gaaaaggaca tgatgctatg gctggaagga aagctgcctg ttccaaaggt cctgcacttt 4860
   gaacggcatg atggctggag caatctgctc atgagtgagg ccgatggcgt cctttgctcg 4920
   gaagagtatg aagatgaaca aagccctgaa aagattatcg agctgtatgc ggagtgcatc 4980
   aggctctttc actccatcga catatcggat tgtccctata cgaatagctt agacagccgc 5040
   ttagccgaat tggattactt actgaataac gatctggccg atgtggattg cgaaaactgg 5100
   gaagaagaca ctccatttaa agatccgcgc gagctgtatg attttttaaa gacggaaaag 5160
   cccgaagagg aacttgtctt ttcccacggc gacctgggag acagcaacat ctttgtgaaa 5220
   gatggcaaag taagtggctt tattgatctt gggagaagcg gcagggcgga caagtggtat 5280
   gacattgcct tctgcgtccg gtcgatcagg gaggatatcg gggaagaaca gtatgtcgag 5340
   ctattttttg acttactggg gatcaagcct gattgggaga aaataaaata ttatatttta 5400
   ctggatgaat tgttttagta cctagatgtg gcgcaacgat gccggcgaca agcaggagcg 5460
   caccgacttc ttccgcatca agtgttttgg ctctcaggcc gaggcccacg gcaagtattt 5520
   gggcaagggg tcgctggtat tcgtgcaggg caagattcgg aataccaagt acgagaagga 5580
   cggccagacg gtctacggga ccgacttcat tgccgataag gtggattatc tggacaccaa 5640
   ggcaccaggc gggtcaaatc aggaataagg gcacattgcc ccggcgtgag tcggggcaat 5700
   cccgcaagga gggtgaatga atcggacgtt tgaccggaag gcatacaggc aagaactgat 5760
   cgacgcgggg ttttccgccg aggatgccga aaccatcgca agccgcaccg tcatgcgtgc 5820
   gccccgcgaa accttccagt ccgtcggctc gatggtccag caagctacgg ccaagatcga 5880
   gcgcgacagc gtgcaactgg ctccccctgc cctgcccgcg ccatcggccg ccgtggagcg 5940
   ttcgcgtcgt ctcgaacagg aggcggcagg tttggcgaag tcgatgacca tcgacacgcg 6000
   aggaactatg acgaccaaga agcgaaaaac cgccggcgag gacctggcaa aacaggtcag 6060
   cgaggccaag caggccgcgt tgctgaaaca cacgaagcag cagatcaagg aaatgcagct 6120
   ttccttgttc gatattgcgc cgtggccgga cacgatgcga gcgatgccaa acgacacggc 6180
   ccgctctgcc ctgttcacca cgcgcaacaa gaaaatcccg cgcgaggcgc tgcaaaacaa 6240
   ggtcattttc cacgtcaaca aggacgtgaa gatcacctac accggcgtcg agctgcgggc 6300
   cgacgatgac gaactggtgt ggcagcaggt gttggagtac gcgaagcgca cccctatcgg 6360
   cgagccgatc accttcacgt tctacgagct ttgccaggac ctgggctggt cgatcaatgg 6420
   ccggtattac acgaaggccg aggaatgcct gtcgcgccta caggcgacgg cgatgggctt 6480
   cacgtccgac cgcgttgggc acctggaatc ggtgtcgctg ctgcaccgct tccgcgtcct 6540
   ggaccgtggc aagaaaacgt cccgttgcca ggtcctgatc gacgaggaaa tcgtcgtgct 6600
   gtttgctggc gaccactaca cgaaattcat atgggagaag taccgcaagc tgtcgccgac 6660
   ggcccgacgg atgttcgact atttcagctc gcaccgggag ccgtacccgc tcaagctgga 6720
   aaccttccgc ctcatgtgcg gatcggattc cacccgcgtg aagaagtggc gcgagcaggt 6780
   cggcgaagcc tgcgaagagt tgcgaggcag cggcctggtg gaacacgcct gggtcaatga 6840
   tgacctggtg cattgcaaac gctagggcct tgtggggtca gttccggctg ggggttcagc 6900
   agccagcgct ttactggcat ttcaggaaca agcgggcact gctcgacgca cttgcttcgc 6960
   tcagtatcgc tcgggacgca cggcgcgctc tacgaactgc cgataaacag aggattaaaa 7020
   ttgacaattg tgattaaggc tcagattcga cggcttggag cggccgacgt gcaggatttc 7080
   cgcgagatcc gattgtcggc cctgaagaaa gctccagaga tgttcgggtc cgtttacgag 7140
   cacgaggaga aaaagcccat ggaggcgttc gctgaacggt tgcgagatgc cgtggcattc 7200
   ggcgcctaca tcgacggcga gatcattggg ctgtcggtct tcaaacagga ggacggcccc 7260
   aaggacgctc acaaggcgca tctgtccggc gttttcgtgg agcccgaaca gcgaggccga 7320
   ggggtcgccg gtatgctgct gcgggcgttg ccggcgggtt tattgctcgt gatgatcgtc 7380
   cgacagattc caacgggaat ctggtggatg cgcatcttca tcctcggcgc acttaatatt 7440
   tcgctattct ggagcttgtt gtttatttcg gtctaccgcc tgccgggcgg ggtcgcggcg 7500
   acggtaggcg ctgtgcagcc gctgatggtc gtgttcatct ctgccgctct gctaggtagc 7560
   ccgatacgat tgatggcggt cctgggggct atttgcggaa ctgcgggcgt ggcgctgttg 7620
   gtgttgacac caaacgcagc gctagatcct gtcggcgtcg cagcgggcct ggcgggggcg 7680
   gtttccatgg cgttcggaac cgtgctgacc cgcaagtggc aacctcccgt gcctctgctc 7740
   acctttaccg cctggcaact ggcggccgga ggacttctgc tcgttccagt agctttagtg 7800
   tttgatccgc caatcccgat gcctacagga accaatgttc tcggcctggc gtggctcggc 7860
   ctgatcggag cgggtttaac ctacttcctt tggttccggg ggatctcgcg actcgaacct 7920
   acagttgttt ccttactggg ctttctcagc cccagatctg gggtcgatca gccggggatg 7980
   catcaggccg acagtcggaa cttcgggtcc ccgacctgta ccattcggtg agcaatggat 8040
   aggggagttg atatcgtcaa cgttcacttc taaagaaata gcgccactca gcttcctcag 8100
   cggctttatc cagcgatttc ctattatgtc ggcatagttc tcaagatcga cagcctgtca 8160
   cggttaagcg agaaatgaat aagaaggctg ataattcgga tctctgcgag ggagatgata 8220
   tttgatcaca ggcagcaacg ctctgtcatc gttacaatca acatgctacc ctccgcgaga 8280
   tcatccgtgt ttcaaacccg gcagcttagt tgccgttctt ccgaatagca tcggtaacat 8340
   gagcaaagtc tgccgcctta caacggctct cccgctgacg ccgtcccgga ctgatgggct 8400
   gcctgtatcg agtggtgatt ttgtgccgag ctgccggtcg gggagctgtt ggctggctgg 8460
   tggcaggata tattgtggtg taaacaaatt gacgcttaga caacttaata acacattgcg 8520
   gacgttttta atgtactggg gtggtttttc ttttcaccag tgagacgggc aacagctgat 8580
   tgcccttcac cgcctggccc tgagagagtt gcagcaagcg gtccacgctg gtttgcccca 8640
   gcaggcgaaa atcctgtttg atggtggttc cgaaatcggc aaaatccctt ataaatcaaa 8700
   agaatagccc gagatagggt tgagtgttgt tccagtttgg aacaagagtc cactattaaa 8760
   gaacgtggac tccaacgtca aagggcgaaa aaccgtctat cagggcgatg gcccactacg 8820
   tgaaccatca cccaaatcaa gttttttggg gtcgaggtgc cgtaaagcac taaatcggaa 8880
   ccctaaaggg agcccccgat ttagagcttg acggggaaag ccggcgaacg tggcgagaaa 8940
   ggaagggaag aaagcgaaag gagcgggcgc cattcaggct gcgcaactgt tgggaagggc 9000
   gatcggtgcg ggcctcttcg ctattacgcc agctggcgaa agggggatgt gctgcaaggc   9060
   gattaagttg ggtaacgcca gggttttccc agtcacgacg ttgtaaaacg acggccagtg   9120
   aattaattcc catcttgaaa gaaatatagt ttaaatattt attgataaaa taacaagtca   9180
   ggtattatag tccaagcaaa aacataaatt tattgatgca agtttaaatt cagaaatatt   9240
   tcaataactg attatatcag ctggtacatt gccgtagatg aaagactgag tgcgatatta   9300
   tgtgtaatac ataaattgat gatatagcta gcttagctca tcgggggatc cgtcgaagct   9360
   agcttgggtc ccgctcagaa gaactcgtca agaaggcgat agaaggcgat gcgctgcgaa   9420
   tcgggagcgg cgataccgta aagcacgagg aagcggtcag cccattcgcc gccaagctct   9480
   tcagcaatat cacgggtagc caacgctatg tcctgatagc ggtccgccac acccagccgg   9540
   ccacagtcga tgaatccaga aaagcggcca ttttccacca tgatattcgg caagcaggca   9600
   tcgccatggg tcacgacgag atcctcgccg tcgggcatgc gcgccttgag cctggcgaac   9660
   agttcggctg gcgcgagccc ctgatgctct tcgtccagat catcctgatc gacaagaccg   9720
   gcttccatcc gagtacgtgc tcgctcgatg cgatgtttcg cttggtggtc gaatgggcag   9780
   gtagccggat caagcgtatg cagccgccgc attgcatcag ccatgatgga tactttctcg   9840
   gcaggagcaa ggtgagatga caggagatcc tgccccggca cttcgcccaa tagcagccag   9900
   tcccttcccg cttcagtgac aacgtcgagc acagctgcgc aaggaacgcc cgtcgtggcc   9960
   agccacgata gccgcgctgc ctcgtcctgc agttcattca gggcaccgga caggtcggtc  10020
   ttgacaaaaa gaaccgggcg cccctgcgct gacagccgga acacggcggc atcagagcag  10080
   ccgattgtct gttgtgccca gtcatagccg aatagcctct ccacccaagc ggccggagaa  10140
   cctgcgtgca atccatcttg ttcaatccaa gctcccatgg gccctcgact agagtcgaga  10200
   tctggattga gagtgaatat gagactctaa ttggataccg aggggaattt atggaacgtc  10260
   agtggagcat ttttgacaag aaatatttgc tagctgatag tgaccttagg cgacttttga  10320
   acgcgcaata atggtttctg acgtatgtgc ttagctcatt aaactccaga aacccgcggc  10380
   tgagtggctc cttcaacgtt gcggttctgt cagttccaaa cgtaaaacgg cttgtcccgc  10440
   gtcatcggcg ggggtcataa cgtgactccc ttaattctcc gctcatgatc ttgatcccct  10500
   gcgccatcag atccttggcg gcaagaaagc catccagttt actttgcagg gcttcccaac  10560
   cttaccagag ggcgccccag ctggcaattc cggttcgctt gctgtccata aaaccgccca  10620
   gtctagctat cgccatgtaa gcccactgca agctacctgc tttctctttg cgcttgcgtt  10680
   ttcccttgtc cagatagccc agtagctgac attcatccgg ggtcagcacc gtttctgcgg  10740
   actggctttc tacgtgttcc gcttccttta gcagcccttg cgccctgagt gcttgcggca  10800
   gcgtgaagct tgcatgcctg caggtcgacg gcgcgccgag ctcctcgagc aaatttacac  10860
   attgccacta aacgtctaaa cccttgtaat ttgtttttgt tttactatgt gtgttatgta  10920
   tttgatttgc gataaatttt tatatttggt actaaattta taacaccttt tatgctaacg  10980
   tttgccaaca cttagcaatt tgcaagttga ttaattgatt ctaaattatt tttgtcttct  11040
   aaatacatat actaatcaac tggaaatgta aatatttgct aatatttcta ctataggaga  11100
   attaaagtga gtgaatatgg taccacaagg tttggagatt taattgttgc aatgctgcat  11160
   ggatggcata tacaccaaac attcaataat tcttgaggat aataatggta ccacacaaga  11220
   tttgaggtgc atgaacgtca cgtggacaaa aggtttagta atttttcaag acaacaatgt  11280
   taccacacac aagttttgag gtgcatgcat ggatgccctg tggaaagttt aaaaatattt  11340
   tggaaatgat ttgcatggaa gccatgtgta aaaccatgac atccacttgg aggatgcaat  11400
   aatgaagaaa actacaaatt tacatgcaac tagttatgca tgtagtctat ataatgagga  11460
   ttttgcaata ctttcattca tacacactca ctaagtttta cacgattata atttcttcat  11520
   agccagccca ccgcggtggg cggccgcctg cagtctagaa ggcctcctgc tttaatgaga  11580
   tatgcgagac gcctatgatc gcatgatatt tgctttcaat tctgttgtgc acgttgtaaa  11640
   aaacctgagc atgtgtagct cagatcctta ccgccggttt cggttcattc taatgaatat  11700
   atcacccgtt actatcgtat ttttatgaat aatattctcc gttcaattta ctgattgtcc  11760
   gtcgagcaaa tttacacatt gccactaaac gtctaaaccc ttgtaatttg tttttgtttt  11820
   actatgtgtg ttatgtattt gatttgcgat aaatttttat atttggtact aaatttataa  11880
   caccttttat gctaacgttt gccaacactt agcaatttgc aagttgatta attgattcta  11940
   aattattttt gtcttctaaa tacatatact aatcaactgg aaatgtaaat atttgctaat  12000
   atttctacta taggagaatt aaagtgagtg aatatggtac cacaaggttt ggagatttaa  12060
   ttgttgcaat gctgcatgga tggcatatac accaaacatt caataattct tgaggataat  12120
   aatggtacca cacaagattt gaggtgcatg aacgtcacgt ggacaaaagg tttagtaatt  12180
   tttcaagaca acaatgttac cacacacaag ttttgaggtg catgcatgga tgccctgtgg  12240
   aaagtttaaa aatattttgg aaatgatttg catggaagcc atgtgtaaaa ccatgacatc  12300
   cacttggagg atgcaataat gaagaaaact acaaatttac atgcaactag ttatgcatgt  12360
   agtctatata atgaggattt tgcaatactt tcattcatac acactcacta agttttacac  12420
   gattataatt tcttcatagc cagcggatcc gatatcgggc ccgctagcgt taaccctgct  12480
   ttaatgagat atgcgagacg cctatgatcg catgatattt gctttcaatt ctgttgtgca  12540
   cgttgtaaaa aacctgagca tgtgtagctc agatccttac cgccggtttc ggttcattct  12600
   aatgaatata tcacccgtta ctatcgtatt tttatgaata atattctccg ttcaatttac  12660
   tgattgtccg tcgagcaaat ttacacattg ccactaaacg tctaaaccct tgtaatttgt  12720
   ttttgtttta ctatgtgtgt tatgtatttg atttgcgata aatttttata tttggtacta  12780
   aatttataac accttttatg ctaacgtttg ccaacactta gcaatttgca agttgattaa  12840
   ttgattctaa attatttttg tcttctaaat acatatacta atcaactgga aatgtaaata  12900
   tttgctaata tttctactat aggagaatta aagtgagtga atatggtacc acaaggtttg  12960
   gagatttaat tgttgcaatg ctgcatggat ggcatataca ccaaacattc aataattctt  13020
   gaggataata atggtaccac acaagatttg aggtgcatga acgtcacgtg gacaaaaggt  13080
   ttagtaattt ttcaagacaa caatgttacc acacacaagt tttgaggtgc atgcatggat  13140
   gccctgtgga aagtttaaaa atattttgga aatgatttgc atggaagcca tgtgtaaaac  13200
   catgacatcc acttggagga tgcaataatg aagaaaacta caaatttaca tgcaactagt  13260
   tatgcatgta gtctatataa tgaggatttt gcaatacttt cattcataca cactcactaa  13320
   gttttacacg attataattt cttcatagcc agcagatctg ccggcatcga tcccgggcca  13380
   tggcctgctt taatgagata tgcgagacgc ctatgatcgc atgatatttg ctttcaattc  13440
   tgttgtgcac gttgtaaaaa acctgagcat gtgtagctca gatccttacc gccggtttcg  13500
   gttcattcta atgaatatat cacccgttac tatcgtattt ttatgaataa tattctccgt  13560
   tcaatttact gattgtccgt cgacgagctc ggcgcgcctc tagaggatcg atgaattcag  13620
   atcggctgag tggctccttc aacgttgcgg ttctgtcagt tccaaacgta aaacggcttg  13680
   tcccgcgtca tcggcggggg tcataacgtg actcccttaa ttctccgctc atgatcagat  13740
   tgtcgtttcc cgccttcagt ttaaactatc agtgtttgac aggatatatt ggcgggtaaa  13800
   cctaagagaa aagagcgttt attagaataa tcggatattt aaaagggcgt gaaaaggttt  13860
   atccttcgtc catttgtatg tgcatgccaa ccacagggtt cccca                  13905
<210> 69
   <211> 1443
   <212> DNA
   <213> Phaeodactylum tricornutum
<220>
   <221> CDS
   <222> (9)..(1442)
   <223> Delta-6-Desaturase
<400> 69
   gatctaaa atg ggc aaa gga ggg gac gct cgg gcc tcg aag ggc tca acg      50
            Met Gly Lys Gly Gly Asp Ala Arg Ala Ser Lys Gly Ser Thr
            1               5                   10
   gcg gct cgc aag atc agt tgg cag gaa gtc aag acc cac gcg tct ccg       98
   Ala Ala Arg Lys Ile Ser Trp Gln Glu Val Lys Thr His Ala Ser Pro
   15                  20                  25                  30
   gag gac gcc tgg atc att cac tcc aat aag gtc tac gac gtg tcc aac      146
   Glu Asp Ala Trp Ile Ile His Ser Asn Lys Val Tyr Asp Val Ser Asn
                   35                  40                  45
   tgg cac gaa cat ccc gga ggc gcc gtc att ttc acg cac gcc ggt gac      194
   Trp His Glu His Pro Gly Gly Ala Val Ile Phe Thr His Ala Gly Asp
               50                  55                  60
   gac atg acg gac att ttc gct gcc ttt cac gca ccc gga tcg cag tcg      242
   Asp Met Thr Asp Ile Phe Ala Ala Phe His Ala Pro Gly Ser Gln Ser
           65                  70                  75
   ctc atg aag aag ttc tac att ggc gaa ttg ctc ccg gaa acc acc ggc      290
   Leu Met Lys Lys Phe Tyr Ile Gly Glu Leu Leu Pro Glu Thr Thr Gly
       80                  85                  90
   aag gag ccg cag caa atc gcc ttt gaa aag ggc tac cgc gat ctg cgc      338
   Lys Glu Pro Gln Gln Ile Ala Phe Glu Lys Gly Tyr Arg Asp Leu Arg
   95                  100                 105                 110
   tcc aaa ctc atc atg atg ggc atg ttc aag tcc aac aag tgg ttc tac      386
   Ser Lys Leu Ile Met Met Gly Met Phe Lys Ser Asn Lys Trp Phe Tyr
                   115                 120                 125
   gtc tac aag tgc ctc agc aac atg gcc att tgg gcc gcc gcc tgt gct      434
   Val Tyr Lys Cys Leu Ser Asn Met Ala Ile Trp Ala Ala Ala Cys Ala
               130                 135                 140
   ctc gtc ttt tac tcg gac cgc ttc tgg gta cac ctg gcc agc gcc gtc      482
   Leu Val Phe Tyr Ser Asp Arg Phe Trp Val His Leu Ala Ser Ala Val
           145                 150                 155
   atg ctg gga aca ttc ttt cag cag tcg gga tgg ttg gca cac gac ttt      530
   Met Leu Gly Thr Phe Phe Gln Gln Ser Gly Trp Leu Ala His Asp Phe
       160                 165                 170
   ctg cac cac cag gtc ttc acc aag cgc aag cac ggg gat ctc gga gga      578
   Leu His His Gln Val Phe Thr Lys Arg Lys His Gly Asp Leu Gly Gly
   175                 180                 185                 190
   ctc ttt tgg ggg aac ctc atg cag ggt tac tcc gta cag tgg tgg aaa      626
   Leu Phe Trp Gly Asn Leu Met Gln Gly Tyr Ser Val Gln Trp Trp Lys
                   195                 200                 205
   aac aag cac aac gga cac cac gcc gtc ccc aac ctc cac tgc tcc tcc      674
   Asn Lys His Asn Gly His His Ala Val Pro Asn Leu His Cys Ser Ser
               210                 215                 220
   gca gtc gcg caa gat ggg gac ccg gac atc gat acc atg ccc ctt ctc      722
   Ala Val Ala Gln Asp Gly Asp Pro Asp Ile Asp Thr Met Pro Leu Leu
           225                 230                 235
   gcc tgg tcc gtc cag caa gcc cag tct tac cgg gaa ctc caa gcc gac      770
   Ala Trp Ser Val Gln Gln Ala Gln Ser Tyr Arg Glu Leu Gln Ala Asp
       240                 245                 250
   gga aag gat tcg ggt ttg gtc aag ttc atg atc cgt aac caa tcc tac      818
   Gly Lys Asp Ser Gly Leu Val Lys Phe Met Ile Arg Asn Gln Ser Tyr
   255                 260                 265                 270
   ttt tac ttt ccc atc ttg ttg ctc gcc cgc ctg tcg tgg ttg aac gag      866
   Phe Tyr Phe Pro Ile Leu Leu Leu Ala Arg Leu Ser Trp Leu Asn Glu
                   275                 280                 285
   tcc ttc aag tgc gcc ttt ggg ctt gga gct gcg tcg gag aac gct gct      914
   Ser Phe Lys Cys Ala Phe Gly Leu Gly Ala Ala Ser Glu Asn Ala Ala
               290                 295                 300
   ctc gaa ctc aag gcc aag ggt ctt cag tac ccc ctt ttg gaa aag gct      962
   Leu Glu Leu Lys Ala Lys Gly Leu Gln Tyr Pro Leu Leu Glu Lys Ala
           305                 310                 315
   ggc atc ctg ctg cac tac gct tgg atg ctt aca gtt tcg tcc ggc ttt     1010
   Gly Ile Leu Leu His Tyr Ala Trp Met Leu Thr Val Ser Ser Gly Phe
       320                 325                 330
   gga cgc ttc tcg ttc gcg tac acc gca ttt tac ttt cta acc gcg acc     1058
   Gly Arg Phe Ser Phe Ala Tyr Thr Ala Phe Tyr Phe Leu Thr Ala Thr
   335                 340                 345                 350
   gcg tcc tgt gga ttc ttg ctc gcc att gtc ttt ggc ctc ggc cac aac      1106
   Ala Ser Cys Gly Phe Leu Leu Ala Ile Val Phe Gly Leu Gly His Asn
                   355                 360                 365
   ggc atg gcc acc tac aat gcc gac gcc cgt ccg gac ttc tgg aag ctc      1154
   Gly Met Ala Thr Tyr Asn Ala Asp Ala Arg Pro Asp Phe Trp Lys Leu
               370                 375                 380
   caa gtc acc acg act cgc aac gtc acg ggc gga cac ggt ttc ccc caa      1202
   Gln Val Thr Thr Thr Arg Asn Val Thr Gly Gly His Gly Phe Pro Gln
           385                 390                 395
   gcc ttt gtc gac tgg ttc tgt ggt ggc ctc cag tac caa gtc gac cac      1250
   Ala Phe Val Asp Trp Phe Cys Gly Gly Leu Gln Tyr Gln Val Asp His
       400                 405                 410
   cac tta ttc ccc agc ctg ccc cga cac aat ctg gcc aag aca cac gca      1298
   His Leu Phe Pro Ser Leu Pro Arg His Asn Leu Ala Lys Thr His Ala
   415                 420                 425                 430
   ctg gtc gaa tcg ttc tgc aag gag tgg ggt gtc cag tac cac gaa gcc      1346
   Leu Val Glu Ser Phe Cys Lys Glu Trp Gly Val Gln Tyr His Glu Ala
                   435                 440                 445
   gac ctt gtg gac ggg acc atg gaa gtc ttg cac cat ttg ggc agc gtg      1394
   Asp Leu Val Asp Gly Thr Met Glu Val Leu His His Leu Gly Ser Val
               450                 455                 460
   gcc ggc gaa ttc gtc gtg gat ttt gta cgc gat gga ccc gcc atg taa a    1443
   Ala Gly Glu Phe Val Val Asp Phe Val Arg Asp Gly Pro Ala Met
           465                 470                 475
<210> 70
   <211> 477
   <212> PRT
   <213> Phaeodactylum tricornutum
<400> 70
   Met Gly Lys Gly Gly Asp Ala Arg Ala Ser Lys Gly Ser Thr Ala Ala
   1               5                   10                  15
   Arg Lys Ile Ser Trp Gln Glu Val Lys Thr His Ala Ser Pro Glu Asp
               20                  25                  30
   Ala Trp Ile Ile His Ser Asn Lys Val Tyr Asp Val Ser Asn Trp His
           35                  40                  45
   Glu His Pro Gly Gly Ala Val Ile Phe Thr His Ala Gly Asp Asp Met
       50                  55                  60
   Thr Asp Ile Phe Ala Ala Phe His Ala Pro Gly Ser Gln Ser Leu Met
   65                  70                  75                  80
   Lys Lys Phe Tyr Ile Gly Glu Leu Leu Pro Glu Thr Thr Gly Lys Glu
                   85                  90                  95
   Pro Gln Gln Ile Ala Phe Glu Lys Gly Tyr Arg Asp Leu Arg Ser Lys
               100                 105                 110
   Leu Ile Met Met Gly Met Phe Lys Ser Asn Lys Trp Phe Tyr Val Tyr
           115                 120                 125
   Lys Cys Leu Ser Asn Met Ala Ile Trp Ala Ala Ala Cys Ala Leu Val
       130                 135                 140
   Phe Tyr Ser Asp Arg Phe Trp Val His Leu Ala Ser Ala Val Met Leu
   145                 150                 155                 160
   Gly Thr Phe Phe Gln Gln Ser Gly Trp Leu Ala His Asp Phe Leu His
                       165                     170                     175
   His Gln Val Phe Thr Lys Arg Lys His Gly Asp Leu Gly Gly Leu Phe
               180                     185                     190
   Trp Gly Asn Leu Met Gln Gly Tyr Ser Val Gln Trp Trp Lys Asn Lys
           195                     200                     205
   His Asn Gly His His Ala Val Pro Asn Leu His Cys Ser Ser Ala Val
       210                     215                     220
   Ala Gln Asp Gly Asp Pro Asp Ile Asp Thr Met Pro Leu Leu Ala Trp
   225                     230                     235                     240
   Ser Val Gln Gln Ala Gln Ser Tyr Arg Glu Leu Gln Ala Asp Gly Lys
                   245                     250                     255
   Asp Ser Gly Leu Val Lys Phe Met Ile Arg Asn Gln Ser Tyr Phe Tyr
               260                     265                     270
   Phe Pro Ile Leu Leu Leu Ala Arg Leu Ser Trp Leu Asn Glu Ser Phe
           275                     280                     285
   Lys Cys Ala Phe Gly Leu Gly Ala Ala Ser Glu Asn Ala Ala Leu Glu
       290                     295                     300
   Leu Lys Ala Lys Gly Leu Gln Tyr Pro Leu Leu Glu Lys Ala Gly Ile
   305                     310                     315                     320
   Leu Leu His Tyr Ala Trp Met Leu Thr Val Ser Ser Gly Phe Gly Arg
                   325                     330                     335
   Phe Ser Phe Ala Tyr Thr Ala Phe Tyr Phe Leu Thr Ala Thr Ala Ser
               340                     345                     350
   Cys Gly Phe Leu Leu Ala Ile Val Phe Gly Leu Gly His Asn Gly Met
           355                     360                     365
   Ala Thr Tyr Asn Ala Asp Ala Arg Pro Asp Phe Trp Lys Leu Gln Val
       370                     375                     380
   Thr Thr Thr Arg Asn Val Thr Gly Gly His Gly Phe Pro Gln Ala Phe
   385                     390                     395                     400
   Val Asp Trp Phe Cys Gly Gly Leu Gln Tyr Gln Val Asp His His Leu
                   405                     410                     415
   Phe Pro Ser Leu Pro Arg His Asn Leu Ala Lys Thr His Ala Leu Val
               420                     425                     430
   Glu Ser Phe Cys Lys Glu Trp Gly Val Gln Tyr His Glu Ala Asp Leu
           435                     440                     445
   Val Asp Gly Thr Met Glu Val Leu His His Leu Gly Ser Val Ala Gly
       450                     455                     460
   Glu Phe Val Val Asp Phe Val Arg Asp Gly Pro Ala Met
   465                     470                     475
<210> 71
   <211> 17061
   <212> DNA
   <213> Phaeodactylum tricornutum, Physcomitrella patens, Caenorhabditis
   elegans
<220>
   <221> CDS
   <222> (4554)..(5987)
   <223> Phaeodactylum tricornutum Delta-6-Desaturase
<220>
   <221> CDS
   <222> (2805)..(3653)
   <223> Caenorhabditis elegans LPLAT
<220>
   <221> CDS
   <222> (1026)..(1898)
   <223> Physcomitrella patens Delta-6-Elongase
<400> 71
   tggggaaccc tgtggttggc atgcacatac aaatggacga aggataaacc ttttcacgcc      60
   cttttaaata tccgattatt ctaataaacg ctcttttctc ttaggtttac ccgccaatat     120
   atcctgtcaa acactgatag tttaaactga aggcgggaaa cgacaatctg atcatgagcg     180
   gagaattaag ggagtcacgt tatgaccccc gccgatgacg cgggacaagc cgttttacgt     240
   ttggaactga cagaaccgca acgttgaagg agccactcag ccgatctgaa ttcatcgatc     300
   ctctagaggc gcgccgagct cctcgagcaa atttacacat tgccactaaa cgtctaaacc     360
   cttgtaattt gtttttgttt tactatgtgt gttatgtatt tgatttgcga taaattttta     420
   tatttggtac taaatttata acacctttta tgctaacgtt tgccaacact tagcaatttg     480
   caagttgatt aattgattct aaattatttt tgtcttctaa atacatatac taatcaactg     540
   gaaatgtaaa tatttgctaa tatttctact ataggagaat taaagtgagt gaatatggta     600
   ccacaaggtt tggagattta attgttgcaa tgctgcatgg atggcatata caccaaacat     660
   tcaataattc ttgaggataa taatggtacc acacaagatt tgaggtgcat gaacgtcacg     720
   tggacaaaag gtttagtaat ttttcaagac aacaatgtta ccacacacaa gttttgaggt      780
   gcatgcatgg atgccctgtg gaaagtttaa aaatattttg gaaatgattt gcatggaagc     840
   catgtgtaaa accatgacat ccacttggag gatgcaataa tgaagaaaac tacaaattta     900
   catgcaacta gttatgcatg tagtctatat aatgaggatt ttgcaatact ttcattcata     960
   cacactcact aagttttaca cgattataat ttcttcatag ccagcccacc gcggtgggcg    1020
   gccgc atg gag gtc gtg gag aga ttc tac ggt gag ttg gat ggg aag gtc    1070
         Met Glu Val Val Glu Arg Phe Tyr Gly Glu Leu Asp Gly Lys Val
         1               5                   10                  15
   tcg cag ggc gtg aat gca ttg ctg ggt agt ttt ggg gtg gag ttg acg      1118
   Ser Gln Gly Val Asn Ala Leu Leu Gly Ser Phe Gly Val Glu Leu Thr
                   20                  25                  30
   gat acg ccc act acc aaa ggc ttg ccc ctc gtt gac agt ccc aca ccc      1166
   Asp Thr Pro Thr Thr Lys Gly Leu Pro Leu Val Asp Ser Pro Thr Pro
               35                  40                  45
   atc gtc ctc ggt gtt tct gta tac ttg act att gtc att gga ggg ctt      1214
   Ile Val Leu Gly Val Ser Val Tyr Leu Thr Ile Val Ile Gly Gly Leu
           50                  55                  60
   ttg tgg ata aag gcc agg gat ctg aaa ccg cgc gcc tcg gag cca ttt      1262
   Leu Trp Ile Lys Ala Arg Asp Leu Lys Pro Arg Ala Ser Glu Pro Phe
       65                  70                  75
   ttg ctc caa gct ttg gtg ctt gtg cac aac ctg ttc tgt ttt gcg ctc      1310
   Leu Leu Gln Ala Leu Val Leu Val His Asn Leu Phe Cys Phe Ala Leu
   80                  85                  90                  95
   agt ctg tat atg tgc gtg ggc atc gct tat cag gct att acc tgg cgg      1358
   Ser Leu Tyr Met Cys Val Gly Ile Ala Tyr Gln Ala Ile Thr Trp Arg
                   100                 105                 110
   tac tct ctc tgg ggc aat gca tac aat cct aaa cat aaa gag atg gcg      1406
   Tyr Ser Leu Trp Gly Asn Ala Tyr Asn Pro Lys His Lys Glu Met Ala
               115                 120                 125
   att ctg gta tac ttg ttc tac atg tct aag tac gtg gaa ttc atg gat      1454
   Ile Leu Val Tyr Leu Phe Tyr Met Ser Lys Tyr Val Glu Phe Met Asp
           130                 135                 140
   acc gtt atc atg ata ctg aag cgc agc acc agg caa ata agc ttc ctc      1502
   Thr Val Ile Met Ile Leu Lys Arg Ser Thr Arg Gln Ile Ser Phe Leu
       145                 150                 155
   cac gtt tat cat cat tct tca att tcc ctc att tgg tgg gct att gct      1550
   His Val Tyr His His Ser Ser Ile Ser Leu Ile Trp Trp Ala Ile Ala
   160                 165                 170                 175
   cat cac gct cct ggc ggt gaa gca tat tgg tct gcg gct ctg aac tca      1598
   His His Ala Pro Gly Gly Glu Ala Tyr Trp Ser Ala Ala Leu Asn Ser
                   180                 185                 190
   gga gtg cat gtt ctc atg tat gcg tat tac ttc ttg gct gcc tgc ctt      1646
   Gly Val His Val Leu Met Tyr Ala Tyr Tyr Phe Leu Ala Ala Cys Leu
               195                 200                 205
   cga agt agc cca aag tta aaa aat aag tac ctt ttt tgg ggc agg tac      1694
   Arg Ser Ser Pro Lys Leu Lys Asn Lys Tyr Leu Phe Trp Gly Arg Tyr
           210                 215                 220
   ttg aca caa ttc caa atg ttc cag ttt atg ctg aac tta gtg cag gct      1742
   Leu Thr Gln Phe Gln Met Phe Gln Phe Met Leu Asn Leu Val Gln Ala
       225                 230                 235
   tac tac gac atg aaa acg aat gcg cca tat cca caa tgg ctg atc aag      1790
   Tyr Tyr Asp Met Lys Thr Asn Ala Pro Tyr Pro Gln Trp Leu Ile Lys
   240                 245                 250                 255
   att ttg ttc tac tac atg atc tcg ttg ctg ttt ctt ttc ggc aat ttt      1838
   Ile Leu Phe Tyr Tyr Met Ile Ser Leu Leu Phe Leu Phe Gly Asn Phe
                   260                 265                 270
   tac gta caa aaa tac atc aaa ccc tct gac gga aag caa aag gga gct      1886
   Tyr Val Gln Lys Tyr Ile Lys Pro Ser Asp Gly Lys Gln Lys Gly Ala
               275                 280                 285
   aaa act gag tga tctagaaggc ctcctgcttt aatgagatat gcgagacgcc          1938
   Lys Thr Glu
           290
   tatgatcgca tgatatttgc tttcaattct gttgtgcacg ttgtaaaaaa cctgagcatg    1998
   tgtagctcag atccttaccg ccggtttcgg ttcattctaa tgaatatatc acccgttact    2058
   atcgtatttt tatgaataat attctccgtt caatttactg attgtccgtc gagcaaattt    2118
   acacattgcc actaaacgtc taaacccttg taatttgttt ttgttttact atgtgtgtta    2178
   tgtatttgat ttgcgataaa tttttatatt tggtactaaa tttataacac cttttatgct    2238
   aacgtttgcc aacacttagc aatttgcaag ttgattaatt gattctaaat tatttttgtc    2298
   ttctaaatac atatactaat caactggaaa tgtaaatatt tgctaatatt tctactatag    2358
   gagaattaaa gtgagtgaat atggtaccac aaggtttgga gatttaattg ttgcaatgct    2418
   gcatggatgg catatacacc aaacattcaa taattcttga ggataataat ggtaccacac    2478
   aagatttgag gtgcatgaac gtcacgtgga caaaaggttt agtaattttt caagacaaca    2538
   atgttaccac acacaagttt tgaggtgcat gcatggatgc cctgtggaaa gtttaaaaat    2598
   attttggaaa tgatttgcat ggaagccatg tgtaaaacca tgacatccac ttggaggatg     2658
   caataatgaa gaaaactaca aatttacatg caactagtta tgcatgtagt ctatataatg    2718
   aggattttgc aatactttca ttcatacaca ctcactaagt tttacacgat tataatttct    2778
   tcatagccag cggatccgcc cacata atg gag aac ttc tgg tct att gtt gtg     2831
                                Met Glu Asn Phe Trp Ser Ile Val Val
                                                295
   ttt ttt cta ctc tca att ctc ttc att tta tat aac ata tcg aca gta      2879
   Phe Phe Leu Leu Ser Ile Leu Phe Ile Leu Tyr Asn Ile Ser Thr Val
   300                 305                 310                 315
   tgc cac tac tat atg cgg att tcg ttt tat tac ttc aca att tta ttg      2927
   Cys His Tyr Tyr Met Arg Ile Ser Phe Tyr Tyr Phe Thr Ile Leu Leu
                   320                 325                 330
   cat gga atg gaa gtt tgt gtt aca atg atc cct tct tgg cta aat ggg      2975
   His Gly Met Glu Val Cys Val Thr Met Ile Pro Ser Trp Leu Asn Gly
               335                 340                 345
   aag ggt gct gat tac gtg ttt cac tcg ttt ttc tat tgg tgt aaa tgg      3023
   Lys Gly Ala Asp Tyr Val Phe His Ser Phe Phe Tyr Trp Cys Lys Trp
           350                 355                 360
   act ggt gtt cat aca aca gtc tat gga tat gaa aaa aca caa gtt gaa      3071
   Thr Gly Val His Thr Thr Val Tyr Gly Tyr Glu Lys Thr Gln Val Glu
       365                 370                 375
   ggt ccg gct gta gtt att tgt aat cat cag agt tct ctc gac att cta      3119
   Gly Pro Ala Val Val Ile Cys Asn His Gln Ser Ser Leu Asp Ile Leu
   380                 385                 390                 395
   tcg atg gca tca atc tgg ccg aag aat tgt gtt gta atg atg aaa cga      3167
   Ser Met Ala Ser Ile Trp Pro Lys Asn Cys Val Val Met Met Lys Arg
                   400                 405                 410
   att ctt gcc tat gtt cca ttc ttc aat ctc gga gcc tac ttt tcc aac      3215
   Ile Leu Ala Tyr Val Pro Phe Phe Asn Leu Gly Ala Tyr Phe Ser Asn
               415                 420                 425
   aca atc ttc atc gat cga tat aac cgt gaa cgt gcg atg gct tca gtt      3263
   Thr Ile Phe Ile Asp Arg Tyr Asn Arg Glu Arg Ala Met Ala Ser Val
           430                 435                 440
   gat tat tgt gca tct gaa atg aag aac aga aat ctt aaa ctt tgg gta      3311
   Asp Tyr Cys Ala Ser Glu Met Lys Asn Arg Asn Leu Lys Leu Trp Val
       445                 450                 455
   ttt ccg gaa gga aca aga aat cgt gaa gga ggg ttc att cca ttc aag      3359
   Phe Pro Glu Gly Thr Arg Asn Arg Glu Gly Gly Phe Ile Pro Phe Lys
   460                 465                 470                 475
   aaa gga gca ttc aat att gca gtt cgt gcg cag att ccc att att cca      3407
   Lys Gly Ala Phe Asn Ile Ala Val Arg Ala Gln Ile Pro Ile Ile Pro
                   480                 485                 490
   gtt gta ttc tca gac tat cgg gat ttc tac tca aag cca ggc cga tat      3455
   Val Val Phe Ser Asp Tyr Arg Asp Phe Tyr Ser Lys Pro Gly Arg Tyr
               495                 500                 505
   ttc aag aat gat gga gaa gtt gtt att cga gtt ctg gat gcg att cca      3503
   Phe Lys Asn Asp Gly Glu Val Val Ile Arg Val Leu Asp Ala Ile Pro
           510                 515                 520
   aca aaa ggg ctc act ctt gat gac gtc agc gag ttg tct gat atg tgt      3551
   Thr Lys Gly Leu Thr Leu Asp Asp Val Ser Glu Leu Ser Asp Met Cys
       525                 530                 535
   cgg gac gtt atg ttg gca gcc tat aag gaa gtt act cta gaa gct cag      3599
   Arg Asp Val Met Leu Ala Ala Tyr Lys Glu Val Thr Leu Glu Ala Gln
   540                 545                 550                 555
   caa cga aat gcg aca cgg cgt gga gaa aca aaa gac ggg aag aaa tct      3647
   Gln Arg Asn Ala Thr Arg Arg Gly Glu Thr Lys Asp Gly Lys Lys Ser
                   560                 565                 570
   gag taa gctagcgtta accctgcttt aatgagatat gcgagacgcc tatgatcgca       3703
   Glu
   tgatatttgc tttcaattct gttgtgcacg ttgtaaaaaa cctgagcatg tgtagctcag    3763
   atccttaccg ccggtttcgg ttcattctaa tgaatatatc acccgttact atcgtatttt    3823
   tatgaataat attctccgtt caatttactg attgtccgtc gagcaaattt acacattgcc    3883
   actaaacgtc taaacccttg taatttgttt ttgttttact atgtgtgtta tgtatttgat    3943
   ttgcgataaa tttttatatt tggtactaaa tttataacac cttttatgct aacgtttgcc    4003
   aacacttagc aatttgcaag ttgattaatt gattctaaat tatttttgtc ttctaaatac    4063
   atatactaat caactggaaa tgtaaatatt tgctaatatt tctactatag gagaattaaa    4123
   gtgagtgaat atggtaccac aaggtttgga gatttaattg ttgcaatgct gcatggatgg    4183
   catatacacc aaacattcaa taattcttga ggataataat ggtaccacac aagatttgag    4243
   gtgcatgaac gtcacgtgga caaaaggttt agtaattttt caagacaaca atgttaccac    4303
   acacaagttt tgaggtgcat gcatggatgc cctgtggaaa gtttaaaaat attttggaaa    4363
   tgatttgcat ggaagccatg tgtaaaacca tgacatccac ttggaggatg caataatgaa    4423
   gaaaactaca aatttacatg caactagtta tgcatgtagt ctatataatg aggattttgc    4483
   aatactttca ttcatacaca ctcactaagt tttacacgat tataatttct tcatagccag    4543
   cagatctaaa atg ggc aaa gga ggg gac gct cgg gcc tcg aag ggc tca       4592
              Met Gly Lys Gly Gly Asp Ala Arg Ala Ser Lys Gly Ser
                              575                 580                 585
   acg gcg gct cgc aag atc agt tgg cag gaa gtc aag acc cac gcg tct      4640
   Thr Ala Ala Arg Lys Ile Ser Trp Gln Glu Val Lys Thr His Ala Ser
                   590                 595                 600
   ccg gag gac gcc tgg atc att cac tcc aat aag gtc tac gac gtg tcc      4688
   Pro Glu Asp Ala Trp Ile Ile His Ser Asn Lys Val Tyr Asp Val Ser
               605                 610                 615
   aac tgg cac gaa cat ccc gga ggc gcc gtc att ttc acg cac gcc ggt      4736
   Asn Trp His Glu His Pro Gly Gly Ala Val Ile Phe Thr His Ala Gly
           620                 625                 630
   gac gac atg acg gac att ttc gct gcc ttt cac gca ccc gga tcg cag      4784
   Asp Asp Met Thr Asp Ile Phe Ala Ala Phe His Ala Pro Gly Ser Gln
       635                 640                 645
   tcg ctc atg aag aag ttc tac att ggc gaa ttg ctc ccg gaa acc acc      4832
   Ser Leu Met Lys Lys Phe Tyr Ile Gly Glu Leu Leu Pro Glu Thr Thr
   650                 655                 660                 665
   ggc aag gag ccg cag caa atc gcc ttt gaa aag ggc tac cgc gat ctg      4880
   Gly Lys Glu Pro Gln Gln Ile Ala Phe Glu Lys Gly Tyr Arg Asp Leu
                   670                 675                 680
   cgc tcc aaa ctc atc atg atg ggc atg ttc aag tcc aac aag tgg ttc      4928
   Arg Ser Lys Leu Ile Met Met Gly Met Phe Lys Ser Asn Lys Trp Phe
               685                 690                 695
   tac gtc tac aag tgc ctc agc aac atg gcc att tgg gcc gcc gcc tgt      4976
   Tyr Val Tyr Lys Cys Leu Ser Asn Met Ala Ile Trp Ala Ala Ala Cys
           700                 705                 710
   gct ctc gtc ttt tac tcg gac cgc ttc tgg gta cac ctg gcc agc gcc      5024
   Ala Leu Val Phe Tyr Ser Asp Arg Phe Trp Val His Leu Ala Ser Ala
       715                 720                 725
   gtc atg ctg gga aca ttc ttt cag cag tcg gga tgg ttg gca cac gac      5072
   Val Met Leu Gly Thr Phe Phe Gln Gln Ser Gly Trp Leu Ala His Asp
   730                 735                 740                 745
   ttt ctg cac cac cag gtc ttc acc aag cgc aag cac ggg gat ctc gga      5120
   Phe Leu His His Gln Val Phe Thr Lys Arg Lys His Gly Asp Leu Gly
                   750                 755                 760
   gga ctc ttt tgg ggg aac ctc atg cag ggt tac tcc gta cag tgg tgg      5168
   Gly Leu Phe Trp Gly Asn Leu Met Gln Gly Tyr Ser Val Gln Trp Trp
               765                 770                 775
   aaa aac aag cac aac gga cac cac gcc gtc ccc aac ctc cac tgc tcc      5216
   Lys Asn Lys His Asn Gly His His Ala Val Pro Asn Leu His Cys Ser
           780                 785                 790
   tcc gca gtc gcg caa gat ggg gac ccg gac atc gat acc atg ccc ctt      5264
   Ser Ala Val Ala Gln Asp Gly Asp Pro Asp Ile Asp Thr Met Pro Leu
       795                 800                 805
   ctc gcc tgg tcc gtc cag caa gcc cag tct tac cgg gaa ctc caa gcc      5312
   Leu Ala Trp Ser Val Gln Gln Ala Gln Ser Tyr Arg Glu Leu Gln Ala
   810                 815                 820                 825
   gac gga aag gat tcg ggt ttg gtc aag ttc atg atc cgt aac caa tcc      5360
   Asp Gly Lys Asp Ser Gly Leu Val Lys Phe Met Ile Arg Asn Gln Ser
                   830                 835                 840
   tac ttt tac ttt ccc atc ttg ttg ctc gcc cgc ctg tcg tgg ttg aac      5408
   Tyr Phe Tyr Phe Pro Ile Leu Leu Leu Ala Arg Leu Ser Trp Leu Asn
               845                 850                 855
   gag tcc ttc aag tgc gcc ttt ggg ctt gga gct gcg tcg gag aac gct      5456
   Glu Ser Phe Lys Cys Ala Phe Gly Leu Gly Ala Ala Ser Glu Asn Ala
           860                 865                 870
   gct ctc gaa ctc aag gcc aag ggt ctt cag tac ccc ctt ttg gaa aag      5504
   Ala Leu Glu Leu Lys Ala Lys Gly Leu Gln Tyr Pro Leu Leu Glu Lys
       875                 880                 885
   gct ggc atc ctg ctg cac tac gct tgg atg ctt aca gtt tcg tcc ggc      5552
   Ala Gly Ile Leu Leu His Tyr Ala Trp Met Leu Thr Val Ser Ser Gly
   890                 895                 900                 905
   ttt gga cgc ttc tcg ttc gcg tac acc gca ttt tac ttt cta acc gcg      5600
   Phe Gly Arg Phe Ser Phe Ala Tyr Thr Ala Phe Tyr Phe Leu Thr Ala
                   910                 915                 920
   acc gcg tcc tgt gga ttc ttg ctc gcc att gtc ttt ggc ctc ggc cac      5648
   Thr Ala Ser Cys Gly Phe Leu Leu Ala Ile Val Phe Gly Leu Gly His
               925                 930                 935
   aac ggc atg gcc acc tac aat gcc gac gcc cgt ccg gac ttc tgg aag      5696
   Asn Gly Met Ala Thr Tyr Asn Ala Asp Ala Arg Pro Asp Phe Trp Lys
           940                 945                 950
   ctc caa gtc acc acg act cgc aac gtc acg ggc gga cac ggt ttc ccc      5744
   Leu Gln Val Thr Thr Thr Arg Asn Val Thr Gly Gly His Gly Phe Pro
       955                 960                 965
   caa gcc ttt gtc gac tgg ttc tgt ggt ggc ctc cag tac caa gtc gac      5792
   Gln Ala Phe Val Asp Trp Phe Cys Gly Gly Leu Gln Tyr Gln Val Asp
   970                 975                 980                 985
   cac cac tta ttc ccc agc ctg ccc cga cac aat ctg gcc aag aca  cac     5840
   His His Leu Phe Pro Ser Leu Pro Arg His Asn Leu Ala Lys Thr  His
                   990                 995                 1000
   gca ctg gtc gaa  tcg ttc tgc aag gag  tgg ggt gtc cag tac  cac       5885
   Ala Leu Val Glu  Ser Phe Cys Lys Glu  Trp Gly Val Gln Tyr  His
               1005                 1010                 1015
   gaa gcc gac ctt  gtg gac ggg acc atg  gaa gtc ttg cac cat  ttg       5930
   Glu Ala Asp Leu  Val Asp Gly Thr Met  Glu Val Leu His His  Leu
               1020                 1025                 1030
   ggc agc gtg gcc  ggc gaa ttc gtc gtg  gat ttt gta cgc gat  gga       5975
   Gly Ser Val Ala  Gly Glu Phe Val Val  Asp Phe Val Arg Asp  Gly
               1035                 1040                 1045
   ccc gcc atg taa agatctgccg gcatcgatcc cgggccatgg cctgctttaa          6027
   Pro Ala Met
   tgagatatgc gagacgccta tgatcgcatg atatttgctt tcaattctgt tgtgcacgtt    6087
   gtaaaaaacc tgagcatgtg tagctcagat ccttaccgcc ggtttcggtt cattctaatg    6147
   aatatatcac ccgttactat cgtattttta tgaataatat tctccgttca atttactgat    6207
   tgtccgtcga cgagctcggc gcgccgtcga cctgcaggca tgcaagcttc acgctgccgc    6267
   aagcactcag ggcgcaaggg ctgctaaagg aagcggaaca cgtagaaagc cagtccgcag    6327
   aaacggtgct gaccccggat gaatgtcagc tactgggcta tctggacaag ggaaaacgca    6387
   agcgcaaaga gaaagcaggt agcttgcagt gggcttacat ggcgatagct agactgggcg   6447
   gttttatgga cagcaagcga accggaattg ccagctgggg cgccctctgg taaggttggg   6507
   aagccctgca aagtaaactg gatggctttc ttgccgccaa ggatctgatg gcgcagggga   6567
   tcaagatcat gagcggagaa ttaagggagt cacgttatga cccccgccga tgacgcggga   6627
   caagccgttt tacgtttgga actgacagaa ccgcaacgtt gaaggagcca ctcagccgcg   6687
   ggtttctgga gtttaatgag ctaagcacat acgtcagaaa ccattattgc gcgttcaaaa   6747
   gtcgcctaag gtcactatca gctagcaaat atttcttgtc aaaaatgctc cactgacgtt   6807
   ccataaattc ccctcggtat ccaattagag tctcatattc actctcaatc cagatctcga   6867
   ctctagtcga gggcccatgg gagcttggat tgaacaagat ggattgcacg caggttctcc   6927
   ggccgcttgg gtggagaggc tattcggcta tgactgggca caacagacaa tcggctgctc   6987
   tgatgccgcc gtgttccggc tgtcagcgca ggggcgcccg gttctttttg tcaagaccga   7047
   cctgtccggt gccctgaatg aactgcagga cgaggcagcg cggctatcgt ggctggccac   7107
   gacgggcgtt ccttgcgcag ctgtgctcga cgttgtcact gaagcgggaa gggactggct   7167
   gctattgggc gaagtgccgg ggcaggatct cctgtcatct caccttgctc ctgccgagaa   7227
   agtatccatc atggctgatg caatgcggcg gctgcatacg cttgatccgg ctacctgccc   7287
   attcgaccac caagcgaaac atcgcatcga gcgagcacgt actcggatgg aagccggtct   7347
   tgtcgatcag gatgatctgg acgaagagca tcaggggctc gcgccagccg aactgttcgc   7407
   caggctcaag gcgcgcatgc ccgacggcga ggatctcgtc gtgacccatg gcgatgcctg   7467
   cttgccgaat atcatggtgg aaaatggccg cttttctgga ttcatcgact gtggccggct   7527
   gggtgtggcg gaccgctatc aggacatagc gttggctacc cgtgatattg ctgaagagct   7587
   tggcggcgaa tgggctgacc gcttcctcgt gctttacggt atcgccgctc ccgattcgca   7647
   gcgcatcgcc ttctatcgcc ttcttgacga gttcttctga gcgggaccca agctagcttc   7707
   gacggatccc ccgatgagct aagctagcta tatcatcaat ttatgtatta cacataatat   7767
   cgcactcagt ctttcatcta cggcaatgta ccagctgata taatcagtta ttgaaatatt   7827
   tctgaattta aacttgcatc aataaattta tgtttttgct tggactataa tacctgactt   7887
   gttattttat caataaatat ttaaactata tttctttcaa gatgggaatt aattcactgg   7947
   ccgtcgtttt acaacgtcgt gactgggaaa accctggcgt tacccaactt aatcgccttg   8007
   cagcacatcc ccctttcgcc agctggcgta atagcgaaga ggcccgcacc gatcgccctt   8067
   cccaacagtt gcgcagcctg aatggcgccc gctcctttcg ctttcttccc ttcctttctc   8127
   gccacgttcg ccggctttcc ccgtcaagct ctaaatcggg ggctcccttt agggttccga   8187
   tttagtgctt tacggcacct cgaccccaaa aaacttgatt tgggtgatgg ttcacgtagt   8247
   gggccatcgc cctgatagac ggtttttcgc cctttgacgt tggagtccac gttctttaat   8307
   agtggactct tgttccaaac tggaacaaca ctcaacccta tctcgggcta ttcttttgat   8367
   ttataaggga ttttgccgat ttcggaacca ccatcaaaca ggattttcgc ctgctggggc   8427
   aaaccagcgt ggaccgcttg ctgcaactct ctcagggcca ggcggtgaag ggcaatcagc   8487
   tgttgcccgt ctcactggtg aaaagaaaaa ccaccccagt acattaaaaa cgtccgcaat   8547
   gtgttattaa gttgtctaag cgtcaatttg tttacaccac aatatatcct gccaccagcc   8607
   agccaacagc tccccgaccg gcagctcggc acaaaatcac cactcgatac aggcagccca   8667
   tcagtccggg acggcgtcag cgggagagcc gttgtaaggc ggcagacttt gctcatgtta   8727
   ccgatgctat tcggaagaac ggcaactaag ctgccgggtt tgaaacacgg atgatctcgc   8787
   ggagggtagc atgttgattg taacgatgac agagcgttgc tgcctgtgat caaatatcat   8847
   ctccctcgca gagatccgaa ttatcagcct tcttattcat ttctcgctta accgtgacag   8907
   gctgtcgatc ttgagaacta tgccgacata ataggaaatc gctggataaa gccgctgagg   8967
   aagctgagtg gcgctatttc tttagaagtg aacgttgacg atatcaactc ccctatccat   9027
   tgctcaccga atggtacagg tcggggaccc gaagttccga ctgtcggcct gatgcatccc   9087
   cggctgatcg accccagatc tggggctgag aaagcccagt aaggaaacaa ctgtaggttc   9147
   gagtcgcgag atcccccgga accaaaggaa gtaggttaaa cccgctccga tcaggccgag   9207
   ccacgccagg ccgagaacat tggttcctgt aggcatcggg attggcggat caaacactaa   9267
   agctactgga acgagcagaa gtcctccggc cgccagttgc caggcggtaa aggtgagcag   9327
   aggcacggga ggttgccact tgcgggtcag cacggttccg aacgccatgg aaaccgcccc   9387
   cgccaggccc gctgcgacgc cgacaggatc tagcgctgcg tttggtgtca acaccaacag   9447
   cgccacgccc gcagttccgc aaatagcccc caggaccgcc atcaatcgta tcgggctacc   9507
   tagcagagcg gcagagatga acacgaccat cagcggctgc acagcgccta ccgtcgccgc   9567
   gaccccgccc ggcaggcggt agaccgaaat aaacaacaag ctccagaata gcgaaatatt   9627
   aagtgcgccg aggatgaaga tgcgcatcca ccagattccc gttggaatct gtcggacgat 9687
   catcacgagc aataaacccg ccggcaacgc ccgcagcagc ataccggcga cccctcggcc 9747
   tcgctgttcg ggctccacga aaacgccgga cagatgcgcc ttgtgagcgt ccttggggcc 9807
   gtcctcctgt ttgaagaccg acagcccaat gatctcgccg tcgatgtagg cgccgaatgc 9867
   cacggcatct cgcaaccgtt cagcgaacgc ctccatgggc tttttctcct cgtgctcgta 9927
   aacggacccg aacatctctg gagctttctt cagggccgac aatcggatct cgcggaaatc 9987
   ctgcacgtcg gccgctccaa gccgtcgaat ctgagcctta atcacaattg tcaattttaa 10047
   tcctctgttt atcggcagtt cgtagagcgc gccgtgcgtc ccgagcgata ctgagcgaag 10107
   caagtgcgtc gagcagtgcc cgcttgttcc tgaaatgcca gtaaagcgct ggctgctgaa 10167
   cccccagccg gaactgaccc cacaaggccc tagcgtttgc aatgcaccag gtcatcattg 10227
   acccaggcgt gttccaccag gccgctgcct cgcaactctt cgcaggcttc gccgacctgc 10287
   tcgcgccact tcttcacgcg ggtggaatcc gatccgcaca tgaggcggaa ggtttccagc 10347
   ttgagcgggt acggctcccg gtgcgagctg aaatagtcga acatccgtcg ggccgtcggc 10407
   gacagcttgc ggtacttctc ccatatgaat ttcgtgtagt ggtcgccagc aaacagcacg 10467
   acgatttcct cgtcgatcag gacctggcaa cgggacgttt tcttgccacg gtccaggacg 10527
   cggaagcggt gcagcagcga caccgattcc aggtgcccaa cgcggtcgga cgtgaagccc 10587
   atcgccgtcg cctgtaggcg cgacaggcat tcctcggcct tcgtgtaata ccggccattg 10647
   atcgaccagc ccaggtcctg gcaaagctcg tagaacgtga aggtgatcgg ctcgccgata 10707
   ggggtgcgct tcgcgtactc caacacctgc tgccacacca gttcgtcatc gtcggcccgc 10767
   agctcgacgc cggtgtaggt gatcttcacg tccttgttga cgtggaaaat gaccttgttt 10827
   tgcagcgcct cgcgcgggat tttcttgttg cgcgtggtga acagggcaga gcgggccgtg 10887
   tcgtttggca tcgctcgcat cgtgtccggc cacggcgcaa tatcgaacaa ggaaagctgc 10947
   atttccttga tctgctgctt cgtgtgtttc agcaacgcgg cctgcttggc ctcgctgacc 11007
   tgttttgcca ggtcctcgcc ggcggttttt cgcttcttgg tcgtcatagt tcctcgcgtg 11067
   tcgatggtca tcgacttcgc caaacctgcc gcctcctgtt cgagacgacg cgaacgctcc 11127
   acggcggccg atggcgcggg cagggcaggg ggagccagtt gcacgctgtc gcgctcgatc 11187
   ttggccgtag cttgctggac catcgagccg acggactgga aggtttcgcg gggcgcacgc 11247
   atgacggtgc ggcttgcgat ggtttcggca tcctcggcgg aaaaccccgc gtcgatcagt 11307
   tcttgcctgt atgccttccg gtcaaacgtc cgattcattc accctccttg cgggattgcc 11367
   ccgactcacg ccggggcaat gtgcccttat tcctgatttg acccgcctgg tgccttggtg 11427
   tccagataat ccaccttatc ggcaatgaag tcggtcccgt agaccgtctg gccgtccttc 11487
   tcgtacttgg tattccgaat cttgccctgc acgaatacca gcgacccctt gcccaaatac 11547
   ttgccgtggg cctcggcctg agagccaaaa cacttgatgc ggaagaagtc ggtgcgctcc 11607
   tgcttgtcgc cggcatcgtt gcgccacatc taggtactaa aacaattcat ccagtaaaat 11667
   ataatatttt attttctccc aatcaggctt gatccccagt aagtcaaaaa atagctcgac 11727
   atactgttct tccccgatat cctccctgat cgaccggacg cagaaggcaa tgtcatacca 11787
   cttgtccgcc ctgccgcttc tcccaagatc aataaagcca cttactttgc catctttcac 11847
   aaagatgttg ctgtctccca ggtcgccgtg ggaaaagaca agttcctctt cgggcttttc 11907
   cgtctttaaa aaatcataca gctcgcgcgg atctttaaat ggagtgtctt cttcccagtt 11967
   ttcgcaatcc acatcggcca gatcgttatt cagtaagtaa tccaattcgg ctaagcggct 12027
   gtctaagcta ttcgtatagg gacaatccga tatgtcgatg gagtgaaaga gcctgatgca 12087
   ctccgcatac agctcgataa tcttttcagg gctttgttca tcttcatact cttccgagca 12147
   aaggacgcca tcggcctcac tcatgagcag attgctccag ccatcatgcc gttcaaagtg 12207
   caggaccttt ggaacaggca gctttccttc cagccatagc atcatgtcct tttcccgttc 12267
   cacatcatag gtggtccctt tataccggct gtccgtcatt tttaaatata ggttttcatt 12327
   ttctcccacc agcttatata ccttagcagg agacattcct tccgtatctt ttacgcagcg 12387
   gtatttttcg atcagttttt tcaattccgg tgatattctc attttagcca tttattattt 12447
   ccttcctctt ttctacagta tttaaagata ccccaagaag ctaattataa caagacgaac 12507
   tccaattcac tgttccttgc attctaaaac cttaaatacc agaaaacagc tttttcaaag 12567
   ttgttttcaa agttggcgta taacatagta tcgacggagc cgattttgaa accacaatta 12627
   tgggtgatgc tgccaactta ctgatttagt gtatgatggt gtttttgagg tgctccagtg 12687
   gcttctgtgt ctatcagctg tccctcctgt tcagctactg acggggtggt gcgtaacggc 12747
   aaaagcaccg ccggacatca gcgctatctc tgctctcact gccgtaaaac atggcaactg 12807
   cagttcactt acaccgcttc tcaacccggt acgcaccaga aaatcattga tatggccatg 12867
   aatggcgttg gatgccgggc aacagcccgc attatgggcg ttggcctcaa cacgatttta 12927
   cgtcacttaa aaaactcagg ccgcagtcgg taacctcgcg catacagccg ggcagtgacg 12987
   tcatcgtctg cgcggaaatg gacgaacagt ggggctatgt cggggctaaa tcgcgccagc 13047
   gctggctgtt ttacgcgtat gacagtctcc ggaagacggt tgttgcgcac gtattcggtg 13107
   aacgcactat ggcgacgctg gggcgtctta tgagcctgct gtcacccttt gacgtggtga 13167
   tatggatgac ggatggctgg ccgctgtatg aatcccgcct gaagggaaag ctgcacgtaa 13227
   tcagcaagcg atatacgcag cgaattgagc ggcataacct gaatctgagg cagcacctgg 13287
   cacggctggg acggaagtcg ctgtcgttct caaaatcggt ggagctgcat gacaaagtca 13347
   tcgggcatta tctgaacata aaacactatc aataagttgg agtcattacc caattatgat 13407
   agaatttaca agctataagg ttattgtcct gggtttcaag cattagtcca tgcaagtttt 13467
   tatgctttgc ccattctata gatatattga taagcgcgct gcctatgcct tgccccctga 13527
   aatccttaca tacggcgata tcttctatat aaaagatata ttatcttatc agtattgtca 13587
   atatattcaa ggcaatctgc ctcctcatcc tcttcatcct cttcgtcttg gtagcttttt 13647
   aaatatggcg cttcatagag taattctgta aaggtccaat tctcgttttc atacctcggt 13707
   ataatcttac ctatcacctc aaatggttcg ctgggtttat cgcacccccg aacacgagca 13767
   cggcacccgc gaccactatg ccaagaatgc ccaaggtaaa aattgccggc cccgccatga 13827
   agtccgtgaa tgccccgacg gccgaagtga agggcaggcc gccacccagg ccgccgccct 13887
   cactgcccgg cacctggtcg ctgaatgtcg atgccagcac ctgcggcacg tcaatgcttc 13947
   cgggcgtcgc gctcgggctg atcgcccatc ccgttactgc cccgatcccg gcaatggcaa 14007
   ggactgccag cgctgccatt tttggggtga ggccgttcgc ggccgagggg cgcagcccct 14067
   ggggggatgg gaggcccgcg ttagcgggcc gggagggttc gagaaggggg ggcacccccc 14127
   ttcggcgtgc gcggtcacgc gcacagggcg cagccctggt taaaaacaag gtttataaat 14187
   attggtttaa aagcaggtta aaagacaggt tagcggtggc cgaaaaacgg gcggaaaccc 14247
   ttgcaaatgc tggattttct gcctgtggac agcccctcaa atgtcaatag gtgcgcccct 14307
   catctgtcag cactctgccc ctcaagtgtc aaggatcgcg cccctcatct gtcagtagtc 14367
   gcgcccctca agtgtcaata ccgcagggca cttatcccca ggcttgtcca catcatctgt 14427
   gggaaactcg cgtaaaatca ggcgttttcg ccgatttgcg aggctggcca gctccacgtc 14487
   gccggccgaa atcgagcctg cccctcatct gtcaacgccg cgccgggtga gtcggcccct 14547
   caagtgtcaa cgtccgcccc tcatctgtca gtgagggcca agttttccgc gaggtatcca 14607
   caacgccggc ggccgcggtg tctcgcacac ggcttcgacg gcgtttctgg cgcgtttgca 14667
   gggccataga cggccgccag cccagcggcg agggcaacca gcccggtgag cgtcgcaaag 14727
   gcgctcggtc ttgccttgct cgtcggtgat gtacttcacc agctccgcga agtcgctctt 14787
   cttgatggag cgcatgggga cgtgcttggc aatcacgcgc accccccggc cgttttagcg 14847
   gctaaaaaag tcatggctct gccctcgggc ggaccacgcc catcatgacc ttgccaagct 14907
   cgtcctgctt ctcttcgatc ttcgccagca gggcgaggat cgtggcatca ccgaaccgcg 14967
   ccgtgcgcgg gtcgtcggtg agccagagtt tcagcaggcc gcccaggcgg cccaggtcgc 15027
   cattgatgcg ggccagctcg cggacgtgct catagtccac gacgcccgtg attttgtagc 15087
   cctggccgac ggccagcagg taggccgaca ggctcatgcc ggccgccgcc gccttttcct 15147
   caatcgctct tcgttcgtct ggaaggcagt acaccttgat aggtgggctg cccttcctgg 15207
   ttggcttggt ttcatcagcc atccgcttgc cctcatctgt tacgccggcg gtagccggcc 15267
   agcctcgcag agcaggattc ccgttgagca ccgccaggtg cgaataaggg acagtgaaga 15327
   aggaacaccc gctcgcgggt gggcctactt cacctatcct gcccggctga cgccgttgga 15387
   tacaccaagg aaagtctaca cgaacccttt ggcaaaatcc tgtatatcgt gcgaaaaagg 15447
   atggatatac cgaaaaaatc gctataatga ccccgaagca gggttatgca gcggaaaagc 15507
   gccacgcttc ccgaagggag aaaggcggac aggtatccgg taagcggcag ggtcggaaca 15567
   ggagagcgca cgagggagct tccaggggga aacgcctggt atctttatag tcctgtcggg 15627
   tttcgccacc tctgacttga gcgtcgattt ttgtgatgct cgtcaggggg gcggagccta 15687
   tggaaaaacg ccagcaacgc ggccttttta cggttcctgg ccttttgctg gccttttgct 15747
   cacatgttct ttcctgcgtt atcccctgat tctgtggata accgtattac cgcctttgag 15807
   tgagctgata ccgctcgccg cagccgaacg accgagcgca gcgagtcagt gagcgaggaa 15867
   gcggaagagc gccagaaggc cgccagagag gccgagcgcg gccgtgaggc ttggacgcta 15927
   gggcagggca tgaaaaagcc cgtagcgggc tgctacgggc gtctgacgcg gtggaaaggg 15987
   ggaggggatg ttgtctacat ggctctgctg tagtgagtgg gttgcgctcc ggcagcggtc 16047
   ctgatcaatc gtcacccttt ctcggtcctt caacgttcct gacaacgagc ctccttttcg 16107
   ccaatccatc gacaatcacc gcgagtccct gctcgaacgc tgcgtccgga ccggcttcgt  16167
   cgaaggcgtc tatcgcggcc cgcaacagcg gcgagagcgg agcctgttca acggtgccgc  16227
   cgcgctcgcc ggcatcgctg tcgccggcct gctcctcaag cacggcccca acagtgaagt  16287
   agctgattgt catcagcgca ttgacggcgt ccccggccga aaaacccgcc tcgcagagga  16347
   agcgaagctg cgcgtcggcc gtttccatct gcggtgcgcc cggtcgcgtg ccggcatgga  16407
   tgcgcgcgcc atcgcggtag gcgagcagcg cctgcctgaa gctgcgggca ttcccgatca  16467
   gaaatgagcg ccagtcgtcg tcggctctcg gcaccgaatg cgtatgattc tccgccagca  16527
   tggcttcggc cagtgcgtcg agcagcgccc gcttgttcct gaagtgccag taaagcgccg  16587
   gctgctgaac ccccaaccgt tccgccagtt tgcgtgtcgt cagaccgtct acgccgacct  16647
   cgttcaacag gtccagggcg gcacggatca ctgtattcgg ctgcaacttt gtcatgcttg  16707
   acactttatc actgataaac ataatatgtc caccaactta tcagtgataa agaatccgcg  16767
   cgttcaatcg gaccagcgga ggctggtccg gaggccagac gtgaaaccca acatacccct  16827
   gatcgtaatt ctgagcactg tcgcgctcga cgctgtcggc atcggcctga ttatgccggt  16887
   gctgccgggc ctcctgcgcg atctggttca ctcgaacgac gtcaccgccc actatggcat  16947
   tctgctggcg ctgtatgcgt tggtgcaatt tgcctgcgca cctgtgctgg gcgcgctgtc  17007
   ggatcgtttc gggcggcggc caatcttgct cgtctcgctg gccggcgcca gatc        17061
<210> 72
   <211> 290
   <212> PRT
   <213> Phaeodactylum tricornutum, Physcomitrella patents, Caenorhabditis
   elegans
<400> 72
   Met Glu Val Val Glu Arg Phe Tyr Gly Glu Leu Asp Gly Lys Val Ser
   1               5                   10                  15
   Gln Gly Val Asn Ala Leu Leu Gly Ser Phe Gly Val Glu Leu Thr Asp
               20                  25                  30
   Thr Pro Thr Thr Lys Gly Leu Pro Leu Val Asp Ser Pro Thr Pro Ile
           35                  40                  45
   Val Leu Gly Val Ser Val Tyr Leu Thr Ile Val Ile Gly Gly Leu Leu
       50                  55                  60
   Trp Ile Lys Ala Arg Asp Leu Lys Pro Arg Ala Ser Glu Pro Phe Leu
   65                  70                  75                  80
   Leu Gln Ala Leu Val Leu Val His Asn Leu Phe Cys Phe Ala Leu Ser
                   85                  90                  95
   Leu Tyr Met Cys Val Gly Ile Ala Tyr Gln Ala Ile Thr Trp Arg Tyr
               100                 105                 110
   Ser Leu Trp Gly Asn Ala Tyr Asn Pro Lys His Lys Glu Met Ala Ile
           115                 120                 125
   Leu Val Tyr Leu Phe Tyr Met Ser Lys Tyr Val Glu Phe Met Asp Thr
       130                 135                 140
   Val Ile Met Ile Leu Lys Arg Ser Thr Arg Gln Ile Ser Phe Leu His
   145                 150                 155                 160
   Val Tyr His His Ser Ser Ile Ser Leu Ile Trp Trp Ala Ile Ala His
                   165                 170                 175
   His Ala Pro Gly Gly Glu Ala Tyr Trp Ser Ala Ala Leu Asn Ser Gly
               180                 185                 190
   Val His Val Leu Met Tyr Ala Tyr Tyr Phe Leu Ala Ala Cys Leu Arg
           195                 200                 205
   Ser Ser Pro Lys Leu Lys Asn Lys Tyr Leu Phe Trp Gly Arg Tyr Leu
       210                 215                 220
   Thr Gln Phe Gln Met Phe Gln Phe Met Leu Asn Leu Val Gln Ala Tyr
   225 230 235 240
   Tyr Asp Met Lys Thr Asn Ala Pro Tyr Pro Gln Trp Leu Ile Lys Ile
   245 250 255
   Leu Phe Tyr Tyr Met Ile Ser Leu Leu Phe Leu Phe Gly Asn Phe Tyr
   260 265 270
   Val Gln Lys Tyr Ile Lys Pro Ser Asp Gly Lys Gln Lys Gly Ala Lys
   275 280 285
   Thr Glu
   290
<210> 73
   <211> 282
   <212> PRT
   <213> Phaeodactylum tricornutum, Physcomitrella patens, Caenorhabditis
   elegans
<400> 73
   Met Glu Asn Phe Trp Ser Ile Val Val Phe Phe Leu Leu Ser Ile Leu
   1 5 10 15
   Phe Ile Leu Tyr Asn Ile Ser Thr Val Cys His Tyr Tyr Met Arg Ile
   20 25 30
   Ser Phe Tyr Tyr Phe Thr Ile Leu Leu His Gly Met Glu Val Cys Val
   35 40 45
   Thr Met Ile Pro Ser Trp Leu Asn Gly Lys Gly Ala Asp Tyr Val Phe
   50 55 60
   His Ser Phe Phe Tyr Trp Cys Lys Trp Thr Gly Val His Thr Thr Val
   65 70 75 80
   Tyr Gly Tyr Glu Lys Thr Gln Val Glu Gly Pro Ala Val Val Ile Cys
   85 90 95
   Asn His Gln Ser Ser Leu Asp Ile Leu Ser Met Ala Ser Ile Trp Pro
   100 105 110
   Lys Asn Cys Val Val Met Met Lys Arg Ile Leu Ala Tyr Val Pro Phe
   115 120 125
   Phe Asn Leu Gly Ala Tyr Phe Ser Asn Thr Ile Phe Ile Asp Arg Tyr
   130 135 140
   Asn Arg Glu Arg Ala Met Ala Ser Val Asp Tyr Cys Ala Ser Glu Met
   145 150 155 160
   Lys Asn Arg Asn Leu Lys Leu Trp Val Phe Pro Glu Gly Thr Arg Asn
   165 170 175
   Arg Glu Gly Gly Phe Ile Pro Phe Lys Lys Gly Ala Phe Asn Ile Ala
   180 185 190
   Val Arg Ala Gln Ile Pro Ile Ile Pro Val Val Phe Ser Asp Tyr Arg
   195 200 205
   Asp Phe Tyr Ser Lys Pro Gly Arg Tyr Phe Lys Asn Asp Gly Glu Val
   210 215 220
   Val Ile Arg Val Leu Asp Ala Ile Pro Thr Lys Gly Leu Thr Leu Asp
   225 230 235 240
   Asp Val Ser Glu Leu Ser Asp Met Cys Arg Asp Val Met Leu Ala Ala
   245 250 255
   Tyr Lys Glu Val Thr Leu Glu Ala Gln Gln Arg Asn Ala Thr Arg Arg
   260 265 270
   Gly Glu Thr Lys Asp Gly Lys Lys Ser Glu
   275 280
<210> 74
   <211> 477
   <212> PRT
   <213> Phaeodactylum tricornutum, Physcomitrella patens, Caenorhabditi
   elegans
<400> 74
   Met Gly Lys Gly Gly Asp Ala Arg Ala Ser Lys Gly Ser Thr Ala Ala
   1               5                   10                  15
   Arg Lys Ile Ser Trp Gln Glu Val Lys Thr His Ala Ser Pro Glu Asp
               20                  25                  30
   Ala Trp Ile Ile His Ser Asn Lys Val Tyr Asp Val Ser Asn Trp His
           35                  40                  45
   Glu His Pro Gly Gly Ala Val Ile Phe Thr His Ala Gly Asp Asp Met
       50                  55                  60
   Thr Asp Ile Phe Ala Ala Phe His Ala Pro Gly Ser Gln Ser Leu Met
   65                  70                  75                  80
   Lys Lys Phe Tyr Ile Gly Glu Leu Leu Pro Glu Thr Thr Gly Lys Glu
                   85                  90                  95
   Pro Gln Gln Ile Ala Phe Glu Lys Gly Tyr Arg Asp Leu Arg Ser Lys
               100                 105                 110
   Leu Ile Met Met Gly Met Phe Lys Ser Asn Lys Trp Phe Tyr Val Tyr
           115                 120                 125
   Lys Cys Leu Ser Asn Met Ala Ile Trp Ala Ala Ala Cys Ala Leu Val
       130                 135                 140
   Phe Tyr Ser Asp Arg Phe Trp Val His Leu Ala Ser Ala Val Met Leu
   145                 150                 155                 160
   Gly Thr Phe Phe Gln Gln Ser Gly Trp Leu Ala His Asp Phe Leu His
                   165                 170                 175
   His Gln Val Phe Thr Lys Arg Lys His Gly Asp Leu Gly Gly Leu Phe
               180                 185                 190
   Trp Gly Asn Leu Met Gln Gly Tyr Ser Val Gln Trp Trp Lys Asn Lys
           195                 200                 205
   His Asn Gly His His Ala Val Pro Asn Leu His Cys Ser Ser Ala Val
       210                 215                 220
   Ala Gln Asp Gly Asp Pro Asp Ile Asp Thr Met Pro Leu Leu Ala Trp
   225                 230                 235                 240
   Ser Val Gln Gln Ala Gln Ser Tyr Arg Glu Leu Gln Ala Asp Gly Lys
                   245                 250                 255
   Asp Ser Gly Leu Val Lys Phe Met Ile Arg Asn Gln Ser Tyr Phe Tyr
               260                 265                 270
   Phe Pro Ile Leu Leu Leu Ala Arg Leu Ser Trp Leu Asn Glu Ser Phe
           275                 280                 285
   Lys Cys Ala Phe Gly Leu Gly Ala Ala Ser Glu Asn Ala Ala Leu Glu
       290                 295                 300
   Leu Lys Ala Lys Gly Leu Gln Tyr Pro Leu Leu Glu Lys Ala Gly Ile
   305                 310                 315                 320
   Leu Leu His Tyr Ala Trp Met Leu Thr Val Ser Ser Gly Phe Gly Arg
                   325                 330                 335
   Phe Ser Phe Ala Tyr Thr Ala Phe Tyr Phe Leu Thr Ala Thr Ala Ser
               340                 345                 350
   Cys Gly Phe Leu Leu Ala Ile Val Phe Gly Leu Gly His Asn Gly Met
           355                 360                 365
   Ala Thr Tyr Asn Ala Asp Ala Arg Pro Asp Phe Trp Lys Leu Gln Val
       370                 375                 380
   Thr Thr Thr Arg Asn Val Thr Gly Gly His Gly Phe Pro Gln Ala Phe
   385                 390                 395                 400
   Val Asp Trp Phe Cys Gly Gly Leu Gln Tyr Gln Val Asp His His Leu
                   405                 410                 415
   Phe Pro Ser Leu Pro Arg His Asn Leu Ala Lys Thr His Ala Leu Val
               420                 425                 430
   Glu Ser Phe Cys Lys Glu Trp Gly Val Gln Tyr His Glu Ala Asp Leu
           435                 440                 445
   Val Asp Gly Thr Met Glu Val Leu His His Leu Gly Ser Val Ala Gly
       450                 455                 460
   Glu Phe Val Val Asp Phe Val Arg Asp Gly Pro Ala Met
   465                 470                 475

## Beschreibung

Die vorliegende Erfindung betrifft allgemein ein Verfahren zur Herstellung von langkettigen mehrfach ungesättigten Fettsäuren in einem Organismus, indem Nukleinsäuren in den Organismus eingebracht werden, die für Polypeptide mit Acyltransferaseaktivität codieren. Vorteilhaft können diese Nukleinsäuresequenzen gegebenenfalls zusammen mit weiteren Nukleinsäuresequenzen, die für Polypeptide der Biosynthese des Fettsäure- oder Lipidstoffwechels codieren, in dem Organismus exprimiert werden. Weiterhin betrifft die Erfindung ein Verfahren zur Herstellung von Ölen und/oder Triacylglyceriden mit einem erhöhten Gehalt an langkettigen mehrfach ungesättigten Fettsäuren.

Die Erfindung betrifft weiterhin die Nukleinsäuresequenzen, Nukleinsäurekonstrukte, Vektoren und Organismen enthaltend die erfindungsgemäßen Nukleinsäuresequenzen gemäß den Patentansprüchen, Vektoren enthaltend die Nukleinsäuresequenzen und/oder die Nukleinsäurekonstrukte sowie transgene Organismen enthalten die vorgenannten Nukleinsäuresequenzen, Nukleinsäurekonstrukte und/oder Vektoren.

Ein weiterer Teil der Beschreibung betrifft Öle, Lipide und/oder Fettsäuren hergestellt nach dem erfindungsgemäßen Verfahren.Die Erfindung betrifft ferner die Verwendung erfindungsgemäßer Lysophosphatidsäure-Acyltransferasen zum Herstellen von Pflanzenölen.

Soweit im Rahmen dieser Beschreibung auf Nukleinsäuren, Polypeptide und Verfahren und Verwendungen solcher Nukleinsäuren und/oder Polypeptide hingewiesen wird, dienen solche Hinweise lediglich zum allgemeinen Beschreiben des technischen Gebiets der Erfindung, ohne den Gegenstand der Patentansprüche zu erweitern.

Fettsäuren und Triacylglyceride haben eine Vielzahl von Anwendungen in der Lebensmittelindustrie, der Tierernährung, der Kosmetik und im Pharmabereich. Je nachdem, ob es sich um freie gesättigte und ungesättigte Fettsäuren oder um Triacylglyceride mit einem erhöhten Gehalt an gesättigten oder ungesättigten Fettsäuren handelt, sind sie für die unterschiedlichsten Anwendungen geeignet. Mehrfach ungesättigte w-3-Fettsäuren und w-6-Fettsäuren stellen dabei einen wichtigen Bestandteil der tierischen und menschlichen Nahrung dar. Aufgrund der heute üblichen Zusammensetzung der menschlichen Nahrung ist ein Zusatz von mehrfach ungesättigten w-3-Fettsäuren, die bevorzugt in Fischölen vorkommen, zur Nahrung besonders wichtig. So werden beispielsweise mehrfach ungesättigte Fettsäuren wie Docosahexaensäure (= DHA, C22:6^{Δ4,7,10,13,16,19}) oder Eisosapentaensäure (= EPA, C20:5^{Δ5,8,11,14,17}) Babynahrung zur Erhöhung des Nährwertes zugesetzt. Der ungesättigten Fettsäure DHA wird dabei ein positiver Effekt auf die Entwicklung des Gehirns zugeschrieben.

Im folgenden werden mehrfach ungesättigte Fettsäuren als PUFA, PUFAs, LCPUFA oder LCPUFAs bezeichnet (**p**oly **u**nsaturated **f**atty **a**cids, PUFA, mehrfach ungesättigte Fettsäuren; **l**ong **c**hain **p**oly **u**nsaturated **f**atty **a**cids, **LCPUFA,** langkettige mehrfach ungesättigte Fettsäuren).

Hauptsächlich werden die verschiedenen Fettsäuren und Triglyceride aus Mikroorganismen wie Mortierella oder Schizochytrium oder aus Öl-produzierenden Pflanzen wie Soja, Raps, Algen wie Crypthecodinium oder Phaeodactylum und weiteren gewonnen, wobei sie in der Regel in Form ihrer Triacylglyceride (= Triglyceride = Triglycerole) anfallen. Sie können aber auch aus Tieren wie z.B. Fischen gewonnen werden. Die freien Fettsäuren werden vorteilhaft durch Verseifung hergestellt. Höhere mehrfach ungesättigte Fettsäuren wie DHA, EPA, Arachidonsäure (= ARA, C20:4^{Δ5,8,11,14}), Dihomo-y-linolensäure (C20:3^{Δ8,11,14}) oder Docosapentaensäure (DPA, C22:5^{Δ7,10,13,16,19}) lassen sich nicht aus Ölfruchtpflanzen wie Raps, Soja, Sonnenblume, Färberdistel oder anderen isolieren. Übliche natürliche Quellen für diese Fettsäuren sind Fische wie Hering, Lachs, Sardine, Goldbarsch, Aal, Karpfen, Forelle, Heilbutt, Makrele, Zander oder Thunfisch oder Algen.

Je nach Anwendungszweck sind Öle mit gesättigten oder ungesättigten Fettsäuren bevorzugt, so sind z.B. in der humanen Ernährung Lipide mit ungesättigten Fettsäuren speziell mehrfach ungesättigten Fettsäuren bevorzugt. Den mehrfach ungesättigten ω-3-Fettsäuren wird dabei ein positiver Effekt auf den Cholesterinspiegel im Blut und damit auf die Möglichkeit der Prävention einer Herzerkrankung zugeschrieben. Durch Zugabe dieser w-3-Fettsäuren zu Nahrung kann das Risiko einer Herzerkrankung, eines Schlaganfalls oder von Bluthochdruck deutlich verringert werden. Auch entzündliche speziell chronisch entzündliche Prozesse im Rahmen immunologischer Erkrankungen wie rheumatroider Arthritis lassen sich durch ω-3-Fettsäuren positiv beeinflussen. Sie werden deshalb Lebensmitteln speziell diätischen Lebensmitteln zugegeben oder finden in Medikamenten Anwendung. w-6-Fettsäuren wie Arachidonsäure haben bei diesen rheumatischen Erkrankungen aufgrund unserer üblichen Nahrungsmittelzusammensetzung eher einen negativen Effekt auf diese Krankheiten.

ω-3- und w-6-Fettsäuren sind Vorläufer von Gewebshormonen, den sogenannten Eicosanoiden wie den Prostaglandinen, die sich von der Dihomo-γ-linolensäure, der Arachidonsäure und der Eicosapentaensäure ableiten, den Thromoxanen und Leukotrienen, die sich von der Arachidonsäure und der Eicosapentaensäure ableiten. Eicosanoide (sog. PG₂-Serie), die aus ω-6-Fettsäuren gebildet werden fördern in der Regel Entzündungsreaktionen, während Eicosanoide (sog. PG₃-Serie) aus ω-3-Fettsäuren geringe oder keine entzündungsfördernde Wirkung haben.

Aufgrund ihrer positiven Eigenschaften hat es in der Vergangenheit nicht an Ansätzen gefehlt, Gene, die an der Synthese von Fettsäuren bzw. Triglyceriden beteiligt sind, für die Herstellung von Ölen in verschiedenen Organismen mit geändertem Gehalt an ungesättigten Fettsäuren verfügbar zu machen. So wird in WO 91/13972 und seinem US-Äquivalent eine Δ-9-Desaturase beschrieben. In WO 93/11245 wird eine Δ-15-Desaturase in WO 94/11516 wird eine Δ-12-Desaturase beansprucht. Weitere Desaturasen werden beispielsweise in EP-A-0 550 162, WO 94/18337, WO 97/30582, WO 97/21340, WO 95/18222, EP-A-0 794 250, Stukey et al., J. Biol. Chem., 265, 1990: 20144-20149, Wada et al., Nature 347, 1990: 200-203 oder Huang et al., Lipids 34, 1999: 649-659 beschrieben. Die biochemische Charakterisierung der verschiedenen Desaturasen ist jedoch bisher nur unzureichend erfolgt, da die Enzyme als membrangebundene Proteine nur sehr schwer zu isolieren und zu charakterisieren sind (McKeon et al., Methods in Enzymol. 71, 1981: 12141-12147, Wang et al., Plant Physiol. Biochem., 26, 1988: 777-792). In der Regel erfolgt die Charakterisierung membrangebundener Desaturasen durch Einbringung in einen geeigneten Organismus, der anschließend auf Enzymaktivität mittels Edukt- und Produktanalyse untersucht wird. Δ-6-Desaturase werden in WO 93/06712, US 5,614,393, US 5,614,393, WO 96/21022, WO 00/21557 und WO 99/27111 beschrieben und auch die Anwendung zur Produktion in transgenen Organismen beschrieben wie in WO 98/46763, WO 98/46764, WO 9846765. Dabei wird auch die Expression verschiedener Desaturasen wie in WO 99/64616 oder WO 98/46776 und Bildung polyungesättigter Fettsäuren beschrieben und beansprucht. Bzgl. der Effektivität der Expression von Desaturasen und ihren Einfluss auf die Bildung polyungesättigter Fettsäuren ist anzumerken, dass durch Expression einer einzelnen Desaturase wie bisher beschrieben lediglich geringe Gehalte an ungesättigten Fettsäuren/Lipiden wie z.B. γ-Linolensäure und Stearidonsäure erreicht wurden. Weiterhin wurde in der Regel ein Gemisch aus w-3- und ω-6-Fettsäuren erhalten.

Besonders geeignete Mikroorganismen zur Herstellung von PUFAs sind Mikroorganismen wie Mikroalgen wie Phaeodactylum tricornutum, Porphoridium-Arten, Thraustochytrien-Arten, Schizochytrien-Arten oder Crypthecodinium-Arten, Ciliaten, wie Stylonychia oder Colpidium, Pilze, wie Mortierella, Entomophthora oder Mucor und/oder Moosen wie Physcomitrella, Ceratodon und Marchantia (R. Vazhappilly & F. Chen (1998) Botanica Marina 41: 553-558; K. Totani & K. Oba (1987) Lipids 22: 1060-1062; M. Akimoto et al. (1998) Appl. Biochemistry and Biotechnology 73: 269-278).. Durch Stammselektion ist eine Anzahl von Mutantenstämmen der entsprechenden Mikroorganismen entwickelt worden, die eine Reihe wünschenswerter Verbindungen, einschließlich PUFAs, produzieren. Die Mutation und Selektion von Stämmen mit verbesserter Produktion eines bestimmten Moleküls wie den mehrfach ungesättigten Fettsäuren ist jedoch ein zeitraubendes und schwieriges Verfahren. Deshalb werden, wenn immer möglich wie oben beschrieben gentechnologische Verfahren bevorzugt. Mit Hilfe der vorgenannten Mikroorganismen lassen sich jedoch nur begrenzte Mengen der gewünschten mehrfach ungesättigten Fettsäuren wie DPA, EPA oder ARA herstellen. Wobei diese in der Regel je nach verwendeten Mikroorganismus als Fettsäuregemische aus beispielsweise EPA, DPA und DHA anfallen.

Die Biosynthese von LCPUFAs und der Einbau von LCPUFAs in Membranen oder Triacylglyceride erfolgt über verschiedene Stoffwechselwege (A. Abbadi et al. (2001) European Journal of Lipid Science & Technology 103:106-113). In Bakterien wie Vibrio und Mikroalgen wie Schizochytrium wird Malonyl-CoA über eine LCPUFA-produzierende Polyketidsynthase zu LCPUFAs umgesetzt (J.G. Metz et al. (2001) Science 293: 290-293; WO 00/42195; WO 98/27203; WO 98/55625). In Mikroalgen wie Phaeodactylum und Moosen wie Physcomitrella werden ungesättigte Fettsäuren wie Linolsäure oder Linolensäure in Form ihrer Acyl-CoAs in mehreren Desaturierungs- und Elongationsschritten zu LCPUFAs umgesetzt (T.K. Zank et al. (2000) Biochemical Society Transactions 28: 654-658). Bei Säugetieren beinhaltet die Biosynthese von DHA zusätzlich zu Desaturierungs- und Elongationsschritten eine Kettenverkürzung über beta-Oxidation.

LCPUFAs liegen in Mikroorganismen und niederen Pflanzen entweder ausschließlich in Form von Membranlipiden vor, wie bei Physcomitrella und Phaeodactylum, oder sie sind in Membranlipiden und Triacylglyceriden vorhanden, wie bei Schizochytrium und Mortierella. Der Einbau von LCPUFAs in Lipide und Öle wird durch verschiedene Acyltransferasen und Transacylasen katalysiert. Diese Enzyme sind bereits bekannt für den Einbau von gesättigten und ungesättigten Fettsäuren [A.R. Slabas (2001) J. Plant Physiology 158: 505-513; M. Frentzen (1998) Fett/Lipid 100: 161-166); S. Cases et al. (1998) Proc. Nat. Acad. Sci. USA 95: 13018-13023]. Bei den Acyltransferasen handelt sich um Enzyme des sogenannten Kennedy-Pathways, die an der cytoplasmatischen Seite des Membransystems des Endoplasmatischen Reticulums, nachfolgend als 'ER' bezeichnet, lokalisiert sind. Experimentell können Membranen des ER als sogenannte ,mikrosomale Fraktionen' aus verschiedenen Organismen isoliert werden [D.S. Knutzon et al. (1995) Plant Physiology 109: 999-1006; S. Mishra & Y Kamisaka (2001) Biochemistry 355: 315-322; US 5968791]. Diese ER-gebundenen Acyltransferasen in der mikrosomalen Fraktion verwenden Acyl-CoA als aktivierte Form der Fettsäuren. Glycerin-3-phosphat Acyltransferase, im folgenden GPAT genannt, katalysiert den Einbau von Acylgruppen an der sn-1 Position von Glycerin-3-phosphat. 1-Acylglycerin-3-phosphat Acyltransferase (E.C. 2.3.1.51), auch Lysophosphatidsäure Acyltransferase, im folgenden LPAAT genannt, katalysiert den Einbau von Acylgruppen an der sn-2 Position von Lysophosphatidsäure, nachfolgend als LPA abgekürzt. Nach Dephosphorylierung von Phosphatidsäure durch Phosphatidsäure Phosphatase katalysiert Diacylglycerin Acyltransferase, im folgenden DAGAT genannt, den Einbau von Acylgruppen an der sn-3 Position von Diacylglycerins. Neben diesen Kennedy Pathway Enzymen sind weitere Enzyme am Einbau von Fettsäuren in Triacylglyceride beteiligt, die Acylgruppen aus Membranlipiden in Triacylglyceride einbauen können. Phospholipid Diacylglycerin Acyltransferase, nachfolgend PDAT genannt und Lysophosphatidylcholin Acyltransferase, nachfolgend LPCAT genannt. Auch andere Enzyme wie Lecithin Cholesterin Acyltransferase (LCAT) können am Transfer von Acylgruppen aus Membranlipiden in Triacylglyceride beteiligt sein.

In WO 98/54302 wird von Tjoelker et al. eine humane Lysophosphatidsäure Acyltransferase offenbart sowie ihre mögliche Verwendung zur Therapie von Krankheiten, als diagnostisches Agens sowie eine Methode zur Identifizierung von Modulatoren der humanen LPAAT. Von Leung et al. werden in WO 98/54303 Säuger Lysophosphatidsäure Acyltransferasen beschrieben. Weiterhin offenbaren Leung et al. ein Verfahren zum Screening von pharmazeutischen Verbindungen für die Anwendung beispielsweise bei der Behandlung von Entzündungen.

Weiterhin sind in der Literatur und Patenten eine Vielzahl von Acyltransferasen mit den verschiedensten enzymatischen Funktionen beschrieben worden, so werden z.B. in WO 98/55632 und WO 93/10241 Fettsäure-Alkohol-Acyltransferasen beschrieben, die an der Wachssynthese beteiligt sind. In WO 98/55631 wird eine DAGAT (Diacylglycerin Acyltransferase) aus Mortierella ramanniana beschrieben sowie eine aus Jojoba stammende Wachssynthase, die auch DAGAT-Aktivität hat. Slabas et al. (WO 94/13814) offenbart eine membrangebundene sn2-spezifische Acyltransferase, die eine andere Selektivität beim Einbau von einfach ungesättigter Erukasäure für die sn2-Position hat und so in Raps eine erhöhte Ausbeute an Erukasäure ermöglicht. In WO 96/24674 wird ein entsprechendes Enzym bzw. Gen aus Limnanthes douglasii beschrieben. Davies et al. beschreiben in WO 95/27791 LPAATs, die spezifisch für mittellange Fettsäuren sind und diese in die sn2-Position von Triglyceriden einbauen. Weitere neue pflanzliche Acyltransferasesequenzen, die über Homologievergleiche mit Sequenzen aus öffentlichen Datenbanken gefunden wurden, werden von Lassner et al. (WO 00/18889) beschrieben. Angaben über die spezifische Funktion dieser Acyltransferasesequenzen oder biochemische Daten zu den entsprechenden Enzymen sind WO 00/18889 nicht zu entnehmen.

Die enzymatische Aktivität einer LPCAT wurde erstmals in Ratten beschrieben [Land (1960) Journal of Biological Chemistry 235: 2233-2237]. In Pflanzen existiert eine plastidäre Isoform der LPCAT [Akermoun et al. (2000) Biochemical Society Transactions 28: 713-715] sowie eine ER gebundene Isoform [Tumaney und Rajasekharan (1999) Biochimica et Biophysica Acta 1439: 47-56; Fraser und Stobart, Biochemical Society Transactions (2000) 28: 715-7718]. LPCAT ist in Tieren wie auch in Pflanzen an der Biosynthese und der Transacylierung von mehrfach ungesättigten Fettsäuren beteiligt [Stymne und Stobart (1984) Biochem. J. 223: 305-314; Stymne und Stobart (1987) in 'The Biochemistry of Plants: a Comprehensive Treatise', Vol. 9 (Stumpf, P. K. ed.) pp. 175-214, Academic Press, New York]. Eine wichtige Funktion der LPCAT oder allgemeiner gesagt einer Acyl-CoA:Lysophospholipid Acyltransferase, nachfolgend LPLAT genannt, bei der ATP-unabhängigen Synthese von Acyl-CoA aus Phospholipiden wurde von Yamashita et al. (2001; Journal of Biological Chemistry 276: 26745-26752) beschrieben.

Trotz vieler biochemischer Daten konnten bisher keine Gene kodierend für LPCAT identifiziert werden. Gene anderer verschiedener pflanzlicher Acyltransferasen konnten isoliert werden und werden in WO 00/18889 (Novel Plant Acyltransferases) beschrieben.

Höhere Pflanzen enthalten mehrfach ungesättigte Fettsäuren wie Linolsäure (C18:2) und Linolensäure (C18:3). ARA, EPA und DHA kommen im Samenöl höherer Pflanzen gar nicht oder nur in Spuren vor (E. Ucciani: Nouveau Dictionnaire des Huiles Vegetales. Technique & Documentation - Lavoisier, 1995. ISBN: 2-7430-0009-0). Es ist vorteilhaft, in höheren Pflanzen, bevorzugt in Ölsaaten wie Raps, Lein, Sonnenblume und Soja, LCPUFAs herzustellen, da auf diese Weise große Mengen qualitativ hochwertiger LCPUFAs für die Lebensmittelindustrie, die Tierernährung und für pharmazeutische Zwecke kostengünstig gewonnen werden können. Hierzu werden vorteilhaft über gentechnische Methoden Gene kodierend für Enzyme der Biosynthese von LCPUFAs in Ölsaaten eingeführt und exprimiert werden. Dies sind Gene kodierend für Δ-6-Desaturase, Δ-6-Elongase, Δ-5-Desaturase, Δ-5-Elongase und Δ-4-Desaturase. Diese Gene können vorteilhaft aus Mikroorganismen und niederen Pflanzen isoliert werden, die LCPUFAs herstellen und in den Membranen oder Triacylglyceriden einbauen. So konnten bereits Δ-6-Desaturase-Gene aus dem Moos Physcomitrella patens und Δ-6-Elongase-Gene aus P. patens und dem Nematoden C. elegans isoliert.

Transgene Pflanzen, die Gene kodierend für Enzyme der LCPUFA-Biosynthese exprimieren, sind geeignet, geringe Mengen dieser LCPUFAs zu produzieren, allerdings besteht die Gefahr, dass diese nicht in Triacylglyceride, sondern in Membranen eingebaut werden, weil die endogenen Acyltransferasen und Transacylasen LCPUFAs eventuell nicht als Substrat erkennen und folglich nicht in Triacylglyceride einbauen. Dies ist aus folgenden Gründen unerwünscht: (i) der Hauptlipidanteil in Ölsaaten sind Triacylglyceride. Daher ist es aus wirtschaftlicher Sicht notwendig, LCPUFAs in Triacylglyceriden anzureichern. In Membranen eingebaute LCPUFAs können die physikalischen Eigenschaften der Membranen verändern und so schädliche Wirkungen auf die Integrität und Transporteigenschaften der Membranen sowie die Stresstoleranz von Pflanzen haben.

Erste transgene Pflanzen, die Gene kodierend für Enzyme der LCPUFA-Biosynthese enthalten und exprimieren und LCPUFAs produzieren wurden beispielsweise in DE 102 19 203 (Verfahren zur Herstellung mehrfach ungesättigter Fettsäuren in Pflanzen) erstmals beschrieben. Diese Pflanzen produzieren allerdings LCPUFAs in Mengen, die für eine Aufarbeitung der in den Pflanzen enthaltenen Öle noch weiter optimiert werden müssen.

Um eine Anreicherung der Nahrung und des Futters mit diesen mehrfach ungesättigten Fettsäuren zu ermöglichen, besteht daher ein großer Bedarf an einem einfachen, kostengünstigen Verfahren zur Herstellung dieser mehrfach ungesättigten Fettsäuren speziell in eukaryontischen Systemen.

Es bestand daher die Aufgabe ein Verfahren zur Herstellung von mehrfach ungesättigten Fettsäuren in einem Organismus vorteilhaft in einem eukaryontischen Organismus bevorzugt in einer Pflanze zu entwickeln. Diese Aufgabe wurde durch das erfindungsgemäße Verfahren gemäß Anspruch 1 gelöst.Das Verfahren umfasst folgende Schritte:
a) Einbringen mindestens einer Nukleinsäuresequenz in den Organismus, nämlich die Ölfruchtpflanze, mit der in SEQ ID NO: 1oder SEQ ID NO: 20 dargestellten Sequenz, die für ein Polypeptid mit einer Lysophosphatidsäure Acyltransferase-Aktivität codiert; oder
b) Einbringen mindestens einer Nukleinsäuresequenz in den Organismus, die sich als Ergebnis des degenerierten genetischen Codes von den in SEQ ID NO: 1oder SEQ ID NO: 20 enthaltenden codierenden Sequenz ableiten lässt, oder
c) Einbringen mindestens einer Nukleinsäuresequenz in den Organismus, die für Polypeptide mit mindestens 80 % Homologie auf Aminosäureebene mit SEQ ID NO: 2 oder SEQ ID NO: 21 codieren und eine Lysophosphatidsäure Acyltransferase-Aktivität aufweisen, und
d) Kultivieren und Ernten des Organismus.

Vorteilhaft enthalten die im erfindungsgemäßen Verfahren hergestellten mehrfach ungesättigten Fettsäuren mindestens zwei vorteilhaft drei, vier oder fünf Doppelbindungen. Besonders vorteilhaft enthalten die Fettsäuren vier oder fünf Doppelbindungen. Im Verfahren hergestellte Fettsäuren haben vorteilhaft 18-, 20-, 22- oder 24 C-Atome in der Fettsäurekette, bevorzugt enthalten die Fettsäuren 20, 22 oder 24 Kohlenstoffatome in der Fettsäurekette. Vorteilhaft werden gesättigte Fettsäuren mit den im Verfahren verwendeten Nukleinsäuren wenig oder gar nicht umgesetzt. Unter wenig ist zu verstehen, das im Vergleich zu mehrfach ungesättigten Fettsäuren die gesättigten Fettsäuren mit weniger als 5 % der Aktivität, vorteilhaft weniger als 3 %, besonders vorteilhaft mit weniger als 2 % umgesetzt werden. Diese hergestellten Fettsäuren können als einziges Produkt im Verfahren hergestellt werden oder in einem Fettsäuregemisch vorliegen.

Bei den im erfindungsgemäßen Verfahren verwendeten Nukleinsäuresequenzen handelt es sich um isolierte Nukleinsäuresequenzen, die für Polypeptide mit Lysophosphatidsäure Acyltransferase-Aktivität, Glycerin-3-phosphat Acyltransferase-Aktivität, Diacylglycerin Acyltransferase-Aktivität und/oder Lecithin Cholesterin Acyltransferase-Aktivität codieren.

Die im Verfahren hergestellten mehrfach ungesättigten Fettsäuren sind vorteilhaft in Membranlipiden und/oder Triacylglyceriden gebunden, können aber auch als freie Fettsäuren oder aber gebunden in Form anderer Fettsäureester in den Organismen vorkommen. Dabei können sie wie gesagt als "Reinprodukte" oder aber vorteilhaft in Form von Mischungen verschiedener Fettsäuren oder Mischungen unterschiedlicher Glyceride vorliegen. Dabei lassen sich die in den Triacylglyceriden gebundenen verschieden Fettsäuren von kurzkettigen Fettsäuren mit 4 bis 6 C-Atomen, mittelkettigen Fettsäuren mit 8 bis 12 C-Atomen oder langkettigen Fettsäuren mit 14 bis 24 C-Atomen ableiten, bevorzugt sind die langkettigen Fettsäuren besonders bevorzugt sind die langkettigen Fettsäuren LCPUFAs von C₁₈-, C₂₀-, C₂₂- und/oder C₂₄-Fettsäuren.

Im erfindungsgemäßen Verfahren werden vorteilhaft Fettsäureester mit mehrfach ungesättigten C₁₈-, C₂₀-, C₂₂- und/oder C₂₄-Fettsäuremolekülen mit mindestens zwei Doppelbindungen im Fettsäureester hergestellt. Bevorzugt enthalten diese Fettsäuremoleküle drei, vier oder fünf Doppelbindungen und führen vorteilhaft zur Synthese von Hexadecadiensäure (C16:2^{Δ9,12}), γ-Linolensäure (= GLA, C18:3^{Δ6,9,12}), Stearidonsäure (= SDA, C18:4^{Δ6,9,12,15),} Dihomo-y-Linolensäure (= DGLA, 20:3Δ^{8,11,14}), Eicosatetraensäure (= ETA, C20:4^{Δ5,8,11,14}), Arachidonsäure (ARA), Eicosapentaensäure (EPA) oder deren Mischungen, bevorzugt EPA und/oder ARA.

Die Fettsäureester mit mehrfach ungesättigten C₁₈-, C₂₀-, C₂₂-, und/oder C₂₄-Fettsäuremolekülen können aus den Organismen, die für die Herstellung der Fettsäureester verwendet wurden, in Form eines Öls oder Lipids beispielsweise in Form von Verbindungen wie Sphingolipide, Phosphoglyceride, Lipide, Glycolipide wie Glycosphingolipid, Phospholipide wie Phosphatidylethanolamin, Phosphatidylcholin, Phosphatidylserin, Phosphatidylglycerol, Phosphatidylinositol oder Diphosphatidylglycerol, Monoacylglyceride, Diacylglyceride, Triacylglyceride oder sonstige Fettsäureester wie die AcetylCoenzymA-Ester, die die mehrfach ungesättigten Fettsäuren mit mindestens zwei bevorzugt drei Doppelbindungen enthalten, isoliert werden, vorteilhaft werden sie in der Form ihrer Diacylglyceride, Triacylglyceride und/oder in Form des Phosphatidylcholin isoliert, besonders bevorzugt in der Form der Triacylglyceride. Neben diesen Estern sind die mehrfach ungesättigten Fettsäuren auch als freie Fettsäuren oder gebunden in anderen Verbindungen in den Organismen vorteilhaft den Pflanzen enthalten. In der Regel liegen die verschiedenen vorgenannten Verbindungen (Fettsäureester und frei Fettsäuren) in den Organismen in einer ungefähren Verteilung von 80 bis 90 Gew.-% Triglyceride, 2 bis 5 Gew.-% Diglyceride, 5 bis 10 Gew.-% Monoglyceride, 1 bis 5 Gew.-% freie Fettsäuren, 2 bis 8 Gew.-% Phospholipide vor, wobei sich die Summe der verschiedenen Verbindungen zu 100 Gew.-% ergänzt.

Im erfindungsgemäßen Verfahren werden die hergestellten LCPUFAs mit einem Gehalt von mindestens 3 Gew.-%, vorteilhaft von mindestens 5 Gew.-%, bevorzugt von mindestens 8 Gew.-%, besonders bevorzugt von mindestens 10 Gew.-%, ganz besonders bevorzugt von mindestens 15 Gew.-% bezogen auf die gesamten Fettsäuren in der transgenen Organismen vorteilhaft in einer transgenen Pflanze hergestellt. Vorteilhaft werden die Fettsäuren in gebundener Form hergestellt. Mit Hilfe der im erfindungsgemäßen Verfahren verwendeten Nukleinsäuren lassen sich diese ungesättigten Fettsäuren an sn1-, sn2- und/oder sn3-Position der vorteilhaft hergestellten Triglyceride bringen. Da im erfindungsgemäßen Verfahren von den Ausgangsverbindungen Hexadecadiensäure (C16:2), Linolsäure (C18:2) bzw. Linolensäure (C18:3) mehrere Reaktionsschritte durchlaufen werden, fallen die Endprodukte des Verfahrens wie beispielsweise Arachidonsäure (ARA) oder Eicosapentaensäure (EPA) nicht als absolute Reinprodukte an, es sind immer auch geringe Spuren der Vorstufen im Endprodukt enthalten. Sind in dem Ausgangsorganismus bzw. in der Ausgangspflanze beispielsweise sowohl Linolsäure als auch Linolensäure vorhanden, so liegen die Endprodukte wie ARA und EPA als Mischungen vor. Die Vorstufen sollten vorteilhaft nicht mehr als 20 Gew.-%, bevorzugt nicht mehr als 15 Gew.-%, besonders bevorzugt nicht als 10 Gew.-%, ganz besonders bevorzugt nicht mehr als 5 Gew.-% bezogen auf die Menge des jeweilige Endprodukts betragen. Vorteilhaft werden in einer transgenen Pflanze als Endprodukte nur ARA oder nur EPA im erfindungsgemäßen Verfahren gebunden oder als freie Säuren hergestellt. Werden beide Verbindungen (ARA + EPA) gleichzeitig hergestellt, werden sie vorteilhaft in einem Verhältnis von mindesten 1:2 (EPA:ARA), vorteilhaft von mindestens 1:3, bevorzugt von 1:4, besonders bevorzugt von 1:5 hergestellt.

Durch die erfindungsgemäßen Nukleinsäuresequenzen kann eine Steigerung der Ausbeute an mehrfach ungesättigten Fettsäuren von mindestens 50 %, vorteilhaft von mindestens 80 %, besonders vorteilhaft von mindestens 100 %, ganz besonders vorteilhaft von mindestens 150 % gegenüber den nicht transgenen Ausgangsorganismus beim Vergleich in der GC-Analyse siehe Beispiele erreicht werden. In einer weiteren vorteilhaften Ausführungsform kann die Ausbeute an mehrfach ungesättigten Fettsäuren um mindestens 200 %, bevorzugt um mindestens 250 %, ganz besonders bevorzugt um mindestens 300 % gesteigert werden.

Auch chemisch reine mehrfach ungesättigte Fettsäuren oder Fettsäurezusammensetzungen sind nach den vorbeschriebenen Verfahren darstellbar. Dazu werden die Fettsäuren oder die Fettsäurezusammensetzungen aus dem Organismus wie den Mikroorganismen oder den Pflanzen oder dem Kulturmedium, in dem oder auf dem die Organismen angezogen wurden, oder aus dem Organismus und dem Kulturmedium in bekannter Weise beispielsweise über Extraktion, Destillation, Kristallisation, Chromatographie oder Kombinationen dieser Methoden isoliert. Diese chemisch reinen Fettsäuren oder Fettsäurezusammensetzungen sind für Anwendungen im Bereich der Lebensmittelindustrie, der Kosmetikindustrie und besonders der Pharmaindustrie vorteilhaft.

Als Organismus für die Herstellung im erfindungsgemäßen Verfahren kommen prinzipiell alle Organismen wie Mikroorganismen, nicht-humane Tiere oder Pflanzen in Frage. Vorteilhaft werden im erfindungsgemäßen Organismen verwendet, die zu den Öl-produzierenden Organismen gehören, das heißt die für die Herstellung von Ölen verwendet werden, insbesondere Ölfruchtpflanzen, die große Mengen an Lipidverbindungen enthalten, wie Erdnuss, Raps, Canola, Sonnenblume, Safflor (Färberdistel), Mohn, Senf, Hanf, Rizinus, Olive, Sesam, Calendula, Punica, Nachtkerze, Königskerze, Distel, Wildrosen, Haselnuss, Mandel, Macadamia, Avocado, Lorbeer, Kürbis, Lein, Soja, Pistazien, Borretsch, Bäume (Ölpalme, Kokosnuss oder Walnuss) oder Feldfrüchte, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa oder Buschpflanzen (Kaffee, Kakao, Tee), Salix-Arten sowie ausdauernde Gräser und Futterfeldfrüchte. Bevorzugte erfindungsgemäße Pflanzen sind Ölfruchtpflanzen, wie Erdnuss, Raps, Canola, Sonnenblume, Safflor (Färberdistel), Mohn, Senf, Hanf, Rhizinus, Olive, Calendula, Punica, Nachtkerze, Kürbis, Lein, Soja, Borretsch, Bäume (Ölpalme, Kokosnuss). Besonders bevorzugt sind C18:2-und/oder C18:3-Fettsäure reiche Pflanzen wie Sonnenblume, Färberdistel, Tabak, Königskerze, Sesam, Baumwolle, Kürbis, Mohn, Nachtkerze, Walnuss, Lein, Hanf, Distel oder Färberdistel. Ganz besonders bevorzugt sind Pflanzen wie Färberdistel, Sonnenblume, Mohn, Nachtkerze, Walnuss, Lein oder Hanf.

Für das erfindungsgemäße beschriebene Verfahren ist es vorteilhaft in den Organismus zusätzlich zu den unter Verfahrensschritt (a) bis (d) eingebrachten Nukleinsäuren zusätzlich weitere Nukleinsäuren einzubringen, die für Enzyme des Fettsäure- oder Lipidstoffwechsels codieren.

Im Prinzip können alle Gene des Fettsäure- oder Lipidstoffwechsels vorteilhaft in Kombination mit der erfinderischen Acyl-CoA:Lysophospholipid-Acyltransferase im Verfahren zur Herstellung mehrfach ungesättigter Fettsäuren verwendet werden vorteilhaft werden Gene des Fettsäure- oder Lipidstoffwechsels ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferasen, Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) in Kombination mit der Acyl-CoA:Lysophospholipid-Acyltransferase verwendet. Besonders bevorzugt werden Gene ausgewählt aus der Gruppe der Acyl-CoA:Lysophospholipid-Acyltransferasen, Δ-4-Desaturasen, Δ-5-Desaturasen, Δ-6-Desaturasen, Δ-8-Desaturasen, Δ-9-Desaturasen, Δ-12-Desaturasen, Δ-5-Elongasen, Δ-6-Elongasen oder Δ-9-Elongasen in Kombination mit den vorgenannten Genen für die Lysophosphatidsäure Acyltransferase, Glycerin-3-phosphat Acyltransferase, Diacylglycerin Acyltransferase oder Lecithin Cholesterin Acyltransferase verwendet, wobei einzelne Gene oder mehrere Gene in Kombination verwendet werden können.

Durch die enzymatische Aktivität der im erfindungsgemäßen Verfahren verwendeten Nukleinsäuren, die für Polypeptide mit Lysophosphatidsäure Acyltransferase -Aktivität codieren, vorteilhaft in Kombination mit Nukleinsäuresequenzen, die für Polypeptide des Fettsäure- oder Lipidstoffwechsels wie der Acyl-CoA:Lysophospholipid-Acyltransferaseaktivität, der Δ-4-, Δ-5-, Δ-6-, Δ-8-Desaturase- oder Δ-5-, Δ-6-oder Δ-9-Elongaseaktivität codieren, können unterschiedlichste mehrfach ungesättigte Fettsäuren im erfindungsgemäßen Verfahren hergestellt werden. Je nach Auswahl der für das erfindungsgemäße Verfahren verwendeten Organismen wie den vorteilhaften Pflanze lassen sich Mischungen der verschiedenen mehrfach ungesättigten Fettsäuren oder einzelne mehrfach ungesättigte Fettsäuren wie EPA oder ARA in freier oder gebundener Form herstellen. Je nachdem welche Fettsäurezusammensetzung in der Ausgangspflanze vorherrscht (C18:2- oder C18:3-Fettsäuren) entstehen so Fettsäuren, die sich von C18:2-Fettsäuren ableiten, wie GLA, DGLA oder ARA oder, die sich von C18:3-Fettsäuren ableiten, wie SDA, ETA oder EPA. Liegt in der für das Verfahren verwendeten Pflanze als ungesättigte Fettsäure nur Linolsäure (= LA, C18:2^{Δ9,12}) vor, so können als Produkte des Verfahrens nur GLA, DGLA und ARA entstehen, die als freie Fettsäuren oder gebunden vorliegen können. Ist in der im Verfahren verwendeten Pflanze als ungesättigte Fettsäure nur α-Linolensäure (= ALA, C18:3^{Δ9,12,15}) beispielsweise wie in Lein, so können als Produkte des Verfahrens nur SDA, ETA und EPA entstehen, die wie oben beschrieben als freie Fettsäuren oder gebunden vorliegen können. Durch Modifikation der Aktivität der an der Synthese beteiligten Enzyme Lysophosphatidsäure Acyltransferase, Glycerin-3-phosphat Acyltransferase, Diacylglycerin Acyltransferase oder Lecithin Cholesterin Acyltransferase vorteilhaft in Kombination mit der Acyl-CoA:Lysophospholipid-Acyltransferase, Δ-5-, Δ-6-Desaturase und/oder Δ-6-Elongase, oder der Acyl-CoA:Lysophospholipid-Acyltransferase, Δ-5-, Δ-8-Desaturase und/oder Δ-9-Elongase oder in Kombination mit nur den ersten drei Gene Acyl-CoA:Lysophospholipid-Acyltransferase, Δ-6-Desaturase und/oder Δ-6-Elongase oder Acyl-CoA:Lysophospholipid-Acyltransferase, Δ-8-Desaturase und/oder Δ -9-Elongase der Synthesekette lassen sich gezielt in den vorgenannten Organismen vorteilhaft in den vorgenannten Pflanzen nur einzelne Produkte herstellten. Durch die Aktivität der Δ-6-Desaturase und Δ-6-Elongase entstehen beispielsweise GLA und DGLA bzw. SDA und ETA, je nach Ausgangspflanze und ungesättigter Fettsäure. Bevorzugt entstehen DGLA bzw. ETA oder deren Mischungen. Wird die Δ-5-Desaturase zusätzlich in die Organismen vorteilhaft in die Pflanze eingebracht, so entstehen zusätzlich ARA oder EPA. Dies gilt auch für Organismen in die vorher die Δ-8-Desaturase und Δ-9-Elongase eingebracht wurde. Vorteilhaft werden nur ARA oder EPA oder deren Mischungen synthetisiert, abhängig von der in im Organismus bzw. in der Pflanze vorliegenden Fettsäure, die als Ausgangssubstanz für die Synthese dient. Da es sich um Biosyntheseketten handelt, liegen die jeweiligen Endprodukte nicht als Reinsubstanzen in den Organismen vor. Es sind immer auch geringe Mengen der Vorläuferverbindungen im Endprodukt enthalten. Diese geringen Mengen betragen weniger als 20 Gew.-%, vorteilhaft weniger als 15 Gew.-%, besonders vorteilhaft weniger als 10 Gew.-%, ganz besonders vorteilhaft weniger als 5, 4, 3, 2 oder 1 Gew.-% bezogen auf das Endprodukt DGLA, ETA oder deren Mischungen bzw. ARA, EPA oder deren Mischungen.

Zur Steigerung der Ausbeute im beschriebenen Verfahren zur Herstellung von Ölen und/oder Triglyceriden mit einem vorteilhaft erhöhten Gehalt an mehrfach ungesättigten Fettsäuren ist es vorteilhaft die Menge an Ausgangsprodukt für die Fettsäuresynthese zu steigern, dies kann beispielsweise durch das Einbringen einer Nukleinsäure in den Organismus, die für ein Polypeptid mit Δ-12-Desaturase codiert, erreicht werden. Dies ist besonders vorteilhaft in Öl-produzierenden Organismen wie Raps, die einen hohen Ölsäuregehalt aufweisen. Da diese Organismen nur einen geringen Gehalt an Linolsäure aufweisen (Mikoklajczak et al., Journal of the American Oil Chemical Society, 38, 1961, 678 - 681) ist die Verwendung der genannten Δ-12-Desaturasen zur Herstellung des Ausgangsprodukts Linolsäure vorteilhaft.

Im erfindungsgemäßen Verfahren verwendeten Nukleinsäuren stammen vorteilhaft aus Pflanzen wie Algen wie Isochrysis oder Crypthecodinium, Algen/Diatomeen wie Phaeodactylum, Moose wie Physcomitrella oder Ceratodon oder höheren Pflanzen wie den Primulaceae wie Aleuritia, Calendula stellata, Osteospermum spinescens oder Osteospermum hyoseroides, Mikroorganismen wie Pilzen wie Aspergillus, Thraustochytrium, Phytophtora, Entomophthora, Mucor oder Mortierella, Bakterien wie Shewanella, Hefen oder Tieren wie Nematoden wie Caenorhabditis, Insekten oder dem Mensch. Vorteilhaft werden im erfindungsgemäßen Verfahren die vorgenannten Nukleinsäuresequenzen oder deren Derivat oder Homologe, die für Polypeptide codieren, die noch die enzymatische Aktivität der durch Nukleinsäuresequenzen codierten Proteine besitzen. Diese Sequenzen werden einzeln oder in Kombination mit der anspruchsgemäß für die Lysophosphatidsäure Acyltransferase codierenden Nukleinsäuresquenzen in Expressionskonstrukte cloniert und zum Einbringen und zur Expression in Organismen verwendet. Diese Expressionskonstrukte ermöglichen durch ihre Konstruktion eine vorteilhafte optimale Synthese der im erfindungsgemäßen Verfahren produzierten mehrfach ungesättigten Fettsäuren.

Bei einer bevorzugten Ausführungsform umfasst das Verfahren ferner den Schritt des Gewinnens einer Zelle oder eines ganzen Organismus, der die im Verfahren verwendeten Nukleinsäuresequenzen enthält, wobei die Zelle und/oder der Organismus mit einer erfindungsgemäßen Nukleinsäuresequenz, die für die Lysophosphatidsäure Acyltransferase (anspruchsgemäß) und gegebenenfalls Glycerin-3-phosphat Acyltransferase, Diacylglycerin Acyltransferase und/oder Lecithin Cholesterin Acyltransferase codiert, einem Genkonstrukt oder einem Vektor wie nachfolgend beschrieben, allein oder in Kombination mit weiteren Nukleinsäuresequenzen, die für Proteine des Fettsäure- oder Lipidsstoffwechsels codieren, transformiert wird. Bei einer weiteren bevorzugten Ausführungsform umfasst dieses Verfahren ferner den Schritt des Gewinnens der Feinchemikalie aus der Kultur. Bei der Kultur kann es sich beispielsweise um um eine Treibhaus- oder Feldkultur einer Pflanze handeln. Die so hergestellte Zelle oder der so hergestellte Organismus ist eine Zelle eines Öl-produzierenden Organismus, nämlicheiner Ölfruchtpflanze, wie beispielsweise Erdnuss, Raps, Canola, Lein, Hanf, Erdnuss, Soja, Safflower, Hanf, Sonnenblumen oder Borretsch.

Unter Anzucht ist beispielsweise die Kultivierung im Falle von Pflanzenzellen, -gewebe oder -organe auf oder in einem Nährmedium oder der ganzen Pflanze auf bzw. in einem Substrat beispielsweise in Hydrokultur, Blumentopferde oder auf einem Ackerboden zu verstehen.

"Transgen" bzw. "Rekombinant" im Sinne der Erfindung bedeutet bezüglich zum Beispiel einer Nukleinsäuresequenz, einer Expressionskassette (= Genkonstrukt) oder einem Vektor enthaltend die erfindungsgemäße Nukleinsäuresequenz oder einem Organismus transformiert mit den erfindungsgemäßen Nukleinsäuresequenzen, Expressionskassette oder Vektor alle solche durch gentechnische Methoden zustandegekommenen Konstruktionen, in denen sich entweder
a) die erfindungsgemäße Nukleinsäuresequenz, oder
b) eine mit der erfindungsgemäßen Nukleinsäuresequenz funktionell verknüpfte genetische Kontrollsequenz, zum Beispiel ein Promotor, oder
c) (a) und (b)
sich nicht in ihrer natürlichen, genetischen Umgebung befinden oder durch gentechnische Methoden modifiziert wurden, wobei die Modifikation beispielhaft eine Substitution, Addition, Deletion, Inversion oder Insertion eines oder mehrerer Nukleotidreste sein kann. Natürliche genetische Umgebung meint den natürlichen genomischen bzw. chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommende Expressionskassette - beispielsweise die natürlich vorkommende Kombination des natürlichen Promotors der erfindungsgemäßen Nukleinsäuresequenzen mit den entsprechenden Lysophosphatidsäure Acyltransferase-, Glycerin-3-phosphat Acyltransferase-, Diacylglycerin Acyltransferase- und/oder Lecithin Cholesterin Acyltransferase-Genen - wird zu einer transgenen Expressionskassette, wenn diese durch nicht-natürliche, synthetische ("künstliche") Verfahren wie beispielsweise einer Mutagenisierung geändert wird. Entsprechende Verfahren sind beispielsweise beschrieben in US 5,565,350 oder WO 00/15815.

Unter transgenen Organismus bzw. transgener Pflanze im Sinne der Erfindung ist wie vorgenannt zu verstehen, dass die im Verfahren verwendeten Nukleinsäuren nicht an ihrer natürlichen Stelle im Genom eines Organismus sind, dabei können die Nukleinsäuren homolog oder heterolog exprimiert werden. Transgen bedeutet aber auch wie genannt, dass die erfindungsgemäßen Nukleinsäuren an ihrem natürlichen Platz im Genom eines Organismus sind, dass jedoch die Sequenz gegenüber der natürlichen Sequenz verändert wurde und/oder das die Regulationssequenzen, der natürlichen Sequenzen verändert wurden. Bevorzugt ist unter transgen die Expression der erfindungsgemäßen Nukleinsäuren an nicht natürlicher Stelle im Genom zu verstehen, das heißt eine homologe oder bevorzugt heterologe Expression der Nukleinsäuren liegt vor. Bevorzugte transgene Organismen sind Pilze wie Mortierella, Moose wie Physcomitrella, Algen wie Cryptocodinium oder Pflanzen wie die Ölfruchtpflanzen.

Als Organismen bzw. Wirtsorganismen für die im erfindungsgemäßen Verfahren verwendeten Nukleinsäuren, die Expressionskassette oder den Vektor eignen sich prinzipiell alle Organismen, die in der Lage sind Fettsäuren speziell ungesättigte Fettsäuren zu synthetisieren bzw. für die Expression rekombinanter Gene geeignet sind. Beispielhaft seien Pflanzen wie Arabidopsis, Asteraceae wie Calendula oder Kulturpflanzen wie Soja, Erdnuss, Rizinus, Sonnenblume, Mais, Baumwolle, Flachs, Raps, Kokosnuss, Ölpalme, Färbersafflor (Carthamus tinctorius) oder Kakaobohne, Mikroorganismen wie Pilze beispielsweise die Gattung Mortierella, Thraustochytrium, Saprolegnia oder Pythium, Bakterien wie die Gattung Escherichia oder Shewanella, Hefen wie die Gattung Saccharomyces, Cyanobakterien, Ciliaten, Algen oder Protozoen wie Dinoflagellaten wie Crypthecodinium genannt. Prinzipiell geeignet sind insbesondereOrganismen, die natürlicherweise Öle in größeren Mengen synthetisieren können wie Pilze wie Mortierella alpina, Pythium insidiosum oder Pflanzen wie Soja, Raps, Kokosnuss, Ölpalme, Färbersafflor, Flachs, Hanf, Rizinus, Calendula, Erdnuss, Kakaobohne oder Sonnenblume oder Hefen wie Saccharomyces cerevisiae, besonders bevorzugt werden Soja, Flachs, Raps, Färbersafflor, Sonnenblume, Calendula, Mortierella oder Saccharomyces cerevisiae. Prinzipiell sind als Wirtsorganismen neben den vorgenannten transgenen Organismen auch transgene Tiere vorteilhaft nicht-humane Tiere geeignet beispielsweise C. elegans.

Nutzbare Wirtszellen sind weiterhin genannt in: Goeddel, Gene Expression Technology: Methods in Enzymology 185, Academic Press, San Diego, CA (1990).

Verwendbare Expressionsstämme z.B. solche, die eine geringere Proteaseaktivität aufweisen sind beschrieben in: Gottesman, S., Gene Expression Technology: Methods in Enzymology 185, Academic Press, San Diego, California (1990) 119-128.

Hierzu gehören Pflanzenzellen und bestimmte Gewebe, Organe und Teile von Pflanzen in all ihren Erscheinungsformen, wie Antheren, Fasern, Wurzelhaare, Stängel, Embryos, Kalli, Kotelydonen, Petiolen, Erntematerial, pflanzliches Gewebe, reproduktives Gewebe und Zellkulturen, das von der eigentlichen transgenen Pflanze abgeleitet ist und/oder dazu verwendet werden kann, die transgene Pflanze hervorzubringen.

Transgene Pflanzen, die die im erfindungsgemäßen Verfahren synthetisierten mehrfach ungesättigten Fettsäuren enthalten, können vorteilhaft direkt vermarktet werden ohne dass die synthetisierten Öle, Lipide oder Fettsäuren isoliert werden müssen. Unter Pflanzen im erfindungsgemäßen Verfahren sind ganze Pflanzen sowie alle Pflanzenteile, Pflanzenorgane oder Pflanzenteile wie Blatt, Stiel, Samen, Wurzel, Knollen, Antheren, Fasern, Wurzelhaare, Stängel, Embryos, Kalli, Kotelydonen, Petiolen, Erntematerial, pflanzliches Gewebe, reproduktives Gewebe, Zellkulturen, die sich von der transgenen Pflanze abgeleiten und/oder dazu verwendet werden können, die transgene Pflanze hervorzubringen. Der Samen umfasst dabei alle Samenteile wie die Samenhüllen, Epidermis- und Samenzellen, Endosperm oder Embyrogewebe. Die im erfindungsgemäßen Verfahren hergestellten Verbindungen können aber auch aus den Organismen vorteilhaft Pflanzen in Form ihrer Öle, Fett, Lipide und/oder freien Fettsäuren isoliert werden. Durch dieses Verfahren hergestellte mehrfach ungesättigten Fettsäuren lassen sich durch Ernten der Organismen entweder aus der Kultur, in der sie wachsen, oder vom Feld ernten. Dies kann über Pressen oder Extraktion der Pflanzenteile bevorzugt der Pflanzensamen erfolgen. Dabei können die Öle, Fette, Lipide und/oder freien Fettsäuren durch sogenanntes kalt schlagen oder kalt pressen ohne Zuführung von Wärme durch Pressen gewonnen werden. Damit sich die Pflanzenteile speziell die Samen leichter aufschließen lassen, werden sie vorher zerkleinert, gedämpft oder geröstet. Die so vorbehandelten Samen können anschließend gepresst werden oder mit Lösungsmittel wie warmen Hexan extrahiert werden. Anschließend wird das Lösungsmittel wieder entfernt. Im Falle von Mikroorganismen werden diese nach Ernte beispielsweise direkt ohne weitere Arbeitsschritte extrahiert oder aber nach Aufschluss über verschiedene dem Fachmann bekannte Methoden extrahiert. Auf diese Weise können mehr als 96 % der im Verfahren hergestellten Verbindungen isoliert werden. Anschließend werden die so erhaltenen Produkte weiter bearbeitet, das heißt raffiniert. Dabei werden zunächst beispielsweise die Pflanzenschleime und Trübstoffe entfernt. Die sogenannte Entschleimung kann enzymatisch oder beispielsweise chemisch/physikalisch durch Zugabe von Säure wie Phosphorsäure erfolgen. Anschließend werden die freien Fettsäuren durch Behandlung mit einer Base beispielsweise Natronlauge entfernt. Das erhaltene Produkt wird zur Entfernung der im Produkt verbliebenen Lauge mit Wasser gründlich gewaschen und getrocknet. Um die noch im Produkt enthaltenen Farbstoffe zu entfernen werden die Produkte einer Bleichung mit beispielsweise Bleicherde oder Aktivkohle unterzogen. Zum Schluss wird das Produkt noch beispielsweise mit Wasserdampf noch desodoriert.

Vorzugsweise sind die durch dieses Verfahren produzierten PUFAs bzw. LCPUFAs C₁₈-, C₂₀-, C₂₂- oder C₂₄-Fettsäuremoleküle mit mindestens zwei Doppelbindungen im Fettsäuremolekül, vorzugsweise drei, vier, fünf oder sechs Doppelbindungen. Diese C₁₈-, C₂₀-, C₂₂- oder C₂₄-Fettsäuremoleküle lassen sich aus dem Organismus in Form eines Öls, Lipids oder einer freien Fettsäure isolieren. Geeignete Organismen sind beispielsweise die vorstehend erwähnten. Bevorzugte Organismen sind transgene Pflanzen.

Erhältlich sind deshalb Öle, Lipide oder Fettsäuren oder Fraktionen davon, die durch das oben beschriebene Verfahren hergestellt worden sind, insbesondere Öl, Lipid oder eine Fettsäurezusammensetzung, die PUFAs umfassen und von transgenen Pflanzen herrühren.

Eine weitere erfindungsgemäße Ausführungsform ist die Verwendung der erfindungsgemäßen Lysophosphatidsäure-Acyltransferase zum Herstellen eines Pflanzenöls in einer Ölfruchtpflanze. Ein derartiges Öl, Lipid, oder Fettsäuren und/oder der Fettsäurezusammensetzung kann verwendet werden in Futtermitteln, Nahrungsmitteln, Kosmetika oder Pharmazeutika.

Unter dem Begriff "Öl", "Lipid" oder "Fett" wird ein Fettsäuregemisch verstanden, das ungesättigte, gesättigte, vorzugsweise veresterte Fettsäure(n) enthält. Bevorzugt ist, dass das Öl, Lipid oder Fett einen hohen Anteil an mehrfach ungesättigten freien oder vorteilhaft veresterten Fettsäure(n), insbesondere Linolsäure, γ-Linolensäure, Dihomo-γ-linolensäure, Arachidonsäure, α-Linolensäure, Stearidonsäure, Eicosatetraensäure, Eicosapentaensäure, Docosapentaensäure oder Docosahexaensäure hat. Vorzugsweise ist der Anteil an ungesättigten veresterten Fettsäuren ungefähr 30 %, mehr bevorzugt ist ein Anteil von 50 %, noch mehr bevorzugt ist ein Anteil von 60 %, 70 %, 80 % oder mehr. Zur Bestimmung kann z.B. der Anteil an Fettsäure nach Überführung der Fettsäuren in die Methylestern durch Umesterung gaschromatographisch bestimmt werden. Das Öl, Lipid oder Fett kann verschiedene andere gesättigte oder ungesättigte Fettsäuren, z.B. Calendulasäure, Palmitin-, Palmitolein-, Stearin-, Ölsäure etc., enthalten. Insbesondere kann je nach Ausgangsorganismus der Anteil der verschiedenen Fettsäuren in dem Öl oder Fett schwanken.

Bei den im Verfahren hergestellten mehrfach ungesättigte Fettsäuren mit vorteilhaft mindestens zwei Doppelbindungen enthalten, handelt es sich wie oben beschrieben beispielsweise um Sphingolipide, Phosphoglyceride, Lipide, Glycolipide, Phospholipide, Monoacylglycerin, Diacylglycerin, Triacylglycerin oder sonstige Fettsäureester.

Aus den so im erfindungsgemäßen Verfahren hergestellten mehrfach ungesättigte Fettsäuren mit vorteilhaft mindestens zwei Doppelbindungen lassen sich die enthaltenden mehrfach ungesättigten Fettsäuren beispielsweise über eine Alkalibehandlung beispielsweise wäßrige KOH oder NaOH oder saure Hydrolyse vorteilhaft in Gegenwart eines Alkohols wie Methanol oder Ethanol oder über eine enzymatische Abspaltung freisetzen und isolieren über beispielsweise Phasentrennung und anschließender Ansäuerung über z.B. H₂SO₄. Die Freisetzung der Fettsäuren kann auch direkt ohne die vorhergehend beschriebene Aufarbeitung erfolgen.

Die im Verfahren verwendeten Nukleinsäuren können nach Einbringung in einem Organismus vorteilhaft einer Pflanzenzelle bzw. Pflanze entweder auf einem separaten Plasmid liegen oder in das Genom der Wirtszelle integriert sein. Bei Integration in das Genom kann die Integration zufallsgemäß sein oder durch derartige Rekombination erfolgen, dass das native Gen durch die eingebrachte Kopie ersetzt wird, wodurch die Produktion der gewünschten Verbindung durch die Zelle moduliert wird, oder durch Verwendung eines Gens in trans, so dass das Gen mit einer funktionellen Expressionseinheit, welche mindestens eine die Expression eines Gens gewährleistende Sequenz und mindestens eine die Polyadenylierung eines funktionell transkribierten Gens gewährleistende Sequenz enthält, funktionell verbunden ist. Vorteilhaft werden die Nukleinsäuren über Multiexpressionskassetten oder Konstrukte zur multiparallelen Expression in die Organismen vorteilhaft zur multiparallelen samenspezifischen Expression von Genen in die Pflanzen gebracht.

Moose und Algen sind die einzigen bekannten Pflanzensysteme, die erhebliche Mengen an mehrfach ungesättigten Fettsäuren, wie Arachidonsäure (ARA) und/oder Eicosapentaensäure (EPA) und/oder Docosahexaensäure (DHA) herstellen. Moose enthalten PUFAs in Membranlipiden während Algen, algenverwandte Organismen und einige Pilze auch nennenswerte Mengen an PUFAs in der Triacylglycerolfraktion akkumulieren. Daher eignen sich Nukleinsäuremoleküle, die aus solchen Stämmen isoliert werden, die PUFAs auch in der Triacylglycerolfraktion akkumulieren, besonders vorteilhaft für das erfindungsgemäße Verfahren und damit zur Modifikation des Lipid- und PUFA-Produktionssystems in einem Wirt, insbesondere Pflanzen, nämlich Ölfruchtpflanzen, beispielsweise Raps, Canola, Lein, Hanf, Soja, Sonnenblumen, Borretsch. Sie sind deshalb vorteilhaft im erfindungsgemäßen Verfahren verwendbar.

Als Substrate der im erfindungsgemäßen Verfahren verwendeten Nukleinsäuren, die für Polypeptide mit Lysophosphatidsäure Acyltransferase-Aktivität, Glycerin-3-phosphat Acyltransferase-Aktivität, Diacylglycerin Acyltransferase-Aktivität oder Lecithin Cholesterin Acyltransferase-Aktivität codieren, und/oder den weiteren verwendeten Nukleinsäuren wie den Nukleinsäuren, die für Polypeptide des Fettsäure- oder Lipidstoffwechsels ausgewählt aus der Gruppe Acyl-CoA-Dehydrogenase(n), Acyl-ACP[= acyl carrier protein]-Desaturase(n), Acyl-ACP-Thioesterase(n), Fettsäure-AcylTransferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) codieren eignen sich vorteilhaft C₁₆-, C₁₈-, C₂₀- oder C₂₂-Fettsäuren. Bevorzugt werden die im Verfahren als Substrate umgesetzten Fettsäuren in Form ihere Acyl-CoA-Ester umgesetzt.

Zur Herstellung der erfindungsgemäßen langkettiger PUFAs müssen die mehrfach ungesättigten C₁₆- oder C₁₈-Fettsäuren zunächst durch die enzymatische Aktivität einer Desaturase zunächst desaturiert und anschließend über eine Elongase um mindestens zwei Kohlenstoffatome verlängert werden. Nach einer Elongationsrunde führt diese Enzymaktivität zu C₁₈- oder C₂₀-Fettsäuren, und nach zwei oder drei Elongationsrunden zu C₂₂- oder C₂₄-Fettsäuren. Die Aktivität der erfindungsgemäßen Verfahren verwendeten Desaturasen und Elongasen führt vorzugsweise zu C₁₈-, C₂₀-, C₂₂- und/oder C₂₄-Fettsäuren vorteilhaft mit mindestens zwei Doppelbindungen im Fettsäuremolekül, vorzugsweise mit drei, vier oder fünf Doppelbindungen, besonders bevorzugt zu C₂₀- und/oder C₂₂-Fettsäuren mit mindestens zwei Doppelbindungen im Fettsäuremolekül, vorzugsweise mit drei, vier oder fünf Doppelbindungen im Molekül. Nachdem eine erste Desaturierung und die Verlängerung stattgefunden hat, können weitere Desaturierungsschritte wie z.B. eine solche in Δ-5-Position erfolgen. Besonders bevorzugt als Produkte des erfindungsgemäßen Verfahrens sind Dihomo-γ-linolensäure, Arachidonsäure, Eicosapentaensäure, Docosapetaensäure und/oder Docosahesaensäure. Die C₁₈-Fettsäuren mit mindestens zwei Doppelbindungen in der Fettsäure können durch die erfindungsgemäße enzymatische Aktivität in Form der freien Fettsäure oder in Form der Ester, wie Phospholipide, Glycolipide, Sphingolipide, Phosphoglyceride, Monoacylglycerin, Diacylglycerin oder Triacylglycerin, verlängert werden.

Der bevorzugte Biosyntheseort von Fettsäuren, Ölen, Lipiden oder Fette in den vorteilhaft verwendeten Pflanzen ist beispielsweise im allgemeinen der Samen oder Zellschichten des Samens, so dass eine samenspezifische Expression der im Verfahren verwendeten Nukleinsäuren sinnvoll ist. Es ist jedoch naheliegend, dass die Biosynthese von Fettsäuren, Ölen oder Lipiden nicht auf das Samengewebe beschränkt sein muss, sondern auch in allen übrigen Teilen der Pflanze - beispielsweise in Epidermiszellen oder in den Knollen - gewebespezifisch erfolgen kann.

Werden im erfindungsgemäßen Verfahren als Organismen Mikroorganismus wie Hefen wie Saccharomyces oder Schizosaccharomyces, Pilze wie Mortierella, Aspergillus, Phytophtora, Entomophthora, Mucor oder Thraustochytrium Algen wie Isochrysis, Phaeodactylum oder Crypthecodinium verwendet, so werden diese Organismen vorteilhaft fermentativ angezogen.

Durch die Verwendung der erfindungsgemäßen Nukleinsäuren, die für eine Lysophosphatidsäure Acyltransferase codieren, können im Verfahren die hergestellten mehrfach ungesättigten Fettsäuren mindestens um 5 % , bevorzugt mindestens um 10 %, besonders bevorzugt mindestens um 20 %, ganz besonders bevorzugt um mindestens 50 % gegenüber dem Wildtyp der Organismen, die die Nukleinsäuren nicht rekombinant enthalten, erhöht werden.

Durch das erfindungsgemäße Verfahren können die hergestellten mehrfach ungesättigten Fettsäuren in den im Verfahren verwendeten Organismen prinzipiell auf zwei Arten erhöht werden. Es kann vorteilhaft der Pool an freien mehrfach ungesättigten Fettsäuren und/oder der Anteil der über das Verfahren hergestellten veresterten mehrfach ungesättigten Fettsäuren erhöht werden. Vorteilhaft wird durch das erfindungsgemäße Verfahren der Pool an veresterten mehrfach ungesättigten Fettsäuren in den transgenen Organismen erhöht.

Stickstoffquellen sind gewöhnlich organische oder anorganische Stickstoffverbindungen oder Materialien, die diese Verbindungen enthalten. Beispielhafte Stickstoffquellen umfassen Ammoniak in flüssiger Form oder Gasform oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle für die Herstellung von schwefelhaltigen Feinchemikalien, insbesondere von Methionin, können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium haltigen Salze verwendet werden.

Die im Verfahren gewonnenen Fettsäuren eignen sich auch als Ausgangsmaterial für die chemische Synthese von weiteren Wertprodukten. Sie können beispielsweise in Kombination miteinander oder allein zur Herstellung von Pharmaka, Nahrungsmittel, Tierfutter oder Kosmetika verwendet werden.

Ein weiterer erfindungsgemäßer Gegenstand sind isolierte Nukleinsäuresequenzen, die für Polypeptide mit Lysophosphatidsäure Acyltransferase-Aktivität gemäß Anspruch 8 codieren, wobei die durch die Nukleinsäuresequenzen codierten Lysophosphatidsäure Acyltransferasen spezifisch C₁₈-, C₂₀-, C₂₂- oder C₂₄-Fettsäuren mit mindestens einer Doppelbindungen im Fettsäuremolekül umsetzen und vorteilhaft letztlich in Diacylglyceride und/oder Triacylglyceride einbauen.

Erfindungsgemäße Nukleinsäuresequenzen sind nur Sequenzen gemäß Anspruch 8.

Weitere vorteilhafte isolierte Nukleinsäuresequenzen sind Sequenzen ausgewählt aus der Gruppe:
a) einer Nukleinsäuresequenz mit der in SEQ ID NO: 22, SEQ ID NO: 24 oder SEQ ID NO: 26 dargestellten Sequenz,
b) Nukleinsäuresequenzen, die sich als Ergebnis des degenerierten genetischen Codes von der in SEQ ID NO: 22, SEQ ID NO: 24 oder SEQ ID NO: 26 enthaltenden codierenden Sequenz ableiten lassen
c) Derivate der in SEQ ID NO: 22, SEQ ID NO: 24 oder SEQ ID NO:-26 dargestellten Nukleinsäuresequenz, die für Polypeptide mit der in SEQ ID NO: 23, SEQ ID NO: 25 oder SEQ ID NO: 27 dargestellten Aminosäuresequenz codieren und mindestens 40 % Homologie auf Aminosäureebene mit SEQ ID NO: 23, SEQ ID NO: 25 oder SEQ ID NO: 27 aufweisen und eine Glycerin-3-phosphat Acyltransferase-Aktivität aufweisen.

Zusätzliche vorteilhafte isolierte Nukleinsäuresequenzen sind Sequenzen ausgewählt aus der Gruppe:
a) einer Nukleinsäuresequenz mit der in SEQ ID NO: 28, SEQ ID NO: 30 oder SEQ ID NO: 32 dargestellten Sequenz,
b) Nukleinsäuresequenzen, die sich als Ergebnis des degenerierten genetischen Codes von der in SEQ ID NO: 28, SEQ ID NO: 30 oder SEQ ID NO: 32 enthaltenden codierenden Sequenz ableiten lassen
c) Derivate der in SEQ ID NO: 28, SEQ ID NO: 30 oder SEQ ID NO: 32 dargestellten Nukleinsäuresequenz, die für Polypeptide mit der in SEQ ID NO: 29, SEQ ID NO: 31 oder SEQ ID NO: 33 dargestellten Aminosäuresequenz codieren und mindestens 40 % Homologie auf Aminosäureebene mit SEQ ID NO: 29, SEQ ID NO: 31 oder SEQ ID NO: 33 aufweisen und eine Diacylglycerin Acyltransferase-Aktivität aufweisen.

Eine weitere Gruppe vorteilhafter isolierte Nukleinsäuresequenzen sind Sequenzen ausgewählt aus der Gruppe:
a) einer Nukleinsäuresequenz mit der in SEQ ID NO: 34 oder SEQ ID NO: 36 dargestellten Sequenz,
b) Nukleinsäuresequenzen, die sich als Ergebnis des degenerierten genetischen Codes von der in SEQ ID NO: 34 oder SEQ ID NO: 36 enthaltenden codierenden Sequenz ableiten lassen
c) Derivate der in SEQ ID NO: 34 oder SEQ ID NO: 36 dargestellten Nukleinsäuresequenz, die für Polypeptide mit der in SEQ ID NO: 35 oder SEQ ID NO: 37 dargestellten Aminosäuresequenz codieren und mindestens 40 % Homologie auf Aminosäureebene mit SEQ ID NO: 35 oder SEQ ID NO: 37 aufweisen und eine Lecithin Cholesterin Acyltransferase-Aktivität aufweisen.

Mit Hilfe dieser isolierten Nukleinsäuren lassen sich in LCPUFA-produzierende Organismen LCPUFAs an allen Positionen beispielsweise eines Triacylglycerins einbauen, wie die Positionsanalysen der Lipide von LCPUFA-produzierenden Organismen zeigten.

Die vorgenannten isolierten Nukleinsäuresequenzen lassen sich vorteilhaft mit den folgenden Nukleinsäuresequenzen kombinieren, die für Polypeptide mit Acyl-CoA:Lysophospholipid-Acyltransferaseaktivität codieren, ausgewählt aus der Gruppe:
a) einer Nukleinsäuresequenz mit der in SEQ ID NO: 39, SEQ ID NO: 41, SEQ ID NO: 43 oder SEQ ID NO: 45 dargestellten Sequenz,
b) Nukleinsäuresequenzen, die sich als Ergebnis des degenerierten genetischen Codes von der in SEQ ID NO: 39, SEQ ID NO: 41, SEQ ID NO: 43 oder SEQ ID NO: 45 enthaltenden codierenden Sequenz ableiten lassen
c) Derivate der in SEQ ID NO: 39, SEQ ID NO: 41, SEQ ID NO: 43 oder SEQ ID NO: 45 dargestellten Nukleinsäuresequenz, die für Polypeptide mit der in SEQ ID NO: 40, SEQ ID NO: 42, SEQ ID NO: 44 oder SEQ ID NO: 46 dargestellten Aminosäuresequenz codieren und mindestens 40 % Homologie auf Aminosäureebene mit SEQ ID NO: 40, SEQ ID NO: 42, SEQ ID NO: 44 oder SEQ ID NO: 46 aufweisen und eine Acyl-CoA:Lysophospholipid-Acyltransferase-aktivität aufweisen.

Vorteilhaft stammen alle die im erfindungsgemäßen Verfahren verwendeten Nukleinsäuresequenzen aus einem eukaryontischen Organismus.

Die im Verfahren verwendeten Nukleinsäuresequenzen, die für Proteine mit Lysophosphatidsäure Acyltransferase-Aktivität, Glycerin-3-phosphat Acyltransferase-Aktivität, Diacylglycerin Acyltransferase-Aktivität oder Lecithin Cholesterin Acyltransferase-Aktivität codieren oder für Proteine des Fettsäure- oder Lipidstoffwechsels vorteilhaft für Proteine mit Acyl-CoA:Lysophospholipid-Acyltransferase-, Δ-4-Desaturase-, Δ-5-Desaturase-, Δ-6-Desaturase-, Δ-8-Desatuase-, Δ-9-Desaturase-, Δ-12-Desaturase-, Δ-5-Elongase-, Δ-6-Elongase- oder Δ-9-Elongase-Aktivität, werden vorteilhaft allein oder bevorzugt in Kombination in einer Expressionskassette (= Nukleinsäurekonstrukt), die die Expression der Nukleinsäuren in einem Organismus vorteilhaft einer Pflanze oder einem Mikroorganismus ermöglicht, eingebracht.

Zum Einbringen werden die im Verfahren verwendeten Nukleinsäuren vorteilhaft einer Amplifikation und Ligation in bekannter Weise unterworfen. Vorzugsweise geht man in Anlehnung an das Protokoll der Pfu-DNA-Polymerase oder eines Pfu/Taq-DNA-Polymerasegemisches vor. Die Primer werden in Anlehnung an die zu amplifizierende Sequenz gewählt. Zweckmäßigerweise sollten die Primer so gewählt werden, dass das Amplifikat die gesamte kodogene Sequenz vom Start- bis zum Stop-Kodon umfasst. Im Anschluss an die Amplifikation wird das Amplifikat zweckmäßigerweise analysiert. Beispielsweise kann die Analyse nach gelelektrophoretischer Auftrennung hinsichtlich Qualität und Quantität erfolgen. Im Anschluss kann das Amplifikat nach einem Standardprotokoll gereinigt werden (z.B. Qiagen). Ein Aliquot des gereinigten Ampliikats steht dann für die nachfolgende Klonierung zur Verfügung. Geeignete Klonierungsvektoren sind dem Fachmann allgemein bekannt. Hierzu gehören insbesondere Vektoren, die in mikrobiellen Systemen replizierbar sind, also vor allem Vektoren, die eine effiziente Klonierung in Hefen oder Pilze gewährleisten, und die stabile Transformation von Pflanzen ermöglichen. Zu nennen sind insbesondere verschiedene für die T-DNA-vermittelte Transformation geeignete, binäre und co-integrierte Vektorsysteme. Derartige Vektorsysteme sind in der Regel dadurch gekennzeichnet, dass sie zumindest die für die Agrobakterium-vermittelte Transformation benötigten vir-Gene sowie die T-DNA begrenzenden Sequenzen (T-DNA-Border) beinhalten. Vorzugsweise umfassen diese Vektorsysteme auch weitere cis-regulatorische Regionen wie Promotoren und Terminatoren und/oder Selektionsmarker, mit denen entsprechend transformierte Organismen identifiziert werden können. Während bei co-integrierten Vektorsystemen vir-Gene und T-DNA-Sequenzen auf demselben Vektor angeordnet sind, basieren binäre Systeme auf wenigstens zwei Vektoren, von denen einer vir-Gene, aber keine T-DNA und ein zweiter T-DNA, jedoch kein vir-Gen trägt. Dadurch sind letztere Vektoren relativ klein, leicht zu manipulieren und sowohl in E.-coli als auch in Agrobacterium zu replizieren. Zu diesen binären Vektoren gehören Vektoren der Serien pBIB-HYG, pPZP, pBecks, pGreen. Bevorzugt verwendet werden Bin19, pB1101, pBinAR, pGPTV und pCAMBIA. Eine Übersicht über binäre Vektoren und ihre Verwendung gibt Hellens et al, Trends in Plant Science (2000) 5, 446-451. Für die Vektorpräparation können die Vektoren zunächst mit Restriktionsendonuklease(n) linearisiert und dann in geeigneter Weise enzymatisch modifiziert werden. Im Anschluss wird der Vektor gereinigt und ein Aliquot für die Klonierung eingesetzt. Bei der Klonierung wird das enzymatisch geschnittenen und erforderlichenfalls gereinigten Amplifikat mit ähnlich präparierten Vektorfragmenten mit Einsatz von Ligase kloniert. Dabei kann ein bestimmtes Nukleinsäurekonstrukt bzw. Vektor- oder Plasmidkonstrukt einen oder auch mehrere kodogene Genabschnitte aufweisen. Vorzugsweise sind die kodogenen Genabschnitte in diesen Konstrukten mit regulatorischen Sequenzen funktional verknüpft. Zu den regulatorischen Sequenzen gehören insbesondere pflanzliche Sequenzen wie die oben beschriebenen Promotoren und Terminatoren. Die Konstrukte lassen sich vorteilhafterweise in Mikroorganismen, insbesondere Escherichia coli und Agrobacterium tumefaciens, unter selektiven Bedingungen stabil propagieren und ermöglichen einen Transfer von heterologer DNA in Pflanzen oder Mikroorganismen.

Unter der vorteilhaften Verwendung von Klonierungsvektoren können die im Verfahren verwendeten Nukleinsäuren, die erfinderischen Nukleinsäuren und Nukleinsäurekonstrukte in Organismen wie Mikroorganismen oder vorteilhaft Pflanzen eingebracht werden und damit bei der Pflanzentransformation verwendet werden, wie denjenigen, die veröffentlicht sind in und dort zitiert sind: Plant Molecular Biology and Biotechnology (CRC Press, Boca Raton, Florida), Kapitel 6/7, S. 71-119 (1993); F.F. White, Vectors for Gene Transfer in Higher Plants; in: Transgenic Plants, Bd. 1, Engineering and Utilization, Hrsgb.: Kung und R. Wu, Academic Press, 1993, 15-38; B. Jenes et al., Techniques for Gene Transfer, in: Transgenic Plants, Bd. 1, Engineering and Utilization, Hrsgb.: Kung und R. Wu, Academic Press (1993), 128-143; Potrykus, Annu. Rev. Plant Physiol. Plant Molec. Biol. 42 (1991), 205-225)). Die im Verfahren verwendeten Nukleinsäuren, die erfinderischen Nukleinsäuren und Nukleinsäurekonstrukte und/oder Vektoren lassen sich damit zur gentechnologischen Veränderung eines breiten Spektrums an Organismen vorteilhaft an Pflanzen verwenden, so dass diese bessere und/oder effizientere Produzenten von PUFAs werden.

Es gibt eine Reihe von Mechanismen, durch die die Veränderung einer erfindungsemäßen Lysophosphatidsäure Acyltransferase und/oder eines Glycerin-3-phosphat Acyltransferase-, Diacylglycerin Acyltransferase- oder Lecithin Cholesterin Acyltransferase-Proteins die Ausbeute, Produktion und/oder Effizienz der Produktion einer Feinchemikalie aus einer Ölfruchtpflanze oder einem Mikroorganismus aufgrund eines veränderten Proteins direkt beeinflussen kann. Die Anzahl oder Aktivität des Lysophosphatidsäure Acyltransferase-, Glycerin-3-phosphat Acyltransferase-, Diacylglycerin Acyltransferase- oder Lecithin Cholesterin Acyltransferase-Proteins oder -Gens sowie von Genkombinationen mit z.B. Acyl-CoA:Lysophospholipid-Acyltransferasen, Desaturasen und/oder Elongasen kann erhöht sein, so dass größere Mengen der produzierten Verbindungen de novo hergestellt werden, weil den Organismen diese Aktivität und Fähigkeit zur Biosynthese vor dem Einbringen des/der entsprechenden Gens/Gene fehlte. Entsprechendes gilt für die Kombination mit weiteren Desaturasen oder Elongasen oder weiteren Enzymen aus dem Fettsäure- und Lipidstoffwechsel. Auch die Verwendung verschiedener divergenter, d.h. auf DNA-Sequenzebene unterschiedlicher Sequenzen kann dabei vorteilhaft sein bzw. die Verwendung von Promotoren zur Genexpression, die eine andere zeitliche Genexpression z.B. abhängig vom Reifegrad eines Samens oder Öl-speichernden Gewebes ermöglicht.

Durch das Einbringen eines Lysophosphatidsäure Acyltransferase-, Glycerin-3-phosphat Acyltransferase-, Diacylglycerin Acyltransferase-, Lecithin Cholesterin Acyltransferase-, Acyl-CoA:Lysophospholipid-Acyltransferase-, Desaturase- und/oder Elongase-Gens oder mehrerer Lysophosphatidsäure Acyltransferase-, Glycerin-3-phosphat Acyltransferase-, Diacylglycerin Acyltransferase-, Lecithin Cholesterin Acyltransferase-, Acyl-CoA:Lysophospholipid-Acyltransferasen-, Desaturase- und/oder Elongase-Gene in einen Organismus allein oder in Kombination mit anderen Genen in eine Zelle kann nicht nur den Biosynthesefluss zum Endprodukt erhöht, sondern auch die entsprechende Triacylglycerin-Zusammensetzung erhöht oder de novo geschaffen werden. Ebenso kann die Anzahl oder Aktivität anderer Gene, die am Import von Nährstoffen, die zur Biosynthese einer oder mehrerer Feinchemikalien (z.B. Fettsäuren, polaren und neutralen Lipiden) nötig sind, erhöht sein, so dass die Konzentration dieser Vorläufer, Cofaktoren oder Zwischenverbindungen innerhalb der Zellen oder innerhalb des Speicherkompartiments erhöht ist, wodurch die Fähigkeit der Zellen zur Produktion von PUFAs, wie im folgenden beschrieben, weiter gesteigert wird. Fettsäuren und Lipide sind selbst als Feinchemikalien wünschenswert; durch Optimierung der Aktivität oder Erhöhung der Anzahl einer oder mehrerer Lysophosphatidsäure Acyltransferase-, Glycerin-3-phosphat Acyltransferase-, Diacylglycerin Acyltransferase-, Lecithin Cholesterin Acyltransferase-, Acyl-CoA:Lysophospholipid-Acyltransferase-, Desaturase- und/oder Elongase-Gene, die an der Biosynthese dieser Verbindungen beteiligt sind, oder durch Zerstören der Aktivität einer oder mehrerer Gene, die am Abbau dieser Verbindungen beteiligt sind, kann es möglich sein, die Ausbeute, Produktion und/oder Effizienz der Produktion von Fettsäure- und Lipidmolekülen aus Organismen und vorteilhaft aus Pflanzen zu steigern.

Die Nukleinsäuremoleküle codieren für Proteine oder Teile von diesen, wobei die Proteine oder das einzelne Protein oder Teile davon eine Aminosäuresequenz enthält, die ausreichend homolog zu einer Aminosäuresequenz der Sequenz SEQ ID NO: 2, SEQ ID NO: 5, SEQ ID NO: 8, SEQ ID NO: 10, SEQ ID NO: 12, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35 oder SEQ ID NO: 37 ist, so dass das Protein oder der Teil davon eine aufweisen und eine äquivalente Lysophosphatidsäure Acyltransferase-Aktivität, Glycerin-3-phosphat Acyltransferase-Aktivität, Diacylglycerin Acyltransferase-Aktivität oder Lecithin Cholesterin Acyltransferase-Aktivität beibehält. Vorzugsweise hat das Protein oder der Teil davon, das/der von dem Nukleinsäuremolekül kodiert wird, noch seine wesentliche enzymatische Aktivität und die Fähigkeit, am Stoffwechsel von zum Aufbau von Zellmembranen oder Lipidkörperchen in Organismen vorteilhaft in Pflanzen notwendigen Verbindungen oder am Transport von Molekülen über diese Membranen teilzunehmen noch hat. Vorteilhaft ist das von den Nukleinsäuremolekülen kodierte Protein zu mindestens etwa 40 %, vorzugsweise mindestens etwa 60 % und stärker bevorzugt mindestens etwa 70 %, 80 % oder 90 % und am stärksten bevorzugt mindestens etwa 95 %, 96 %, 97 %, 98 %, 99 % oder mehr homolog zu einer Aminosäuresequenz der Sequenz SEQ ID NO: 5, SEQ ID NO: 8, SEQ ID NO: 10, SEQ ID NO: 12, SEQ ID NO: 15, SEQ ID NO: 17, SEQ ID NO: 19, SEQ ID NO: 23, SEQ ID NO: 25, SEQ ID NO: 27, SEQ ID NO: 29, SEQ ID NO: 31, SEQ ID NO: 33, SEQ ID NO: 35 oder SEQ ID NO: 37. Im Sinne der Erfindung ist unter Homologie oder homolog, Identität oder identisch zu verstehen. Das von der Nukleinsäure codierte Protein hat anspruchsgemäß zumindest 80% Identität zu der in Sequenz SEQ ID NO:2 und/oder SEQ ID NO:21, bevorzugt mindestens etwa 90%, 95 %, 96 %, 97 %, 98 %, 99 % oder mehr.

Unter wesentlicher enzymatischer Aktivität der verwendeten Lysophosphatidsäure Acyltransferasen, Glycerin-3-phosphat Acyltransferasen, Diacylglycerin Acyltransferasen oder Lecithin Cholesterin Acyltransferasen ist zu verstehen, dass sie gegenüber den durch die Sequenz mit SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 und deren Derivate codierten Proteinen/Enzymen im Vergleich noch mindestens eine enzymatische Aktivität von mindestens 10 %, bevorzugt 20 %, besonders bevorzugt 30 % und ganz besonders 40 % aufweisen und damit am Stoffwechsel von zum Aufbau von Fettsäuren, Fettsäureester wie Diacylglyceride und/oder Triacylglyceride in einem Organismus vorteilhaft einer Pflanzenzelle notwendigen Verbindungen oder am Transport von Molekülen über Membranen teilnehmen können, wobei desaturierte C18-, C20-, C22- oder C24-Kohlenstoffketten im Fettsäuremolekül mit Doppelbindungen an mindestens zwei, vorteilhaft drei, vier oder fünf Stellen gemeint sind.

Vorteilhaft im Verfahren verwendbare Nukleinsäuren stammen aus Bakterien, Pilzen oder Pflanzen wie Algen oder Moosen wie den Gattungen Shewanella, Physcomitrella, Thraustochytrium, Fusarium, Phytophtora, Ceratodon, Isochrysis, Aleurita, Muscarioides, Mortierella, Borago, Phaeodactylum, Crypthecodinium oder aus Nematoden wie Caenorhabditis, speziell aus den Gattungen und Arten Shewanella hanedai, Physcomitrella patens, Phytophtora infestans, Fusarium graminaeum, Cryptocodinium cohnii, Ceratodon purpureus, Isochrysis galbana, Aleurita farinosa, Muscarioides viallii, Mortierella alpina, Borago officinalis, Phaeodactylum tricornutum oder aus Caenorhabditis elegans.

Alternativ können die verwendeten isolierten Nukleotidsequenzen für Lysophosphatidsäure Acyltransferasen, Glycerin-3-phosphat Acyltransferasen, Diacylglycerin Acyltransferasen oder Lecithin Cholesterin Acyltransferasen codieren, die an eine Nukleotidsequenz der SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 hybridisieren, z.B. unter stringenten Bedingungen hybridisieren.

Die im Verfahren verwendeten Nukleinsäuresequenzen werden vorteilhaft in einer Expressionskassette, die die Expression der Nukleinsäuren in Organismen wie Mikroorganismen oder Pflanzen ermöglicht, eingebracht.

Dabei werden die Nukleinsäuresequenzen, die für die Lysophosphatidsäure Acyltransferasen, Glycerin-3-phosphat Acyltransferasen, Diacylglycerin Acyltransferasen oder Lecithin Cholesterin Acyltransferasen codieren sowie die Nukleinsäuresequenzen, die für die in Kombination verwendeten Acyl-CoA:Lysophospholipid-Acyltransferasen, die Desaturasen und/oder die Elongasen codieren, mit einem oder mehreren Regulationssignalen vorteilhafterweise zur Erhöhung der Genexpression funktionell verknüpft. Diese regulatorischen Sequenzen sollen die gezielte Expression der Gene und der Proteinexpression ermöglichen. Dies kann beispielsweise je nach Wirtsorganismus bedeuten, dass das Gen erst nach Induktion exprimiert und/oder überexprimiert wird, oder dass es sofort exprimiert und/oder überexprimiert wird. Beispielsweise handelt es sich bei diesen regulatorischen Sequenzen um Sequenzen an die Induktoren oder Repressoren binden und so die Expression der Nukleinsäure regulieren. Zusätzlich zu diesen neuen Regulationssequenzen oder anstelle dieser Sequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Die Expressionskassette (= Expressionskonstrukt = Genkonstrukt) kann aber auch einfacher aufgebaut sein, das heißt es wurden keine zusätzlichen Regulationssignale vor die Nukleinsäuresequenz oder dessen Derivate inseriert und der natürliche Promotor mit seiner Regulation wurde nicht entfernt. Stattdessen wurde die natürliche Regulationssequenz so mutiert, dass keine Regulation mehr erfolgt und/oder die Genexpression gesteigert wird. Diese veränderten Promotoren können in Form von Teilsequenzen (= Promotor mit Teilen der erfindungsgemäßen Nukleinsäuresequenzen) auch allein vor das natürliche Gen zur Steigerung der Aktivität gebracht werden. Das Genkonstrukt kann außerdem vorteilhafterweise auch eine oder mehrere sogenannte "enhancer Sequenzen" funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen inseriert werden wie weitere regulatorische Elemente oder Terminatoren. Die Lysophosphatidsäure Acyltransferase-, Glycerin-3-phosphat Acyltransferase-, Diacylglycerin Acyltransferase- oder Lecithin Cholesterin Acyltransferase-Gene sowie die vorteilhaft verwendeten Acyl-CoA:Lysophospholipid-Acyltransferase-, Δ-4-Desaturase-, Δ5-Desaturase-, Δ-6-Desaturase- und/oder Δ-8-Desaturase-Gene und/oder die Δ-5-Elongase-, Δ-6-Elongase- und/oder Δ-9-Elongase-Gene können in einer oder mehreren Kopien in der Expressionskassette (= Genkonstrukt) enthalten sein. Vorteilhaft liegt nur jeweils eine Kopie der Gene in der Expressionskassette vor. Dieses Genkonstrukt oder die Genkonstrukte können zusammen im Wirtsorganismus exprimiert werden. Dabei kann das Genkonstrukt oder die Genkonstrukte in einem oder mehreren Vektoren inseriert sein und frei in der Zelle vorliegen oder aber im Genom inseriert sein. Es ist vorteilhaft für die Insertion weiterer Gene im Wirtsgenom, wenn die zu exprimierenden Gene zusammen in einem Genkonstrukt vorliegen.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Angegeben sind ferner ein oder mehrere Genkonstrukte, die eine oder mehrere Sequenzen enthalten, die durch SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 oder dessen Derivate definiert sind und für Polypeptide gemäß SEQ ID NO: 2, SEQ ID NO: 5, SEQ ID NO: 8, SEQ ID NO: 10, SEQ ID NO: 12, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35 oder SEQ ID NO: 37 kodieren. Die genannten Lysophosphatidsäure Acyltransferasen, Glycerin-3-phosphat Acyltransferasen, Diacylglycerin Acyltransferasen oder Lecithin Cholesterin Acyltransferasen führen dabei vorteilhaft zu einem Austausch bzw. Einbau der Fettsäuren zwischen dem Mono-, Di- und/oder Triglyceridpool der Zelle und dem CoA-Fettsäureester-Pool, wobei das Substrat vorteilhaft ein, zwei, drei, vier oder fünf Doppelbindungen aufweist und vorteilhaft 18, 20, 22 oder 24 Kohlenstoffatome im Fettsäuremolekül aufweist. Gleiches gilt für ihre Homologen, Derivate oder Analoga, die funktionsfähig mit einem oder mehreren Regulationssignalen, vorteilhafterweise zur Steigerung der Genexpression, verbunden sind.

Vorteilhafte Regulationssequenzen für das neue Verfahren liegen beispielsweise in Promotoren vor, wie dem cos-, tac-, trp-, tet-, trp-tet-, Ipp-, lac-, Ipp-lac-, laclq-, T7-, T5-, T3-, gal-, trc-, ara-, USP6-, λ-PR- oder λ-PL-Promotor und werden vorteilhafterweise in Gram-negativen Bakterien angewendet. Weitere vorteilhafte Regulationssequenzen liegen beispielsweise in den Gram-positiven Promotoren amy und SPO2, in den Hefe- oder Pilzpromotoren ADC1, MFα, AC, P-60, CYC1, GAPDH, TEF, rp28, ADH oder in den Pflanzenpromotoren CaMV/35S [Franck et al., Cell 21 (1980) 285-294], PRP1 [Ward et al., Plant. Mol. Biol. 22 (1993)], SSU, OCS, lib4, usp, STLS1, B33, nos oder im Ubiquitin- oder Phaseolin-Promotor vor. In diesem Zusammenhang vorteilhaft sind ebenfalls induzierbare Promotoren, wie die in EP-A-O 388 186 (Benzylsulfonamid-induzierbar), Plant J. 2,1992:397-404 (Gatz et al., Tetracyclin-induzierbar), EP-A-O 335 528 (Abzisinsäure-induzierbar) oder WO 93/21334 (Ethanol- oder Cyclohexenol-induzierbar) beschriebenen Promotoren. Weitere geeignete Pflanzenpromotoren sind der Promotor von cytosolischer FBPase oder der ST-LSI-Promotor der Kartoffel (Stockhaus et al., EMBO J. 8, 1989, 2445), der Phosphoribosylpyrophosphatamidotransferase-Promotor aus Glycine max (Genbank-Zugangsnr. U87999) oder der in EP-A-O 249 676 beschriebene nodienspezifische Promotor. Besonders vorteilhafte Promotoren sind Promotoren, welche die Expression in Geweben ermöglichen, die an der Fettsäurebiosynthese beteiligt sind. Ganz besonders vorteilhaft sind samenspezifische Promotoren, wie der ausführungsgemäße USP Promotor aber auch andere Promotoren wie der LeB4-, DC3, Phaseolin- oder Napin-Promotor. Weitere besonders vorteilhafte Promotoren sind samenspezifische Promotoren, die für monokotyle oder dikotyle Pflanzen verwendet werden können und in US 5,608,152 (Napin-Promotor aus Raps), WO 98/45461 (Oleosin-Promotor aus Arobidopsis), US 5,504,200 (Phaseolin-Promotor aus Phaseolus vulgaris), WO 91/13980 (Bce4-Promotor aus Brassica), von Baeumlein et al., Plant J., 2, 2, 1992:233-239 (LeB4-Promotor aus einer Leguminose) beschrieben sind, wobei sich diese Promotoren für Dikotyledonen eignen. Die folgenden Promotoren eignen sich beispielsweise für Monokotyledonen lpt-2- oder Ipt-1-Promotor aus Gerste (WO 95/15389 und WO 95/23230), Hordein-Promotor aus Gerste und andere, in WO 99/16890 beschriebene geeignete Promotoren.

Es ist im Prinzip möglich, alle natürlichen Promotoren mit ihren Regulationssequenzen, wie die oben genannten, für das neue Verfahren zu verwenden. Es ist ebenfalls möglich und vorteilhaft, zusätzlich oder alleine synthetische Promotoren zu verwenden, besonders wenn sie eine Samen-spezifische Expression vermitteln, wie z.B. beschrieben in WO 99/16890.

Um einen besonders hohen Gehalt an PUFAs vor allem in transgenen Pflanzen zu erzielen, sollten die PUFA-Biosynthesegene vorteilhaft samenspezifisch in Ölsaaten exprimiert werden. Hierzu können Samen-spezifische Promotoren verwendet werden, bzw. solche Promotoren die im Embryo und/oder im Endosperm aktiv sind. Samenspezifische Promotoren können prinzipiell sowohl aus dikotolydonen als auch aus monokotolydonen Pflanzen isoliert werden. Im folgenden sind vorteilhafte bevorzugte Promotoren aufgeführt: USP (= unknown seed protein) und Vicilin (Vicia faba) [Bäumlein et al., Mol. Gen Genet., 1991, 225(3)], Napin (Raps) [US 5,608,152], Acyl-Carrier Protein (Raps) [US 5,315,001 und WO 92/18634], Oleosin (Arabidopsis thaliana) [WO 98/45461 und WO 93/20216], Phaseolin (Phaseolus vulgaris) [US 5,504,200], Bce4 [WO 91/13980], Leguminosen B4 (LegB4-Promotor) [Bäumlein et al., Plant J., 2,2, 1992], Lpt2 und Ipt1 (Gerste) [WO 95/15389 u. WO 95/23230], Samen-spezifische Promotoren aus Reis, Mais u. Weizen [WO 99/16890], Amy32b, Amy 6-6 und Aleurain [US 5,677,474], Bce4 (Raps) [US 5,530,149], Glycinin (Soja) [EP 571 741], Phosphoenol-Pyruvatcarboxylase (Soja) [JP 06/62870], ADR12-2 (Soja) [WO 98/08962], Isocitratlyase (Raps) [US 5,689,040] oder α-Amylase (Gerste) [EP 781 849].

Die Pflanzengenexpression lässt sich auch über einen chemisch induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Um eine stabile Integration der Biosynthesegene in die transgene Pflanze über mehrere Generation sicherzustellen, sollte jede der im Verfahren verwendeten Nukleinsäuren, die für die Lysophosphatidsäure Acyltransferase, Glycerin-3-phosphat Acyltransferase, Diacylglycerin Acyltransferase und/oder die Lecithin Cholesterin Acyltransferase, die vorteilhafte Acyl-CoA:Lysophospholipid-Acyltransferase, Δ-4-Desaturase, die Δ-5-Desaturase, die Δ-6-Desaturase, die Δ-8-Desaturase und/oder die Δ-5-Elongase, die Δ-6-Elongase und/oder die Δ-9-Elongase codieren, unter der Kontrolle eines eigenen bevorzugt eines unterschiedlichen Promotors exprimiert werden, da sich wiederholende Sequenzmotive zu Instabilität der T-DNA bzw. zu Rekombinationsereignissen führen können. Die Expressionskassette ist dabei vorteilhaft so aufgebaut, dass einem Promotor eine geeignete Schnittstelle zur Insertion der zu expremierenden Nukleinsäure folgt vorteilhaft in einem Polylinker anschließend gegebenenfalls ein Terminator hinter dem Polylinker liegt. Diese Abfolge wiederholt sich mehrfach bevorzugt drei-, vier- oder fünfmal, so dass bis zu fünf Gene in einem Konstrukt zusammengeführt werden und so zur Expression in die transgene Pflanze eingebracht werden können. Vorteilhaft wiederholt sich die Abfolge bis zu dreimal. Die Nukleinsäuresequenzen werden zur Expression über die geeignete Schnittstelle beispielsweise im Polylinker hinter den Promotor inseriert. Vorteilhaft hat jede Nukleinsäuresequenz ihren eigenen Promotor und gegebenenfalls ihren eigenen Terminator. Es ist aber auch möglich mehrere Nukleinsäuresequenzen hinter einem Promotor und ggf. vor einem Terminator zu inserieren. Dabei ist die Insertionsstelle bzw. die Abfolge der inserierten Nukleinsäuren in der Expressionskassette nicht von entscheidender Bedeutung, das heißt eine Nukleinsäuresequenz kann an erster oder letzter Stelle in der Kassette inseriert sein, ohne dass dadurch die Expression wesentlich beeinflusst wird. Es können in der Expressionskassette vorteilhaft unterschiedliche Promotoren wie beispielsweise der USP-, LegB4 oder DC3-Promotor und unterschiedliche Terminatoren verwendet werden. Es ist aber auch möglich nur einen Promotortyp in der Kassette zu verwenden. Dies kann jedoch zu unerwünschten Rekombinationsereignissen führen.

Wie oben beschrieben sollte die Transkription der eingebrachten Gene vorteilhaft durch geeignete Terminatoren am 3'-Ende der eingebrachten Biosynthesegene (hinter dem Stoppcodon) abgebrochen werden. Verwendet werden kann hier z.B. der OCS1 Terminator. Wie auch für die Promotoren, so sollten hier für jedes Gen unterschiedliche Terminatorsequenzen verwendet werden.

Das Genkonstrukt kann, wie oben beschrieben, auch weitere Gene umfassen, die in die Organismen eingebracht werden sollen. Es ist möglich und vorteilhaft, in die Wirtsorganismen Regulationsgene, wie Gene für Induktoren, Repressoren oder Enzyme, welche durch ihre Enzymaktivität in die Regulation eines oder mehrerer Gene eines Biosynthesewegs eingreifen, einzubringen und darin zu exprimieren. Diese Gene können heterologen oder homologen Ursprungs sein. Weiterhin können vorteilhaft im Nukleinsäurekonstrukt bzw. Genkonstrukt weitere Biosynthesegene des Fettsäure-oder Lipidstoffwechsels enthalten sein oder aber diese Gene können auf einem weiteren oder mehreren weiteren Nukleinsäurekonstrukten liegen. Vorteilhaft werden als Biosynthesegene des Fettsäure- oder Lipidstoffwechsels ein Gen ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) oder deren Kombinationen verwendet. Besonders vorteilhafte Nukleinsäuresequenzen sind Biosynthesegene des Fettsäure- oder Lipidstoffwechsels ausgewählt aus der Gruppe der Acyl-CoA:Lysophospholipid-Acyltransferase, Δ-4-Desaturase-, Δ-5-Desaturase-, Δ-6-Desaturase-, Δ-8-Desatuase-, Δ-9-Desaturase-, Δ-12-Desaturase-, Δ-5-Elongase-, Δ-6-Elongase- oder Δ-9-Elongase.

Dabei können die vorgenannten Nukleinsäuren bzw. Gene in Kombination mit anderen Elongasen und Desaturasen in erfindungsgemäßen Expressionskassetten kloniert werden und zur Transformation von Pflanzen mithilfe von Agrobakterium eingesetzt werden.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird. Die Expressionskassetten können prinzipiell direkt zum Einbringen in die Pflanze verwendet werden oder aber in einen Vektoren eingebracht werden.

Diese vorteilhaften Vektoren, vorzugsweise Expressionsvektoren, enthalten die im Verfahren verwendeten Nukleinsäuren, die für Lysophosphatidsäure Acyltransferasen, Glycerin-3-phosphat Acyltransferasen, Diacylglycerin Acyltransferasen oder Lecithin Cholesterin Acyltransferasen codieren, oder ein Nukleinsäurekonstrukt, die die verwendeten Nukleinsäure allein oder in Kombination mit weiteren Biosynthesegenen des Fettsäure- oder Lipidstoffwechsels wie den Acyl-CoA:Lysophospholipid-Acyltransferasen, Δ-4-Desaturase, Δ-5-Desaturase, Δ-6-Desaturase, Δ-8-Desatuase, Δ-9-Desaturase, Δ-12-Desaturase, Δ-5-Elongase, Δ-6-Elongase und/oder Δ-9-Elongase. Wie hier verwendet, betrifft der Begriff "Vektor" ein Nukleinsäuremolekül, das eine andere Nukleinsäure transportieren kann, an welche es gebunden ist. Ein Vektortyp ist ein "Plasmid", was für eine zirkuläre doppelsträngige DNA-Schleife steht, in die zusätzlichen DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (z.B. Bakterienvektoren mit bakteriellem Replikationsursprung). Andere Vektoren werden vorteilhaft beim Einbringen in die Wirtszelle in das Genom einer Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier als "Expressionsvektoren" bezeichnet. Gewöhnlich haben Expressionsvektoren, die für DNA-Rekombinationstechniken geeignet sind, die Form von Plasmiden. In der vorliegenden Beschreibung können "Plasmid" und "Vektor" austauschbar verwendet werden, da das Plasmid die am häufigsten verwendete Vektorform ist. Die Erfindung soll jedoch diese anderen Expressionsvektorformen, wie virale Vektoren, die ähnliche Funktionen ausüben, umfassen. Ferner soll der Begriff Vektor auch andere Vektoren, die dem Fachmann bekannt sind, wie Phagen, Viren, wie SV40, CMV, TMV, Transposons, IS-Elemente, Phasmide, Phagemide, Cosmide, lineare oder zirkuläre DNA, umfassen.

Die im Verfahren vorteilhaft verwendeten rekombinanten Expressionsvektoren umfassen die die unten beschriebenen Nukleinsäuren oder das oben beschriebene Genkonstrukt in einer Form, die sich zur Expression der verwendeten Nukleinsäuren in einer Wirtszelle eignen, was bedeutet, dass die rekombinanten Expressionsvektoren eine oder mehrere Regulationssequenzen, ausgewählt auf der Basis der zur Expression zu verwendenden Wirtszellen, die mit der zu exprimierenden Nukleinsäuresequenz funktionsfähig verbunden ist, umfasst. In einem rekombinanten Expressionsvektor bedeutet "funktionsfähig verbunden", dass die Nukleotidsequenz von Interesse derart an die Regulationssequenz(en) gebunden ist, dass die Expression der Nukleotidsequenz möglich ist und sie aneinander gebunden sind, so dass beide Sequenzen die vorhergesagte, der Sequenz zugeschriebene Funktion erfüllen (z.B. in einem In-vitro-Transkriptions-/Translationssystem oder in einer Wirtszelle, wenn der Vektor in die Wirtszelle eingebracht wird). Der Begriff "Regulationssequenz" soll Promotoren, Enhancer und andere Expressionskontrollelemente (z.B. Polyadenylierungssignale) umfassen. Diese Regulationssequenzen sind z.B. beschrieben in Goeddel: Gene Expression Technology: Methods in Enzymology 185, Academic Press, San Diego, CA (1990), oder siehe: Gruber und Crosby, in: Methods in Plant Molecular Biology and Biotechnolgy, CRC Press, Boca Raton, Florida, Hrsgb.: Glick und Thompson, Kapitel 7, 89-108, einschließlich der Literaturstellen darin. Regulationssequenzen umfassen solche, welche die konstitutive Expression einer Nukleotidsequenz in vielen Wirtszelltypen steuern, und solche, welche die direkte Expression der Nukleotidsequenz nur in bestimmten Wirtszellen unter bestimmten Bedingungen steuern. Der Fachmann weiß, dass die Gestaltung des Expressionsvektors von Faktoren, wie der Auswahl der zu transformierenden Wirtszelle, dem Ausmaß der Expression des gewünschten Proteins usw., abhängen kann.

Die verwendeten rekombinanten Expressionsvektoren können zur Expression von Lysophosphatidsäure Acyltransferasen, Glycerin-3-phosphat Acyltransferasen, Diacylglycerin Acyltransferasen oder Lecithin Cholesterin Acyltransferasen, Acyl-CoA:Lysophospholipid-Acyltransferasen, Desaturasen und Elongasen in prokaryotischen oder eukaryotischen Zellen gestaltet sein. Dies ist vorteilhaft, da häufig Zwischenschritte der Vektorkonstruktion der Einfachheit halber in Mikroorganismen durchgeführt werden. Beispielsweise können Lysophosphatidsäure Acyltransferase-, Glycerin-3-phosphat Acyltransferase-, Diacylglycerin Acyltransferase-, Lecithin Cholesterin Acyltransferase-, Acyl-CoA:Lysophospholipid-Acyltransferase-, Desaturase- und/oder Elongase-Gene in bakteriellen Zellen, Insektenzellen (unter Verwendung von Baculovirus-Expressionsvektoren), Hefe- und anderen Pilzzellen (siehe Romanos, M.A., et al. (1992) "Foreign gene expression in yeast: a review", Yeast 8:423-488; van den Hondel, C.A.M.J.J., et al. (1991) "Heterologous gene expression in filamentous fungi", in: More Gene Manipulations in Fungi, J.W. Bennet & L.L. Lasure, Hrsgb., S. 396-428: Academic Press: San Diego; und van den Hondel, C.A.M.J.J., & Punt, P.J. (1991) "Gene transfer systems and vector development for filamentous fungi, in: Applied Molecular Genetics of Fungi, Peberdy, J.F., et al., Hrsgb., S. 1-28, Cambridge University Press: Cambridge), Algen (Falciatore et al., 1999, Marine Biotechnology.1, 3:239-251), Ciliaten der Typen: Holotrichia, Peritrichia, Spirotrichia, Suctoria, Tetrahymena, Paramecium, Colpidium, Glaucoma, Platyophrya, Potomacus, Desaturaseudocohnilembus, Euplotes, Engelmaniella und Stylonychia, insbesondere der Gattung Stylonychia lemnae, mit Vektoren nach einem Transformationsverfahren, wie beschrieben in WO 98/01572, sowie bevorzugt in Zellen vielzelliger Pflanzen (siehe Schmidt, R. und Willmitzer, L. (1988) "High efficiency Agrobacterium tumefaciens-mediated transformation of Arabidopsis thaliana leaf and cotyledon explants" Plant Cell Rep.:583-586; Plant Molecular Biology and Biotechnology, C Press, Boca Raton, Florida, Kapitel 6/7, S.71-119 (1993); F.F. White, B. Jenes et al., Techniques for Gene Transfer, in: Transgenic Plants, Bd. 1, Engineering and Utilization, Hrsgb.: Kung und R. Wu, Academic Press (1993), 128-43; Potrykus, Annu. Rev. Plant Physiol. Plant Molec. Biol. 42 (1991), 205-225 (und darin zitierte Literaturstellen)) exprimiert werden. Geeignete Wirtszellen werden ferner erörtert in Goeddel, Gene Expression Technology: Methods in Enzymology 185, Academic Press, San Diego, CA (1990). Der rekombinante Expressionsvektor kann alternativ, zum Beispiel unter Verwendung von T7-Promotor-Regulationssequenzen und T7-Polymerase, in vitro transkribiert und translatiert werden.

Die Expression von Proteinen in Prokaryoten erfolgt meist mit Vektoren, die konstitutive oder induzierbare Promotoren enthalten, welche die Expression von Fusions- oder nicht-Fusionsproteinen steuern. Typische Fusions-Expressionsvektoren sind u.a. pGEX (Pharmacia Biotech Inc; Smith, D.B., und Johnson, K.S. (1988) Gene 67:31-40), pMAL (New England Biolabs, Beverly, MA) und pRIT5 (Pharmacia, Piscataway, NJ), bei denen Glutathion-S-Transferase (GST), Maltose E-bindendes Protein bzw. Protein A an das rekombinante Zielprotein fusioniert wird.

Beispiele für geeignete induzierbare nicht-Fusions-E. coli-Expressionsvektoren sind u.a. pTrc (Amann et al. (1988) Gene 69:301-315) und pET 11d (Studier et al., Gene Expression Technology: Methods in Enzymology 185, Academic Press, San Diego, Kalifornien (1990) 60-89). Die Zielgenexpression vom pTrc-Vektor beruht auf der Transkription durch Wirts-RNA-Polymerase von einem Hybrid-trp-lac-Fusionspromotor. Die Zielgenexpression aus dem pET 11 d-Vektor beruht auf der Transkription von einem T7-gn10-lac-Fusions-Promotor, die von einer coexprimierten viralen RNA-Polymerase (T7 gn1) vermittelt wird. Diese virale Polymerase wird von den Wirtsstämmen BL21 (DE3) oder HMS174 (DE3) von einem residenten λ-Prophagen bereitgestellt, der ein T7 gn1-Gen unter der Transkriptionskontrolle des lacUV 5-Promotors birgt.

Andere in prokaryotischen Organismen geeignete Vektoren sind dem Fachmann bekannt, diese Vektoren sind beispielsweise in E. coli pLG338, pACYC184, die pBR-Reihe, wie pBR322, die pUC-Reihe, wie pUC18 oder pUC19, die M113mp-Reihe, pKC30, pRep4, pHS1, pHS2, pPLc236, pMBL24, pLG200, pUR290, pIN-III113-B1, λgt11 or pBdCl, in Streptomyces pIJ101, plJ364, plJ702 oder plJ361, in Bacillus pUB110, pC194 oder pBD214, in Corynebacterium pSA77 oder pAJ667.

Bei einer weiteren Ausführungsform ist der Expressionsvektor ein Hefe-Expressionsvektor. Beispiele für Vektoren zur Expression in der Hefe S. cerevisiae umfassen pYeDesaturasec1 (Baldari et al. (1987) Embo J. 6:229-234), pMFa (Kurjan und Herskowitz (1982) Cell 30:933-943), pJRY88 (Schultz et al. (1987) Gene 54:113-123) sowie pYES2 (Invitrogen Corporation, San Diego, CA). Vektoren und Verfahren zur Konstruktion von Vektoren, die sich zur Verwendung in anderen Pilzen, wie den filamentösen Pilzen, eignen, umfassen diejenigen, die eingehend beschrieben sind in: van den Hondel, C.A.M.J.J., & Punt, P.J. (1991) "Gene transfer systems and vector development for filamentous fungi, in: Applied Molecular Genetics of fungi, J.F. Peberdy et al., Hrsgb., S. 1-28, Cambridge University Press: Cambridge, oder in: More Gene Manipulations in Fungi [J.W. Bennet & L.L. Lasure, Hrsgb., S. 396-428: Academic Press: San Diego]. Weitere geeignete Hefevektoren sind beispielsweise pAG-1, YEp6, YEp13 oder pEMBLYe23.

Alternativ können die Lysophosphatidsäure Acyltransferasen, Glycerin-3-phosphat Acyltransferasen, Diacylglycerin Acyltransferasen, Lecithin Cholesterin Acyltransferasen, Acyl-CoA:Lysophospholipid-Acyltransferasen, Desaturasen und/oder Elongasen in Insektenzellen unter Verwendung von Baculovirus-Expressionsvektoren exprimiert werden. Baculovirus-Vektoren, die zur Expression von Proteinen in gezüchteten Insektenzellen (z.B. Sf9-Zellen) verfügbar sind, umfassen die pAc-Reihe (Smith et al. (1983) Mol. Cell Biol.. 3:2156-2165) und die pVL-Reihe (Lucklow und Summers (1989) Virology 170:31-39).

Die oben genannten Vektoren bieten nur einen kleinen Überblick über mögliche geeignete Vektoren. Weitere Plasmide sind dem Fachmann bekannt und sind zum Beispiel beschrieben in: Cloning Vectors (Hrsgb. Pouwels, P.H., et al., Elsevier, Amsterdam-New York-Oxford, 1985, ISBN 0 444 904018). Weitere geeignete Expressionssysteme für prokaryotische und eukaryotische Zellen siehe in den Kapiteln 16 und 17 von Sambrook, J., Fritsch, E.F., und Maniatis, T., Molecular Cloning: A Laboratory Manual, 2. Auflage, Cold Spring Harbor Laboratory, Cold Spring Harbor Laboratory Press, Cold Spring Harbor, NY, 1989.

Bei einer weiteren Ausführungsform des Verfahrens können die Lysophosphatidsäure Acyltransferasen, Glycerin-3-phosphat Acyltransferasen, Diacylglycerin Acyltransferasen, Lecithin Cholesterin Acyltransferasen, Acyl-CoA:Lysophospholipid-Acyltransferasen, Desaturasen und/oder Elongasen in einzelligen Pflanzenzellen (wie Algen), siehe Falciatore et al., 1999, Marine Biotechnology 1 (3):239-251 und darin zitierte Literaturangaben, und Pflanzenzellen aus höheren Pflanzen (z.B. Spermatophyten, wie Feldfrüchten) exprimiert werden; erfindungsgemäß umfassen solche Verfahren zumindest die Merkmale eines der Ansprüche 1 bis 7. Beispiele für Pflanzen-Expressionsvektoren umfassen solche, die eingehend beschrieben sind in: Becker, D., Kemper, E., Schell, J., und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721; Vectors for Gene Transfer in Higher Plants; in: Transgenic Plants, Bd. 1, Engineering and Utilization, Hrsgb.: Kung und R. Wu, Academic Press, 1993, S. 15-38.

Eine Pflanzen-Expressionskassette enthält vorzugsweise Regulationssequenzen, welche die Genexpression in Pflanzenzellen steuern können und funktionsfähig verbunden sind, so dass jede Sequenz ihre Funktion, wie Termination der Transkription, erfüllen kann, beispielsweise Polyadenylierungssignale. Bevorzugte Polyadenylierungssignale sind diejenigen, die aus Agrobacterium tumefaciens-T-DNA stammen, wie das als Octopinsynthase bekannte Gen 3 des Ti-Plasmids pTiACH5 (Gielen et al., EMBO J. 3 (1984) 835ff.) oder funktionelle Äquivalente davon, aber auch alle anderen in Pflanzen funktionell aktiven Terminatoren sind geeignet.

Da die Pflanzengenexpression sehr oft nicht auf Transkriptionsebenen beschränkt ist, enthält eine Pflanzen-Expressionskassette vorzugsweise andere funktionsfähig verbunden Sequenzen, wie Translationsenhancer, beispielsweise die Overdrive-Sequenz, welche die 5'-untranslatierte Leader-Sequenz aus Tabakmosaikvirus, die das Protein/RNA-Verhältnis erhöht, enthält (Gallie et al., 1987, Nucl. Acids Research 15:8693-8711).

Die Pflanzengenexpression muss wie oben beschrieben funktionsfähig mit einem geeigneten Promotor verbunden sein, der die Genexpression auf rechtzeitige, zell- oder gewebespezifische Weise durchführt. Nutzbare Promotoren sind konstitutive Promotoren (Benfey et al., EMBO J. 8 (1989) 2195-2202), wie diejenigen, die von Pflanzenviren stammen, wie 35S CAMV (Franck et al., Cell 21 (1980) 285-294), 19S CaMV (siehe auch US 5352605 und WO 84/02913) oder Pflanzenpromotoren, wie der in US 4,962,028 beschriebene der kleinen Untereinheit der Rubisco.

Andere bevorzugte Sequenzen für die Verwendung zur funktionsfähigen Verbindung in Pflanzengenexpressions-Kassetten sind Targeting-Sequenzen, die zur Steuerung des Genproduktes in sein entsprechendes Zellkompartiment notwendig sind (siehe eine Übersicht in Kermode, Crit. Rev. Plant Sci. 15, 4 (1996) 285-423 und darin zitierte Literaturstellen), beispielsweise in die Vakuole, den Zellkern, alle Arten von Plastiden, wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen.

Die Pflanzengenexpression lässt sich auch wie oben beschrieben über einen chemisch induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Auch Promotoren, die auf biotische oder abiotische Stressbedingungen reagieren, sind geeignete Promotoren, beispielsweise der pathogeninduzierte PRP1-Gen-Promotor (Ward et al., Plant. Mol. Biol. 22 (1993) 361-366), der hitzeinduzierbare hsp80-Promotor aus Tomate (US 5,187,267), der kälteinduzierbare Alphaamylase-Promotor aus Kartoffel (WO 96/12814) oder der durch Wunden induzierbare pinll-Promotor (EP-A-0 375 091).

Es sind insbesondere solche Promotoren bevorzugt, welche die Genexpression in Geweben und Organen herbeiführen, in denen die Fettsäure-, Lipid- und Ölbiosynthese stattfindet, in Samenzellen, wie den Zellen des Endosperms und des sich entwickelnden Embryos. Geeignete Promotoren sind der Napingen-Promotor aus Raps (US 5,608,152), der USP-Promotor aus Vicia faba (Baeumlein et al., Mol Gen Genet, 1991, 225 (3):459-67), der Oleosin-Promotor aus Arabidopsis (WO 98/45461), der Phaseolin-Promotor aus Phaseolus vulgaris (US 5,504,200), der Bce4-Promotor aus Brassica (WO 91/13980) oder der Legumin-B4-Promotor (LeB4; Baeumlein et al., 1992, Plant Journal, 2 (2):233-9) sowie Promotoren, welche die samenspezifische Expression in Monokotyledonen-Pflanzen, wie Mais, Gerste, Weizen, Roggen, Reis usw. herbeiführen. Geeignete beachtenswerte Promotoren sind der Ipt2- oder Ipt1-Gen-Promotor aus Gerste (WO 95/15389 und WO 95/23230) oder die in WO 99/16890 beschriebenen (Promotoren aus dem Gersten-Hordein-Gen, dem Reis-Glutelin-Gen, dem Reis-Oryzin-Gen, dem Reis-Prolamin-Gen, dem Weizen-Gliadin-Gen, Weizen-Glutelin-Gen, dem Mais-Zein-Gen, dem Hafer-Glutelin-Gen, dem Sorghum-Kasirin-Gen, dem Roggen-Secalin-Gen).

Insbesondere kann die multiparallele Expression der im Verfahren verwendeten Lysophosphatidsäure Acyltransferasen, Glycerin-3-phosphat Acyltransferasen, Diacylglycerin Acyltransferasen oder Lecithin Cholesterin Acyltransferasen allein oder in Kombination mit Acyl-CoA:Lysophospholipid-Acyltransferasen, Desaturasen und/oder Elongasen gewünscht sein. Die Einführung solcher Expressionskassetten kann über eine simultane Transformation mehrerer einzelner Expressionskonstrukte erfolgen oder bevorzugt durch Kombination mehrerer Expressionskassetten auf einem Konstrukt. Auch können mehrere Vektoren mit jeweils mehreren Expressionskassetten transformiert und auf die Wirtszelle übertragen werden.

Ebenfalls besonders geeignet sind Promotoren, welche die plastidenspezifische Expression herbeiführen, da Plastiden das Kompartiment sind, in dem die Vorläufer sowie einige Endprodukte der Lipidbiosynthese synthetisiert werden. Geeignete Promotoren, wie der virale RNA-Polymerase-Promotor, sind beschrieben in WO 95/16783 und WO 97/06250, und der clpP-Promotor aus Arabidopsis, beschrieben in WO 99/46394.

Vektor-DNA lässt sich in prokaryotische oder eukaryotische Zellen über herkömmliche Transformations- oder Transfektionstechniken einbringen. Die Begriffe "Transformation" und "Transfektion", Konjugation und Transduktion, wie hier verwendet, sollen eine Vielzahl von im Stand der Technik bekannten Verfahren zum Einbringen fremder Nukleinsäure (z.B. DNA) in eine Wirtszelle, einschließlich Calciumphosphat- oder Calciumchlorid-Copräzipitation, DEAE-Dextran-vermittelte Transfektion, Lipofektion, natürliche Kompetenz, chemisch vermittelter Transfer, Elektroporation oder Teilchenbeschuss, umfassen. Geeignete Verfahren zur Transformation oder Transfektion von Wirtszellen, einschließlich Pflanzenzellen, lassen sich finden in Sambrook et al. (Molecular Cloning: A Laboratory Manual., 2. Aufl., Cold Spring Harbor Laboratory, Cold Spring Harbor Laboratory Press, Cold Spring Harbor, NY, 1989) und anderen Labor-Handbüchern, wie Methods in Molecular Biology, 1995, Bd. 44, Agrobacterium protocols, Hrsgb: Gartland und Davey, Humana Press, Totowa, New Jersey.

Wirtszellen, die im Prinzip zum Aufnehmen der erfindungsgemäßen Nukleinsäure, des erfindungsgemäßen Genproduktes oder des erfindungsgemäßen Vektors geeignet sind, sind alle prokaryotischen oder eukaryotischen Organismen. Die vorteilhafterweise verwendeten Wirtsorganismen sind Mikroorganismen, wie Pilze oder Hefen oder Pflanzenzellen vorzugsweise Pflanzen oder Teile davon. Pilze, Hefen oder Pflanzen werden vorzugsweise verwendet, besonders bevorzugt Pflanzen, ganz besonders bevorzugt Pflanzen, wie Ölfruchtpflanzen, die große Mengen an Lipidverbindungen enthalten, wie Raps, Nachtkerze, Hanf, Diestel, Erdnuss, Canola, Lein, Soja, Safflor, Sonnenblume, Borretsch, oder Pflanzen, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa, Buschpflanzen (Kaffee, Kakao, Tee), Salix-Arten, Bäume (Ölplame, Kokosnuss) sowie ausdauernde Gräser und Futterfeldfrüchte. Erfindungsgemäße Pflanzen sind Ölfruchtpflanzen, wie Soja, Erdnuß, Raps, Canola, Lein, Hanf, Nachtkerze, Sonnenblume, Safflor, Bäume (Ölpalme, Kokosnuß).

Ein weiterer Gegenstand sind wie oben beschrieben isolierte Nukleinsäuresequenzen, die für Polypeptide mit Lysophosphatidsäure Acyltransferase-Aktivität, Glycerin-3-phosphat Acyltransferase-Aktivität, Diacylglycerin Acyltransferase-Aktivität oder Lecithin Cholesterin Acyltransferase-Aktivität codieren, wobei die durch die Nukleinsäuresequenzen codierten Lysophosphatidsäure Acyltransferasen, Glycerin-3-phosphat Acyltransferasen, Diacylglycerin Acyltransferasen oder Lecithin Cholesterin Acyltransferasen spezifisch C₁₈-, C₂₀-, C₂₂- oder C₂₄-Fettsäuren mit mindestens einer Doppelbindungen im Fettsäuremolekül umsetzen.

Vorteilhafte isolierte Nukleinsäuresequenzen sind Sequenzen ausgewählt aus der Gruppe:
a) einer Nukleinsäuresequenz mit der in SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18 oder SEQ ID NO: 20 dargestellten Sequenz,
b) Nukleinsäuresequenzen, die sich als Ergebnis des degenerierten genetischen Codes von der in SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18 oder SEQ ID NO: 20 enthaltenden codierenden Sequenz ableiten lassen,
c) Derivate der in SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18 oder SEQ ID NO: 20 dargestellten Nukleinsäuresequenz, die für Polypeptide mit der in SEQ ID NO: 2, SEQ ID NO: 5, SEQ ID NO: 8, SEQ ID NO: 10, SEQ ID NO: 12, SEQ ID NO: 15, SEQ ID NO: 17, SEQ ID NO: 19 oder SEQ ID NO: 21 dargestellten Aminosäuresequenz codieren und mindestens 40 % Homologie auf Aminosäureebene mit SEQ ID NO: 2, SEQ ID NO: 5, SEQ ID NO: 8, SEQ ID NO: 10, SEQ ID NO: 12, SEQ ID NO: 15, SEQ ID NO: 17, SEQ ID NO: 19 oder SEQ ID NO: 21 aufweisen und eine Lysophosphatidsäure Acyltransferase-Aktivität aufweisen.

Weitere vorteilhafte isolierte Nukleinsäuresequenzen sind Sequenzen ausgewählt aus der Gruppe:
a) einer Nukleinsäuresequenz mit der in SEQ ID NO: 22, SEQ ID NO: 24 oder SEQ ID NO: 26 dargestellten Sequenz,
b) Nukleinsäuresequenzen, die sich als Ergebnis des degenerierten genetischen Codes von der in SEQ ID NO: 22, SEQ ID NO: 24 oder SEQ ID NO: 26 enthaltenden codierenden Sequenz ableiten lassen,
c) Derivate der in SEQ ID NO: 22, SEQ ID NO: 24 oder SEQ ID NO: 26 dargestellten Nukleinsäuresequenz, die für Polypeptide mit der in SEQ ID NO: 23, SEQ ID NO: 25 oder SEQ ID NO: 27 dargestellten Aminosäuresequenz codieren und mindestens 40 % Homologie auf Aminosäureebene mit SEQ ID NO: 23, SEQ ID NO: 25 oder SEQ ID NO: 27 aufweisen und eine Glycerin-3-phosphat Acyltransferase-Aktivität aufweisen.

Weitere vorteilhafte isolierte Nukleinsäuresequenzen sind Sequenzen ausgewählt aus der Gruppe:
a) einer Nukleinsäuresequenz mit der in SEQ ID NO: 28, SEQ ID NO: 30 oder SEQ ID NO: 32 dargestellten Sequenz,
b) Nukleinsäuresequenzen, die sich als Ergebnis des degenerierten genetischen Codes von der in SEQ ID NO: 28, SEQ ID NO: 30 oder SEQ ID NO: 32 enthaltenden codierenden Sequenz ableiten lassen
c) Derivate der in SEQ ID NO: 28, SEQ ID NO: 30 oder SEQ ID NO: 32 dargestellten Nukleinsäuresequenz, die für Polypeptide mit der in SEQ ID NO: 29, SEQ ID NO: 31 oder SEQ ID NO: 33 dargestellten Aminosäuresequenz codieren und mindestens 40 % Homologie auf Aminosäureebene mit SEQ ID NO: 29, SEQ ID NO: 31 oder SEQ ID NO: 33 aufweisen und eine Diacylglycerin Acyltransferase-Aktivität aufweisen.

Weitere vorteilhafte isolierte Nukleinsäuresequenzen sind Sequenzen ausgewählt aus der Gruppe:
a) einer Nukleinsäuresequenz mit der in SEQ ID NO: 34 oder SEQ ID NO: 36 dargestellten Sequenz,
b) Nukleinsäuresequenzen, die sich als Ergebnis des degenerierten genetischen Codes von der in SEQ ID NO: 34 oder SEQ ID NO: 36 enthaltenden codierenden Sequenz ableiten lassen
c) Derivate der in SEQ ID NO: 34 oder SEQ ID NO: 36 dargestellten Nukleinsäuresequenz, die für Polypeptide mit der in SEQ ID NO: 35 oder SEQ ID NO: 37 dargestellten Aminosäuresequenz codieren und mindestens 40 % Homologie auf Aminosäureebene mit SEQ ID NO: 35 oder SEQ ID NO: 37 aufweisen und eine Lecithin Cholesterin Acyltransferase-Aktivität aufweisen.

Die oben genannten, teilweise erfindungsgemäßen Nukleinsäuren stammen von Organismen, wie Tieren, Ciliaten, Pilzen, Pflanzen wie Algen oder Dinoflagellaten, die PUFAs synthetisieren können.

Der Begriff "Nukleinsäure(molekül)", wie hier verwendet, umfasst in einer vorteilhaften Ausführungsform zudem die am 3'- und am 5'-Ende des kodierenden Genbereichs gelegene untranslatierte Sequenz: mindestens 500, bevorzugt 200, besonders bevorzugt 100 Nukleotide der Sequenz stromaufwärts des 5'-Endes des kodierenden Bereichs und mindestens 100, bevorzugt 50, besonders bevorzugt 20 Nukleotide der Sequenz stromabwärts des 3'-Endes des kodierenden Genbereichs. Ein "isoliertes" Nukleinsäuremolekül wird von anderen Nukleinsäuremolekülen abgetrennt, die in der natürlichen Quelle der Nukleinsäure vorliegen. Eine "isolierte" Nukleinsäure hat vorzugsweise keine Sequenzen, welche die Nukleinsäure in der genomischen DNA des Organismus, aus dem die Nukleinsäure stammt, natürlicherweise flankieren (z.B. Sequenzen, die sich an den 5'- und 3'-Enden der Nukleinsäure befinden). Bei verschiedenen Ausführungsformen kann das isolierte Lysophosphatidsäure Acyltransferase-, Glycerin-3-phosphat Acyltransferase-, Diacylglycerin Acyltransferase- und/oder Lecithin Cholesterin Acyltransferasemolekül zum Beispiel weniger als etwa 5 kb, 4 kb, 3 kb, 2 kb, 1 kb, 0,5 kb oder 0,1 kb an Nukleotidsequenzen enthalten, die natürlicherweise das Nukleinsäuremolekül in der genomischen DNA der Zelle, aus der die Nukleinsäure stammt flankieren.

Die im Verfahren verwendeten Nukleinsäuremoleküle, z.B. ein Nukleinsäuremolekül mit einer Nukleotidsequenz der SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 oder eines Teils davon, kann unter Verwendung molekularbiologischer Standardtechniken und der hier bereitgestellten Sequenzinformation isoliert werden. Auch kann mithilfe von Vergleichsalgorithmen beispielsweise eine homologe Sequenz oder homologe, konservierte Sequenzbereiche auf DNA oder Aminosäureebene identifiziert werden. Diese können als Hybridisierungssonde sowie Standard-Hybridisierungstechniken (wie z.B. beschrieben in Sambrook et al., Molecular Cloning: A Laboratory Manual. 2. Aufl., Cold Spring Harbor Laboratory, Cold Spring Harbor Laboratory Press, Cold Spring Harbor, NY, 1989) zur Isolierung weiterer im Verfahren nützlicher Nukleinsäuresequenzen verwendet werden. Überdies lässt sich ein Nukleinsäuremolekül, umfassend eine vollständige Sequenz der SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 oder einen Teil davon, durch Polymerasekettenreaktion isolieren, wobei Oligonukleotidprimer, die auf der Basis dieser Sequenz oder von Teilen davon, verwendet werden (z.B. kann ein Nukleinsäuremolekül, umfassend die vollständigen Sequenz oder einen Teil davon, durch Polymerasekettenreaktion unter Verwendung von Oligonukleotidprimern isoliert werden, die auf der Basis dieser gleichen Sequenz erstellt worden sind). Zum Beispiel lässt sich mRNA aus Zellen isolieren (z.B. durch das Guanidiniumthiocyanat-Extraktionsverfahren von Chirgwin et al. (1979) Biochemistry 18:5294-5299) und cDNA mittels Reverser Transkriptase (z.B. Moloney-MLV-Reverse-Transkriptase, erhältlich von Gibco/BRL, Bethesda, MD, oder AMV-Reverse-Transkriptase, erhältlich von Seikagaku America, Inc., St.Petersburg, FL) herstellen. Synthetische Oligonukleotidprimer zur Amplifizierung mittels Polymerasekettenreaktion lassen sich auf der Basis einer der in SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 gezeigten Sequenzen oder mithilfe der in SEQ ID NO: 2, SEQ ID NO: 5, SEQ ID NO: 8, SEQ ID NO: 10, SEQ ID NO: 12, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35 oder SEQ ID NO: 37 dargestellten Aminosäuresequenzen erstellen. Eine erfindungsgemäße Nukleinsäure kann unter Verwendung von cDNA oder alternativ von genomischer DNA als Matrize und geeigneten Oligonukleotidprimern gemäß Standard-PCR-Amplifikationstechniken amplifiziert werden. Die so amplifizierte Nukleinsäure kann in einen geeigneten Vektor kloniert werden und mittels DNA-Sequenzanalyse charakterisiert werden. Oligonukleotide, die einer Desaturase-Nukleotidsequenz entsprechen, können durch Standard-Syntheseverfahren, beispielsweise mit einem automatischen DNA-Synthesegerät, hergestellt werden.

Homologe der verwendeten Lysophosphatidsäure Acyltransferase-, Glycerin-3-phosphat Acyltransferase-, Diacylglycerin Acyltransferase- oder Lecithin Cholesterin Acyltransferase-Nukleinsäuresequenzen mit der Sequenz SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 bedeutet beispielsweise allelische Varianten mit mindestens etwa 40 bis 60 %, vorzugsweise mindestens etwa 60 bis 70 %, stärker bevorzugt mindestens etwa 70 bis 80 %, 80 bis 90 % oder 90 bis 95 % und noch stärker bevorzugt mindestens etwa 95 %, 96 %, 97 %, 98 %, 99 % oder mehr Homologie zu einer in SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 gezeigten Nukleotidsequenzen oder ihren Homologen, Derivaten oder Analoga oder Teilen davon. Weiterhin sind isolierte Nukleinsäuremoleküle einer Nukleotidsequenz, die an eine der in SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 gezeigten Nukleotidsequenzen oder einen Teil davon hybridisieren, z.B. unter stringenten Bedingungen hybridisiert. Allelische Varianten umfassen insbesondere funktionelle Varianten, die sich durch Deletion, Insertion oder Substitution von Nukleotiden aus/in der in SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 dargestellten Sequenz erhalten lassen, wobei aber die Absicht ist, dass die Enzymaktivität der davon herrührenden synthetisierten Proteine für die Insertion eines oder mehrerer Gene vorteilhafterweise beibehalten wird. Proteine, die noch die enzymatische Aktivität der Lysophosphatidsäure Acyltransferase, Glycerin-3-phosphat Acyltransferase, Diacylglycerin Acyltransferase oder Lecithin Cholesterin Acyltransferase besitzen, das heißt deren Aktivität im wesentlichen nicht reduziert ist, bedeutet Proteine mit mindestens 10 %, vorzugsweise 20 %, besonders bevorzugt 30 %, ganz besonders bevorzugt 40 % der ursprünglichen Enzymaktivität, verglichen mit dem durch SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 kodierten Protein.

Homologen der SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 bedeuten beispielsweise auch bakterielle, Pilz- und Pflanzenhomologen, verkürzte Sequenzen, einzelsträngige DNA oder RNA der kodierenden und nicht-kodierenden DNA-Sequenz.

Homologen der SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 bedeutet auch Derivate, wie beispielsweise Promotorvarianten. Die Promotoren stromaufwärts der angegebenen Nukleotidsequenzen können durch einen oder mehrere Nukleotidaustausche, durch Insertion(en) und/oder Deletion(en) modifiziert werden, ohne dass jedoch die Funktionalität oder Aktivität der Promotoren gestört wird. Es ist weiterhin möglich, dass die Aktivität der Promotoren durch Modifikation ihrer Sequenz erhöht ist oder dass sie vollständig durch aktivere Promotoren, sogar aus heterologen Organismen, ersetzt werden.

Die vorgenannten Nukleinsäuren und Proteinmoleküle mit Lysophosphatidsäure Acyltransferase-Aktivität, Glycerin-3-phosphat Acyltransferase-Aktivität, Diacylglycerin Acyltransferase-Aktivität oder Lecithin Cholesterin Acyltransferase-Aktivität, die am Stoffwechsel von Lipiden und Fettsäuren, PUFA-Cofaktoren und Enzymen oder am Transport lipophiler Verbindungen über Membranen beteiligt sind, werden im erfindungsgemäßen Verfahren zur Modulation der Produktion von PUFAs in transgenen Organismen verwendet, entweder direkt (z.B. wenn die Überexpression oder Optimierung eines Fettsäurebiosynthese-Proteins einen direkten Einfluss auf die Ausbeute, Produktion und/oder Effizienz der Produktion der Fettsäure aus modifizierten Organismen hat) verwendet und/oder können eine indirekt Auswirkung haben, die dennoch zu einer Steigerung der Ausbeute, Produktion und/oder Effizienz der Produktion der PUFAs oder einer Abnahme unerwünschter Verbindungen führt (z.B. wenn die Modulation des Stoffwechsels von Lipiden und Fettsäuren, Cofaktoren und Enzymen zu Veränderungen der Ausbeute, Produktion und/oder Effizienz der Produktion oder der Zusammensetzung der gewünschten Verbindungen innerhalb der Zellen führt, was wiederum die Produktion einer oder mehrerer Fettsäuren beeinflussen kann).

Die Kombination verschiedener Vorläufermoleküle und Biosyntheseenzyme führt zur Herstellung verschiedener Fettsäuremoleküle, was eine entscheidende Auswirkung auf die Zusammensetzung der Lipide hat. Da mehrfach ungesättigte Fettsäuren (= PUFAs) nicht nur einfach in Triacylglycerin sondern auch in Membranlipide eingebaut werden.

Die Lipidsynthese lässt sich in zwei Abschnitte unterteilen: die Synthese von Fettsäuren und ihre Bindung an sn-Glycerin-3-Phosphat sowie die Addition oder Modifikation einer polaren Kopfgruppe. Übliche Lipide, die in Membranen verwendet werden, umfassen Phospholipide, Glycolipide, Sphingolipide und Phosphoglyceride. Die Fettsäuresynthese beginnt mit der Umwandlung von Acetyl-CoA in Malonyl-CoA durch die Acetyl-CoA-Carboxylase oder in Acetyl-ACP durch die Acetyltransacylase. Nach einer Kondensationsreaktion bilden diese beiden Produktmoleküle zusammen Acetoacetyl-ACP, das über eine Reihe von Kondensations-, Reduktions- und Dehydratisierungsreaktionen umgewandelt wird, so dass ein gesättigtes Fettsäuremolekül mit der gewünschten Kettenlänge erhalten wird. Die Produktion der ungesättigten Fettsäuren aus diesen Molekülen wird durch spezifische Desaturasen katalysiert, und zwar entweder aerob mittels molekularem Sauerstoff oder anaerob (bezüglich der Fettsäuresynthese in Mikroorganismen siehe F.C. Neidhardt et al. (1996) E. coli und Salmonella. ASM Press: Washington, D.C., S. 612-636 und darin enthaltene Literaturstellen; Lengeler et al. (Hrsgb.) (1999) Biology of Procaryotes. Thieme: Stuttgart, New York, und die enthaltene Literaturstellen, sowie Magnuson, K., et al. (1993) Microbiological Reviews 57:522-542 und die enthaltenen Literaturstellen). Die so hergestellten an Phospholipide gebundenen Fettsäuren müssen anschließend wieder für die weitere Elongationen aus den Phospholipiden in den FettsäureCoA-Ester-Pool überführt werden. Dies ermöglichen Acyl-CoA:Lysophospholipid-Acyltransferasen. Weiterhin können diese Enzyme die elongierten Fettsäuren wieder von den CoA-Estern auf die Phospholipide übertragen. Diese Reaktionsabfolge kann gegebenenfalls mehrfach durchlaufen werden.

Vorläufer für die PUFA-Biosynthese sind beispielsweise Ölsäure, Linol- und Linolensäure. Diese C₁₈-Kohlenstoff-Fettsäuren müssen auf C₂₀ und C₂₂ verlängert werden, damit Fettsäuren vom Eicosa- und Docosa-Kettentyp erhalten werden. Mithilfe der im Verfahren verwendeten Lysophosphatidsäure Acyltransferasen, Glycerin-3-phosphat Acyltransferasen, Diacylglycerin Acyltransferasen, Lecithin Cholesterin Acyltransferasen, vorteilhaft in Kombination mit Acyl-CoA:Lysophospholipid-Acyltransferasen, Desaturasen wie der Δ-4-, Δ-5-, Δ-6- und Δ-8-Desaturasen und/oder der Δ-5-, Δ-6-, Δ-9-Elongase können Arachidonsäure, Eicosapentaensäure, Docosapentaensäure oder Docosahexaensäure sowie verschiedene andere langkettige PUFAs erhalten, extrahiert und für verschiedene Zwecke bei Nahrungsmittel-, Futter-, Kosmetik- oder pharmazeutischen Anwendungen verwendet werden. Mit den genannten Enzymen können vorzugsweise C₁₈-, C₂₀-, C₂₂- und/oder C₂₄-Fettsäuren mit mindestens zwei vorteilhaft mindestens drei, vier, fünf oder sechs Doppelbindungen im Fettsäuremolekül, vorzugsweise zu C₂₀-, C₂₂- und/oder C₂₄-Fettsäuren mit vorteilhaft drei, vier oder fünf Doppelbindungen im Fettsäuremolekül hergestellt werden. Die Desaturierung kann vor oder nach Elongation der entsprechenden Fettsäure erfolgen. Daher führen die Produkte der Desaturaseaktivitäten und der möglichen weiteren Desaturierung und Elongation zu bevorzugten PUFAs mit höherem Desaturierungsgrad, einschließlich einer weiteren Elongation von C₂₀ zu C₂₂-Fettsäuren,zu Fettsäuren wie γ-Linolensäure, Dihomo-γ-linolensäure, Arachidonsäure, Stearidonsäure, Eicosatetraensäure oder Eicosapentaensäure. Substrate der Lysophosphatidsäure Acyltransferasen, Glycerin-3-phosphat Acyltransferasen, Diacylglycerin Acyltransferasen oder Lecithin Cholesterin Acyltransferasen im erfindungsgemäßen Verfahren sind C₁₈-, C₂₀- oder C₂₂-Fettsäuren wie zum Beispiel Linolsäure, γ-Linolensäure, α-Linolensäure, Dihomo-γ-linolensäure, Eicosatetraensäure oder Stearidonsäure. Bevorzugte Substrate sind Linolsäure, γ-Linolensäure und/oder α-Linolensäure, Dihomo-γ-linolensäure bzw. Arachidonsäure, Eicosatetraensäure oder Eicosapentaensäure. Die C₁₈-, C₂₀- oder C₂₂-Fettsäuren mit mindestens zwei Doppelbindungen in der Fettsäure fallen im erfindungsgemäßen Verfahren in Form der freien Fettsäure oder in Form ihrer Ester beispielsweise in Form ihrer Glyceride an.

Unter dem Begriff "Glycerid" wird ein mit ein, zwei oder drei Carbonsäureresten verestertes Glycerin verstanden (Mono-, Di- oder Triglycerid). Unter "Glycerid" wird auch ein Gemisch an verschiedenen Glyceriden verstanden. Das Glycerid oder das Gylceridgemisch kann weitere Zusätze, z.B. freie Fettsäuren, Antioxidantien, Proteine, Kohlenhydrate, Vitamine und/oder andere Substanzen enthalten.

Unter einem "Glycerid" im Sinne des erfindungsgemäßen Verfahrens werden ferner vom Glycerin abgeleitete Derivate verstanden. Dazu zählen neben den oben beschriebenen Fettsäureglyceriden auch Glycerophospholipide und Glyceroglycolipide. Bevorzugt seien hier die Glycerophospholipide wie Lecithin (Phosphatidylcholin), Cardiolipin, Phosphatidylglycerin, Phosphatidylserin und Alkylacylglycerophospholipide beispielhaft genannt.

Ferner müssen Fettsäuren anschließend an verschiedene Modifikationsorte transportiert und in das Triacylglycerin-Speicherlipid eingebaut werden. Ein weiterer wichtiger Schritt bei der Lipidsynthese ist der Transfer von Fettsäuren auf die polaren Kopfgruppen, beispielsweise durch Glycerin-Fettsäure-Acyltransferase (s. Frentzen, 1998, Lipid, 100(4-5):161-166).

Veröffentlichungen über die Pflanzen-Fettsäurebiosynthese, Desaturierung, den Lipidstoffwechsel und Membrantransport von fetthaltigen Verbindungen, die Beta-oxidation, Fettsäuremodifikation und Cofaktoren, Triacylglycerin-Speicherung und -Assemblierung einschließlich der Literaturstellen darin siehe in den folgenden Artikeln: Kinney, 1997, Genetic Engeneering, Hrsgb.: JK Setlow, 19:149-166; Ohlrogge und Browse, 1995, Plant Cell 7:957-970; Shanklin und Cahoon, 1998, Annu. Rev. Plant Physiol. Plant Mol. Biol. 49:611-641; Voelker, 1996, Genetic Engeneering, Hrsgb.: JK Setlow, 18:111-13; Gerhardt, 1992, Prog. Lipid R. 31:397-417; Gühnemann-Schäfer & Kindl, 1995, Biochim. Biophys Acta 1256:181-186; Kunau et al., 1995, Prog. Lipid Res. 34:267-342; Stymne et al., 1993, in: Biochemistry and Molecular Biology of Membrane and Storage Lipids of Plants, Hrsgb.: Murata und Somerville, Rockville, American Society of Plant Physiologists, 150-158, Murphy & Ross 1998, Plant Journal. 13(1):1-16.

Die im Verfahren hergestellten PUFAs, umfassen eine Gruppe von Molekülen, die höhere Tiere nicht mehr synthetisieren können und somit aufnehmen müssen oder die höhere Tiere nicht mehr ausreichend selbst herstellen können und somit zusätzlich aufnehmen müssen, obwohl sie leicht von anderen Organismen, wie Bakterien, synthetisiert werden, beispielsweise können Katzen Arachidonsäure nicht mehr synthetisieren.

Der Begriff "Lysophosphatidsäure Acyltransferase, Glycerin-3-phosphat Acyltransferase, Diacylglycerin Acyltransferase oder Lecithin Cholesterin Acyltransferase" im Sinne der Erfindung umfasst Proteine, die an der Biosynthese von Fettsäuren beteiligt sind. Unter Phospholipiden im Sinne der Erfindung sind zu verstehen Phosphatidylcholin, Phosphatidylethanolamin, Phosphatidylserin, Phosphatidylglycerin, und/oder Phosphatidylinositol vorteilhafterweise Phosphatidylcholin. Die Begriffe Lysophosphatidsäure Acyltransferase-, Glycerin-3-phosphat Acyltransferase-, Diacylglycerin Acyltransferase- oder Lecithin Cholesterin Acyltransferase-Nukleinsäuresequenz(en) umfassen Nukleinsäuresequenzen, die eine Lysophosphatidsäure Acyltransferase, Glycerin-3-phosphat Acyltransferase, Diacylglycerin Acyltransferase oder Lecithin Cholesterin Acyltransferase kodieren und bei denen ein Teil eine kodierende Region und ebenfalls entsprechende 5'- und 3'-untranslatierte Sequenzbereiche sein können. Die Begriffe Produktion oder Produktivität sind im Fachgebiet bekannt und beinhalten die Konzentration des Fermentationsproduktes (Verbindungen der Formel I), das in einer bestimmten Zeitspanne und einem bestimmten Fermentationsvolumen gebildet wird (z.B. kg Produkt pro Stunde pro Liter). Der Begriff Effizienz der Produktion umfasst die Zeit, die zur Erzielung einer bestimmten Produktionsmenge nötig ist (z.B. wie lange die Zelle zur Aufrichtung einer bestimmten Durchsatzrate einer Feinchemikalie benötigt). Der Begriff Ausbeute oder Produkt/Kohlenstoff-Ausbeute ist im Fachgebiet bekannt und umfasst die Effizienz der Umwandlung der Kohlenstoffquelle in das Produkt (d.h. die Feinchemikalie). Dies wird gewöhnlich beispielsweise ausgedrückt als kg Produkt pro kg Kohlenstoffquelle. Durch Erhöhen der Ausbeute oder Produktion der Verbindung wird die Menge der gewonnenen Moleküle oder der geeigneten gewonnenen Moleküle dieser Verbindung in einer bestimmten Kulturmenge über einen festgelegten Zeitraum erhöht. Die Begriffe Biosynthese oder Biosyntheseweg sind im Fachgebiet bekannt und umfassen die Synthese einer Verbindung, vorzugsweise einer organischen Verbindung, durch eine Zelle aus Zwischenverbindungen, beispielsweise in einem Mehrschritt- und stark regulierten Prozess. Die Begriffe Abbau oder Abbauweg sind im Fachgebiet bekannt und umfassen die Spaltung einer Verbindung, vorzugsweise einer organischen Verbindung, durch eine Zelle in Abbauprodukte (allgemeiner gesagt, kleinere oder weniger komplexe Moleküle) beispielsweise in einem Mehrschritt- und stark regulierten Prozess. Der Begriff Stoffwechsel ist im Fachgebiet bekannt und umfasst die Gesamtheit der biochemischen Reaktionen, die in einem Organismus stattfinden. Der Stoffwechsel einer bestimmten Verbindung (z.B. der Stoffwechsel einer Fettsäure) umfasst dann die Gesamtheit der Biosynthese-, Modifikations- und Abbauwege dieser Verbindung in der Zelle, die diese Verbindung betreffen.

Bei einer weiteren Ausführungsform kodieren Derivate des Nukleinsäuremoleküls wieder gegeben in SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 Proteine mit mindestens 40 %, vorteilhaft etwa 50 bis 60 %, vorzugsweise mindestens etwa 60 bis 70 % und stärker bevorzugt mindestens etwa 70 bis 80 %, 80 bis 90 %, 90 bis 95 % und am stärksten bevorzugt mindestens etwa 96 %, 97 %, 98 %, 99 % oder mehr Homologie (= Identität) zu einer vollständigen Aminosäuresequenz der SEQ ID NO: 2, SEQ ID NO: 5, SEQ ID NO: 8, SEQ ID NO: 10, SEQ ID NO: 12, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35 oder SEQ ID NO: 37. Anspruchsgemäß sind nur die in Anspruch 8c angegebenen Nukleinsäurederivate, die übrigen hier dargestellten Derivate dienen nur der prinzipiellen Beschreibung. Die Homologie wurde über den gesamten Aminosäure- bzw. Nukleinsäuresequenzbereich berechnet. Für die Sequenzvergleiche wurde das Programm PileUp verwendet (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970) und Smith and Waterman (Adv. Appl. Math. 2; 482-489 (1981)], die im GCG Software-Packet [Genetics Computer Group, 575 Science Drive, Madison, Wisconsin, USA 53711 (1991)] enthalten sind. Die oben in Prozent angegebenen Sequenzhomologiewerte wurden mit dem Programm BestFit über den gesamten Sequenzbereich mit folgenden Einstellungen ermittelt: Gap Weight: 8, Length Weight: 2.

Hier beschrieben sind zudem Nukleinsäuremoleküle, die sich von einer der in SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 gezeigten Nukleotidsequenzen (und Teilen davon) aufgrund des degenerierten genetischen Codes unterscheiden und somit die gleiche Lysophosphatidsäure Acyltransferase, Glycerin-3-phosphat Acyltransferase, Diacylglycerin Acyltransferase oder Lecithin Cholesterin Acyltransferase codieren wie diejenige, die von den in SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 gezeigten Nukleotidsequenzen kodiert wird.

Zusätzlich zu den in SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 gezeigten Lysophosphatidsäure Acyltransferase-, Glycerin-3-phosphat Acyltransferase-, Diacylglycerin Acyltransferase- oder Lecithin Cholesterin Acyltransferase-Nukleotidsequenzen erkennt der Fachmann, dass DNA-Sequenzpolymorphismen, die zu Änderungen in den Aminosäuresequenzen der Lysophosphatidsäure Acyltransferase, Glycerin-3-phosphat Acyltransferase, Diacylglycerin Acyltransferase oder Lecithin Cholesterin Acyltransferase führen, innerhalb einer Population existieren können. Diese genetischen Polymorphismen im Lysophosphatidsäure Acyltransferase-, Glycerin-3-phosphat Acyltransferase-, Diacylglycerin Acyltransferase- oder Lecithin Cholesterin Acyltransferase-Gen können zwischen Individuen innerhalb einer Population aufgrund von natürlicher Variation existieren. Diese natürlichen Varianten bewirken üblicherweise eine Varianz von 1 bis 5 % in der Nukleotidsequenz des Lysophosphatidsäure Acyltransferase-, Glycerin-3-phosphat Acyltransferase-, Diacylglycerin Acyltransferase- oder Lecithin Cholesterin Acyltransferase-Gens. Sämtliche und alle dieser Nukleotidvariationen und daraus resultierende Aminosäurepolymorphismen in der Lysophosphatidsäure Acyltransferase, Glycerin-3-phosphat Acyltransferase, Diacylglycerin Acyltransferase oder Lecithin Cholesterin Acyltransferase, die das Ergebnis natürlicher Variation sind und die funktionelle Aktivität von nicht verändern.

Für das erfindungsgemäße Verfahren vorteilhafte Nukleinsäuremoleküle können auf der Grundlage ihrer Homologie zu den hier offenbarten Lysophosphatidsäure Acyltransferase-, Glycerin-3-phosphat Acyltransferase-, Diacylglycerin Acyltrans ferase- oder Lecithin Cholesterin Acyltransferase-Nukleinsäuren unter Verwendung der Sequenzen oder eines Teils davon als Hybridisierungssonde gemäß Standard-Hybridisierungstechniken unter stringenten Hybridisierungsbedingungen isoliert werden. Dabei können beispielsweise isolierte Nukleinsäuremoleküle verwendet werden, die mindestens 15 Nukleotide lang sind und unter stringenten Bedingungen mit dem Nukleinsäuremolekülen, die eine Nukleotidsequenz der SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 umfassen, hybridisieren. Es können auch Nukleinsäuren mindestens 25, 50, 100, 250 oder mehr Nukleotide verwendet werden. Der Begriff "hybridisiert unter stringenten Bedingungen", wie hier verwendet, soll Hybridisierungs- und Waschbedingungen beschreiben, unter denen Nukleotidsequenzen, die mindestens 60 % homolog zueinander sind, gewöhnlich aneinander hybridisiert bleiben. Die Bedingungen sind vorzugsweise derart, dass Sequenzen, die mindestens etwa 65 %, stärker bevorzugt mindestens etwa 70 % und noch stärker bevorzugt mindestens etwa 75 % oder stärker zueinander homolog sind, gewöhnlich aneinander hybridisiert bleiben. Diese stringenten Bedingungen sind dem Fachmann bekannt und lassen sich in Current Protocols in Molecular Biology, John Wiley & Sons, N. Y. (1989), 6.3.1-6.3.6., finden. Ein bevorzugtes, nicht einschränkendes Beispiel für stringente Hybridisierungsbedingungen sind Hybridisierungen in 6 x Natriumchlorid/Natriumcitrat (sodium chloride/sodiumcitrate = SSC) bei etwa 45°C, gefolgt von einem oder mehreren Waschschritten in 0,2 x SSC, 0,1 % SDS bei 50 bis 65°C. Dem Fachmann ist bekannt, dass diese Hybridisierungsbedingungen sich je nach dem Typ der Nukleinsäure und, wenn beispielsweise organische Lösungsmittel vorliegen, hinsichtlich der Temperatur und der Konzentration des Puffers unterscheiden. Die Temperatur unterscheidet sich beispielsweise unter "Standard-Hybridisierungsbedingungen" je nach dem Typ der Nukleinsäure zwischen 42°C und 58°C in wässrigem Puffer mit einer Konzentration von 0,1 bis 5 x SSC (pH 7,2). Falls organisches Lösungsmittel im obengenannten Puffer vorliegt, zum Beispiel 50 % Formamid, ist die Temperatur unter Standardbedingungen etwa 42°C. Vorzugsweise sind die Hybridisierungsbedingungen für DNA:DNA-Hybride zum Beispiel 0,1 x SSC und 20°C bis 45°C, vorzugsweise zwischen 30°C und 45°C. Vorzugsweise sind die Hybridisierungsbedingungen für DNA:RNA-Hybride zum Beispiel 0,1 x SSC und 30°C bis 55°C, vorzugsweise zwischen 45°C und 55°C. Die vorstehend genannten Hybridisierungstemperaturen sind beispielsweise für eine Nukleinsäure mit etwa 100 bp (= Basenpaare) Länge und einem G + C-Gehalt von 50 % in Abwesenheit von Formamid bestimmt. Der Fachmann weiß, wie die erforderlichen Hybridisierungsbedingungen anhand von Lehrbüchern, wie dem vorstehend erwähnten oder aus den folgenden Lehrbüchern Sambrook et al., "Molecular Cloning", Cold Spring Harbor Laboratory, 1989; Hames und Higgins (Hrsgb.) 1985, "Nucleic Acids Hybridization: A Practical Approach", IRL Press at Oxford University Press, Oxford; Brown (Hrsgb.) 1991, "Essential Molecular Biology: A Practical Approach", IRL Press at Oxford University Press, Oxford, bestimmt werden können.

Zur Bestimmung der prozentualen Homologie (= Identität) von zwei Aminosäuresequenzen (z.B. einer der Sequenzen der SEQ ID NO: 2, SEQ ID NO: 5, SEQ ID NO: 8, SEQ ID NO: 10, SEQ ID NO: 12, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35 oder SEQ ID NO: 37) oder von zwei Nukleinsäuren (z.B. SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36) werden die Sequenzen zum Zweck des optimalen Vergleichs untereinander geschrieben (z.B. können Lücken in die Sequenz eines Proteins oder einer Nukleinsäure eingefügt werden, um ein optimales Alignment mit dem anderen Protein oder der anderen Nukleinsäure zu erzeugen). Die Aminosäurereste oder Nukleotide an den entsprechenden Aminosäurepositionen oder Nukleotidpositionen werden dann verglichen. Wenn eine Position in einer Sequenz durch den gleichen Aminosäurerest oder das gleiche Nukleotid wie die entsprechende Stelle in der anderen Sequenz belegt wird, dann sind die Moleküle an dieser Position homolog (d.h. Aminosäure- oder Nukleinsäure-"Homologie", wie hier verwendet, entspricht Aminosäure- oder Nukleinsäure-"Identität"). Die prozentuale Homologie zwischen den beiden Sequenzen ist eine Funktion der Anzahl an identischen Positionen, die den Sequenzen gemeinsam sind (d.h. % Homologie = Anzahl der identischen Positionen/Gesamtanzahl der Positionen x 100). Die Begriffe Homologie und Identität sind damit als Synonym anzusehen. Die verwendeten Programme bzw. Algorithmen sind oben beschrieben.

Ein isoliertes Nukleinsäuremolekül, das eine Lysophosphatidsäure Acyltransferase, Glycerin-3-phosphat Acyltransferase, Diacylglycerin Acyltransferase oder Lecithin Cholesterin Acyltransferase kodiert, die zu einer Proteinsequenz der SEQ ID NO: 2, SEQ ID NO: 5, SEQ ID NO: 8, SEQ ID NO: 10, SEQ ID NO: 12, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35 oder SEQ ID NO: 37 homolog ist, kann durch Einbringen einer oder mehrerer Nukleotidsubstitutionen, -additionen oder -deletionen in eine Nukleotidsequenz der SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 erzeugt werden, so dass eine oder mehrere Aminosäuresubstitutionen, -additionen oder -deletionen in das kodierte Protein eingebracht werden. Mutationen können in eine der Sequenzen der SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 durch Standardtechniken, wie stellenspezifische Mutagenese und PCR-vermittelte Mutagenese, eingebracht werden. Vorzugsweise werden konservative Aminosäuresubstitutionen an einer oder mehreren der vorhergesagten nicht-essentiellen Aminosäureresten hergestellt. Bei einer "konservativen Aminosäuresubstitution" wird der Aminosäurerest gegen einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.B. Asparaginsäure, Glutaminsäure), ungeladenen polaren Seitenketten (z.B. Glycin, Asparagin, Glutamin, Serin, Threonin, Tyrosin, Cystein), unpolaren Seitenketten, (z.B. Alanin, Valin, Leucin, Isoleucin, Prolin, Phenylalanin, Methionin, Tryptophan), beta-verzweigten Seitenketten (z.B. Threonin, Valin, Isoleucin) und aromatischen Seitenketten (z.B. Tyrosin, Phenylalanin, Tryptophan, Histidin). Ein vorhergesagter nicht-essentieller Aminosäurerest in einer Lysophosphatidsäure Acyltransferase, Glycerin-3-phosphat Acyltransferase, Diacylglycerin Acyltransferase oder Lecithin Cholesterin Acyltransferase wird somit vorzugsweise durch einen anderen Aminosäurerest aus der gleichen Seitenkettenfamilie ausgetauscht. Alternativ können bei einer anderen Ausführungsform die Mutationen zufallsgemäß über die gesamte oder einen Teil der Lysophosphatidsäure Acyltransferase, Glycerin-3-phosphat Acyltransferase, Diacylglycerin Acyltransferase oder Lecithin Cholesterin Acyltransferase kodierenden Sequenz eingebracht werden, z.B. durch Sättigungsmutagenese, und die resultierenden Mutanten können nach der hier beschriebenen Lysophosphatidsäure Acyltransferase-, Glycerin-3-phosphat Acyltransferase-, Diacylglycerin Acyltransferase- oder Lecithin Cholesterin Acyltransferase-Aktivität durchmustert werden, um Mutanten zu identifizieren, die die Lysophosphatidsäure Acyltransferase-, Glycerin-3-phosphat Acyltransferase-, Diacylglycerin Acyltransferase- oder Lecithin Cholesterin Acyltransferase-Aktivität beibehalten haben. Nach der Mutagenese einer der Sequenzen der SEQ ID NO: 1, SEQ ID NO: 3, SEQ ID NO: 4, SEQ ID NO: 6, SEQ ID NO: 7, SEQ ID NO: 9, SEQ ID NO: 11, SEQ ID NO: 13, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34 oder SEQ ID NO: 36 kann das kodierte Protein rekombinant exprimiert werden, und die Aktivität des Proteins kann z.B. unter Verwendung der hier beschriebenen Tests bestimmt werden.

Diese Erfindung wird durch die nachstehenden Beispiele weiter veranschaulicht, die nicht als beschränkend aufgefasst werden sollten.

### Beispiele

### Beispiel 1: Allgemeine Verfahren

### a) Allgemeine Klonierungsverfahren:

Klonierungsverfahren, wie beispielsweise Restriktionsspaltungen, Agarosegelelektrophorese, Reinigung von DNA-Fragmenten, Transfer von Nukleinsäuren auf Nitrocellulose- und Nylonmembranen, Verbindung von DNA-Fragmenten, Transformation von Escherichia coli- und Hefe-Zellen, Anzucht von Bakterien und Sequenzanalyse rekombinanter DNA, wurden durchgeführt wie beschrieben in Sambrook et al. (1989) (Cold Spring Harbor Laboratory Press: ISBN 0-87969-309-6) oder Kaiser, Michaelis und Mitchell (1994) "Methods in Yeast Genetics" (Cold Spring Harbor Laboratory Press: ISBN 0-87969-451-3).

### b) Chemikalien

Die verwendeten Chemikalien wurden, wenn im Text nicht anders angegeben, in p.A.-Qualität von den Firmen Fluka (Neu-Ulm), Merck (Darmstadt), Roth (Karlsruhe), Serva (Heidelberg) und Sigma (Deisenhofen) bezogen. Lösungen wurden unter Verwendung von reinem pyrogenfreiem Wasser, im nachstehenden Text als H₂O bezeichnet, aus einer Milli-Q-Wassersystem-Wasserreinigungsanlage (Millipore, Eschborn) hergestellt. Restriktionsendonukleasen, DNA-modifizierende Enzyme und olekularbiologische Kits wurden bezogen von den Firmen AGS (Heidelberg), Amersham (Braunschweig), Biometra (Göttingen), Boehringer (Mannheim), Genomed (Bad Oeynhausen), New England Biolabs (Schwalbach/Taunus), Novagen (Madison, Wisconsin, USA), Perkin-Elmer (Weiterstadt), Pharmacia (Freiburg), Qiagen (Hilden) und Stratagene (Amsterdam, Niederlande). Wenn nicht anders angegeben, wurden sie ach den Anweisungen des Herstellers verwendet.

### c) Klonierung und Expression von Desaturasen und Elongasen

Der Escherichia coli-Stamm XL1 Blue MRF' kan (Stratagene) wurde zur Subklonierung der Δ-6-Desaturase aus Physcomitrella patens verwendet. Für die funktionelle Expression dieses Gens wurde der Saccharomyces cerevisiae-Stamm INVSc 1 (Invitrogen Co.) verwendet. E. coli wurde in Luria-Bertani-Brühe (LB, Duchefa, Haarlem, Niederlande) bei 37°C kultiviert. Wenn nötig, wurde Ampicillin (100 mg/Liter) zugegeben, und 1,5 % Agar (Gew./Vol.) wurde für feste LB-Medien hinzugefügt. S. erevisiae wurde bei 30°C entweder in YPG-Medium oder in komplettem Minimaledium ohne Uracil (CMdum; siehe in: Ausubel, F.M., Brent, R., Kingston, R.E., Moore, D.D., Seidman, J.G., Smith, J.A., Struhl, K., Albright, L.B., Coen, D.M., und Varki, A. (1995) Current Protocols in Molecular Biology, John Wiley & Sons, New York) mit entweder 2 % (Gew./Vol.) Raffinose oder Glucose kultiviert. Für feste Medien wurden 2 % (Gew./Vol.) Bacto™-Agar (Difco) hinzugefügt. Die zur Klonierung und Expression verwendeten Plasmide sind pUC18 (Pharmacia) und pYES2 (Invitrogen Co.).

### d) Klonierung und Expression PUFA-spezifischer Desaturasen und Elongasen

Für die Expression in Pflanzen wurden cDNA Klone aus SEQ ID NO: 46 (Physcomitrella patens Δ-6-Desaturase), 48 (Physcomitrella patens Δ-6-Elongase) oder 50 (Phaedactylum tricornutum Δ-5-Desaturase) so modifiziert, dass lediglich die Codierregion mittels Polymerase Kettenreaktion unter Zuhilfenahme zweier Oligonukleotide amplifiziert werden. Dabei wurde darauf geachtet, dass eine Konsensusequenz vor dem Startcodon zur effizienten Translation eingehalten wurde. Entweder wurde hierzu die Basenfolge ATA oder AAA gewählt und vor das ATG in die Sequenz eingefügt [Kozak, M. (1986) Point mutations define a sequence flanking the AUG initiator codon that modulates translation by eukaryotic ribosomes, Cell 44, 283-2929]. Vor diesem Konsensustriplett wurde zusätzlich eine Restriktionsschnittstelle eingeführt, die kompatibel sein muss zur Schnittstelle des Zielvektors, in den das Fragment kloniert werden soll und mit dessen Hilfe die Genexpression in Mikroorganismen oder Pflanzen erfolgen soll.

Die PCR-Reaktion wurde mit Plasmid-DNA als Matrize in einem Thermocycler (Biometra) mit der Pfu-DNA-(Stratagene)Polymerase und dem folgenden Temperaturprogramm durchgeführt: 3 min bei 96°C, gefolgt von 30 Zyklen mit 30 s bei 96°C, 30 s bei 55°C und 2 min bei 72°C, 1 Zyklus mit 10 min bei 72°C und Stop bei 4°C. Die Anlagerungstemperatur wurde je nach gewählten Oligonukleotiden variiert. Pro Kilobasenpaare DNA ist von einer Synthesezeit von etwa einer Minute auszugehen. Weitere Parameter, die Einfluss auf die PCR haben wie z.B. Mg-Ionen, Salz, DNA Polymerase etc., sind dem Fachmann auf dem Gebiet geläufig und können nach Bedarf variiert werden.

Die korrekte Größe des amplifizierten DNA-Fragments wurde mittels Agarose-TBE-Gelelektrophorese bestätigt. Die amplifizierte DNA wurde aus dem Gel mit dem QIAquick-Gelextraktionskit (QIAGEN) extrahiert und in die Smal-Restriktionsstelle des dephosphorylierten Vektors pUC18 unter Verwendung des Sure Clone Ligations Kit (Pharmacia) ligiert, wobei die pUC-Derivate erhalten wurden. Nach der Transformation von E. coli XL1 Blue MRF' kan wurde eine DNA-Minipräparation [Riggs, M.G., & McLachlan, A. (1986) A simplified screening procedure for large numbers of plasmid mini-preparation. BioTechniques 4, 310-313] an ampicillinresistenten Transformanden durchgeführt, und positive Klone mittels BamHI-Restriktionsanalyse identifiziert. Die Sequenz des klonierten PCR-Produktes wurde mittels Resequenzierung unter Verwendung des ABI PRISM Big Dye Terminator Cycle Sequencing Ready Reaction Kit (Perkin-Elmer, Weiterstadt) bestätigt.

### e) Transformation von Agrobacterium

Die Agrobacterium-vermittelte Pflanzentransformation wurde, wenn nicht anders beschrieben, wie von Deblaere et al. (1984, Nucl. Acids Res. 13, 4777-4788) mit Hilfe eines Agrobacterium tumefaciens-Stamms durchgeführt.

### f) Pflanzentransformation

Die Agrobacterium-vermittelte Pflanzentransformation wurde, wenn nicht anders beschrieben, unter Verwendung von Standard-Transformations- und Regenerationstechniken durchgeführt (Gelvin, Stanton B., Schilperoort, Robert A., Plant Molecular Biology Manual, 2. Aufl., Dordrecht: Kluwer Academic Publ., 1995, in Sect., Ringbuc Zentrale Signatur: BT11-P ISBN 0-7923-2731-4; Glick, Bernard R., Thompson, John E., Methods in Plant Molecular Biology and Biotechnology, Boca Raton: CRC Press, 1993, 360 S., ISBN 0-8493-5164-2).

Nach diesen kann beispielsweise Raps mittels Kotyledonen- oder Hypokotyltransformation transformiert werden (Moloney et al., Plant Cell 8 (1989) 238-242; De Block et al., Plant Physiol. 91 (1989) 694-701). Die Verwendung von Antibiotika für die Agrobacterium- und Pflanzenselektion hängt von dem für die Transformation verwendeten binären Vektor und Agrobacterium-Stamm ab. Die Rapsselektion wird dabei gewöhnlich unter Verwendung von Kanamycin als selektierbarem Pflanzenmarker durchgeführt.

Die Transformation von Soja kann unter Verwendung von beispielsweise einer in EP-A-0 0424 047 (Pioneer Hi-Bred International) oder in EP-A-0 0397 687, US 5,376,543, US 5,169,770 (University Toledo) beschriebenen Technik durchgeführt werden.

Die Pflanzentransformation unter Verwendung von Teilchenbeschuss, Polyethylenglycol-vermittelter DNA-Aufnahme oder über die Siliziumcarbonatfaser-Technik ist beispielsweise beschrieben von Freeling und Walbot "The maize handbook" (1993) ISBN 3-540-97826-7, Springer Verlag New York).

Der Agrobacterium-vermittelte Gentransfer in Lein (Linum usitatissimum) wurde, wenn nicht anders beschrieben, wie bei Mlynarova et al. [(1994) Plant Cell Report 13:282-285] beschriebenen Technik durchführt.

### g) Plasmide für die Pflanzentransformation

Zur Pflanzentransformation wurden binäre Vektoren auf Basis der Vektoren pBinAR (Höfgen und Willmitzer, Plant Science 66 (1990) 221-230) oder pGPTV (Becker et al 1992, Plant Mol. Biol. 20:1195-1197) verwendet. Die Konstruktion der binären Vektoren, die die zu exprimierenden Nukleinsäuren enthalten, erfolgt durch Ligation der cDNA in Sense-Orientierung in die T-DNA erfolgen. 5' der cDNA aktiviert ein Pflanzenpromotor die Transkription der cDNA. Eine Polyadenylierungssequenz befindet sich 3' von der cDNA. Die binären Vektoren können unterschiedliche Markergene tragen wie beispielsweise das Acetolactat Synthasegens (AHAS oder ALS) [Ott et al., J. Mol. Biol. 1996, 263:359-360], das eine Resistenz gegen die Imidazolinone vermittelt oder das nptII-Markergen, das für eine Kanamycin-Resistenz vermittelt durch Neomycinphosphotransferase codiert.

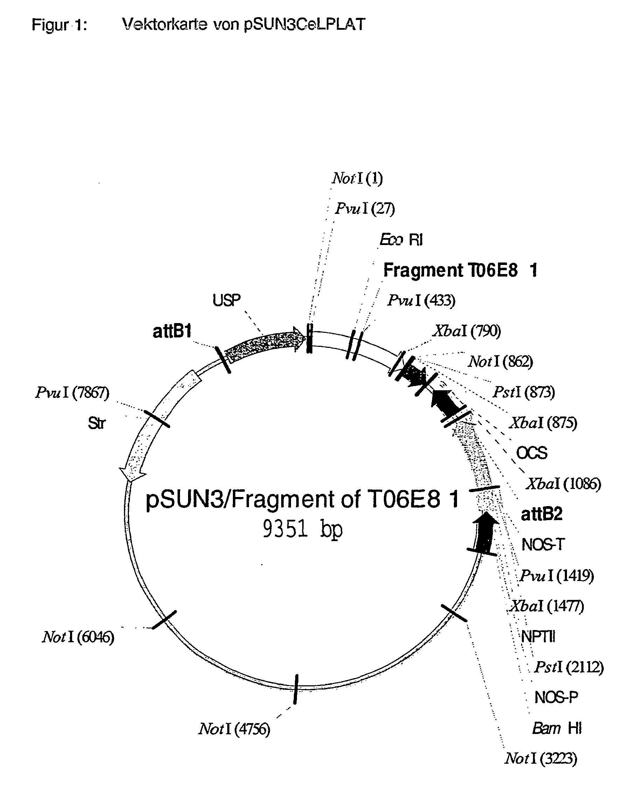

Die gewebespezifische Expression der Nukleinsäuren lässt sich unter Verwendung eines gewebespezifischen Promotors erzielen. Wenn nicht anders beschrieben wurde der LeB4- oder der USP-Promotor oder der Phaseolin-Promotor 5' der cDNA einkloniert wird. Als Terminatoren wurde der NOS-Terminator und der OCS-Terminator verwendet (siehe Figur 1). Figur 1 zeigt eine Vektorkarte des zur Expression verwendeten Vektor pSUN3CeLPLAT.

Auch jedes andere samenspezifische Promotorelement wie z.B. der Napin- oder Arcelin Promotor Goossens et al. 1999, Plant Phys. 120(4):1095-1103 und Gerhardt et al. 2000, Biochimica et Biophysica Acta 1490(1-2):87-98) kann verwendet werden.

Zur konstitutiven Expression in der ganzen Pflanzen lässt sich der CaMV-35S-Promotor oder ein v-ATPase C1 Promotor verwenden.

Die im Verfahren verwendeten Nukleinsäuren, die für die Acyl-CoA:Lysophospholipid-Acyltransferasen; Desaturasen oder Elongasen codieren, wurden durch Konstruktion mehrerer Expressionskassetten hintereinander in einen binären Vektor kloniert, um den Stoffwechselweg in Pflanzen nachzubilden.

Innerhalb einer Expressionskassette kann das zu exprimierende Protein unter Verwendung eines Signalpeptids, beispielsweise für Plastiden, Mitochondrien oder das Endoplasmatische Retikulum, in ein zelluläres Kompartiment dirigiert werden (Kermode, Crit. Rev. Plant Sci. 15, 4 (1996) 285-423). Das Signalpeptid wird 5' im Leseraster mit der cDNA einkloniert, um die subzelluläre Lokalisierung des Fusionsprotein zu erreichen.

Beispiele für Multiexpressionskassetten wurden in DE 102 19 203 offenbart und sind im folgenden nochmals wiedergegeben.

### i.) Promotor-Terminator-Kassetten

Expressionskassetten bestehen aus wenigstens zwei funktionellen Einheiten wie einem Promotor und einem Terminator. Zwischen Promotor und Terminator können weitere gewünschte Gensequenzen wie Targetting-Sequenzen, Codierregionen von Genen oder Teilen davon etc. eingefügt werden. Zum Aufbau der Expressionskassetten wurden Promotoren und Terminatoren (USP Promotor: Baeumlein et al., Mol Gen Genet, 1991, 225 (3):459-67); OCS Terminator: Gielen et al. EMBO J. 3 (1984) 835ff.) mithilfe der Polymerasekettenreaktion isoliert und mit flankierenden Sequenzen nach Wahl auf Basis von synthetischen Oligonukleotiden maßgeschneidert.

Folgende Oligonukleotide können beispielsweise verwendet werden:
USP1 vorne:
   - CCGGAATTCGGCGCGCCGAGCTCCTCGAGCAAATTTACACATTGCCA -
USP2 vorne:
   - CCGGAATTCGGCGCGCCGAGCTCCTCGAGCAAATTTACACATTGCCA -
USP3 vorne:
   - CCGGAATTCGGCGCGCCGAGCTCCTCGAGCAAATTTACACATTGCCA -
USP1 hinten:
   - AAAACTGCAGGCGGCCGCCCACCGCGGTGGGCTGGCTATGAAGAAATT -
USP2 hinten:
   - CGCGGATCCGCTGGCTATGAAGAAATT -
USP3 hinten:
   - TCCCCCGGGATCGATGCCGGCAGATCTGCTGGCTATGAAGAAATT -
OCS1 vorne:
   - AAAACTGCAGTCTAGAAGGCCTCCTGCTTTAATGAGATAT -
OCS2 vorne:
   - CGCGGATCCGATATCGGGCCCGCTAGCGTTAACCCTGCTTTAATGAGATAT -
OCS3 vorne:
   - TCCCCCGGGCCATGGCCTGCTTTAATGAGATAT -
OCS1 hinten:
   - CCCAAGCTTGGCGCGCCGAGCTCGAATTCGTCGACGGACAATCAGTAAATTGA -
OCS2 hinten:
   - CCCAAGCTTGGCGCGCCGAGCTCGAATTCGTCGACGGACAATCAGTAAATTGA -
OCS3 hinten:
   - CCCAAGCTTGGCGCGCCGAGCTCGTCGACGGACAATCAGTAAATTGA -

Die Methoden sind dem Fachmann auf dem Gebiet bekannt und sind allgemein literaturbekannt.

In einem ersten Schritt wurden ein Promotor und ein Terminator über PCR amplifiziert. Dann wurde der Terminator in ein Empfängerplasmid kloniert und in einem zweiten Schritt der Promotor vor den Terminator inseriert. Dadurch wurde eine Expressionskassette in das Basis-Plasmid cloniert. Auf Basis des Plamides pUC19 wurden so die Plasmide pUT1, 2 und 3 erstellt.

Die entsprechenden Konstrukte bzw. Plasmide sind in SEQ ID NO: 52, 53 und 54 definiert. Sie enthalten den USP-Promotor und den OCS Terminator. Auf Basis dieser Plasmide wurde das Konstrukt pUT12 erstellt, indem pUT1 mittels Sall/Scal geschnitten wurde und pUT2 mittels Xhol/Scal geschnitten wurde. Die die Expressionskassetten enthaltenden Fragmente wurden ligiert und in E. coli XL1 blue MRF transformiert. Es wurde nach Vereinzelung von ampicillinresistenten Kolonien DNA präpariert und per Restriktionsanalyse solche Klone identifiziert, die zwei Expressionskassetten enthalten. Die Xhol/Sall Ligation kompatibler Enden hat dabei die beiden Schnittstellen Xhol und Sall zwischen den Expressionskassetten eleminiert. Das resultierende Plasmid pUT12 wird in SEQ ID NO: 55 wiedergegeben. Anschließend wurde pUT12 wiederum mittels Sal/Scal geschnitten und pUT3 mittels Xhol/Scal geschnitten. Die die Expressionskassetten enthaltenden Fragmente wurden ligiert und in E. coli XLI blue MRF transformiert. Es wurde wieder nach Vereinzelung aus ampicillinresistenten Kolonien DNA präpariert und per Restriktionsanalyse solche Klone identifiziert, die drei Expressionskassetten enthalten. Auf diese Weise wurde ein Set von Multiexpressionskassetten geschaffen, dass für die Insertion gewünschter DNA genutzt werden kann und in Tabelle 1 beschrieben wird und zudem noch weitere Expressionskassetten aufnehmen kann.

Diese enthalten folgende Elemente:

**Tabelle 1**

| PUC19-Derivat | Schnittstellen vor dem USP Promotor | Multiple Klonierungs-Schnittstellen | Schnittstellen hinter dem OCS-Terminator |
|---|---|---|---|
| PUT1 | EcoRI/AscI/ SacI/XhoI | BstXI/NotI/ PstI/XbaI/StuI | SalIEcoRI/ SacI/AscI/ HindIII |
| PUT2 | EcoRI/AscI/ SacI/XhoI | BamHI/EcoRV/ ApaI/NheI/ HpaI | SalI/EcoRI/ SacI/AscI/ HindIII |
| PUT3 | EcoRI/AscI/ SacI/XhoI | BgIII/NaeI/ ClaI/SmaI/NcoI | SalI/SacI/ AscI/HindIII |
| PUT12 Doppelexpressionskasette | EcoRI/AscI/ SacI/XhoI | BstXI/NotI/ PstI/XbaI/StuI Und | SalI/EcoRI/ SacI/AscI/ HindIII |
| | | BamHI/EcoRV/ ApaI/NheI/ HpaI | |
| PUT123 Tripelexpressionskassette | EcoRI/AscI/ SacI/XhoI | 1. BstXI/NotI/ PstI/XbaI/StuI und | SalI/SacI/ AscI/HindIII |
| | | 2. BamHI/EcoRV/ ApaI/NheI/ HpaI und | |
| | | 3. BglII/NaeI/ ClaI/SmaI/NcoI | |

Weiterhin lassen sich wie beschrieben und wie in Tabelle 2 näher spezifiziert weitere Multiexpressionskassetten mithilfe des
i) USP-Promotors oder mithilfe des
ii) 700 Basenpaare 3'-Fragmentes des LeB4-Promotors oder mithilfe des
iii) DC3-Promotors erzeugen und für samenspezifische Genexpression einsetzen.

Der DC3-Promotor ist beschrieben bei Thomas, Plant Cell 1996, 263:359-368 und besteht lediglich aus der Region -117 bis +26 weshalb er mithin einer der kleinsten bekannten samenspezifischen Promotoren darstellt. Die Expressionskassetten können mehrfach den selben Promotor enthalten oder aber über drei verschiedene Promotoren aufgebaut werden.

Vorteilhaft verwendete Polylinker- bzw. Polylinker-Terminator-Polylinker sind den Sequenzen SEQ ID NO: 60 bis 62 zu entnehmen.

**Tabelle 2: Multiple Expressionskassetten**

| Plasmidname des pUC19-Derivates | Schnittstellen vor dem jeweiligen Promotor | Multiple Klonierungs-Schnittstellen | Schnittstellen hinter dem OCS-Terminator |
|---|---|---|---|
| pUT1 (pUC19 mit USP-OCS1) | EcoRI/AscI/SacI/XhoI | (1) BstXI/NotI/PstI/ XbaI/StuI | SalI/EcoR1/SacI/AscI/ HindIII |
| PDCT (pUC19 mit DC3-OCS) | EcoRI/AscI/SacI/XhoI | (2) BamHI/EcoRV/ ApaI/NheI/ HpaI | SalI/EcoR1/SacI/AscI/ HindIII |
| PleBT (pUC19-mit LeB4(700)-OCS) | EcoRI/AscI/SacI/XhoI | (3) BglII/NaeI/ ClaI/SmaI/NcoI | SalI/SacI/AscI/HindIII |
| PUD12 (pUC 19 mit mit USP-OCS1 und mit DC3-OCS) | EcoRI/AscI/SacI/XhoI | (1) BstXI/NotI/PstI/XbaI/StuI und | SalI/EcoRI/SacI/AscI/ HindIII |
| | | (2) BamHI/EcoRV/ ApaI/NheI/ HpaI | |
| PUDL123 Triple expression cassette (pUC19 mit USP/DC3 und LeB4-700) | EcoRI/AscI/SacI/XhoI | (1) BstXI/NotI/ PstI/XbaI/StuI und | SalI/SacI/AscI/HindIII |
| | | (2) BamHI/ (EcoRV*)/ApaI/NheI/ HpaI und | |
| | | (3) BglII/NaeI/ ClaI/SmaI/NcoI | |

| | | | |
|---|---|---|---|
| * EcoRV Schnittstelle schneidet im 700 Basenpaarfragment des LeB4 Promotors (LeB4-700) | | | |

Analog lassen sich weitere Promotoren für Multigenkonstrukte erzeugen insbesondere unter Verwendung des
a) 2,7 kB Fragmentes des LeB4-Promotors oder mithilfe des
b) Phaseolin-Promotors oder mithilfe des
c) konstitutiven v-ATPase c1-Promotors.

Es kann insbesondere wünschenswert sein, weitere besonders geeignete Promotoren zum Aufbau samenspezifischer Multiexpressionskassetten wie z.B. den Napin-Promotor oder den Arcelin-5 Promotor zu verwenden.

Weitere in Pflanzen nutzbare Vektoren mit einer bzw. zwei oder drei Promotor-Terminator-Expressionkassetten sind den Sequenzen SEQ ID NO: 63 bis SEQ ID NO: 68 zu entnehmen.

### ii.) Erstellung von Expressionskonstrukten, die Promotor, Terminator und gewünschte Gensequenz zur PUFA Genexpression in pflanzlichen Expressionskassetten enthalten.

In pUT123 wird zunächst über BstXI und Xbal die Δ-6-Elongase Pp_PSE1 in die erste Kassette inseriert. Dann wird die Δ-6-Desaturase aus Moos (Pp_des6) über BamHI/Nael in die zweite Kassette inseriert und schließlich die Δ-5-Desaturase aus Phaeodactylum (Pt_des5) über BgIII/NcoI in die dritte Kassette inseriert (siehe SEQ ID NO: 56). Das Dreifachkonstrukt erhält den Namen pARA1. Unter Berücksichtigung sequenzspezifischer Restriktionsschnittstellen können weitere Expressionskassetten gemäß Tabelle 3 mit der Bezeichnung pARA2, pARA3 und pARA4 erstellt werden.

**Tabelle 3: Kombinationen von Desaturasen und Elongasen**

| Gen Plasmid | Δ-6-Desaturase | Δ-5-Desaturase | Δ-6-Elongase |
|---|---|---|---|
| pARA1 | Pp des6 | Pt des5 | Pp PSE1 |
| pARA2 | Pt des6 | Pt_des5 | Pp PSE1 |
| pARA3 | Pt des6 | Ce des5 | Pp_PSE1 |
| PARA4 | Ce_des6 | Ce_des5 | Ce_PSE1 |

| | | | |
|---|---|---|---|
| des5 = PUFA spezifische Δ-5-Desaturase des6 = PUFA spezifische Δ-6-Desaturase PSE = PUFA spezifische Δ-6-Elongase Pt_des5 = Δ-5-Desaturase aus Phaeodactylum tricornutum Pp_des6 oder Pt_des6 = Δ-6-Desaturase aus Physcomitrella patens bzw. Phaeodactylum tricornutum Pp = Physcomitrella patens, Pt = Phaeodactylum tricornutum Pp_PSE1 = Δ-6-Elongase aus Physcomitrella patens Pt_PSE1 = Δ-6-Elongase aus Phaeodactylum tricornutum Ce_des5 = Δ-5-Desaturase aus Caenorhabditis elegans (Genbank Acc. Nr. AF078796) Ce_des6 = Δ-6-Desaturase aus Caenorhabditis elegans (Genbank Acc. Nr. AF031477, Basen 11-1342) Ce_PSE1 = Δ-6-Elongase aus Caenorhabditis elegans (Genbank Acc. Nr. AF244356, Basen 1-867) | | | |

Auch weitere Desaturasen oder Elongasegensequenzen können in Expressionskassetten beschriebener Art inseriert werden wie z.B. Genbank Acc. Nr. AF231981, NM_013402, AF206662, AF268031, AF226273, AF110510 oder AF110509.

### iii.) Transfer von Expressionskassetten in Vektoren zur Transformation von Agrobakterium tumefaciens und zur Transformation von Pflanzen

Die so erstellten Konstrukte wurden mittels AscI in den binären Vektor pGPTV inseriert. Die multiple Klonierungssequenz wurde zu diesem Zweck um eine AscI Schnittstelle erweitert. Zu diesem Zweck wurde der Polylinker als zwei doppelsträngige Oligonukleotide neu synthetisiert, wobei eine zusätzliche AscI DNA Sequenz eingefügt wird. Das Oligonukleotid wurde mittels EcoRI und HindIII in den Vektor pGPTV inseriert. Die notwendigen Kloniertechniken sind dem Fachmann bekannt und können einfach wie in Beispiel 1 beschrieben nachgelesen werden.

Für die im folgenden beschriebenen Versuche wurden als Nukleinsäuresequenzen für die Δ-5-Desaturase (SEQ ID NO: 50), die Δ-6-Desaturase (SEQ ID NO: 46) und die Δ-6-Elongase (SEQ ID NO: 48), die Sequenzen aus Physcomitrella patens und Phaedactylum tricornutum verwendet. Die entsprechenden Aminosäuresequenzen sind den Sequenzen SEQ ID NO: 47, SEQ ID NO: 49 und SEQ ID NO: 51 zu entnehmen. Ein Vektor der alle vorgenannten Gene enthält ist in SEQ ID NO: 56 wiedergegeben. Die korrespondierenden Aminosäurensequenzen der Gene sind SEQ ID NO: 57, SEQ ID NO: 58 und SEQ ID NO: 59 zu entnehmen.

### Beispiel 2: Klonierung und Charakterisierung der ceLPLATs (SEQ ID NO: 38 - 44)

### a) Datenbanken-Suche

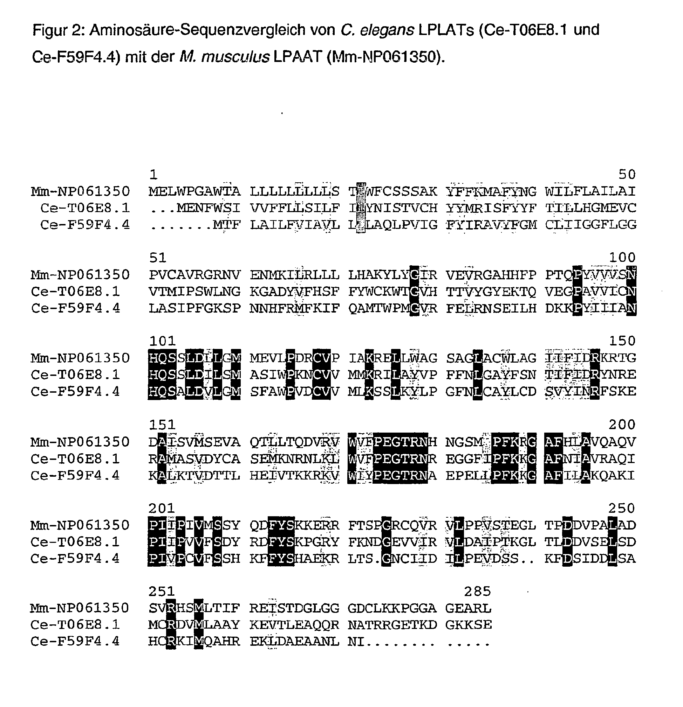

Die Identifizierung der ceLPLATs (= Acyl-CoA:Lysophospholipid-Acyltransferase aus Caenorhabditis elegans) erfolgte durch Sequenzvergleiche mit bekannten LPA-ATs. Die Suche wurde mit Hilfe des BLAST-Psi-Algorithmus (Altschul et al., J. Mol. Biol. 1990, 215: 403-410) auf das Nematodengenom (*Caenorhabditis elegans*) beschränkt, da dieser Organismus LCPUFAs synthetisiert. Für den Sequenzvergleich diente als Sonde eine LPAAT Proteinsequenz aus *Mus musculus* (MsLPAAT Accession Nr. NP_061350). LPLAT katalysiert durch eine reversible Transferasereaktion die ATPunabhängige Synthese von Acyl-CoAs aus Phospholipiden mit Hilfe von CoA als Cofactor (Yamashita et al., J. Biol. Chem. 2001, 20: 26745-26752). Durch Sequenzvergleiche konnten zwei putative ceLPLAT-Sequenzen identifiziert werden (Accession Nr. *T06E8.1* bzw. *F59F4.4*). Die identifizierten Sequenzen weisen die größte Ähnlichkeit jeweils zueinander und zu MsLPAATs auf (Figur 2). Das Alignment wurde mit dem Programm Clustal erstellt.

### b) Klonierung der CeLPLATs

Auf der Basis der ceLPLAT-Nukleinsäuresequenzen wurden Primerpaare synthetisiert (Tabelle 4) und mittels PCR-Verfahren die zugehörigen cDNAs aus einer *C*. *elegans*cDNA-Bank isoliert. Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der LPLAT-cDNAs wurde jeweils mit 2 µl cDNA-Bank-Lösung als Template, 200 µM dNTPs, 2,5 U "proofreading" *pfu*-Polymerase und 50 pmol eines jeden Primers in einem Gesamtvolumen von 50 µl durchgeführt. Die Bedingungen für die PCR waren wie folgt: Erste Denaturierung bei 95°C für 5 Minuten, gefolgt von 30 Zyklen bei 94°C für 30 Sekunden, 58°C für eine Minute und 72°C für 2 Minuten sowie ein letzter Verlängerungsschritt bei 72°C für 10 Minuten. Die Sequenz der LPLAT-cDNAs wurde durch DNA-Sequenzierung bestätigt.

**Tabelle 4: Nukleotidsequenzen der PCR-Primer zur Klonierung von CeLPLATs**

| Primer | Nukleotidsequenz |
|---|---|
| 5' T06E8.1f* | 5' ACATAATGGAGAACTTCTGGTCGATCGTC 3' |
| 3' T06E8.1r* | 5' TTACTCAGATTTCTTCCCGTCTTT 3' |
| 5' F59F4.4f* | 5' ACATAATGACCTTCCTAGCCATATTA 3' |
| 3' F59F4.4r* | 5' TCAGATATTCAAATTGGCGGCTTC 3' |

| | |
|---|---|
| * f: forward, r: reverse | |

### Beispiel 3: Analyse der Auswirkung der rekombinanten Proteine auf die Produktion des gewünschten Produktes

### a) Aufarbeitungsmöglichkeiten

Die Auswirkung der genetischen Modifikation in Pilzen, Algen, Ciliaten oder wie in den Beispielen weiter oben beschrieben in Hefen auf die Produktion der mehrfach ungesättigten Fettsäuren oder Pflanzen kann bestimmt werden, indem die modifizierten Mikroorganismen oder die modifizierte Pflanze unter geeigneten Bedingungen (wie den vorstehend beschriebenen) gezüchtet werden und das Medium und/oder die zellulären Komponenten auf die erhöhte Produktion der Lipide oder Fettsäuren untersucht wird. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A., et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren zum Nachweis von Fettsäuren in Hefen werden Pflanzenlipide aus Pflanzenmaterial wie von Cahoon et al. (1999) Proc. Natl. Acad. Sci. USA 96 (22):12935-12940, und Browse et al. (1986) Analytic Biochemistry 152:141-145, beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergamon Press, 1 (1952) - 16 (1977) u.d.T.: Progress in the Chemistry of Fats and Other Lipids CODEN.

So kann die Analyse von Fettsäuren oder Triacylglycerin (= TAG, Abkürzungen in Klammern angegeben) z.B. mittels Fettsäuremethylester (= FAME), Gas-Flüssigkeitschromatographie-Massenspektrometrie (= GC-MS) oder Dünnschichtchromatographie (TLC) erfolgen.

Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl.: Christie, Oily Press, Dundee, 119-169; 1998, Gaschromatographie-Massenspektrometrie-Verfahren, Lipide 33:343-353).

Das zu analysierende Pflanzenmaterial kann dazu entweder durch Ultraschallbehandlung, Mahlen in der Glasmühle, flüssigen Stickstoff und Mahlen oder über andere anwendbare Verfahren aufgebrochen werden. Das Material wird dann anschließend nach dem Aufbrechen zentrifugiert. Das Sediment wird danach in Aqua dest. resuspendiert, 10 min bei 100°C erhitzt, auf Eis abgekühlt und erneut zentrifugiert, gefolgt von Extraktion in 0,5 M Schwefelsäure in Methanol mit 2 % Dimethoxypropan für 1 Std. bei 90°C, was zu hydrolysierten Öl- und Lipidverbindungen führt, die transmethylierte Lipide ergeben. Diese Fettsäuremethylester können anschließend in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen werden. Die Identität der erhaltenen Fettsäuremethylester lassen sich unter Verwendung von Standards, die aus kommerziellen Quellen erhältlich sind (d.h. Sigma), definieren.

Bei Fettsäuren, für die keine Standards verfügbar sind, kann die Identität über Derivatisierung und anschließende GC-MS-Analyse gezeigt werden. Beispielsweise wird die Lokalisierung von Fettsäuren mit Dreifachbindung über GC-MS nach Derivatisierung mit 4,4-Dimethoxyoxazolin-Derivaten (Christie, 1998, siehe oben) gezeigt.

### b) Fettsäureanalyse in Pflanzen

Die Gesamt-Fettsäuren wurden aus Pflanzensamen extrahiert und mittels Gaschromatographie analysiert.

Die Samen wurden mit 1 % Natriummethanolat in Methoanol aufgenommen und 20 min bei RT (ca. 22 °C) inkubiert. Anschließend wurde mit NaCl Lösung gewaschen und die FAME in 0,3 ml Heptan aufgenommen.

Die Proben wurden auf einer ZEBRON-ZB-Wax-Kapillarsäule (30 m, 0,32 mm, 0,25 mikro m; Phenomenex) in einem Hewlett Packard-6850-Gaschromatograph mit einem Flammenionisationsdetektor aufgetrennt. Die Ofentemperatur wurde von 70°C (1 min halten) bis 200°C mit einer Rate von 20°C/min, dann auf 250°C (5 min halten) mit einer Rate von 5°C/min und schließlich auf 260°C mit einer Rate von 5°C/min programmiert. Stickstoff wurde als Trägergas verwendet (4,5 ml/min bei 70°C). Die Fettsäuren wurden durch Vergleich mit Retentionszeiten von FAME-Standards (SIGMA) identifiziert.

### Beispiel 4: Funktionelle Charakterierung der CeLPLATs in Hefe

### a) Heterologe Expression in Saccharomyces cerevisiae

Zur Charakterisierung der Funktion der CeLPLATs aus *C*. *elegans* (SEQ ID NO: 38 - 44) wurden die offenen Leserahmen der jeweilgen cDNAs stromabwärts des Galactose-induzierbaren GAL1-Promotors von pYes2.1Topo unter Verwendung des pY-es2.1TOPO TA Expression Kit (Invitrogen) kloniert, wobei pYes2-T06E8.1 und pYes2-F59F4.4 erhalten wurden.

Da die Expression der CeLPLATs zu einem effizienten Austausch der Acyl-Substrate führen sollte, wurde weiterhin das Doppelkonstrukt pESCLeu-PpD6-Pse1 hergestellt, das die offenen Leserahmen einer Δ6-Desaturase (PpD6) und einer Δ6-Elongase (PSE1) aus *Physcomitrella patens* (siehe DE 102 19 203) beinhaltet. Die Nukleinsäuresequenz der Δ6-Desaturase (PpD6) und der Δ6-Elongase (Pse1) werden jeweils in SEQ ID NO: 46 und SEQ ID NO: 48 wiedergegeben. Die korrespondierenden Aminosäuresequenzen sind SEQ ID NO: 47 und SEQ ID NO: 49 zu entnehmen.

Die *Saccharomyces cerevisiae*-Stämme C13ABYS86 (Protease-defizient) und INVSc1 wurde mittels eines modifizierten PEG/Lithiumacetat-Protokolls gleichzeitig mit den Vektoren pYes2-T06E8.1 und pESCLeu-PpD6-Pse1 bzw. pYes2-F59F4.4 und pESCLeu-PpD6-Pse1 transformiert. Als Kontrolle wurde eine Hefe verwendet, die mit dem Vektor pESCLeu-PpD6-Pse1 und dem leeren Vektor pYes2 transformiert wurde. Die Selektion der transformierten Hefen erfolgte auf Komplett-Minimalmedium (CMdum)-Agarplatten mit 2% Glucose, aber ohne Uracil und Leucin. Nach der Selektion wurden 4 Transformanten, zwei pYes2-T06E8.1/pESCLeu-PpD6-Pse1 und zwei pYes2-F59F4.4/pESCLeu-PpD6-Pse1 und eine pESCLeu-PpD6-Pse1/ pYes2 zur weiteren funktionellen Expression ausgewählt. Die beschriebenen Experimente wurden auch im Hefestamm INVSc1 durchgeführt.

Für die Expresssion der CeLPAATs wurden zunächst Vorkulturen aus jeweils 2 ml CMdum-Flüssigmedium mit 2% (w/v) Raffinose, aber ohne Uracil und Leucin mit den ausgewählten Transformanten angeimpft und 2 Tage bei 30°C, 200rpm inkubiert. 5 ml CMdum-Flüssigmedium (ohne Uracil und Leucin) mit 2% Raffinose, 1% (v/v) Tergitol NP-40 und 250 µM Linolsäure (18:2^{Δ9,12}) oder Linolensäure (18:3^{Δ9,12,15}) wurden dann mit den Vorkulturen auf eine OD₆₀₀ von 0,08 angeimpft. Die Expression wurde bei einer OD₆₀₀ von 0,2-0,4 durch die Zugabe von 2% (w/v) Galactose induziert. Die Kulturen wurden für weitere 48 h bei 20°C inkubiert.

### Fettsäureanalyse

Die Hefezellen aus den Hauptkulturen wurden durch Zentrifugation (100 x g, 10 min, 20°C) geerntet und mit 100 mM NaHCO₃, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM NaHCO₃, pH 8,0 und 2 ml Aqua dest. gewaschen. Anschließend wurden die PE-Phasen mit Na₂SO₄ getrocknet, unter Argon eingedampft und in 100 µl PE aufgenommen. Die Proben wurden auf einer DB-23-Kapillarsäule (30 m, 0,25 mm, 0,25 µm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate 5°C/min und schließlich 10 min bei 250°C (halten) programmiert.

Die Identifikation der Signale erfolgte durch Vergleiche der Retentionszeiten mit entsprechenden Fettsäurestandards (Sigma).

### Acyl-CoA Analyse

Die Acyl-CoA-Analyse erfolgte wie bei Larson and Graham (2001; Plant Journal 25: 115-125) beschrieben.

### Expressionsanalyse

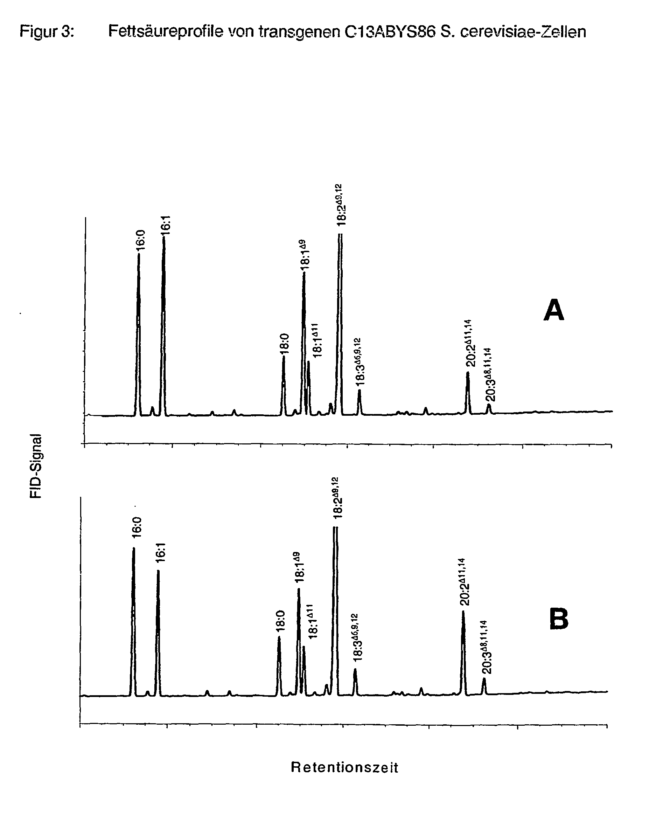

Figuren 2 A und B sowie 3 A und B zeigen die Fettsäureprofile von transgenen C13ABYS86 Hefen, die mit 18:2^{Δ9,12} bzw. 18:3^{Δ9,12,15} gefüttert wurden. Die gefütterten Substrate sind in großen Mengen in allen transgenen Hefen nachzuweisen. Alle vier transgenen Hefen zeigen eine Synthese von 18:3^{Δ6,9,12} und 20:3^{Δ8,11,14} bzw. 18:4^{Δ6,9,12,15} und 20:4^{Δ8,11,14,17}, den Produkten der Δ-6- Desaturase und Δ-6-Elongase Reaktionen. Dies bedeutet, dass die Gene PpD6 und Pse1 funktional exprimiert werden konnten.

Figur 3 gibt wie oben beschrieben die Fettsäureprofile von transgenen C13ABYS86 *S. cerevisiae*-Zellen. Die Synthese der Fettsäuremethylester erfolgte durch saure Methanolyse intakter Zellen, die entweder mit den Vektoren pESCLeu-PpD6-Pse1/pYes2 (**A**) oder pYes2-T06E8.1/pESCLeu-PpD6-Pse1 (**B**) transformiert worden waren. Die Hefen wurden in Minimalmedium in Gegenwart von 18:2^{Δ9,12} kultiviert. Anschließend wurden die Fettsäuremethylester über GLC analysiert.

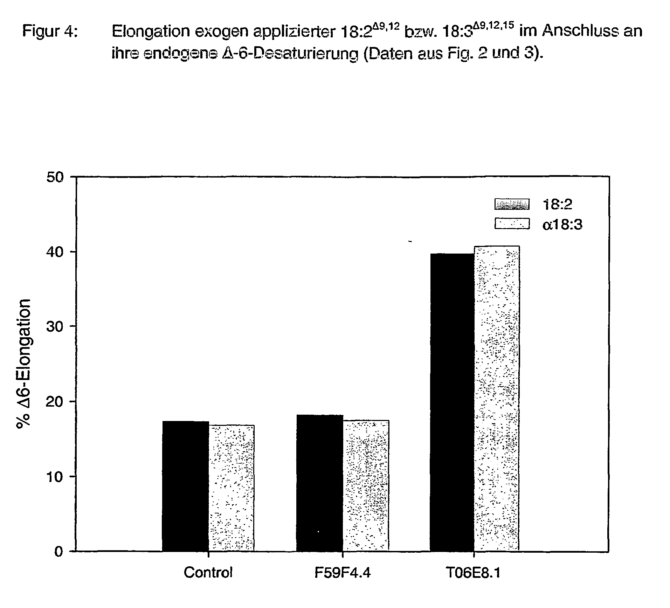

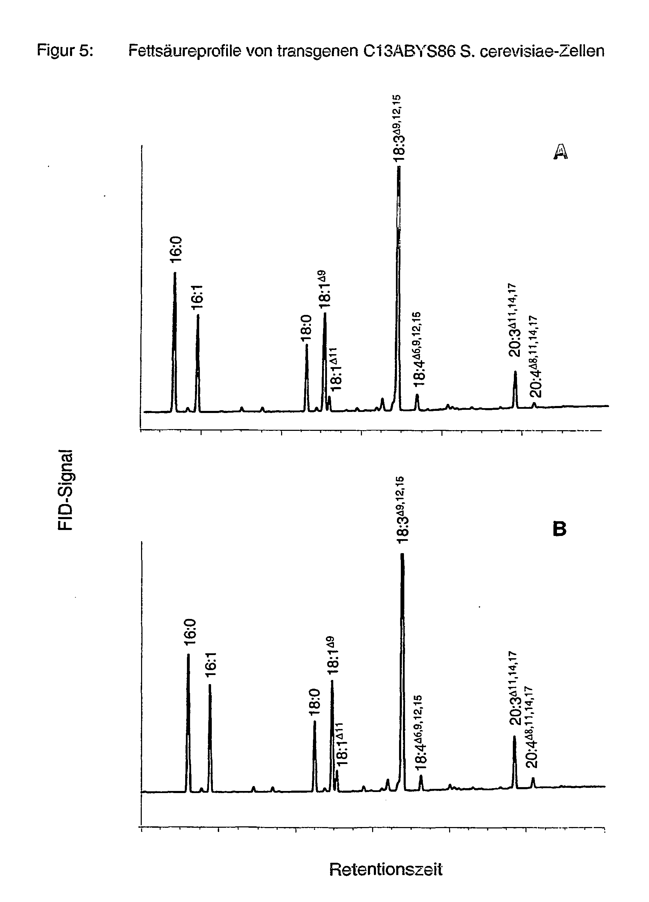

In den Kontroll-Hefen, die mit den Vektoren pESCLeu-PpD6-Pse1/pYes2 transformiert wurden, ist der Anteil von 20:3^{Δ8,11,14}, zu dem 18:3^{Δ6,9,12} durch Pse1 elongiert wird, wesentlich niedriger als in den Hefen, die zusätzlich die LPLAT T06E8.1 exprimieren. Tatsächlich konnte die Elongation von 18:3^{Δ6,9,12} und 18:4^{Δ6,9,12,15} durch die zusätzliche Expression von CeLPLAT (T06E8.1) um 100-150% verbessert werden (Figur 4). Diese signifikante Erhöhung des LCPUFA-Gehalts ist nur wie folgt zu erklären: die exogen gefütterten Fettsäuren (18:2^{Δ9,12} bzw. 18:3^{Δ9,12,15}) werden zunächst in Phospholipide eingebaut und dort von der Δ-6-Desaturase zu 18:3^{Δ6,9,12} und 18:4^{Δ6,9,12,15} desaturiert. Erst nach Reäquilibrierung mit dem Acyl-CoA-Pool können 18:3^{Δ6,9,12} und 18:4^{Δ6,9,12,15} durch die Elongase zu 20:3^{Δ8,11,14}- bzw. 20:4^{Δ8,11,14,17}-CoA elongiert und dann wieder in die Lipide eingebaut werden. Die LPLAT T06E8.1 ist in der Lage, die Δ6-desaturierten Acylgruppen sehr effizient in CoA-Thioester zurückzuverwandeln. Interessanterweise konnte auch die Elongation der gefütterten Fettsäuren 18:2^{Δ9,12} und 18:3^{Δ9,12,15} verbessert werden. (Figur 2 A und B bzw. 5 A und B).

Figur 5 gibt die Fettsäureprofile von transgenen C13ABYS86 *S*. *cerevisiae*-Zellen. Die Synthese der Fettsäuremethylester erfolgte durch saure Methanolyse intakter Zellen, die entweder mit den Vektoren pESCLeu-PpD6-Pse1/pYes2 (**A**) oder pYes2-T06E8.1/pESCLeu-PpD6-Pse1 (**B**) transformiert worden waren. Die Hefen wurden in Minimalmedium in Gegenwart von 18:3^{Δ9,12,15} kultiviert. Anschließend wurden die Fettsäuremethylester über GLC analysiert.

Die Expression einer anderen CeLPLAT (F59F4.4) hat dagegen keinen Einfluss auf die Elongation (Figur 4). Offenbar kodiert F59F4.4 nicht für eine LPLAT. Nicht jede der putativen LPLAT Nukleinsäuresequenzen ist also enzymatisch aktiv.

Figur 4 gibt die Elongation exogen applizierter 18:2^{Δ9,12} bzw. 18:3^{Δ9,12,15} im Anschluss an ihre endogene Δ-6-Desaturierung (Daten aus Fig. 2 und 5) wieder. Die exogen gefütterten Fettsäuren werden zunächst in Phospholipide eingebaut und dort zu 18:3^{Δ6,9,12} und 18:4^{Δ6,9,12,15} desaturiert. Erst nach Reäquilibrierung mit dem Acyl-CoA-Pool können 18:3^{Δ6,9,12} und 18:4^{Δ6,9,12,15} durch die Elongase zu 20:3^{Δ8,11,14}- bzw. 20:4^{Δ8,11,14,17}-CoA elongiert und dann wieder in die Lipide eingebaut werden. Die LPLAT T06E8.1 ist in der Lage, die Δ-6-desaturierten Acylgruppen effizient in CoA-Thioester zurückzuverwandeln.

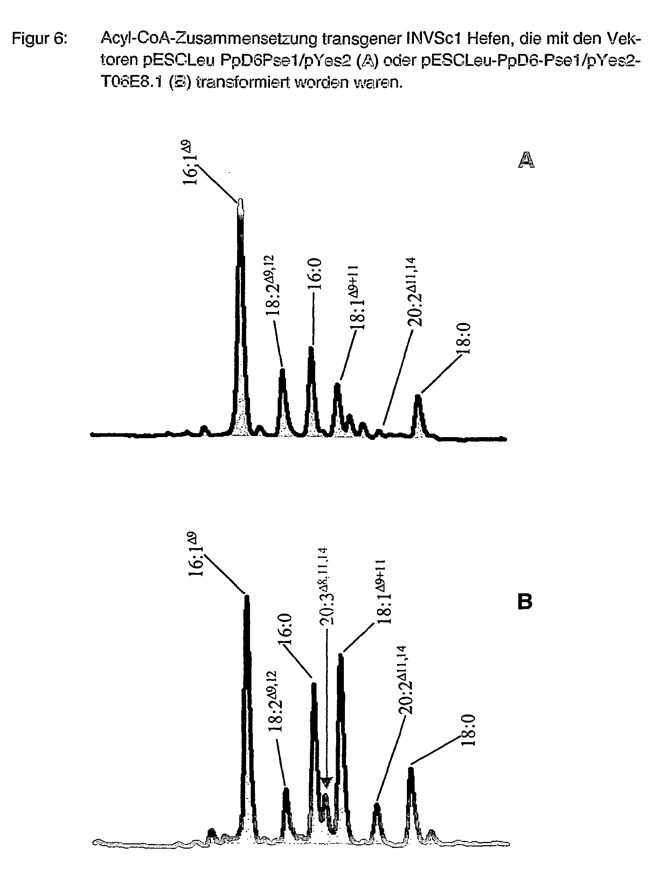

Diese Ergebnisse zeigen, dass die CeLPLAT (T06E8.1) nach Co-expression mit der Δ-6-Desaturase und Δ-6-Elongase zu einer effizienten Produktion von C20-PUFAs führt. Diese Ergebnisse sind dadurch zu erklären, dass die CeLPLAT (T06E8.1) einen effizienten Austausch der neusynthetisierten Fettsäuren zwischen Lipiden und dem Acyl-CoA-Pool ermöglicht (siehe Figur 6).

Figur 6 gibt die Acyl-CoA-Zusammensetzung transgener INVSc1 Hefen, die mit den Vektoren pESCLeu PpD6Pse1/pYes2 (**A**) oder pESCLeu-PpD6-Pse1/pYes2-T06E8.1 (**B**) transformiert worden waren, wieder. Die Hefezellen wurden in Minimalmedium ohne Uracil und Leucin in Gegenwart von 250 µM 18:2^{□Δ9,12} kultiviert. Die Acyl-CoA-Derivate wurden über HPLC analysiert.

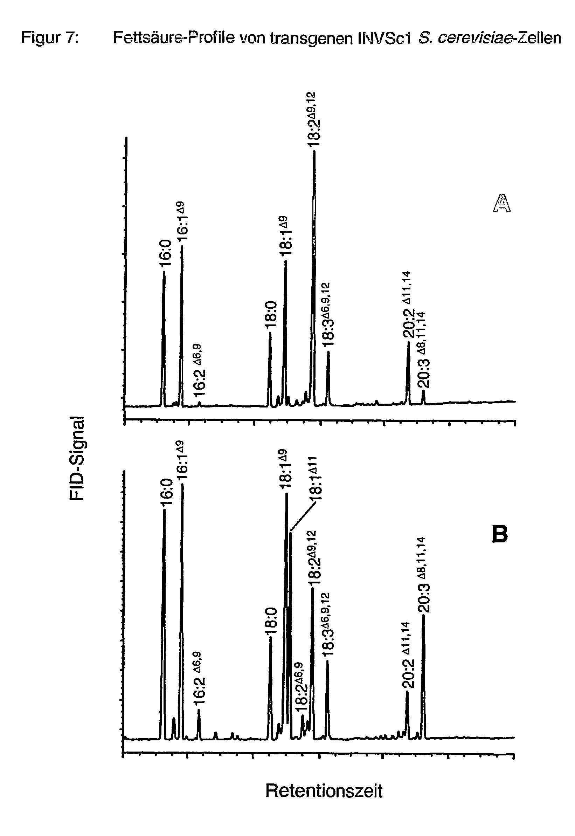

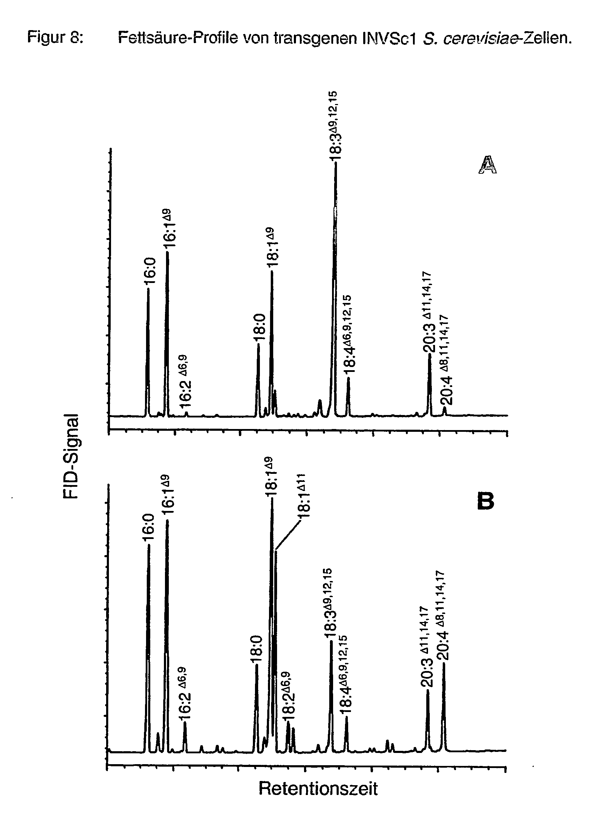

Bei Verwendung des Hefe-Stammes INVSc1 zur Co-Expression von CeLPLAT (T06E8.1) zusammen mit PpD6 und Pse1 ergibt sich folgendes Bild: Kontrollhefen, die PpD6 und Pse1 exprimieren, enthalten wie schon bei Verwendung des Stammes C13ABYS86 gezeigt nur geringe Mengen des Elongationsprodukts (20:3^{Δ8,11,14} bei Fütterung von 18:2 bzw. 20:4^{Δ8,11,14,17} bei Fütterung von 18:3; siehe Figur 7 A und 8 A). Bei zusätzlicher Expression von CeLPLAT (T06E8.1) erfolgt ein deutlicher Anstieg dieser Elongationsprodukte (siehe Figur 7 B und 8 B). Tabelle 5 zeigt, dass die zusätzliche Expression von CeLPLAT überraschenderweise eine 8-fache Erhöhung des Gehaltes an 20:3^{Δ8,11,14} (bei Fütterung von 18:2) bzw. 20:4^{Δ8,11,14,17} (bei Fütterung von 18:3) bewirkt. Daneben zeigt sich, dass auch C16:2^{Δ6,9} zu C18:2^{Δ6,9} effizienter elongiert wird.

Figur 7 ist das Fettsäure-Profile von transgenen INVSc1 S. cerevisiae-Zellen zu entnehmen. Die Synthese der Fettsäuremethylester erfolgte durch saure Methanolyse intakter Zellen, die entweder mit den Vektoren pESCLeu-PpD6-Pse1/pYes2 (A) oder pYes2-T06E8.1/pESCLeu-PpD6-Pse1 (B) transformiert worden waren. Die Hefen wurden in Minimalmedium in Gegenwart von 18:2^{Δ9,12} kultiviert. Anschließend wurden die Fettsäuremethylester über GLC analysiert.

Figur 8 gibt die Fettsäure-Profile von transgenen INVSc1 *S. cerevisiae*-Zellen wieder. Die Synthese der Fettsäuremethylester erfolgte durch saure Methanolyse intakter Zellen, die entweder mit den Vektoren pESCLeu-PpD6-Pse1/pYes2 (**A**) oder pYes2-T06E8.1/pESCLeu-PpD6-Pse1 (**B**) transformiert worden waren. Die Hefen wurden in Minimalmedium in Gegenwart von 18:3^{Δ,12,15} kultiviert. Anschließend wurden die Fettsäuremethylester über GLC analysiert.

**Tabelle 5: Fettsäure-Zusammensetzung (in mol %) transgener Hefen, die mit den Vektoren pESCLeu PpD6Pse1/pYes2 (PpD6 Pse1) oder pESCLeu-PpD6-Pse1/pYes2-T06E8.1 (PpD6 Pse1 + T06E8) transformiert worden waren. Die Hefezellen wurden in Minimalmedium ohne Uracil und Leucin in Gegenwart von 250 µM 18:2 ^{Δ9,12} oder 18:3□^{Δ9,12,15} kultiviert. Die Fettsäuremethylester wurden durch saure Methanolyse ganzer Zellen gewonnen und über GLC analysiert. Jeder Wert gibt den Mittelwert (n = 4) ± Standardabweichung wieder.**

| | Fütterung mit 250 µM 18:2^{Δ9,12} | | Fütterung mit 250 µM 18:3^{Δ9,12,15} | |
|---|---|---|---|---|
| Fettsäuren | PpΔ6/Pse1 | PpΔ6/Pse1+ T06E8 | PpΔ6/Pse1 | PpΔ6/Pse1+ T06E8 |
| 16:0 | 15,31 ± 1,36 | 15,60 ± 1,36 | 12,20 ± 0,62 | 16,25 ± 1,85 |
| 16:1 Δ9 | 23,22 ± 2,16 | 15,80 ± 3,92 | 17,61 ± 1,05 | 14,58 ± 1,93 |
| 18:0 | 5,11 ± 0,63 | 7,98 ± 1,28 | 5,94 ± 0,71 | 7,52 ± 0,89 |
| 18:1^{Δ9} | 15,09 ± 0,59 | 16,01 ± 2,53 | 15,62 ± 0,34 | 15,14 ± 2,61 |
| 18:1^{Δ11} | 4,64 ± 1,09 | 11,80 ± 1,12 | 4,56 ± 0,18 | 13,07 ± 1,66 |
| 18:2 ^{Δ9,12} | 28,72 ± 3,25 | 14,44 ± 1,61 | - | - |
| 18:3^{Δ6,9,12} | 3,77 ± 0,41 | 4,72 ± 0,72 | - | - |
| 18:3^{Δ9,12,15} | - | - | 32,86 ± 1,20 | 14,14 ± 2,52 |
| 18:4^{Δ6,9,12,15} | - | - | 5,16± 1,04 | 3,31 ± 1,15 |
| 20:2 ^{Δ11,14} | 2,12 ± 0,86 | 4,95 ± 4,71 | - | - |
| 20:3^{Δ8,11,14} | 1,03 ± 0,14 | 8,23 ± 1,59 | - | - |
| 20:3^{Δ11,14,17} | - | - | 4,12 ± 1,54 | 6,95 ± 2,52 |
| 20:4^{Δ8,11,14,17} | - | - | 1,34 ± 0,28 | 8,70 ± 1,11 |

Ein Maß für die Effizienz der LCPUFA-Biosynthese in transgener Hefe stellt der Quotient aus Gehalt der erwünschten Δ-6-Elongationsprodukt nach Δ-6-Desaturierung (20:3^{Δ8,11,14} bzw. 20:4^{Δ8,11,14,17}) zu Gehalt an zugefütterter Fettsäure (18:2^{Δ9,12} bzw. 18:3^{Δ9,12,15}) dar. Dieser Quotient beträgt 0,04 in INVSc1 Kontrollhefen, die PpD6 und Pse1 exprimieren, und 0,60 in Hefen die zusätzlich zu PpD6 und Pse1 CeLPLAT exprimieren. In anderen Worten: der Gehalt an erwünschtem Δ-6-Elongationsprodukt nach Δ-6-Desaturierung bei Co-Expression von CeLPLAT beträgt 60% des Gehalts der jeweils zugefütterten Fettsäure. In Kontrollhefen beträgt dieser Gehalt nur ca. 4%. Dies bedeutet eine 15-fache Erhöhung der Effizienz der LCPUFA-Biosynthese in transgener Hefe durch Co-Expression von LPLAT.

Interessanterweise bewirkt die Co-Expression von CeLPLAT nicht nur eine Erhöhung der genannten Elongationsprodukte 20:3^{Δ8,11,14} bzw. 20:4^{Δ8,11,14,17}, sondern auch eine Erhöhung des Verhältnisses 20:3^{Δ8,11,14} : 20:2^{Δ11,14} bzw. 20:4^{Δ8,11,14,17} : 20:3^{Δ11,14,17}. Dies bedeutet, dass in Anwesenheit der LPLAT die Δ-6-Elongase bevorzugt mehrfach ungesättigte Fettsäuren (18:3^{Δ6,9,12} und 18:4^{Δ6,9,12,15}) als Substrat verwendet, während bei Abwesenheit der LPLAT keine ausgeprägte Substratspezifität zu erkennen ist (auch 18:2^{Δ9,12} und 18:3^{Δ9,12,15} werden elongiert). Grund hierfür können Protein-Protein-Wechselwirkungen zwischen Δ-6-Elongase, Δ-6-Desturase und LPLAT oder posttranslationale Modifikationen (z.B. partielle Proteolyse) sein. Dies würde auch erklären, warum der oben beschriebene Anstieg von Δ-6-Elongationsprodukten bei Co-Expression von Δ-6-Desaturase, Δ-6-Elongase und LPLAT bei Verwendung eines protease-defizienten Hefestamms geringer ausfällt.

Acyl-CoA Analysen von transgenen INVSc1 Hefen, die mit 18:2^{Δ9,12} gefüttert wurden, ergaben folgendes Ergebnis: in Kontrollhefen, die PpD6 und Pse1 exprimieren, ist kein 18:3^{Δ6,9,12}-CoA und 20:3^{Δ8,11,14}-CoA nachweisbar. Dies weist darauf hin, dass weder das Substrat (18:3^{Δ6,9,12}-CoA) noch das Produkt (20:3^{Δ8,11,14}-CoA) der Δ-6-Elongase in Kontrollhefen in nachweisbaren Mengen vorhanden ist. Dies lässt darauf schließen, das der Transfer von 18:3^{Δ6,9,12} aus Membranlipiden in den Acyl-CoA Pool nicht oder nicht richtig stattfindet. Das bedeutet, dass kaum Substrat für die vorhandene Δ-6-Elongase zur Verfügung steht, was wiederum den geringen Gehalt an Elongationsprodukt in Kontrollhefen erklärt. INVSc1 Hefen, die zusätzlich zur PpD6 und Pse1 die CeLPLAT exprimieren und mit 18:2^{Δ9,12} gefüttert worden waren, weisen keine signifikanten Mengen an 18:3^{Δ6,9,12}-CoA auf, wohl aber 20:3^{Δ8,11,14}-CoA. Dies deutet darauf hin, dass LPLAT sehr effizient 18:3^{Δ6,9,12} aus den Membranlipiden in den Acyl-CoA-Pool überführt. 18:3^{Δ6,9,12}-CoA wird dann von der Δ-6-Elongase elongiert, so dass kein 18:3^{Δ6,9,12}-CoA, wohl aber 20:3^{Δ8,11,14}-CoA nachweisbar ist.

### b) Funktionelle Charakterierung der CeLPLATs in transgenen Pflanzen

### Expression funktionaler CeLPLAT in transgenen Pflanzen

In DE 102 19 203 wurden transgene Pflanzen beschrieben, deren Samenöl durch samenspezifische Expression funktioneller Gene kodierend für Δ-6-Desaturase, Δ-6-Elongase und Δ-5-Desaturase geringe Mengen an ARA und EPA enthält. Der zur Transformation dieser Pflanzen benutzte Vektor ist SEQ ID NO: 56 zu entnehmen. Um den Gehalt an diesen LCPUFAs zu erhöhen, wurde in den genannten transgenen Pflanzen zusätzlich das Gen CeLPLAT (T06E8.1) in Samen exprimiert.

Zu diesem Zweck wurde der kodierende Bereich von CeLPLAT über PCR amplifiziert.

In Tabelle 6 sind die Primer wiedergegeben, die zur Klonierung eines weiteren Clones der ceLPLAT in binäre Vektoren verwendet wurden.

**Tabelle 6: Nukleotidsequenzen der PCR-Primer zur Klonierung von CeLPLAT (T06E8.1) in den binären Vektor pSUN3**

| Primer | Nukleotidsequenz |
|---|---|
| ARe503f* | 5' TTAAGCGCGGCCGCATGGAGAACTTCTGGTCG 3' |
| ARe504r* | 5' ACCTCGGCGGCCGCCCTTTTACTCAGATTTC 3' |

| | |
|---|---|
| * f: forward, r: reverse | |

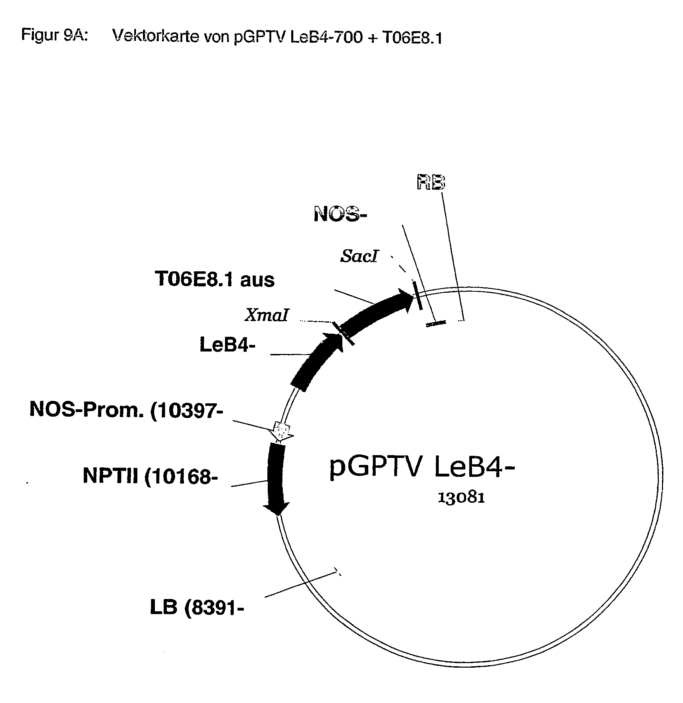

Das PCR-Produkt wurde in einen pENTRY Vektor zwischen USP Promotor und OCS-Terminator kloniert. Anschließend wurde die Expressionskassette in die binären Vektoren pSUN300 kloniert. Der entstandene Vektor wurde mit pSUN3CeLPLAT (Figur 1) bezeichnet. Darüber hinaus wurde der kodierende Bereiche von CeLPLAT amplifiziert und zwischen LegB4 Promotor und OCS-Terminator kloniert. Dieser Vektor wurde mit pGPTVCeLPLAT bezeichnet (Figur 9A).

Darüberhinaus wurde der kodierende Bereich von CeLPLAT über PCR amplifiziert und zwischen LegB4 Promotor und OCS-Terminator kloniert. Die hierfür verwendeten PCR Primer wurden so ausgewählt, dass in das PCR-Produkt eine effiziente Kosaksequenz eingeführt wurde. Außerdem wurde die DNA-Sequenz von CeLPLAT so verändert, dass sie der codon usage von höheren Pflanzen angepasst war.

Folgende Primer wurden für die PCR verwendet:
Forward primer: 5'-ACATAATGGAGAACTTCTGGTCTATTGTTGTGTTTTTTCTA-3'
Reverse primer: 5'- CTAGCTAGCTTACTCAGATTTCTTCCCGTCTTTTGTTTCTC-3'

Das PCR Produkt wurde in den Klonierungsvektor pCR Script kloniert und über die Restriktionsenzyme Xmal und SacI in den Vektor pGPTV LegB4-700 kloniert. Das entstandene Plasmid wurde mit pGPTV LegB4-700 + T06E8.1 bezeichnet (Figur 9A).

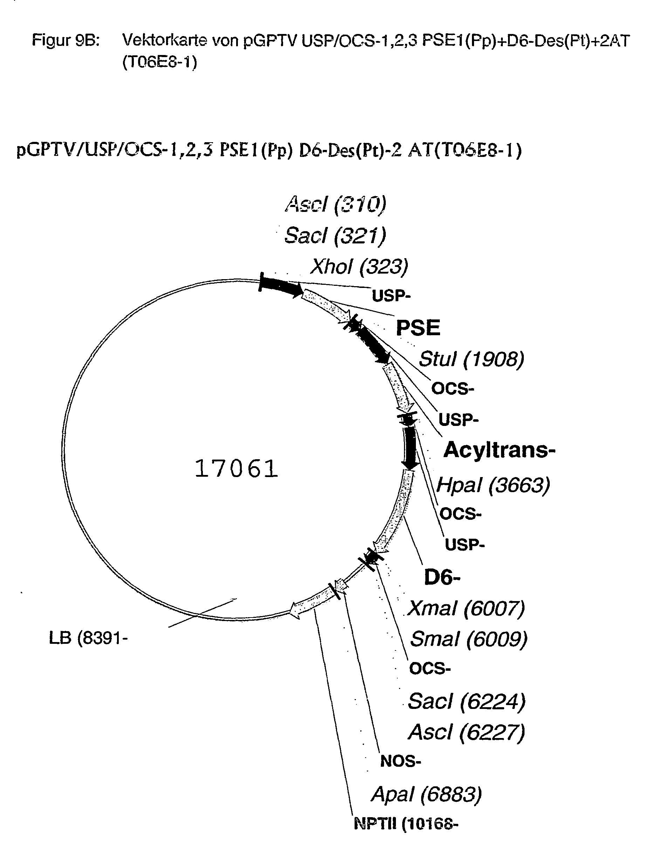

Das gleiche PCR Produkt wurde darüber hinaus in einen Multigen-Expressionsvektor kloniert, der bereits die Gene für eine Delta-6-Desaturase aus Phaeodactylum tricornutum (SEQ ID NO: 69, Aminosäuresequenz SEQ ID NO: 70) und einer Delta-6-Elongase aus P. patens enthielt. Das entstandene Plasmid wurde mit pGPTV USP/OCS-1,2,3 PSE1 (Pp)+D6-Des(Pt)+2AT (T06E8-1) bezeichnet (Figur 9B). Die Sequenzen des Vektors sowie der Gene sind SEQ ID NO:.71, SEQ ID NO: 72,

SEQ ID NO: 73 und SEQ ID NO: 74 zu entnehmen. Die Δ-6-Desaturase aus Phaeodactylum tricornutum reicht von Nukleotid 4554 bis 5987 in der SEQ ID NO: 71. Die Δ-6-Elongase aus Physcomitrella patens reicht von Nukleotid 1026 bis 1898 und die der LPLAT aus Caenorhabditis elegans reicht von Nukleotid 2805 bis 3653 in der SEQ ID NO: 71.

Tabakpflanzen wurden co-transformiert mit dem Vektor pSUN3CeLPLAT und dem in DE 102 19 203 und SEQ ID NO: 56 beschriebenen Vektor enthaltend Gene kodierend für Δ-6-Desaturase, Δ-6-Elongase und Δ-5-Desaturase, wobei die Selektion transgener Pflanzen mit Kanamycin erfolgte.

Tabakpflanzen wurden außerdem transformiert mit dem Vektor pGPTV USP/OCS-1,2,3 PSE1(Pp)+D6-Des(Pt)+2AT (T06E8-1) [siehe SEQ ID NO:.71, SEQ ID NO: 72, SEQ ID NO: 73 und SEQ ID NO: 74].

Lein wurde mit dem Vektor pSUN3CeLPLAT transformiert. Die entstandenen transgenen Pflanzen wurden mit solchen transgenen Leinpflanzen gekreuzt, die bereits geringe Mengen an ARA und EPA aufgrund der funktionellen Genexpression von Δ-6-Desaturase, Δ-6-Elongase und Δ-5-Desaturase enthielten.

Weiterhin wurde Lein mit dem Vektor pGPTV LegB4-700 + T06E8.1 transformiert. Die entstandenen transgenen Pflanzen wurden mit solchen transgenen Leinpflanzen gekreuzt, die bereits geringe Mengen an ARA und EPA aufgrund der funktionellen Expression von Δ-6-Desaturase, Δ-6-Elongase und Δ-5-Desaturase enthielten.

Die Samen von transgenen Tabak- und Leinpflanzen wurden wie weiter vorne beschrieben [Beispiel 3 b)] auf erhöhte Gehalte an LCPUFAs in untersucht.

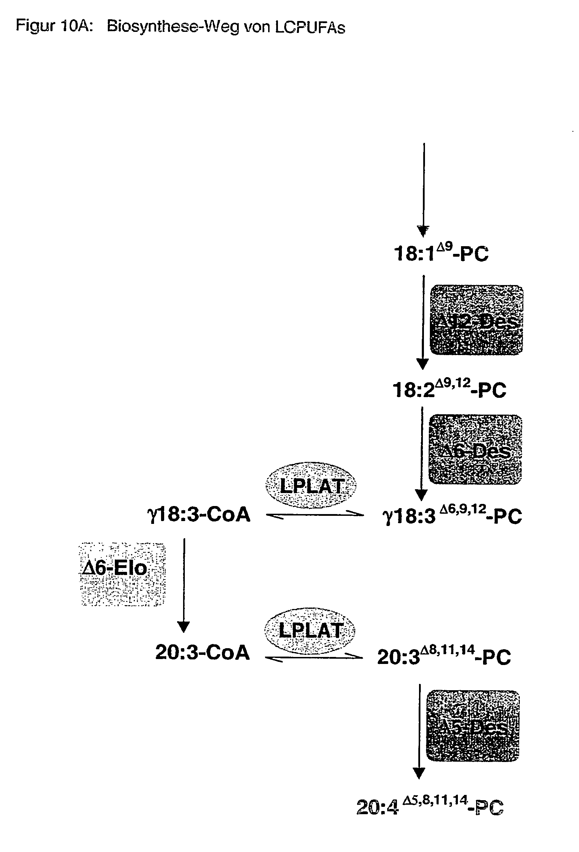

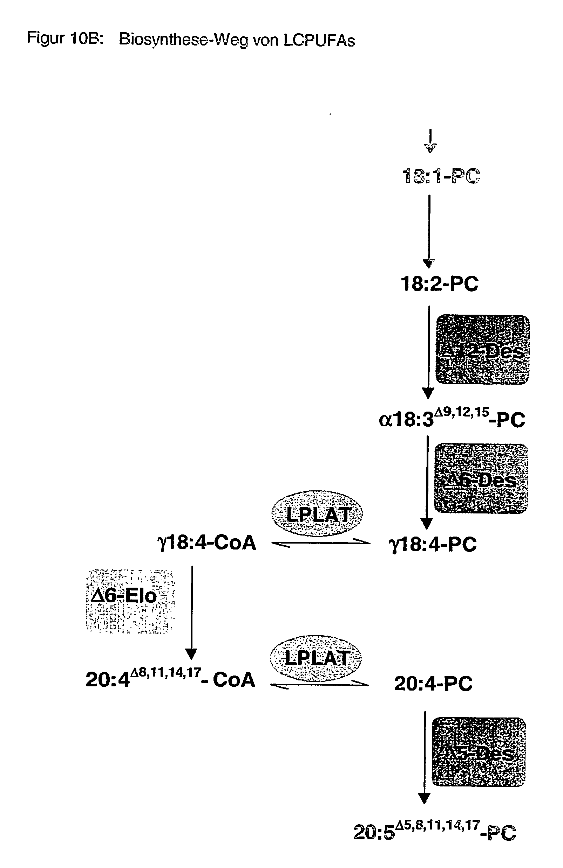

Aus den hier vorliegenden Arbeiten lässt sich die Funktion der Acyl-CoA:Lysophopholipid-Acyltranserase (LPLAT) wie in Figur 10 A und 10 B dargestellt ableiten. Der Biosynthese-Weg der LCPUFAS stellt sich damit wie folgt dar.

Desaturasen katalysieren die Einführung von Doppelbindungen in lipidgekoppelte Fettsäuren (sn2-Acyl-Phosphatidylcholin), während die Elongasen exklusiv die Elongation Coenzym A-veresterter Fettsäuren (Acyl-CoAs) katalysieren. Nach diesem Mechanismus erfordert die alternierende Wirkung von Desaturasen und Elongasen einen ständigen Austausch von Acyl-Substraten zwischen Phospholipiden und Acyl-CoA-Pool und somit die Existenz einer zusätzlichen Aktivität, die die Acyl-Substrate in die jeweils notwendige Substratform, d.h. Lipide (für Desaturasen) oder CoA-Thioester (für Elongasen), überführt. Dieser Austausch zwischen Acyl-CoA Pool und Phospholipiden wird durch LCPUFA-spezifische LPLAT ermöglicht. Die Biosynthese von ARA (**A**) erfolgt analog zu EPA (**B**), mit dem Unterschied, dass bei EPA der Δ-6-Desaturierung eine Δ-15-Desaturierung vorgeschaltet ist, so dass α18:3-PC als Substrat für die Δ-6-Desaturase fungiert. Die Biosynthese von DHA macht einen weiteren Austausch zwischen Phospholipiden und Acyl-CoA-Pool über LPLAT notwendig: 20:5^{Δ5,8,11,14,17} wird vom Phospholipid- zum CoA-Pool transferiert und nach erfolgter Δ-5-Elongation wird 22:5^{Δ7,10,13,16,19} vom CoA- zum Phospholipid-Pool transferiert und schließlich durch Δ-4-Desaturase zu DHA umgesetzt. Gleiches gilt für den Austausch im Biosyntheseweg unter Verwendung der Δ-8-Desaturase, der Δ-9-Elongase und der Δ-5-Desaturase.

### Beispiel 5: Funktionelle Charakterierung der Acyltransferasen

Um die Substratspezifität von Acyltransferasen höherer Pflanzen und LCPUFA-produzierender Organismen zu vergleichen, wurden aus dem LCPUFA-produzierenden Organismus Mortierella alpina und aus Sonnenblume mikrosomale Fraktionen isoliert. Die GPAT- und LPAAT-Aktivitäten wurden mit verschiedenen Acyl-CoAs als Substrat getestet.

Um zu überprüfen, ob der LCPUFA-Produzent Thraustochytrium tatsächlich DHA in der sn-2 Position der Lipide einbaut, wurde eine Positionsanalyse der Lipide durchgeführt.

Um LCPUFA-spezische Acyltransferasen zu isolieren, wurde ausgehend von mRNA der LCPUFA-produzierenden Organismen Thraustochytrium, Physcomitrella, Cryptecodinium cohnii und Fusarium cDNA-Banken, sowie einer Shewanella genomischen Bank erstellt und diese über DNA-Sequenzierung näher analysiert. Über Sequenzhomologien wurden Acyltransferaseklone identifiziert. Alternativ wurden über PCR-Techniken Acyltransferasen amplifiziert.

Transgene E. coli Zellen, Hefen, Insektenzellen und Pflanzenzellen mit erhöhter Expression mindestens einer LCPUFA-spezifischen Acyltransferase weisen einen erhöhten Gehalt an LCPUFAs in ihren Lipiden auf.

### Beispiel 6: Isolierung mikrosomaler Fraktionen aus Mortierella, Sonnenblume und Leinsamen und Analyse der Substratspezifität von Acyltransferasen für verschiedene Acyl-CoAs.

Um herauszufinden, ob höhere Pflanzen, insbesondere Ölsaaten wie Sonnenblume, Lein, Raps oder Soja LCPUFAs in ihre Lipide einbauen können, wurden aus Sonnenblume und Leinsamen Mikrosomen präpariert und verschiedene Acyltransferase-Aktivitäten hinsichtlich ihrer Substratspezifität für LCPUFA-CoAs untersucht. Im einzelnen wurden GPAT-, LPAAT- und LPCAT-Aktivitäten untersucht. Diese Ergebnisse wurden verglichen mit den entsprechenden Acyltransferase-Aktivitäten der LCPUFA-Produzenten Mortierella alpina, der bekanntermaßen hohe Gehalte der LCPUFA Arachidonsäure in seinen Lipiden und im Triacylglycerin enthält (C. Ming et al. (1999) Bioresource Technology 67: 101-110).

### Präparation mikrosomaler Membranen aus Cotyledonen reifender Samen von Sonnenblume und Lein

Alle Arbeiten wurden bei 4°C durchgeführt. Die Cotyledonen von reifenden Sonnenblumen- und Leinsamen wurden ungefähr 10 Tage nach Anthesis geerntet und in 0.1 M Natriumphosphatpuffer (pH 7,2), der 0,33 M Saccharose und 0,1 % BSA (fettsäurefrei) enthielt, suspendiert. Nach Zerkleinerung in einem Glashomogenisator wurde das Homogenat bei 20.000 x g, 30 Minuten lang zentrifugiert. Der Überstand wurde durch eine Lage Miracloth filtriert und bei 100.000 x g in einer Ultrazentrifuge 90 Minuten lang zentrifugiert. Die pelletierten mikrosomalen Membranen wurden mit 0,1 M Natriumphosphatpuffer (pH 7,2) gewaschen und in einem kleinen Volumen Puffer resuspendiert, wobei ein Glashomogenisator verwendet wurde. Die mikrosomalen Membranpräparationen wurden entweder sofort weiterverwendet oder bei -80°C gelagert.

### Präparation mikrosomaler Membranen aus Mortierella

Kulturen von Mortierella wurden nach 5 Tagen geerntet und auf Eis gestellt. Alle weiteren Arbeiten wurden bei 4°C ausgeführt. Das Mycelium wurde in 0,1 M Natriumphosphatpuffer (pH 7,2), der 0,33 M Saccharose, 0,1 % BSA (fettsäurefrei), 1000 units Katalase/ml und 1 mM Pefabloc enthielt, suspendiert. Die nachfolgenden Schritte wurden wie unter ,Präparationen mikrosomaler Membranen aus Cotyledonen reifender Samen von Sonnenblume und Lein' beschrieben durchgeführt.

### Acyl-CoA Substratspezifität von GPAT: Umsetzung einzelner Acyl-CoA Substrate in der Acylierung von [¹⁴C] Glycerin-3-phosphat

Die Spezifität der GPAT wurde untersucht, um zu überprüfen ob das Enzym eine Präferenz für bestimmte Acyl-CoAs hat, insbesondere, um zu ermitteln, ob die GPAT von Ölsaaten LCPUFA-CoAs umsetzt. Mikrosomale Membranen wurden inkubiert mit 0,5 mM (Mortierella) bzw. 0,2 mM (Sonnenblume und Leinsamen) eines der folgenden Acyl-CoAs: Myristoyl-CoA (14:0-CoA), Palmitoyl-CoA (16:0-CoA), Palmitoleoyl-CoA (16:1-CoA), Stearoyl-CoA (18:0-CoA), Oleoyl-CoA (18:1-CoA), Linoleoyl-CoA (18:2-CoA), Dihomo-gamma-linolenoyl-CoA (20:3-CoA) oder Arachidonyl-CoA (20:4-CoA) und 5 mM [¹⁴C] G3P. Mikrosomale Membranen (äquivalent 50 µg Protein bei Sonnenblume und Mortierella bzw. 150 µg Protein bei Leinsamen) wurden dem Reaktionsgemisch zugesetzt, um die Reaktion zu starten. Nach 5 Minuten Inkubationszeit wurden die Lipide nach Bligh & Dyer extrahiert und die in komplexen Lipiden eingebaute Radioaktivität bestimmt.

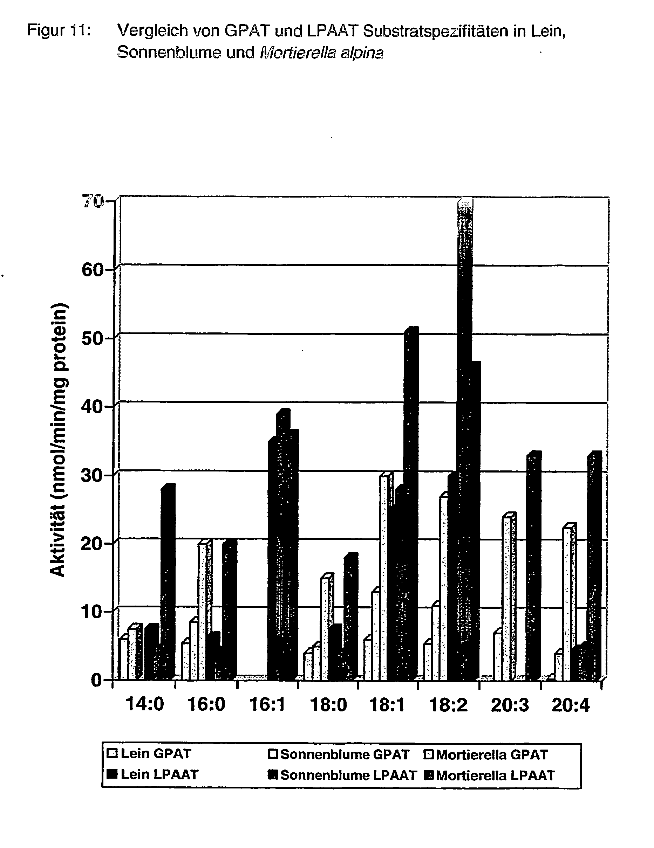

In Figur 11 und Tabelle 7a und 7b sind die GPAT-Aktivitäten von Mortierella, Sonnenblume und Leinsamen bei verschiedenen Acyl-CoA-Sustraten dargestellt.

Die GPAT von Mortierella baut ungesättigte Fettsäuren effizienter ein als gesättigte Fettsäuren. Oleat und Linoleat wurden mit ähnlichen Einbauraten umgesetzt (100% bzw. 90%). Der Einbau von polyungesättigten Fettsäuren (20:3-CoA und 20:4-CoA) war nur unwesentlich niedriger (80% bzw. 75%).

In mikrosomalen Membranen von Sonnenblume sind ebenfalls Oleat und Linoleat die besten Substrate für die GPAT (100% bzw. 85% Aktivtät). Acyl-CoAs der gesättigten Fettsäuren Stearat und Palmitat werden nur ca. halb so gut eingebaut (40% bzw. 64%). Ähnliches gilt für 20:3-CoA (55%). Arachidonyl-CoA ist für GPAT von Sonnenblume ein relativ schlechtes Substrat (23%).

Die GPAT in mikrosomalen Membranen von Leinsamen hat die niedrigste spezifische Aktivität aller untersuchten GPAT-Enzyme. Mit 6 nmol/min/mg Protein ist sie nur halb so aktiv wie Sonnenblumen GPAT und 5 mal weniger aktiv als das Enzym aus Mortierella. Bezüglich der Substratspezifitäten verhält sich Die effizientesten Acyl-CoA-Substrate der GPAT aus Leinsamen sind wie bei Sonnenblume Oleat und Linoleat (100% bzw. 90%). Die Einbauraten der gesättigten Fettsäuren Stearat und Palmitat sind mit 65% und 90% deutlich höher als bei Sonnenblume. Arachidonyl-CoA hingegen ist für GPAT von Leinsamen ein äußerst schlechtes Substrat (5%).

### Acyl-CoA Substratspezifität von LPAAT: Umsetzung einzelner Acyl-CoA Substrate bei der Acylierung von Lysophosphatidsäure

Die Spezifität der LPAAT wurde untersucht, um zu überprüfen, ob das Enzym eine Präferenz für bestimmte Acyl-CoAs hat, insbesondere, um zu ermitteln, ob die LPAAT von Ölsaaten LCPUFA-CoAs umsetzt. LPAAT Aktivität wurde in einem kontinuierlichen spektralphotometrischen Assay bestimmt, bei dem 5,5-dithio-bis-2-nitrobenzoat (DTNB) verwendet wurde, und die Änderung der Absorption bei 409 nm und 25°C verfolgt wurde (F.M. Jackson et al. (1998) Microbiology 144: 2639-2645). Der Assay enthielt sn-1-Oleoyl-Lysophosphatidsäure (30 nmol), DTNB (50 nmol) und 20 nmol eines der folgenden Acyl-CoAs: Palmitoyl-CoA (16:0-CoA), Stearoyl-CoA (18:0-CoA), Oleoyl-CoA (18:1-CoA), Linoleoyl-CoA (18:2-CoA), Dihomo-gamma-linolenyl-CoA (20:3-CoA) oder Arachidonyl-CoA (20:4-CoA) in 1 ml 0,1 M Phosphatpuffer, pH 7,2. Das in der Reaktion freigesetzte CoA wurde mit Hilfe der Anfangssteigung und des Extinktionskoeffizienten von 13,6 mM-1 x cm-1 quantifiziert. Mikrosomale Membranen (äquivalent 10 µg Protein bei Mortierella bzw. 40 µg Protein bei Sonnenblume und Leinsamen) wurden dem Reaktionsansatz zugesetzt, um die Reaktion zu starten.

In Figur 11 und Tabelle 7a und 7b sind die LPAAT-Aktivitäten von Mortierella, Sonnenblume und Leinsamen bei verschiedenen Acyl-CoA-Sustraten dargestellt.

Die LPAAT von Mortierella baut Oleoyl-CoA am effizientesten ein (100 %). Linoleoyl-CoA wird ebenfalls sehr gut umgesetzt (90 %). Die gesättigten Fettsäuresubstrate 16:0-CoA und 18:0-CoA werden zu nur 40 % bzw. 36 % eingebaut, die LCPUFA-Substrate 20:3-CoA und 20:4-CoA hingegen mit einer relativ hohen Effizienz (je 65 %).

In mikrosomalen Membranen von Sonnenblume ist Linoleoyl-CoA das am effizientesten in Phosphatidsäure eingebaute Substrat der LPAAT (250 % relativ zu Oleoyl-CoA). Sowohl gesättigte als auch polyungesättigte Acyl-CoA waren schlechte Substrate für Sonnenblumen LPAAT (relative Aktivtäten kleiner 20 %).

Ein ganz ähnliches Bild ergibt sich für LPAAT aus Leinsamen: Linoleoyl-CoA stellt das beste Substrat dar (120% relativ zu Oleoyl-CoA). Gesättigte Fettsäuren sind schlechte LPAAT-Substrate (25% und 30% für 16:0-CoA und 18:0-CoA). Arachidonyl-CoA wird am schlechtesten umgesetzt (19% relative Aktivität).

### Acyl-CoA Substratspezifität von LPCAT: Umsetzung einzelner Acyl-CoA Substrate bei der Acylierung von Lysophosphatidylcholin

In höheren Pflanzen und Pilzen werden Fettsäuren zur Herstellung polyungesättigter Fettsäuren desaturiert, während sie mit Phosphatidylcholin (PC) verestert sind (A.K. Stobart und S. Stymne (1985) Planta 163: 119-125; F.M. Jackson et al. (1998) Microbiology 144: 2639-2645). Die Beteiligung von PC bei der Desaturierung von Fettsäuren auch in Pilzen setzt voraus, dass es ein funktionierendes Transfersystem von Fettsäuren zu und von der sn-2-Position des PC gibt, ähnlich dem, wie es für entwickelnde Ölsamen beschrieben wurde (Jackson et al., 1998; Stobart et al., 1983). Es wird vermutet, dass dieser Transfer des Acylgruppe von Acyl-CoA zur sn-2 Position des PC durch LPCAT katalysiert wird. Hier wurde die Spezifität von LPCAT untersucht, um zu überprüfen, ob das Enzym eine Präferenz für bestimmte Acyl-CoAs hat, insbesondere, um zu ermitteln, ob die LPCAT von Ölsaaten LCPUFA-CoAs umsetzt.

LPCAT Aktivität wurde in einem kontinuierlichen spektralphotometrischen Assay bestimmt, bei dem 5,5-dithio-bis-2-nitrobenzoat (DTNB) verwendet wurde und die Änderung der Absorption bei 409 nm und 25°C verfolgt wurde. Der Assay enthielt sn-1-Palmitoyl-Lysophosphatidylcholin (30 nmol) als Acyl-Akzeptor, DTNB (50 nmol) und 20 nmol eines der folgenden Acyl-CoAs: Myristoyl-CoA (14:0-CoA), Palmitoyl-CoA (16:0-CoA), Palmitoleoyl-CoA (16:1-CoA), Stearoyl-CoA (18:0-CoA), Oleoyl-CoA (18:1-CoA), Linoleoyl-CoA (18:2-CoA), Dihomo-gamma-linolenoyl-CoA (20:3-CoA) oder Arachidonyl-CoA (20:4-CoA) in 1 ml 50 mM Phosphatpuffer, pH 7,2. Die Reaktion wurde durch Zugabe mikrosomaler Membranpräparation gestartet. Die Menge zugegebener mikrosomaler Membranenpräparation betrug 5 µg (Mortierella und Sonnenblume) bzw. 30 µg (Leinsamen). Das in der Reaktion freigesetzte CoA wurde mit Hilfe der Anfangssteigung und des Extinktionskoeffizienten von 13,6 mM-1 x cm-1 bei 409 nm quantifiziert.

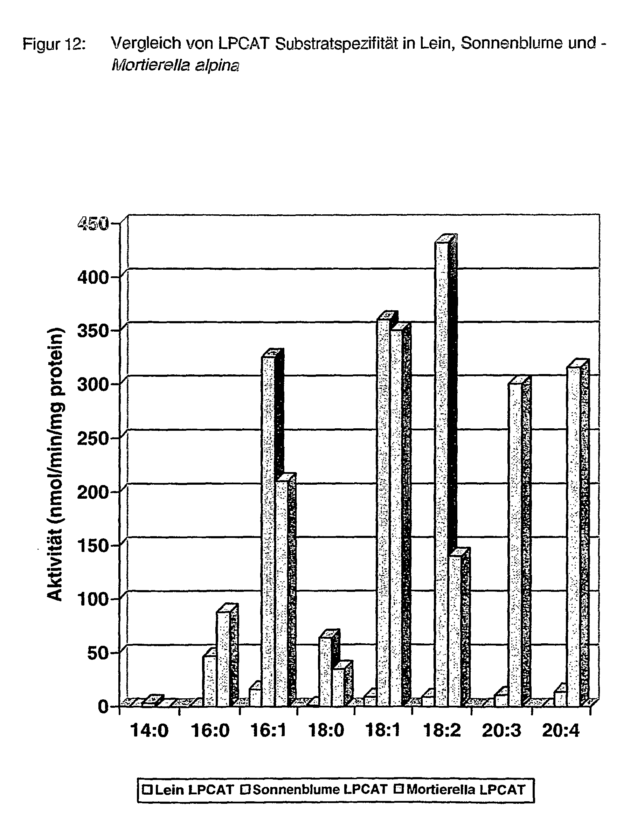

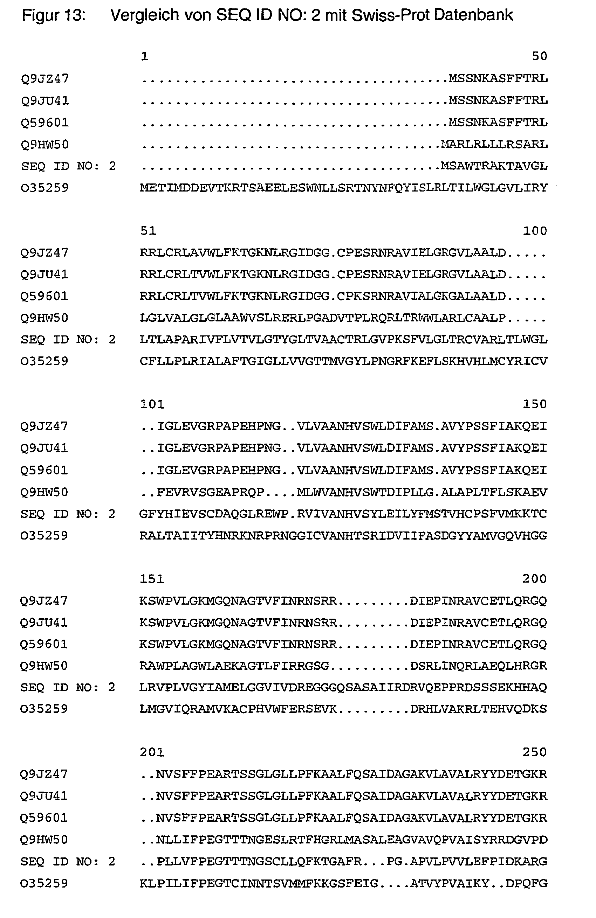

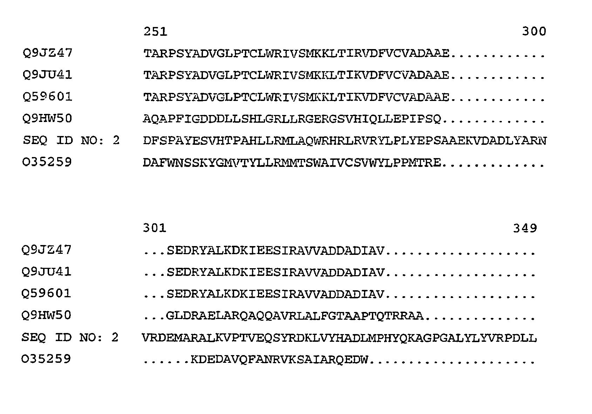

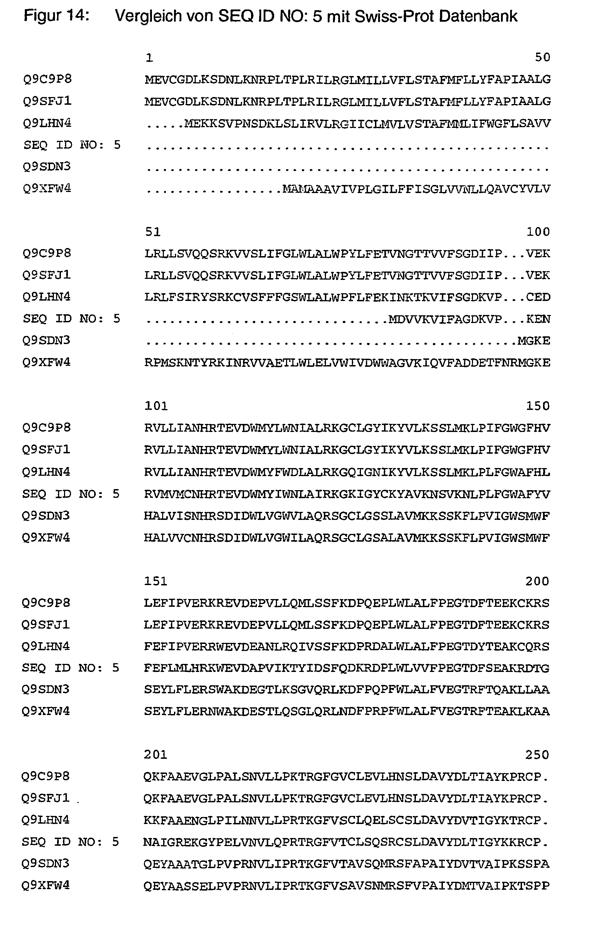

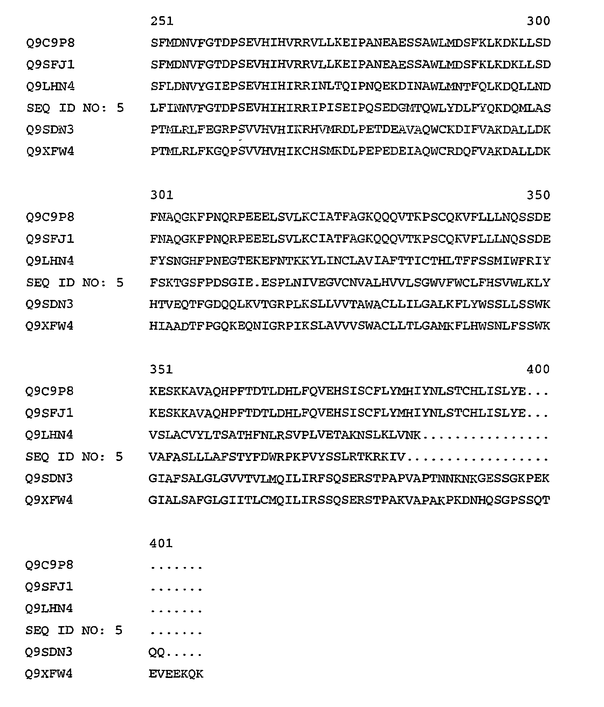

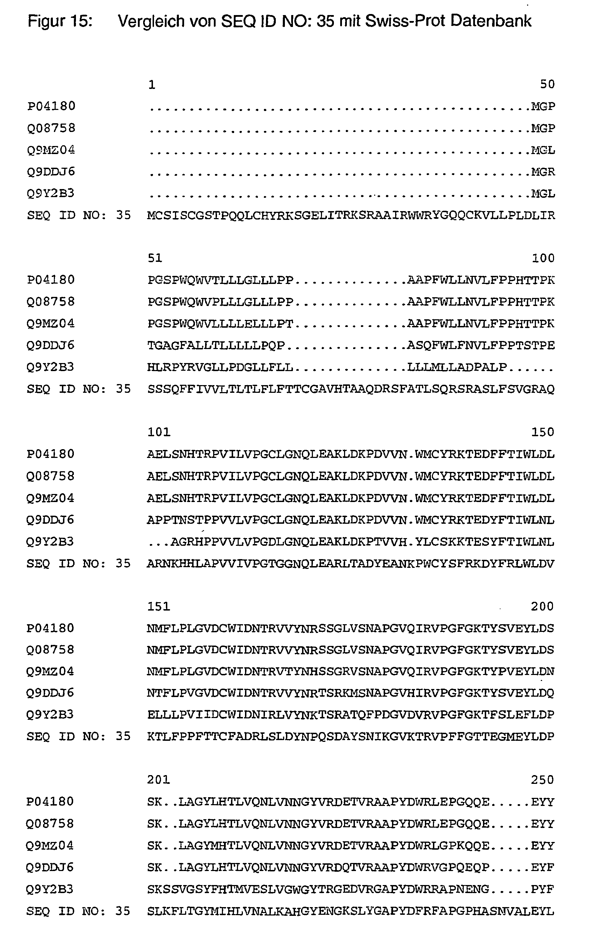

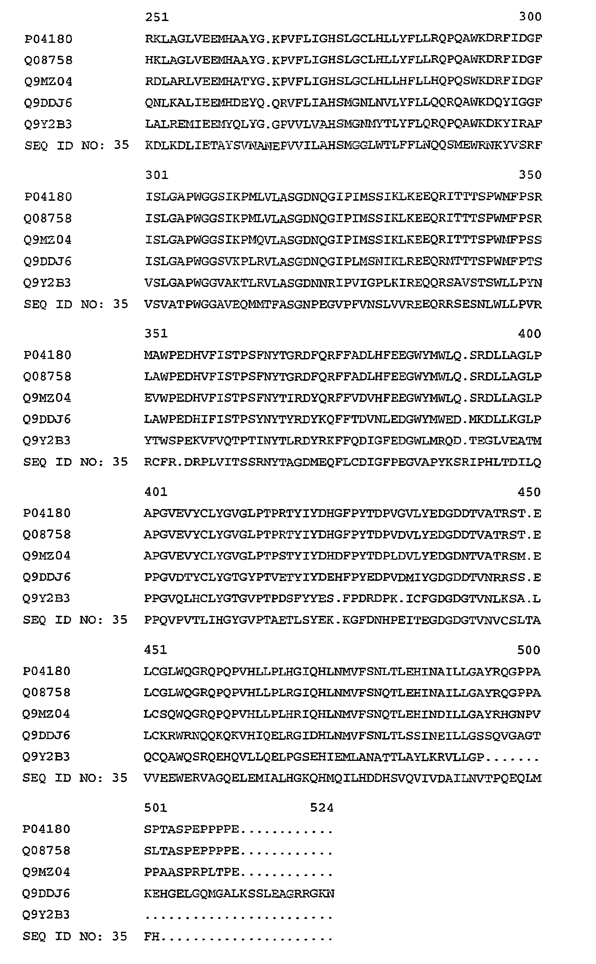

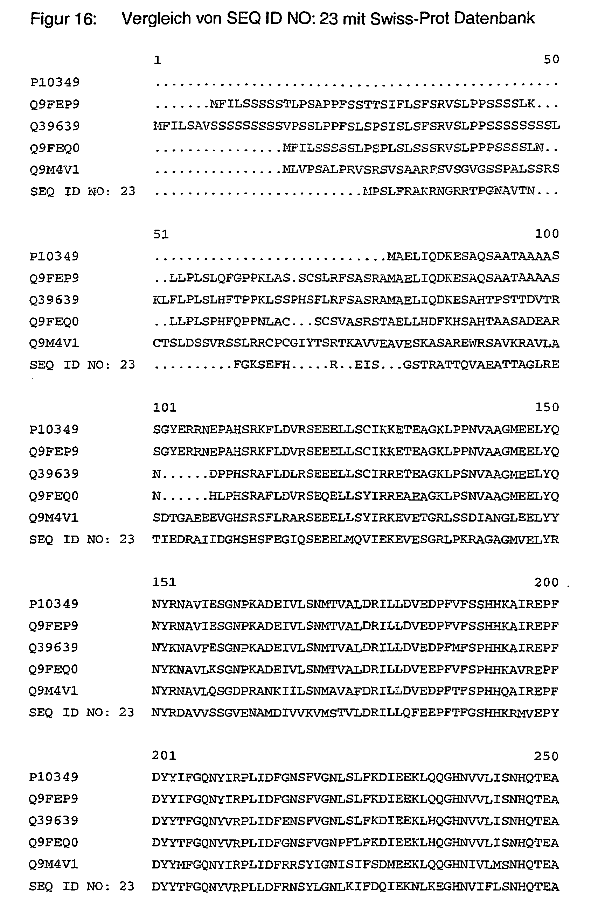

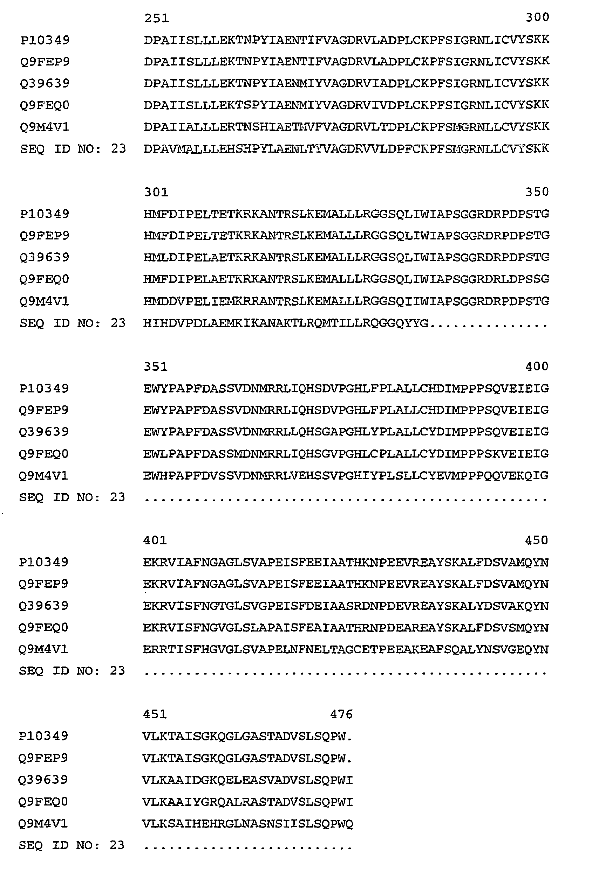

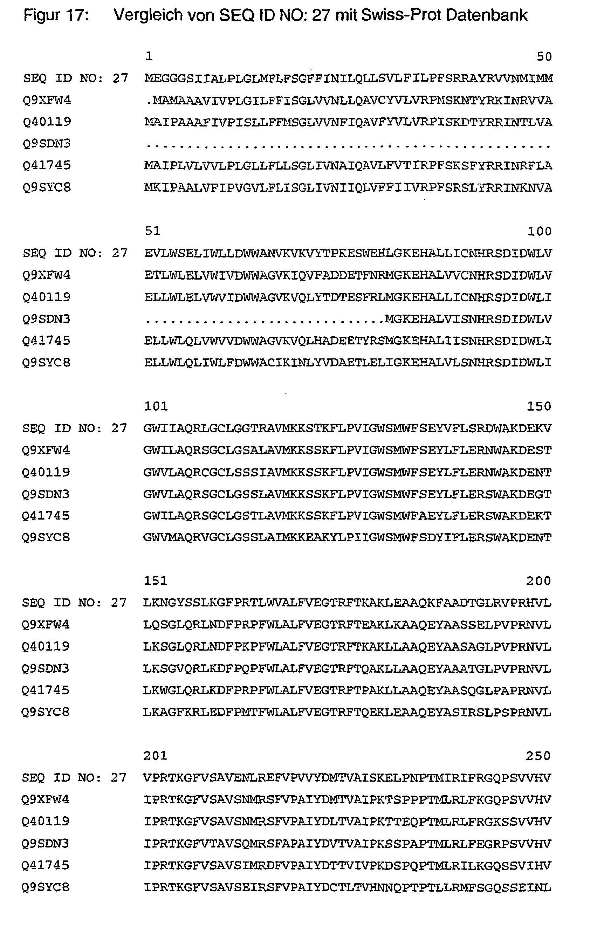

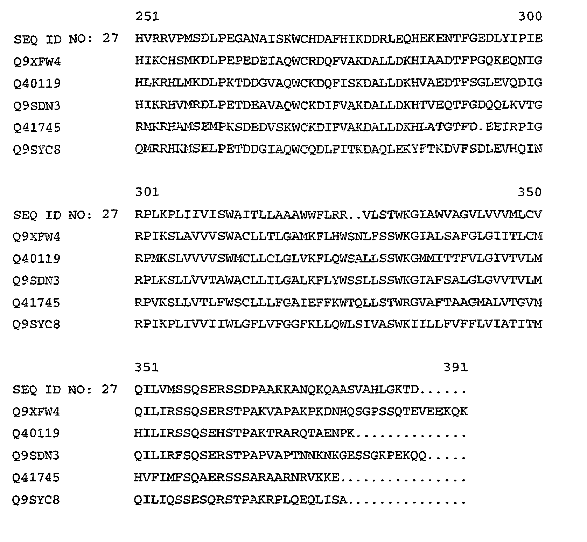

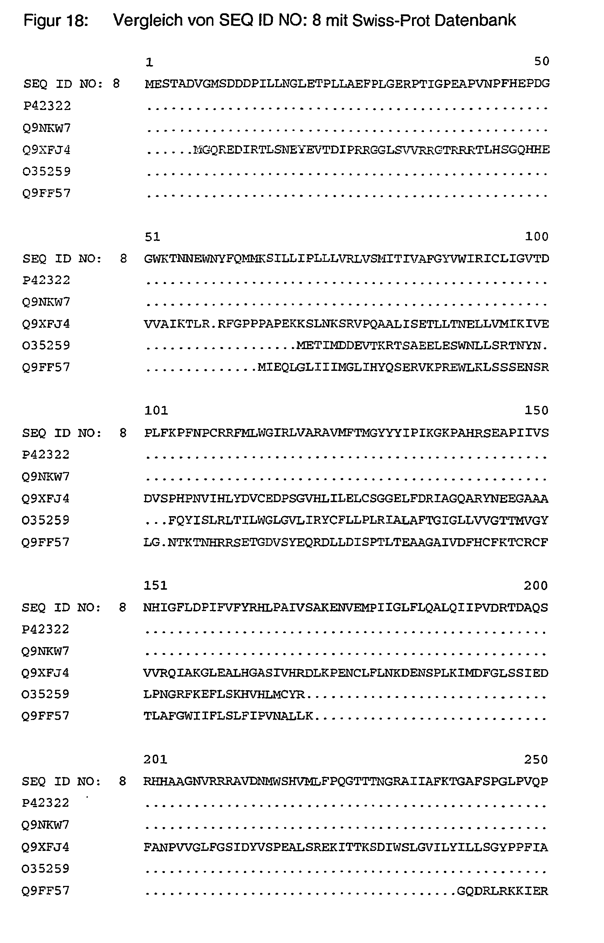

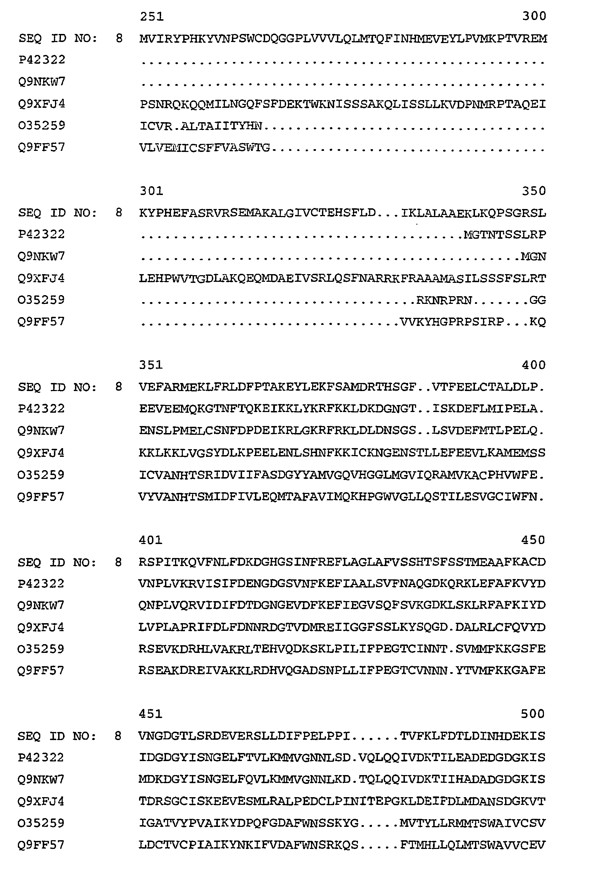

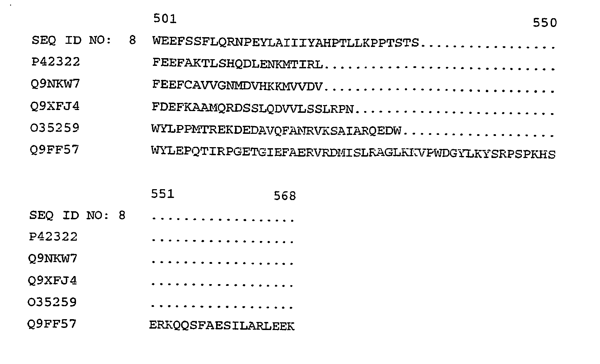

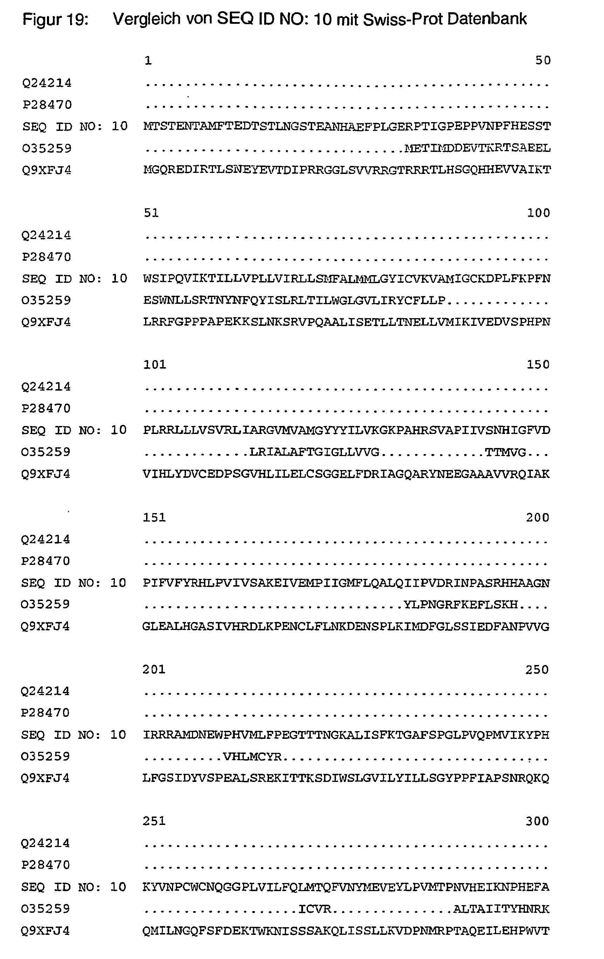

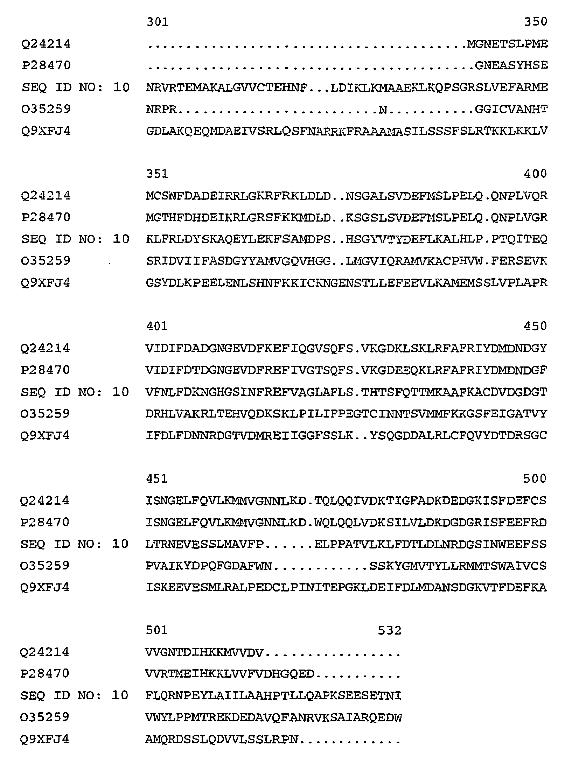

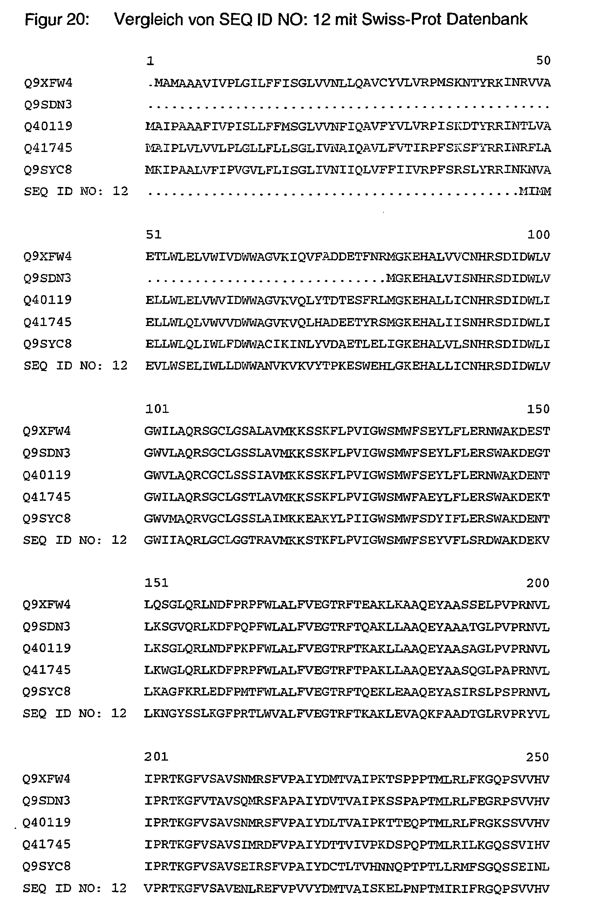

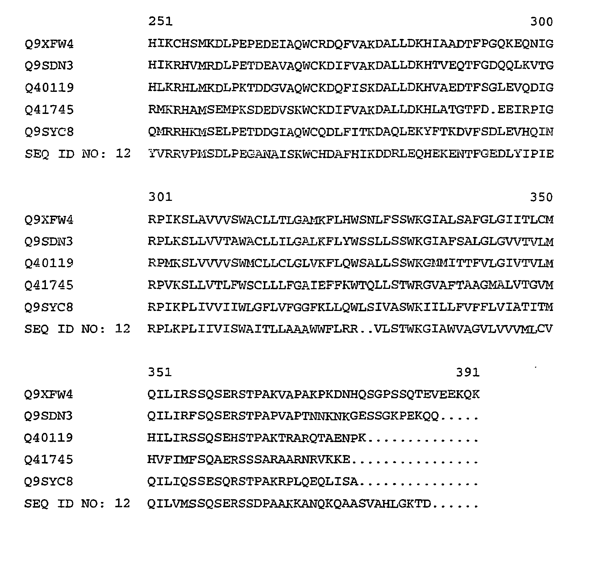

In Figur 12 und Tabelle 7a und 7b sind die LPCAT-Aktivitäten von Mortierella, Sonnenblume und Leinsamen bei verschiedenen Acyl-CoA-Sustraten dargestellt.

Die Ergebnisse zeigen, dass LPCAT in mikrosomalen Membranen von Sonnenblume und Mortierella wesentlich aktiver ist als bei Leinsamen (siehe Tab. 10a und 10b). Mortierella LPCAT setzt neben 18:1 (100 %) auch 18:2 (40%), 20:3 (85 %) und 20:4 (90%) sehr effizient um. Gesättigte Fettsäuren werden quasi nicht umgesetzt (relative Aktivität kleiner 25 %).

Sonnenblumen LPCAT setzt Oleoyl-CoA und Linoeoyl-CoA ähnlich gut um (100 % bzw. 120 % relative Aktivitäten). Palmitoyl-CoA und Stearoyl-CoA sind schlechte Substrate (relative Aktivität kleiner 20 %). 20:3-CoA und 20:4-CoA werden quasi nicht umgesetzt (relative Aktivitäten kleiner 5 %).

Ähnlich verhält sich LPCAT aus Leinsamen: Oleoyl-CoA und Linoleoyl-CoA werden gleichermaßen gut umgesetzt, hingegen konnte für 20:3-CoA und 20:4-CoA keine LPCAT-Aktivität nachgewiesen werden.

### Diskussion der Daten zur Acyl-CoA-Spezifität von GPAT, LPAAT und LPCAT

Die Substratspezifität von G3P acylierenden Enzymen wurde intensiv untersucht, um den Mechanismus der Verteilung von Fettsäuren in Phospholipiden und Triacylglycerin zu verstehen. Mikrosomale GPAT von Säugetieren verwendet gesättigte und ungesättigte Acyl-CoAs (Yamada & Okuyama, 1978; Haldar et al., 1979; Tamai & Lands, 1974). Gleiches wurde für pflanzliche mikrosomale GPATs gezeigt (Frentzen, 1993; Bafor et al. 1990). Jackson et al. (1998) zeigten außerdem, dass weder GPAT noch LPAAT des Pilzes Mucor circinelloides eine ausgeprägte Substratspezifität für Acyl-CoAs aufweist. Gesättigte wie ungesättigte Fettsäuren werden bei Mucor an beiden Positionen acyliert. Eine gereinigte GPAT der Membranfraktion von Mortierella ramanniana zeigte jedoch eine klare Präferenz für Oleoyl-CoA gegenüber Palmitoyl-CoA (Mishra & Kamisaka, 2001).

Um zu untersuchen, ob GPAT in mikrosomalen Membranen von Mortierella, Sonnenblume und Leinsamen eine starke Spezifität für bestimmte Acyl-CoA Spezies aufweist, wurden einzelne Acyl-CoAs den Mikrosomen zugesetzt. Die GPAT von Mortierella weist insofern Ähnlichkeit zu anderen pflanzlichen, tierischen und pilzlichen GPATs auf, als sie eine breite Spezifität für Acyl-CoAs hat, d.h. gesättigte und ungesättigte Fettsäuren werden an der sn-1 Position von G3P acyliert. Auch die GPATs von Sonnenblumen und Leinsamen mikrosomalen Membranen verwenden gesättigte und ungesättigte Acyldonatoren, in ähnlicher Weise, wie dies für Färberdistel und Turnip rape (Bafor et al., 1990) gezeigt wurde, allerdings mit einer Präferenz für ungesättigte Fettsäuren. Generell ist die Mortierella GPAT weniger diskriminierend wie das Sonnenblumen- und Leinsamenenzym. Auffällig ist allerdings, dass Sonnenblumen und Leinsamen GPATs Arachidonyl-CoA quasi gar nicht umsetzt, wogegen das Mortierella-Enzym Arachidonyl-CoA sehr effizient acyliert.

Im zweiten Acylierungsschritt ist LPAAT von Mortierella, Sonnenblume und Leinsamen aktiv mit sn-1-Oleoyl Lysophosphatidsäure als Acylakzeptor. Ähnlich der GPAT weist auch LPAAT von Mortierella eine breite Spezifität für Acyl-CoAs auf. Diese Daten sind ähnlich denen aus Meerschweinchen und Rattenleber Mikrosomen, wo mit Ausnahme von Stearoyl-CoA LPAAT alle Acyl-CoAs mit 16 und 18 C-Atomen, unabhängig vom Sättigungsgrad verestert (Hill und Lands, 1968). In der vorliegenden Arbeit zeigten die Sonnenblumen- und Leinsamen-LPAATs eine starke Spezifität zu Linoleat und Oleat. Gesättigte Fettsäuren hingegen wurden kaum umgesetzt. Diese Daten stimmen überein mit der Beobachtung, dass bei den meisten Ölsaaten LPAAT eine höhere Spezifität für ungesättigte Fettsäuren zeigen (Griffiths et al., 1985; Ichihara et al., 1987). Bei Sonnenblume und Leinsamen ist Arachidonyl-CoA auch für LPAAT ein schlechtes Substrat. Verglichen mit GPAT ist die LPAAT-Aktivität von Sonnenblume und Leinsamen aber etwas höher.

Die Spezifität von LPCAT in mikrosomalen Präparationen von Mortierella und Sonnenblume wurde ebenfalls untersucht. In Mortierella zeigte LPCAT ein breites Spektrum der Substratspezifität auf. Die Aktivität des Enzyms mit verschiedenen Acyl-CoAs nahm in der Reihenfolge 18:1-CoA > 20:4-CoA > 20:3-CoA > 16:1-CoA > 18:2-CoA ab. LPCAT aus Sonnenblume und Leinsamen zeigte kaum Aktivität mit 20:3 und 20:4-CoA.LPCAT in Rinderhirn-Mikrosomen zeigten auch eine schwache Aktivität mit gesättigten Acyl-CoAs und eine größere Aktivität mit Linoleoyl- und Oleoyl-CoA (Deka et al., 1986). LPCAT von Rinder-Herzmuskel-Mikrosomen akzeptieren einen großen Bereich von Substraten, obwohl die Aktivität besonders hoch mit Arachidonyl-, Linoleoyl- und Oleoyl-CoA-Substraten ist (Sanjawara et al., 1988). In Pflanzen wurde die Acyl-Spezifität und Selektivität von LPCAT in Mikrosomen von Färberdistel (Stymne et al., 1983; Griffith et al., 1985) und Leinsamen (Stymne & Stobart, 1985a) untersucht. Oleat und Linoleat wurden mit ungefähr der gleichen Umsatzrate an die sn-2-Position von PC acyliert. Die Aktivität mit alpha-Linoleat betrug nur etwa die Hälfte. Palmitat und Stearat waren wesentlich schlechtere LPCAT-Substrate, wenn sie als einzelne Acyl-CoAs angeboten wurden. Wurde eine Mischung aus gesättigten und ungesättigten Acyl-CoAs angeboten, so wurden Palmitat und Stearat vollständig vom PC ausgeschlossen. Auch LPCAT in mikrosomalen Membranen von Mucor circinelloides verwendet Oleoyl- und Linoeoyl-CoA wesentlich effizienter als gesättigte Fettsäuren. Es gibt also eine große Übereinstimmung bei der Spezifität von pflanzlicher, tierischer und pilzlicher LPCATs. Die Tatsache, dass LPCAT aus mikrosomalen Membranen von Mortierella nur eine schwache Aktivität mit Stearoyl-CoA und eine gute Aktivität mit Oleoyl- und Linoleoyl-CoA aufweist, könnte darauf hinweisen, dass Phosphatidylcholin als Substrat für Desaturasen dient. Es wurde demonstriert, dass Oleat an der sn-1 und der sn-2 Position von PC als Substrat für die Δ-12-Desaturase in Ölsaaten dient (Stymne & Stobart, 1986; Griffiths et al., 1988). Ähnliche Ergebnisse wurden für Mucor circinelloides berichtet (Jackson et al., 1998). Die Δ-6-Desaturase verwendet auch Linoleat and der sn-2 Position von PC in mikrosomalen Membranpräparationen von Mucor (Jackson et al., 1998). Auch die Δ-6-Desaturase von Borretsch verwendet ausschließlich Linoleat an der sn-2 Position des Phospholipids (Stymne & Stobart, 1986; Griffiths et al., 1988).

Die in Beispiel 6 beschriebenen Ergebnisse zeigen, dass Acyltransferasen von Sonnenblume und Lein LCPUFAs wie Dihomo-γ-Linolenat und Arachidonat nicht effizient in die Membran- und Speicherlipide einbauen können. Obwohl LCPUFAs in Ölsaaten wie Sonnenblume, Lein oder Soja produziert werden können, indem die entsprechenden Biosynthesegene funktional exprimiert werden, ist davon auszugehen, dass die gebildeten LCPUFAs aufgrund fehlender Acyltransferase-Aktivitäten nicht effizient in Triacylglycerin eingebaut werden, was zu einem niedrigen Ertrag führt. Zusätzlich zu LCPUFA-Biosynthesegenen (z.B. Desaturasen und Elongasen oder Polyketidsynthasen) müssen also Acyltransferasen mit einer hohen Spezifität für LCPUFA-CoAs in Ölsaaten transformiert werden. Hierfür eignen sich Acyltransferasen von LCPUFA-produzierenden Organismen wie Mortierella, Phaeodactylum, Crypthecodinium, Physcomitrella, Euglena und Thraustochytrium.

Tabelle 7a und 7b geben die Aktivität und Acyl-Spezifität von Lein, Sonnenblume und Mortierella alpina Acyltransferasen wieder.

**Tabelle 7a: Aktivität und Acyl-Spezifität von Lein- und Sonnenblume- Acyltransferasen**

| Enzymaktivität | Lein | | | Sonnenblume | | |
|---|---|---|---|---|---|---|
| | GPAT | LPAAT | LPCA T | GPAT | LPAAT | LPCA T |
| Rate (nmol/min/mg protein) des Ölsäure-Einbaus | 6 | 25 | 9 | 13 | 28 | 360 |
| | | | | | | |

| Prozentualer Einbau im Vergleich zum Ölsäureeinbau | | | | | | |
|---|---|---|---|---|---|---|
| Myristoyl-CoA | 100 | 30 | 0 | 57 | 16 | 1 |
| Palmitoyl-CoA | 90 | 25 | 5 | 64 | 15 | 13 |
| Palmitololeoyl-CoA | | 140 | 180 | | 140 | 90 |
| Stearoyl-CoA | 65 | 30 | 15 | 40 | 14 | 18 |
| Oleoyl-CoA | 100 | 100 | 100 | 100 | 100 | 100 |
| Linoleoyl-CoA | 90 | 120 | 100 | 85 | 250 | 120 |
| 20:3-CoA | | | 0 | 55 | | 3 |
| Arachidonoyl-CoA | 5 | 19 | 0 | 23 | 18 | 4 |

**Tabelle 7b: Aktivität und Acyl-Spezifität von Mortierella alpina -Acyltransferasen**

| Enzymaktivität | *Mortierella alpina* | | |
|---|---|---|---|
| | GPAT | LPAAT | LPCA T |
| Rate (nmol/min/mg protein) des Ölsäure-Einbaus | 30 | 51 | 350 |

| Prozentualer Einbau im Vergleich zum Olsäureeinbau | | | |
|---|---|---|---|
| Myristoyl-CoA | | 55 | 0 |
| Palmitoyl-CoA | 66 | 40 | 25 |
| Palmitololeoyl-CoA | | 70 | 60 |
| Stearoyl-CoA | 50 | 36 | 10 |
| Oleoyl-CoA | 100 | 100 | 100 |
| Linoleoyl-CoA | 90 | 90 | 40 |
| 20:3-CoA | 80 | 65 | 85 |
| Arachidonoyl-CoA | 75 | 65 | 90 |

### Beispiel 7: Positionsanalyse der Lipide von Thraustochytrium

In Beispiel 6 wurde gezeigt, dass LCPUFA-Produzenten wie Mortierella über membrangebundene Acyltransferase-Aktivitäten verfügen, die LCPUFA-CoAs in Membran- und Speicherlipide einbauen. Durch Positionsanalysen der Lipide von LCPUFA-Produzenten kann man Rückschlüsse auf die in-vivo-Aktivitäten der einzelnen Acyltransferasen ziehen. Daher wurde im folgenden untersucht, welche Fettsäuren an den einzelnen Positionen der Lipide des DHA-Produzenten Thraustochytrium verestert sind.

### a) Kultivierung von Thraustochytrium spec.(TS) ATCC 26185

Die Kultivierung des Pilzes TS erfolgte in TS-Flüssigkultur und durch Ausstreichen auf TS-Platten. Alle drei Wochen wurden die Pilze auf neue Platten überimpft, zwei Tage bei 28°C gelagert und anschließend bei RT (ca. 23°C) aufbewahrt. Die Flüssigkultur wurde bei 30°C unter Schütteln inkubiert und nach 6 Tagen geerntet. Das Schütteln der Kultur unter Lichteinstrahlung erhöht die Lipidausbeute (Daten nicht gezeigt).

### 1) TS-Medium: (Bajpai et al. (1991) JAOCS 68: 507-514)

### a ) 10x Lösung A (g/l):

| | |
|---|---|
| 250 g/l | NaCl |
| 50 g/l | MgSO₄7H₂O |
| 10 g/l | KCl |
| 20 g/l | Na-Glutamat |
| 2 g/l | (NH4)₂SO₄ |
| 20 g/l | Glucose |

Lösung autoklavieren.

### b) 10x Lösung B (g/l)

| | |
|---|---|
| 200 g/l | Glucose |
| 20 g/l | Hefeextrakt |

Lösung B wurde sterilfiltriert.

### c) 10x Lösung C (g/l)

| | |
|---|---|
| 2 g/l | CaCO₃ |

Zum Lösen des CaCO₃ wurde die Lösung mit HCl angesäuert und anschließend autoklaviert.

### d) 10x Lösung D (g/l)

| | |
|---|---|
| 1 g/l | KH₂PO₄ |
| 1 g/l | NaHCO₃ |

Die Lösung wurde autoklaviert.

### Suplemente: Thiamin und Vitamin B₁₂

Zu 600 ml autoklaviertem dest. Wasser wurde je 100 ml der 10x Lösungen a) bis d) und 10 µg/l Thiamin und 1 µg/l Vitamin B₁₂ zugegeben

### b) Lipidanalyse von Thraustochytrium (Bligh & Dyer (1959) Canadian J. Biochem. 37: 911-917)

Zur Extraktion der Gesamtlipide aus TS in Flüssigkultur wurden diese durch Zentrifugation bei 3000g für 10 Minuten sedimentiert. Nach Resuspension der Zellen in 10 ml 0,45% NaCl wurden diese für 10 Minuten im Wasserbad gekocht. Nach einem weiteren Zentrifugationsschritt (wie oben) der in 40 ml-Schliffgläschen umgefüllten Suspension wurde das Sediment in Trichlormethan/Methanol 1:2 (v/v) aufgenommen. Dabei richtete sich das Volumen des Lösungsmittelgemisches nach dem Volumen des Sedimentes. Im allgemeinen wurden für die Extraktion einer 100 ml-Kultur 10 ml des Gemisches benötigt. Die erste Extraktion fand für mindestens 6 Stunden, zumeist allerdings über Nacht bei 8°C auf einem Schüttler statt. Anschließend wurden die Zellreste erneut sedimentiert und der Überstand wurde bei 8°C aufbewahrt. Die zweite Extraktion fand entsprechend der Ersten, allerdings mit Trichlormethan/Methanol 2:1 (v/v) über Nacht statt. Nach der zweiten Extraktion wurden die Zellreste erneut sedimentiert und der Überstand wurde mit dem der ersten Extraktion vereinigt. Die vereinigten Extrakte wurden dann auf das Verhältnis Trichlormethan/Methanol/0,45 % NaCl 2:1:0,7 eingestellt und geschüttelt. Dabei werden nicht erwünschte, coextrahierte Substanzen wie Zucker ausgeschüttelt und gelangen in die wässrige Phase. Daraufhin wurde der Extrakt bis zur Phasentrennung zentrifugiert, die organische Unterphase abgenommen und zur Befreiung von Schwebstoffen durch Watte in einen Rundkolben filtriert. Der Lipidextrakt wurde am Rotationsverdamfer bis zur Trockene eingeengt, die Gesamtlipide wurden in Trichlormethan/Methanol 2:1 (v/v) und in ein Schliffglasröhrchen überführt. Dann wurde der Extrakt unter Stickstoff erneut bis zur Trockene eingeengt und abschließend in Trichlormethan/Methanol 2:1 (v/v) in einem definierten Volumen aufgenommen.

### c) Lipidanalyse aus Thraustochytrium-Membranen

Isolierte Thraustochytrium-Membranen wurden in ein Schliffröhrchen überführt und in 0,45% NaCl aufgenommen und im Wasserbad 5 Minuten lang aufgekocht, um lipidabbauende Enzyme zu inaktivieren. Nach Zentrifugation (5 Minuten, 3000 x g) wurde der wässrige Überstand dekantiert. Die Extraktion der Lipide erfolgte eine Stunde lang bei 4°C in Trichlormethan/Methanol (2:1). Nach Zugabe von 1/3 Volumen 0,45% NaCl wurden die Proben zur besseren Phasentrennung zentrifugiert (5 Minuten, 3000 x g). Die untere, lipidhaltige Phase wurde entnommen und unter Vakuum eingeengt. Die Lipide wurden in einem geeigneten Volumen Trichlormethan aufgenommen.

Im direkten Anschluß wurden die Lipide auf Kieselgelplatten (Kieselgel 60, 20 x 20 cm, 0,25 mm Schichtdicke; Merck, Darmstadt) zur dünnschicht-chromatographischen Trennung der Phospholipide mit geeigneten Standards aufgetragen. Als Laufmittel wurde Trichlormethan/Methanol/Eisessig/H₂0 91/30/4/4 (v/v/v/v) verwendet. Die Laufzeit betrug 1,5 Stunden. Nach Eindampfen des Lösungsmittels wurden die Platten mit 2',7'-Dichlorfluorescein (Merck, Darmstadt; in 0,3% iso-Propanol) angefärbt und unter UV-Licht (366 nm) sichtbar gemacht.

### d) Lipaseverdau der Thraustochytrium-Gesamtlipide

Der enzymatische Verdau erfolgt mittels Pankreaslipase (EC 3.1.1.3). Die hydrolytische Spaltung erfolgt an der Phasengrenze zwischen Fett und Wasser, wobei das Enzym in Triacylglycerolen (TAGs) spezifisch die randständigen Esterbindungen in sn-1 und sn-3-Position angreift. Intermediär werden 1,2- und 2,3-Diacyl-sn-glycerole angereichert, die anschließend zu sn-2 Monoacylglycerolen weiter verdaut werden. Nach dünnschichtchromatographischer Auftrennung und Gewinnung der sn-2 Monoacylglycerol-Fraktion wird die Fettsäure-Zusammensetzung der TAGs in der mittleren Position ermittelt.

In ein Glasschliffröhrchen wurden 50 mg des Gesamtlipides eingewogen. Nach Zusatz von 0,5 ml Tris-Puffer, 0,1 ml CaCl₂-Lösung und 0,25 ml Gallensalzlösung (0,05 % (w/v) Gallensalz; Sigma, Deisenhofen) wurde das Schliffröhrchen verschlossen. Das Gemisch wurde eine Minute lang durchmischt und anschließend eine Minute in einem Wasserbad bei 40°C vortemperiert, um die Probe zu emulgieren.

Die Hydrolyse erfolgte nach Zusatz von Pankreaslipase (EC 3.1.1.3; Sigma, Deisenhofen; 2 mg Lipase pro 5 mg Lipid; Lipase frisch gelöst in 0,5 ml Tris-Puffer) bei 38°C und hoher Schüttelfrequenz (möglichst 1200 U/min). Nach 30 Minuten wurde die Reaktion durch Zusatz von 1 ml HCl (6 N) und 1 ml Ethanol abgebrochen.

Das Reaktionsgemisch wurde im Zentrifugenglas 2 mal mit je 4 ml Dietylether extrahiert. Dabei wurde die obere etherische Phase abgenommen. Die verbleibende wässrige Phase wurde erneut mit Diethylether extrahiert. Die Entstehung von Emulsionen wurde bei jedem Extraktionsschritt zusätzlich durch Zentrifugation unterbunden. Die vereinigten etherischen Phasen wurden durch ausschütteln mit je 3 ml Wasser (dest.) gewaschen. Die organische Phase wurde in ein neues Röhrchen überführt und mit Natriumsulfat getrocknet. Nach 2-minütiger Zentrifugation bei 3000 x g wurde der klare Überstand abgenommen und das Natriumsulfatpellet erneut mit Diethylether ausgeschüttelt, wie oben angegeben zentrifugiert und die organischen Phasen vereinigt. Nach Einengung des Etherextraktes unter Vakuum wurde im direkten Anschluss der Extrakt auf Kieselgelplatten (Kieselgel 60, 20 x 20 cm, 0,25 mm Schichtdicke; Merck, Darmstadt) zur dünnschicht-chromatographischen Trennung der Partialglyceride aufgetragen. Als Laufmittel (mobile Phase) wurde Diisopropylether-Eisessig 40:1 (v/v) verwendet. Die Laufzeit betrug 35-45 Minuten. Nach Verflüchtigung des Lösungsmittels wurden die Platten mit 2',7'-Dichlorfluorescein (Merck, Darmstadt; in 0,3% iso-Propanol) angefärbt und unter UV-Licht sichtbar gemacht. Die einzelnen Lipidfraktionen wurden in folgender Reihenfolge aufgetrennt: Monoacylglycerole (sn-2 MAGs, unmittelbar über der Startlinie), Diacylglycerole (sn-1,2- und sn-2,3-DAGs) freie Fettsäuren (FFA) und die nicht umgesezten TAGs.

Die MAG-Bande wurde von der Kieselgelplatte abgekratzt. Die Bestimmung der Fettsäurezusammensetzung der TAGs erfolgte durch Transmethylierung und anschließender gaschromatographischer Auftrennung der Fettsäure-Methylester (FAME).

Tris-Puffer:
1 M Tris/HCl, pH mit HCl auf 8,0 einstellen
CaCl-Lösung
2,2% (w/v) CaCl₂

### e) Lipaseverdau der Thraustochytrium-Membranlipide (Fischer et al., 1973)

Die Positionsanlyse der Membranlipide erfolgte durch enzymatische Hydrolyse der sn-2-Esterbindung mit Phospholipase A₂ (EC 3.1.1.4).

Die isolierten Membranlipide wurden unter Vakuum eingeengt, mit 0,5 ml Hydrolysepuffer versetzt und 5 min lang mit dem Ultraschallstab dispergiert. Die Hydrolyse erfolgte bei RT nach Zugabe von 50 U der Phospholipase A₂. Die Reaktion wurde durch Zugabe von 4 ml Trichlormethan/Methanol 2:1 (v/v) und 0,45% NaCl gestoppt. Die organische Unterphase wurde in ein neues Gefäß überführt, am Rotationsverdampfer eingeengt und in 200 µl Trichlormethan/Methanol 2:1 (v/v) aufgenommen.

Im direkten Anschluss wurde der Ansatzt auf Kieselgelplatten (Kieselgel 60, 20 x 20 cm, 0,25 mm Schichtdicke; Merck, Darmstadt) zur dünnschicht-chromatographischen Trennung der Phospholipide aufgetragen. Als Laufmittel wurde Trichlormethan/Methanol/Eisessig/H₂0 91/30/4/4 (v/v/v/v) verwendet. Die Laufzeit betrug 1,5 Stunden. Nach Eindampfen des Lösungsmittels wurden die Platten mit 2',7'-Dichlorfluorescein (Merck, Darmstadt; in 0,3% iso-Propanol) angefärbt und unter UV-Licht sichtbar gemacht. Interessante Banden wurden von der Kieselgelplatte abgekratzt, transmethyliert und anschließend am Gaschromatographen analysiert.

### Hydrolysepuffer

| | |
|---|---|
| 0,1 M | Borsäure, pH 8,0 |
| 3 mM | CaCl₂ |
| 1,4 mM | Na-Desoxycholat |

### f) Transmethylierung von Fettsäuren mit Na-Methylat (nach Lühs)

Lipidproben wurde nach Abdampfen des Lösungsmittels bzw. nach Abkratzen von der Dünnschichtplatte (z.B. bei sn-2 Analyse der Gesamtlipide) mit 2 ml Na-methylatlösung zur Umesterung versetzt. Der Ansatz wurde gut geschüttelt und zur Transmethylierung der Fettsäuren ca. 30 Minuten bei Raumtemperatur inkubiert. Anschließend wurde 1,5 ml iso-Octan zugegeben und vorsichtig zweimal geschüttelt. Der Ansatz wurde 30 Minuten lang bei 4°C gelagert, wobei die Fettsäure-Methylester (FAME) in die iso-Octanphase übergehen. Nachdem sich die Phasen deutlich getrennt hatten wurde die obere iso-Octanphase in ein GC-Gläschen abpipettiert und die Probe am Gaschromatographen gemessen.

### Na-Methylatlösung

5 g Natriummethylat wurden in 800 ml Methanol (99%) mittels Magnetrührer bei 50°C gelöst und nach dem Abkühlen mit iso-Octan auf 1000 ml aufgefüllt.

### g) Methylierung freier Fettsäuren mit methanolischer Schwefelsäure

In einem Pyrexröhrchen mit Gewindedeckel wurde 1 ml 1 N methanolischer Schwefelsäure zu dem eingeengtem Lipidextrakt zugegeben. Der Ansatz wurde eine Stunde lang bei 80°C inkubiert. Nach kurzem Abkühlen wurde der Ansatz mit 1 ml 0,9% NaCl versetzt und durchmischt. Anschließend wurde gleiches Volumen Hexan zugegeben, gut gemischt und der Ansatz bei 4°C, 30 Minuten lang bis zur Phasentrennung inkubiert. Die obere Hexanphase wurde in ein GC-Gläschen überführt und am Gaschromatographen analysiert.

### Methanolische Schwefelsäure

Zu 100 ml Methanol (wasserfrei) wurden mit 2 ml Dimethoxypropane und 0,5 M H₂SO₄ zugegeben.

### h) Gaschromatographische Analyse

Für die GC-Analysen wurden folgende Parameter des gaschromatographischen Systems eingehalten:

| | |
|---|---|
| Gerätetyp | HP 6890 GC |
| Injektor | HP GC Injector |
| Detektor | Flammen Ionisations Detektor (FID), Temp. 250°C |
| Säule | J&W DW23 50% Cyanopropyl/methylsiloxane, 30 m, 0,5 mm Durchmesser |
| Ofentemperatur | 220°C |
| Trägergas | Wasserstoff |
| Autosampler | HP 7673, Einspritzmenge 1 µl Probe |

### i) Die Lipidanalyse der Thraustochytrium-Lipide lieferte folgende Ergebnisse

| Lipidfraktion | Fettsäurezusammensetzung | | | |
|---|---|---|---|---|
| | 16:0 | 22:3 ω -3 | 22:4 w -3 | 22:6 w -3 |
| TAG gesamt | 24 % | 12% | 31 % | 23 % |
| TAG sn-2 | 21 % | 26% | | 43 % |
| Membranlipide gesamt | 16% | 13% | | 23 % |
| Membranlipide sn-2 | 34 % | 18% | | 36 % |

Die Ergebnisse zeigen, dass Thraustochytrium einen hohen Gehalt an DHA in seinen Lipiden besitzt. DHA stellt mit neben Palmitat die Hauptkomponente der Triacylglyerole dar und ist die dominierende Fettsäure der Membranlipide. Auffällig ist, dass DHA an der sn-2 Position sowohl des Triacylclycerols als auch der Membranlipide deutlich angereichert ist: 36-43% der Fettsäuren an der sn-2 Position ist DHA. Aufgrund dieser Daten kann man davon ausgehen, dass Thraustochytrium über eine aktive LPAAT verfügt, die eine hohe Spezifität für DHA-CoA aufweist.

### Beispiel 8: Isolierung von Gesamt-RNA und poly(A)⁺-RNA

Die Isolierung von Gesamt-RNA aus Pflanzen wie Lein und Raps etc. erfolgte nach einer bei Logemann et al. beschriebenen Methode (Anal. Biochem. (1987) 163: 21). Aus dem Moos Physcomitrella patens kann die Gesamt-RNA aus Protonema-Gewebe nach dem GTC-Verfahren (Reski et al. (1994) Mol. Gen. Genet. 244: 351-359) gewonnen werden.

### a) RNA Isolierung aus Thraustochytrium, Cryptecodinium und Shewanella:

Tiefgefrorene Algenproben (-70°C) wurden in einem eiskaltem Mörser unter Flüssigstickstoff zu feinem Pulver zerreiben. 2 Volumen Homogenisationsmedium (12,024 g Sorbitol, 40,0 ml 1 M Tris-RC1, pH 9 (0,2 M); 12,0 ml 5 M NaCl (0,3 M), 8,0 ml 250 mM EDTA, 761,0 mg EGTA, 40,0 ml 10% SDS wurden auf 200 ml mit H₂0 aufgefüllt und der pH auf 8,5 eingestellt) und 4 Volumen Phenol mit 0,2% Mercaptoethanol wurden bei 40-50°C unter gutem Mischen zu gefrorenem Zellpulver gegeben. Danach wurden 2 Volumen Chloroform hinzufügen und für 15 min kräftig gerührt. Es wurde 10 min bei 10000g zentrifugiert und die wässrige Phase mit Phenol/Chloroform (2Vol/2Vol) und abschließend mit Chloroform extrahiert.

Das erhaltene Volumen der wässrigen Phase wurde mit 1/20 Vol 4 M Na-Acetat (pR 6) und 1 Vol Isopropanol (eiskalt) versetzt und die Nukleinsäuren bei -20°C ÜN (= über Nacht) gefällt. Es wurde 30 min bei 10000 g zentrifugiert und der Überstand abgesogen. Es folgte ein Waschschritt mit 70% EtOH und erneute Zentrifugation. Das Sediment wurde in Tris-Borat-Puffer (80 mM Tris-Borat-Puffer, 10 mM EDTA, pH 7,0). Dann wurde der Überstand mit 1/3 Vol 8 M LiCl versetzt, gemischt 30 min bei 4°C inkubiert. Nach erneutem zentrifugieren wurde das Sediment mit 70% Ethanol gewaschen, zentrifugiert und das Sediment anschließend in RNAse-freiem Wasser gelöst.

Die Isolierung von poly(A}+-RNA erfolgte unter Verwendung von Dyna Beads (Dynal, Oslo, Finnland) nach den Anweisungen im Protokoll des Herstellers.

Nach der Bestimmung der RNA- oder poly(A}+-RNA-Konzentration wurde die RNA durch Zugabe von 1/10 Volumina 3 M Natriumacetat, pH 4,6, und 2 Volumina Ethanol gefällt und bei -70°C aufbewahrt.

Für die Analyse wurden jeweils 20 µg RNA in einem Formaldehydhaltigen 1,5%igen Agarosegel aufgetrennt und auf Nylon Membranen (Hybond, Amersham) überführt. Der Nachweis spezifischer Transkripte wurde wie bei Amasino beschrieben durchgeführt (Amasino (1986) Anal. Biochem. 152: 304).

### Beispiel 9: Konstruktion von cDNA-Banken

Zur Konstruktion der cDNA-Banken aus Physcomitrella, Thraustochytrium und Fusarium wurde die Erststrangsynthese unter Verwendung von Reverser Transkriptase aus Maus-Leukämie-Virus (Roche, Mannheim, Deutschland} und Oligo-d(T)-Primern, die Zweitstrangsynthese durch Inkubation mit DNA-Polymerase I, Klenow-Enzym und RNAse H-Spaltung bei 12°C (2 Std.), 16°C (1 Std.) und 22°C (1 Std.) erzielt: Die Reaktion wurde durch Inkubation bei 65°C (10 min) gestoppt und anschließend auf Eis überführt. Doppelsträngige DNA-Moleküle wurde mit T4-DNA-Polymerase (Roche, Mannheim) bei 37°C (30 min) mit glatten Enden versehen. Die Nukleotide wurden durch Phenol/Chloroform-Extraktion und Sephadex-G50-Zentrifugiersäulen entfernt. EcoRI/XhoI-Adapter (Pharmacia, Freiburg, Deutschland) wurden mittels T4-DNA-Ligase (Roche, 12°C, über Nacht) an die cDNA-Enden ligiert, mit Xhol nachgeschnitten und durch Inkubation mit Polynukleotidkinase (Roche, 37°C, 30 min) phosphoryliert. Dieses Gemisch wurde der Trennung auf einem Low-Melting-Agarose-Gel unterworfen. DNA-Moleküle über 300 Basenpaaren wurden aus dem Gel eluiert, phenol-extrahiert, auf Elutip-D-Säulen (Schleicher und Schüll, Dassel, Deutschland) konzentriert und an Vektorarme ligiert und in lambda-ZAPII-Phagen oder lambda-ZAP-Express-Phagen unter Verwendung des Gigapack Gold-Kits (Stratagene, Amsterdam, Niederlande} verpackt, wobei Material des Herstellers verwendet und seine Anweisungen befolgt wurden.

### Beispiel 10: DNA-Sequenzierung und Computeranalyse

cDNA-Banken, wie im Beispiel 9 beschrieben, wurden zur DNA-Sequenzierung nach Standardverfahren, insbesondere durch das Kettenterminationsverfahren unter Verwendung des ABI PRISM Big Dye Terminator Cycle Sequencing Ready Reaction-Kit (Perkin-Elmer, Weiterstadt, Deutschland), verwendet. Die Sequenzierung zufälliger, vereinzelter Klone wurde anschließend an die präparative Plasmidgewinnung aus cDNA-Banken über in vivo-Massenexcision und Retransformation von DH10B auf Agarplatten durchgeführt (Einzelheiten zu Material und Protokoll von Stratagene, Amsterdam, Niederlande). Plasmid-DNA wurde aus über Nacht gezüchteten E. coli-Kulturen, die in Luria-Brühe mit Ampicillin (siehe Sambrook et al. (1989) (Cold Spring Harbor Laboratory Press: ISBN 0-87969-309-6)) gezüchtet worden waren, an einem Qiagen-DNA-Präparations-Roboter (Qiagen, Hilden) nach den Protokollen des Herstellers präpariert. Sequenzierprimer mit den folgenden Nukleotidsequenzen wurden verwendet:
5'-CAGGAAACAGCTATGACC-3'
5'-CTAAAGGGAACAAAAGCTG-3'
5'-TGTAAAACGACGGCCAGT-3'

Die Sequenzen wurden unter Verwendung des Standard-Softwarepakets EST-MAX, das kommerziell von Bio-Max (München, Deutschland) geliefert wird, prozessiert und annotiert. Durch Nutzung von Vergleichsalgorithmen und unter Verwendung einer Suchsequenz wurde mit Hilfe des BLAST-Programms nach homologen Genen gesucht (Altschul et al. (1997) "Gapped BLAST and PSI-BLAST: A new generation of protein database search programs", Nucleic Acids Res. 25: 3389-3402).

### Beispiel 11: Identifikation von Genen mittels Hybridisierung

Gensequenzen lassen sich zur Identifikation homologer oder heterologer Gene aus cDNA- oder genomischen Banken verwenden.

Homologe Gene (d.h. Volllängen-cDNA-Klone, die homolog sind, oder Homologe) lassen sich über Nukleinsäurehybridisierung unter Verwendung von beispielsweise cDNA-Banken isolieren: Je nach der Häufigkeit des Gens von Interesse werden 100000 bis zu 1000000 rekombinante Bakteriophagen plattiert und auf eine Nylonmembran überführt. Nach der Denaturierung mit Alkali wird die DNA auf der Membran z.B. durch UV-Vernetzung immobilisiert. Die Hybridisierung erfolgt bei hochstringenten Bedingungen. In wässriger Lösung werden die Hybridisierung und die Waschschritte bei einer Ionenstärke von 1 M NaCl und einer Temperatur von 68°C durchgeführt. Hybridisierungssonden wurden z.B. durch Markierung mittels radioaktiver (32P) Nicktranskription (High Prime, Roche, Mannheim, Deutschland) hergestellt. Die Signale werden mittels Autoradiographie nachgewiesen.

Partiell homologe oder heterologe Gene, die verwandt, aber nicht identisch sind, lassen sich analog zum oben beschriebenen Verfahren unter Verwendung niedrigstringenter Hybridisierungs- und Waschbedingungen identifizieren. Für die wässrige Hybridisierung wurde die Ionenstärke gewöhnlich bei 1 M NaCl gehalten, wobei die Temperatur nach und nach von 68 auf 42°C gesenkt wurde.

Die Isolatierung von Gensequenzen, die nur zu einer einzelnen Domäne von beispielsweise 10 bis 20 Aminosäuren Homologien aufweisen, läßt sich unter Verwendung synthetischer, radioaktiv markierter Oligonukleotidsonden durchführen. Radioaktiv markierte Oligonukleotide werden mittels Phosphorylierung des 5'-Endes zweier komplementärer Oligonukleotide mit T4-Polynukleotidkinase hergestellt. Die komplementären Oligonukleotide werden aneinander hybridisiert und ligiert, so dass Konkatemere entstehen. Die doppelsträngigen Konkatemere werden beispielsweise durch Nicktranskription radioaktiv markiert. Die Hybridisierung erfolgt gewöhnlich bei niedrig-stringenten Bedingungen unter Verwendung hoher Oligonukleotidkonzentrationen.

Oligonukleotid-Hybridisierungslösung:
6 x SSC
0,01 M Natriumphosphat
1 mM EDTA (pH 8)
0,5% SDS
100 µg/ml denaturierte Lachssperma-DNA
0,1% fettarme Trockenmilch

Während der Hybridisierung wurde die Temperatur schrittweise auf 5-10°C unter die berechnete Oligonukleotid-Tm oder bis auf Raumtemperatur bedeutet RT = 23°C in allen Experimenten, wenn nicht anders angegeben) gesenkt, gefolgt von Waschschritten und Autoradiographie. Das Waschen wurde mit extrem niedriger Stringenz durchgeführt, zum Beispiel 3 Waschschritte unter Verwendung von 4 x SSC. Weitere Einzelheiten sind wie von Sambrook, J., et al. (1989), "Molecular Cloning: A Laboratory Manual", Cold Spring Harbor Laboratory Press, oder Ausubel, F.M., et al. (1994) "Current Protocols in Molecular Biology", John Wiley & Sons, beschrieben.

### Beispiel 12: Isolierung und Klonierung eines Volllängenklons für LPAAT aus Thraustochytrium

### Durchmustern einer cDNA-Bank von Thraustochytrium

Entsprechend unter Beispiel 9 beschrieben, wurde eine cDNA Bank von Thraustochytrium erstellt. Im nächsten Schritt wurde die Phagenbank nach Herstellerangaben mittels eines Helferphagen in eine Plasmidbank umgesetzt. Die Plasmidbank wurde auf LB-Medium, 0,8 % Agar, 100 mg/ L Ampicillin ausplattiert und inkubiert. Gewachsene Bakterienkolonien wurden zufällig ausgewählt, in Flüssigmedium (LB, 100 mg/ L Ampicillin) angezogen und wie in Beispiel 10 beschrieben, einer Sequenzierung unterworfen.

Die erhaltenen Sequenzen wurden nach Redundanzen durchsucht und diese entfernt. Dadurch konnte ein Sequenzsortiment erhalten werden, dass ein Unigen-Set beschreibt. Dieses Sequenz-Set wurde in die Pedant-Datenbank (Biomax AG, Martinsried, Deutschland) eingelesen. Mittels BLAST Analyse anhand von konservierten Bereichen innerhalb von Acyltransferasen wurde ein kurzes Sequenzstück mit niedriger Ähnlichkeit zu bekannten Acyltransferasen gefunden. Die vorhandene Sequenzinformation wurde verwendet, um Primer zu generieren (LPAAT069-5' und LPAAT069-3'). Mit diesem Fragment wurde dann in der cDNA-Bank nach einem Volllänge-Klon gesucht (Tabelle8).

### Tabelle 8: Sequenzen der eingesetzten Primer;

Die Schmelztemperatur Tₘ (°C) der Oligonukleotide wurde nach Suggs et al. (1981) berechnet: Tₘ (°C) = 4 (G+C) + 2 (A+T) Tₘ-Werte in Klammern beziehen sich auf tatsächlich bindende Nukleotide von Primern, deren Enden durch zusätzlich eingeführte Schnittstellen modifiziert wurden.

| Primer | Sequenz | Tₘ (°C) |
|---|---|---|
| LPAAT069-5' | 5'-GCT ACA TTG CCA TGG AGC-3' | 56 |
| LPAAT069-3' | 5'-GCT ACA AGA GGT CAG GTC G-3' | 59 |
| ACtrau-5' | 5'-CTG GAT CCA TGA GCG CGT GGA CGA G-3' | 69 (52) |
| ACtrau-3' | 5'-TTG GAT CCC AAG AGG TCA GGT CGG A-3' | 66 (54) |
| ACtrau-3'stop | 5'-TTG GAT CCC TAC AAG AGG TCA GGT CG-3' | 66 (48) |
| YES-HIS-5' | 5'-CTG AGC TCA TGA GCG CGT GGA G-3' | 69 (56) |
| YES-HIS-3' | 5'-ATG GAT CCG TGA TGG TGA TGG TGA TGC AAG AGG TC-3' | 72 (40) |

Bei den PCR-Experimenten wurden die unten angegebenen Bestandteile eines PCR-Standardansatzes auf Eis in ein PCR-Reaktionsgefäß pipettiert, in den Thermoblock gestellt und das unten dargestellte Temperaturprofil gestartet. Als Polymerase wurde in fast allen Fällen die Taq-Polymerase (Gibco BRL) eingesetzt. Lediglich bei Amlifikationen im Rahmen der funktionalen Expression in E. coli JC201 wurde die Pfu-Polymerase (Stratagene) verwendet. Die Zugabe der Polymerase erfolgte bei allen Experimenten über einen sogenannten "Heißstart", bei dem das Enzym erst nach 5 min Denaturierung des DNA-Templates zugegeben wird. Die Annealingtemperaturen (Tₐ) wurden 3-5°C unter der mittleren Schmelztemperatur Tₘ der Primerpaare gewählt.

### PCR-Standardansatz (für Taq-Polymerase)

5 µl 10 x PCR-Puffer (100 mM Tri-HCl, pH 8,3; 15 mM MgCl₂, 500 mM KCI)
1 µl dNTP-Mix (10 mM dATP, dGTP, dTTP u. dCTP)
1 µl Primer 1 (30 µM)
1 µl Primer 2 (30 µM)
1 U Taq-Polymerase
50-100 ng Plasmid-DNA-Template
mit Aqua dest. auf 50 µl auffüllen

### Heißstartprogramm

1. Denaturierung 95°C, 5 min
2. Heißstart 25°C, 3 min → Zugabe der Polymerase
3. Denaturierung 94°C 30 s
4. Annealing Tₘ-5°C, 30 s
5. Polymerisation 72°C, 1 - 3 min (für 1,0 kbp ca. 60 s)
   Die Schritte 3. bis 5. wurden 25 bis 30 mal zyklisch wiederholt.
6. Polymerisation 72°C, 5 min
7. Termination 4°C

### a) Nichtradioaktive Markierung von DNA

DNA-Sonden wurden nichtradioaktiv mit dem "PCR DIG PROBE SYNTHESIS KIT" (Boehringer Mannheim) markiert. Dabei wurden DNA-Fragmente in einer PCR-Reaktion mit Digoxigenin-markierten Desoxyuridintriphosphat (DIG-dUTP) markiert. Die Detektion erfolgte anschließend mittels eines Anti-Digoxygenin-Antikörpers, der mit alkalischer Phosphatase konjugiert ist, und Zugabe von Chemilumineszenz- oder Farbsubstraten.

Um Hintergrundsignale zu vermeiden, die auf Vektorsequenzen zurückzuführen sind, wurde für die PCR-Markierung zunächst in einer ersten PCR mit unmarkierten dNTPs die gewünschte DNA amplifiziert, das lineare Fragment über ein Agarosegel gereinigt und als Template für die eigentliche PCR-Markierung benutzt, bei der wieder das Primerpaar der ersten PCR eingesetzt wurde. Die Durchführung der Markierungsreaktion richtete sich nach den Angaben des Herstellers. Die gewählten Primerkombinationen sind in der folgenden Tabelle zusammengefasst.

| Primer | Sequenz |
|---|---|
| LPAAT069-5' | 5'- GCT ACA TTG CCA TGG AGC -3' |
| LPAAT069-3' | 5'- GCT ACA AGA GGT CAG GTC G -3' |

### b) Screening einer cDNA-Bank

Zur Isolation eines vollständigen Klons wurde eine Thraustochytrium cDNA-Bank (in λTriplEx2) mit der DIG-markierten Sonde abgesucht. Die Erstellung der Sonde erfolgte mit den Primern LPAAT069-3' und LPAAT069-5, abgeleitet von dem EST-Klon s_t002038069 bekannten cDNA-Sequenz die möglicherweise für eine LPAAT aus Thraustochytrium kodiert.

Es wurden je 5 x 10⁴ Plaques auf 10 große NZY-Platten, entsprechend den Angaben des Herstellers (Stratagene) ausplattiert. Für den Transfer der Phagen auf Nitrocellulose-Filter (Hybond^{™}-C, Amersham) wurden die Filter 1 min auf die Platten gelegt und ihre genaue Lage durch 3 Einstiche mit einer Kanüle markiert. Anschließend wurden die Filter mit der Abdruckseite nach oben zunächst 5 min mit Denaturierungs-Lösung, dann 5 min mit Neutralisierungs-Lösung und schließlich 15 min mit 2 x SSC-Lösung behandelt. Dies erfolgte auf 3 Bögen Whatman 3 MM Papier, die mit den Lösungen getränkt waren. Nach fünfminütigem Trocknen der Filter wurde die DNA durch UV-Behandlung mit 0,12 Joule/cm² (UV-Crosslinker, Hoefer Scientific Instruments) fixiert. Hybridisierung und kolorimetrische Detektion erfolgten mit dem "Dig System für Filter Hybridisierung" von Boehringer (Mannheim) entsprechend den Angaben des Herstellers. Als Hybridisierungs-Puffer wurde Standard-Puffer verwendet, wobei die Hybridisierung in 80 ml Hybridisierungs-Puffer mit 15 µl des Sonden-PCR-Ansatzes durchgeführt wurde. Nach erfolgter Detektion wurden die genaue Lage der Signale sowie die drei Orientierungspunkte der Filter auf Plastikfolien übertragen, um mit diesen als Schablone die positiven Plaques auf den Platten zu identifizieren. Diese wurden dann mit einem abgeflammten Korkbohrer (Durchmesser 5 mm) ausgestochen, in 1 ml SM-Puffer mit 20 µl CHCl₃ überführt und die Phagen aus den Agarstücken über Nacht bei 4°C eluiert. Ein exaktes Ausstechen der Plaques war durch deren hohe Dichte und geringe Größe kaum möglich. Daher werden in der Regel ein bis zwei "Rescreens" durchgeführt. In diesem Fall wurden die Phagenlysate mittels PCR und den Primern LPAAT069-3' und LPAAt-5 auf Fragmente von ca. 570 bp untersucht. Dazu wurden Aliquots der Phagenlysate mit EDTA (Endkonzentration 10 mM) versetzt und daraus 1 µl für die PCR als Template eingesetzt. Mit positiven Lysaten wurden in-vivo-Exzisionen nach Angaben des "ZAP-cDNA® Gigapack® II Gold Cloning Kit" (Stratagene) durchgeführt, wobei von den infizierten SOLR-Zellen statt der angegebenen 10-50 µl nur 2µl auf LB-Amp-Platten ausplattiert und über Nacht bei 37°C inkubiert wurden. Die Plasmide der erhaltenen Kolonien wurden direkt mittels PCR und den Primern LPAAT-3' und LPAAT-5' untersucht. Dazu wurden "Pools" erstellt, indem je 6 Kolonien mit sterilen Zahnstochern in einem Eppendorfreaktionsgefäß in 20 µl Aqua dest. eingerieben wurden, 3 x eingefroren und wieder aufgetaut, um die Zellen zu lysieren, 5 min bei 14.000 x g zentrifugiert und vom Überstand 2 µl als Template in die PCR-Reaktion eingesetzt. Positive "Pools" wurden vereinzelt, die Plasmide über Plasmid-Minipräparationen isoliert und über PCR, Restriktionsanalysen sowie DNA-Sequenzierungen analysiert.

Schließlich wurde ein Volllängenklon für LPAAT aus Thraustochytrium identifiziert, dessen DNA-Sequenz in SEQ ID NO:1 dargestellt ist. Die abgeleitete Aminosäuresequenz ist in SEQ ID NO:2 gezeigt.

### NZY-Medium (pro Liter, NZY-Platten mit 15 g Agar)

5 g NaCl
5 g Hefeextrakt
10 g NZ-Amin (Caseinhydrolysat)
pH 7,5 (NaOH)
2 g MgSO₄ (sterilfiltriert)

### Denaturierungs-Lösung

0,5 M NaOH
1,5 M NaCl

### Neutralisierungs-Lösung

1,0 M Tris-HCl, pH 7,5
1,5 M NaCl
20 x SSC
3,0 M NaCl
0,3 M Natriumcitrat, pH 7,0

### Standard-Puffer

5 x SSC
0,1 % (w/v) N-Laurylsarcosin
0,02% (w/v) SDS
1 % Blocking Reagens

### SM-Puffer (pro Liter)

5,8 g NaCl
2, g MgSO₄
50 ml 1 M Tris-HCl, pH 7,5
5 ml 2% Gelatine

### Beispiel 13: Isolierung und Klonierung von Volllängenklonen für PUFA spezifische Acyltransferasen aus Physcomitrella patens, Mortierella alpina und Shewanella hanedai

Wie unter Beispiel 8 und 9 beschrieben, wurde aus Physcomitrella patens und Mortierella alpina RNA isoliert und eine cDNA-Bank hergestellt.

Im nächsten Schritt wurde die Phagenbank nach Herstellerangaben mittels eines Helferphagen in eine Plasmidbank umgesetzt. Die Plasmidbank wurde auf LB-Medium, 0,8 % Agar, 100 mg/ L Ampicillin ausplattiert und inkubiert. Gewachsene Bakterienkolonien wurden zufällig ausgewählt, in Flüssigmedium (LB, 100 mg/ L Ampicillin) angezogen und wie in Beispiel 10 beschrieben, einer Sequenzierung unterworfen.

Die erhaltenen Sequenzen wurden nach Redundanzen durchsucht und diese entfernt. Dadurch konnte ein Sequenzsortiment erhalten werden, dass ein Unigen-Set beschreibt. Dieses Sequenz-Set wurde in die Pedant-Datenbank (Biomax AG, Martinsried, Deutschland) eingelesen. Mittels BLAST Analyse anhand von konservierten Bereichen innerhalb von Acyltransferasen wurden kurze Sequenzstücke mit niedriger Ähnlichkeit zu bekannten Acyltransferasen gefunden (Tabelle 9). Die vorhandene Sequenzinformation wurde verwendet, um Primer zu generieren (Tabelle 10). Mit diesen Primern konnten die Volllänge-Klone amplifiziert werden.

Für die Acyltransferase aus Shewanella hanedai wurde die öffentliche Datenbank von Shewanella putrefaciens MR1 (TIGR Datenbank http://tigrblast.tigr.org/ufmg/) nach Acyltransferasesn durchsucht. Es konnte eine Sequenz in der Datenbank mit Homologie zu Acyltransferasen gefunden werden. Von dieser Sequenz wurde ein PCR-Fragement generiert mittels Standard-Primer T7 und T3. Das erhaltene Produkt wurde wie in Beispiel 10 a) und b) erläutert, markiert und zum Durchsuchen einer genomischen Shewanella hanedai Bank eingesetzt.

Genomische DNA aus Shewanella hanedai wurde nach folgendem Protokoll isoliert: Eine 100 ml Kultur wurde bis zu einer optischen Dichte von 1,0 bei 30°C angezogen. Davon wurden 60 ml abzentrifugiert bei 3000 xg für 3 min. Das Pellet wurde in 6 ml doppelt-destilliertem H₂O resuspendiert und auf 1,5 ml Gefäße verteilt, abzentrifugiert und der Überstand verworfen. Die Pellets wurden mit 200 µl Lösung A, 200 µL Phenol/Chloroform (1:1) und 0,3 g Glaskugeln durch Vortexen resuspendiert und lysiert. Nach Zugabe von 200 µl TE-Puffer pH 8,0 wurde für 5 min zentrifugiert. Der Überstand wurde einer Ethanolfällung mit 1 ml Ethanol unterzogen. Das erhaltene Pellet nach Fällung wurde in 400 µL TE-Puffer pH 8,0 + 30 µg/mL RnaseA gelöst. Nach Inkubation für 5 min bei 37°C wurden 18 µL 3 M Natriumacetat-Lösung pH 4,8 und 1 mL Ethanol zugegeben und die präzipitierte DNA durch Zentrifugation pelletiert. Das DNA-Pellet wurde in 25 µL doppelt-destilliertem H₂O gelöst. Die Konzentration der genomischen DNA wurde durch deren Absorption bei 260 nm bestimmt.

### Lösung A:

2 % Trition-X100
1 % SDS
0,1 M NaCl
0,01 M Tris-HCl pH 8,0
0,001 M EDTA

Die erhaltene genomische DNA wurde 1 Stunden bei 25 °C mit dem Restriktionsenzym Sau3A (New England Biolabs) nach Herstellerangaben inkubiert. Die erhaltene Fragmente wurden dann in einen mit BamHI verdautes pUC18 Plasmid mittels T4 Ligase (Roche) ligiert. Die erhaltene Bank wurde dann in gleicherweise wie in Beispiel 10 beschrieben, durchsucht. Es konnte ein Klon mit einem 1,7 kb grosses genomisches Fragment gefunden werden, der eine 687 bp lange codierende Sequenz mit Ähnlichkeit zu Acyltransferasen zeigt.

Die Sequenz aus Shewanella hanedai zeigt eine besonders hohe Ähnlichkeit zu der LPCAT aus Chaenorabdidis elegans. Die Ähnlichkeit der beiden Sequenzen auf Aminosäureebene beträgt 26 %.

**Tabelle 9: Identifizierte Acyltransferase aus den genannten cDNA-Banken**

| **Klon-Nr.** | **Organismus** | **Homologie zu** |
|---|---|---|
| MaLPAAT1.1 | M. alpina | LPAAT |
| MaLPAAT1.2 | M. alpina | LPAAT |
| ShLPAAT | S. hanedai | LPAAT |
| T6 | Thrausto. | LPAAT |
| pp004064045r | P. patens | LPAAT |
| pp020064227r | P. patens | LPAAT |
| pp015052144r | P. patens | GPAT/LPAT |
| pp004034225r | P. patens | GPAT |
| pp004104272r | P. patens | Ca-LPAAT |
| pp020018156r | P. patens | Ca-LPAAT |
| pp015034341 r | P. patens | LPAAT |
| pp015033362r | P. patens | LCAT |
| Fg003028298 | Fusarium | LCAT |

**Tabelle 10: Sequenzen der eingesetzten Primer;**

| Clone nr. | Organism | Primersequenz 5'-3' Orientierung | Länge in bp |
|---|---|---|---|
| MaLPAAT1.1 | M. alpina | | 1254 |
| MaLPAAT1.2 | M. alpina | | 1170 |
| ShLPAAT | S. hanedai | | 687 |
| T6 | Thrausto. | | 918 |
| Pp00406404 | P. patens | | 714 |
| Pp02006422 | P. patens | | 657 |
| Pp01505214 | P. patens | | 444 |
| Pp00403422 | P. patens | | 1305 |
| Pp00410427 | P. patens | | 1566 |
| Pp02001815 | P. patens | | 1560 |
| Pp01503434 | P. patens | | 1014 |
| Pp01503336 | P. patens | | 1503 |
| Fg003028298 | Fusarium | | 1893 |

Bei den PCR-Experimenten wurden die unten angegebenen Bestandteile eines PCR-Standardansatzes auf Eis in ein PCR-Reaktionsgefäß pipettiert, in den Thermoblock gestellt und das unten dargestellte Temperaturprofil gestartet. Als Polymerase wurde in fast allen Fällen die Taq-Polymerase (Gibco BRL) eingesetzt. Lediglich bei Amlifikationen im Rahmen der funktionalen Expression in E. coli JC201 wurde die Pfu-Polymerase (Stratagene) verwendet. Die Zugabe der Polymerase erfolgte bei allen Experimenten über einen sogenannten "Heißstart", bei dem das Enzym erst nach 5 min Denaturierung des DNA-Templates zugegeben wird. Die Annealingtemperaturen (Tₐ) wurden 3-5°C unter der mittleren Schmelztemperatur Tₘ der Primerpaare gewählt.

### PCR-Standardansatz (für Taq-Polymerase)

5 µl 10 x PCR-Puffer (100 mM Tri-HCl, pH 8,3; 15 mM MgCl₂, 500 mM KCl)
1 µl dNTP-Mix (10 mM dATP, dGTP, dTTP u. dCTP)
1 µl Primer 1 (30 µM)
1 µl Primer 2 (30 µM)
1 U Taq-Polymerase
50-100 ng Plasmid-DNA-Template
mit Aqua dest. auf 50 µl auffüllen

### Heißstartprogramm

1. Denaturierung 95°C, 5 min
2. Heißstart 25°C, 3 min → Zugabe der Polymerase
3. Denaturierung 94°C 30 s
4. Annealing Tₘ -5°C, 30 s
5. Polymerisation 72°C, 1 - 3 min (für 1,0 kbp ca. 60 s)
   Die Schritte 3. bis 5. wurden 25 bis 30 mal zyklisch wiederholt.
6. Polymerisation 72°C, 5 min
7. Termination 4°C

| | | |
|---|---|---|
| GSP: | TCT CTT TTT CGT GCT GCT CCA GCC GAT (Are 297) | |
| PCR-Programm: | 10min. 95°C | |
| | 1 min. 95°C (40 Cycles) | |
| | 1min. 65°C | |
| | 2min. 72°C | |
| | 10min. 72°C | Pause 4°C |
| PCR-Maschine: | Biometra Trio Thermoblock | |

Zunächst PCR auf der RACE-Bank Moos mit AP1 und GSP, bei richtiger Größe PCR mit nested AP2 und GSP, positive werden in pCRII-TOPO-TA Cloning Vector für Sequenzierung kloniert.

### Beispiel 14: Expression von Thraustochytrium LPAAT (ThLPAAT) in Hefe

Um die Funktionalität von ThLPAAT nachzuweisen, wurde in einem ersten Ansatz der kodierende Bereich der cDNA in einem Hefe-Expressionsvektor kloniert und in S. cerevisiae exprimiert. Die in der Hefe produzierte LPAAT sollte zugesetzte über Acyltransferase-Aktivität in mikrosomalen Fraktionen nachgewiesen werden.

Sämtliche Fest- und Flüssigmedien für Hefe wurden nach Protokollen von Ausubel et al. (Current Protocols in Molecular Biology, John Wiley & Sons, New York, 1995) hergestellt.

Die ThLPAAT-cDNA wurde über Restriktionsverdau mit HindIII/BamHI aus dem Vektor pGEM-T ausgeschnitten, in den HindIII/BamHI geschnittenen shuttle-Vektor pYES2 (Invitrogen, Carlsbad, USA) kloniert und der so entstandene Vektor pYES2-ThLPAAT in E. coli XL1 blue transformiert. pYES2-ThLPAAT wurde mit Hilfe der LiAc-Methode in S. cerevisiae INCSc1 (Invitrogen, Carlsbad, USA) transformiert, wo die Expression der ThLPAAT-cDNA unter der Kontrolle des GAL1-Promotors stand.

Die Expression von ThLPAAT in S. cerevisiae INVSc1 erfolgte modifiziert nach Avery et al. (Appl. Environ. Microbiol., 62, 1996: 3960-3966) und Girke et al. (The Plant Journal, 5, 1998: 39-48). Um eine Starterkultur herzustellen, wurden 20 ml SD-Medium mit Glucose und Aminosäurelösung ohne Histidin mit einer Hefe-Einzelkolonie angeimpft und über Nacht bei 30 °C bei 140 rpm inkubiert. Die Zellkultur wurde zwei mal gewaschen durch Abzentrifugieren und Resuspendieren in SD-Medium ohne Supplemente und ohne Zucker. Mit den gewaschenen Zellen wurde eine Hauptkultur auf eine OD₆₀₀ von 0,1 bis 0,3 angeimpft. Die Anzucht der Hauptkultur erfolgte in 25 ml SD-Medium mit 2 % (w/v) Galaktose, Aminosäurelösung ohne Histidin, 0,02 % Linolsäure (2%ige Stammlösung in 5 % Tergitol NP40), 10 % Tergitol NP40 72 h lang bei 30°C. Die Ernte der Hauptkultur erfolgte über Zentrifugation. Das Zellpellet wurde bei -20°C eingefroren und anschließend für ca. 18 h lyophilisiert.

Nach Expression des Konstruktes pYES2-ThLPAAT in Hefe konnte kein aktives Protein gereinigt werden. Auch die subzellulären Fraktionen aus den verschiedenen transgenen Zellen zeigten keine höheren LPAAT-Aktivitäten als die entsprechenden Kontrollfraktionen.

Zur Erhöhung der Löslichkeit des exprimierten Proteins wurde ein weiteres Konstrukt pDest15-GST-ThLPAAT (pDest15-Vektor von Invitrogen) über die Gateway-Reaktion erstellt. Dazu wurden nach Herstellerangaben folgende Primer synthetisiert:
5'-Primer att1ThLPAAT:
   GGGGACAAGTTTGTACAAAAAAGCAGGCTCCATGAGCGCGTGGACGAGGGCC
3'-Primer att2ThLPAAT:

Mit diesen Primern wurden folgende PCR-Reaktion durchgeführt:

### PCR-Standardansatz (für Taq-Polymerase)

5 µl 10 x PCR-Puffer (100 mM Tri-HCl, pH 8,3; 15 mM MgCl₂, 500 mM KCI)
1 µl dNTP-Mix (10 mM dATP, dGTP, dTTP u. dCTP)
1 µl Primer 1 (30 µM)
1 µl Primer 2 (30 µM)
1 U Taq-Polymerase
50-100 ng pYES2-ThLPAAT
mit Aqua dest. auf 50 µl auffüllen

| | | |
|---|---|---|
| PCR-Programm: | 2min. 95°C | |
| 1 min. 95°C | (30 Cycles) | |
| 1 min. 65°C | | |
| 2 min. 72°C | | |
| 10 min. 72°C | | Pause 4°C |
| PCR-Maschine: | Biometra Trio Thermoblock | |

Das PCR-Product wurde per Gateway-Reaktion (BP-Reaktion; Invitrogen) nach Herstellerangaben in den Vektor pDONOR221 transferiert und die Sequenz durch Sequenzierung überprüft. In einem nächsten Schritt wurde die ThLPAAT-Sequenz dann durch die LR-Reaktion in den Vektor pDES15 übertragen und zur Expression in E. coli BL21 Zellen eingesetzt. Die ThLPAAT-Sequenz wurde entsprechend der Herstellerangaben (Invitrogen) an den offenen Leserahmen der im Plasmid codierten Glutathion-S-Transferase (GST) angehängt. Dadurch konnte ein Fusionsprotein aus GST und ThLPAAT erzeugt werden.

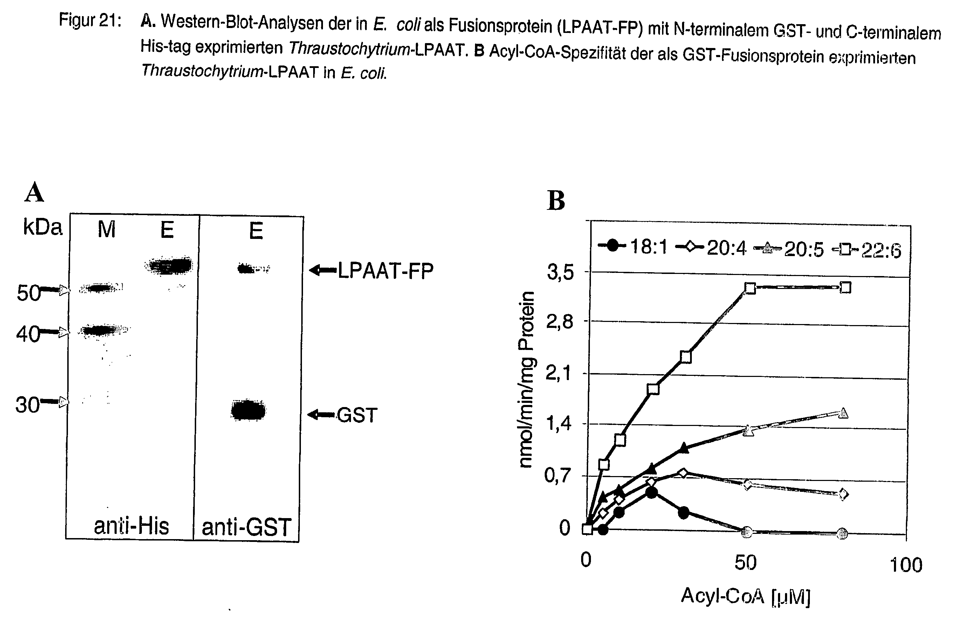

Nach Expression unter Standardbedingungen in E. coli konnte exprimiertes Protein nachgewiesen werden (Fig. 21A) und dieses über eine Glutathion-Säule gereinigt werden.

Das gereinigte Fusionsprotein zeigte LPAAT Aktivität, wie in Fig. 21 B gezeigt. Die höchste Aktivität konnte dabei für DHA-CoA (22:6) erhalten werden, was eine Nutzung dieser Acyltransferase zur Herstellung von PUFA ermöglicht.

Figur 21 A zeigt die Western-Blot-Analysen der in *E*. *coli* als Fusionsprotein (LPAAT-FP) mit N-terminalem GST- und C-terminalem His-tag exprimierten *Thraustochytrium-*LPAAT (Spuren E: 7 µg lösliche Proteinfraktion, Spur M: Größenstandard). Figur 21 B zeigt die Acyl-CoA-Spezifität der als GST-Fusionsprotein exprimierten *Thraustochytrium-*LPAAT in *E*. *coli.* Die Enzymtests wurden mit 0,4 µg löslicher Proteinfraktion in Gegenwart von 100 mM Tricine-NaOH (pH 8,2), 30 µM 1-Oleoyl-[U-¹⁴C]glycerin-3-phosphat und steigenden Konzentrationen der angegebenen Thioester ermittelt.

### Beispiel 15: Expression von Shewanella-LPAAT

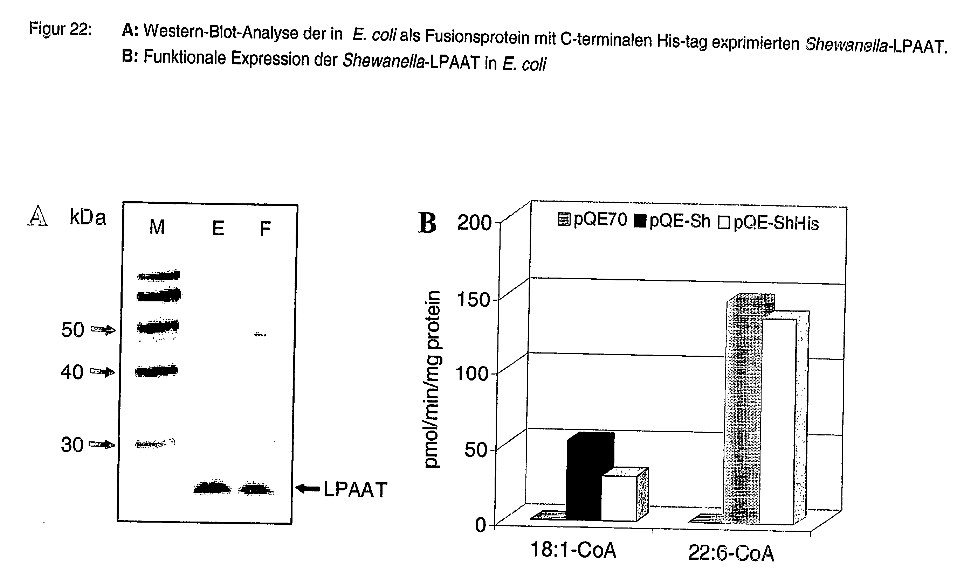

Zur Klonierung eines LPAAT-Gens aus dem prokaryoten Organismus *Shewanella* wurde die genomische DNA aus *Shewanella hanedai* isoliert, partiell mit Sau3a verdaut und in den Vektor pUC18 ligiert. Diese genomische Bank wurde mittels PCR unter Verwendung verschiedener Primerkombinationen auf LPAAT-Gene abgesucht. Mit dieser Methode ist es gelungen, einen 1486 bp langen Klon zu identifizieren, dessen offener Leserahmen ein 25,2 kDa LPAAT-Protein kodiert. Die ShLPAAT-Sequenz wurde gemäß Herstellerangaben in den Vektor pQE70 (Qiagen) eingebracht. Die so entstandenen Plasmid pQE70-Sh und pQE70-ShHis sowie der Leervektor pQE70 wurden in E. coli BL21 Zellen transformiert und bei 10 °C exprimiert (Figur 22 A). Nur bei dieser Temperatur konnte aktives Protein erhalten werden (Figur 22 B). Für die weiteren Versuche wurden dazu die Membranfraktionen verwendet. Diese Fraktion zeigten mit beiden Expressionsformen hohe Aktivität gegenüber dem Einbau von DHA-CoA (22:6-Co**A**). Die hohe Einbaurate gegenüber PUFA Acyl-CoA-Resten ist für die Verwendung zur Herstellung von PUFA notwendig.

Figur 22 **A**: zeigt die Western-Blot-Analyse der in *E. coli* als Fusionsprotein mit C-terminalen His-tag exprimierten *Shewanella*-LPAAT. (Spur E: 7 µg Einschlusskörperfraktion, Spur F: 7 µg Membranfraktion, Spur M: Größenstandard). Figur 22 **B**: gibt die funktionale Expression der *Shewanella*-LPAAT in *E*. *coli.* Enzymtests wieder. Die Assays wurden mit Extrakten (1 µg) aus *E*. *coli,* die den Leervektor (pQE70) oder ein Shewanella-Konstrukt ohne (pQE-Sh) bzw. mit His-Tag-Sequenz am 3'-Ende (pQE-ShHis) enthielten, in Gegenwart von 30 µM 1-Oleoyl-[U-¹⁴C]glycerin-3-phosphat und 30µM der angegebenen Thioster durchgeführt.

### Beispiel 16: Expression von Mortierella LPAAT (MaLPAAT, MaB4) in Hefe

Die MaLPAAT-cDNA wurde über PCR mit den angegebenen Primern MaLPAAT2.1 amplifiziert, das PCR Produkt in den Vektor pENTR-SD-D-TOPO (Invitrogen, Carlsbad, USA) nach Herstellerangaben kloniert und in E. coli XL1 blue transformiert. Aus dem so entstandenen Vektor pENTR-SD-D-MaLPAAT wurde über Gateway-Reaktion nach Herstellerangaben (Invitrogen, Carlsbad, USA) das MaLPAAT Fragment in den Vektor pYES54Dest transferiert, resultierend in dem Vektor pYES52Dest-MaLPAAT. PY-ES52Dest-MaLPAAT wurde mit Hilfe der LiAc-Methode in S. cerevisiae INCSc1 (Invitrogen, Carlsbad, USA) transformiert.

Hefezellen, die mit dem Plasmid pYES52Dest-MaLPAAT transformiert wurden, wurden folgendermaßen analysiert:

Hefekolonien, die auf Minimalmedium ohne Uracil nach der Transformation wachsen konnten, wurden erneut auf Minimalmedium ohne Uracil ausgestrichen und dann auf flüssigem Minimalmedium bis zu einer OD600 von 0,8 gezogen. Aus dieser Vorkultur wurde dann die Hauptkultur inokuliert, die neben dem Minimalmedium noch 2 % (w/v) Galaktose sowie 250 µM der Fettsäuren beinhaltet. Nach 24 h Inkubation der Hauptkultur bei 30 °C wurden die Zellen durch Zentrifugation (100 x g, 10 min, 20°C) geerntet und mit 100 mM NaHCO₃, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methonolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM NaHCO₃, pH 8,0 und 2 ml Aqua dest. gewaschen. Anschließend wurden die PE-Phasen mit Na₂SO₄ getrocknet, unter Argon eingedampft und in 100 µl PE aufgenommen. Die Proben wurden auf einer DB-23-Kapillarsäule (30 m, 0,25 mm, 0,25 µm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.

Die Identifikation der Signale erfolgte durch Vergleiche der Retentionszeiten mit entsprechenden Fettsäurestandards (Sigma).

Die Methodik ist zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218 beschrieben.

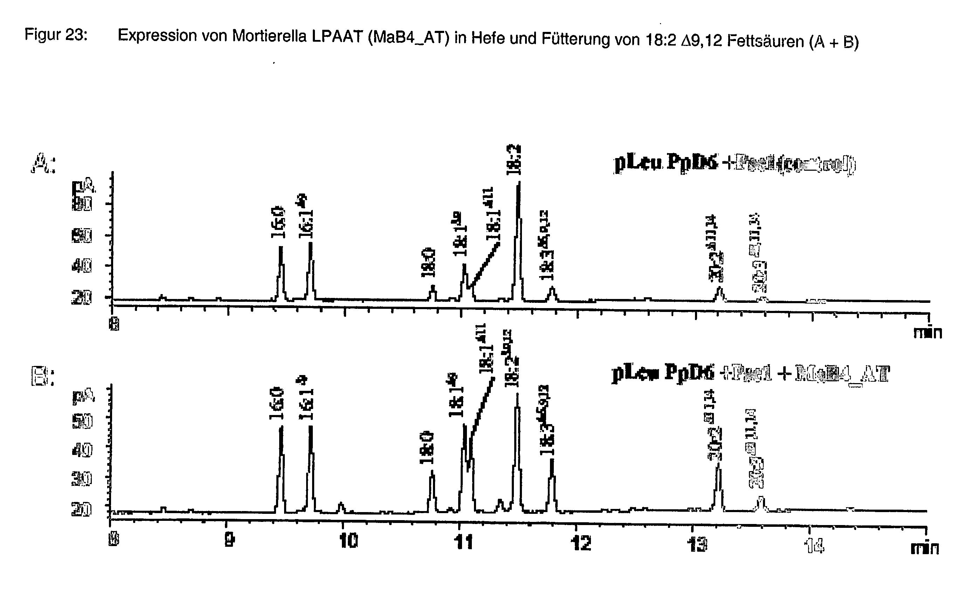

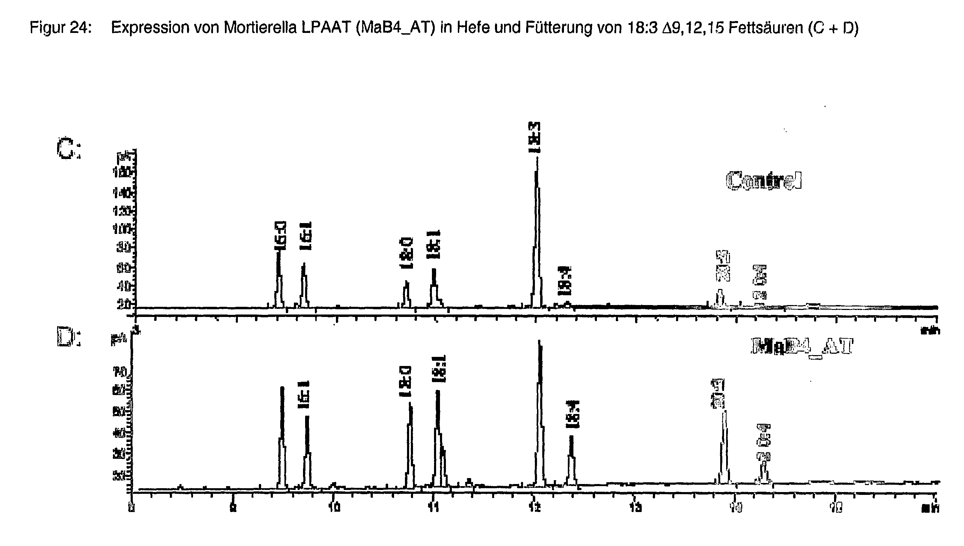

In Figur 23 sind die Ergebnisse der Fütterungsversuche mit den Hefezellen, die das Plasmid pYES52Dest-MaLPAAT (MaB4_AT) enthalten, gezeigt. In Fig. 23, A/B wurden die Hefe-Kulturen mit Linolsäure (18:2 Δ9,12) gefüttert. Im Vergleich zu der Kontroll-Kultur (Fig. 23, A) zeigten die Hefezellen mit der MaLPAAT deutlich höhere Umsetzung (4fach erhöht) von 18:2 zu γ-Linolensäure (18:3 Δ6,9,12), sowie eine 3,5fache Erhöhung der aus 18:2 elongierten Fettsäure 20:2 Δ11, 14. Entsprechend konnte bei der Fütterung mit Linolensäure (18:3 Δ9, 12, 15) eine deutlich höhere Umsetzung zu Stearidonsäure (18:4 Δ6,9,12,15) und iso-Arachidonsäure (20:4 Δ8,11,14,17) im Vergleich zu den Kontrollen beobachtet werden (Figur 24).

Neben dieser Aktivität konnte in beiden Fütterungsexperimenten eine verstärkte Umsetzung von 16:1 Δ9 (endogene Fettsäure in Hefe) zu cis-Vakzensäure (18:1 Δ11) beobachtet werden.

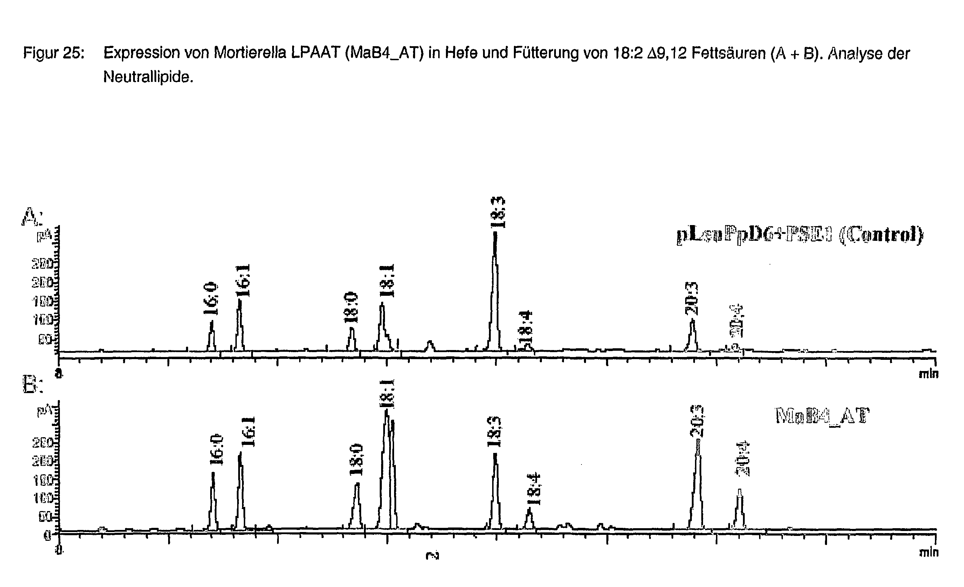

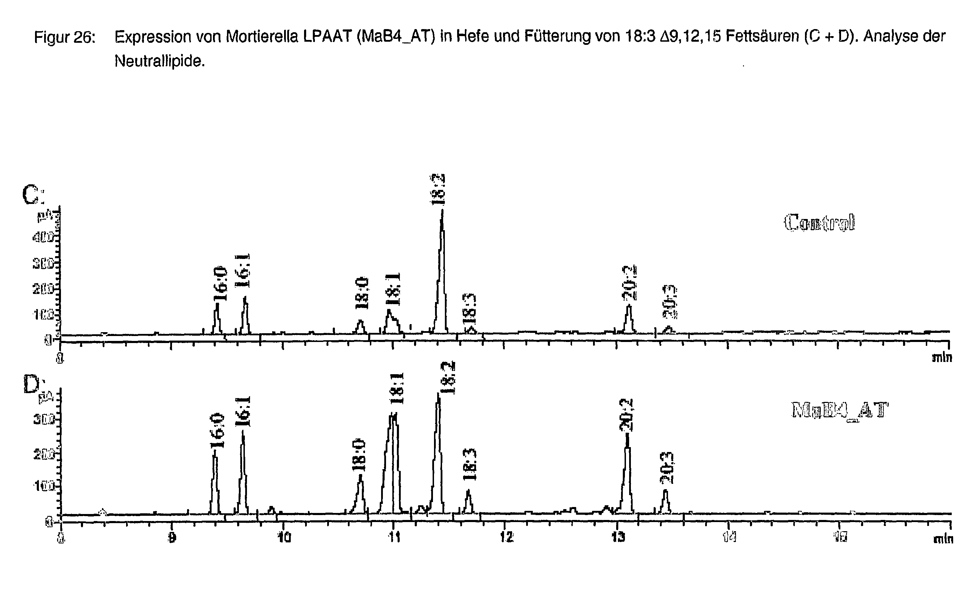

Figur 25 und Figur 26 zeigen, dass die beobachteten erhöhten Umsetzungen der Substrate durch die Desaturase und Elongase auch zu einer Erhöhung der polyungesättigten Fettsäuren in den Neutrallipid (Öl) führt. Nach Fütterung der Hefen mit Linol- bzw. Linolensäure wurden die Hefezellen in Chloroform:Methanol (2:1) extrahiert und auf eine Silica-Dünnschichtplatte (Machery&Nagel, Düren) aufgetragen. Die Dünnschichtplatte wurde in einer Kammer mit Chloroform-Methanol-H2O (65:25:4) für 45 min inkubiert. Die Neutrallipide (Triacylglyceride) wandern dabei mit der Lösungsmittelfront. Nach Ende der Inkubation wurden die Neutrallipide von der Platte abgekratzt, mit Chloroform:Methanol extrahiert und durch Gas-Chromatographie analysiert. Deutlich kann die Erhöhung des Umsatzes an PUFA's, die in den Gesamtextrakten beobachtet wurde, auch in den Neutrallipiden verfolgt werden. Für die Fütterung mit Linolsäure (Fig. 25 A und B) konnte eine 2fache Steigerung der Umsetzung von Linolsäure zu γ-Linolensäure (18:3 Δ6,9,12) und eine 3fache Erhöhung des Gehalts an 20:2 Δ9,12 beobachtet werden. Bei der Fütterung mit Linolensäure (Fig. 26, C und D) wurden ähnliche Werte erhalten (Umsetzung von 18:3 zu 18:4 3fach, von 18:3 zu 20:3 3fach).

Damit konnte gezeigt werden, dass die Erhöhung des Gehaltes an PUFA durch die MaLPAAT zu einer Erhöhung der PUFAs im Öl (Neutrallipde) der Hefen führt.

### Beispiel 16: Plasmide für die Pflanzentransformation

Zur Pflanzentransformation können binäre Vektoren, wie pBinAR verwendet werden (Höfgen und Willmitzer (1990) Plant Science 66: 5221-230). Die Konstruktion der binären Vektoren kann durch Ligation der cDNA in Sense- oder Antisense-Orientierung in T-DNA erfolgen. 5' der cDNA aktiviert ein Pflanzenpromotor die Transkription der cDNA. Eine Polyadenylierungssequenz befindet sich 3' von der cDNA.

Die gewebespezifische Expression läßt sich unter Verwendung eines gewebespezifischen Promotors erzielen. Beispielsweise kann die samenspezifische Expression erreicht werden, indem der Napin- oder der LeB4- oder der USP-Promotor 5' der cDNA einkloniert wird. Auch jedes andere samenspezifische Promotorelement kann verwendet werden. Zur konstitutiven Expression in der ganzen Pflanzen läßt sich der CaMV-35S-Promotor verwenden. Das exprimierte Protein kann unter Verwendung eines Signalpeptids, beispielsweise für Plastiden, Mitochondrien oder das Endoplasmatische Retikulum, in ein zelluläres Kompartiment dirigiert werden (Kermode (1996) Crit. Rev. Plant Sci. 15: 285-423). Das Signalpeptid wird 5' im Leseraster mit der cDNA einkloniert, um die subzelluläre Lokalisierung des Fusionsprotein zu erreichen.

### Beispiel 17: Transformation von Agrobacterium

Die Agrobacterium-vermittelte Pflanzentransformation kann z.B. unter Verwendung des GV3101- (pMP90-) (Koncz und Schell (1986) Mol. Gen. Genet. 204: 383-396) oder LBA4404- (Clontech) Agrobacterium tumefaciens-Stamms durchgeführt werden. Die Transformation kann durch Standard-Transformationstechniken durchgeführt werden (Deblaere et al. (1984) Nucl. Acids. Res. 13: 4777-4788).

### Beispiel 18: Pflanzentransformation und Expression von PUFA-spezifischen Acyltransferasen in Pflanzen

Die Expression von LCPUFA-spezifischen Acyltransferasen in transgenen Pflanzen ist vorteilhaft, um den LCPUFA-Gehalt in diesen Pflanzen zu erhöhen. Dazu wurden die erfindungsgemäßen Acyltransferase-cDNAs in binäre Vektoren kloniert und über Agrobacterium-vermittelten DNA-Transfer in Arabidopsis thaliana, Nicotiana tabacum, Brassica napus und Linum usitatissimum übertragen. Die Expression der Acyltransferase cDNA stand dabei unter der Kontrolle des konstitutiven CaMV 35 S-Promotors bzw. des samenspezifischen USP-Promotors.

Besonders bevorzugt sind hierbei transgene Pflanzen, die bereits die für Synthese von LCPUFAs notwendigen Desaturasen und Elongasen exprimieren und geringe Mengen dieser LCPUFAs herstellen.

Als Expressionsvektoren wurden der Vektor pBinAR (Höfgen und Willmitzer, Plant Science, 66, 1990: 221 - 230) bzw. das pBinAR Derivat pBinAR-USP, bei dem der CaMV 35 S-Promotor gegen den USP-Promotor aus V. faba ausgetauscht war, verwendet. Ebenfalls verwendet wurden die Vektoren pGPTV und pGPTV-USP. Zur Umklonierung mußte die CalDes-cDNA aus dem Vektor pGEM-T ausgeschnitten und in pBinAR bzw. pBinAR-USP kloniert werden. Ein weiterer verwendeter binärer Vektor war pSUN.

Die entstandenen binären Vektoren mit Acyltransferasegenen wurden in Agrobacterium tumefaciens transformiert (Höfgen und Willmitzer, Nucl. Acids Res., 16, 1988: 9877). Die Transformation von A. thaliana erfolgte mittels "floral dip" (Clough und Bent, Plant Journal, 16, 1998: 735 - 743), die von N. tabacum über Cokultivierung von Tabakblattstückchen mit transformierten A. tumefaciens Zellen, die von Lein und Raps durch Cokultivierung von Hypokotylstücken mit transformierten A. tumefaciens Zellen.

Die Expression der Acyltransferase-Gene in transgenen Arabidopsis-, Tabak-, Raps- und Leinpflanzen wurde über Northern-Blot Analyse untersucht. Ausgewählte Pflanzen wurden auf ihren Gehalt an Punicinsäure bzw. anderen konjugierten Fettsäuren wie CLA im Samenöl untersucht.

Analog zum USP-Promotor kann auch der Napin-Promotor verwendet werden, um eine samenspezifische Expression von PuFADX und PuFAD12 zu erreichen.

Die Agrobacterium-vermittelte Pflanzentransformation kann unter Verwendung von Standard-Transformations- und Regenerationstechniken durchgeführt werden (Gelvin, Stanton B., Schilperoort, Robert A., Plant Molecular Biology Manual, 2. Aufl., Dordrecht: Kluwer Academic Publ., 1995, in Sect., Ringbuc Zentrale Signatur: BT11-P ISBN 0-7923-2731-4; Glick, Bernard R., Thompson, John E., Methods in Plant Molecular Biology and Biotechnology, B. Raton: CRC Press, 1993, 360 S., ISBN 0-8493-5164-2).

Beispielsweise kann Raps mittels Kotyledonen- oder Hypokotyltransformation transformiert werden (Moloney et al., Plant Cell Report 8 (1989) 238-242; De Block et al., Plant Physiol. 91 (1989) 694-701). Die Verwendung von Antibiotika für die Agrobacterium- und Pflanzenselektion hängt von dem für die Transformation verwendeten binären Vektor und Agrobacteriumstamm ab. Die Rapsselektion wird gewöhnlich unter Verwendung von Kanamycin als selektierbarem Pflanzenmarker durchgeführt. Der Agrobacterium-vermittelte Gentransfer in Lein (Linum usitatissimum) läßt sich unter Verwendung von beispielsweise einer von Mlynarova et al. (1994) Plant Cell Report 13: 282-285 beschriebenen Technik durchführen.

Die Transformation von Soja kann unter Verwendung von beispielsweise einer in EP-A-O 0424047 (Pioneer Hi-Bred International) oder in EP-A-O 0397687, US 5,376,543, us 5,169,770 (University Toledo) beschriebenen Technik durchgeführt werden. Die Pflanzentransformation unter Verwendung von Teilchenbeschuss, Polyethylenglycol-vermittelter DNA-Aufnahme oder über die Siliziumcarbonatfaser-Technik ist beispielsweise beschrieben von Freeling und Walbot "The maize handbook" (1993) ISBN 3-540-97826-7, Springer Verlag New York).

### Beispiel 19: Untersuchung der Expression eines rekombinanten Genproduktes in einem transformierten Organismus

Die Aktivität eines rekombinanten Genproduktes im transformierten Wirtsorganismus wurde auf der Transkriptions- und/oder der Translationsebene gemessen.

Ein geeignetes Verfahren zur Bestimmung der Menge an Transkription des Gens (ein Hinweis auf die Menge an RNA, die für die Translation des Genproduktes zur Verfügung steht) ist die Durchführung eines Northern-Blots wie unten ausgeführt (als Bezugsstelle siehe Ausubel et al. (1988) Current Protocols in Molecular Biology, Wiley: New York, oder den oben erwähnten Beispielteil) wobei ein Primer, der so gestaltet ist, dass er an das Gen von Interesse bindet, mit einer nachweisbaren Markierung (gewöhnlich radioaktiv oder chemilumineszent) markiert wird, so dass, wenn die Gesamt-RNA einer Kultur des Organismus extrahiert, auf einem Gel aufgetrennt, auf eine stabile Matrix transferiert und mit dieser Sonde inkubiert wird, die Bindung und das Ausmaß der Bindung der Sonde das Vorliegen und auch die Menge der mRNA für dieses Gen anzeigt. Diese Information zeigt den Grad der Transkription des transformierten Gens an. Zelluläre Gesamt-RNA kann aus Zellen, Geweben oder Organen mit mehreren Verfahren, die alle im Fachgebiet bekannt sind, wie zum Beispiel das von Bormann, E.R., et al. (1992) Mol. Microbiol. 6:317-326 beschriebene, präpariert werden.

### Northern-Hybridisierung:

Für die RNA-Hybridisierung wurden 20 µg Gesamt-RNA oder 1 µg poly(A)⁺-RNA mittels Gelelektrophorese in Agarosegelen mit einer Stärke von 1,25% unter Verwendung von Formaldehyd, wie beschrieben in Arnasino (1986, Anal. Biochem. 152, 304) aufgetrennt, mittels Kapillaranziehung unter Verwendung von 10 x SSC auf positiv geladene Nylonmembranen (Hybond N⁺, Amersham, Braunschweig) übertragen, mittels UV-Licht immobilisiert und 3 Stunden bei 68°C unter Verwendung von Hybridisierungspuffer (10% Dextransulfat Gew./Vol., 1 M NaCl, 1 % SDS, 100 mg Heringssperma-DNA) vorhybridisiert. Die Markierung der DNA-Sonde mit dem Highprime DNA labeling-Kit (Roche, Mannheim, Deutschland) erfolgte während der Vorhybridisierung unter Verwendung von alpha-32P-dCTP (Amersham, Braunschweig, Deutschland). Die Hybridisierung wurde nach Zugabe der markierten DNA-Sonde im gleichen Puffer bei 68°C über Nacht durchgeführt. Die Waschschritte wurden zweimal für 15 min unter Verwendung von 2 X SSC und zweimal für 30 min unter Verwendung von 1 X SSC, 1 % SDS, bei 68°C durchgeführt. Die Exposition der verschlossenen Filter wurde bei -70°C für einen Zeitraum von 4 Stunden bis zu 3 Tagen durchgeführt.

Zur Untersuchung des Vorliegens oder der relativen Menge an von dieser mRNA translatiertem Protein können Standardtechniken, wie ein Western-Blot, eingesetzt werden (siehe beispielsweise Ausubel et al. (1988) Current Protocols in Molecular Biology, Wiley: New York). Bei diesem Verfahren werden die zellulären Gesamtproteine extrahiert, mittels Gelelektrophorese aufgetrennt, auf eine Matrix, wie Nitrozellulose, übertragen und mit einer

Sonde, wie einem Antikörper, der spezifisch an das gewünschte Protein bindet, inkubiert. Diese Sonde ist gewöhnlich mit einer chemilumineszenten oder kolorimetrischen Markierung versehen, die sich leicht nachweisen läßt. Das Vorliegen und die Menge der beobachteten Markierung zeigt das Vorliegen und die Menge des gewünschten, in der Zelle vorliegenden mutierten Proteins an.

### Beispiel 20: Analyse der Auswirkung der rekombinanten Proteine auf die Produktion des gewünschten Produktes

Die Auswirkung der genetischen Modifikation in Pflanzen, Pilzen, Algen, Ciliaten oder auf die Produktion einer gewünschten Verbindung (wie einer Fettsäure) kann bestimmt werden, indem die modifizierten Mikroorganismen oder die modifizierte Pflanze unter geeigneten Bedingungen (wie den vorstehend beschriebenen) gezüchtet werden und das Medium und/oder die zellulären Komponenten auf die erhöhte Produktion des gewünschten Produktes (d.h. von Lipiden oder einer Fettsäure) untersucht wird. Diese Analysetechniken sind dem Fachmann bekannt und umfassen Spektroskopie,

Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Bio chemistry and Molecular Biology, Bd. 17; Rehm et al. (1993) Biotechnology, Bd. 3, Kapitel III:

"Product recovery and purification", S. 469-714, VCH: Weinheim; Belter, P.A., et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren werden Pflanzenlipide aus Pflanzenmaterial wie von Cahoon et al. (1999) Proc. Natl. Acad. Sci. USA 96 (22) :12935-12940, und Browse et al. (1986) Analytic Biochemistry 152:141-145, beschrieben extrahiert. Die qualitative und quantitative Lipid- oder Fettsäureanalyse ist beschrieben bei Christie, Williarn W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergarnon Press, 1 (1952) -16 (1977) u.d.T.: Progress in the Chemistry of Fats and Other Lipids CODEN.

Zusätzlich zur Messung des Endproduktes der Fermentation ist es auch möglich, andere Komponenten der Stoffwechselwege zu analysieren, die zur Produktion der gewünschten Verbindung verwendet werden, wie Zwischen- und Nebenprodukte, um die Gesamteffizienz der Produktion der Verbindung zu bestimmen. Die Analyseverfahren umfassen Messungen der Nährstoffmengen im Medium (z.B. Zucker, Kohlenwasserstoffe, Stickstoffquellen, Phosphat und andere Ionen), Messungen der Biomassezusammensetzung und des Wachstums, Analyse der Produktion üblicher Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsgb., IRL Press, 10 S. 131-163 und 165-192 (ISBN: 0199635773) und darin angegebenen Literaturstellen beschrieben.

Ein Beispiel ist die Analyse von Fettsäuren (Abkürzungen: FAME, Fettsäuremethylester; GC-MS, Gas-Flüssigkeitschromatographie-Massenspektrometrie; TAG, Triacylglycerin; TLC, Dünnschichtchromatographie).

Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl. : Christie, Oily Press, Dundee, 119-169; 1998, Gaschromatographie-Massenspektrometrie-Verfahren, Lipide 33:343-353).

Das zu analysierende Material kann durch Ultraschallbehandlung, Mahlen in der Glasmühle, flüssigen Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei I00°C erhitzt, auf Eis abgekühlt und erneut zentrifugiert, gefolgt von

Extraktion in 0,5 M Schwefelsäure in Methanol mit 2 % Dimethoxypropan für 1 Std. bei 90°C, was zu hydrolysierten Öl- und Lipidverbindungen führt, die transmethylierte Lipide ergeben. Diese Fettsäuremethylester werden in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen.

Die Identität der erhaltenen Fettsäuremethylester muss unter Verwendung von Standards, die aus kommerziellen Quellen erhältlich sind (d.h. Sigma), definiert werden.

Bei Fettsäuren, für die keine Standards verfügbar sind, muss die Identität über Derivatisierung und anschließende GC-MS-Analyse gezeigt werden. Beispielsweise muss die Lokalisierung von Fettsäuren mit Dreifachbindung über GC-MS nach Derivatisierung mit 4,4-Dimethoxyoxazolin-Derivaten (Christie, 1998, siehe oben) gezeigt werden.

## Patentansprüche

1. Verfahren zur Herstellung mehrfach ungesättigter Fettsäuren in einer Ölfruchtpflanze, **dadurch gekennzeichnet, dass** das Verfahren folgende Schritte umfasst:
a) Einbringen mindestens einer Nukleinsäuresequenz in die Pflanze mit der in SEQ ID NO: 1oder SEQ ID NO: 20 dargestellten Sequenz, die für ein Polypeptid mit einer Lysophosphatidsäure Acyltransferase-Aktivität codiert; oder
b) Einbringen mindestens einer Nukleinsäuresequenz in die Pflanze, die sich als Ergebnis des degenerierten genetischen Codes von der in SEQ ID NO: 4oder SEQ ID NO: 20 enthaltenen codierenden Sequenz ableiten lässt, oder
c) Einbringen einer Nukleinsäuresequenz in die Pflanze, die für Polypeptide mit mindestens 80 % Homologie auf Aminosäureebene mit SEQ ID NO: 2 oder SEQ ID NO: 21 codieren und eine Lysophosphatidsäure Acyltransferase-Aktivität aufweisen, und
d) Kultivieren und Ernten des Organismus.

2. Verfahren zur Herstellung mehrfach ungesättigter Fettsäuren nach Anspruch 1, **dadurch gekennzeichnet, dass** zusätzlich zu den unter (a) bis (d) genannten Nukleinsäuresequenzen weitere Nukleinsäuresequenzen in den Organismus eingebracht wurden, die für Polypeptide des Fettsäure-oder Lipidstoffwechsels ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) codieren.

3. Verfahren zur Herstellung mehrfach ungesättigter Fettsäuren nach Anspruch 1 oder 2, **dadurch gekennzeichnet, dass** zusätzlich zu den unter (a) bis (d) genannten Nukleinsäuresequenzen weitere Nukleinsäuresequenzen in den Organismus eingebracht wurden, die für Polypeptide codieren ausgewählt aus der Gruppe Acyl-CoA:Lysophospholipid-Acyltransferase, delta-4-Desaturase, delta-5-Desaturase, delta-6-Desaturase, delta-8-Desatuase, delta-9-Desaturase, delta-12-Desaturase, delta-5-Elongase, delta-6-Elongase oder delta-9-Elongase.

4. Verfahren zur Herstellung mehrfach ungesättigter Fettsäuren nach einem der Ansprüche 1 bis 3, **dadurch gekennzeichnet, dass** es sich bei den hergestellten mehrfach ungesättigten Fettsäuren um C18-, C20-, C22- oder C24-Fettsäuren handelt.

5. Verfahren zur Herstellung mehrfach ungesättigter Fettsäuren nach einem der Ansprüche 1 bis 4, **dadurch gekennzeichnet, dass** die mehrfach ungesättigten Fettsäuren aus dem Organismus in Form eines Öls, Lipids oder einer freien Fettsäure isoliert werden.

6. Verfahren zur Herstellung mehrfach ungesättigter Fettsäuren nach einem der Ansprüche 1 bis 5, **dadurch gekennzeichnet, dass** es sich bei den im Verfahren hergestellten mehrfach ungesättigten Fettsäuren um C18-, C20-, C22- oder C24-Fettsäuren mit mindestens zwei Doppelbindungen im Molekül handelt.

7. Verfahren zur Herstellung mehrfach ungesättigter Fettsäuren nach einem der Ansprüche 1 bis 6, **dadurch gekennzeichnet, dass** im Verfahren eine mehrfach ungesättigte Fettsäure ausgewählt aus der Gruppe Eisosapentaensäure, Docosapentaensäure und Docosahexaensäure hergestellt wird.

8. Nukleinsäuresequenz ausgewählt aus der Gruppe:
a) einer Nukleinsäuresequenz mit der in SEQ ID NO: 1oder SEQ ID NO: 20 dargestellten Sequenz,
b) Nukleinsäuresequenzen, die sich als Ergebnis des degenerierten genetischen Codes von der in SEQ ID NO: 1oder SEQ ID NO: 20 enthaltenen codierenden Sequenz ableiten lassen,
c) Nukleinsäuresequenzen, die für Polypeptide mit mindestens 80 % Identität auf Aminosäureebene mit SEQ ID NO: 2 oder SEQ ID NO: 21 codieren und eine Lysophosphatidsäure Acyltransferase-Aktivität aufweisen.

9. Aminosäuresequenz, die von einer Nukleinsäuresequenz nach Anspruch 8 codiert wird und Lysophosphatidsäure Acyltransferase-Aktivität besitzt.

10. Genkonstrukt, enthaltend eine Nukleinsäure nach Anspruch 8, wobei die Nukleinsäure funktionsfähig mit einem oder mehreren Regulationssignalen verbunden ist.

11. Genkonstrukt nach Anspruch 10, **dadurch gekennzeichnet, dass** das Genkonstrukt zusätzliche Biosynthesegene des Fettsäure- oder Lipidstoffwechsels enthält ausgewählt aus der Gruppe Acyl-CoA-Dehydrogenase(n), Acyl-ACP[= acyl -carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n).

12. Genkonstrukt nach Anspruch 10 oder 11, **dadurch gekennzeichnet, dass** das Genkonstrukt zusätzliche Biosynthesegene des Fettsäure-oder Lipidstoffwechsels enthält ausgewählt aus der Gruppe der Acyl-CoA:Lysophospholipid-Acyltransferase, delta-4-Desaturase, delta-5-Desaturase, delta-6-Desaturase, delta-8-Desatuase, delta-9-Desaturase, delta-12-Desaturase, delta-5-Elongase, delta-6-Elongase oder delta-9-Elongase.

13. Vektor, enthaltend eine Nukleinsäure nach Anspruch 8 oder ein Genkonstrukt nach einem der Ansprüche 10 bis 12.

14. Ölfruchtpflanze, enthaltend mindestens eine Nukleinsäure nach Anspruch 8, ein Genkonstrukt nach einem der Ansprüche 10 bis 12 oder einen Vektor nach Anspruch 13.

15. Verwendung einer Lysophosphatidsäure Acyltransferase gemäß Anspruch 9 zum Herstellen eines Pflanzenöls in einer Ölfruchtpflanze.

## Claims

1. A process for the production of polyunsaturated fatty acids in an oil crop plant, which comprises the following steps:
a) introducing, into the plant, at least one nucleic acid sequence with the sequence shown in SEQ ID NO: 1 or SEQ ID NO: 20, which codes for a polypeptide with lysophosphatidic acid acyltransferase activity; or
b) introducing, into the plant, at least one nucleic acid sequence which can be derived from the coding sequence in SEQ ID NO: 1 or SEQ ID NO: 20 as the result of the degeneracy of the genetic code, or
c) introducing, into the plant, a nucleic acid sequence which codes for polypeptides having at least 80% homology at the amino acid level with SEQ ID NO: 2 or SEQ ID NO: 21 and has a lysophosphatidic acid acyltransferase activity, and
d) culturing and harvesting the organism.

2. The process for the production of polyunsaturated fatty acids according to claim 1, wherein, additionally to the nucleic acid sequences mentioned under (a) to (d), further nucleic acid sequences which code for polypeptides of the fatty acid metabolism or lipid metabolism selected from the group consisting of acyl-CoA dehydrogenase(s), acyl-ACP[= acyl carrier protein] desaturase(s), acyl-ACP thioesterase(s), fatty acid acyltransferase(s), acyl-CoA:lysophospholipid acyltransferase(s), fatty acid synthase(s), fatty acid hydroxylase(s), acetyl-coenzyme A carboxylase(s), acyl-coenzyme A oxidase(s), fatty acid desaturase(s), fatty acid acetylenase(s), lipoxygenase(s), triacylglycerol lipase(s), allene oxide synthase(s), hydroperoxide lyase(s) or fatty acid elongase(s) were introduced into the organism.

3. The process for the production of polyunsaturated fatty acids according to claim 1 or 2, wherein, additionally to the nucleic acid sequences mentioned under (a) to (d), further nucleic acid sequences which code for polypeptides selected from the group consisting of acyl-CoA:lysophospholipid acyltransferase, delta-4-desaturase, delta-5-desaturase, delta-6-desaturase, delta-8-desaturase, delta-9-desaturase, delta-12-desaturase, delta-5-elongase, delta-6-elongase or delta-9-elongase, were introduced into the organism.

4. The process for the production of polyunsaturated fatty acids according to any of claims 1 to 3, wherein the polyunsaturated fatty acids produced are C18-, C20-, C22- or C24-fatty acids.

5. The process for the production of polyunsaturated fatty acids according to any of claims 1 to 4, wherein the polyunsaturated fatty acids are isolated from the organism in the form of an oil, a lipid or a free fatty acid.

6. The process for the production of polyunsaturated fatty acids according to any of claims 1 to 5, wherein the polyunsaturated fatty acids produced in the process are C18-, C20-, C22- or C24-fatty acids with at least two double bonds in the molecule.

7. The process for the production of polyunsaturated fatty acids according to any of claims 1 to 6, wherein a polyunsaturated fatty acid selected from the group consisting of eicosapentaenoic acid, docosapentaenoic acid and docosahexaenoic acid is produced in the process.

8. A nucleic acid sequence selected from the group consisting of:
a) a nucleic acid sequence with the sequence shown in SEQ ID NO: 1 or SEQ ID NO: 20,
b) nucleic acid sequences which, as the result of the degeneracy of the genetic code, can be derived from the coding sequence in SEQ ID NO: 1 or SEQ ID NO: 20,
c) nucleic acid sequences which code for polypeptides having at least 80% identity at the amino acid level with SEQ ID NO: 2 or SEQ ID NO: 21 and have lysophosphatidic acid acyltransferase activity.

9. An amino acid sequence which is encoded by a nucleic acid sequence according to claim 8 and has lysophosphatidic acid acyltransferase activity.

10. A gene construct comprising a nucleic acid according to claim 8, where the nucleic acid is linked functionally to one or more regulatory signals.

11. The gene construct according to claim 10, wherein the gene construct comprises additional biosynthesis genes of the fatty acid metabolism or lipid metabolism selected from the group consisting of acyl-CoA dehydrogenase(s), acyl-ACP[= acyl carrier protein] desaturase(s), acyl-ACP thioesterase(s), fatty acid acyltransferase(s), acyl-CoA:lysophospholipid acyltransferase(s), fatty acid synthase(s), fatty acid hydroxylase(s), acetyl-coenzyme A carboxylase(s), acyl-coenzyme A oxidase(s), fatty acid desaturase(s), fatty acid acetylenase(s), lipoxygenase(s), triacylglycerol lipase(s), allene oxide synthase(s), hydroperoxide lyase(s) or fatty acid elongase(s).

12. The gene construct according to claim 10 or 11, wherein the gene construct comprises additional biosynthesis genes of the fatty acid metabolism or lipid metabolism selected from the group consisting of acyl-CoA:lysophospholipid acyltransferase, delta-4-desaturase, delta-5-desaturase, delta-6-desaturase, delta-8-desaturase, delta-9-desaturase, delta-12-desaturase, delta-5-elongase, delta-6-elongase or delta-9-elongase.

13. A vector comprising a nucleic acid according to claim 8 or a gene construct according to any of claims 10 to 12.

14. An oil crop plant comprising at least one nucleic acid according to claim 8, a gene construct according to any of claims 10 to 12 or a vector according to claim 13.

15. The use of a lyophosphatidic acid acyltransferase according to claim 9 for producing a vegetable oil in an oil crop plant.

## Revendications

1. Procédé de préparation d'acides gras polyinsaturés dans une plante oléagineuse, **caractérisé en ce que** le procédé comprend les étapes suivantes :
a) introduction d'au moins une séquence d'acide nucléique dans la plante, ayant la séquence présentée dans SEQ ID N° 1 ou SEQ ID N° 20, qui code pour un polypeptide ayant une activité d'acide lysophosphatidique acyltransférase ; ou
b) introduction d'au moins une séquence d'acide nucléique dans la plante, qui peut dériver, en conséquence du code génétique dégénéré, de la séquence codante contenue dans SEQ ID N° 1 ou SEQ ID N° 20, ou
c) introduction d'une séquence d'acide nucléique dans la plante, qui code pour des polypeptides ayant une homologie d'au moins 80 % au plan des acides aminés avec SEQ ID N° 2 ou SEQ ID N° 21 et présente une activité d'acide lysophosphatidique acyltransférase, et
d) culture et récolte de l'organisme.

2. Procédé de préparation d'acides gras polyinsaturés selon la revendication 1, **caractérisé en ce que**, en plus des séquences d'acides nucléiques mentionnées en (a) à (d), des séquences d'acides nucléiques supplémentaires ont été introduites dans l'organisme, qui codent pour des polypeptides du métabolisme des acides gras ou des lipides, choisis dans le groupe consistant en la ou les acyl-CoA-déshydrogénases, la ou les acyl-ACP[= protéine transporteuse d'acyle]-désaturases, la ou les acyl-ACP-thioestérases, la ou les acide gras-acyl-transférases, la ou les acyl-CoA:lysophospholipide-acyltransférases, la ou les acide gras-synthases, la ou les acide gras-hydroxylases, la ou les acétyl-coenzyme A-carboxylases, la ou les acyl-coenzyme A-oxydases, la ou les acide gras-désaturases, la ou les acide gras-acétylénases, la ou les lipoxygénases, la ou les triacylglycérol-lipases, la ou les oxyde d'allène-synthases, la ou les hydroperoxyde-lyases ou la ou les acide gras-élongases.

3. Procédé de préparation d'acides gras polyinsaturés selon la revendication 1 ou 2, **caractérisé en ce que**, en plus des séquences d'acides nucléiques mentionnées en (a) à (d), on a introduit dans l'organisme des séquences d'acides nucléiques supplémentaires, qui codent pour des polypeptides choisis dans le groupe consistant en l'acyl-CoA:lysophospholipideacyltransférase, la delta-4-désaturase, la delta-5-désaturase, la delta-6-désaturase, la delta-8-désaturase, la delta-9-désaturase, la delta-12-désaturase, la delta-5-élongase, la delta-6-élongase ou la delta-9-élongase.

4. Procédé de préparation d'acides gras polyinsaturés selon l'une des revendications 1 à 3, **caractérisé en ce que**, pour ce qui concerne les acides gras polyinsaturés préparés, il s'agit d'acides gras en C18, en C20, en C22 ou en C24.

5. Procédé de préparation d'acides gras polyinsaturés selon l'une des revendications 1 à 4, **caractérisé en ce que** les acides gras polyinsaturés sont isolés de l'organisme sous forme d'une huile, d'un lipide ou d'un acide gras libre.

6. Procédé de préparation d'acides gras polyinsaturés selon l'une des revendications 1 à 5, **caractérisé en ce que**, pour ce qui concerne les acides gras polyinsaturés préparés par ce procédé, il s'agit d'acides gras en C18, C20, C22 ou C24, ayant au moins deux doubles liaisons dans leur molécules.

7. Procédé de préparation d'acides gras polyinsaturés selon l'une des revendications 1 à 6, **caractérisé en ce que**, dans le procédé, on prépare un acide gras polyinsaturé choisi dans le groupe consistant en l'acide éicosapentaénoïque, l'acide docosapentaénoïque et l'acide docosahexaénoïque.

8. Séquence d'acide nucléique choisie dans le groupe
a) d'une séquence d'acide nucléique ayant la séquence présentée dans SEQ ID N° 1 ou SEQ ID N° 20,
b) des séquences d'acides nucléiques qui, en conséquence du code génétique dégénéré, dérivent de la séquence codante contenue dans SEQ ID N° 1 ou SEQ ID N° 20,
c) des séquences d'acides nucléiques qui codent pour des polypeptides ayant une identité d'au moins 80 % au niveau des acides aminés avec SEQ ID N° 2 ou SEQ ID N° 21 et présentent une activité d'acide lysophosphatidique acyltransférase.

9. Séquence d'acides aminés, qui est codée par une séquence d'acide nucléique selon la revendication 8 et possède une activité d'acide lysophosphatidique acyltransférase.

10. Construction génique contenant un acide nucléique selon la revendication 8, l'acide nucléique étant fonctionnellement lié à un ou plusieurs signaux de régulation.

11. Construction génique selon la revendication 10, **caractérisée en ce que** la construction génique contient des gènes supplémentaires de la biosynthèse du métabolisme des acides gras ou des lipides, choisis dans le groupe consistant en la ou les acyl-CoA-déshydrogénases, la ou les acyl-ACP[= protéine transporteur d'acyle]-désaturases, la ou les acyl-ACP-thioestérases, la ou les acide gras-acyl-transférases, la ou les acyl-CoA:lysophospholipide-acyltransférases, la ou les acide gras-synthases, la ou les acide gras-hydroxylases, la ou les acétyl-coenzyme A-carboxylases, la ou les acyl-coenzyme A-oxydases, la ou les acide gras-désaturases, la ou les acide gras-acétylénases, la ou les lipoxygénases, la ou les triacylglycérol-lipases, la ou les oxyde d'allène-synthases, la ou les hydroperoxyde-lyases ou les acides gras-élongases.

12. Construction génique selon la revendication 10 ou 11, **caractérisée en ce que** la construction génique contient des gènes supplémentaires de la biosynthèse du métabolisme des acides gras ou des lipides, choisis dans le groupe de l'acyl-CoA:lysophospholipide-acyltransférase, de la delta-4-désaturase, de la delta-5-désaturase, de la delta-6-désaturase, de la delta-8-désaturase, de la delta-9-désaturase, de la delta-12-désaturase, de la delta-5-élongase, de la delta-6-élongase ou de la delta-9-élongase.

13. Vecteur contenant un acide nucléique selon la revendication 8 ou une construction génique selon l'une des revendications 10 à 12.

14. Plante oléagineuse contenant au moins un acide nucléique selon la revendication 8, une construction génique selon l'une des revendications 10 à 12 ou un vecteur selon la revendication 13.

15. Utilisation d'une acide lysophosphatidique acyltransférase selon la revendication 9 pour préparer une huile végétale dans une plante oléagineuse.
